# EUROPEAN PATENT SPECIFICATION

(11) **EP 1 661 897 B1**
(45) Date of publication and mention of the grant of the patent: **04.12.2013**
(21) Application number: 04772648.4
(22) Date of filing: 26.08.2004
(51) Int. Cl.: C07D 487/04, A61K 31/519, A61P 3/10, A61P 17/14, A61P 25/24, A61P 25/28, A61P 29/00, A61P 35/00, A61P 37/02, A61P 43/00

(54) **PYRROLOPYRIMIDINONE DERIVATIVE**
PYRROLOPYRIMIDINONDERIVAT
DERIVE DE PYRROLOPYRIMIDINONE

(30) Priority: 26.08.2003 JP 2003301022; 30.03.2004 JP 2004100022
(43) Date of publication of application: 31.05.2006
(73) Proprietor: Teijin Pharma Limited, Tokyo 100-8585 (JP)
(72) Inventor: TSUTSUMI, Takaharu, c/o Teijin Pharma Limited, Hino-shi, Tokyo 1910065 (JP); SUGIURA, Satoshi, c/o Teijin Pharma Limited, Hino-shi, Tokyo 1910065 (JP); KOGA, Masahiro, Yamaguchi Pref. 740-0032 (JP); MATSUMOTO, Yoshiyuki, c/o Teijin Pharma Limited, Hino-shi, Tokyo 1910065 (JP); ISHII, Toshihiro, c/o Teijin Pharma Limited, Hino-shi, Tokyo 1910065 (JP); NAKANO, Akira, c/o Teijin Pharma Limited, Hino-shi, Tokyo 1910065 (JP); UNOKI, Gen, c/o Teijin Pharma Limited, Hino-shi, Tokyo 1910065 (JP); SAKAI, Yuri, c/o Teijin Pharma Limited, Hino-shi, Tokyo 1910065 (JP); TAKARADA, Reiko, c/o Teijin Pharma Limited, Hino-shi, Tokyo 1910065 (JP); OGAWA, Hiroko, c/o Teijin Pharma Limited, Hino-shi, Tokyo 1910065 (JP)
(74) Representative: Hallybone, Huw George
(86) International application number: PCT/JP2004/012692
(87) International publication number: WO 2005/019219

(56) References cited:
- WO-A1-02/18371
- WO-A1-95/01355
- WO-A1-97/49706
- WO-A1-02/085909
- WO-A1-03/070729
- WO-A1-2004/018496
- WO-A2-03/100009
- JP-A- 02 204 414
- JP-A- 07 507 062
- JP-A- 10 506 126
- JP-A- 58 026 888
- JP-A- 2001 519 437
- US-A1- 2003 096 813
- US-A1- 2003 114 466
- US-B1- 6 458 799
- EVANS GARY B. ET AL.: 'Exploring structure-activity relationships of transition state analogues of human ourine nucleoside phosphorylase' JOURNAL OF MEDICINAL CHEMISTRY vol. 46, no. 15, 2003, pages 3412 - 3423, XP008037412
- LEWANDOWICZ ANDRZEJ ET AL.: 'Achieving the ultimate physiological goal in transition state analogue inhibitors for purine nucleoside phosphorylase' JOURNAL OF BIOLOGICAL CHEMISTRY vol. 278, no. 34, 2003, pages 31465 - 31468, XP008037416
- LEWANDOWICZ ANDRZEJ ET AL.: 'Over-the-barrier transition state analogues and crystal structure with mycobacterium tuberculosis purine nucleoside phosphorylase' BIOCHEMISTRY vol. 42, no. 20, 2003, pages 6057 - 6066, XP002982670
- SHIH HSIENCHENG ET AL.: 'Facile synthesis of 9-substituted 9-deazapurines as potential purine nucleoside phosphorylase inhibitors' CHEMICAL & PHARMACEUTICAL BULLETIN vol. 50, no. 3, 2002, pages 364 - 367, XP002982671
- CHUN BYOUNG K. ET AL.: 'Stereocontrolled syntheses of carbocyclic C-nucleosides and related compounds' JOURNAL OF ORGANIC CHEMISTRY vol. 66, no. 14, 2001, pages 4852 - 4858, XP002982672
- ANDRICOPULO ADRIANO D. ET AL.: 'Structure-activity relationships for a collection of structurally diverse inhibitors of purine nucleoside phosphorylase' CHEMICAL & PHARMACEUTICAL BULLETIN vol. 49, no. 1, 2001, pages 10 - 17, XP001183767
- FURNEAUX RICHARD H. ET AL.: 'Improved synthesis of 3H,5H-pyrrolo[3,2-d]pyrimidines' JOURNAL OF ORGANIC CHEMISTRY vol. 64, no. 22, 1999, pages 8411 - 8412, XP001038388
- FARUTIN VICTOR ET AL.: 'Structure-activity relationships for a class of inhibitors of purine nucleoside phosphorylase' JOURNAL OF MEDICINAL CHEMISTRY vol. 42, no. 13, 1999, pages 2422 - 2431, XP002982673
- LIM MU ILL ET AL.: 'Synthesis of "9-deazaguanosine" and other new pyrrolo[3,2-d]pyrimidine C-nucleosides' JOURNAL OF ORGANIC CHEMISTRY vol. 48, no. 6, 1983, pages 780 - 788, XP002982674
- JUKIC LUCIJA ET AL.: 'A synthesis and transformations of alkyl 2-[2-cyano-2-(2-pyridinyl)ethenyl]amino-3-d imethylaminopropenoates. A one-pot synthesis of pyrrolo[3,2-d]pyrimidine-4-ones' HETEROCYCLES vol. 53, no. 4, 2000, pages 805 - 820, XP002982675
- NIWAS SHRI; CHAND POORAN; PATHAK VED P; MONTGOMERY JOHN A: "Structure-based design of inhibitors of purine nucleoside phosphorylase. 5. 9-Deazahypoxanthines.", JOURNAL OF MEDICINAL CHEMISTRY 1994, vol. 37, no. 15, 1994, pages 2477-2480,
- THORNBER C W: "ISOSTERISM AND MOLECULAR MODIFICATION IN DRUG DESIGN", CHEMICAL SOCIETY REVIEWS, CHEMICAL SOCIETY, LONDON, GB, vol. 8, no. 4, 1 January 1979 (1979-01-01) , pages 563-580, XP001167283, ISSN: 0306-0012, DOI: 10.1039/CS9790800563

## Description

### TECHNICAL FIELD

The present invention relates to novel pyrrolopyrimidinone derivatives that have an action inhibiting glycogen synthase kinase-3 (GSK-3). More particularly, the invention relates to novel pyrrolo[3,2-d] pyrimidinone derivatives useful as pharmaceutical agents for treating and/or preventing disorders mediated by GSK-3 activity, particularly, impaired glucose tolerance, type I diabetes, type II diabetes, diabetic complications (retinopathy, nephropathy, neuropathy or great vessel hindrance), Alzheimer's disease, neurodegenerative diseases (AIDS encephalophy, Huntington's disease, Parkinson's disease, amyotrophic lateral sclerosis or multiple sclerosis), bipolar affective disorder (manic depressive psychosis), traumatic cerebrospinal injury, epilepsy, obesity, atherosclerosis, hypertension, polycystic ovary syndrome, syndrome X, alopecia, inflammatory diseases (arthrosis deformans, rheumatism, atopic dermatitis, psoriasis, ulcerative colitis, Crohn's disease, sepsis or systemic inflammatory response syndrome), cancer and immunodeficiency.

### BACKGROUND ART

GSK-3 is a serine/threonine protein kinase. Two isoforms, i.e., α and β, which are encoded by distinct genes, have been identified (see Trends Biochem. Sci., 1991, Vol. 16, p. 177).
Both GSK-3 isoforms have a monomeric structure and are constitutively active in resting cells. GSK-3 was originally identified as a kinase that inhibits glycogen synthase by direct phosphorylation (see Eur. J. Biochem., 1980, Vol. 107, p. 519). Upon insulin activation, GSK-3 is inactivated, thereby allowing the activation of glycogen synthase and possibly other insulin-dependent events, such as glucose transport. Also, it has been known that GSK-3 activity is inactivated by other growth factors, such as IGF-1 or FGF, through signaling from preceptor tyrosine kinases (see Biochem. J., UK, 1993, Vol. 294, p. 625; Biochem. J., UK, 1994, Vol. 303, p. 21; Biochem. J., UK, 1994, Vol. 303, p. 27).

GSK-3 inhibitors are useful in the treatment of disorders that are mediated by GSK-3 activity. In addition, inhibition of GSK-3 mimics the activation of growth factor signaling pathways and consequently GSK-3 inhibitors are useful in the treatment of diseases in which such pathways are inactive. Examples of diseases that can be treated with GSK-3 inhibitors are described below.

Type I diabetes is induced due to autoimmune destruction of β cells as pancreatic insulin production cells, resulting in deficiency of insulin. From this, it is necessary for a type I diabetic patient to routinely be administered insulin for maintaining life. However, in current insulin therapy, strict control of the blood glucose levels like the ability of normal β cells cannot be reproduced. Thus, type I diabetes is liable to induce diabetic complications such as retinopathy, nephropathy, neuropathy, great vessels hindrance or the like.

Type II diabetes is a multifactorial disease. Hyperglycemia is due to insulin resistance in the liver, skeletal muscle and lipid tissues coupled with inadequate or defective secretion of insulin from pancreatic islets. As a result, diabetic complications such as retinopathy, nephropathy, neuropathy, or great vessels hindrance are induced. Skeletal muscle is the major site for insulin-stimulated glucose uptake, and glucose removed from the circulation is either metabolized through glycolysis and the TCA cycle or stored as glycogen. Muscle glycogen deposition plays a very important role in glucose homeostasis, and Type II diabetic subjects have defective muscle glycogen storage. GSK-3, which is known to phosphorylate glycogen synthase, inhibits the accumulation of glycogen in peripheral tissues and lowers the reactivity of insulin, leading to an increase in blood level of glucose.

Recently, it has been reported that the expression of GSK-3 is stimulated in skeletal muscles of type II diabetic patients, and the GSK-3α activity and insulin in skeletal muscles are inversely correlated (see Diabetes, USA, 2000, Vol. 49, p. 263). Where GSK-3β and active GSK-3β variants (S9A, S9E) are overexpressed in HEK-293 cells, the GSK activity is inhibited (see Proc. Natl. Acad. Sci., USA, 1996, Vol. 93, p. 10228). In CHO cells in which insulin receptor and insulin receptor substrate 1 (IRS-1) are expressed, overexpression of GSK-3β brings about a decrease in the insulin activity (see 8: Proc. Natl. Acad. Sci., USA, 1997, Vol. 94, 9660). Recent research carried out using C57BL/6J mice with pyknic type diabetes has clearly shown that GSK-3 activity stimulation and insulin resistance are correlated to the progress of type II diabetes (see Diabetes, USA, 1999, Vol. 48, p. 1662).

Conventionally, lithium salts have been known to have inhibitory effects of GSK-3 activity (see Proc. Natl. Acad. Sci., USA, 1996, Vol. 93, p. 8455). It has been reported that the therapy using the lithium salts lowers glucose levels in both type I and II diabetic patients, thereby alleviating the severity of the disease (see Biol. Trace Elements Res., 1997, Vol. 60, p. 131). However, lithium salts have also been found to exhibit various side effects on molecular targets other than GSK-3.

From the findings described above, it can be concluded that GSK-3 inhibitors are effective therapeutics for the treatment of impaired glucose tolerance, type I diabetes, type II diabetes and complications thereof.

It is also suggested that GSK-3 is associated with progress of Alzheimer's disease. The characteristic pathological features of Alzheimer's disease are senile plaques due to agglomeration of amyloid beta (Aβ) peptide and the formation of intracellular neurofibrillary tangles, leading to a large quantity of neuronal cell death, resulting in dementia. It is believed that GSK-3 involves abnormal phosphorylation of tau protein, which causes a neurofibrillary change in the course of progress of Alzheimer's disease (see Acta Neuropathol., 2002, Vol. 103, p. 91). Also, it has been found that GSK-3 inhibitors can prevent neuronal cell death (see J. Neurochem., 2001, Vol. 77, p. 94). Therefore, it is believed that the application of GSK-3 inhibitors to Alzheimer's disease can delay the progress of the disease. To date, therapeutic agents for Alzheimer's disease have mainly been used in conjunction with allopathy (see Expert Opin. Pharmacother., 1999, Vol. 1, p. 121). However, there is no known pharmaceutical agent that is effective in preventing neuronal cell death and delaying the onset or progress of Alzheimer's disease. These findings imply that GSK-3 inhibitors are effective pharmaceutical agents in alleviating the severity of Alzheimer's dementia.

There is a report that GSK-3 inhibitors suppress neuronal cell death, specifically, neuronal cell death due to overexcitement through glutamic acid (see Proc. Natl. Acad. Sci., USA, 1996, Vol. 95, p. 2642; J. Neurochem., 2001, Vol. 77, p. 94). This suggests that GSK=3 inhibitors are possibly useful in the treatment of bipolar affective disorder such as manic depressive psychosis, epilepsy or other degenerative brain injury or neurodegenerative diseases. Examples of the neurodegenerative disease include in addition to the Alzheimer's disease, AIDS encephalopathy, Huntington's disease, Parkinson's disease, amyotrophic lateral sclerosis, multiple sclerosis, Pick's disease, progressive supranuclear palsy and so on. Also, overexcitement through glutamic acid is presumably a principal cause of brain dysfunction in stroke, including cerebral infarction, intracerebral hemorrhage and subarachnoid hemorrhage, traumatic cerebrospinal injury, bacteria/virus infectious disease, and GSK-3 inhibitors are expected to be effectively used in the treatment of these diseases. All of such diseases accompany neuronal death. Currently, no therapeutic agents for effectively suppressing neuronal death are available. Therefore, GSK-3 inhibitors are believed to become potentially effective pharmaceutical agents for the treatment of various kinds of neurodegenerative diseases, dipolar affective disorders (manic-depressive psychosis), epilepsy, stroke, traumatic cerebrospinal injury, and the like.

Several in vitro research results have led to a report that Wint10B potently suppresses the differentiation of preadipocytes to mature fat cells (see Science, 2000, Vol. 289, p. 950). GSK-3 specific inhibitors mimic Wint10B signaling in preadipocytes, that is, GSK-3 specific inhibitors stabilize free β-catenin in cytoplasm and suppress the induction of C/EBPα and PPARγ, thereby suppressing the formation of fat (see J. Biol. Chem, 2002, Vol. 277, p. 30998). GSK-3 inhibitors are therefore potentially useful as effective pharmaceutical compositions for treating obesity.

Also, β-catenin has been known to be a GSK-3 substrate in vivo. After phosphorylation by GSK-3, β-catenin is subjected to proteosome-dependent degradation (see EMBO J., 1998, Vol. 17, p. 1371). Meanwhile, transient β-catenin stabilization may lead to increase hair development (see Cell, 1998, Vol. 95, p. 605). Consequently, GSK-3 inhibitors are believed to be a useful medicament for the treatment of alopecia.

Further, research into GSK-3β knock out mouse-derived fibroblasts implies that GSK-3β regulates the activity of transcription factor NFκB to be at a positive level (see Nature, 2000, Vol. 406, p. 86). NFκB is in charge of cell responsiveness to numerous inflammatory stimuli. Thus, GSK-3 inhibitors may have beneficial effects in the treatment of inflammatory diseases such as arthrosis deformans, rheumatism, atopic dermatitis, psoriasis, ulcerative colitis, Crohn's Disease, sepsis, or systemic inflammatory response syndrome, by adjusting the NFκB activity to be at a negative level.

A transcription factor NF-AT is dephosphorylated by calcineurine and increases immunosuppressive response (see Science, 1997, Vol. 275, p. 1930). Conversely, GSK-3 phosphorylates NF-AT and transports the same from nuclei, thereby suppressing the expression of initial immune response gene. Thus, GSκ-3 inhibitors could be useful to immunity activation for cancer immunotherapy.

Examples of materials that have conventionally been known to have GSK-3 inhibiting activity include hymenialdisine derivatives (see Chemistry & Biology, 2000, Vol. 7, p. 51, and WO01/41768 pamphlet), maleiimide derivatives (see Chemistry & Biology, 2000, Vol. 7, p. 793), paullone derivatives (see Eur. J. Biochem., 2000, Vol. 267, p.5983 and WO01/60374 Pamphlet), purine derivatives (see WO98/16528 Pamphlet), pyrimidine and pyridine derivatives (see WO99/65897 Pamphlet), hydroxyflavone derivatives (see WO00/17184 Pamphlet), pyrimidone derivatives (see WO00/18758, WO01/70683, WO01/70729, WO01/70728, WO01/70727, WO01/70726, and WO01/70725 Pamphlets), pyrrole-2,5-dione derivatives (see WO00/21927 and WO01/74771 Pamphlets), diamino-1,2,4-triazolecarboxylic acid derivatives (see WO01/09106 Pamphlet), pyrazine derivatives (see WO01/44206 Pamphlet), bicyclic inhibitor (see WO01/44246 Pamphlet), indirubine derivatives (see WO01/37819 Pamphlet), carboxamide derivatives (see WO01/42224 Pamphlet), peptide inhibitors (see WO01/49709 Pamphlet), 2,4-diaminothiazole derivatives(see WO01/56567 Pamphlet), thiadiazolidindione derivatives(see WO01/8568 Pamphlet), aromatic amide derivatives (see WO01/81345 Pamphlet), and so on.

Also, the claims of WO02/085909 Pamphlet show a wide variety of compounds including pyrrolopyrimidine derivatives. However, bicyclic pyrrolopyrimidine derivatives actually synthesized are those having cyano groups at the 7-position of pyrrolopyrimidine ring and limited variety of substituents at other substitutable positions. In addition, while it discloses a method for assaying inhibitory activity of GSK-3 etc., it is silent about which compounds have such activities.

### DISCLOSURE OF THE INVENTION

An object of the present invention is to provide novel compounds which are specific to and capable of strongly inhibiting the activity of GSK-3 while being clinically applicable, and pharmaceutical compositions as GSK-3 inhibitors using them as effective ingredients.

Also, another object of the present invention is to provide an agent for treating or preventing a GSK-3-mediated disease.

Also disclosed is a method for treating a GSK-3-mediated disease.

The present inventors studied the above objects and consequently reached the following inventions.

Namely, the present invention provides a compound represented by the following formula (I) or a pharmaceutically acceptable salt thereof, as defined in appended claim 1 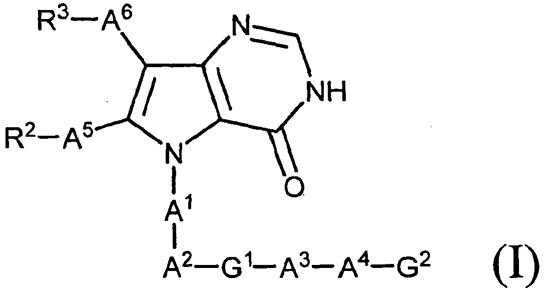 in the Formula (I),
A¹ represents a single bond or represents a divalent acyclic aliphatic hydrocarbon group having 1 to 6 carbon atoms that links a nitrogen atom bonded to A¹ with A² on the same or different carbon atom;
A² represents a single bond or represents a group that links A¹ with G¹ in the form of
A¹-C(=O)-G¹,
A¹-C(=O)-O-G¹,
A¹-C(=O)-NR¹⁰¹-G¹,
A¹-C(=S)-NR¹⁰²-G1,
A¹-C(=NR¹⁰³)-G¹,
A¹-O-G¹,
A¹-OC(=O)-G¹,
A¹-NR¹⁰⁴-G¹,
A¹-NR¹⁰⁵-C(=O)-G¹,
A¹-NR¹⁰⁶-S(=O)₂-G¹,
A¹-NR¹⁰⁷-C(=O)-O-G¹,
A¹-NR¹⁰⁸-C(=O)-NR¹⁰⁹-G¹,
A¹-NR¹¹⁰C(=S)-G¹,
A¹-NR¹¹¹-C(=S)-NR¹¹²-G¹,
A¹-S-G¹,
A¹-S(=O)-G¹,
A¹-S(=O)₂-G¹,
A¹-S(=O)₂-NR¹¹³-G¹,
A¹-CR¹¹⁴=CH-G¹,
A¹-CR¹¹⁵=CF-G¹,
A¹-CH=CR¹¹⁶-G¹, or
A¹-CF=CR¹¹⁷-G¹;
G¹ represents a single bond or represents a divalent group which is obtainable by removing two hydrogen atoms from any one of an optionally substituted alicyclic hydrocarbon group having 3 to 10 carbon atoms, an optionally substituted aromatic hydrocarbon having 6 to 14 carbon atoms, and an optionally substituted heterocyclic compound having 1 to 4 atoms selected from the group consisting of an oxygen atom, a nitrogen atom and a sulfur atom, in the ring.
A³ represents a single bond or represents an optionally substituted aliphatic hydrocarbon group having 1 to 10 carbon atoms that links G¹ with A⁴ on the same or different carbon atom;
A⁴ represents a single bond or represents a group that links A³ with G² in the form of
A³ -C(=O-G²,
A³-C (=O)-O-G²,
A³-C (=O)-NR¹²¹-G²,
A³-C(=S)-NR¹²²-G²,
A³-C(=NR¹²³)-G²,
A³-O-G²,
A³-O-C(=O)-G²,
A³-NR¹²⁴-G²,
A³-NR¹²⁵-C(=O)-G²,
A³-NR¹²⁶-S(=O)₂-G²,
A³-NR¹²⁷-C(=O)-O-G²,
A³-NR¹²⁸-C(=O)-NR¹²⁹-G²,
A³-NR¹³⁰-C(=S)-G²,
A³-NR¹³¹-C(=S)-NR¹³²-G²,
A³-S-G²,
A³-S(=O)-G²,
A³-S(=O)₂-G²,
A³-S(=O)₂-NR¹³³-G² or
A³-S (=O)₂-O-G²;
G² represents a hydrogen atom, an optionally substituted aliphatic hydrocarbon group having 1 to 10 carbon atoms, an optionally substituted alicyclic hydrocarbon group having 3 to 10 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
A⁵ represents a single bond or -NR²⁰¹-;
R² represents a hydrogen atom, a fluorine atom, a chlorine atom, a bromine atom, an iodine atom, an optionally substituted aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
A⁶ represents a single bond or represents a group that links R³ with a carbon atom of a pyrrole ring to which A⁶ is bonded, in the form of
R³-NR³⁰¹-pyrrole ring,
R³-C(=O)-pyrrole ring,
R³-NR³⁰²-C(=O)-pyrrole ring,
R³-NR³⁰³-C(=S)-pyrrole ring,
R³-NR³⁰⁴-C(=O)-NR³⁰⁵-pyrrole ring,
R³-C(=O)-NR³⁰⁶-pyrrole ring,
R³-NR³⁰⁷-CH=N-pyrrole ring,
R³-C(=O)-O-pyrrole ring,
R³-O-C(=O)-pyrrole ring,
R³-O-pyrrole ring,
R³-S-pyrrole ring,
R³-S(=O)-pyrrole ring,
R³-S(=O)₂-pyrrole ring,
R³-CR³⁰⁸=CR³⁰⁹-pyrrole ring,
R³-C≡C-pyrrole ring, or
R³-S(=O)₂-C≡C-pyrrole ring;

Also disclosed is a pharmaceutical composition comprising the compound or a pharmaceutically acceptable salt thereof represented by the formula (I); and a pharmaceutically acceptable carrier.

Further, the present invention provides a GSK-3 inhibitor comprising the compound or a pharmaceutically acceptable salt thereof represented by the formula (I) as an effective ingredient.

The present invention also provides. An agent for treating or preventing a GSK-3-mediated disease, comprising the compound or a pharmaceutically acceptable salt thereof as defined in any one of appended claims claims 1 to 38, for use in a methode of treating or preventing a GSK-3-mediated disease selected from the group consisting of diabetes, diabetic complications, Alzheimer's disease, neurodegenerative disease, manic depressive psychosis, traumatic brain injury, alopecia, inflammatory disease, cancer, and immunodeficiency.

Furthermore, the present invention provides. The use of an agent comprising the compound or a pharmaceutically acceptable salt thereof as defined in any one of appended claims 1 to 38 for the manufacture of a medicament for treating or preventing a GSK-3-mediated disease selected from the group consisting of diabetes, diabetic complications, Alzheimer's disease, neurodegenerative disease, manic depressive psychosis, traumatic brain injury, alopecia, inflammatory disease, cancer, and immunodeficiency.

Also disclosed is a method for treating a GSK-3-mediated disease, comprising a step of injecting the compound or a pharmaceutically acceptable salt thereof represented by the formula (I) in treatment valid amount to a patient.

Note that, in A¹-G² portion in the formula (1), there also exists a case where different combinations consequently represent the same substituent according to the combination of A¹, A², G¹, A³, A⁴, and G², and combinations containing also substituents of them where they may have substituents. However, the scope of the present invention will not become clear due to this.

Note that the corresponding pyrimidine-thione derivative can be derived from the compound represented by the formula (I) of the present invention through the pyrrolopyrimidine derivative represented by the following formula (II). 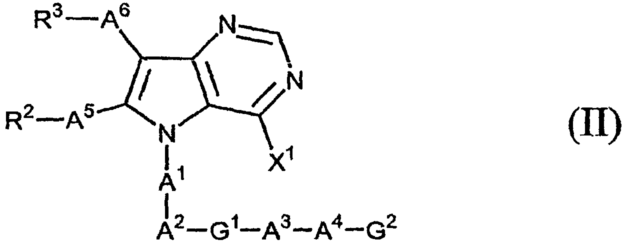 in formula (I), A¹, A², A³, A⁴, A⁵, A⁶, G¹, G², R² and R³ are as defined in the formula (I); and X¹ is a chlorine atom, a bromine atom, an iodine atom, a C₂-C₁₀ acylthio group, a C₂-C₈ alkoxymethylthio group, a C₁-C₈ alkyl group, or a C₁-C₈ arylsulfonyloxy group).

Still further, the present invention is a compound represented by the following formula (Ic) which can be used as the manufacture intermediate of the pyrrolopyrimidinone derivative represented by the formula (I). 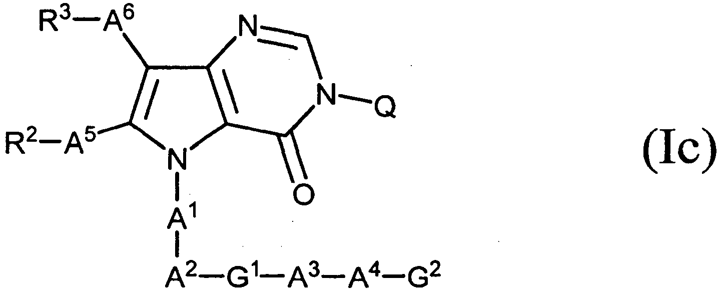 in formula (Ic), A¹, A², A³, A⁴, A⁵, A⁶, G¹, G², R², and R³ are as defined in the formula (I); and Q represents an optionally substituted a C₂-C₁₀ acyl group, an optionally substituted C₂-C₁₀ alkoxymethyl group or an optionally substituted benzyl group.

### BEST MODE FOR WORKING THE INVENTION

The "acyclic aliphatic hydrocarbon group" in the present description contains a straight or branched acyclic aliphatic hydrocarbon group. It may be saturated so far as it is the acyclic aliphatic hydrocarbon group as well and may have one or more double bonds or triple bonds in a chemically possible range.

The "alkyl group" in the present description represents a straight or branched saturated acyclic aliphatic hydrocarbon group, for example methyl, ethyl, propyl, butyl, pentyl, hexyl, heptyl, octyl, isopropyl, isobutyl, s-butyl, t-butyl, isopentyl, neopentyl, t-pentyl, or isohexyl.

The "pyridyl group" in the present description contains N-oxide thereof as well.

The term "cycloalkyl group" in the present description means a saturated alicyclic hydrocarbon group, for example cyclopropyl, cyclobutyl, or cyclohexyl.

The term "heterocyclic" in the present description is not particularly limited so far as it can chemically stably exist if it is monocyclic to tricyclic having 1 to 4 atoms selected from among a group consisting of an oxygen atom, a nitrogen atom, and a sulfur atom, in the ring, but preferably monocyclic or bicyclic having carbon atoms not more than 9 containing 1 to 3, preferably 1 or 2 atoms selected from among a group consisting of an oxygen atom, a nitrogen atom, and a sulfur atom, in the ring.

In the formula (I), A¹ is -(CH₂)₂- or -(CH₂)₃-.

In the formula (I), A² represents a single bond or represents a group that links A¹ and G¹ in the form of A¹-C(=O)-G¹, A¹-C(=O)-O-G¹, A¹-C(=O)-NR¹⁰¹-G¹, A¹-C(=S)-NR¹⁰²-G¹, A¹-C(=NR¹⁰³)-G¹, A¹-O-G¹, A¹-O-C(=O)-G¹, A¹-NR¹⁰⁴-G¹, A¹-NR¹⁰⁵-C(=O)-G¹, A¹-NR¹⁰⁶-S(=O)₂-G¹. A¹-NR¹⁰⁷-C(=O)-O-G¹, A¹-NR¹⁰⁸-C(=O)-NR¹⁰⁹-G¹, A¹-NR¹¹⁰-C(=S)-G¹, A¹-NR¹¹¹⁻C(=S)-NR¹¹²-G¹, A¹-S-G¹, A¹-S(=O)-G¹_{,} A¹-S(=O)₂-G¹, A¹-S(=O)₂-NR¹¹³-G¹, A¹-CR¹¹⁴=CH-G¹, A¹-CR¹¹⁵=CF-G¹, A¹-CH=CR¹¹⁶-G¹ or A¹-CF=CR¹¹⁷-G¹ (R¹⁰¹ ∼ R¹¹⁷ are independently a hydrogen atom or a acyclic aliphatic hydrocarbon group having 1 to 4 carbon atoms).

When A¹ and G¹ are linked to each other in the form of A¹-C (=O)-NR¹⁰¹-G¹, examples of the C₁-C₄ aliphatic hydrocarbon group of R¹⁰¹ include methyl, ethyl, propyl, isopropyl, butyl, isobutyl, s-butyl, t-butyl, cyclopropylmethyl, 2-propenyl, 2-butenyl, 3-butenyl, 2-propynyl, 2-butynyl and 3-butynyl group. The C₁-C₄ acyclic aliphatic hydrocarbon group may also be substituted with one or more substituents selected from the group consisting of a fluorine atom, a chlorine atom, a bromine atom, an iodine atom, a hydroxy group, a methoxy group, an ethoxy group, an oxo group, a cyano group, a carboxyl group, a carbamoyl group, an amino group, a sulfo group, and a phenyl group. Examples of preferred R¹⁰¹ include a hydrogen atom, methyl, ethyl, and propyl group. Particularly, a hydrogen atom is preferred.

When A¹ and G¹ are linked to each other in the form of A¹-C (=S)-NR¹⁰²-G¹, examples of the C₁-C₄ aliphatic hydrocarbon group of R¹⁰² include the same as those selected as the examples of R¹⁰¹. Examples of preferred R¹⁰² include a hydrogen atom, methyl, ethyl, and propyl group. Particularly, a hydrogen atom is preferred.

When A¹ and G¹ are linked to each other in the form of A¹-C(=NR¹⁰³)-G¹, examples of the C₁-C₄ aliphatic hydrocarbon group of R¹⁰³ include the same as those selected as the examples of R¹⁰¹. Examples of preferred R¹⁰³ include a hydrogen atom, methyl, ethyl, and propyl group. Particularly, a hydrogen atom is preferred.

When A¹ and G¹ are linked to each other in the form of A¹-NR¹⁰⁴-G¹, examples of the C₁-C₄ aliphatic hydrocarbon group of R¹⁰⁴ include the same as those selected as the examples of R¹⁰¹. Examples of preferred R¹⁰⁴ include a hydrogen atom, methyl, ethyl, and propyl group. Particularly, a hydrogen atom is preferred.

When A¹ and G¹ are linked to each other in the form of A¹-NR¹⁰⁵-C(=O)-G¹, examples of the C₁-C₄ acyclic aliphatic hydrocarbon group of R¹⁰⁵ include the same as those selected as the examples of R¹⁰¹. Examples of preferred R¹⁰⁵ include a hydrogen atom, methyl, ethyl, and propyl group. Particularly, a hydrogen atom is preferred.

When A¹ and G¹ are linked to each other in the form of A¹-NR¹⁰⁶-S(=O)₂-G¹, examples of the C₁-C₄ aliphatic hydrocarbon group of R¹⁰⁶ include the same as those selected as the examples of R¹⁰¹. Examples of preferred R¹⁰⁶ include a hydrogen atom, methyl, ethyl, and propyl group. Particularly, a hydrogen atom is preferred.

When A¹ and G¹ are linked to each other in the form of A¹-NR¹⁰⁷-C(=O)-O-G¹, examples of the C₁-C₄ aliphatic hydrocarbon group of R¹⁰⁷ include the same as those selected as the examples of R¹⁰¹. Examples of preferred R¹⁰⁷ include a hydrogen atom, methyl, ethyl, and propyl group. Particularly, a hydrogen atom is preferred.

When A¹ and G¹ are linked to each other in the form of A¹-NR¹⁰⁸-C(=O)NR¹⁰⁹-G¹, examples of such preferred C₁-C₄ aliphatic hydrocarbon group of R¹⁰⁸ and R¹⁰⁹ include the same as those selected as the examples of R¹⁰¹. Examples of preferred R¹⁰⁸ and R¹⁰⁹ include a hydrogen atom, methyl, ethyl, and propyl group. Particularly, a hydrogen atom is preferred.

When A¹ and G¹ are linked to each other in the form of A¹-NR¹¹⁰-C(=S)-G¹, examples of such preferred C₁-C₄ aliphatic hydrocarbon group of R¹¹⁰ include the same as those selected as the examples of R¹⁰¹. Examples of preferred R¹¹⁰ include a hydrogen atom, methyl, ethyl, and propyl group. Particularly, a hydrogen atom is preferred.

When A¹ and G¹ are linked to each other in the form of A¹-NR¹¹¹-C(=S)-NR¹¹²-G¹, examples of the C₁-C₄ aliphatic hydrocarbon group of R¹¹¹ and R¹¹² include the same as those selected as the examples of R¹⁰¹. Examples of preferred R¹¹¹ and R¹¹² include a hydrogen atom, methyl, ethyl, and propyl group. Particularly, a hydrogen atom is preferred.

When A¹ and G¹ are linked to each other in the form of A¹-S (=O)₂-NR¹¹³-G¹, examples of the C₁-C₄ aliphatic hydrocarbon group of R¹¹³ include the same as those selected as the examples of R¹⁰¹. Examples of preferred R¹¹³ include a hydrogen atom, methyl, ethyl, and propyl group. Particularly, a hydrogen atom is preferred.

When A¹ and G¹ are linked to each other in the form of A¹-CR¹¹⁴=CR¹¹⁵-G¹, examples of the C₁-C₄ aliphatic hydrocarbon group of R¹¹⁴ and R¹¹⁵ include the same as those selected as the examples of R¹⁰¹. Examples of preferred R¹¹⁴ and R¹¹⁵ include a hydrogen atom, methyl, ethyl, and propyl group. Particularly, a hydrogen atom is preferred.

When A¹ and G¹ are linked to each other in the form of A¹-CF=CR¹¹⁷-G¹, examples of the C₁-C₄ aliphatic hydrocarbon group of R¹¹⁷ include the same as those selected as the examples of R¹⁰¹. Examples of preferred R¹¹⁷ include a hydrogen atom, methyl, ethyl, and propyl group. Particularly, a hydrogen atom is preferred.

When A¹ and G¹ are linked to each other in the form of A¹-CF=CR¹¹⁷-G¹, examples of the C₁-C₄ aliphatic hydrocarbon group of R¹¹⁷ include the same as those selected as the examples of R¹⁰¹. Examples of preferred R¹¹⁷ include a hydrogen atom, methyl, ethyl, and propyl group. Particularly, a hydrogen atom is preferred.

Examples of preferred A² include groups that link A¹ and G¹ in the form of A¹-C(=O)-G¹, A¹-C(=O)-NR¹⁰¹-G¹, A¹-O-G¹, A¹-NR¹⁰⁴-G¹, A¹-NR¹⁰⁵-C(=O)-G¹, A¹-NR¹⁰⁸-C(=O)-NR¹⁰⁹-G¹, A¹-NR¹¹⁰-C(=S)-G¹ and A¹-NR¹¹¹-C(=S)NR¹¹²-G¹, especially preferably in the form of A¹-C(=O)-G¹, A¹-C(=O)-NR¹⁰¹-G¹, A¹-NR¹⁰⁴-G¹, A¹-NR¹⁰⁵-C(=O)-G¹, A¹-NR¹⁰⁸-C(=O)-NR¹⁰⁹-G¹, and A¹-NR¹¹⁰-C(=S)-G¹. Among them, examples of more preferred A² include groups that link A¹ and G¹ in the form of A¹-C(=O)-NR¹⁰¹-G¹, A¹-NR¹⁰⁵-C(=O)-G¹, and A¹-NR¹⁰⁸-C(=O)-NR¹⁰⁹-G¹. Here, forms of linkage exemplified as preferred and more preferred A² are preferably combined with structures in which A¹ exists in the form of -(CH₂)₂- or -(CH₂)₃- in the formula (I).

In the formula (I), A³ represents a single bond or represents an optionally substituted divalent aliphatic hydrocarbon group having 1 to 10 carbon atoms that links G¹ and A⁴ on the same or different carbon atoms.

Examples of the acyclic aliphatic hydrocarbon group having 1 to 10 carbon atoms of A³ include, in addition to the same as those selected as the examples of A¹, -CH=CH-, -C(CH₃)=CH-, -C(CH₃)=C(CH₃)-, -C(CH₂CH₃)=CH-, - C(CH₂CH₃)=C(CH₃)-, -C (CH₂CH₃)=C (CH₂CH₃)-, -C (CH₂CH₂CH₃)=CH-, - C (CH₂CH₂CH₃)=C (CH₃)-, -CH=CHCH₂-, -C (CH₃) =CHCH₂-, - CH=C(CH₃)CH₂-, -CH=CHCH(CH₃)-, -C(CH₃)=C(CH₃)CH₂-, - C(CH₃)=CHCH(CH₃)-, -C(CH₃)=C(CH₃)CH(CH₃)-, - C(CH₃)=CHC(CH₃)₂-, -C (CH₂CH₃)=CHCH₂-, -CH=C (CH₂CH₃)CH₂-, - CH=CHCH (CH₂CH₃)-, -C (CH₂CH₃)=C (CH₃)CH₂-, - C(CH₂CH₃)=CHCH(CH₃)-, -C(CH₃)=C(CH₂CH₃)CH₂-, - CH=C(CH₂CH₃)CH(CH₃)-, -CH=CHCH(CH₂CH₃)-, - C(CH₃)=CHCH(CH₂CH₃)-, -CH=C(CH₃)CH(CH₂CH₃)-, -CH=CH(CH₂)₂-, -C (CH₃)=CH(CH₂)₂-, -CH=C (CH₃)(CH₂)₂-, -CH=CHC(CH₃)CH₂-, -C H=CHCH₂CH(CH₃)-, -C (CH₃)=C (CH₃)(CH₂)₂- , - C (CH₃)=CHCH (CH₃)CH₂-, -C (CH₃)=CHCH₂CH(CH₃)-, -CH₂CH=CHCH₂-, -CH(CH₃)CH=CHCH₂-, -CH₂C (CH₃)=CHCH₂-, -CH(CH₃)C(CH₃)=CHCH₂-, -CH(CH₃)CH=CHCH(CH₃)-, -CH(CH₃)CH=C(CH₃)CH₂-, - CH₂C(CH₃)=C(CH₃)CH₂-, -CH(CH₂CH₃)CH=CHCH₂-, and - CH₂C (CH₂CH₃)=CHCH₂-.

Substituents of divalent substituted aliphatic hydrocarbon group having 1 to 10 carbon atoms of A³ include a hydrocarbon group having 1 to 6 carbon atoms, an alicyclic hydrocarbon group having 3 to 6 carbon atoms, a halogen atom, an alkoxy group having 1 to 6 carbon atoms, a phenoxy group, an amino group, or an alkyl amino group having 1 to 6 carbon atoms.

Examples of such preferred A³ include a single bond, -CH₂-, - (CH₂)₂-, -(CH₂)₃-,-(CH₂)₄-, -CH(CH₃)CH₂-, - CH(CH₃)CH(CH₃)-, -CH(CH₃)(CH₂)₂-, -CH=CH- and -CH=CHCH₂-. Further, examples of more preferred A³ include a single bond, -CH₂-, -(CH₂)₂- and - (CH₂)₃-. The same applies when A³ is substituted, but a single bond is excluded.

In the formula (I), A⁴ represents a single bond or represents a group that links A³ and G² in the form of A³-C(=O)-G², A³-C(=O)-O-G², A³-C(=O)-NR¹²¹-G², A³-C(=S)-NR¹²²-G², A³-C(=NR¹²³)-G², A³-O-G², A³-O-C(=O)-G², A³-NR¹²⁴-G², A³-NR¹²⁵-C(=O)-G², A³-NR¹²⁶-S(=O)₂-G², A³-NR¹²¹-C(=O)-O-G², A³-NR¹²⁸-C(=O)-NR¹²⁹-G², A³-NR¹³⁰-C(=S)-G², A³-NR¹³¹-C(=S)-NR¹³²-G² A³-S-G², A³-S(=O)-G², A³-S(=O)₂-G²_{,} A³-S(=O)₂-NR¹³³-G² or A³-S(=O)₂-O-G² (in which R¹²¹ through R¹³³ are each independently a hydrogen atom or a acyclic aliphatic hydrocarbon group having 1 to 4 carbon atoms).

When A³ and G² are linked to each other in the form of A³-C(=O)-NR¹²¹-G²_{,} examples the C₁-C₄ acyclic aliphatic hydrocarbon group of R¹²¹ include the same as those selected as the examples of R¹⁰¹ in A². Examples of preferred R¹²¹ include a hydrogen atom, methyl, ethyl, and propyl group. Particularly, a hydrogen atom is preferred.

When A³ and G² are linked to each other in the form of A³-C(=S)-NR¹²²-G²' examples of the C₁-C₄ acyclic aliphatic hydrocarbon group of R¹²² include the same as those selected as the examples of R¹⁰¹ in A². Examples of preferred R¹²² include a hydrogen atom, methyl, ethyl, and propyl group. Particularly, a hydrogen atom is preferred.

When A³ and G² are linked to each other in the form of A³-C(=NR¹²³)-G², examples of the C₁-C₄ acyclic aliphatic hydrocarbon group of R¹²³ include the same as those selected as the examples of R¹⁰¹ in A². Examples of preferred R¹²³ include a hydrogen atom, methyl, ethyl, and propyl group. Particularly, a hydrogen atom is preferred.

When A³ and G² are linked to each other in the form of A³-NR¹²⁴-G², examples of the C₁-C₄ acyclic aliphatic hydrocarbon group of R¹²⁴ include the same as those selected as the examples of R¹⁰¹ in A². Examples of preferred R¹²⁴ include a hydrogen atom, methyl, ethyl, and propyl group. Particularly, a hydrogen atom is preferred.

When A³ and G² are linked to each other in the form of A³-NR¹²⁵-C(=O)-G², examples of the C₁-C₄ aliphatic hydrocarbon group of R¹²⁵ include the same as those selected as the examples of R¹⁰¹ in A². Examples of preferred R¹²⁵ include a hydrogen atom, methyl, ethyl, and propyl group. Particularly, a hydrogen atom is preferred.

When A³ and G² are linked to each other in the form of A³-NR¹²⁶-S(=O)₂-G²' examples of the C₁-C₄ acyclic aliphatic hydrocarbon group of R¹²⁶ include the same as those selected as the examples of R¹⁰¹ in A². Examples of preferred R¹²⁶ include a hydrogen atom, methyl, ethyl, and propyl group. Particularly, a hydrogen atom is preferred.

When A³ and G² are linked to each other in the form of A³-NR¹²⁷-C(=O)-O-G², examples of the C₁-C₄ acyclic aliphatic hydrocarbon group of R¹²⁷ include the same as those selected as the examples of R¹⁰¹ in A². Examples of preferred R¹²⁷ include a hydrogen atom, methyl, ethyl, and propyl group. Particularly, a hydrogen atom is preferred.

When A³ and G² are linked to each other in the form of A³-NR¹²⁸-C(=O)-NR¹²⁹-G², examples of the C₁-C₄ aliphatic hydrocarbon group of R¹²⁸ and R¹²⁹ include the same as those selected as the examples of R¹⁰¹ in A². Examples of preferred R¹²⁸ and R¹²⁹ include a hydrogen atom, methyl, ethyl, and propyl group. Particularly, a hydrogen atom is preferred.

When A³ and G² are linked to each other in the form of A³-NR¹³⁰-C(=S)-G², examples of the C₁-C₄ acyclic aliphatic hydrocarbon group of R¹³⁰ include the same as those selected as the examples of R¹⁰¹ in A² Examples of preferred R¹³⁰ include a hydrogen atom, methyl, ethyl, and propyl group. Particularly, a hydrogen atom is preferred.

When A³ and G² are linked to each other in the form of A³-NR¹³¹-C(=S) -NR¹³²-G², examples of the C₁-C₄ acyclic aliphatic hydrocarbon group of R¹³¹ and R¹³² include the same as those selected as the examples of R¹⁰¹ in A². Examples of preferred R¹³¹ and R¹³² include a hydrogen atom, methyl, ethyl, and propyl group. Particularly, a hydrogen atom is preferred.

When A³ and G² are linked to each other in the form of A³-S(=O)₂-NR¹³³-G², examples of the C₁-C₄ acyclic aliphatic hydrocarbon group of R¹³³ include the same as those selected as the examples of R¹⁰¹ in A². Examples of preferred R¹³³ include a hydrogen atom, methyl, ethyl, and propyl group. Particularly, a hydrogen atom is preferred.

Examples of such A⁴ include a single bond and a group that links A³ and G² in the form of A³-C(=O)-G², A³-C(=O)-O-G², A³-C(=O)-NR¹²¹-G², A³-O-G², A³-NR¹²⁴-G², A³-NR¹²⁵-C(=O)-G²_{,} A³-S(=O)₂-G² or A³-S(=O)₂-O-G².

In the formula (I), G¹ represents a single bond or a divalent group obtainable by removing two hydrogen atoms from any of groups consisting of a substituted or unsubstituted alicyclic hydrocarbon group having 3 to 10 carbon atoms, a substituted or unsubstituted aromatic hydrocarbon group having 6 to 14 carbon atoms, and a heterocyclic compound having 1 to 4 atoms selected from the group consisting of an oxygen atom, a nitrogen atom and a sulfur atom on its substituted or unsubstituted ring.

In the formula (I), when G¹ represents a substituted or unsubstituted divalent alicyclic hydrocarbon group having 3 to 10 carbon atoms, examples of the alicyclic hydrocarbon group having 3 to 10 carbon atoms include cyclopropane, cyclobutane, cyclopentane, cyclopentene, cyclohexane, cyclohexene, cycloheptane, cycloheptene, cyclooctane, bicyclo[2.2.1] heptane, bicyclo[2. 2. 1]heptene, bicyclo[3.1. 1]heptane and bicyclo[2. 2. 2]octane. Examples of such preferred C₃-C₁₀ alicyclic hydrocarbon of G¹ include monocyclic alicyclic hydrocarbon group having 3 to 6 carbon atoms such as cyclopropane, cyclopentane, cyclohexane and the like.

The substituent for the substituted alicyclic hydrocarbon group having 3 to 10 carbon atoms of G¹ may be: a fluorine atom, a chlorine atom, a bromine atom, an iodine atom, a hydroxy group, methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methyl- pentyloxy, 1-ethylbutoxy, cyclopropyloxy, cyclobutyloxy, cyclopentyloxy, cyclohexyloxy, cyclopropylmethyloxy, cyclopropylethyloxy, cyclopentylmethyloxy and cyclohexylmethyloxy or another C₁-C₇ alkoxy group consisting of a straight or branched alkyl, cycloalkyl and oxy group, ethylene dioxy or another C₁-C₄ alkylenedioxy group, phenoxy, 1-naphthoxy and 2-naphthoxy or another C₆-C₁₀ aryloxy group, benzyloxy, α-phenethyloxy, β-phenethyloxy and phenylpropyloxy or another C₇-C₉ aralkoxy group, acetoxy, propionyloxy, butyryloxy, isobutyryloxy, valeryloxy, isovaleryloxy, pivaloyloxy and hexanoyloxy or another C₂-C₇ acyloxy group, oxo, methylsulfonyloxy, ethylsulfonyloxy, propylsulfonyloxy, butylsulfonyloxy and t-butylsulfonyloxy or another C₁-C₆ alkylsulfonyloxy group consisting of a straight or branched alkyl and sulfonyloxy, acetyl, propionyl, butyryl, isobutyryl, valeryl, isovaleryl, pivaroyl and hexanoyl or another C₂-C₇ acyl group, carboxy, methoxycarbonyl, ethoxycarbonyl, propoxycarbonyl, isopropoxycarbonyl, butoxycarbonyl, isobutoxycarbonyl, s-butoxycarbonyl and t-butoxycarbonyl or another C₂-C₇ alkoxycarbonyl group consisting of a straight or branched alkyl and oxycarbonyl group, carbamoyl, N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl and N,N-dipropylcarbamoyl or another C₂-C₇ alkylcarbamoyl group consisting of a straight or branched alkyl, cycloalkyl and carbamoyl group, amino, methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutyl- amino, N-methyl-t-butylamino, N-ethylisopropylamino, dipropylamino, diisopropylamino and ethylbutylamino or another C₁-C₆ alkylamino group consisting of a straight or branched alkyl, cycloalkyl and amino group, acetylamino, propionylamino, butyrylamino, isobutyrylamino, valerylamino and hexanoylamino or another C₂-C₇ acylamino group, methoxycarbonylamino, ethoxycarbonylamino and t-butoxycarbonylamino or another C₂-C₈ alkoxycarbonylamino group, methylsulfonylamino, ethylsulfonylamino, butylsulfonylamino and t-butylsulfonylamino or another C₁-C₆ alkylsulfonylamino group, a cyano group, a nitro group, methylthio, ethylthio, propylthio, isopropylthio, butylthio, isobutylthio, s-butylthio, t-butylthio, pentylthio and hexylthio or another C₁-C₆ alkylthio group, methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl and cyclopentylsulfynyl or another C₁-C₆ alkylsulfynyl group consisting of a straight or branched alkyl, cycloalkyl and sulfynyl group, methylsulfonyl, ethylsulfonyl, propylsulfonyl, isopropylsulfonyl, butyl- sulfonyl, isobutylsulfonyl, s-butylsulfonyl, t-butylsulfonyl, pentylsulfonyl, hexylsulfonyl, cyclopentylsulfonyl and cyclohexylsulfonyl or another C₁-C₆ alkylsulfonyl group consisting of a straight or branched alkyl, cycloalkyl and sulfonyl group, a sulfo group, a sulfamoyl group, methylaminosulfonyl, ethylaminosulfonyl, propylaminosulfonyl, isopropylaminosulfonyl, butylaminosulfonyl, isobutylamino- sulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl and cyclopropylmethylaminosulfonyl or another C₁-C₆ aminosulfonyl group consisting of a straight or branched alkyl, a cycloalkyl and aminosulfonyl group, cyclopropyl, cyclobutyl, cyclopentyl and cyclohexyl or another alicyclic hydrocarbon group having 3 to 6 carbon atoms, methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2- propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl or another aliphatic hydrocarbon group having 1 to 6 carbon atoms which may contain a straight or branched unsaturated bond.

As the substituent of the substituted alicyclic hydrocarbon group having 3 to 10 carbon atoms as G¹, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms or an aliphatic hydrocarbon group having 1 to 6 carbon atoms, may further be substituted with (one or more substituents selected from the group consisting of a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a C₂-C₇ acyl group such as methoxymethyloxy group, 2-methoxyethoxy group, formyl group, trifluoroacetyl group, acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a C₁-C₆ alkylamino group such as methylamino, ethylamino, propylamino, isopropylamino, dimethylamino, N-ethylmethylamino, diethylamino, N-methyl- propylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a C₁-C₇ acylamino group such as trifluoroacetylamino group, formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; and a cyano group).

In the formula (I), when G¹ represents a substituted or unsubstituted divalent aromatic hydrocarbon group having 6 to 14 carbon atoms, examples of the aromatic hydrocarbon group having 6 to 14 carbon atoms include a compound having at least one aromatic ring on its molecule, such as benzene, indene, indane, naphthalene, 1,2-dihydronaphthalene, 1,2,3,4-tetrahydronaphthalene, azulene, acenaphthylene, acenaphthene, fluorene, phenanthrene or anthracene.

Examples of such preferred aromatic hydrocarbon group having 6 to 14 carbon atoms of G¹ include benzene, naphthalene and indane. Examples of more preferred aromatic hydrocarbon group having 6 to 14 carbon atoms of G¹ include benzene.

The substituents of the substituted aromatic hydrocarbon group having 6 to 14 carbon atoms of G¹ may be a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms and an optionally substituted aliphatic hydrocarbon group having 1 to 6 carbon atoms.

Specific examples of the substituent of the substituted aromatic hydrocarbon group having 6 to 14 carbon atoms of G¹ include the same as those specifically exemplified as the substituents of the substituted alicyclic hydrocarbon group having 3 to 10 carbon atoms of G¹_{.}

As the substituent of the substituted aromatic hydrocarbon group having 6 to 14 carbon atoms, the C₁-C₇ alkoxy group, the C₂-C₇ acyl group, the C₂-C₇ alkylcarbamoyl group, the C₁-C₆ alkylamino group, the C₂-C₇ acylamino group, the alicyclic hydrocarbon group having 3 to 6 carbon atoms or the aliphatic hydrocarbon group having 1 to 6 carbon atoms, may further be substituted with (one or more substituents selected from the group consisting of a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; and a cyano group).

Preferred examples of the substituents of the substituted aromatic hydrocarbon group having 6 to 14 carbon atoms of G₁ include a fluorine atom; a chlorine atom; a bromine atom; a C₁-C₆ alkoxy group consisting of a straight or branched alkyl and oxy group, including methoxy, ethoxy, propoxy, isopropoxy, butoxy, isobutoxy, s-butoxy, t-butoxy, pentyloxy, isopentyloxy, neopentyloxy, t-pentyloxy and hexyloxy; a cyano group; a nitro group; a carboxyl group; a hydroxy group; an amino group; a C₁-C₆ mono or dialkylamino group consisting of a straight or branched alkyl and amino group, including methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutyl- amino, N-methyl-t-butylamino, N-ethylisopropylamino, dipropylamino, diisopropylamino and ethylbutylamino; a carbamoyl group; an aminosulfonyl group; an alicyclic hydrocarbon group having 3 to 6 carbon atoms, including cyclopropyl, cyclobutyl, cyclopentyl and cyclohexyl; a C₂-C₇ acyl group including acetyl, propionyl, butyryl, isobutyryl, pivaroyl and hexanoyl; a C₁-C₆ alkylthio group including methylthio, ethylthio, propylthio, isopropylthio, butylthio, isobutylthio, s-butylthio, t-butylthio, pentylthio and hexylthio; a C₁-C₆ alkylsulfonyl group including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl and hexylsulfonyl; a C₂-C₇ alkoxycarbonyl group including acetoxy, propionyloxy, butyryloxy, isobutyryloxy, valeryloxy, isovaleryloxy, pivaloyloxy and hexanoyloxy; a C₂-C₇ acylamino group including acetylamino, propionylamino, butyrylamino, isobutyrylamino, valerylamino and hexanoylamino; trifluoromethyl group, trifluoromethoxy group,and an aliphatic hydrocarbon group having 1 to 6 carbon atoms which may contain a straight or branched unsaturated bond, including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1- propenyl, 1-methyl-2-propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl.

Specifically, examples of more preferred substituents of the substituted aromatic hydrocarbon group having 6 to 14 carbon atoms include a fluorine atom, a chlorine atom, a bromine atom, C₁-C₆ alkoxy group, cyano group, a nitro group, a carboxyl group, a hydroxy group, an amino group, a C₁-C₆ mono or dialkylamino group, a carbamoyl group, an alicyclic hydrocarbon group having 3 to 6 carbon atoms, a C₂-C₇ acyl group, a C₁-C₆ alkylsulfonyl group, a C₂-C₇ alkoxycarboxyl group, trifluoromethyl group, trifluoromethoxy group, and a C₁-C₆ alkyl group including methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl. Examples of particularly preferred substituents include a fluorine atom, a chlorine atom, a C₁-C₆ alkoxy group, a cyano group, a nitro group, a carboxyl group, a hydroxy group, an amino group, a C₁-C₆ mono or dialkylamino group, an alicyclic hydrocarbon group having 3 to 6 carbon atoms, a C₂-C₇ acyl group, a trifluoromethyl group, a trifluoromethoxy group and a C₁-C₆ alkyl group.

In the formula (I), when G¹ represents a divalent group derived from heterocyclic compounds having 1 to 4 atoms selected from the group consisting of an oxygen atom, a nitrogen atom and a sulfur atom on its substituted or unsubstituted ring, examples of such heterocyclic compounds include monocyclic, bicyclic or tricyclic heterocyclic compounds, such as furan, thiophene, pyrrole, pyrroline, pyrrolidine, oxazole, oxazolidine, isooxazole, isooxazolidine, thiazole, thiazolidine, isothiazole, isothiazolidine, furazan, imidazole, imidazoline, imidazolidine, pyrazole, pyrazoline, pyrazolidine, triazole, thiadiazole, oxadiazole, tetrazole, pyran, tetrahydropyran, thiopyran, tetrahydrothiopyran, tetrahydrofuran, 1,3-dioxolane, 1,4-dioxane, pyridine, pyrazine, pyrimidine, pyridazine, benzofuran, dibenzofuran, 1, 4-dioxacycloheptane, benzothiophene, indole, 1,2-methylene- dioxybenzene, benzimidazole, benzothiazole, benzooxazole, chroman, isochroman, quinoline, decahydroquinoline, isoquinoline, phthalazine, cinnoline, 1,8-naphthylidine, 1,2,3,4-tetrahydroisoquinoline, quinazoline, quinoxaline, purine, pteridine, azetidine, morpholine, thiomorpholine, piperidine, homopiperidine, piperazine, homopiperazine, indoline, isoindoline, phenoxazine, phenazine, phenothiazine, pyrrolopyrimidine, pyrazolpyrimidine or quinuclidine.

Preferred examples of the heterocyclic compound having 1 to 4 atoms selected from the group consisting of an oxygen atom, a nitrogen atom and a sulfur atom, in the ring of G¹ include monocyclic or bicyclic C₂-C₉ aromatic heterocyclic compounds having 1 to 3 atoms selected from the group consisting of an oxygen atom, a nitrogen atom and a sulfur atom, in the ring, such as furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxybenzene, benzimidazole, indole, quinoline, isoquinoline, quinazoline, phthalazine, cinnoline or 1,8-naphthylidin; or monocyclic C₂-C₉ heterocyclic compounds having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine.

The heterocyclic group having 1 to 4 atoms selected from the group consisting of an oxygen atom, a nitrogen atom and a sulfur atom, in the ring of G¹ links to A² on a carbon atom or a nitrogen atom.

More preferred examples of the heterocyclic group linking to A² on a carbon atom include divalent groups derived from monocyclic or bicyclic C₃-C₉ aromatic heterocyclic compounds having 1 or 2 atoms selected from the group consisting of an oxygen atom, a nitrogen atom and a sulfur atom, in the ring, such as furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxybenzene, benzimidazole, indole, quinoline, isoquinoline or quinazoline.

Meanwhile, preferred examples of the heterocyclic group having 1 to 4 atoms selected from the group consisting of an oxygen atom, a nitrogen atom and a sulfur atom, in the ring, the heterocyclic group linking to A² on a nitrogen atom, include divalent groups derived from monocyclic or bicyclic C₂-C₉ heterocyclic compounds having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine. More preferred examples of the monocyclic C₂-C₉ heterocyclic compounds having 1 or 2 atoms selected from the group consisting of an oxygen atom, a nitrogen atom and a sulfur atom, in the ring, include piperidine, homopiperidine, morpholine, homopiperazine and piperazine.

The substituent of the heterocyclic group having 1 to 4 atoms may be selected from the group consisting of an oxygen atom, a nitrogen atom and a sulfur atom on its substituted ring of G¹, include a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, and an optionally substituted aliphatic hydrocarbon group having 1 to 6 carbon atoms.

Specific examples of the substituent of the heterocyclic group having 1 to 4 atoms selected from the group consisting of an oxygen atom, a nitrogen atom and a sulfur atom on its substituted ring of G¹ include the same as those exemplified in the substituted alicyclic hydrocarbon group having 3 to 10 carbon atoms of G¹.

As the substituent of the heterocyclic group having 1 to 4 atoms selected from the group consisting of an oxygen atom, a nitrogen atom and a sulfur atom on its substituted ring of G¹, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms and aliphatic hydrocarbon group having 1 to 6 carbon atoms may further be substituted with (one or more substituents selected from the group consisting of a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropyl- carbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; and a cyano group).

Preferred examples of the substituent of the heterocyclic group having 1 to 4 atoms selected from the group consisting of an oxygen atom, a nitrogen atom and a sulfur atom on its substituted ring of G¹, include a fluorine atom; a chlorine atom; a bromine atom; a C₁-C₆ alkoxy group consisting of a straight or branched alkyl and oxy group, including methoxy, ethoxy, propoxy, isopropoxy, butoxy, isobutoxy, s-butoxy, t-butoxy, pentyloxy, isopentyloxy, neopentyloxy, t-pentyloxy and hexyloxy; a cyano group; a nitro group; a carboxyl group; a hydroxy group; an amino group; a C₁-C₆ mono or dialkylamino group consisting of a straight or branched alkyl and amino group, including methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t- butylamino, N-ethylisopropylamino, dipropylamino, diisopropylamino and ethylbutylamino; a carbamoyl group; an aminosulfonyl group; an alicyclic hydrocarbon group having 3 to 6 carbon atoms, including cyclopropyl, cyclobutyl, cyclopentyl and cyclohexyl; a C₂-C₇ acyl group including acetyl, propionyl, butyryl, isobutyryl, pivaroyl and hexanoyl; a C₁-C₆ alkylthio group including methylthio, ethylthio, propylthio, isopropylthio, butylthio, isobutylthio, s-butylthio, t-butylthio, pentylthio and hexylthio; a C₁-C₆ alkylsulfonyl group, including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl and hexylsulfonyl; a C₂-C₇ alkoxycarbonyl group including acetoxy, propionyloxy, butyryloxy, isobutyryloxy, valeryloxy, isovaleryloxy, pivaloyloxy and hexanoyloxy; a C₂-C₇ acylamino group including acetylamino, propionylamino, butyrylamino, isobutyrylamino, valerylamino and hexanoylamino; trifluoromethyl, trifluoromethoxy, and an aliphatic hydrocarbon group having 1 to 6 carbon atoms which may contain a straight or branched unsaturated bond, including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2- propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl.

Specifically, more preferred examples of the substituent of the heterocyclic group having 1 to 4 atoms selected from the group consisting of an oxygen atom, a nitrogen atom and a sulfur atom on its substituted ring of G¹, include a fluorine atom, a chlorine atom, a bromine atom, C₁-C₆ alkoxy group, cyano group, a nitro group, a carboxyl group, a hydroxy group, an amino group, a C₁-C₆ mono or dialkylamino group, a carbamoyl group, an alicyclic hydrocarbon group having 3 to 6 carbon atoms, a C₂-C₇ acyl group, a C₁-C₆ alkylsulfonyl group, a C₂-C₇ alkoxycarboxyl group, a trifluoromethyl group, a trifluoromethoxy group, and a C₁-C₆ alkyl group including methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl. Examples of particularly preferred substituents include a fluorine atom, a chlorine atom, a C₁-C₆ alkoxy group, a cyano group, a nitro group, a carboxyl group, a hydroxy group, an amino group, a C₁-C₆ mono or dialkylamino group, an alicyclic hydrocarbon group having 3 to 6 carbon atoms, a C₂-C₇ acyl group, a trifluoromethyl group, a trifluoromethoxy group and a C₁-C₆ alkyl group.

In the present invention, G¹ in the formula (I) is preferably a single bond, a monocyclic aliphatic hydrocarbon group having 3 to 6 carbon atoms, a phenylene group, a monocyclic or bicyclic aromatic hydrocarbon group having 3 to 9 carbon atoms having 1 or 2 atoms selected from among a group consisting of an oxygen atom, a nitrogen atom, and a sulfur atom, in the ring, or a monocyclic heterocyclic group having 2 to 9 carbon atoms having 1 or 2 atoms selected from among a group consisting of an oxygen atom, a nitrogen atom, and a sulfur atom, in the ring.

In the formula (I), G² represents a hydrogen atom, a substituted or unsubstituted acyclic aliphatic hydrocarbon group having 1 to 10 carbon atoms, a substituted or unsubstituted alicyclic hydrocarbon group having 3 to 10 carbon atoms, a substituted or unsubstituted aromatic hydrocarbon group having 6 to 14 carbon atoms, or a substituted or unsubstituted heterocyclic group having 1 to 4 atoms selected from among a group consisting of an oxygen atom, a nitrogen atom, and a sulfur atom, in the ring.

In formula (I), when G² represents a substituted or unsubstituted acyclic aliphatic hydrocarbon group having 1 to 10 carbon atoms, examples of such a acyclic aliphatic hydrocarbon group having 1 to 10 carbon atoms of G² include an alkyl group such as methyl, ethyl, propyl, isopropyl, butyl, isobutyl, s-butyl, t-butyl, pentyl, isopentyl, neopentyl, t-pentyl, 2-methylpentyl, 4-methylpentyl, 1-ethylbutyl, hexyl, heptyl, 2-methylhexyl, 5-methylhexyl, 1,1-dimethylpentyl, 6-methylheptyl, octyl, nonyl or decyl, an alkenyl group such as vinyl, 1-methylvinyl, 1-ethylvinyl, 1-propenyl, 2-propenyl, 2-methyl-1-propenyl, 1-butenyl, 2-butenyl, 2-methyl-1-butenyl, 1,3-butadienyl, 1-pentenyl, 2-pentenyl, 4-methyl-1-pentenyl, 1-hexenyl, 2-hexenyl, 3-hexenyl, 4-hexenyl, 5-hexenyl, 1, 5-hexadienyl, 2-heptenyl, 2-octenyl, 2-nonenyl or 2-decenyl, or an alkynyl group such as ethynyl, 1-propynyl, 2-propynyl, 1-butynyl, 3-methyl-1-butynyl, 3, 3-dimethyl-1-butynyl, 1-pentynyl, 2-pentynyl, 3-pentynyl, 1-hexynyl, 2-hexynyl, 3-hexynyl, 4-hexynyl, 1-methyl-3-pentynyl, 1-methyl-3-hexynyl, 2-heptynyl, 2-octynyl, 2-nonynyl or 2-decynyl.

Specifically, more preferred examples of such aliphatic hydrocarbon group having 1 to 10 carbon atoms include a straight or branched C₁-C₆ alkyl group which may contain a unsaturated bond such as methyl, ethyl, propyl, isopropyl, butyl, pentyl, hexyl, vinyl, 1-prophenyl, 1-butenyl, ethynyl or 1-propynyl. Particularly preferred examples of such a acyclic aliphatic hydrocarbon group having 1 to 10 carbon atoms include a straight or branched C₁-C₆ alkyl group such as methyl, ethyl, propyl, isopropyl, butyl or hexyl.

Substituents of the substituted acyclic aliphatic hydrocarbon group having 1 to 10 carbon atoms of G² include: a fluorine atom; a chlorine atom; a bromine atom; an iodine atom; a hydroxy group; a C₁-C₇ alkoxy group consisting of a straight or branched alkyl group, cycloalkyl group and oxy group, including methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methylpentyloxy, 1-ethylbutoxy, cyclopropyloxy, cyclobutyloxy, cyclopentyloxy, cyclohexyloxy, cyclopropylmethyloxy, cyclopropylethyloxy, cyclopentyl methyloxy and cyclohexylmethyloxy; an alkyldioxy group having 1 to 4 carbon atoms such as ethylene dioxy; a C₆-C₁₀ aryloxy group, including phenoxy, 1-naphthoxy and 2-naphthoxy; a C₇-C₉ aralkoxy group, including benzyloxy, α-phenethyloxy, β-phenethyloxy and phenylpropyloxy; a C₂-C₇ acyloxy group including acetoxy, propionyloxy, butyryloxy, isobutyryloxy, valeryloxy, isovaleryloxy, pivaloyloxy and hexanoyloxy; an oxo group; a C₁-C₆ alkylsulfonyloxy group consisting of a straight or branched alkyl and sulfonyloxy, including oxo, methylsulfonyloxy, ethylsulfonyloxy, propylsulfonyloxy, butylsulfonyloxy and t-butylsulfonyloxy; a C₂-C₇ acyl group, including acetyl, propionyl, butyryl, isobutyryl, valeryl, isovaleryl, pivaroyl and hexanoyl; a carboxyl group; a C₂-C₇ alkoxycarbonyl group consisting of a straight or branched alkyl and oxycarbonyl group, including methoxycarbonyl, ethoxycarbonyl, propoxycarbonyl, isopropoxycarbonyl, butoxycarbonyl, isobutoxycarbonyl, s-butoxycarbonyl and t-butoxycarbonyl; a carbamoyl group; a C₂-C₇ alkylcarbamoyl group consisting of a straight or branched alkyl, cycloalkyl and carbamoyl group, including N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl and N,N-dipropylcarbamoyl; an amino group; a C₁-C₆ alkylamino group consisting of a straight or branched alkyl, cycloalkyl and amino group, including methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutylamino, N-methyl-t-butylamino, N-ethylisopropyl- amino, dipropylamino, diisopropylamino and ethylbutylamino; a C₂-C₇ acylamino group including acetylamino, propionylamino, butyrylamino, isobutyrylamino, valerylamino and hexanoylamino; a C₂-C₈ alkoxycarbonylamino group, including methoxycarbonylamino, ethoxycarbonylamino and t-butoxy- carbonylamino; a C₁-C₆ alkylsulfonylamino group including methylsulfonylamino, ethylsulfonylamino, butylsulfonylamino and t-butylsulfonylamino; a cyano group; a nitro group; a C₁-C₆ alkylthio group including methylthio, ethylthio, propylthio, isopropylthio, butylthio, isobutylthio, s-butylthio, t-butylthio, pentylthio and hexylthio; a C₁-C₆ alkylsulfynyl group consisting of a straight or branched alkyl, cycloalkyl and sulfynyl group, including methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl and cyclopentylsulfynyl; a C₁-C₆ alkylsulfonyl group consisting of a straight or branched alkyl, cycloalkyl and sulfonyl group, including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, cyclopentylsulfonyl and cyclohexylsulfonyl; a sulfo group; a sulfamoyl group; a C₁-C₆ aminosulfonyl group consisting of a straight or branched alkyl, cycloalkyl and aminosulfonyl group, including methylaminosulfonyl, ethylaminosulfonyl, propylaminosulfonyl, isopropylaminosulfonyl, butylaminosulfonyl, isobutyl- aminosulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl and cyclopropylmethylaminosulfonyl; an alicyclic hydrocarbon group having 3 to 6 carbon atoms, including cyclopropyl, cyclobutyl, cyclopentyl and cyclohexyl; and an aliphatic hydrocarbon group having 1 to 6 carbon atoms which may contain a straight or branched unsaturated bond, including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2- propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl; an aromatic hydrocarbon group having 6 to 14 carbon atoms which is a monovalent group derived from monocyclic, bicyclic or tricyclic aromatic hydrocarbon group, including benzene, naphthalene, indene, indane, 1,2,3,4-tetrahydronaphthalene, and fluorene; and a monovalent group derived from monocyclic, bicyclic or tricyclic heterocyclic compound, including furan, thiophene, pyrrole, pyrroline, pyrrolidine, oxazole, oxazolidine, isooxazole, isooxazolidine, thiazole, thiazolidine, isothiazole, isothiazolidine, imidazole, imidazoline, imidazolidine, pyrazole, pyrazoline, pyrazolidine, triazole, thiadiazole, oxadiazole, tetrazole, pyran, tetrahydropyran, thiopyran, tetrahydrothiopyran, pyridine, pyrazine, pyrimidine, pyridazine, benzofuran, dibenzofuran, benzothiophene, indole, benzimidazole, benzothiazole, benzooxazole, chroman, isochroman, quinoline, decahydroquinoline, isoquinoline, quinazolin, quinoxaline, purine, pteridine, azetidine, morpholine, thiomorpholine, piperidine, homopiperidine, piperazine, homopiperazine, indoline, isoindoline, phenoxazine, phenazine, phenothiazine and quinuclidine, the heterocyclic compound (having 1 to 4 atoms selected from the group consisting of an oxygen atom, a nitrogen atom and a sulfur atom, in the ring).

Preferred examples of the substituent of the substituted acyclic aliphatic hydrocarbon group having 1 to 10 carbon atoms as G² include a fluorine atom, a hydroxy group, an optionally substituted C₁-C₇ alkoxy group, an oxo group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₁-C₆ alkylsulfonylamino group, a cyano group, a C₁-C₆ alkylsulfonyl group, a sulfamoyl group, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

More preferred exemplary substituents of the substituted acyclic aliphatic hydrocarbon group having 1 to 10 carbon atoms as G² include a fluorine atom, a hydroxy group, an optionally substituted C₁-C₇ alkoxy group, a carboxyl group, an amino group, an optionally substituted C₁-C₆ alkylamino group, a cyano group, a benzyl group, and an optionally substituted heterocyclic group (having 1 to 4 atoms selected from the group consisting of an oxygen atom, a nitrogen atom and a sulfur atom, in the ring).

As the substituent of the substituted acyclic aliphatic hydrocarbon group having 1 to 10 carbon atoms as G², the heterocyclic group (having 1 to 4 atoms selected from the group consisting of an oxygen atom, a nitrogen atom and a sulfur atom, in the ring), links to the aliphatic hydrocarbon group having 1 to 10 carbon atoms as G² on a carbon atom or a nitrogen atom.

Preferred examples of the heterocyclic group (having 1 to 4 atoms selected from the group consisting of an oxygen atom, a nitrogen atom and a sulfur atom, in the ring), links to the aliphatic hydrocarbon group having 1 to 10 carbon atoms as G² on a carbon atom, include a monovalent group derived from a monocyclic or bicyclic C₃-C₉ aromatic heterocyclic compound, including furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxybenzene, benzimidazole, indole, quinoline, isoquinoline and quinazolin, the monovalent group having 1 or 2 atoms selected from the group consisting of an oxygen atom, a nitrogen atom and a sulfur atom, in the ring.

Meanwhile, preferred examples of the heterocyclic group (having 1 to 4 atoms selected from the group consisting of an oxygen atom, a nitrogen atom and a sulfur atom, in the ring), links to the acyclic aliphatic hydrocarbon group having 1 to 10 carbon atoms as G² on a nitrogen atom, include a monovalent group derived from a monocyclic C₂-C₉ heterocyclic compound, including pyrrolidine, piperidine, morpholine, thiomorpholine, homopiperidine, homopiperazine, 1,2,3,6-tetrahydropyridine and piperazine, the monovalent group having 1 or 2 atoms selected from the group consisting of an oxygen atom, a nitrogen atom and a sulfur atom, in the ring.

As the substituent of the substituted acyclic aliphatic hydrocarbon group having 1 to 10 carbon atoms as G², a C₁-C₇ alkoxy group, a C₂-C₇ acyl group, C₂-C₇ alkylcarbamoyl, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, aromatic hydrocarbon group having 6 to 14 carbon atoms, and heterocyclic group having 1 to 4 atoms selected from the group consisting of an oxygen atom, a nitrogen atom and a sulfur atom, in the ring, may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group.

In the formula (I), when G² represents a substituted or unsubstituted alicyclic hydrocarbon group having 3 to 10 carbon atoms, examples of the alicyclic hydrocarbon group having 3 to 10 carbon atoms of G² include cyclopropyl, cyclobutyl, cyclopentyl, cyclopentenyl, cyclohexyl, cyclohexenyl, cycloheptyl, cycloheptenyl, and cyclooctyl group. Preferred examples of the alicyclic hydrocarbon group having 3 to 10 carbon atoms of G² include cyclopropyl, cyclobutyl, cyclopentyl, cyclohexyl, 3-cyclopentenyl, 4-cyclopentenyl, 1-cyclohexenyl, 3-cyclohexenyl, 4-cyclohexenyl, and 1-cycloheptenyl.

The substituents of the substituted alicyclic hydrocarbon group having 3 to 10 carbon atoms of G² include a fluorine atom, a chlorine atom, a bromine atom, an iodine atom, a hydroxy group, an optionally substituted C₁-C₇ alkoxy group, C₁-C₄ alkylenedioxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, and an optionally substituted aliphatic hydrocarbon group having 1 to 6 carbon atoms, an optionally substituted aromatic hydrocarbon group having 6 to 14 carbon atoms.

Specific examples of the substituent of the substituted alicyclic hydrocarbon group having 3 to 10 carbon atoms of G² include the same as those exemplified in the substituted aliphatic hydrocarbon group having 1 to 10 carbon atoms of G².

As the substituent of the substituted alicyclic hydrocarbon group having 3 to 10 carbon atoms of G², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, and an aromatic hydrocarbon group having 6 to 14 carbon atoms, may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino, a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

In the formula (I), when G² represents a substituted or unsubstituted aromatic hydrocarbon group having 6 to 14 carbon atoms, examples of the aromatic hydrocarbon group having 6 to 14 carbon atoms include a monovalent group having at least one aromatic ring on its molecule, such as benzene, indene, indane, naphthalene, 1,2-dihydronaphthalene, 1,2,3,4- tetrahydronaphthalene, azulene, acenaphthylene, acenaphthene, fluorene, phenanthrene or anthracene. Examples of such preferred aromatic hydrocarbon group having 6 to 14 carbon atoms of G² include a phenyl group.

The substituents of the aromatic hydrocarbon group having 6 to 14 carbon atoms of G² include at least one substituent selected from the group consisting of a fluorine atom, a chlorine atom, a bromine atom, an iodine atom, a hydroxy group, an optionally substituted C₁-C₇ alkoxy group, a C₁-C₄ alkylenedioxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, and an optionally substituted aromatic hydrocarbon group having 6 to 14 carbon atoms.

Specific examples of the substituent of the substituted C₆-C₁₄ aromatic hydrocarbon group of G² include the same as those exemplified in the substituent of the substituted aliphatic hydrocarbon group having 1 to 10 carbon atoms of G².

As the substituents of the aromatic hydrocarbon group having 6 to 14 carbon atoms of G², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, and an aromatic hydrocarbon group having 6 to 14 carbon atoms, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

In the formula (I), when G² represents a heterocyclic group having 1 to 4 atoms selected from the group consisting of an oxygen atom, a nitrogen atom and a sulfur atom on its substituted or unsubstituted ring, examples of such heterocyclic group include a monovalent group derived from monocyclic, bicyclic or tricyclic compounds, including furan, thiophene, pyrrole, pyrroline, pyrrolidine, oxazole, oxazolidine, isooxazole, isooxazolidine, thiazole, thiazolidine, isothiazole, isothiazolidine, imidazole, imidazoline, imidazolidine, pyrazole, pyrazoline, pyrazolidine, triazole, thiadiazole, oxadiazole, tetrazole, pyran, tetrahydropyran, thiopyran, tetrahydrothiopyran, pyridine, pyrazine, pyrimidine, pyridazine, benzofuran, dibenzofuran, benzothiophene, indole, 1,2-methylenedioxybenzene, benzimidazole, benzothiazole, benzooxazole, chroman, isochroman, quinoline, decahydroquinoline, isoquinoline, quinazolin, quinoxaline, purine, pteridine, azetidine, morpholine, thiomorpholine, piperidine, homopiperidine, piperazine, homopiperazine, indoline, isoindoline, phenoxazine, phenazine, phenothiazine and quinuclidine.

Preferred examples of the heterocyclic group having 1 to 4 atoms selected from the group consisting of an oxygen atom, a nitrogen atom and a sulfur atom, in the ring of G² include 2-pyridyl, 3-pyridyl, 4-pyridyl, piperidino, 2-piperizyl, 3-piperizyl, 4-piperizyl, morpholino, 1-homopiperidinyl, 1-pyrrolidinyl, 2-thiazolyl, 4-thiazolyl, 5-thiazolyl, 2-oxazolyl, 4-oxazolyl, 5-oxazolyl, 1-imidazolyl, 2-imidazolyl, 4-imidazolyl, 5-imidazolyl, 3-pyrazolyl, 2-furyl, 3-furyl, 2-thienyl, 3-thienyl, 2-pyrrolyl, 3-pyrrolyl, 4-isooxazolyl, 2-pyrimidinyl, 4-pyrimidinyl, 2-pyrazinyl, 4-triazolyl, 5-tetrazolyl, 1-piperazinyl, 4-tetrahydropyranyl, 2-1,3,4-oxadiazolyl, 4-1,2,3-thiadiazolyl, 2-benzofuranyl, 2-benzothiazolyl, 2-indolyl, 3-indolyl, 5-benzoimidazolyl and 2-1,2,3,4-tetrahydroisoquinolinyl group.

The heterocyclic group having 1 to 4 atoms selected from the group consisting of an oxygen atom, a nitrogen atom and a sulfur atom, in the ring of G², links to A⁴ on a carbon atom or a nitrogen atom.

More preferred examples of the heterocyclic group having 1 to 4 atoms selected from the group consisting of an oxygen atom, a nitrogen atom and a sulfur atom, in the ring of G², the heterocyclic group linking to A⁴ on a carbon atom, include a monovalent group derived from a monocyclic or bicyclic C₃-C₉ aromatic heterocyclic compound having 1 or 2 atoms selected from the group consisting of an oxygen atom, a nitrogen atom and a sulfur atom, in the ring, such as furan, pyrrole, thiophene, pyrazole, oxazole, thiazole, isooxazole, isothiazole, pyrazole, imidazole, pyridine, pyrimidine, pyrazine, pyridazine, benzothiophene, benzofuran, 1,2-methylenedioxy- benzene, benzimidazole, indole, quinoline, isoquinoline or quinazolin.

Meanwhile, preferred examples of the heterocyclic group having 1 to 4 atoms selected from the group consisting of an oxygen atom, a nitrogen atom and a sulfur atom, in the ring of G², the heterocyclic group linking to A⁴ on a nitrogen atom, include a monovalent group derived from a monocyclic C₂-C₉ heterocyclic compound having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine.

More preferred examples of the heterocyclic group as G² include a monovalent group derived from a monocyclic C₄-C₆ heterocyclic compound having 1 or 2 atoms selected from the group consisting of an oxygen atom, a nitrogen atom and a sulfur atom, in the ring, such as piperidine, homopiperidine, morpholine, homopiperazine, or piperazine.

The substituents of the heterocyclic group having 1 to 4 atoms selected from the group consisting of an oxygen atom, a nitrogen atom and a sulfur atom on its substituted ring of G² include at least one substituent selected from the group consisting of a fluorine atom, a chlorine atom, a bromine atom, an iodine atom, a hydroxy group, an optionally substituted C₁-C₇ alkoxy group, a C₁-C₄ alkylenedioxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, and an optionally substituted aromatic hydrocarbon group having 6 to 14 carbon atoms and.

The substituents of the heterocyclic group having 1 to 4 atoms selected from the group consisting of an oxygen atom, a nitrogen atom and a sulfur atom, in the ring of G² are as defined above for the substituent of the substituted aliphatic hydrocarbon group having 1 to 10 carbon atoms of G².

As the substituent of the heterocyclic group having 1 to 4 atoms selected from the group consisting of an oxygen atom, a nitrogen atom and a sulfur atom on its substituted ring of G², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, and an aromatic hydrocarbon group having 6 to 14 carbon atoms, may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropyl- carbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

In the present description, when G¹, G², or the substituent of G² represents a substituted or unsubstituted aromatic hydrocarbon group, a substituted or unsubstituted alicyclic hydrocarbon group, or a substituted or unsubstituted heterocyclic group, the aromatic hydrocarbon group, alicyclic hydrocarbon group, or heterocyclic group is preferably selected from the group consisting of cyclopropane, cyclopentane, cyclohexane, cyclohexene, cycloheptane, *nolvolnane, adamantine, benzene, naphthalene, indane, indoles, 1,3-benzodioxol, benzoimidazol, benzotriazol, pyrazol, imidazol, pyrazoron, thiazol, tetrazol, 1,2,4-oxadiazol, isooxazol, furan, thiophene, pyridine, pyradine, pyrrole, morpholine, benzofuran, benzothiophene, piperazine, pyrrolidine, homopiperizine, tetrahydroisoquinoline, pyrimidine, and quinazoline.

Next, an explanation will be given of preferred combinations of A¹, A², G¹, A³, A⁴ and G² in the formula (I).

When both of A¹ and A³ represent aliphatic hydrocarbon group, at least one of A² and G¹ is not a single bond.

The preferred combinations of A¹, A², G¹, A³, A⁴ and G², and preferred combinations including also substituents of them if they have substituents are basically preferably combinations of those preferably selected from among A¹, A², G¹, A³, A⁴ and G², and substituents of them. Then, more preferred combinations are combinations of more preferred elements.

In the formula (I), A¹ is, -(CH₂)₂- or -(CH₂)₃-. A₂ especially preferably represents -C(=O)-, -C(=O)-O-, -C(=O)-NH-, -C(=O)-NMe-, - NH-, -NH-C(=O)-, -NH-C(=O)-O-, -NH-C(=O)-NH-, -NH-C(=O)-NMe-, or -NH-C(=S)-. Specifically preferably A² represents -C(=O)-NH-, -NH-, -NH-C(=O)-, -NH-C(=O)-O-, or -NH-C(=O)-NH-.

Preferred combinations of G¹, A³, A⁴ and G² of G¹-G² portion include combinations of 1 to 10 of the following table.

| Combination | G¹ | A³ | A⁴ | G² |
|---|---|---|---|---|
| 1 | Group other than single bond | Single bond | Single bond | Hydrogen atom |
| 2 | Single bond | Group other than single bond | Single bond | Hydrogen atom |
| 3 | Group other than single bond | Single bond | Single bond | Group other than a hydrogen atom |
| 4 | Single bond | Group other than single bond | Single bond | Group other than a hydrogen atom |
| 5 | Group other than single bond | Single bond | Group other than single bond | Group other than a hydrogen atom |
| 6 | Single bond | Group other than single bond | Group other than single bond | Group other than a hydrogen atom |
| 7 | Group other than single bond | Group other than single bond | Single bond | Group other than a hydrogen atom |
| 8 | Group other than single bond | Group other than single bond | Group other than single bond | Group other than a hydrogen atom |
| 9 | Group other than single bond | Group other than single bond | Group other than single bond | Hydrogen atom |
| 10 | Single bond | Single bond | Single bond | Hydrogen atom |

In the table, in combinations of numbers 4 to 7, A³ represents an alkylene group having 1 to 3 carbon atoms.

Also, in the combination of number 5, A⁴ preferably represents -C(=O)-, -C(=O)-NH-, -O-, or -NH-C(=O)-.

Also, in the combination of number 8, A⁴ preferably represents -O-.

Further, combinations of the following a) to f) are preferable.
a) A¹ represents -(CH₂)₂- or -(CH₂)₃-, A² represents -NH-(C=O)- or -NH-(C=O)-NH-, G¹ represents a single bond, and A³ represents a divalent acyclic aliphatic hydrocarbon group having 1 to 10 carbon atoms.
b) A¹ represents -(CH₂)₂- or -(CH₂)₃-, A² represents -NH-(C=O)-, -NH-(C=O)-NH-, -NH-, or -C-(=O)-NH-, and G¹ represents a group other than the single bond.
d) A¹ represents a divalent acyclic aliphatic hydrocarbon group having 1 to 6 carbon atoms, specifically -(CH₂)₂- or -(CH₂)₃-, A² represents those other than a single bond, and G¹ represents an optionally substituted aromatic hydrocarbon group, an optionally substituted alicyclic hydrocarbon group having 7 to 10 carbon atoms, or an optionally substituted heterocyclic group (note, where the aromatic hydrocarbon group of G¹ is a phenyl group, or where the heterocyclic group of G¹ is 5 or 6 membered monocyclic ring, the phenyl group or 5 or 6 membered monocyclic heterocyclic group of G¹ is substituted, or A³-G² portion represents those other than the hydrogen atom).
e) A¹ represents a divalent acyclic aliphatic hydrocarbon group having 1 to 6 carbon atoms, specifically -(CH₂)₂- or -(CH₂)₃-, A² represents those other than a single bond, G¹ and A⁴ represent the single bond, A³ represents an optionally substituted acyclic aliphatic hydrocarbon group having 1 to 10 carbon atoms, G² represents an optionally substituted alicyclic hydrocarbon group having 5 to 10 carbon atoms, an optionally substituted aromatic hydrocarbon group, or optionally substituted heterocyclic group. f) A¹ represents a divalent acyclic aliphatic hydrocarbon group having 1 to 6 carbon atoms, specifically -(CH₂)₂- or -(CH₂)₃-, A² represents those other than a single bond, G¹ represents the single bond, A³ represents an optionally substituted acyclic aliphatic hydrocarbon group having 1 to 10 carbon atoms, and A⁴ represents -C(=O)-, -C(=O)-NR¹²¹-, -C(=S)-NR¹²²-,-C(=NR¹²³)-, -O-C(=O)-, -NR¹²⁵-C(=O)-, -NR¹²⁶-S(=O)₂-, -NR¹²⁷-C(=O)-O-, -NR¹²⁸-C(=O) -NR¹²⁹-, -NR¹³⁰-C(=S)-, -NR¹³¹-C(=S)-NR¹³²-, -S-, -S(=O)-, *-*S(=O)₂-, -S(=O)₂-NR¹³³- or -S(=O)₂-O-.

In the cases of d) to f), A² preferably represents - C(=O)-, -C(=O)-O-, -C(=O)-NH-, -C(=O)-NMe-, -NH-, -NH-C(=O)-, -NH-C(=O)-O-, -NH-C(=O)-NH-, -NH-C(=O)-NMe-, or - NH-C(=S)-, especially preferably represents -C(=O)-NH-, - NH-, -NH-C(=O)-, -NH-C(=O)-O-, or -NH-C(=O)-NH-.

In the formula (I), A⁵ represents a single bond or represents a group that links R² with a carbon atom of a pyrrole ring to which A⁵ is bonded, in the form of R²-NR²⁰¹-pyrrole ring (R²⁰¹ represents a hydrogen atom or a acyclic aliphatic hydrocarbon group having 1 to 4 carbon atoms), when A⁵ bonds R² and a carbon atom of a pyrrole ring to which A⁵ is bonded, in the form of R²-NR²⁰¹-pyrrole ring, examples of the acyclic aliphatic hydrocarbon group having 1 to 4 carbon atoms of R²⁰¹ are the same as those exemplified as R¹⁰¹ of A² described above. Preferred examples of R¹⁰² include a hydrogen atom, methyl, ethyl or propyl group, and specifically preferably hydrogen atom and methyl group.

Preferred examples of A⁵ include a single bond, -NH-, and N(CH₃)-, and specifically preferably single bond.

In the formula (I), R² represents a hydrogen atom, a fluorine atom, a chlorine atom, a bromine atom, an iodine atom, an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring.

R² in the formula (I) is preferably a chlorine atom or a bromine atom among a fluorine atom, a chlorine atom, a bromine atom, and an iodine atom.

In the formula (I), when R² represents an optionally substituted acyclic aliphatic hydrocarbon group having 1 to 10 carbon atoms, examples of acyclic aliphatic hydrocarbon group having 1 to 10 carbon atoms of R² are the same as those exemplified of the acyclic aliphatic hydrocarbon group having 1 to 10 carbon atoms of G². Preferred examples of the acyclic aliphatic hydrocarbon group having 1 to 10 carbon atoms of R² include methyl, ethyl, isopropyl, butyl, isobutyl, t-butyl, t-pentyl, vinyl, 2-propenyl, 2-methyl-1-propenyl, and 2-propenyl.

Substituents for the substituted acyclic aliphatic hydrocarbon group having 1 to 10 carbon atoms of R² include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂₋C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 1 to 6 carbon atoms, an optionally substituted acyclic aliphatic hydrocarbon group having 1 to 6 carbon atoms, an optionally substituted aromatic hydrocarbon group having 6 to 14 carbon atoms, and an optionally substituted heterocyclic group (having 1 to 4 atoms selected from the group consisting of an oxygen atom, a nitrogen atom and a sulfur atom, in the ring).

Specific examples of the substituent of the substituted acyclic aliphatic hydrocarbon group having 1 to 10 carbon atoms of R² include the same as those exemplified as the substituents of the substituted acyclic aliphatic hydrocarbon group having 1 to 10 carbon atoms of G².

As the substituent of substituted acyclic aliphatic hydrocarbon group having 1 to 10 carbon atoms as R², a C₁-C₇ alkoxy group, a C₂-C₇ acyl group, C₂-C₇ alkylcarbamoyl group, a C₁-C₆ alkylamino group, a C₂-C₇ acylamino group, an alicyclic hydrocarbon group having 3 to 6 carbon atoms, a acyclic aliphatic hydrocarbon group having 1 to 6 carbon atoms, aromatic hydrocarbon group having 6 to 14 carbon atoms, and heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a G₁-C₆ alkyl group including methyl, ethyl, propyl, isopropyl, butyl, isobutyl, s-butyl, and t-butyl; a trifluoromethyl group; and a trifluoromethoxy group).

In the formula (I), when R² represents a substituted or unsubstituted alicyclic hydrocarbon group having 3 to 8 carbon atoms, examples of the alicyclic hydrocarbon group having 3 to 8 carbon atoms of R² the same as defined above for the substituents of the substituted alicyclic hydrocarbon group having 3 to 8 carbon atoms of G². Preferred examples of the alicyclic hydrocarbon group having 3 to 8 carbon atoms of R² include cyclopropyl, cyclobutyl, cyclopentyl and cyclohexyl. Among them, the cyclopropyl group is preferred.

Substituents of the substituted alicyclic hydrocarbon group having 3 to 8 carbon atoms of R² include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group (having 1 to 4 atoms selected from the group consisting of an oxygen atom, a nitrogen atom and a sulfur atom, in the ring).

Specific examples of the substituent of the substituted alicyclic hydrocarbon group having 3 to 8 carbon atoms of R² include the same as those exemplified in the substituted alicyclic hydrocarbon group having 1 to 10 carbon atoms of G².

As the substituent of the substituted alicyclic hydrocarbon group having 3 to 8 carbon atoms of R², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group, and a trifluoromethoxy group).

In the formula (I), when R² is a substituted or unsubstituted aromatic hydrocarbon group having 6 to 14 carbon atoms, examples of the aromatic hydrocarbon group having 6 to 14 carbon atoms of R² include the same as those exemplified in the aromatic hydrocarbon group having 6 to 14 carbon atoms of G². Examples of such preferred aromatic hydrocarbon group having 6 to 14 carbon atoms of R² include a phenyl group.

Substituents of the substituted aromatic hydrocarbon group having 6 to 14 carbon atoms include at least one substituent selected from the group consisting of a fluorine atom, a chlorine atom, a bromine atom, an iodine atom, a hydroxy group, a C₁-C₆ alkyl group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group (having 1 to 4 atoms selected from the group consisting of an oxygen atom, a nitrogen atom and a sulfur atom, in the ring).

Specific examples of the substituent of the substituted aromatic hydrocarbon group having 6 to 14 carbon atoms R² include the same as those exemplified for the substituted acyclic aliphatic hydrocarbon group having 1 to 10 carbon atoms of G².

As the substituent of the aromatic hydrocarbon group having 6 to 14 carbon atoms of R², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group, and a trifluoromethoxy group).

In the formula (I), when R² represents a heterocyclic group having 1 to 4 atoms selected from the group consisting of an oxygen atom, a nitrogen atom and a sulfur atom on its substituted or unsubstituted ring of R², examples of heterocyclic group of R² include the same as those exemplified for the heterocyclic group of G². The heterocyclic group of R² links to A⁵ on a carbon atom or a nitrogen atom.

Examples of preferred heterocyclic group linking to A⁵ on a carbon atom include a monocyclic or cyclic C₃-C₉ aromatic heterocyclic group having 1 to 3 atoms selected from the group consisting of an oxygen atom, a nitrogen atom and a sulfur atom, in the ring, such as furyl, thienyl, pyrrolyl, pyrazolyl, oxazolyl, isooxazolyl, thiazolyl, isothiazolyl, imidazolyl, pyridyl, pyrazinyl, pyrimidinyl, pyridazinyl, benzofuranyl, indolyl, benzothienyl, quinolyl, isoquinolyl, quinazolyl, benzoimidazolyl or benzooxazolyl. More preferred example of the heterocyclic group include a monocyclic or bicyclic C₃-C₉ aromatic heterocyclic compound having 1 or 2 atoms selected from the group consisting of an oxygen atom, a nitrogen atom and a sulfur atom, in the ring, such as 2-furyl, 2-thienyl, 2-pyrrolyl, 2-imidazolyl, 5-imidazolyl, 4-pyrazolyl, 2-oxazolyl, 5-oxazolyl, 5-isooxazolyl, 2-thiazolyl, 5-thiazolyl, 5-isothiazolyl, 3-isothiazolyl, 2-pyridyl, 2-pyrimidinyl, 2-benzofuranyl or 2-benzothiophenyl group. Further, particularly preferable examples of the heterocyclic group include a monocyclic C₃-C₅ aromatic heterocyclic group having 1 or 2 atoms selected from the group consisting of an oxygen atom, a nitrogen atom and a sulfur atom, in the ring, and most preferably, 2-furyl, 2-thienyl, 2-pyrrolyl, 2-pyridyl or 4-pyrazolyl.

Meanwhile, preferred examples of the heterocyclic group having 1 to 4 atoms selected from the group consisting of an oxygen atom, a nitrogen atom and a sulfur atom, in the ring, links to A⁵ on a nitrogen atom, include 1-pyrazolyl, 1-imidazolyl, 1-pyrrolidinyl, piperidino, morpholino, 1-homopiperidinyl and 1-piperazinyl. When the heterocyclic group having 1 to 4 atoms selected from the group consisting of an oxygen atom, a nitrogen atom and a sulfur atom on a ring of R², links to A⁵ on a nitrogen atom, A⁵ represents a single bond.

Substituents of the heterocyclic group having 1 to 4 atoms selected from the group consisting of an oxygen atom, a nitrogen atom and a sulfur atom on the substituted ring of R² include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group (having 1 to 4 atoms selected from the group consisting of an oxygen atom, a nitrogen atom and a sulfur atom, in the ring).

Specific examples of the substituent of the heterocyclic group having 1 to 4 atoms selected from the group consisting of an oxygen atom, a nitrogen atom and a sulfur atom on its substituted ring of R² include the same as those exemplified as the substituents of the substituted aliphatic hydrocarbon group having 1 to 10 carbon atoms of G².

As the substituent of the heterocyclic group having 1 to 4 atoms selected from the group consisting of an oxygen atom, a nitrogen atom and a sulfur atom on its substituted ring of R², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, aliphatic hydrocarbon group having 1 to 6 carbon atoms, an aromatic hydrocarbon group having 6 to 14 carbon atoms, and a heterocyclic group (having 1 to 4 atoms selected from the group consisting of an oxygen atom, a nitrogen atom and a sulfur atom, in the ring) may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

Among exemplary substituents of the heterocyclic group having 1 to 4 atoms selected from the group consisting of an oxygen atom, a nitrogen atom and a sulfur atom on its substituted ring of R², preferred examples of the substituent include a fluorine atom, a chlorine atom, a bromine atom, an iodine atom, a hydroxy group, a cyano group, a nitro group, an amino group, a C₁-C₆ mono or dialkylamino group consisting of a straight or branched alkyl group and an amino group, such as substituted or unsubstituted methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t- butylamino, N-ethylisopropylamino, dipropylamino, diisopropylamino and ethylbutylamino, a carboxyl group, an optionally substituted saturated a C₁-C₆ alkyl group including a substituted or unsubstituted methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl, an alicyclic hydrocarbon group having 3 to 6 carbon atoms including cyclopropyl, cyclobutyl, cyclo pentyl and cyclohexyl, an optionally substituted C₁-C₆ alkoxy group consisting of a straight or branched alkyl group and an oxy group, including a substituted or unsubstituted methoxy, ethoxy, propoxy, isopropoxy, butoxy, isobutoxy, s-butoxy, t-butoxy, pentyloxy, isopentyloxy, neopentyloxy, t-pentyloxy and hexyloxy, a C₂-C₇ acyl group, including a substituted or unsubstituted acetyl, propionyl, butyryl, isobutyryl, pivaroyl and hexanoyl, a C₁-C₆ alkylthio group, including methylthio, ethylthio, propylthio, isopropylthio, butylthio, isobutylthio, s-butylthio, t-butylthio, pentylthio and hexylthio, trifluoromethyl group, trifluoromethoxy group, a C₂-C₇ acylamino group, including substituted or unsubstituted acetylamino, propionylamino, butyrylamino, isobutyrylamino, valerylamino and hexanoylamino, and a C₂-C₇ alkylcarbamoyl group consisting of a straight or branched alkyl group and a carbamoyl group, including a substituted or unsubstituted N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N,N-dimethylcarbamoyl, N-ethyl-N-methylcarbamoyl and N,N-diethylcarbamoyl.

More preferred examples of the substituent of the heterocyclic group having 1 to 4 atoms selected from the group consisting of an oxygen atom, a nitrogen atom and a sulfur atom on its substituted ring as R², include one or more of a fluorine atom, a chlorine atom, a bromine atom, an acyl group having 2 to 4 carbon atoms, a hydroxy group, a carboxyl group, an alkoxycarbonyl group, a substituted or unsubstituted C₁-C₆ alkyl group, a hydroxy group, and a substituted or unsubstituted C₁-C₆ alkoxy group.

Here, an explanation will be given of preferred combinations of R² and A⁵ of the formula (I).

In combinations of R² and A⁵ of the formula (I) in the present invention, when R² is a fluorine atom, a chlorine atom, a bromine atom, or an iodine atom, A⁵ represents a single bond.

Preferred examples of the combinations of R² and A⁵ of the formula (I) in the present invention include those representing an aliphatic hydrocarbon group having 1 to 10 carbon atoms wherein A⁵ represents a single bond, and R² may be substituted, an optionally substituted alicyclic hydrocarbon group having 3 to 8 carbon atoms, an optionally substituted aromatic hydrocarbon group having 6 to 14 carbon atoms, or an optionally substituted heterocyclic group. Specific preferred combinations are combinations representing an aliphatic hydrocarbon group having 1 to 10 carbon atoms wherein A⁵ represents a single bond, and R² may be substituted, an optionally substituted alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom and a sulfur atom, in the ring. Among them, cases where R² represents a cyclopropyl group, a cyclobutyl group, a cyclopropylmethyl group, a methyl group, an ethyl group, a vinyl group, an isopropyl group, an isobutyl group or 2-methyl-1-propenyl group are preferred.

Also, combinations of A⁵ and R² in which A⁵ represents a single bond and R² represents a thienyl group, a pyridyl group, a furyl group, a pyrazolyl group or a phenyl group are preferable, wherein the thienyl group, the pyridyl group, the furyl group, the pyrazolyl group or the phenyl group may be further substituted by one or more of a C₁-C₄ alkyl group, a C₁-C₄ alkoxy group, a C₂-C₄ acyl group, a hydroxy group, a carboxyl group, an alkoxycarbonyl group, a fluorine atom or a chlorine atom.

Also a combination wherein A⁵ is NR²⁰¹-, and R² represents a hydrogen atom or an optionally substituted aliphatic hydrocarbon group having 1 to 10 carbon atoms is preferred.

In the formula (I), A⁶ is a single bond.

In the formula (I), R³ represents a substituted or unsubstituted aromatic hydrocarbon group having 6 to 14 carbon atoms, or a heterocyclic group having 1 to 4 atoms selected from the group consisting of an oxygen atom, a nitrogen atom and a sulfur atom. alkoxycarbonylamino group, a C₁-C₆ alkylsulfonylamino group, a cyano group, a nitro group, a C₁-C₆ alkylthio group, a C₁-C₆ alkylsulfynyl group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples of the substituent of the substituted acyclic saturated aliphatic hydrocarbon group having 1 to 10 carbon atoms of R³ include the same as those exemplified in the substituted acyclic aliphatic hydrocarbon group having 1 to 10 carbon atoms of G².

As the substituent of the substituted acyclic saturated aliphatic hydrocarbon group having 1 to 10 carbon atoms of R³, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethyl-amino, diethylamino, N-methylpropylamino, N-methylisopropyl- amino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

In the formula (I), when R³ represents a substituted or unsubstituted alicyclic hydrocarbon group having 3 to 8 carbon atoms, examples of the alicyclic hydrocarbon group having 3 to 8 carbon atoms of R² include the same as those exemplified in the alicyclic hydrocarbon group having 3 to 10 carbon atoms of G². Preferred examples of the alicyclic hydrocarbon group having 3 to 8 carbon atoms of R² include cyclopropyl, cyclobutyl and cyclopentyl, cyclohexyl.

Exemplary substituents of the substituted alicyclic hydrocarbon group having 3 to 8 carbon atoms of R³ include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a cyano group, a nitro group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples of the substituent of the substituted alicyclic hydrocarbon group having 3 to 8 carbon atoms of R³ include the same as those exemplified as the substituents of the substituted the substituents of the substituted aliphatic hydrocarbon group having 1 to 10 carbon atoms of G².

As the substituent of the substituted alicyclic hydrocarbon group having 3 to 8 carbon atoms of R³, a C₁-C₇ alkoxy group, a C₂-C₇ acyl group, a C₂-C₇ alkylcarbamoyl group, a C₁-C₆ alkylamino group, a C₂-C₇ acylamino, an alicyclic hydrocarbon group having 3 to 6 carbon atoms, an aliphatic hydrocarbon group having 1 to 6 carbon atoms, an aromatic hydrocarbon group having 6 to 14 carbon atoms, and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a

In the formula (I), when R³ represents a substituted or unsubstituted aromatic hydrocarbon group having 6 to 14 carbon atoms, examples of the aromatic hydrocarbon group having 6 to 14 carbon atoms of R³ include the same as those exemplified in the aromatic hydrocarbon group having 6 to 14 carbon atoms of G². Preferred examples of the aromatic hydrocarbon group having 6 to 14 carbon atoms of R³ include a phenyl group.

Substituents of the substituted aromatic hydrocarbon group having 6 to 14 carbon atoms of R³ include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring.

Specific examples of the substituent of the substituted aromatic hydrocarbon group having 6 to 14 carbon atoms of R³ include the same as those exemplified in the substituent of the substituted aliphatic hydrocarbon group having 1 to 10 carbon atoms of G².

As the substituent of the substituted aromatic hydrocarbon group having 6 to 14 carbon atoms of R³, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and a heterocyclic group (having 1 to 4 atoms selected from the group consisting of an oxygen atom, a nitrogen atom and a sulfur atom, in the ring), may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

In the formula (I), when R³ represents a heterocyclic group having 1 to 4 atoms selected from the group consisting of an oxygen atom, a nitrogen atom and a sulfur atom on its substituted or unsubstituted ring, examples of such heterocyclic group having 1 to 4 atoms selected from the group consisting of an oxygen atom, a nitrogen atom and a sulfur atom on the ring of R³ include the same as those exemplified in the heterocyclic group having 1 to 4 atoms selected from the group consisting of an oxygen atom, a nitrogen atom and a sulfur atom on the ring of G².

The heterocyclic group having 1 to 4 atoms selected from the group consisting of an oxygen atom, a nitrogen atom and a sulfur atom on the ring of R³ links to A⁶ on a carbon atom or a nitrogen atom.

Preferred examples of the heterocyclic group having 1 to 4 atoms selected from the group consisting of an oxygen atom, a nitrogen atom and a sulfur atom on the ring of R³ and linking to A⁶ on a carbon atom, include a monocyclic or bicyclic C₃-C₉ aromatic heterocyclic group having 1 to 3 atoms selected from the group consisting of an oxygen atom, a nitrogen atom and a sulfur atom, in the ring, including furyl, thienyl, pyrrolyl, pyrazolyl, oxazolyl, isooxazolyl, thiazolyl, isothiazolyl, imidazolyl, pyridyl, N-oxopyridyl, pyrazinyl, pyrimidinyl, pyridazinyl, benzofuranyl, indolyl, benzothienyl, quinolyl, isoquinolyl, quinazolyl, benzoimidazolyl and benzooxazolyl, preferably 2-furyl, 3-furyl, 2-thienyl, 3-thienyl, 2-pyrrolyl, 2-oxazolyl, 2-thiazolyl, 2-pyridyl, 3-pyridyl, 4-pyridyl, 2-N-oxopyridyl, 3-N-oxopyridyl, 4-N-oxopyridyl, 3-pyrazolyl, 4-pyrazolyl, 4-imidazolyl, 2-pyrimidinyl, or 5-pyrimidinyl.

Meanwhile, preferred examples of the heterocyclic group having 1 to 4 atoms selected from the group consisting of an oxygen atom, a nitrogen atom and a sulfur atom on the ring of R³ and linking to A⁶ on a nitrogen atom, include 1-imidazolyl, 2-pyrazolyl, 1-pyrrolyl, 1-pyrrolidinyl, piperidino, morpholino, 1-homopiperidinyl and 1-piperazinyl, preferably 1-imidazolyl.

When the heterocyclic group having 1 to 4 atoms selected from the group consisting of an oxygen atom, a nitrogen atom and a sulfur atom on the ring of R³ links to A⁶ on a nitrogen atom, A⁶ is a single bond, or a group that links a carbon atom of a pyrrole ring in which R³ and A⁶ are linked to each other in the form of R³-C(=O)-pyrrole ring.

Substituents of the heterocyclic group having 1 to 4 atoms selected from the group consisting of an oxygen atom, a nitrogen atom and a sulfur atom on the ring of R³ include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms and an optionally substituted heterocyclic group (having 1 to 4 atoms selected from the group consisting of an oxygen atom, a nitrogen atom and a sulfur atom, in the ring).

Specific examples of the substituent of the heterocyclic group having 1 to 4 atoms selected from the group consisting of an oxygen atom, a nitrogen atom and a sulfur atom on its substituted ring of R³ include the same as those exemplified in the substituted aliphatic hydrocarbon group having 1 to 10 carbon atoms of G².

As the substituent of the heterocyclic group having 1 to 4 atoms selected from the group consisting of an oxygen atom, a nitrogen atom and a sulfur atom on its substituted ring of R³, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms and an aliphatic hydrocarbon group having 1 to 6 carbon atoms, an aromatic hydrocarbon group having 6 to 14 carbon atoms, and a heterocyclic group (having 1 to 4 atoms selected from the group consisting of oxygen atom, a nitrogen atom and a sulfur atom in the ring) may further be substituted with (one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropyl-carbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group).

Among those exemplified as substituents of the heterocyclic group having 1 to 4 atoms selected from the group consisting of an oxygen atom, a nitrogen atom and a sulfur atom on the ring of R³, preferred examples thereof include: a fluorine atom; a chlorine atom; a bromine atom; an iodine atom; a hydroxy group; a cyano group; a nitro group; an amino group; a C₁-C₆ mono or dialkylamino group consisting of a straight or branched alkyl group and an amino group, including a substituted or unsubstituted methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t-butylamino, N-ethylisopropylamino, dipropylamino, diisopropylamino and ethylbutylamino; a carboxyl group; a saturated a C₁-C₆ alkyl group including a substituted or unsubstituted methyl, ethyl, propyl, isopropyl, butyl, isobutyl, s-butyl, t-butyl, pentyl, isopentyl, neopentyl, t-pentyl, hexyl, isohexyl, 2-methylpentyl and 1-ethylbutyl; an alicyclic hydrocarbon group having 3 to 6 carbon atoms including cyclopropyl, cyclobutyl, cyclopentyl and cyclohexyl; a C₁-C₆ alkoxy group consisting of a straight or branched alkyl group and an oxy group, including a substituted or unsubstituted methoxy, ethoxy, propoxy, isopropoxy, butoxy, isobutoxy, s-butoxy, t-butoxy, pentyloxy, isopentyloxy, neopentyloxy, t-pentyloxy and hexyloxy; a C₂-C₇ acyl group including a substituted or unsubstituted acetyl, propionyl, butyryl, isobutyryl, pivaroyl and hexanoyl; a C₁-C₆ alkylthio group, including methylthio, ethylthio, propylthio, isopropylthio, butylthio, isobutylthio, s-butylthio, t-butylthio, pentylthio and hexylthio; a trifluoromethyl group; a trifluoromethoxy group; a C₂-C₇ acylamino group including a substituted or unsubstituted acetylamino, propionylamino, butyrylamino, isobutyrylamino, valerylamino and hexanoylamino; and a C₂-C₇ alkylcarbamoyl group consisting of a straight or branched alkyl group and a carbamoyl group including a substituted or unsubstituted N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N, N-dimethylcarbamoyl, N-ethyl-N-methylcarbamoyl and N,N-diethylcarbamoyl.

More preferred substituents of the heterocyclic group having 1 to 4 atoms selected from the group consisting of an oxygen atom, a nitrogen atom and a sulfur atom on the ring of R³, include a fluorine atom, a chlorine atom, a bromine atom, a substituted or unsubstituted C₁-C₆ alkyl group, a hydroxy group, and a substituted or unsubstituted C₁-C₆ alkoxy group. Specifically, a methyl group and an ethyl group are preferred.

Preferably R³ represents an optionally substituted aromatic hydrocarbon group having 6 to 14 carbon atoms or an optionally substituted heterocyclic group. Among them, a case where R³ represents a thienyl group, a furyl group, a pyrrolyl group, a pyrazolyl group or a phenyl group optionally substituted with one or more alkyl group having 1 to 4 carbon atoms is preferred.

R³ may represent a pyridyl group or 1-oxypyridyl group or pyrazolyl group or N-methylpyrazolyl group optionally substituted by an alkyl group having 1 to 4 carbon atoms or one halogen atom.

In addition R³ is a substituted or unsubstituted aromatic hydrocarbon group having 6 to 14 carbon atoms; group that links R³ and a carbon atom of a pyrrole ring in which A⁶ is linked in the form of the carbon atom of a R³-C(=O)-NH-pyrrole ring, and R³ is a substituted or unsubstituted aromatic hydrocarbon group having 6 to 14 carbon atoms; a combination in which A⁶ is a group that links R³ and a carbon atom of a pyrrole ring in which A⁶ is linked in the form of the carbon atom of the R³-C(=O)-NH-pyrrole ring, and R³ is a monocyclic C₃-C₅ aromatic heterocyclic group having 1 or 2 atoms selected from the group consisting of an oxygen atom, a nitrogen atom and a sulfur atom on the substituted or unsubstituted ring; a combination in which A⁶ is a group that links R³ and a carbon atom of a pyrrole ring in which A⁶ is linked in the form of the carbon atom of a R³-NH-pyrrole ring, and R³ is a hydrogen atom; a combination in which A⁶ is a group that links R³ and a carbon atom of a pyrrole ring in which A⁶ is linked in the form of the carbon atom of a R³-NH-pyrrole ring, and R³ is a substituted or unsubstituted acyclic saturated aliphatic hydrocarbon group having 1 to 10 carbon atoms; a combination in which A⁶ is a group that links R³ and a carbon atom of a pyrrole ring in which A⁶ is linked in the form of the carbon atom of a R³-NH-pyrrole ring, and R³ is a substituted or unsubstituted alicyclic hydrocarbon group having 3 to 8 carbon atoms; a combination in which A⁶ is a group that links R³ and a carbon atom of a pyrrole ring in which A⁶ is linked in the form of the carbon atom of a R³-NH-pyrrole ring, and R³ is a substituted or unsubstituted aromatic hydrocarbon group having 6 to 14 carbon atoms; a combination in which A⁶ is a group that links R³ and a carbon atom of a pyrrole ring in which A⁶ is linked in the form of the carbon atom of R³-NH-pyrrole ring, and R³ is a monocyclic C₃-C₅ aromatic heterocyclic group having 1 or 2 atoms selected from the group consisting of an oxygen atom, a nitrogen atom and a sulfur atom on the substituted or unsubstituted ring; a combination in which A⁶ is a group that links R³ and a carbon atom of a pyrrole ring in which A⁶ is linked in the form of the carbon atom of a R³-HC=CH-pyrrole ring, and _{R}³ is a hydrogen atom; a combination in which A⁶ is a group that links R³ and a carbon atom of a pyrrole ring in which A⁶ is linked to each other in the form of the carbon atom of a R³-HC=CH-pyrrole ring, and R³ is a substituted or unsubstituted acyclic saturated aliphatic hydrocarbon group having 1 to 10 carbon atoms; a combination in which A⁶ is a group that links a carbon atom of a pyrrole ring in which R³ and A⁶ are linked to each other in the form of the carbon atom of a R³-HC=CH-pyrrole ring, and R³ is a substituted or unsubstituted alicyclic hydrocarbon group having 3 to 8 carbon atoms; a combination in which A⁶ is a group that links R³ and a carbon atom of a pyrrole ring in which A⁶ is linked in the form of the carbon atom of a R³-HC=CH-pyrrole ring, and R³ is a substituted or unsubstituted aromatic hydrocarbon group having 6 to 14 carbon atoms; a combination in which A⁶ is a group that links R³ and a carbon atom of a pyrrole ring in which A⁶ is linked in the form of the carbon atom of a R³-HC=CH-pyrrole ring, and R³ is a monocyclic C₃-C₅ aromatic heterocyclic group having 1 or 2 atoms selected from the group consisting of an oxygen atom, a nitrogen atom and a sulfur atom on the substituted or unsubstituted ring; a combination in which A⁶ is a group that links R³ and a carbon atom of a pyrrole ring in which A⁶ is linked in the form of the carbon atom of a R³-C=C-pyrrole ring, and R³ is a hydrogen atom; a combination in which A⁶ is a group that links R³ and a carbon atom of a pyrrole ring in which A⁶ is linked in the form of the carbon atom of a R³-C≡C-pyrrole ring, and R³ is a substituted or unsubstituted acyclic saturated aliphatic hydrocarbon group having 1 to 10 carbon atoms; a combination in which A⁶ is a group that links R³ and a carbon atom of a pyrrole ring in which A⁶ is linked in the form of the carbon atom of a R³-C≡C-pyrrole ring, and R³ is a substituted or unsubstituted alicyclic hydrocarbon group having 3 to 8 carbon atoms; a

Here, an explanation will be given of preferred combinations of R²-A⁵ portion and R³- A⁶ portion in the formula (I).

Preferred combinations of R²-A⁵ portion and R³- A⁶ portion include cases where both of A⁵ and A⁶ represent a single bond. In this case, more preferred combinations include cases where R² represents a cyclopropyl group, a cyclobutyl group, a cyclopropylmethyl group, a methyl group, an ethyl group, a vinyl group, an isopropyl group, an isobutyl group or 2-methyl-1-propenyl group, and R³ represents a pyridyl group or 1-oxypyridyl group or pyrazolyl group or N-methylpyrazolyl group optionally substituted with one alkyl group having 1 to 4 carbon atoms or one halogen atom.

Also combinations wherein both of A⁵ and A⁶ represent a single bond, and R² represents a C₁-C₄ alkyl group, a C₁-C₄ alkoxy group, a C₂-C₄ acyl group, a hydroxy group, a carboxyl group, an alkoxycarbonyl group, a thienyl group, a pyridyl group, a furyl group, a pyrrolyl group, a pyrazolyl group or phenyl group which may be substituted by one or more of a fluorine atom or a chlorine atom, and R³ represents a pyridyl group or 1-oxypyridyl group or pyrazolyl group or N-methylpyrazolyl group which may be substituted by one alkyl group having 1 to 4 carbon atoms . or one halogen atom can be mentioned as preferred examples.

Further, an explanation will be given of preferred combinations of A¹-G² portion, R²- A⁵ portion and R³- A⁶ portion in the formula (I). Basically, preferably those mentioned as preferred examples for A¹-G² portion, R²- A⁵ portion and R³- A⁶ portion are combined, and more preferably more preferred examples are combined.

More specifically, in the combinations of the following a) to f) mentioned as preferred combinations of the A¹-G² portion, further a case where both of A⁵ and A⁶ represent a single bond is preferred.
a) A¹ represents -(CH₂)₂- or -(CH₂)₃-, A² represents -NH-(C=O)- or -NH-(C=O)-NH-, G¹ represents a single bond, and A³ represents a divalent acyclic aliphatic hydrocarbon group having 1 to 10 carbon atoms.
b) A¹ represents -(CH₂)₂- or -(CH₂)₃-, A² represents -NH-(C=O)-, -NH-(C=O)-NH-, -NH-, or -C-(=O)-NH-, and G¹ represents a group other than the single bond.
c) A¹ is -(CH₂)₂- or -(CH₂)₃-, A² represents a single bond, and G¹ represents an optionally substituted heterocyclic group (note, where a heterocyclic group of G¹ is 5 or 6 membered monocyclic ring, 5 or 6 membered monocyclic heterocyclic group of G¹ is substituted, or A³-G² portion represents those other than a hydrogen atom).
d) A¹ is -(CH₂)₂- or -(CH₂)₃-, A² represents those other than a single bond, and G¹ represents an optionally substituted aromatic hydrocarbon group, an optionally substituted alicyclic hydrocarbon group having 7 to 10 carbon atoms, or an optionally substituted heterocyclic group (note, where the aromatic hydrocarbon group of G¹ is a phenyl group, or where the heterocyclic group of G¹ is 5 or 6 membered monocyclic ring, the phenyl group of G¹ or 5 or 6 membered monocyclic heterocyclic group is substituted, or A³-G² portion represents those other than a hydrogen atom).
e) A¹ is -(CH₂)₂- or -(CH₂)₃-, A² represents those other than a single bond, G¹ and A⁴ represent the single bond, A³ represents an optionally substituted acyclic aliphatic hydrocarbon group having 1 to 10 carbon atoms, G² represents an optionally substituted alicyclic hydrocarbon group having 5 to 10 carbon atoms, an optionally substituted aromatic hydrocarbon group, or optionally substituted heterocyclic group.
f) A¹ is -(CH₂)₂- or -(CH₂)₃-, A² represents those other than a single bond, G¹ represents the single bond, A³ represents an optionally substituted acyclic aliphatic hydrocarbon group having 1 to 10 carbon atoms, and A⁴ represents -C(=O)-, -C(=O)-NR¹²¹-, -C(=S)-NR¹²²-,-C(=NR¹²³)-, -O-C(=O)-, -NR¹²⁵-C(=O)-, -NR¹²⁶-S(=O)₂-, -NR¹²⁷-C(=O)-O-, -NR¹²⁸-C(=O)-NR¹²⁹-, -NR¹³⁰-C(=S)-, -NR¹³¹-C(=S)-NR¹³²-, -S-, -S(=O)-, -S(=O)₂-, -S(=O)₂-NR¹³³- or -S(=O)₂-O-.

In the cases of d) to f), A² preferably represents-C(=O)-, -C(=O)-O-, -C(=O)-NH-, -C(=O)-NMe-, -NH-, -NH-C(=O)-, -NH-C(=O)-O-, -NH-C(=O)-NH-, -NH-C(=O)-NMe-, or - NH-C(=S)-, especially preferably represents -C(=O)-NH-,-NH-, -NH-C(=O)-, -NH-C(=O)-O-, or -NH-C(=O)-NH-.

In these cases of combinations, further preferably R² represents an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms or an optionally substituted heterocyclic group, and R³ represents an optionally substituted aromatic hydrocarbon group having 6 to 14 carbon atoms or an optionally substituted heterocyclic group.

In further detail, in these cases, combinations wherein R² represents an aliphatic hydrocarbon group having 1 to 10 carbon atoms, an alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom and a sulfur atom, in the ring, and R³ represents a thienyl group, a pyridyl group, a furyl group, a pyrrolyl group, a pyrazolyl group or a phenyl group optionally substituted with one or more alkyl group having 1 to 4 carbon atoms are specifically preferred. Especially, preferred combinations can include cases where R² represents a cyclopropyl group, a methyl group, an ethyl group, a vinyl group, an isopropyl group, an isobutyl group or 2-methyl-1-propenyl group, and R³ represents a pyridyl group or 1-oxypyridyl group or pyrazolyl group or N-methylpyrazolyl group which may be substituted by an alkyl group having 1 to 4 carbon atoms or one halogen atom, and cases where R² represents a thienyl group, a pyridyl group, a furyl group, a pyrrolyl group, a pyrazolyl group or phenyl group which may be substituted by one or more of a C₁-C₄ alkyl group, a C₁-C₄ alkoxy group, and a chlorine group, and R³ represents a pyridyl group or 1-oxypyridyl group or pyrazolyl group or N-methylpyrazolyl group which may be substituted by an alkyl group having 1 to 4 carbon atoms or one halogen atom.

In the pyrrolo-pyrimidinone derivatives of the formula (I), specific preferred combinations of -G¹-A³-A⁴-G² portion include groups represented by the following formulae, K001-K431. In the respective chemical formula, symbol "---" is used to denote a binding site between A² and the group -G¹-A³-A⁴-G².

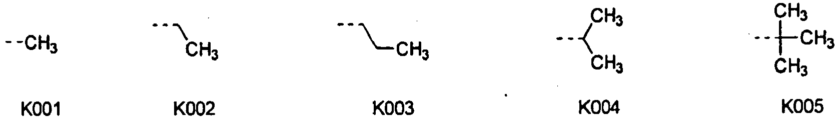

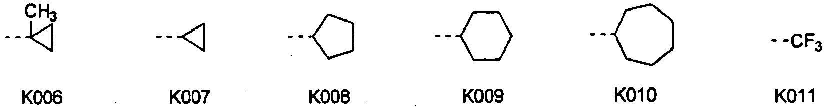

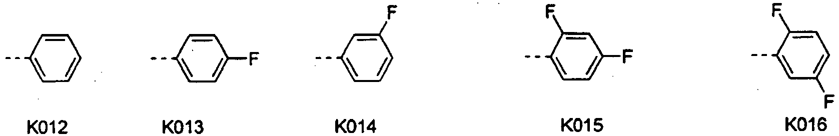

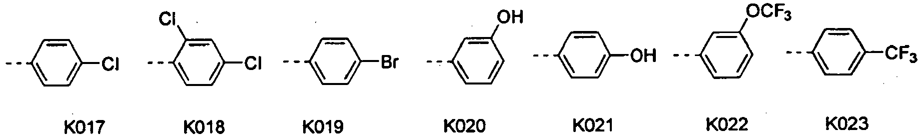

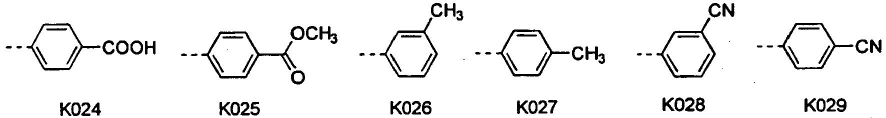

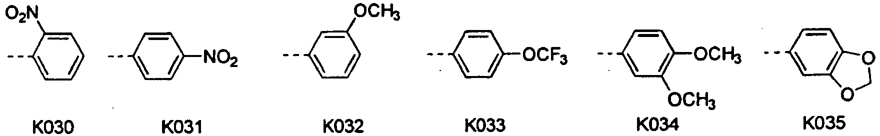

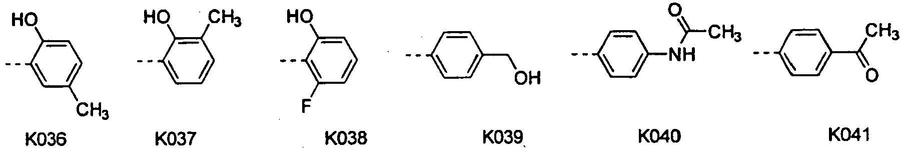

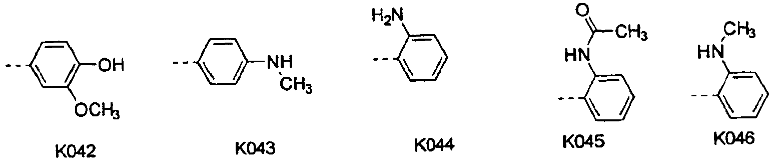

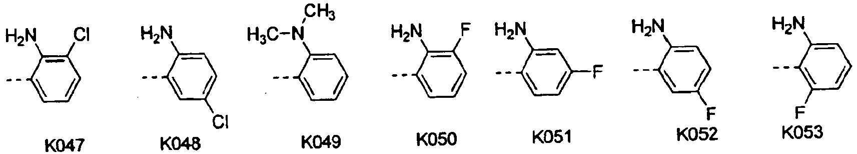

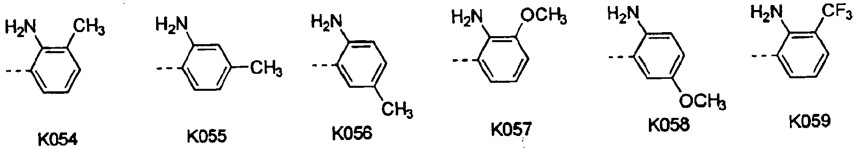

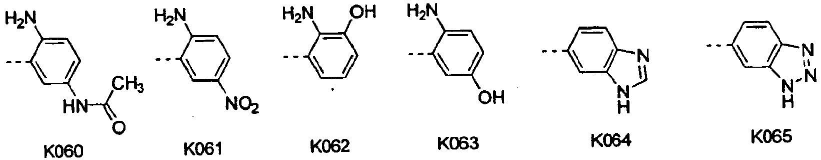

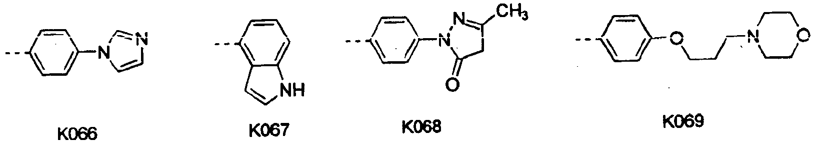

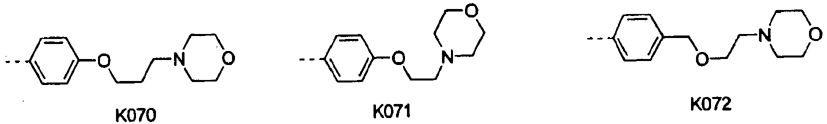

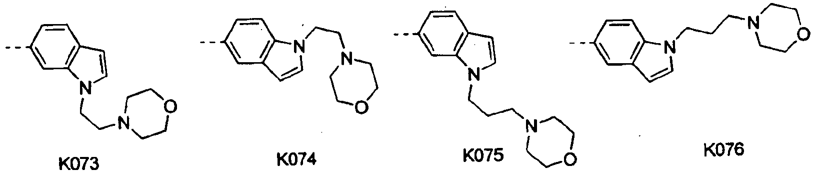

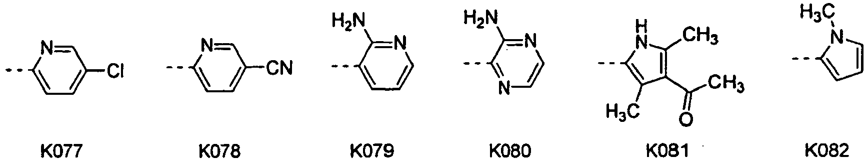

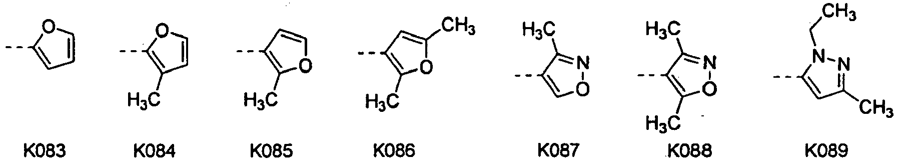

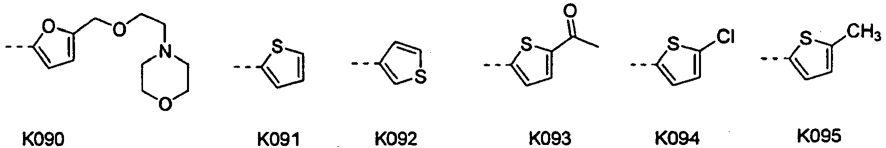

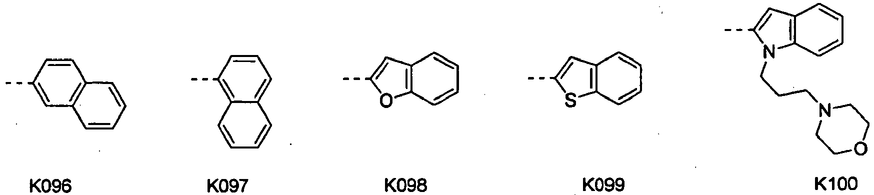

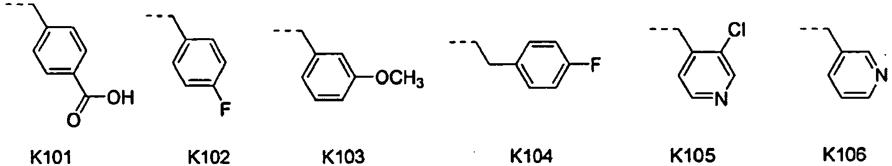

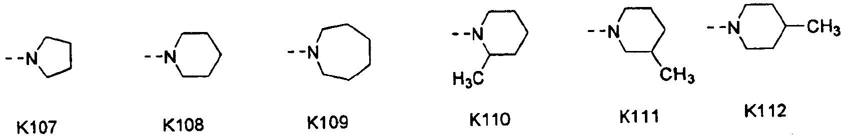

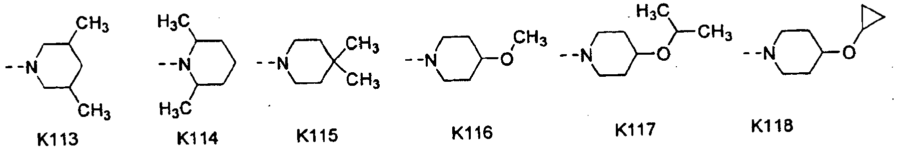

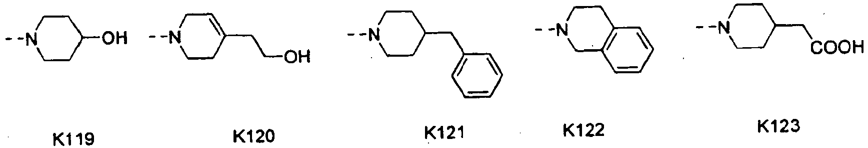

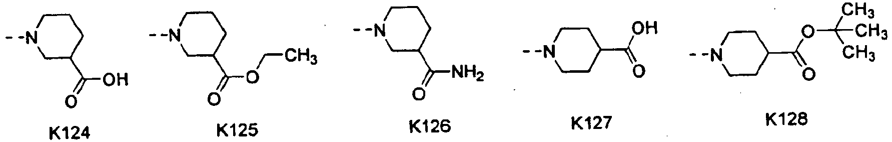

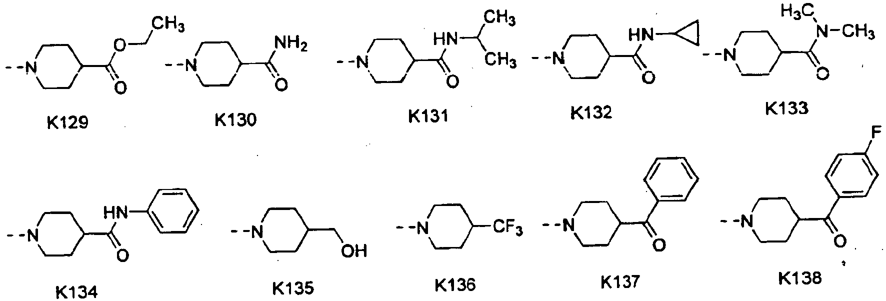

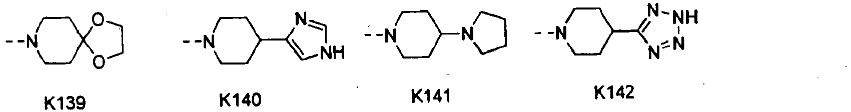

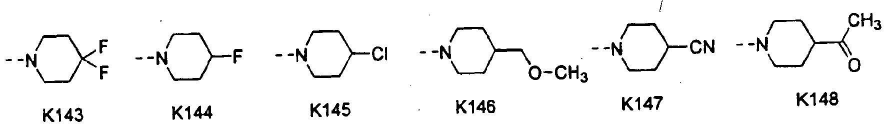

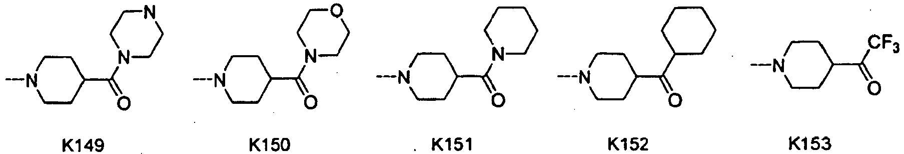

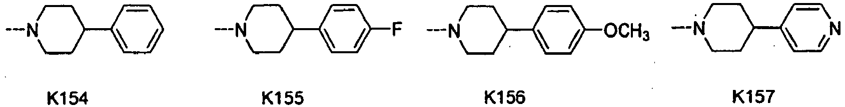

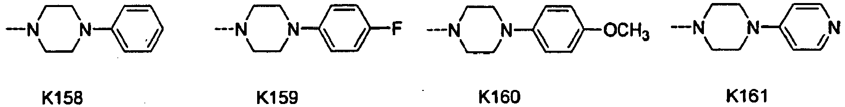

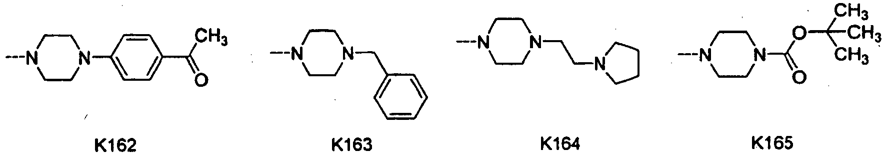

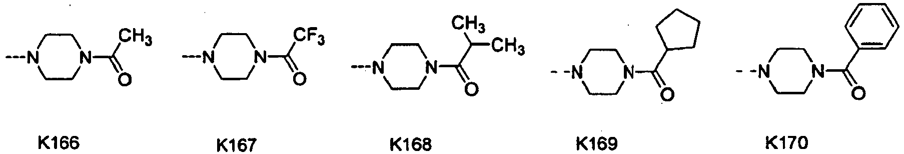

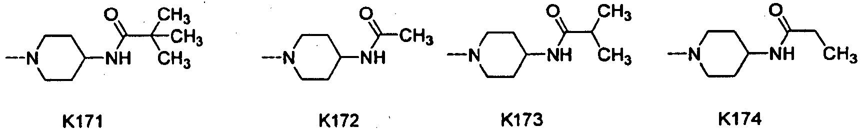

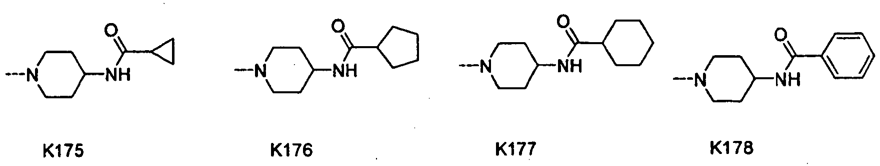

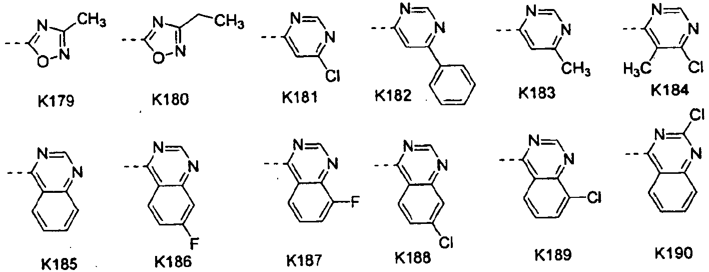

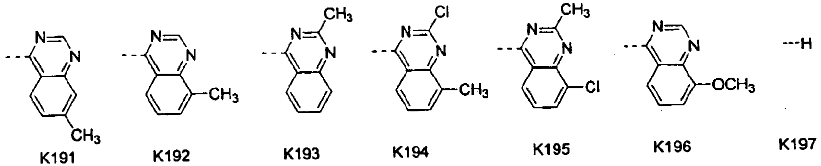

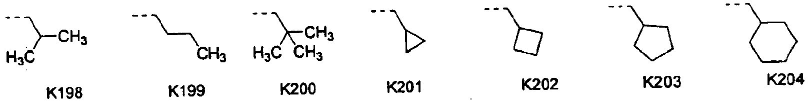

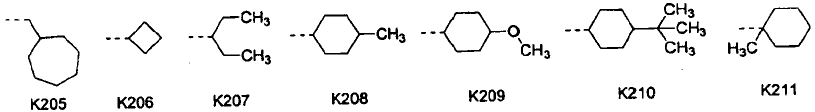

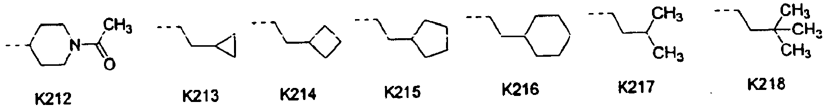

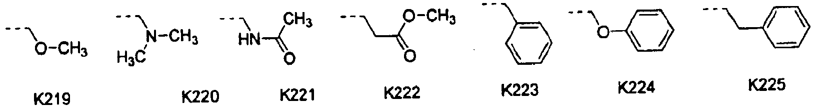

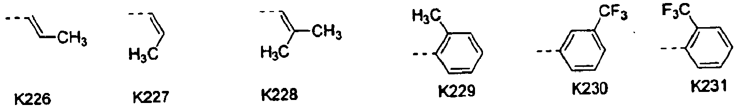

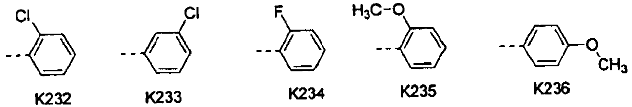

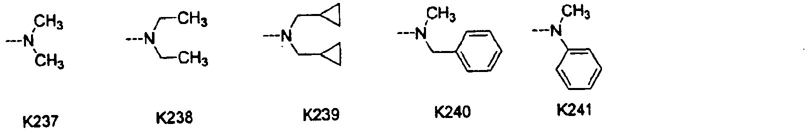

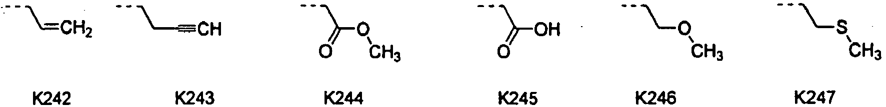

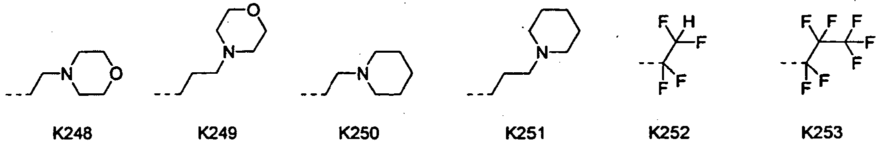

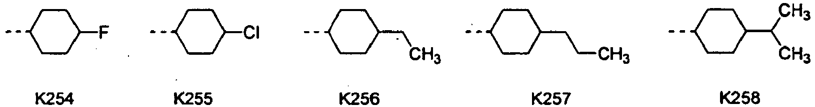

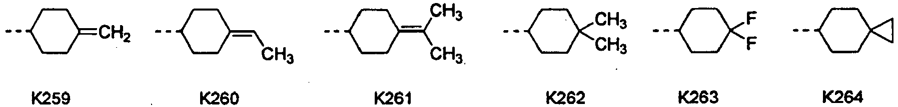

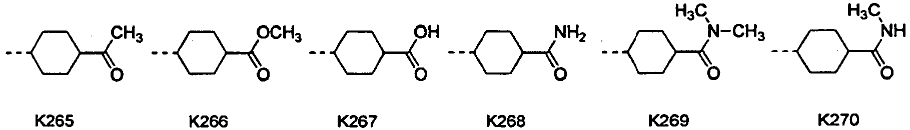

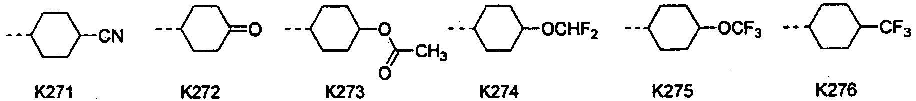

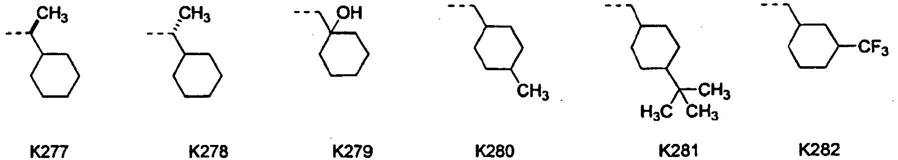

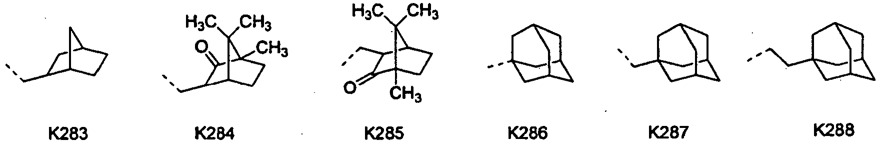

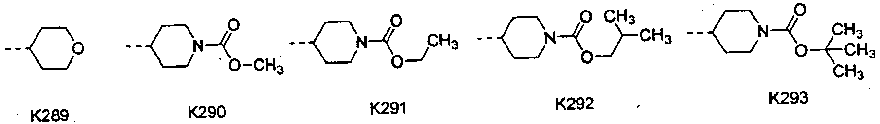

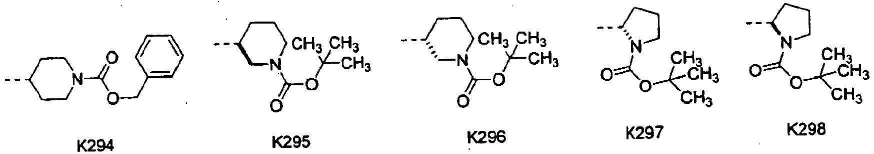

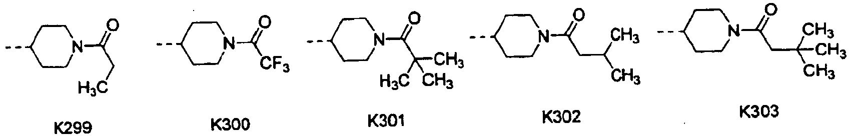

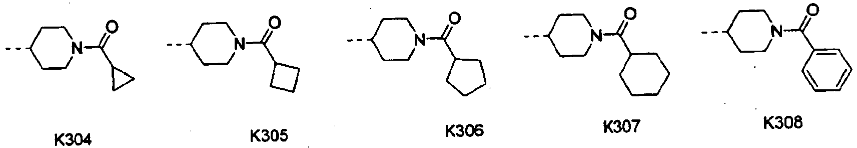

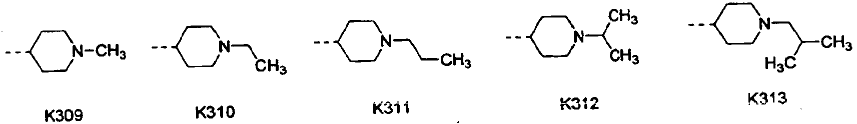

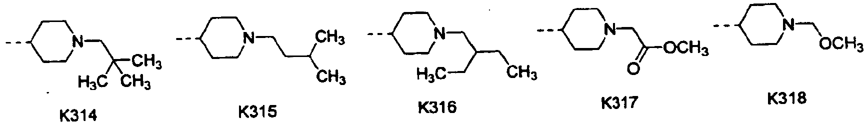

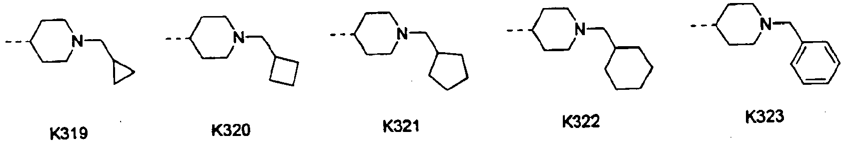

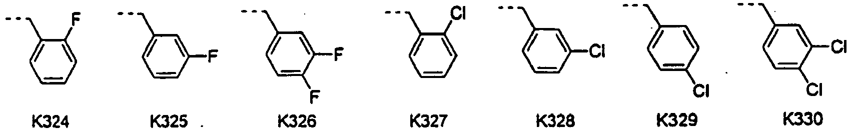

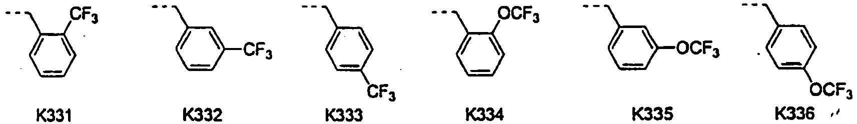

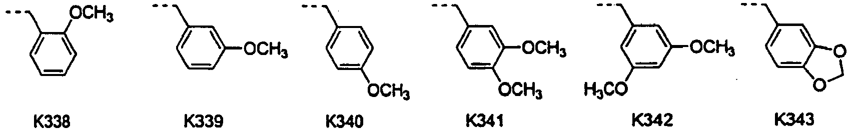

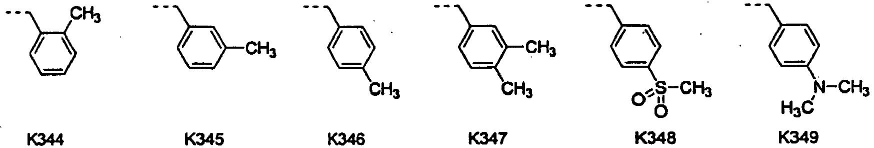

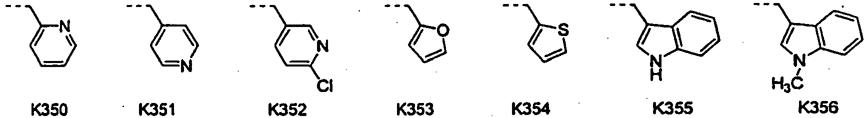

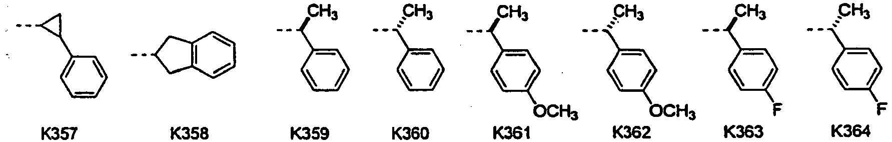

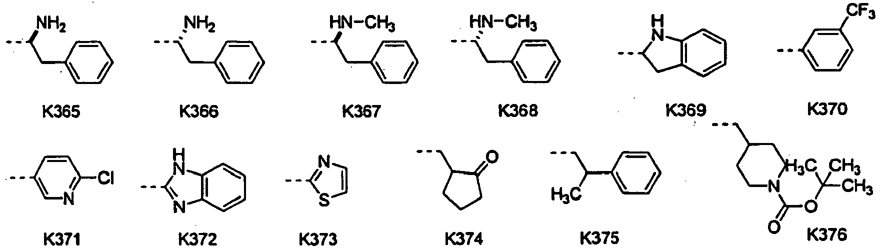

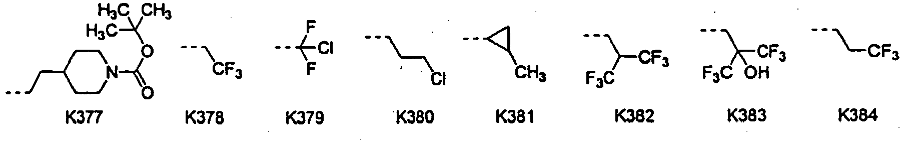

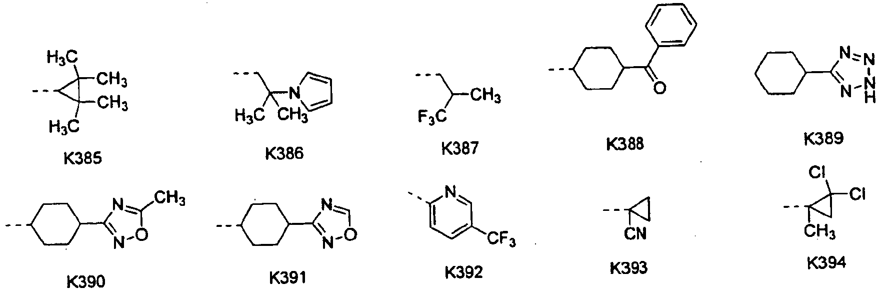

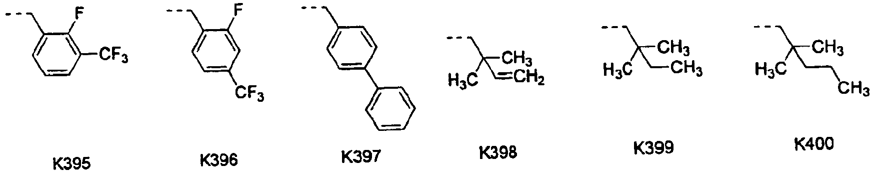

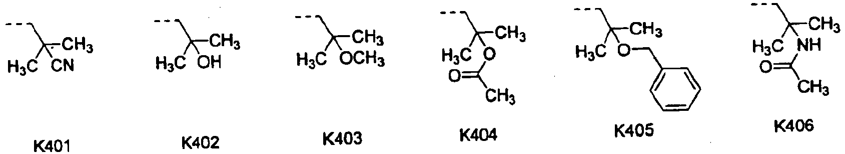

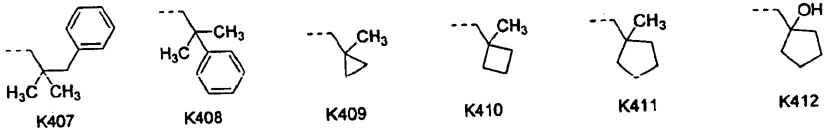

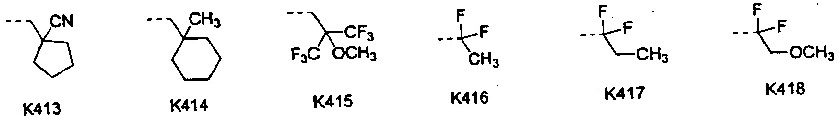

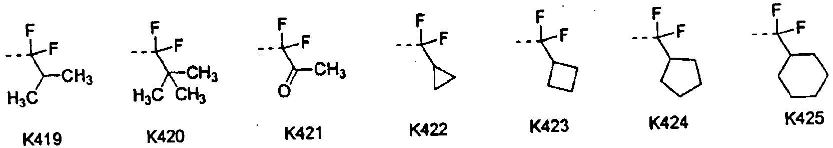

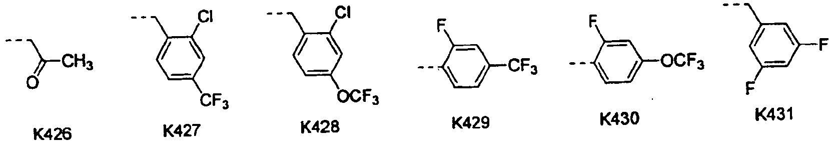

In the pyrrolo-pyrimidinone derivatives of the formula (I), as specific examples of preferred combinations of the -A⁵-R² portion, groups represented by the following formulae, J001-J166 may be mentioned. In the respective chemical formulae, symbol "---" indicates a binding site between a carbon atom of a pyrrole ring and -A⁵-R².

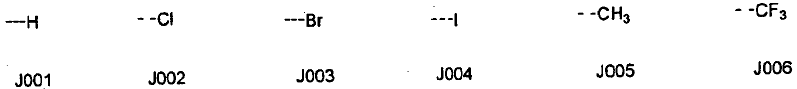

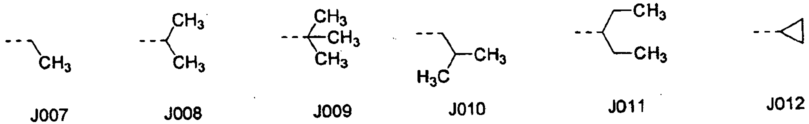

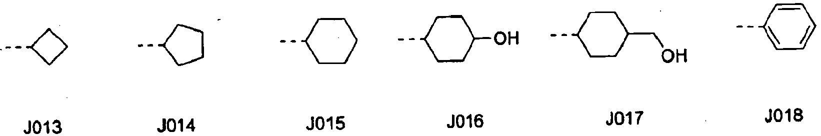

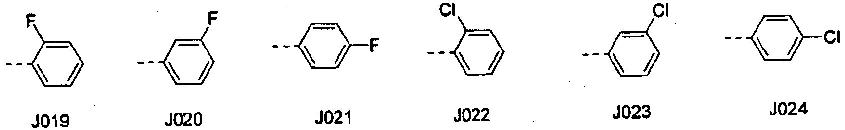

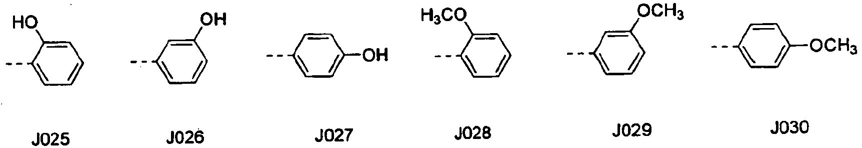

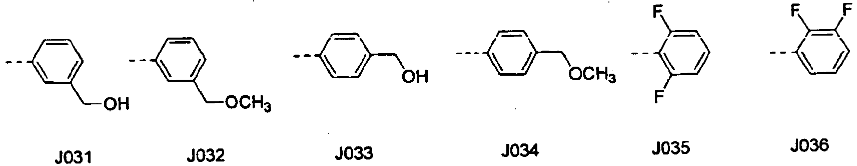

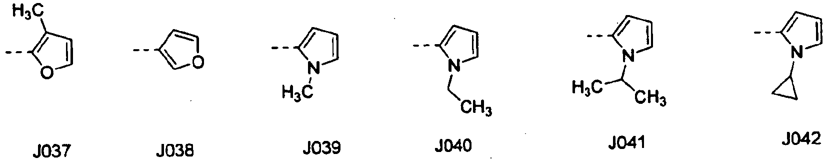

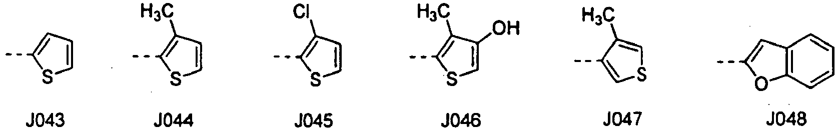

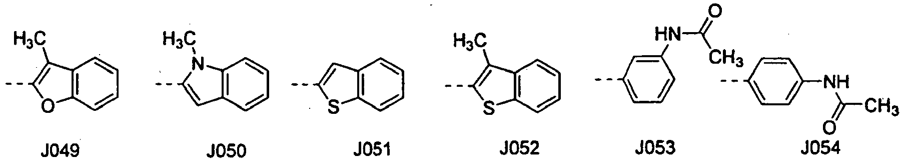

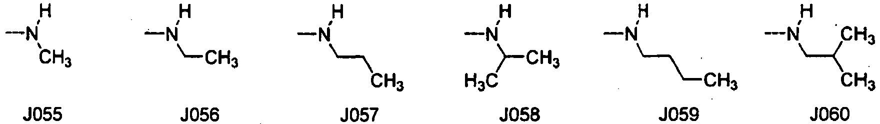

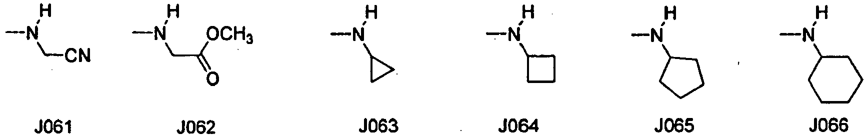

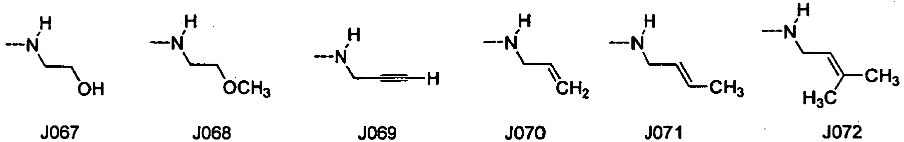

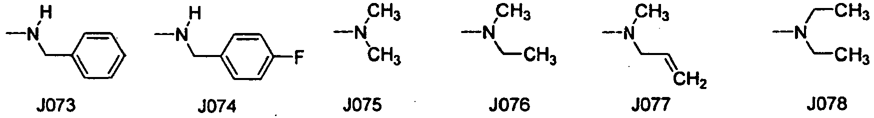

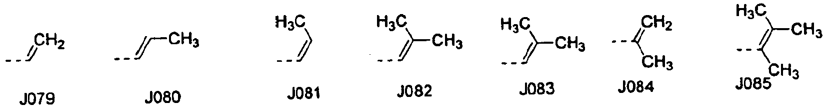

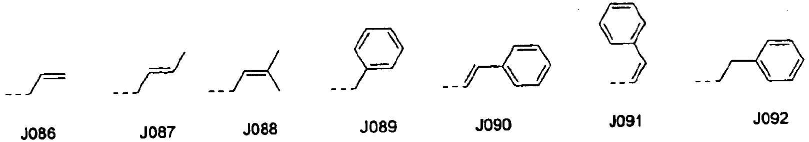

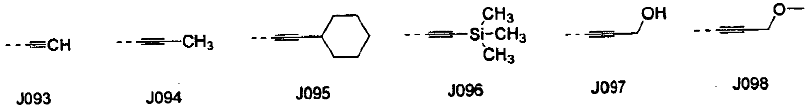

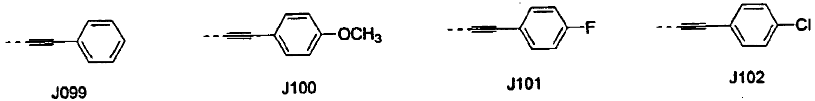

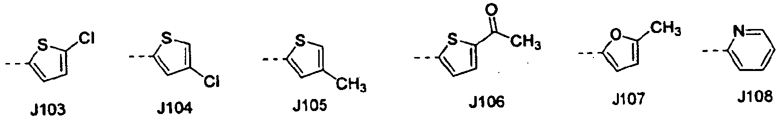

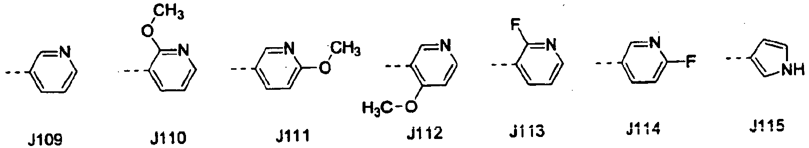

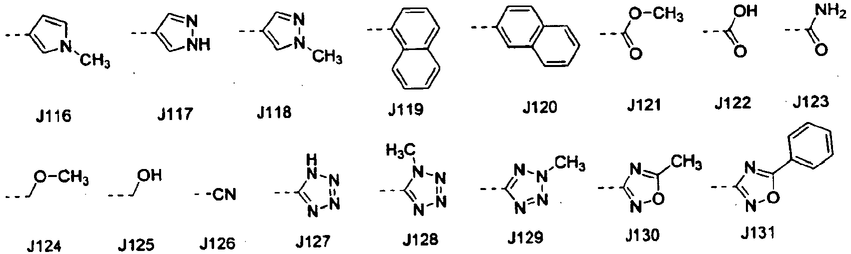

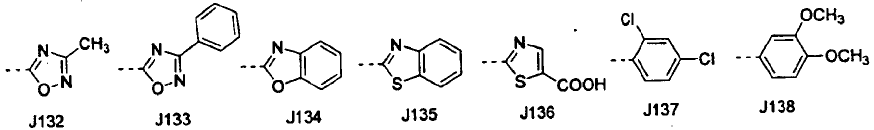

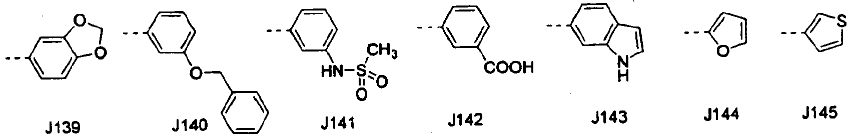

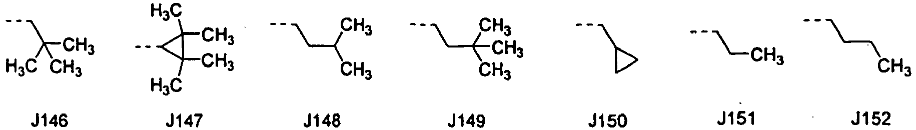

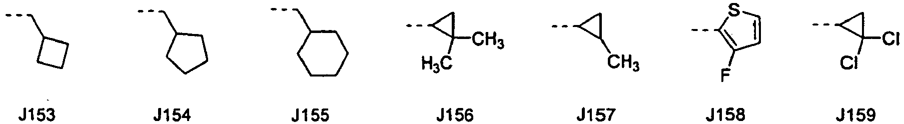

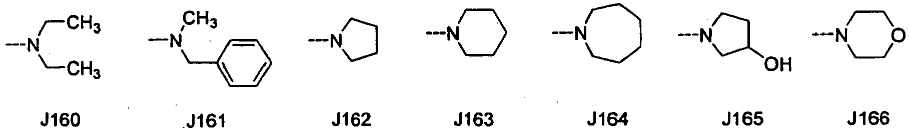

In the pyrrolo-pyrimidinone derivatives of the formula (I), as specific examples of preferred combinations of the -A⁶-R³ portion, groups represented by the following formulae, T001-T181 may be mentioned. In the respective chemical formulae, symbol "---" indicates a binding site between a carbon atom of a pyrrole ring and -A⁶-R³.

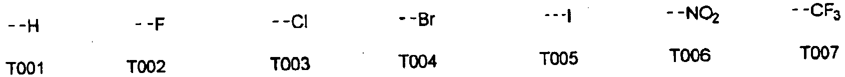

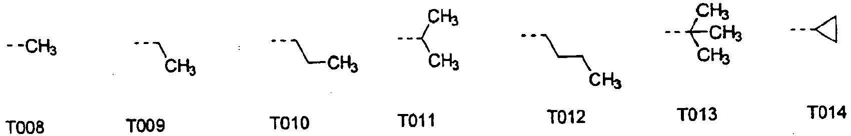

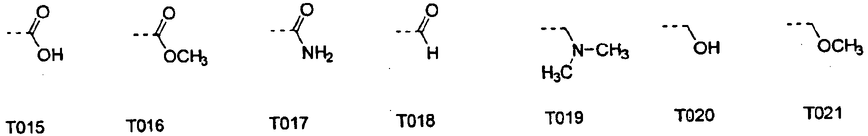

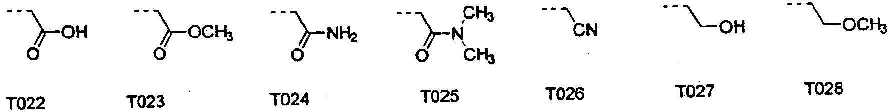

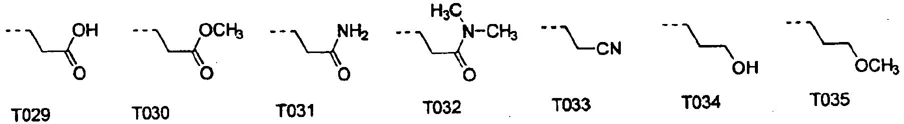

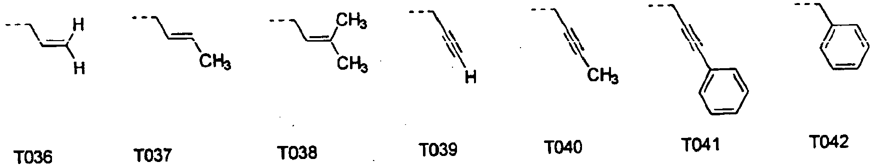

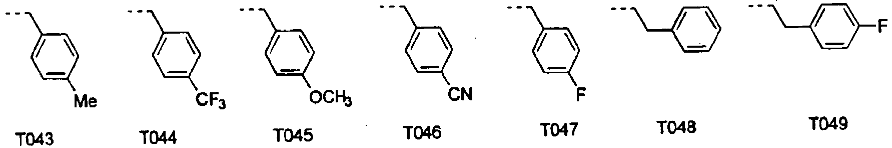

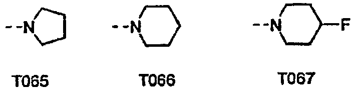

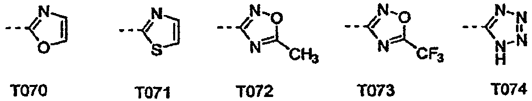

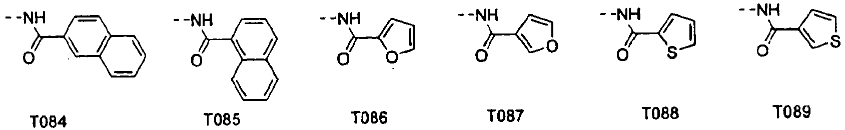

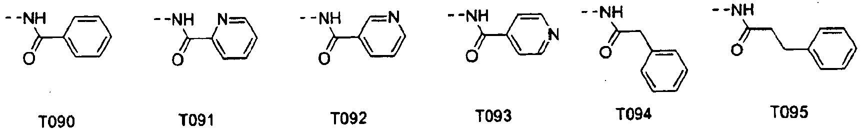

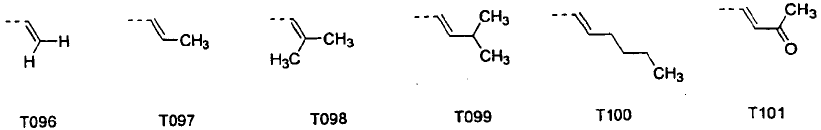

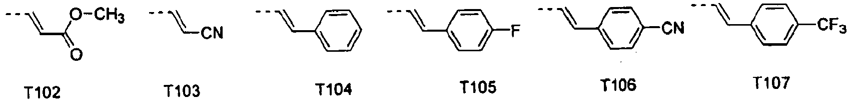

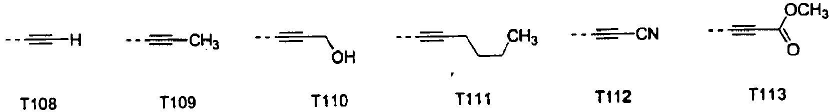

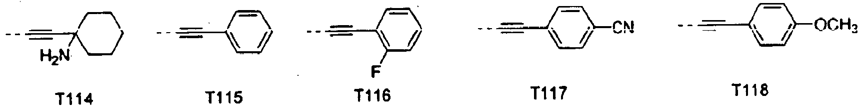

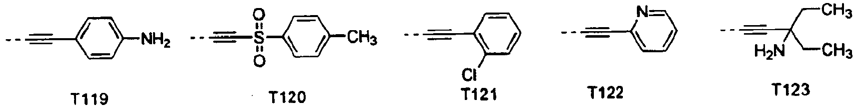

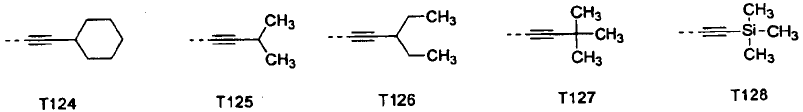

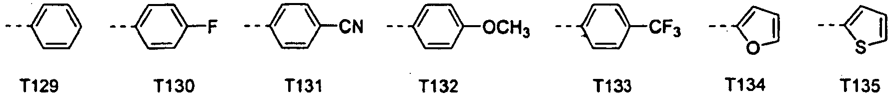

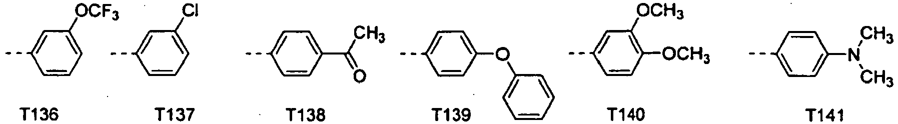

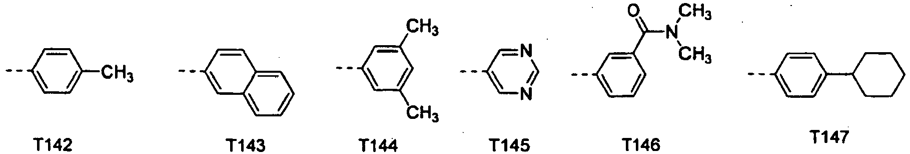

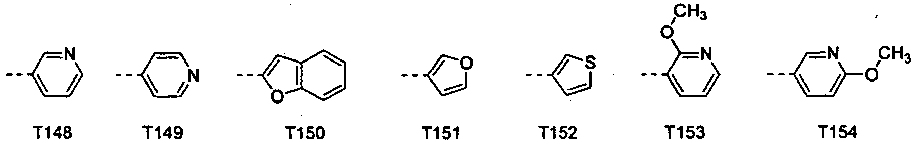

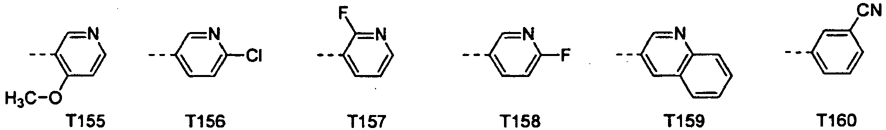

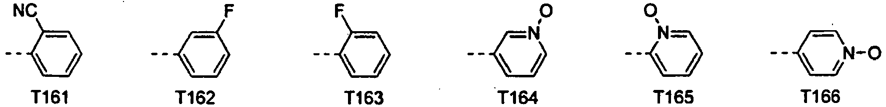

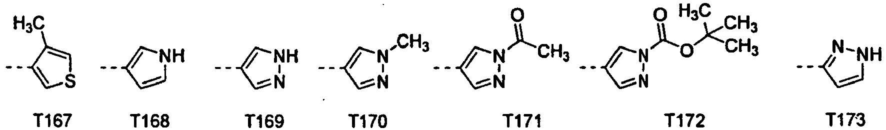

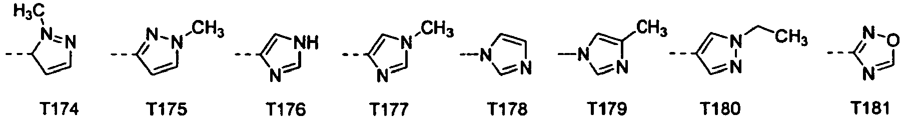

Specific examples of the pyrrolo-pyrimidinone derivatives of formula (I) include the compounds having groups described in the following Table 1 as A¹, the compounds having groups described in the following Table 1 as A², the compounds having groups represented by K001-K431 indicated in the formula as -G¹-A³-A⁴-G², the compounds having groups represented by J01-J166 indicated in the formula as -A⁵-R², the compounds having groups represented by T001-T181 indicated in the formula as -A⁶-R³, and the compounds consisting of any combination of groups mentioned above with regard to each moiety. Preferable examples among such compounds are listed in Tables below.

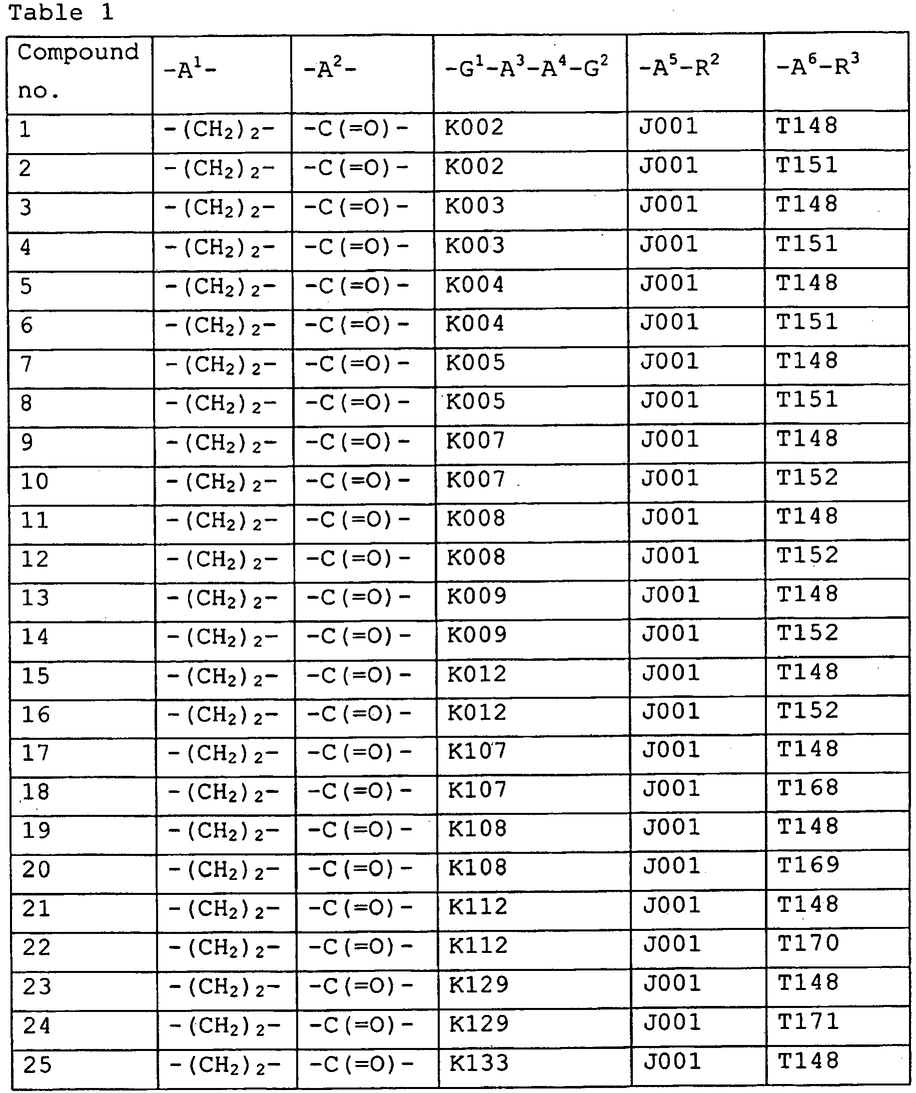

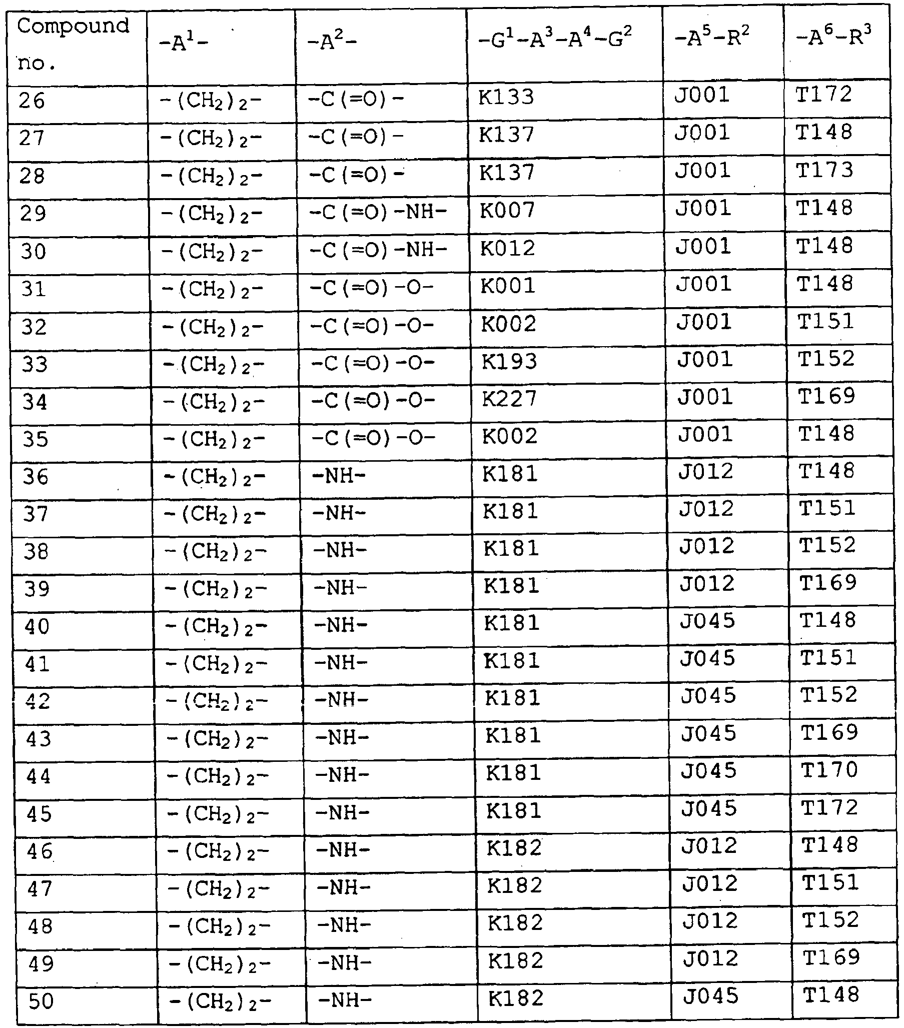

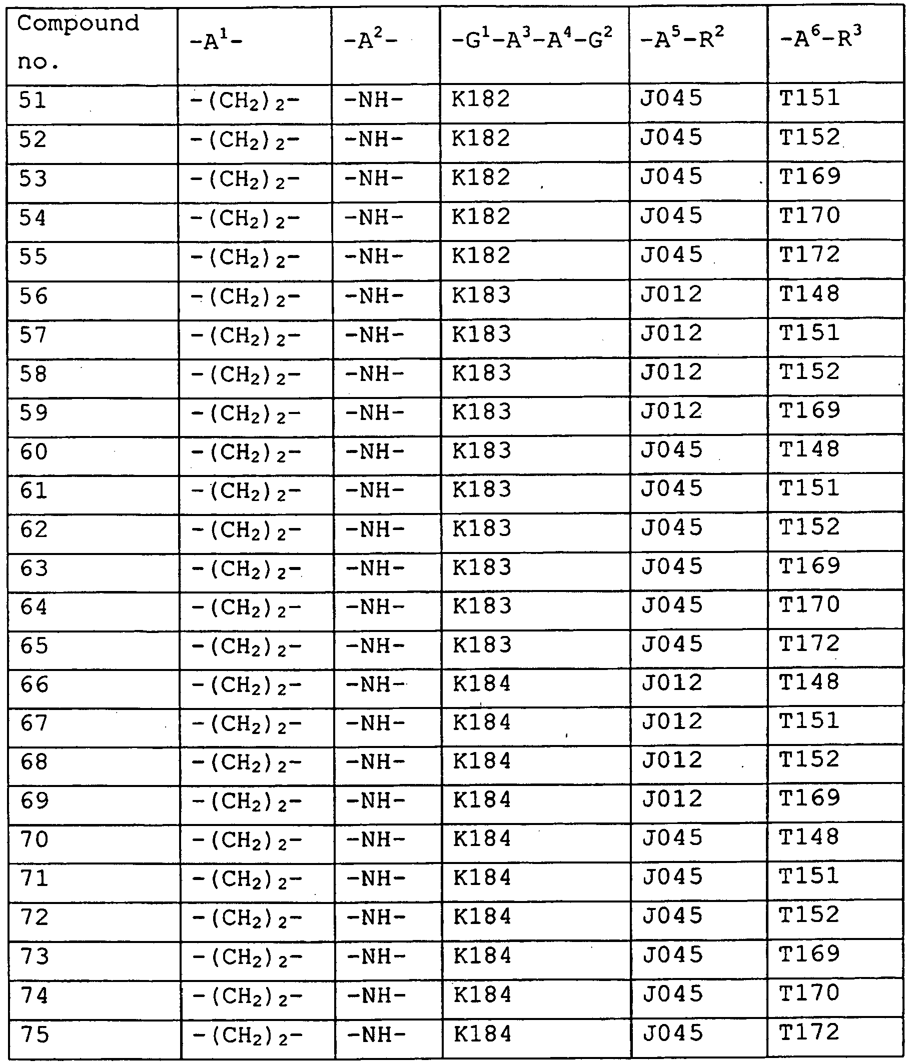

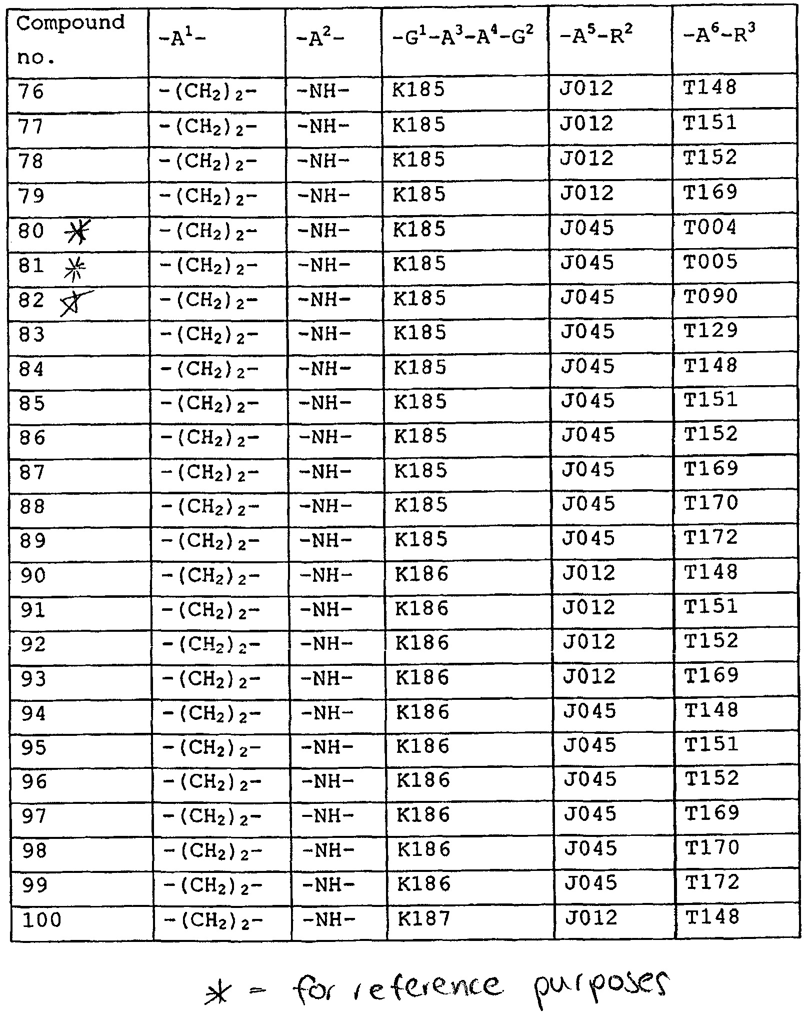

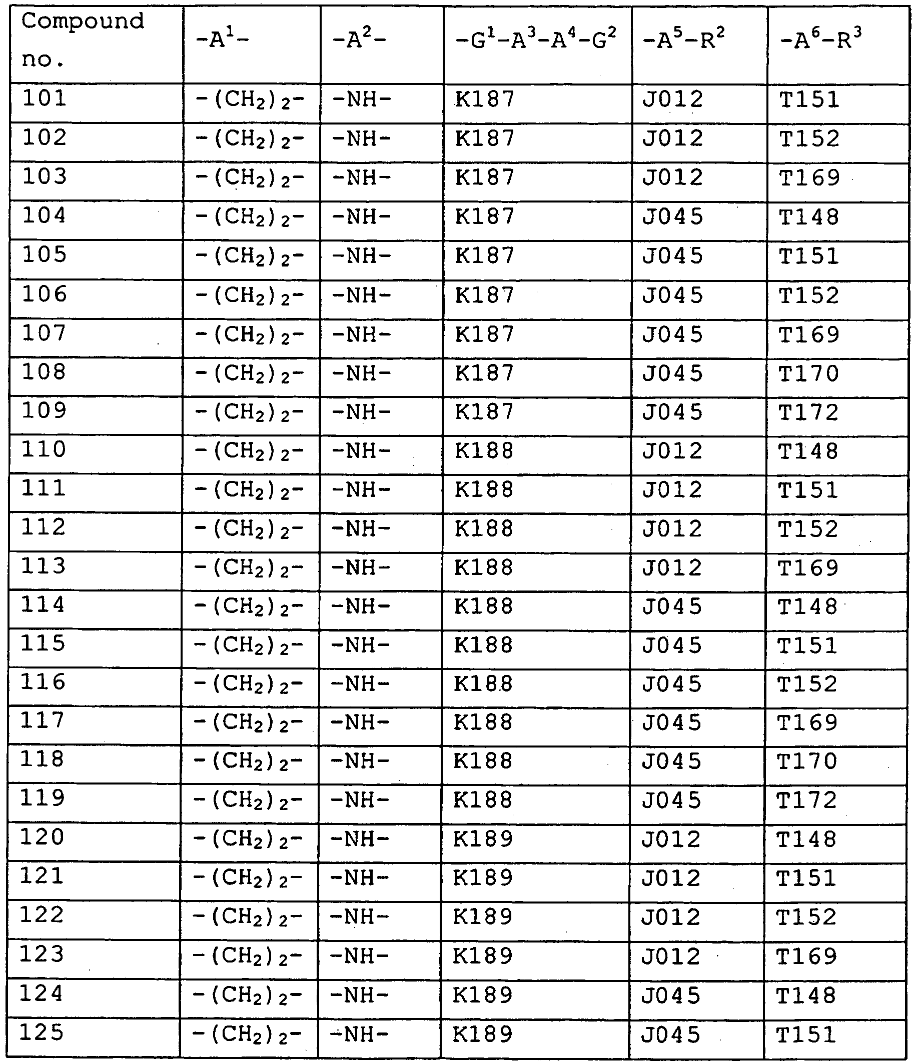

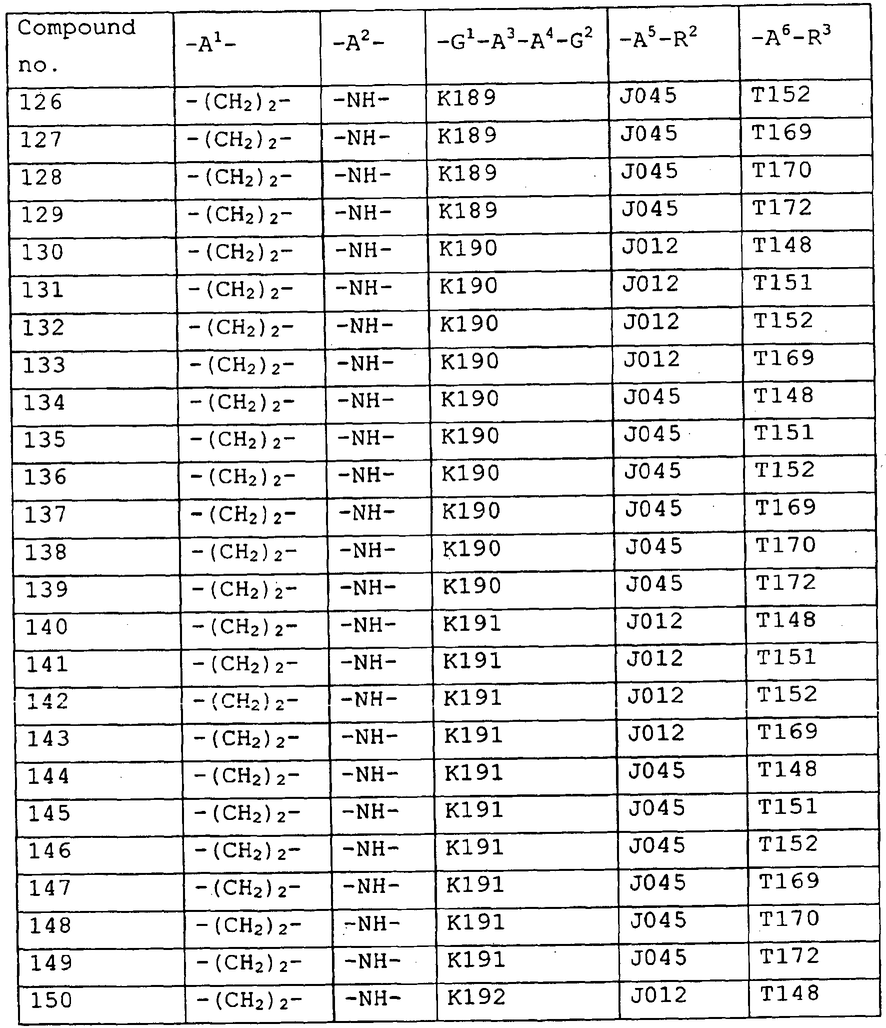

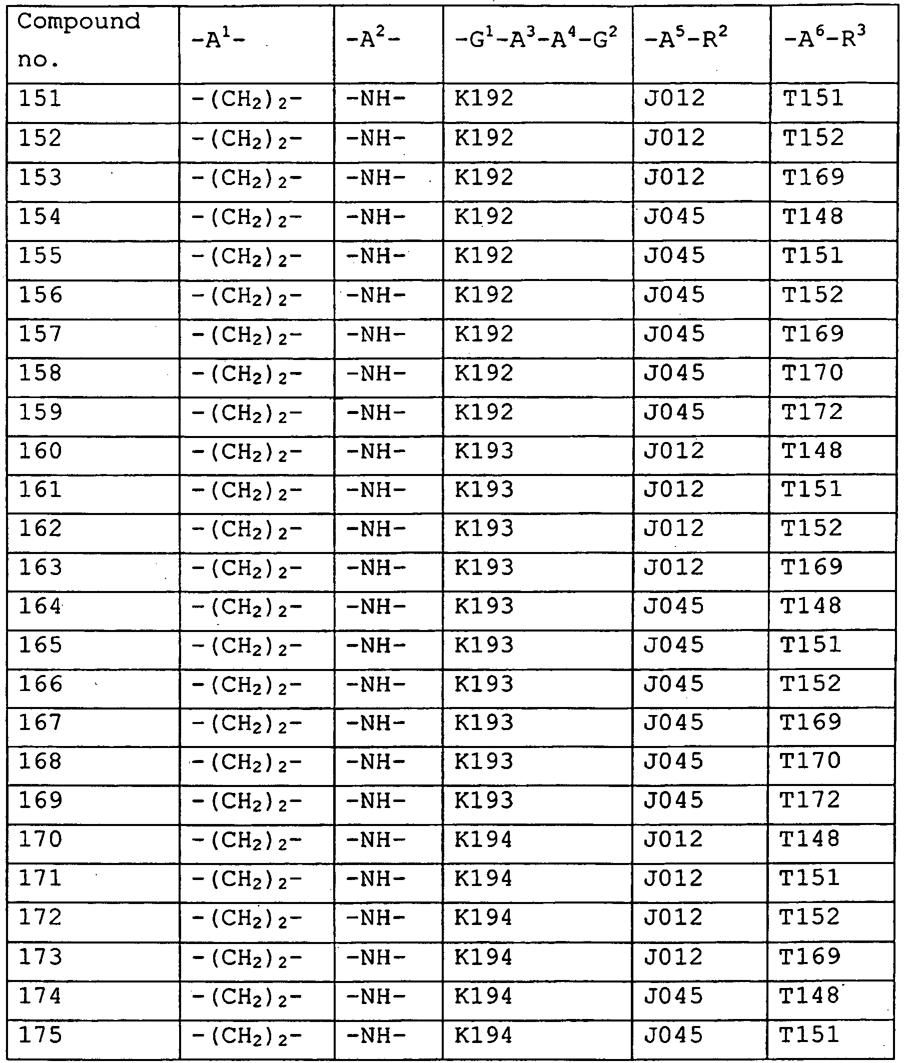

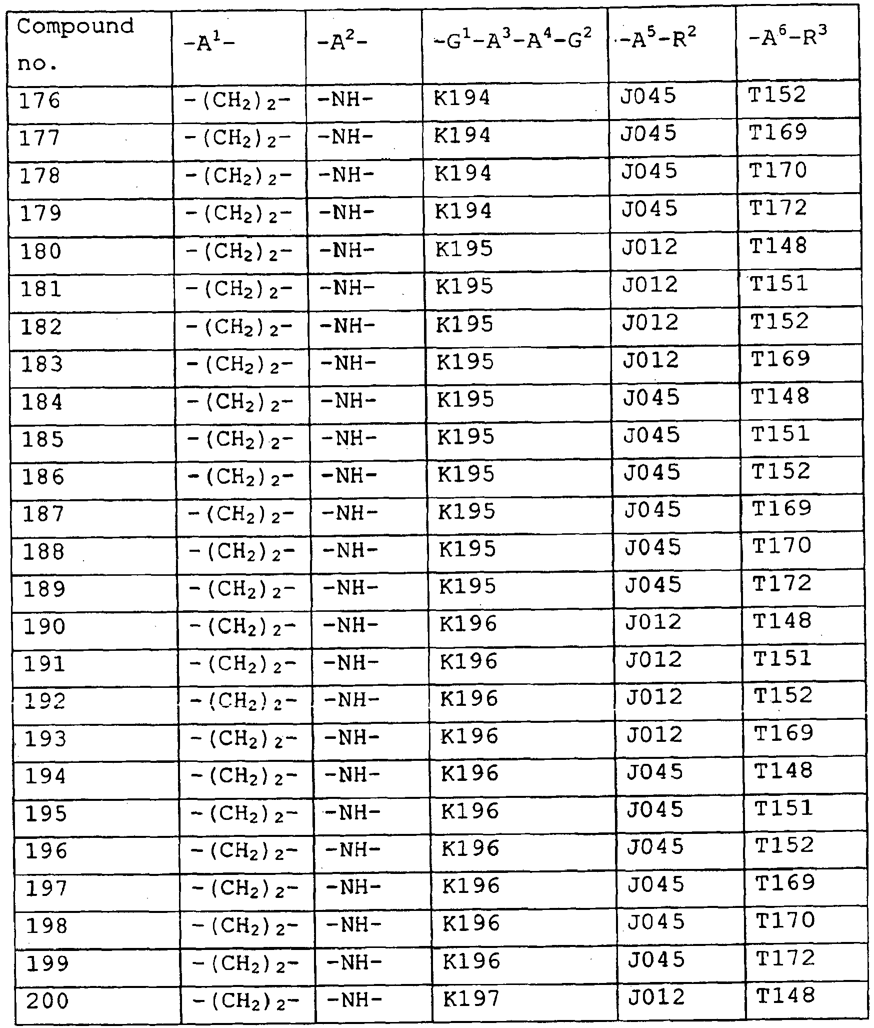

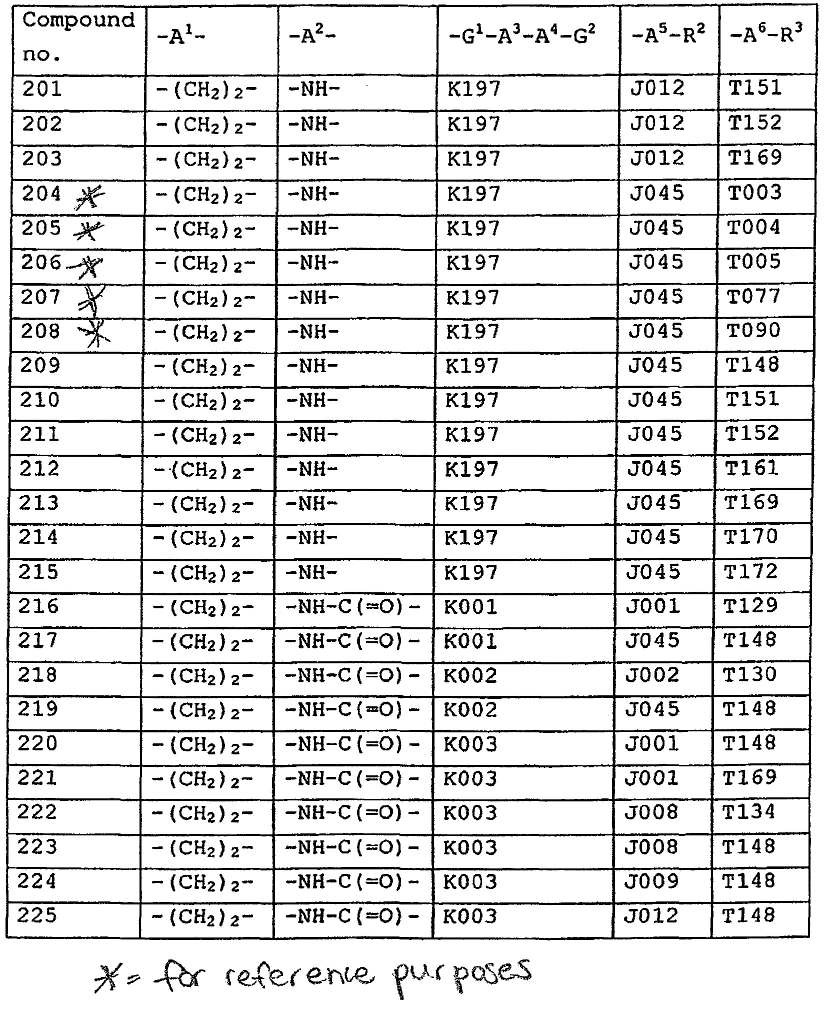

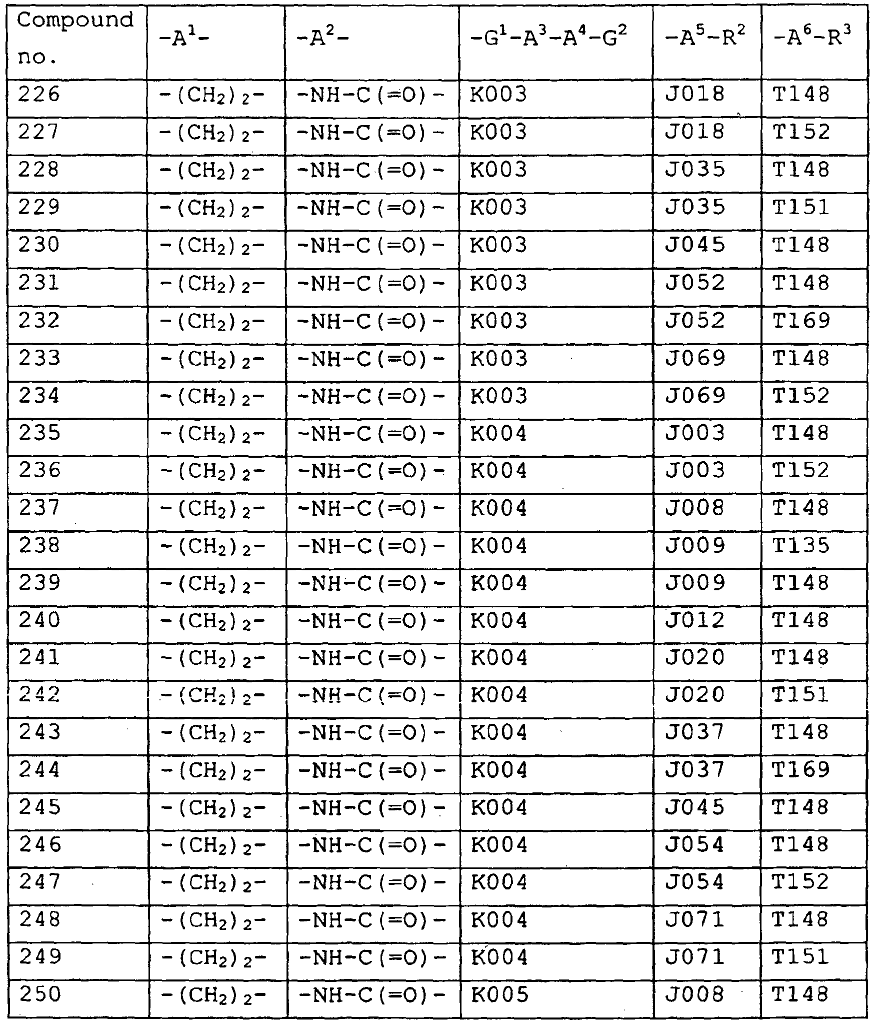

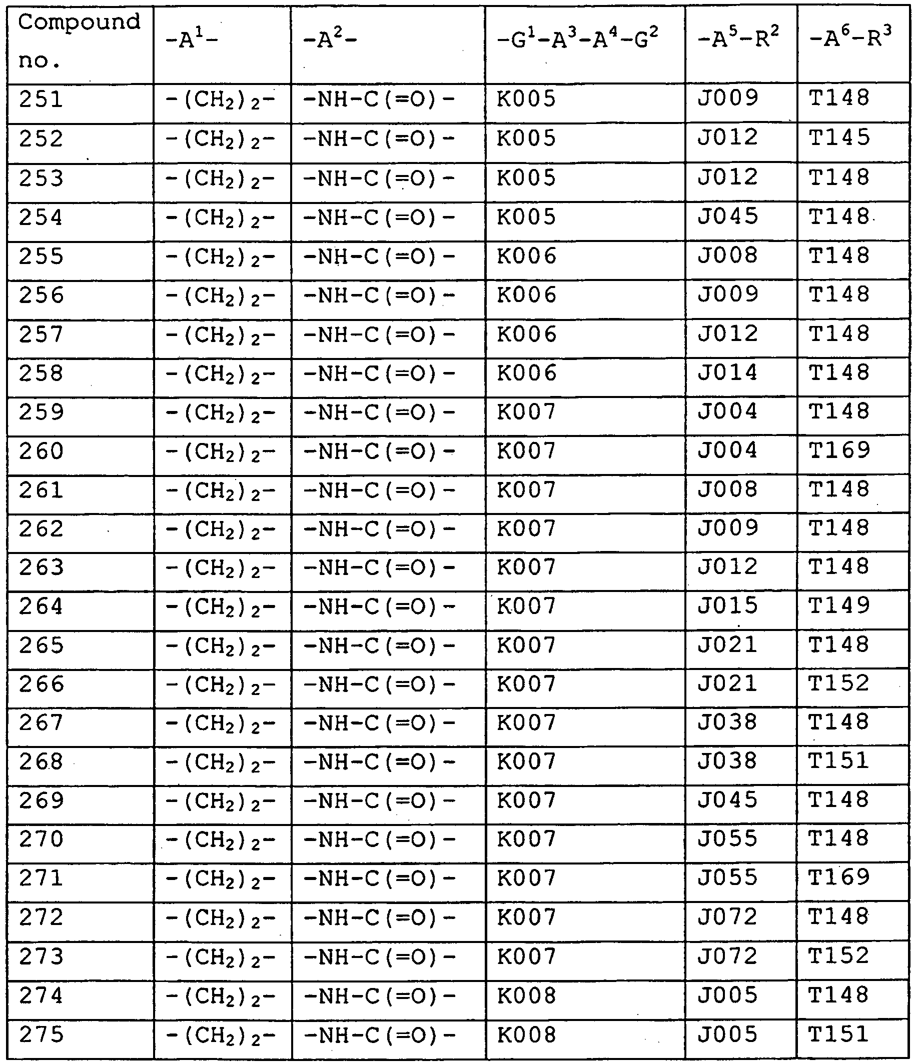

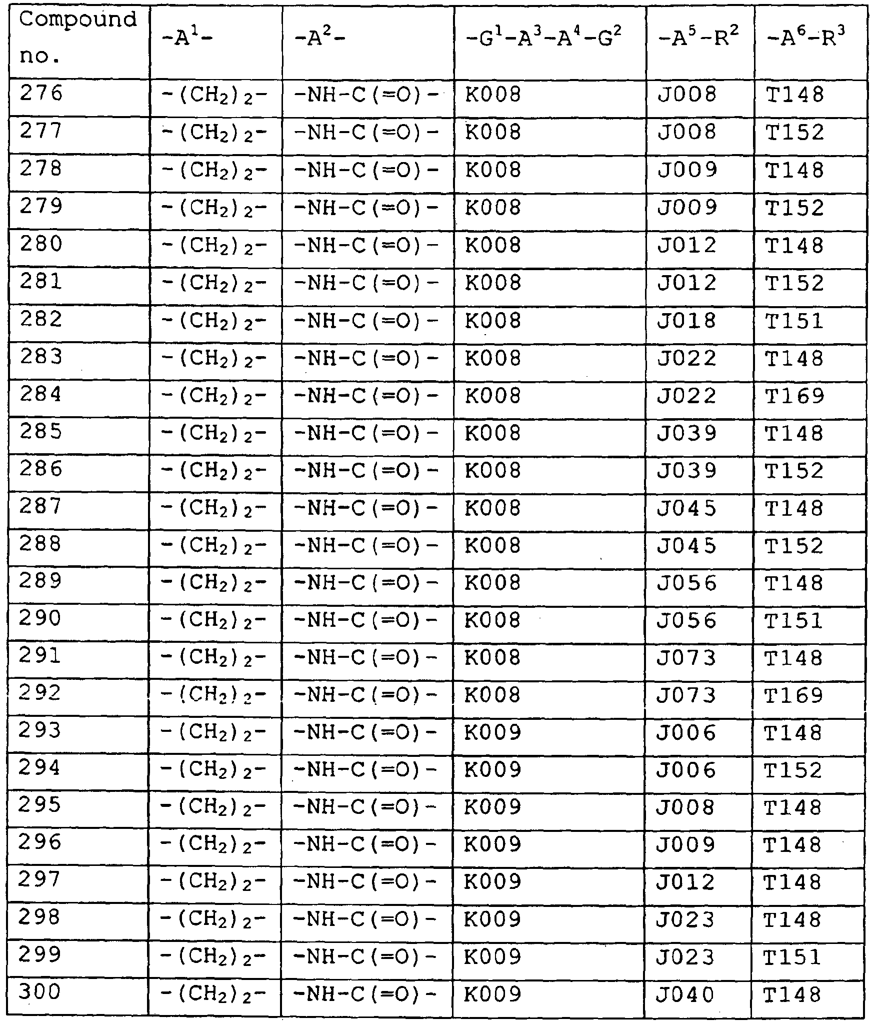

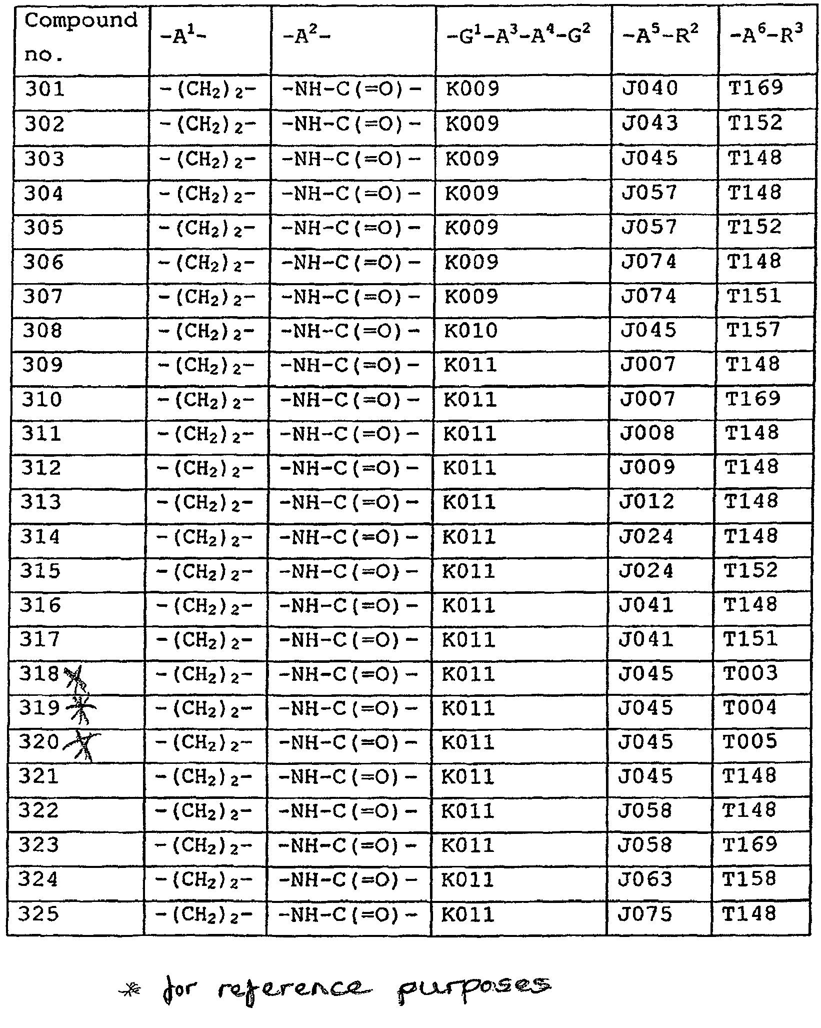

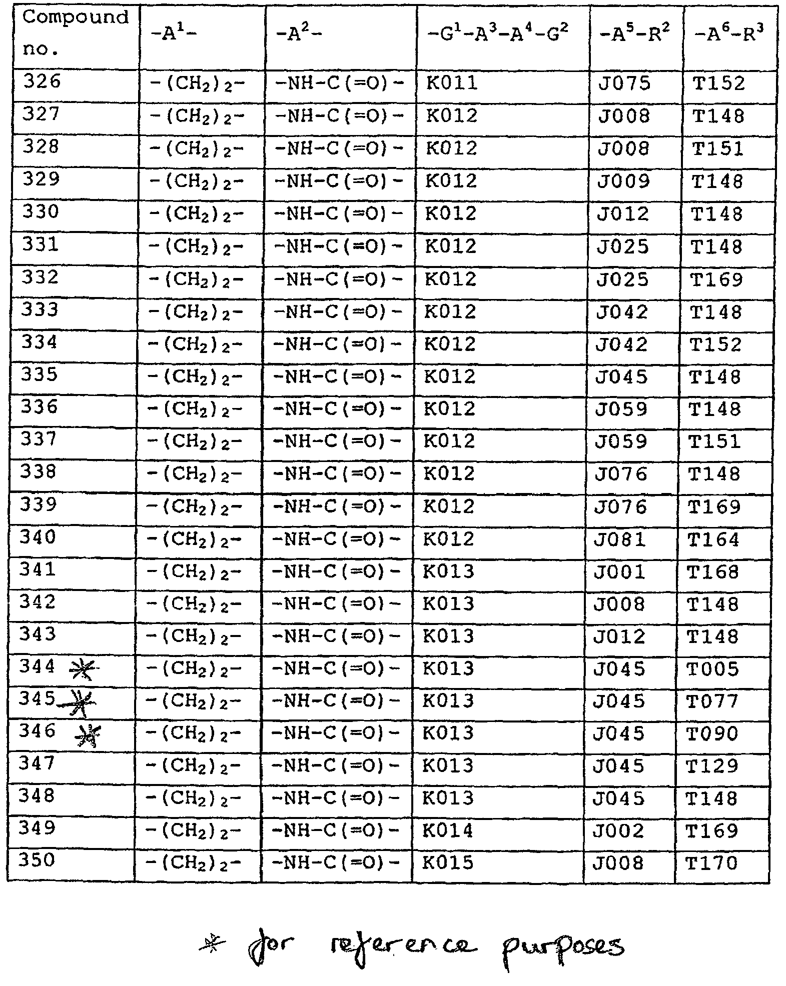

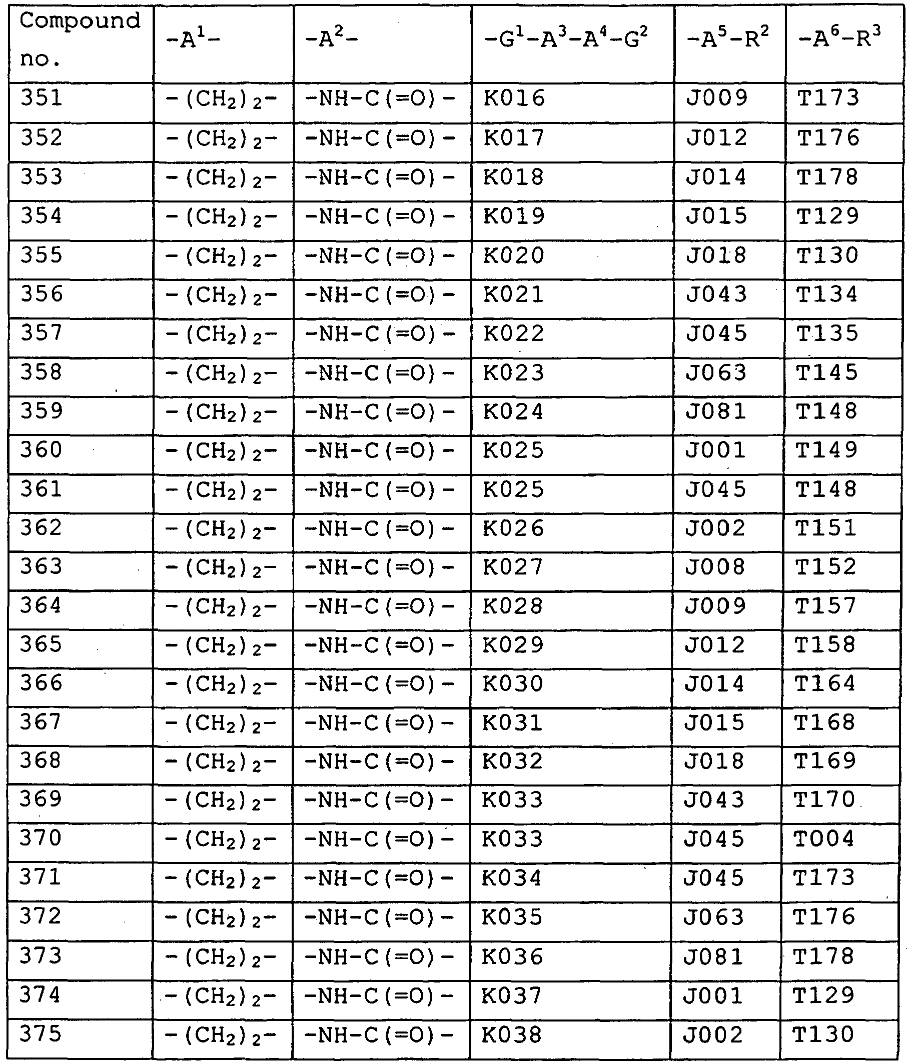

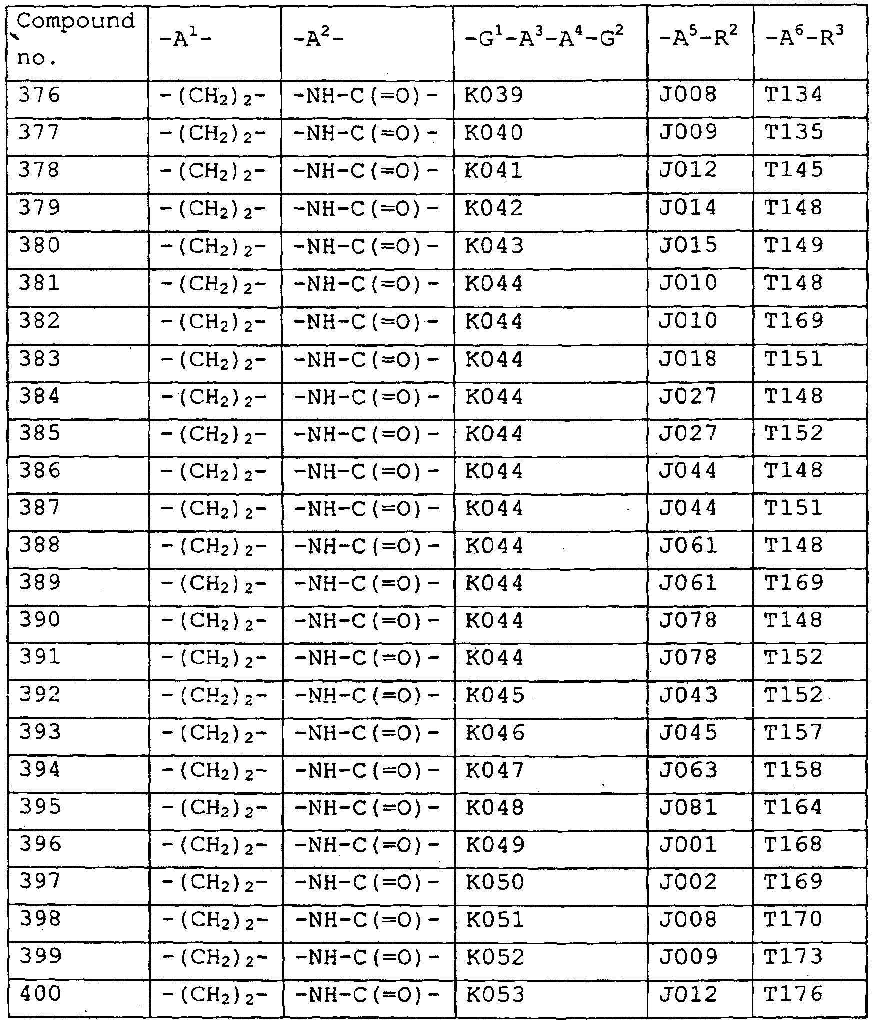

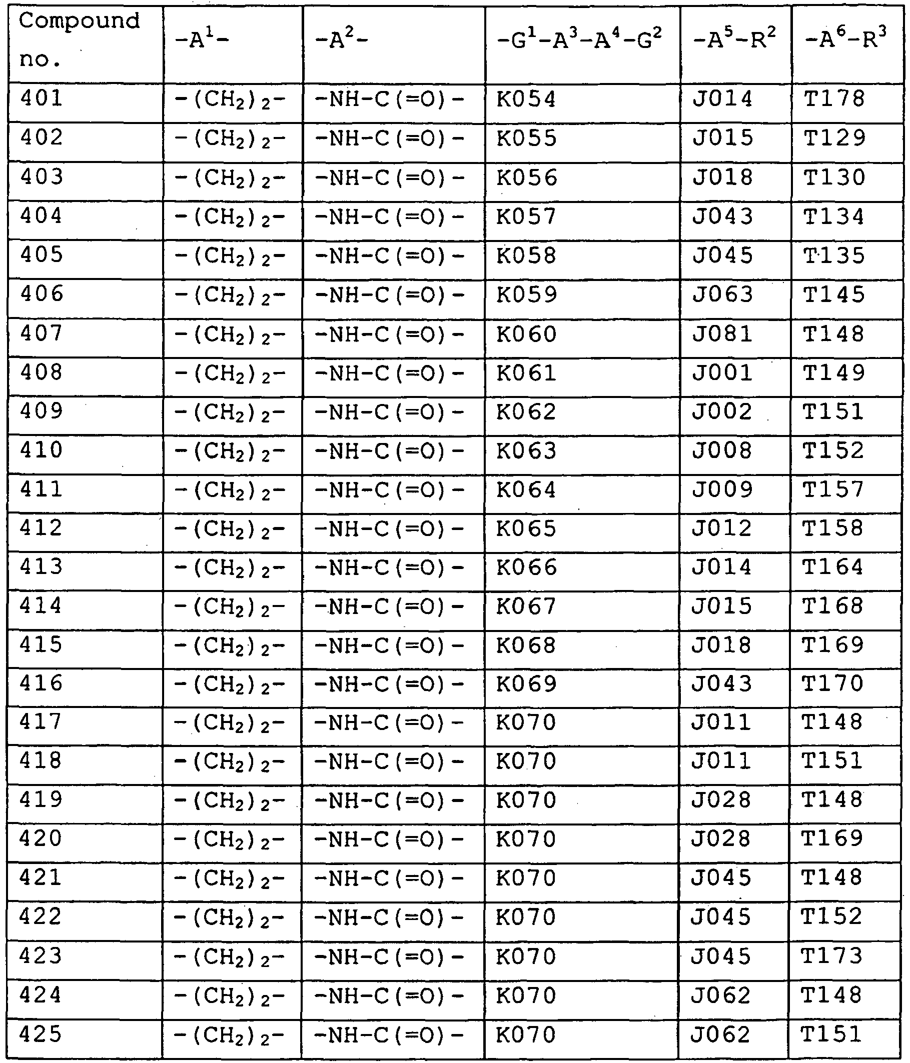

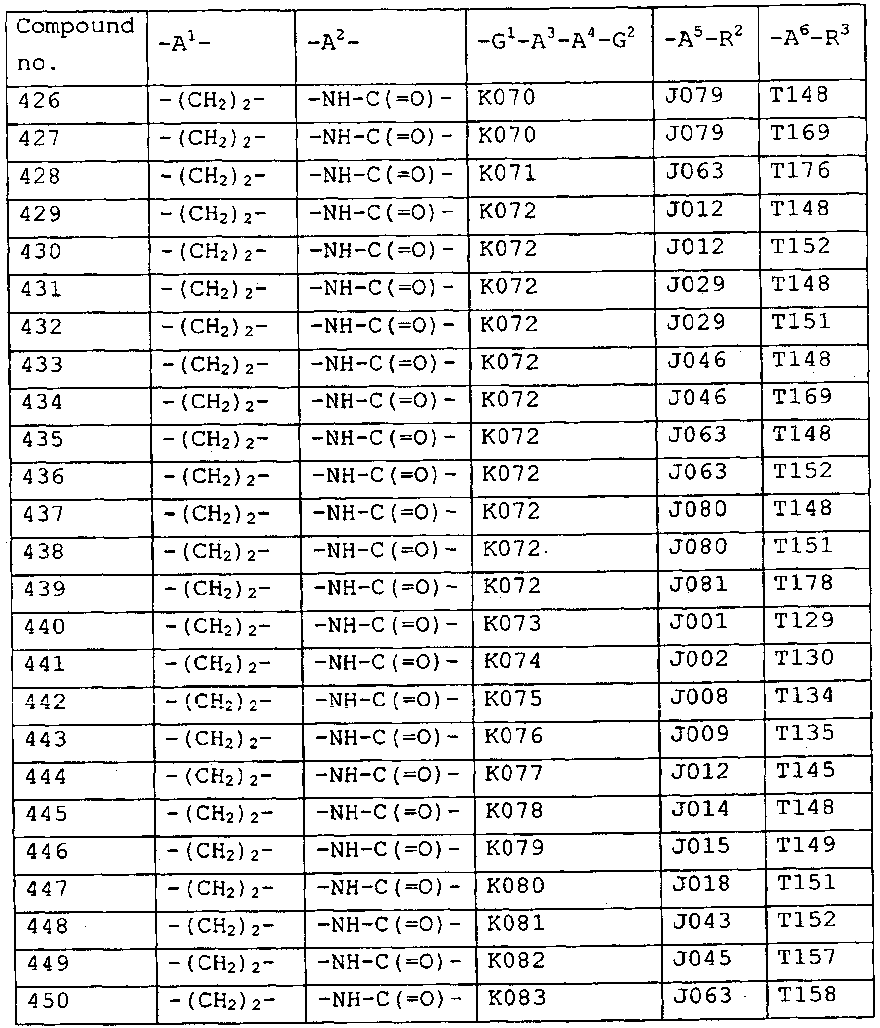

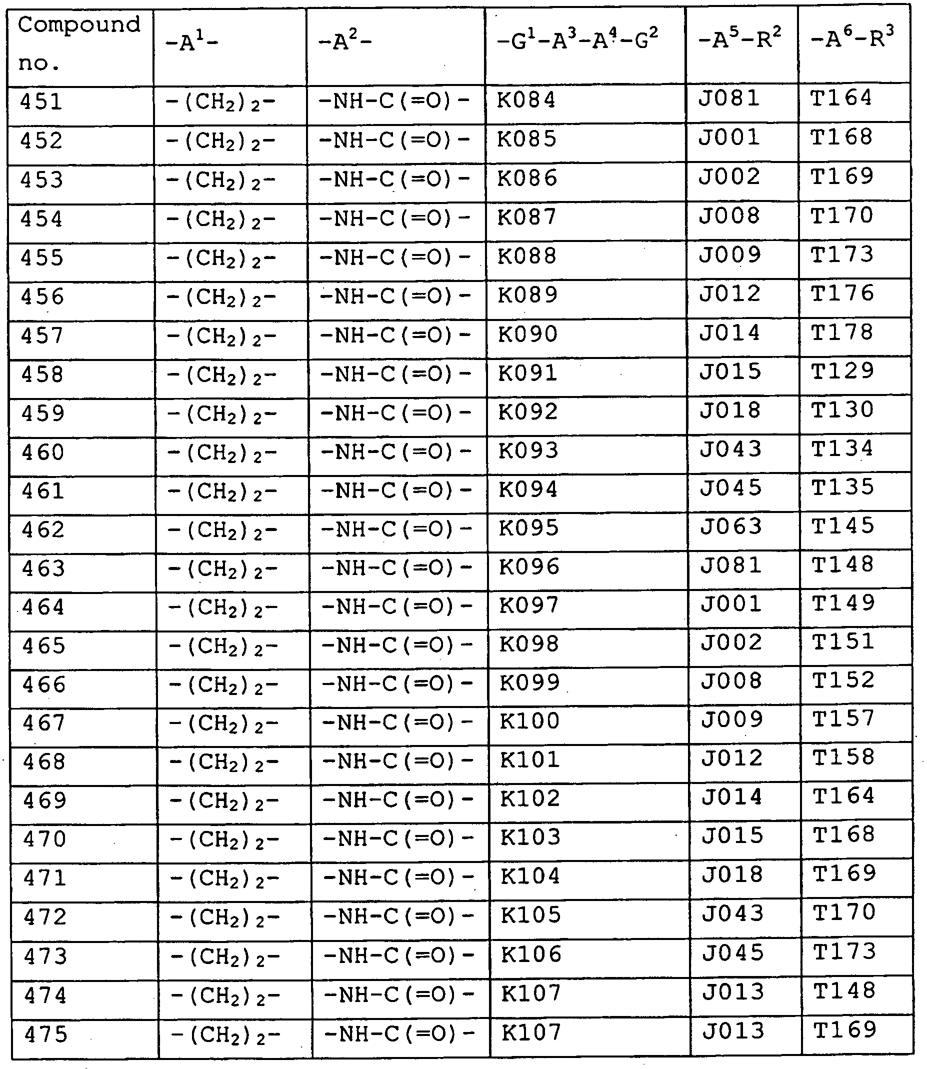

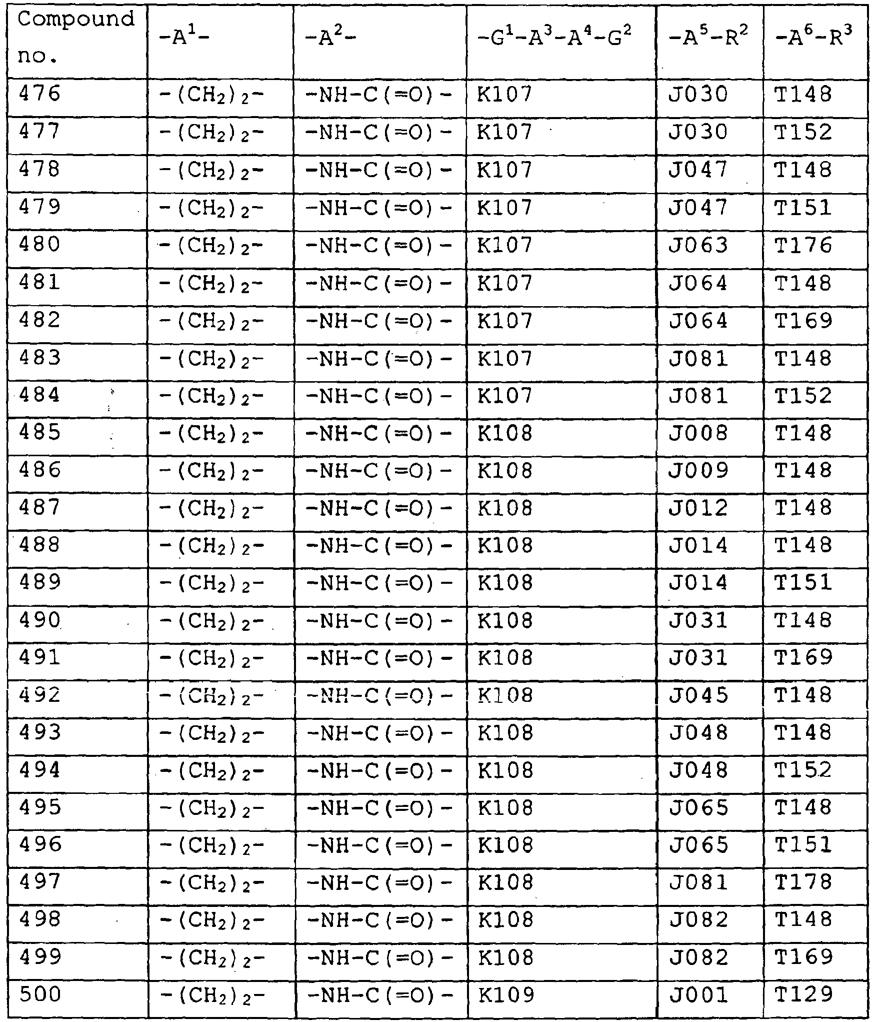

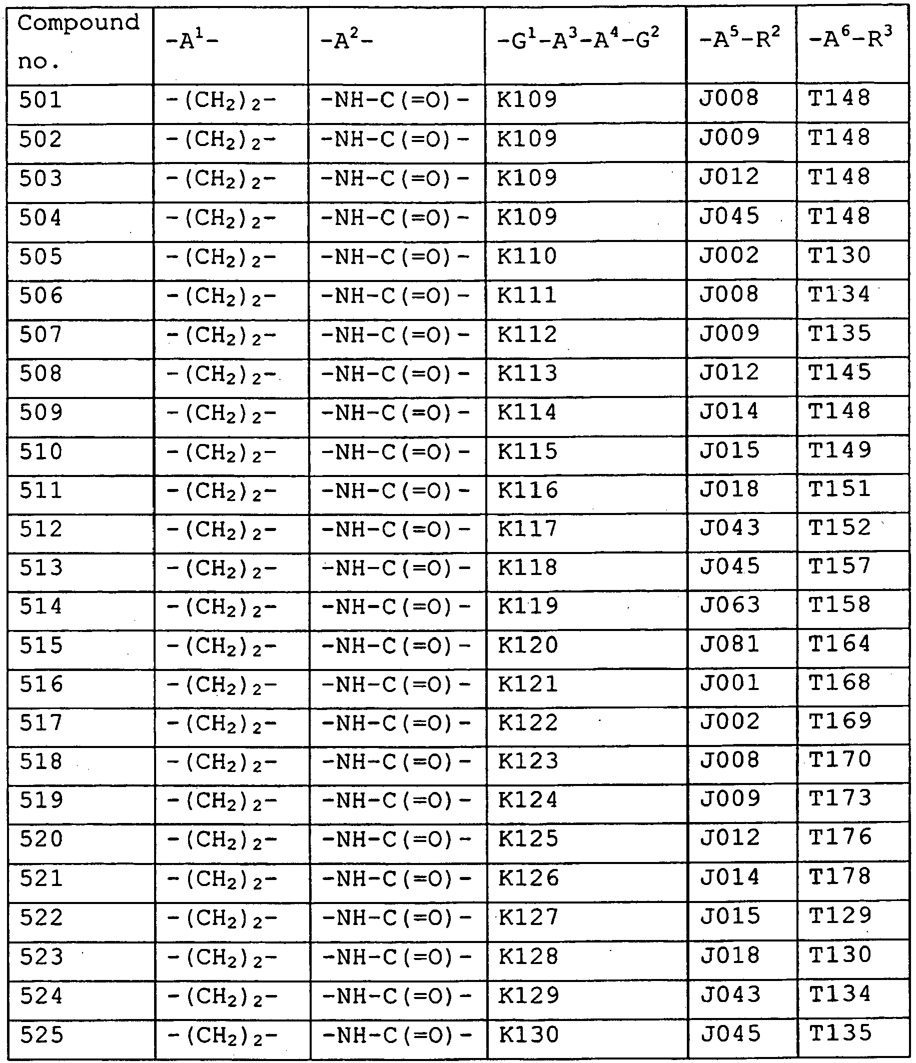

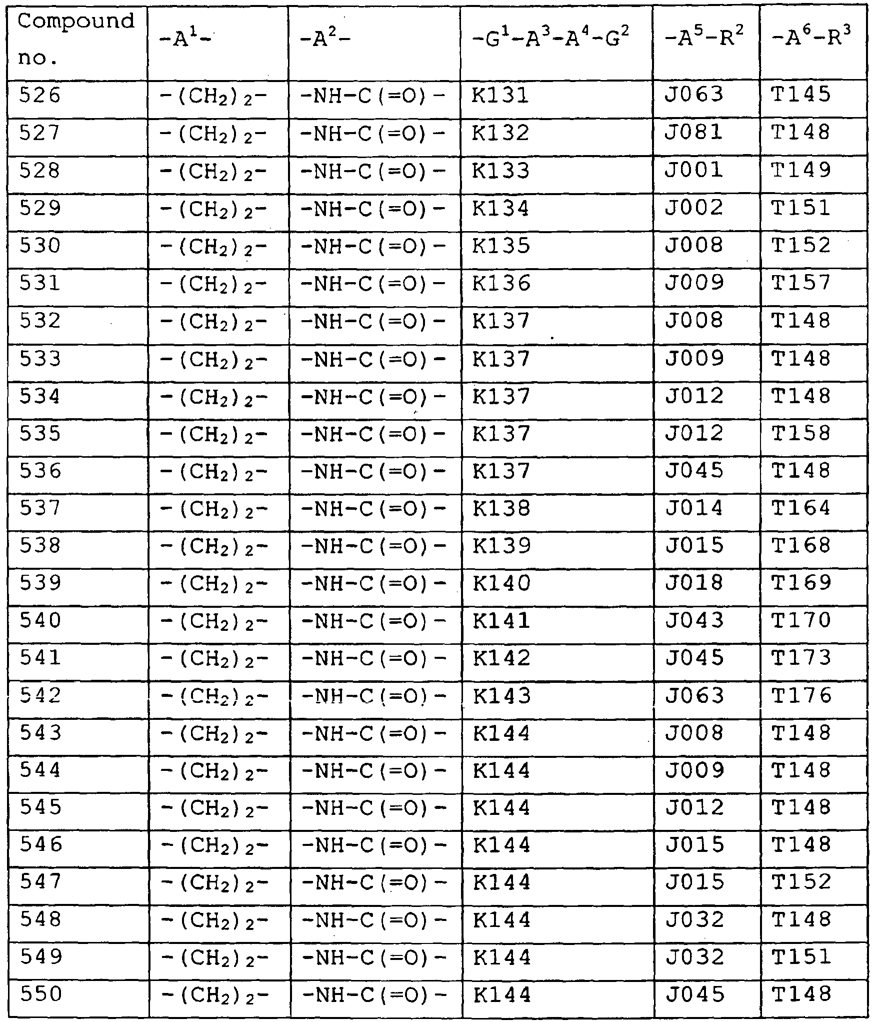

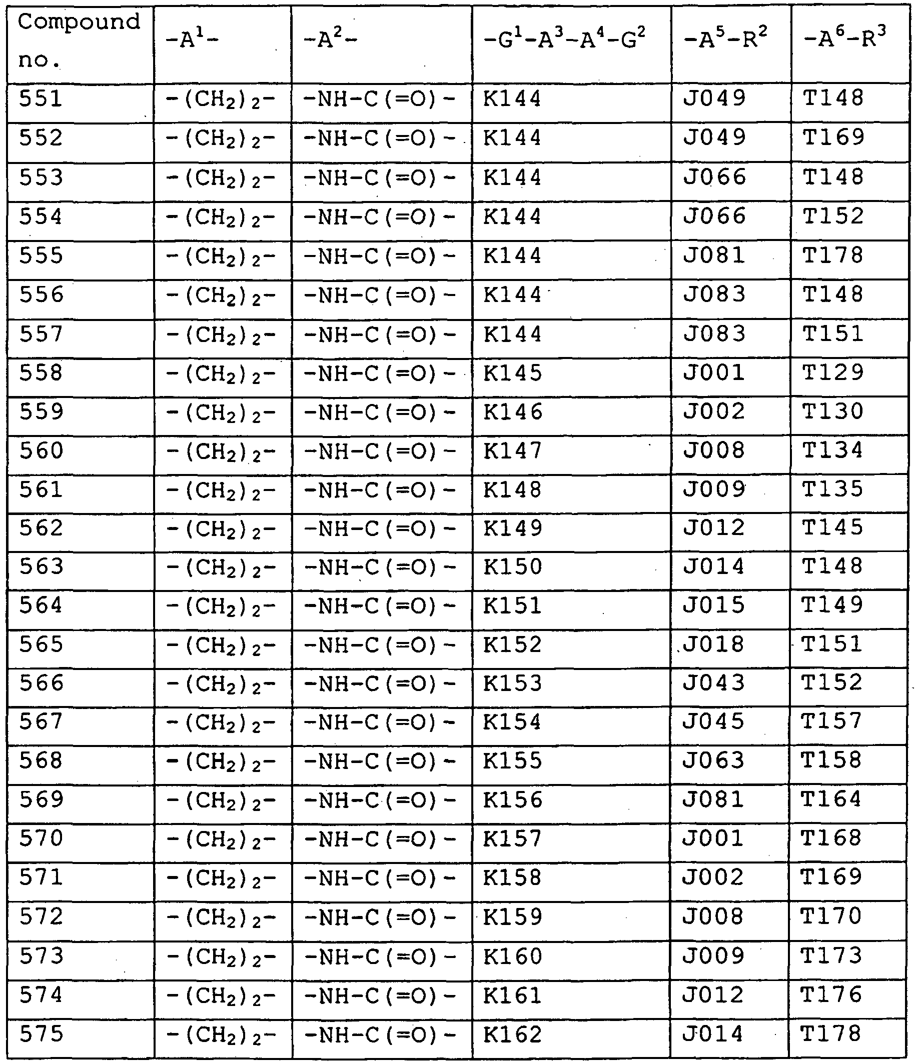

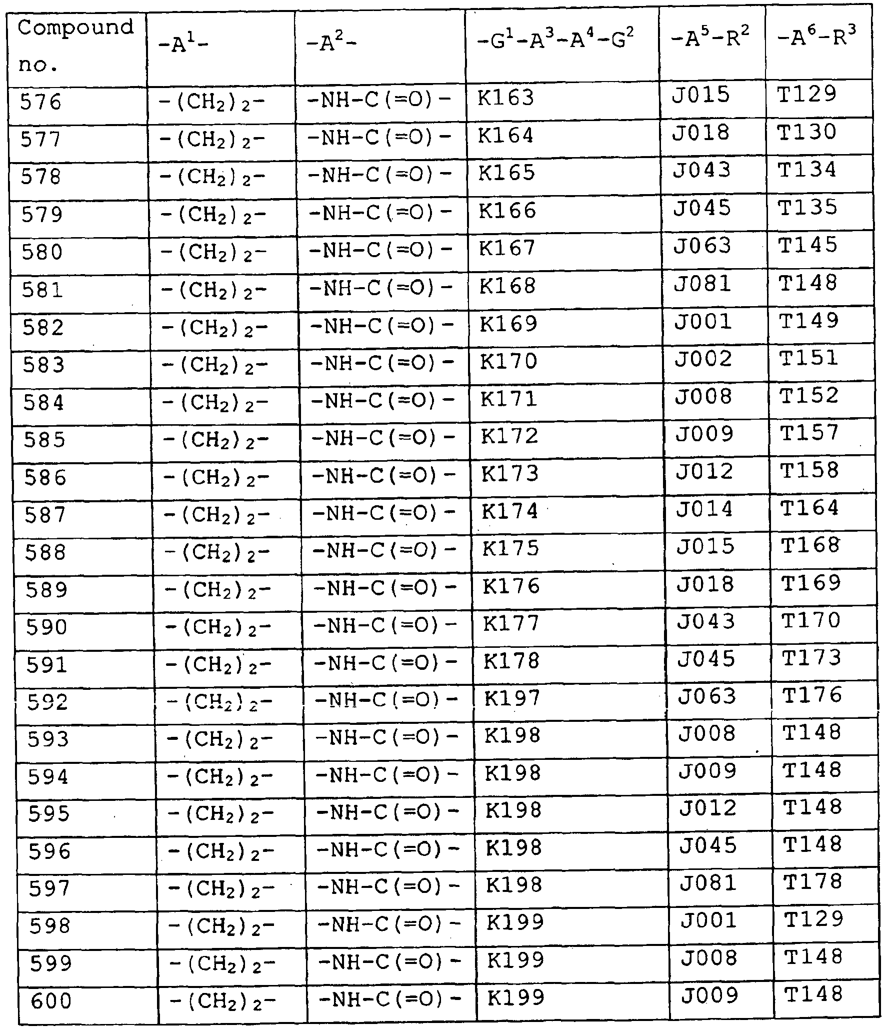

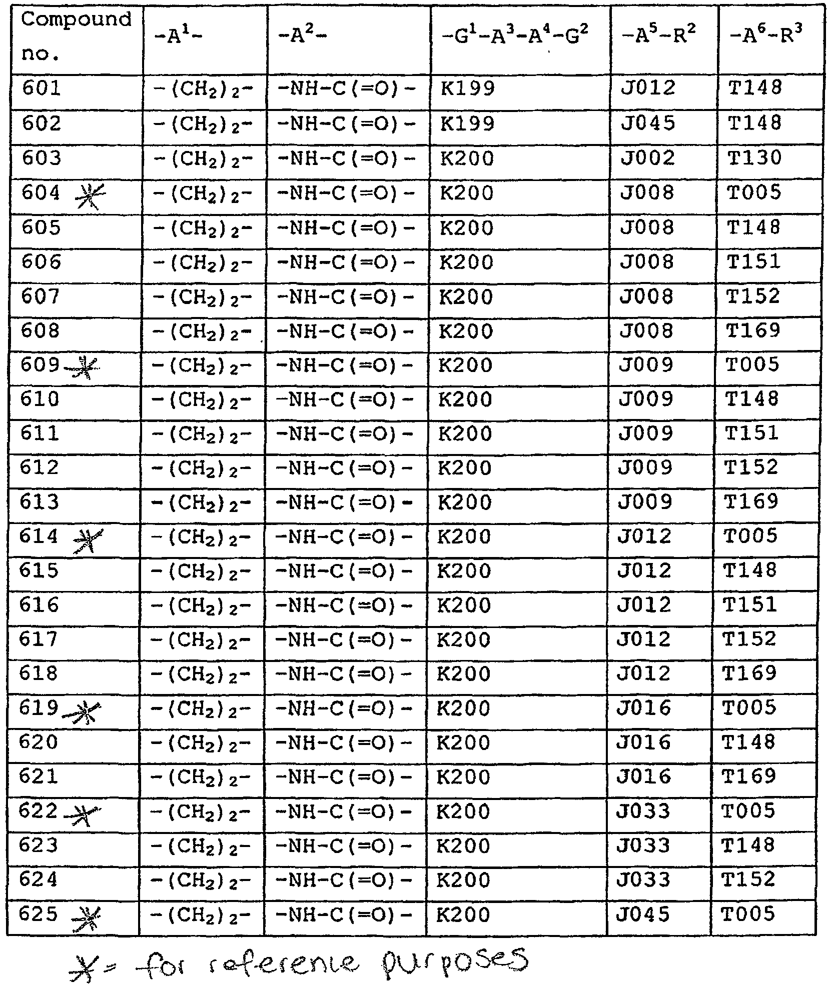

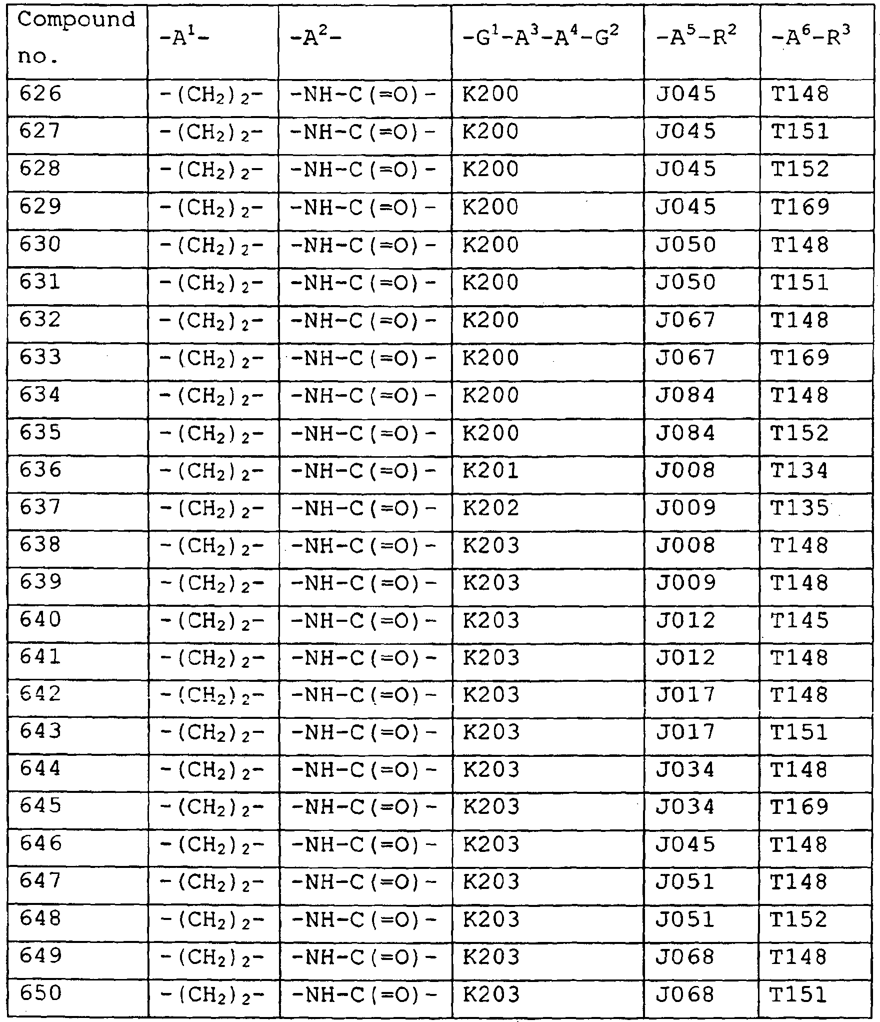

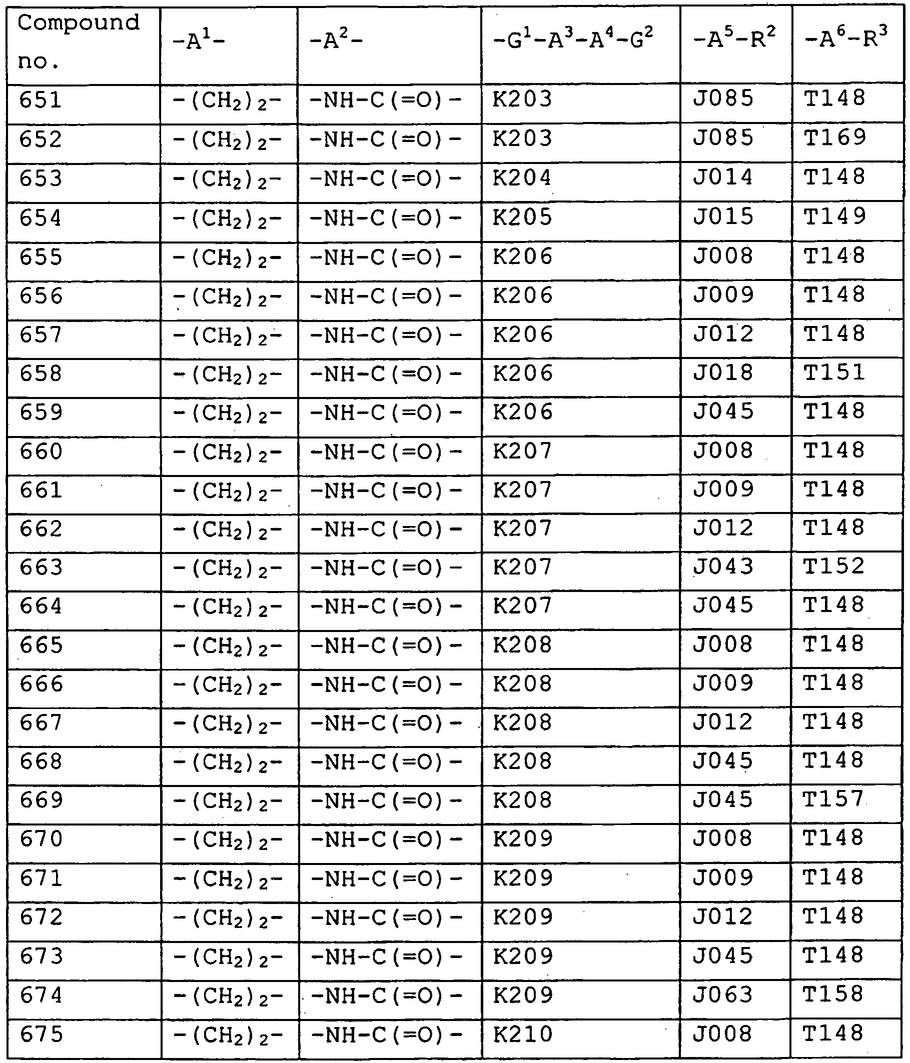

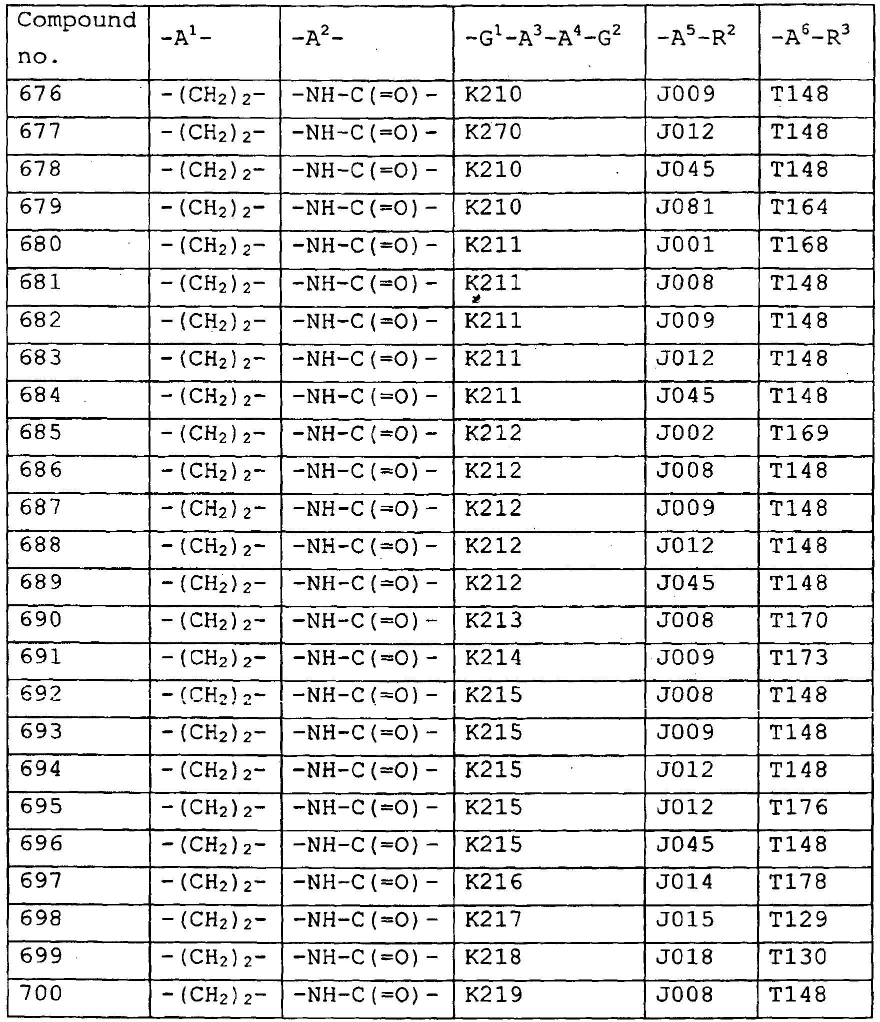

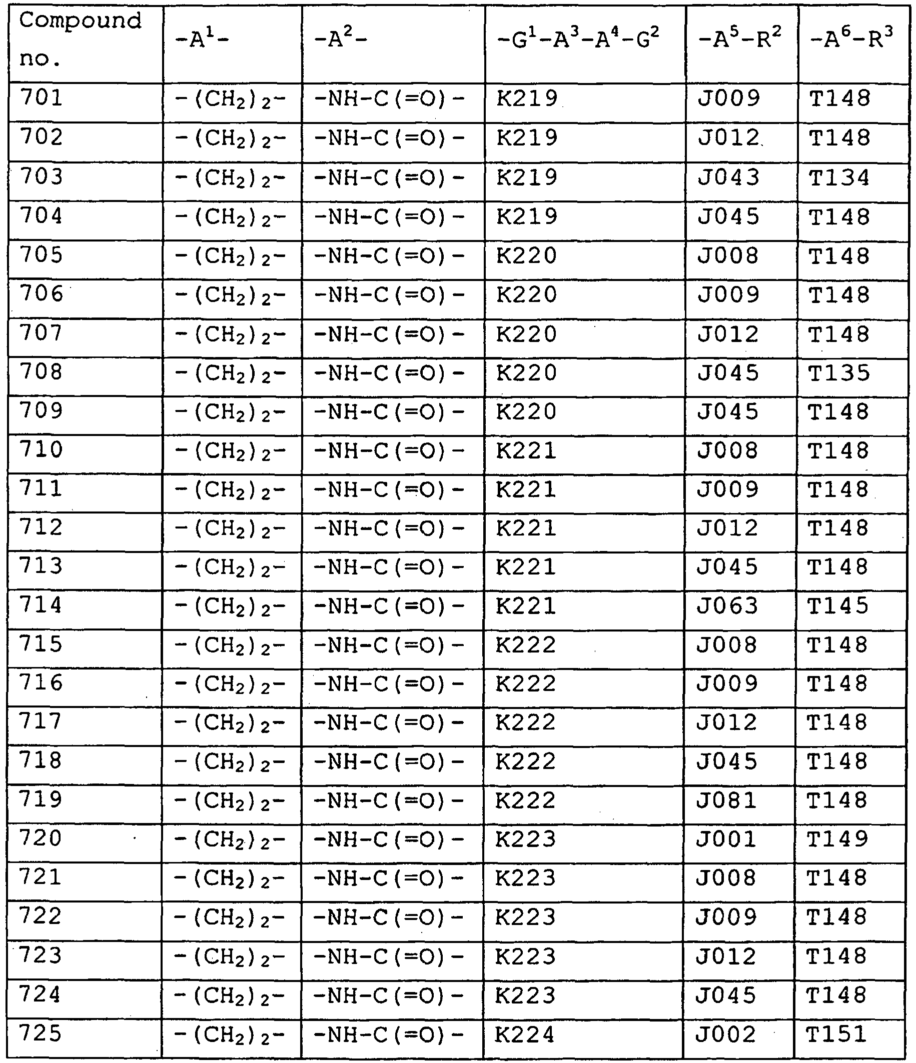

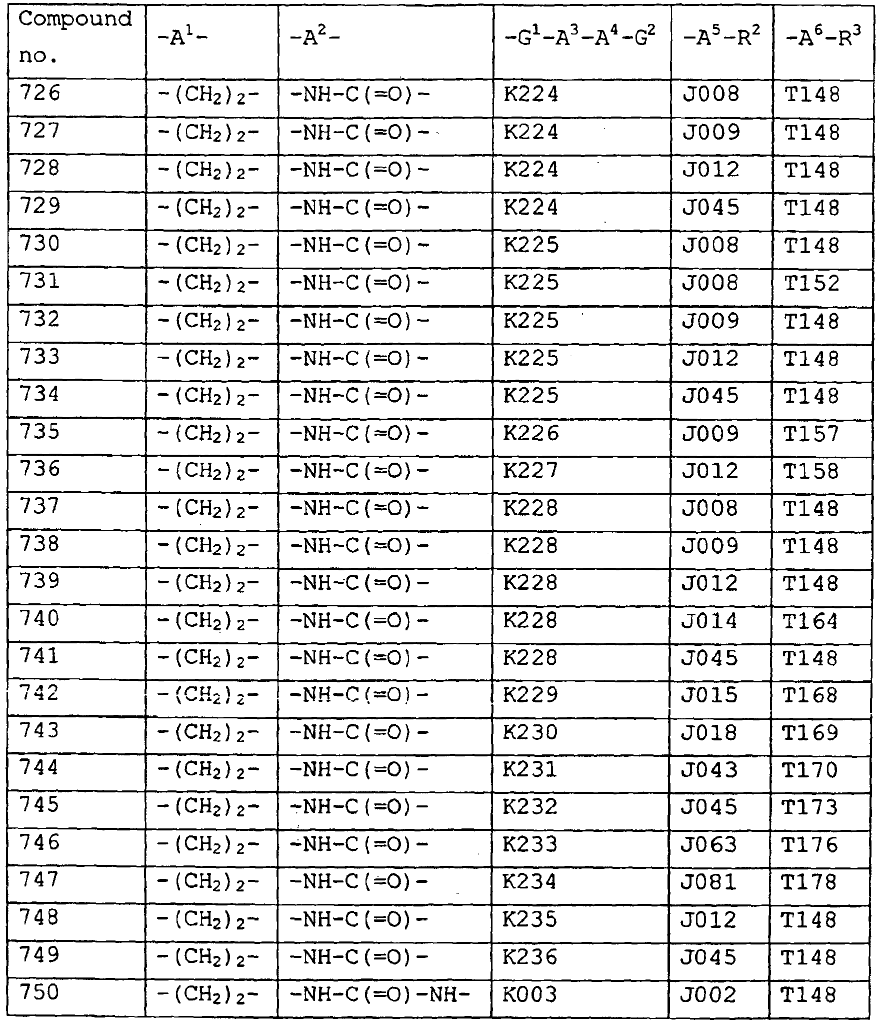

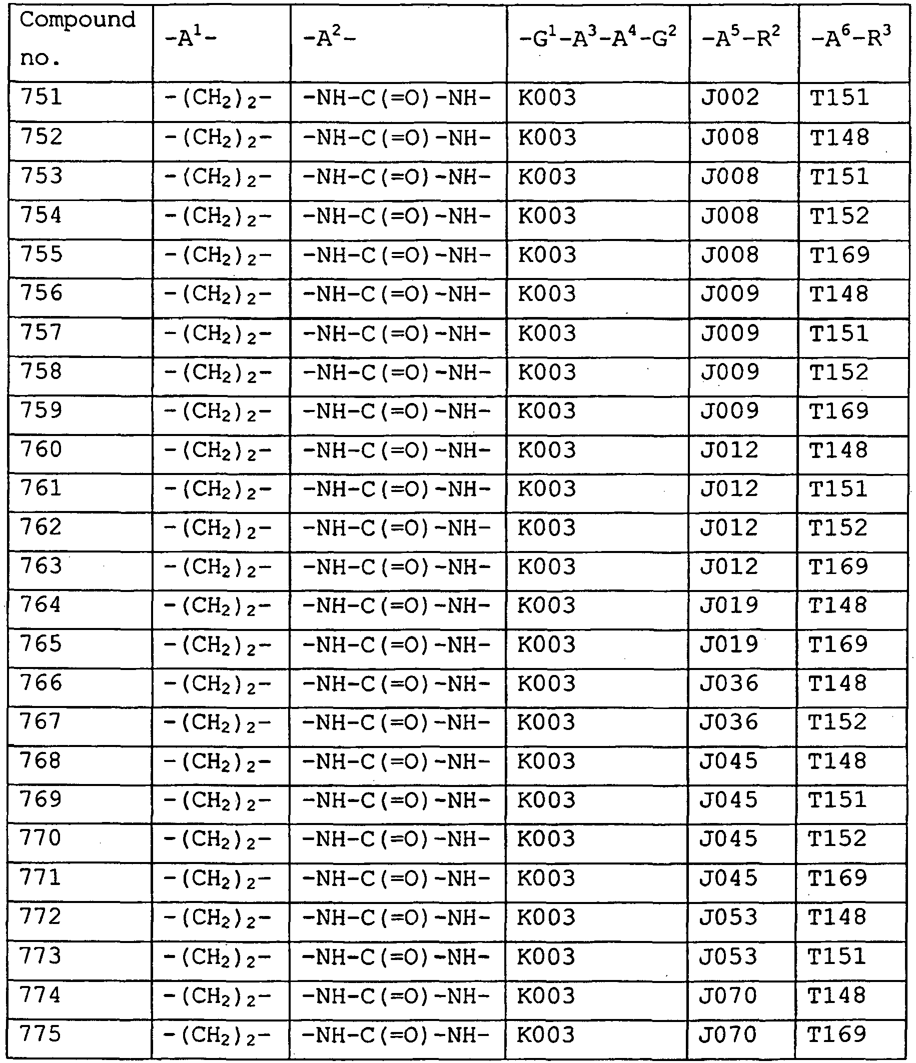

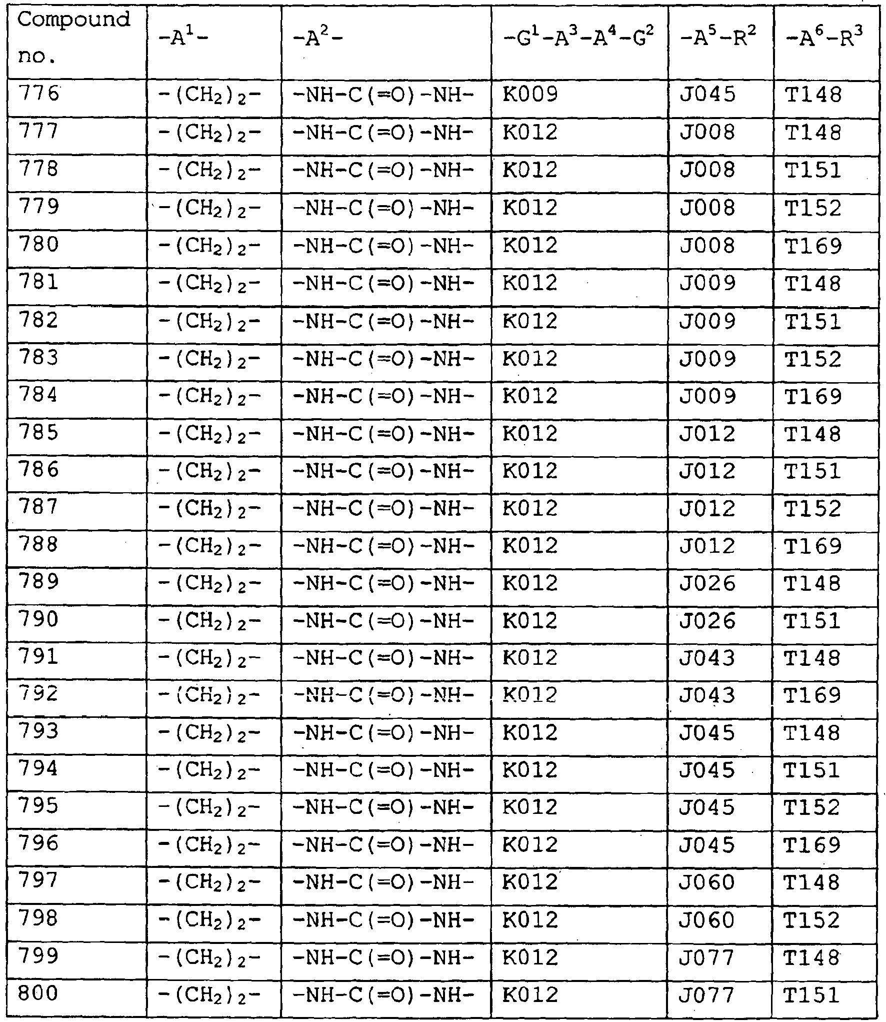

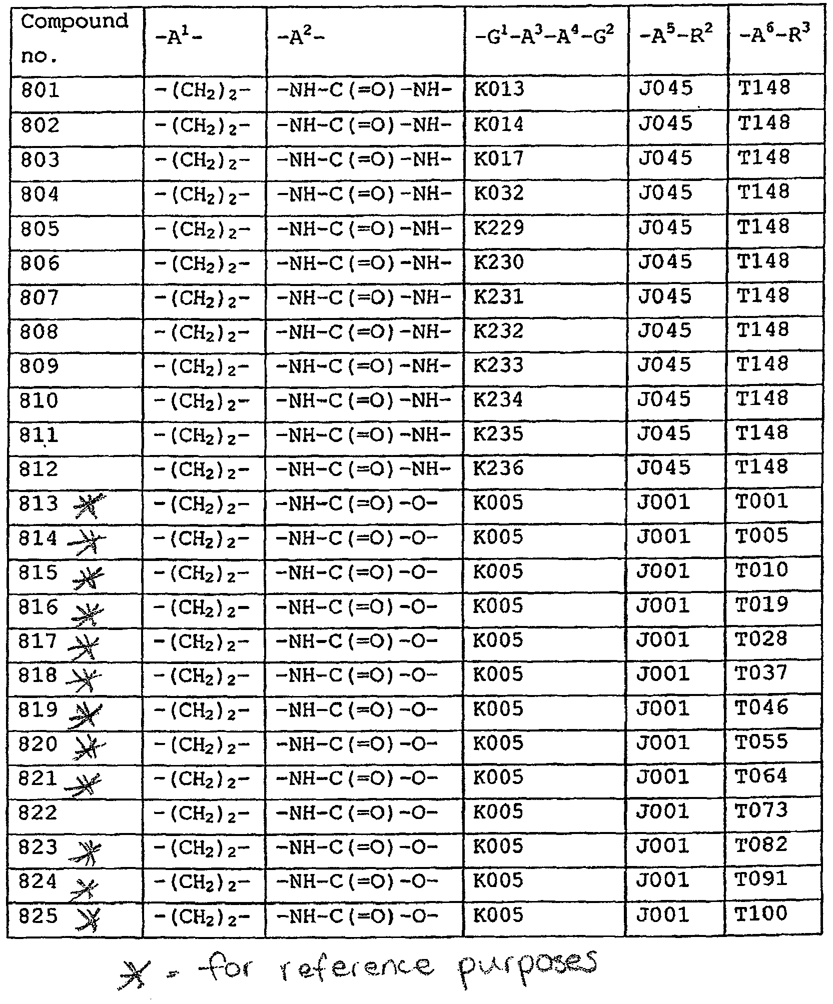

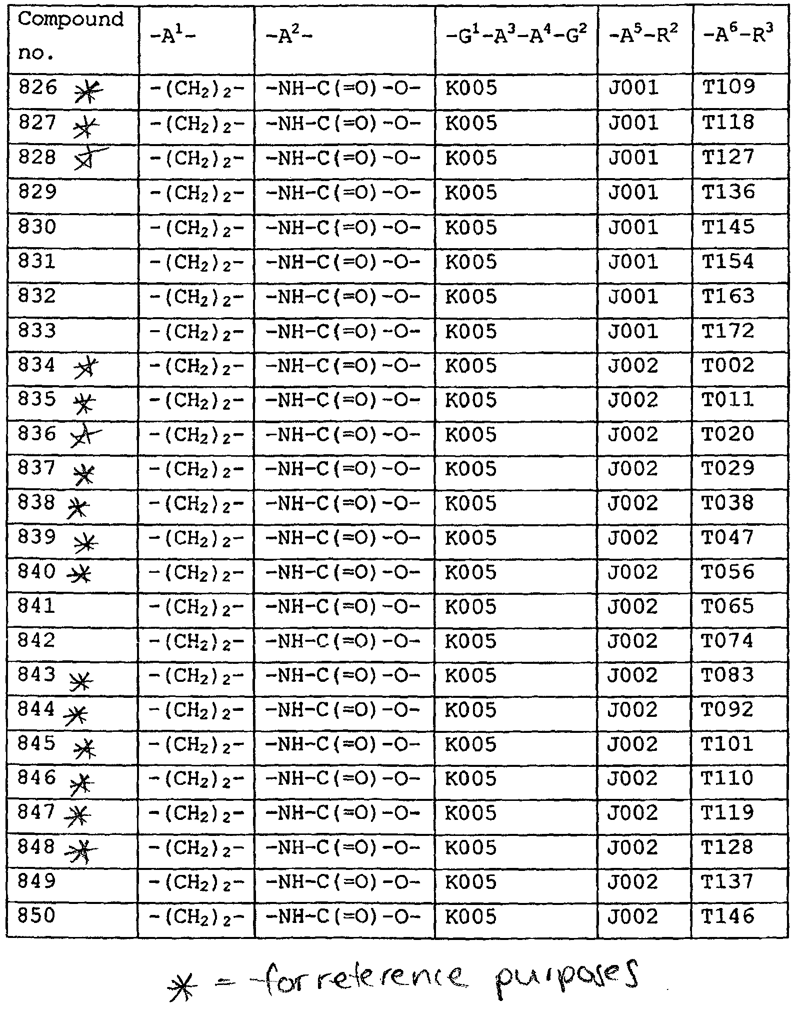

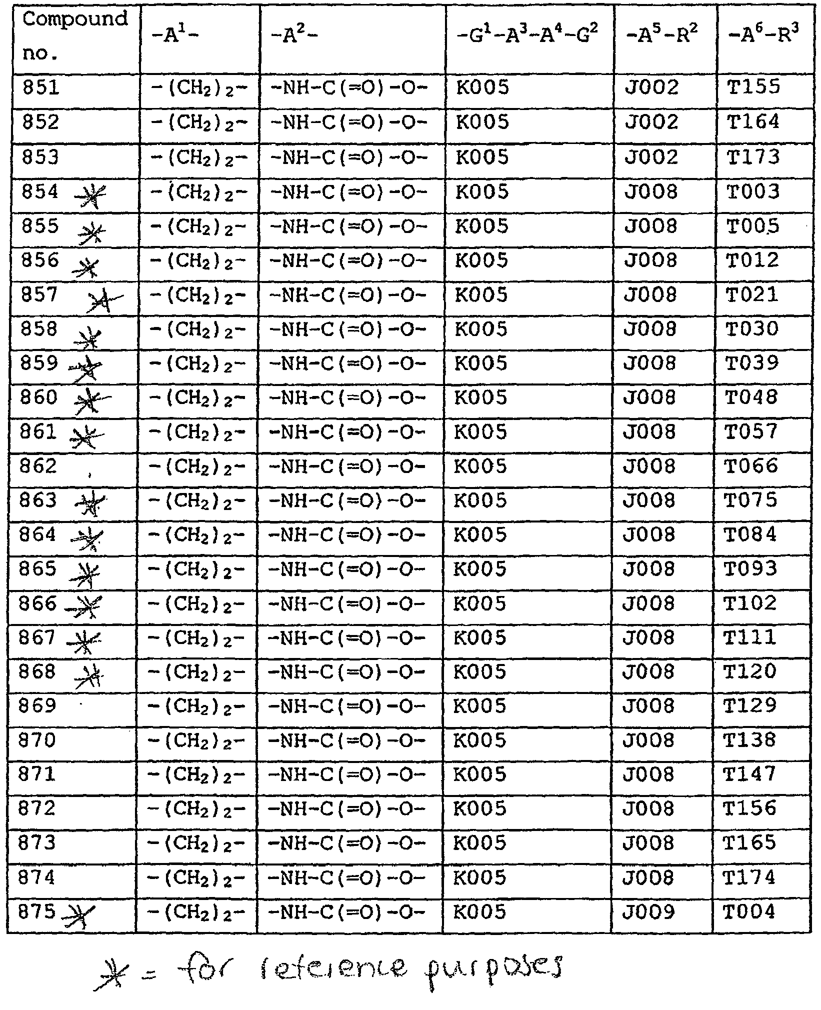

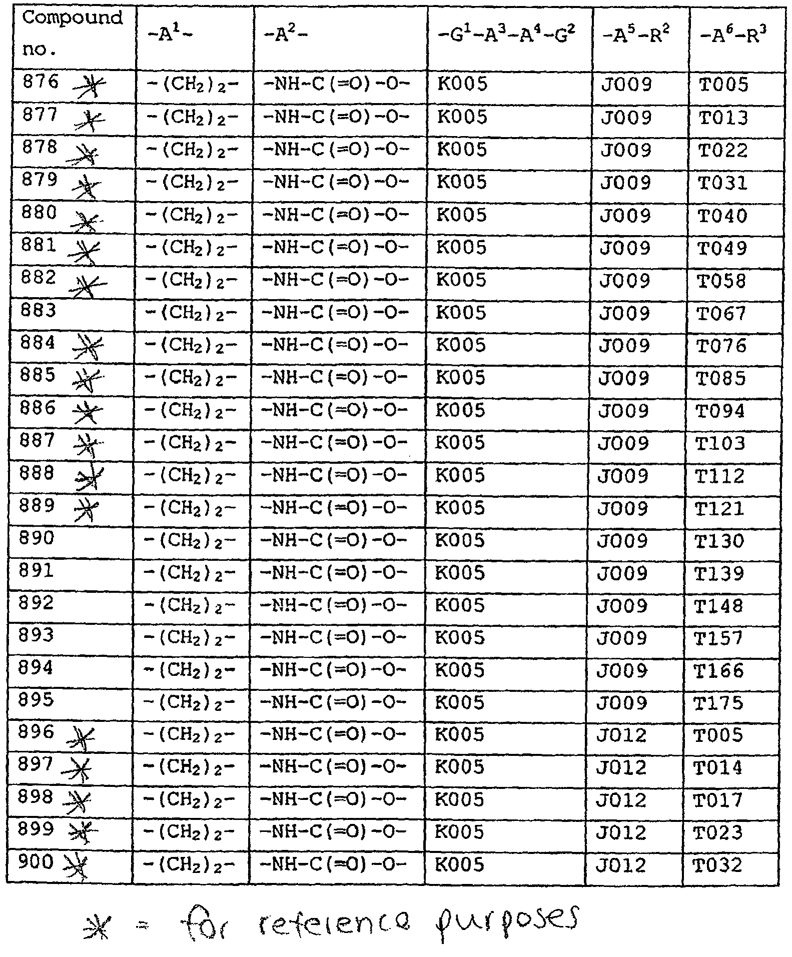

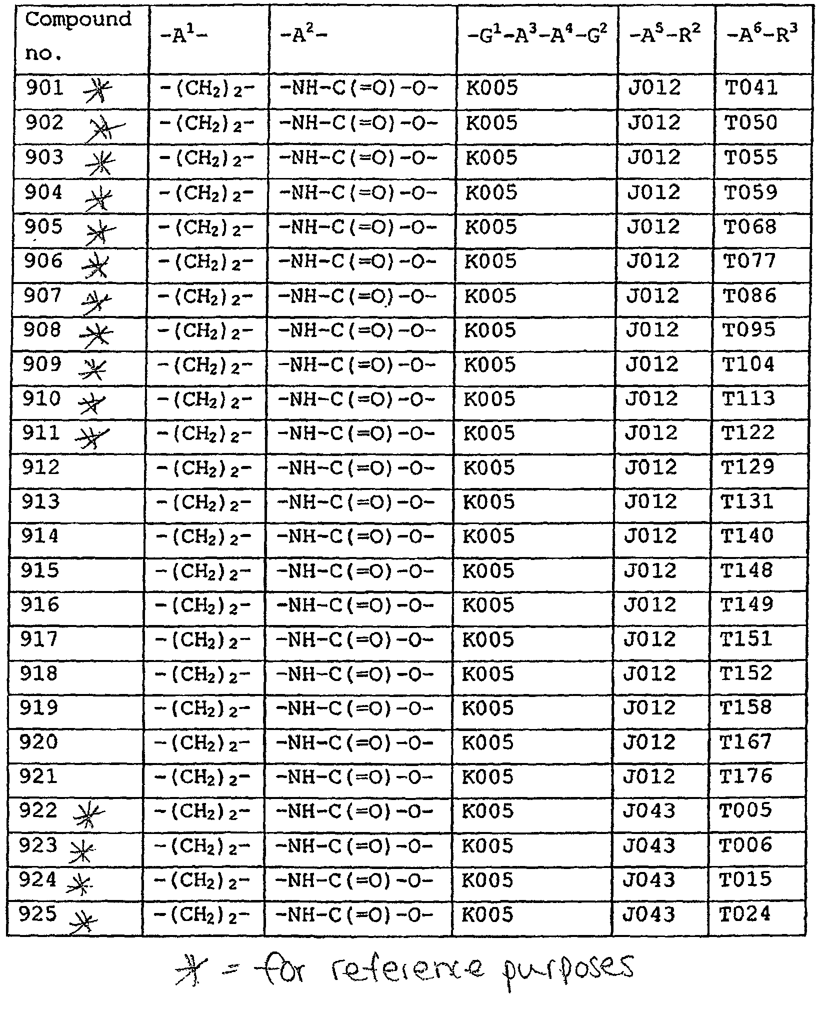

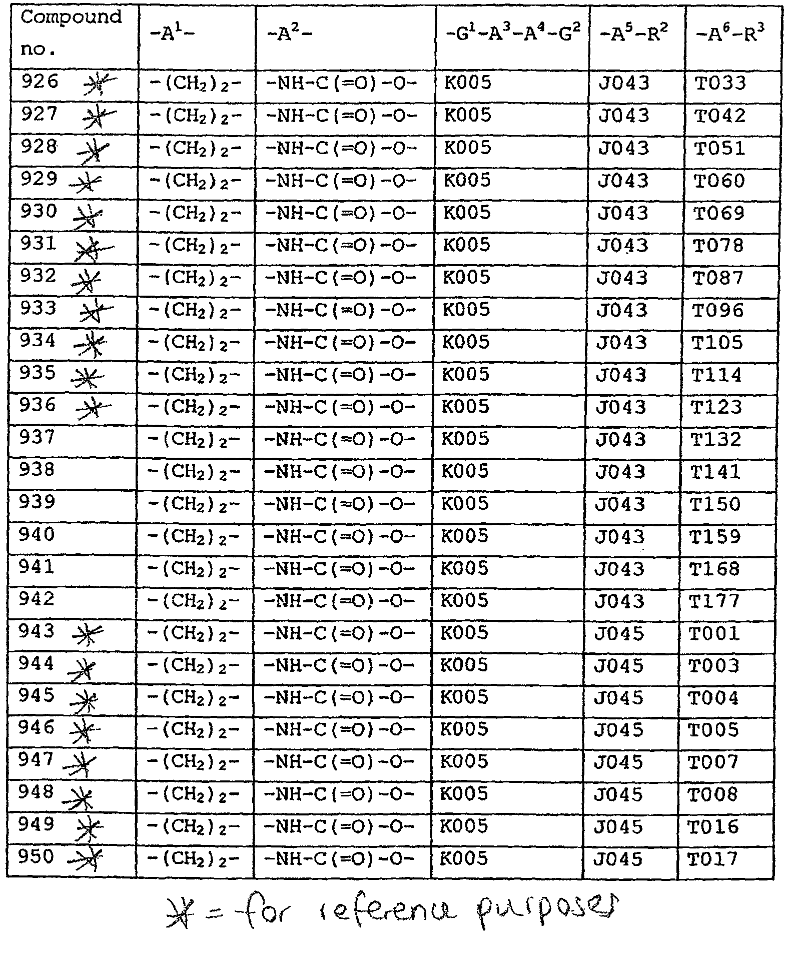

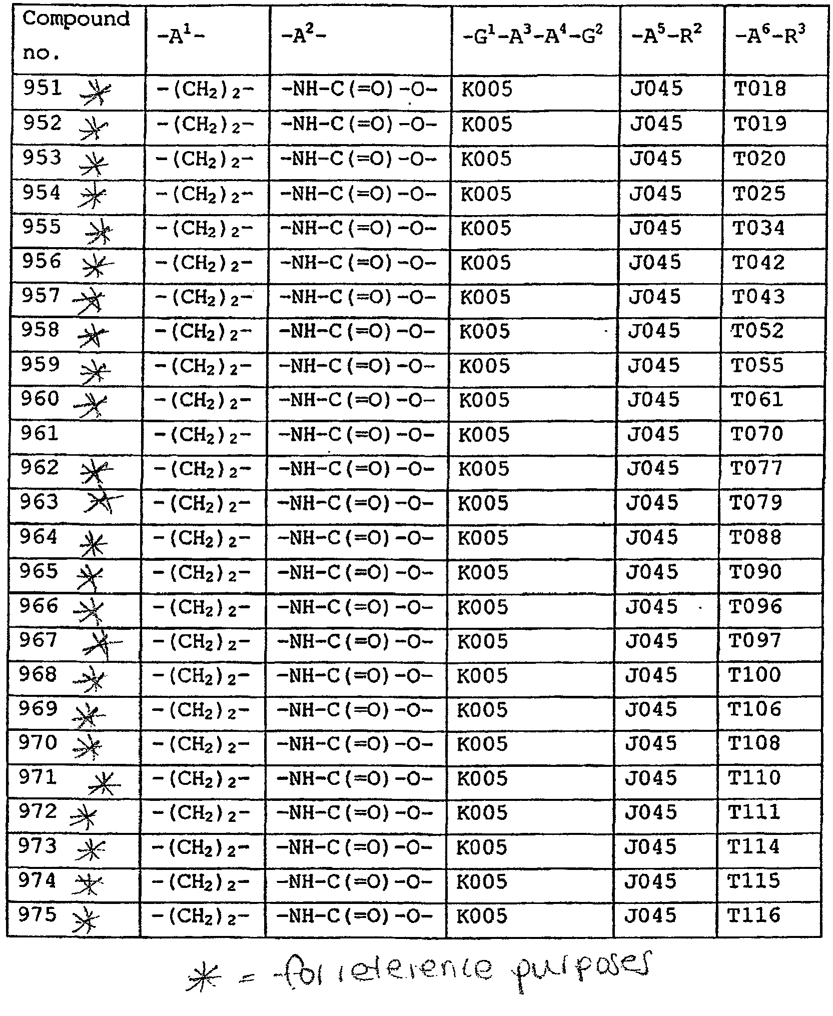

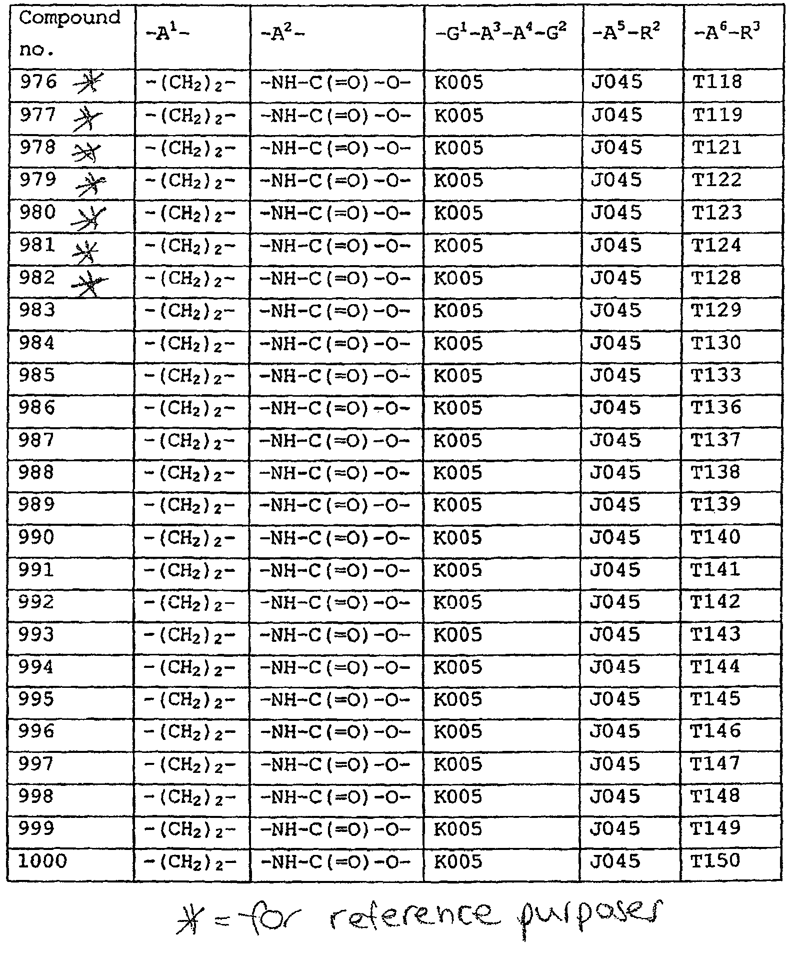

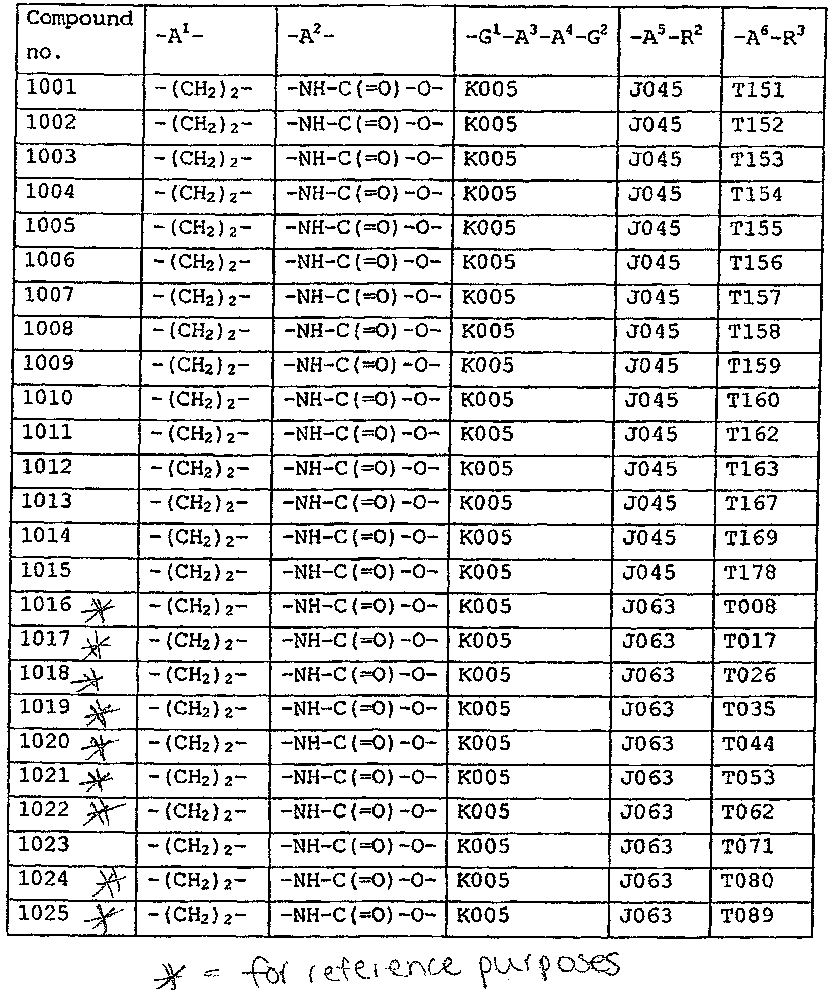

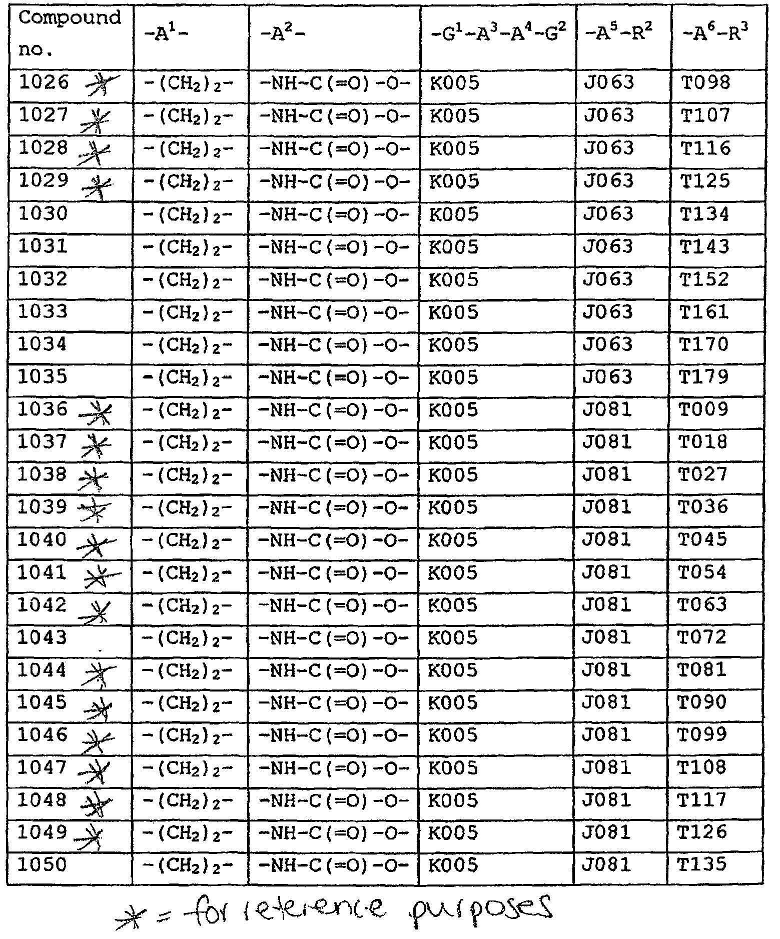

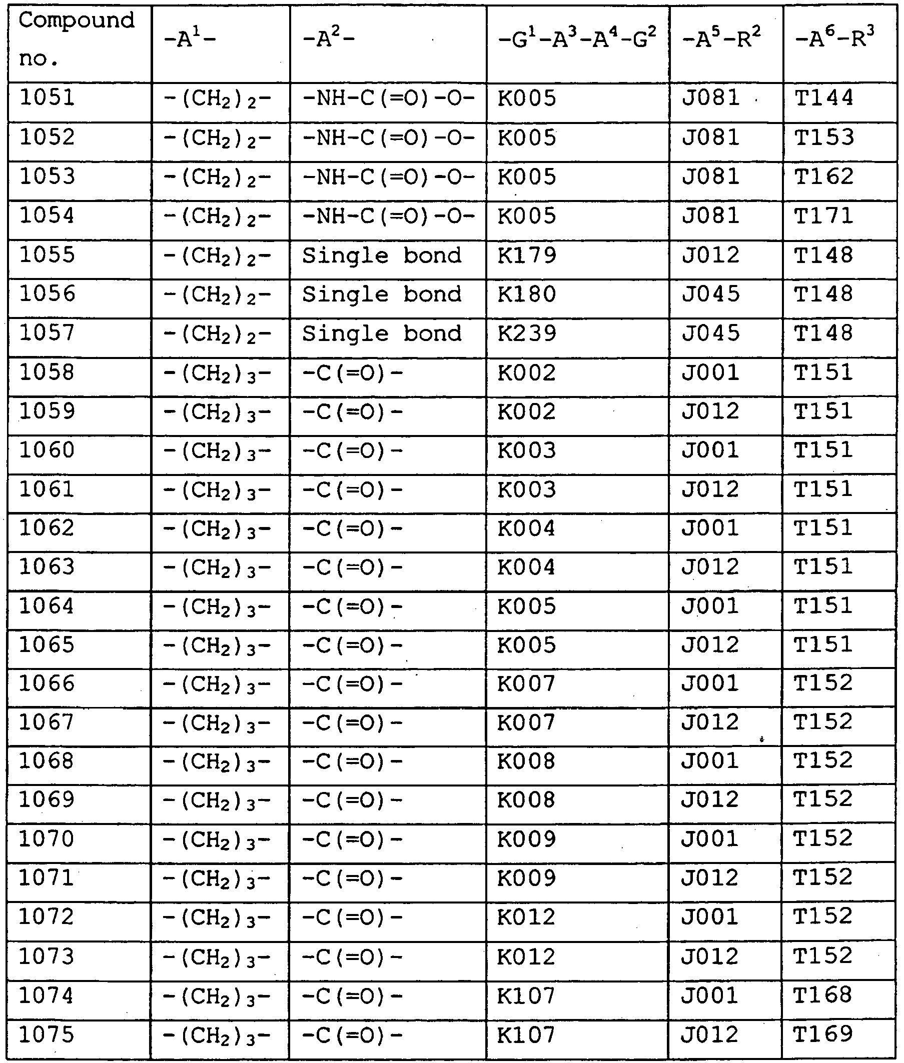

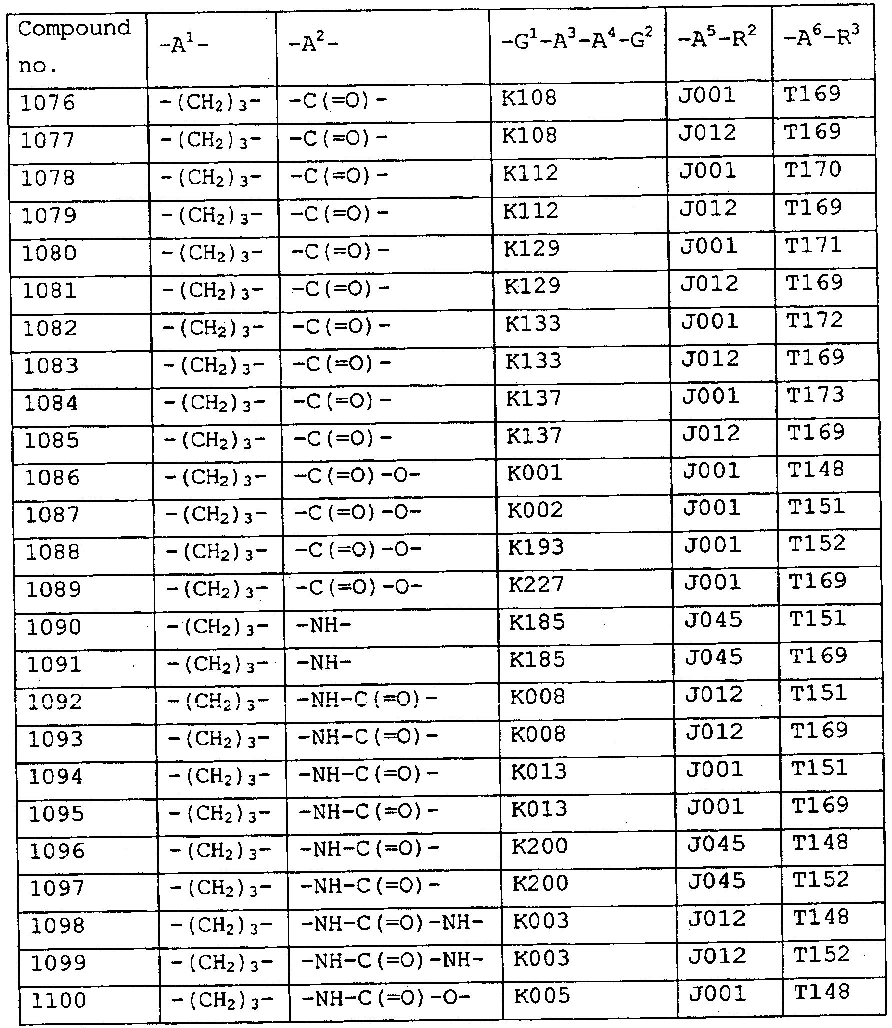

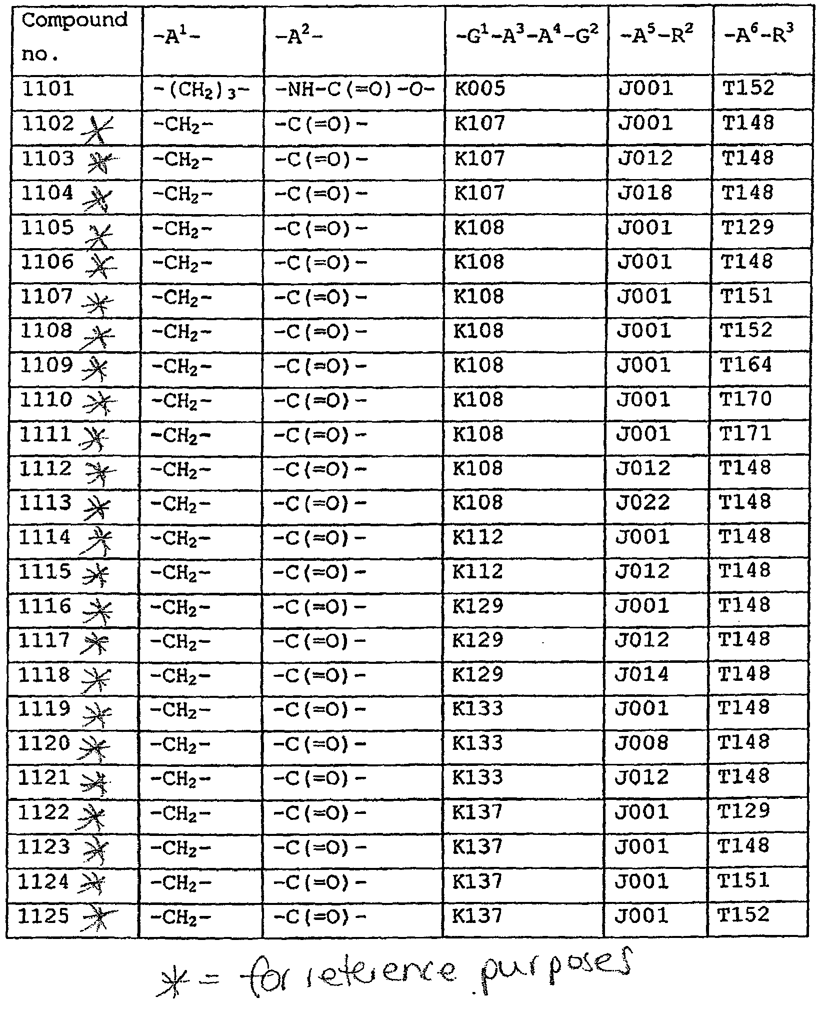

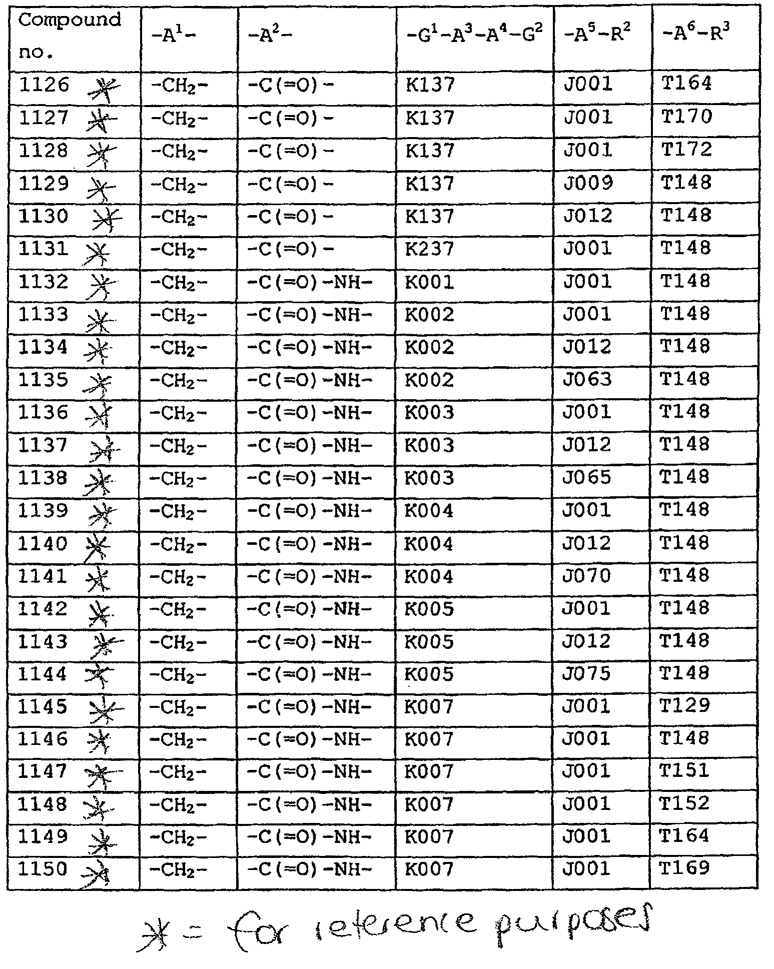

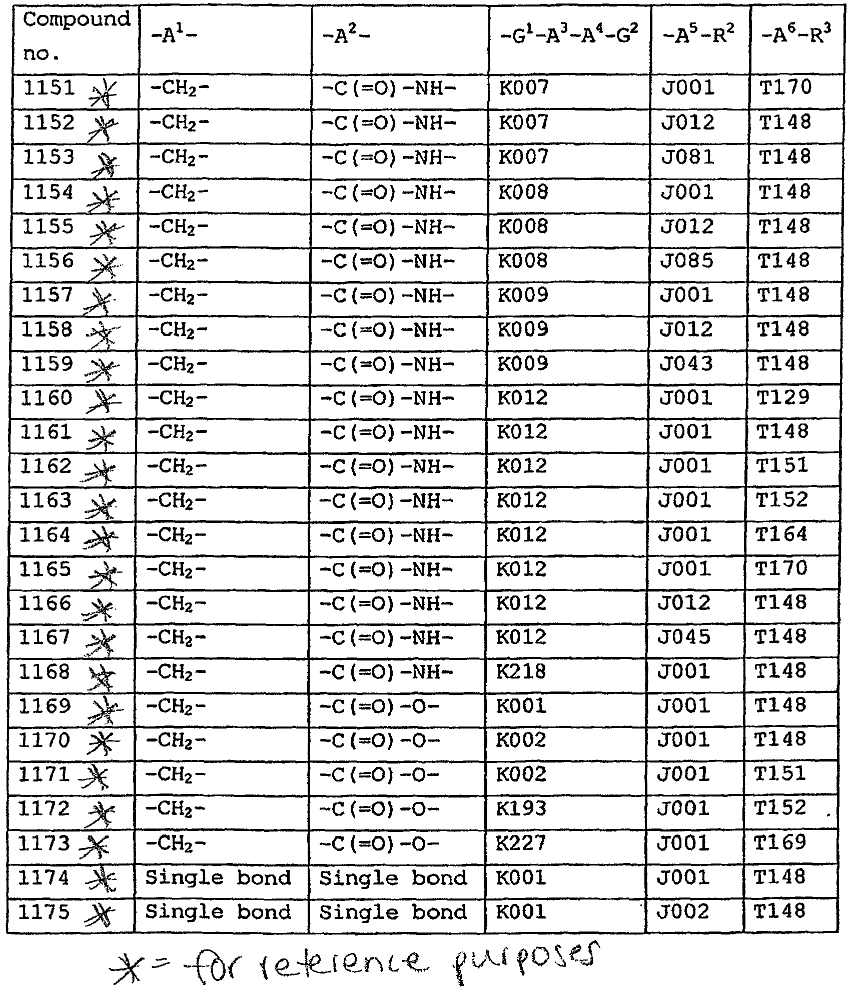

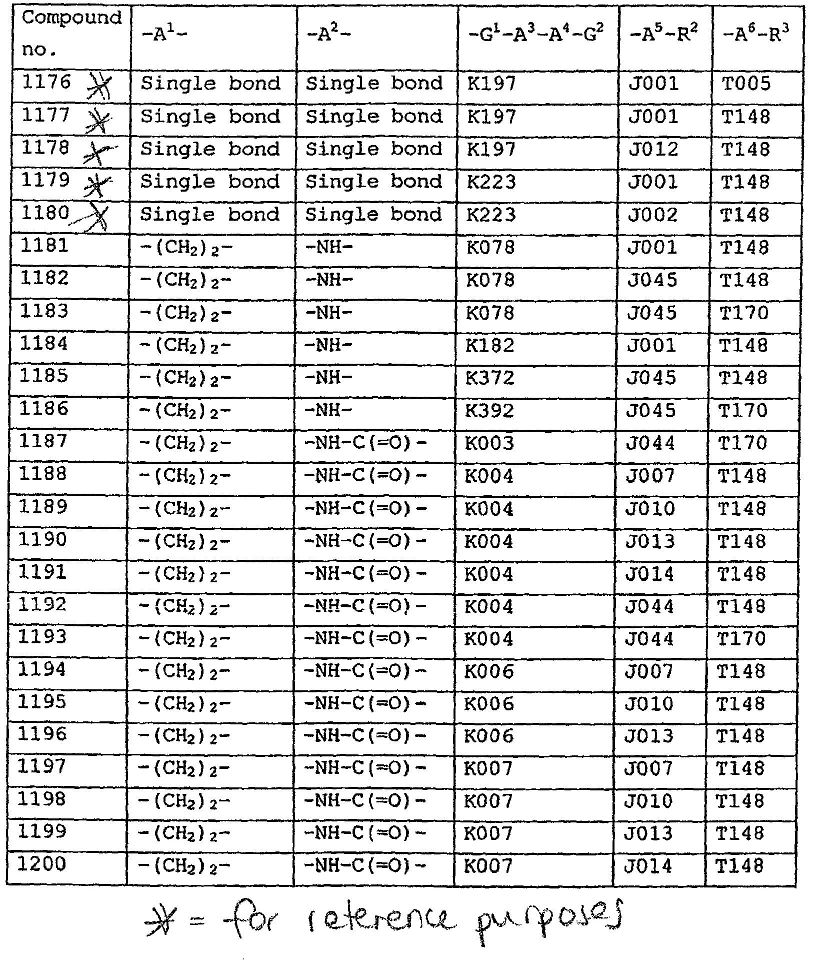

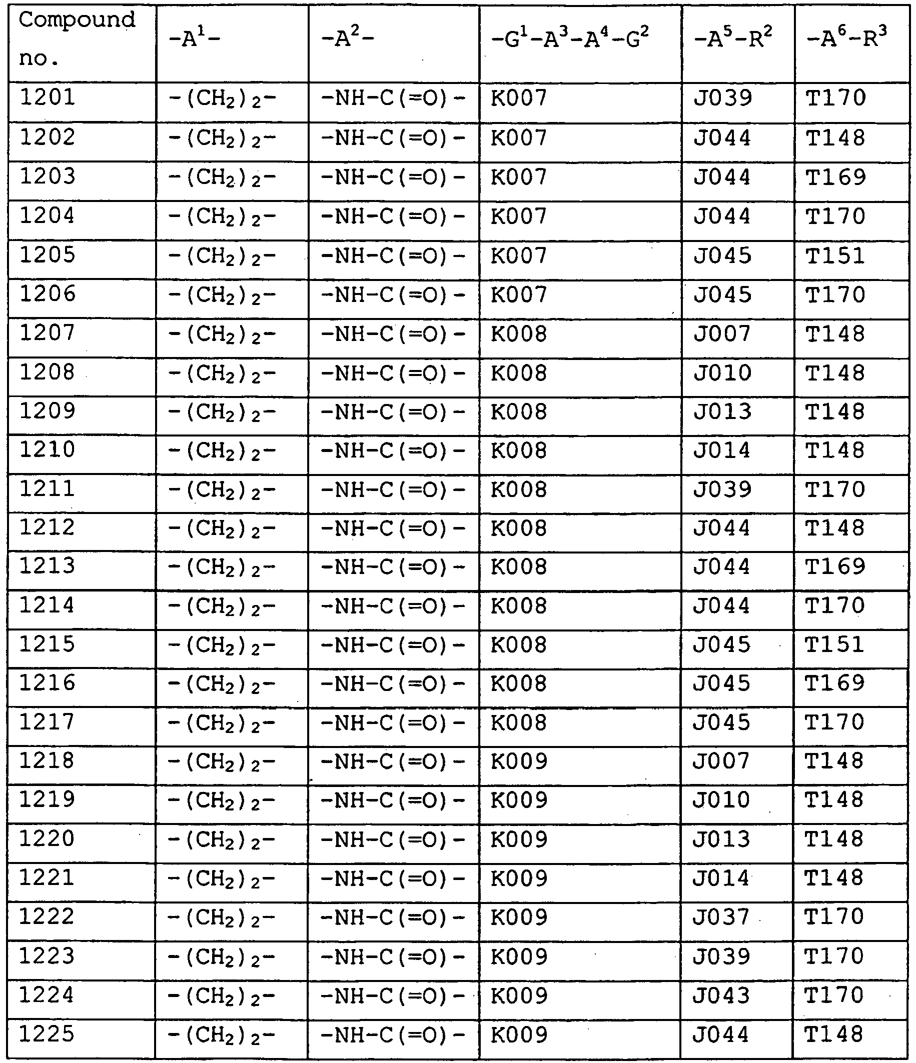

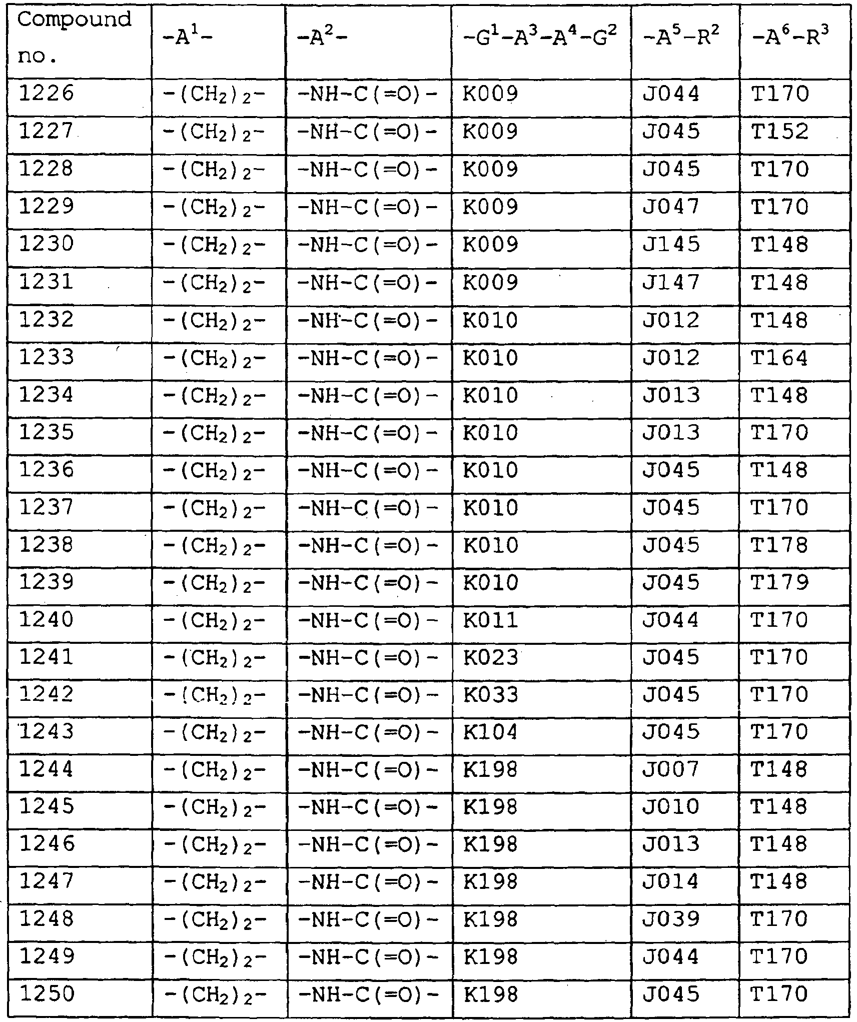

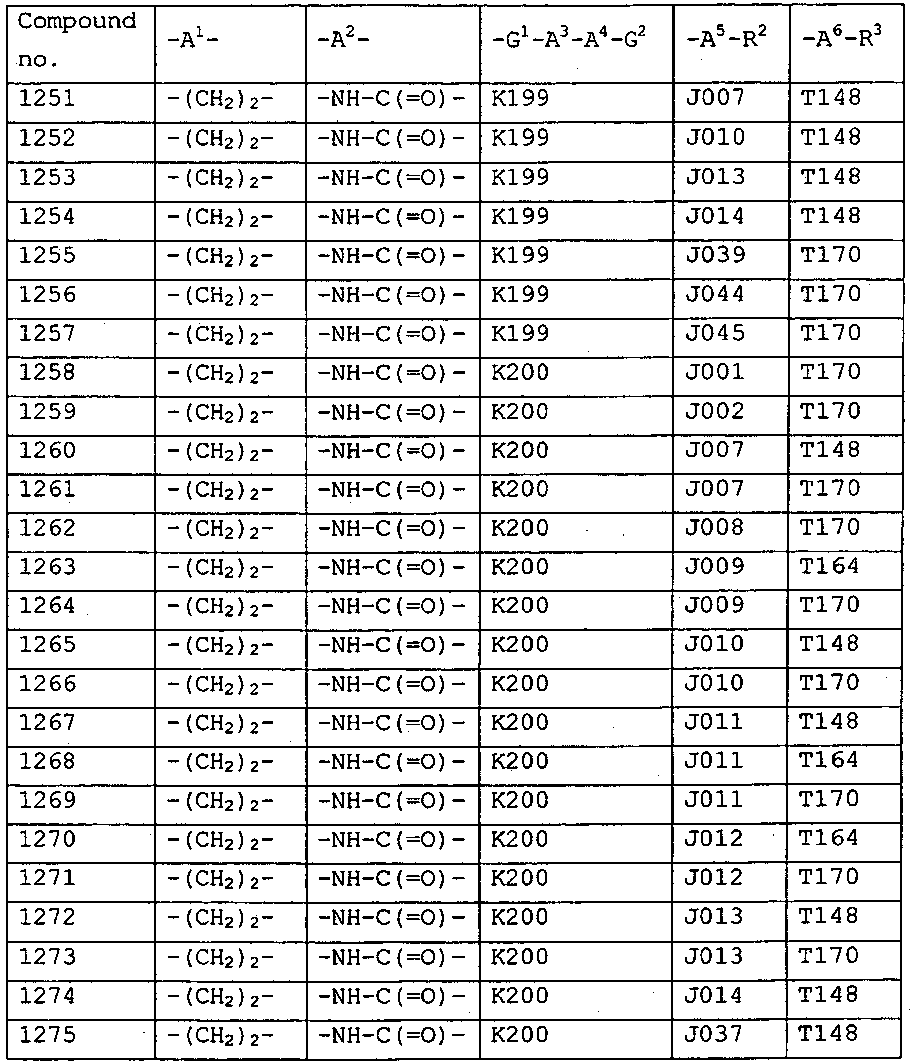

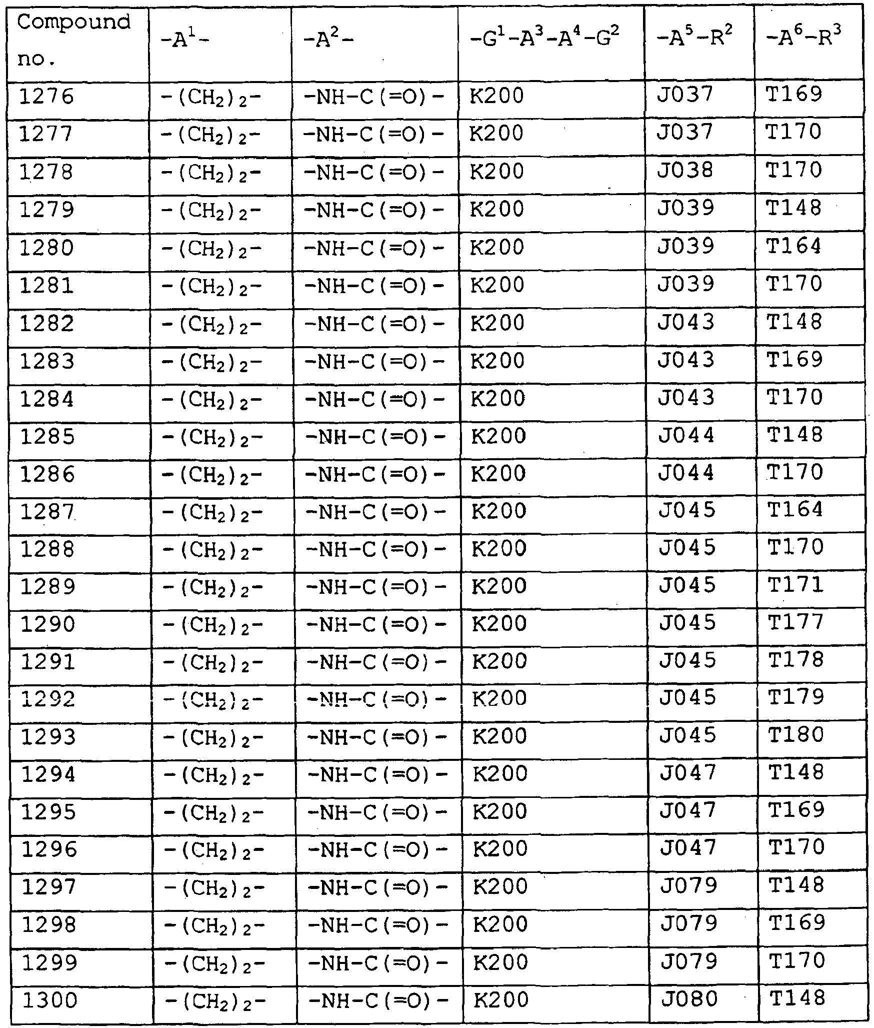

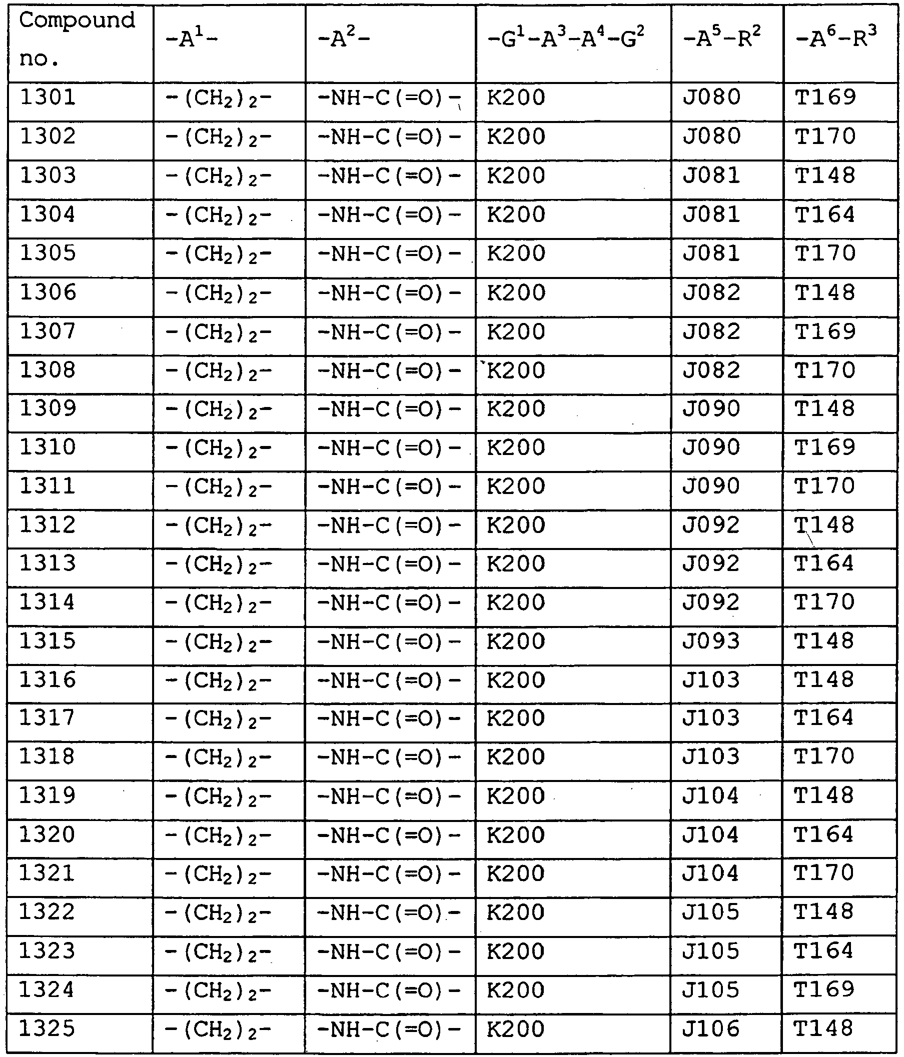

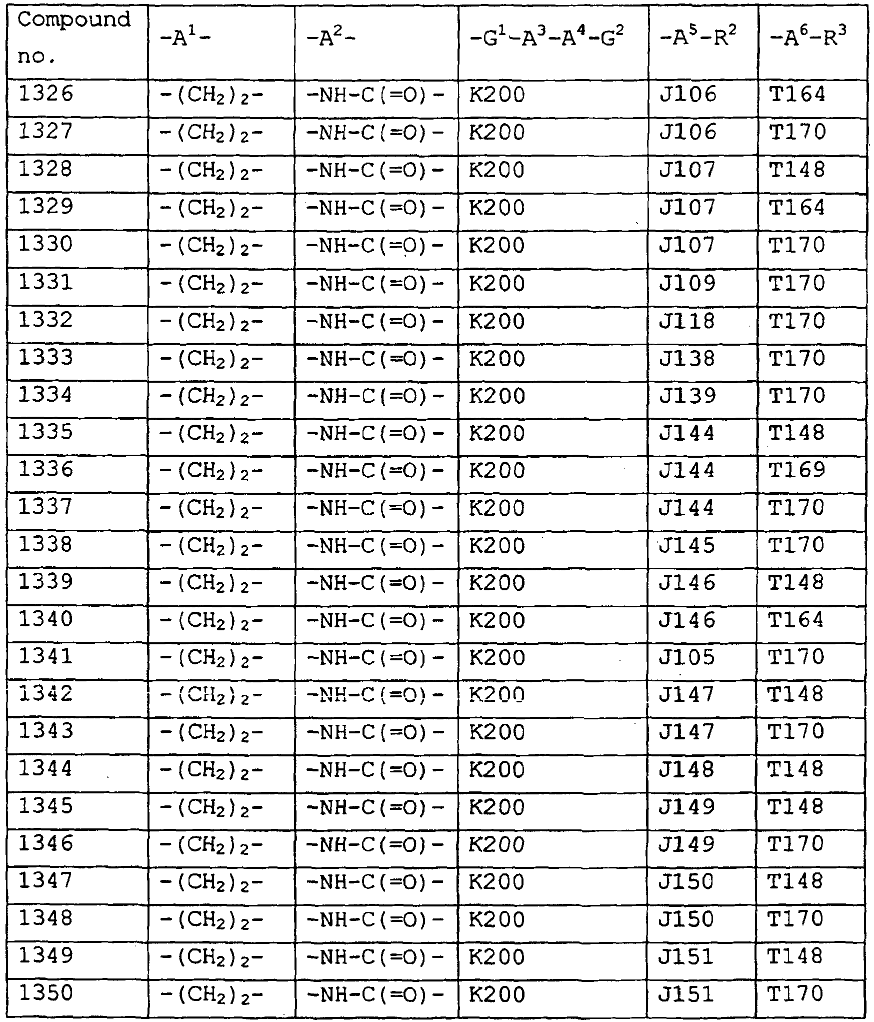

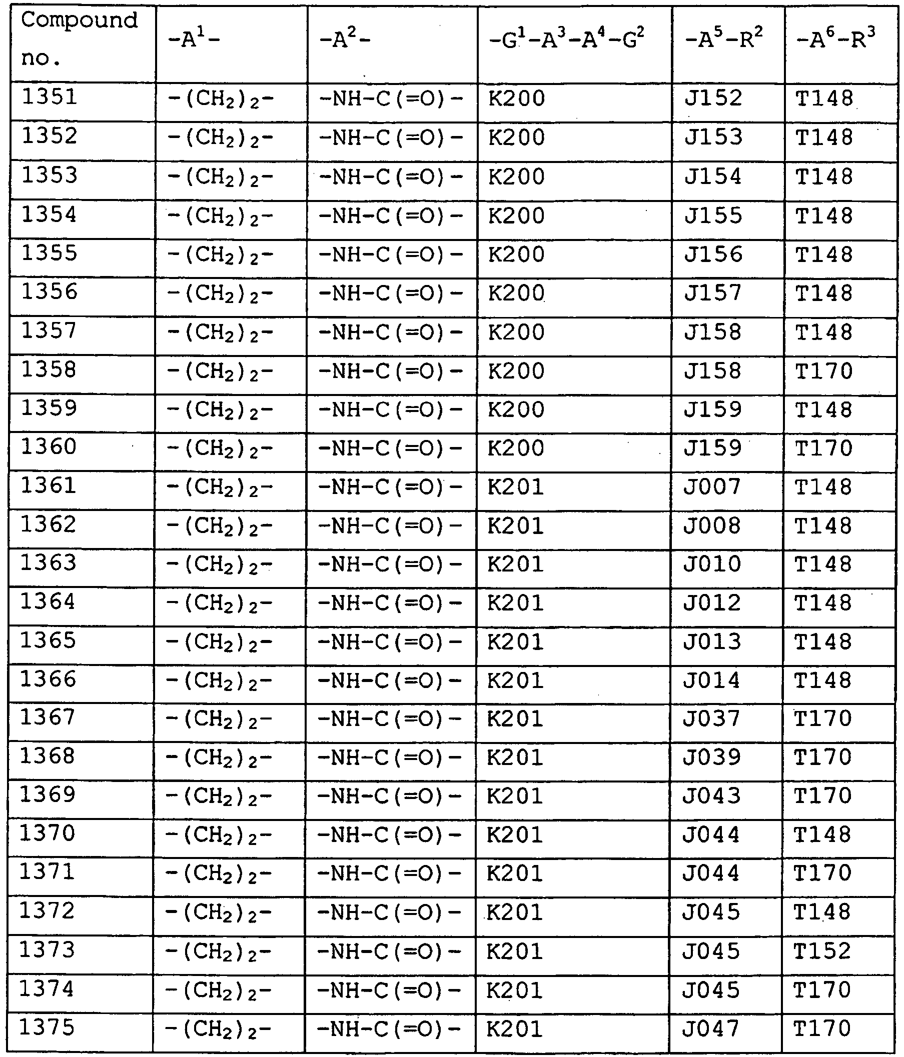

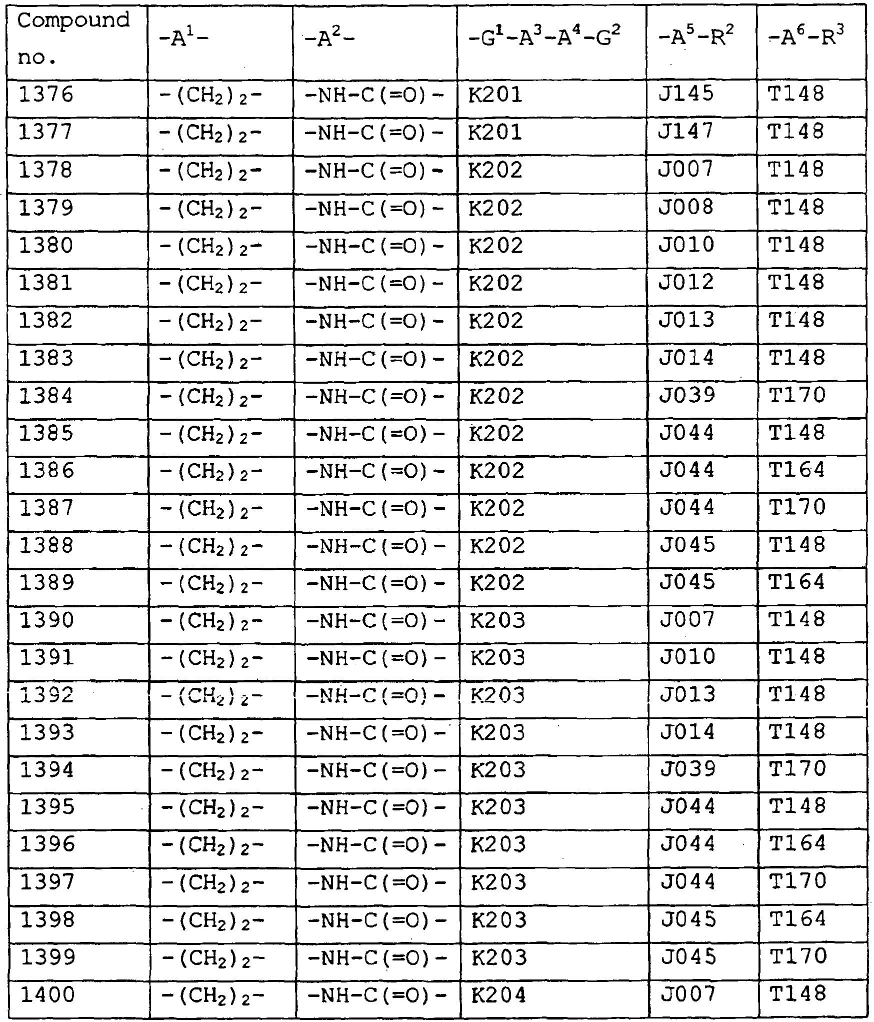

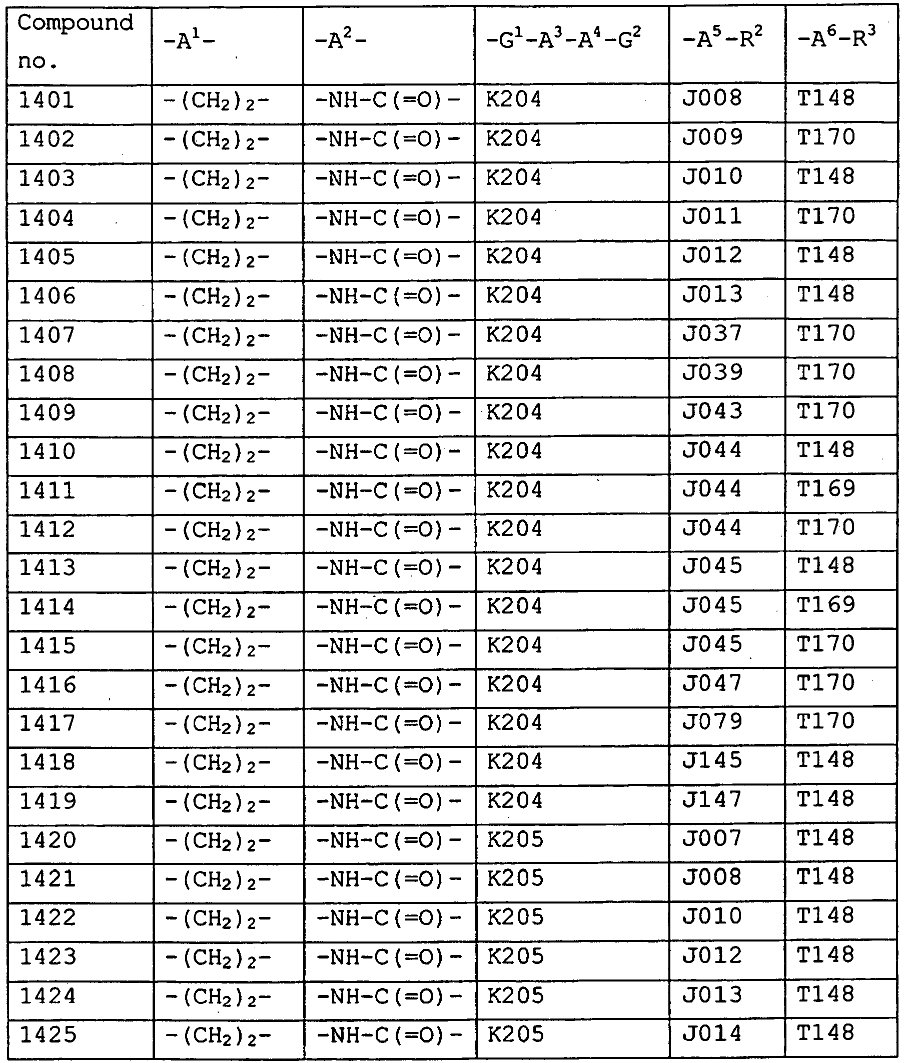

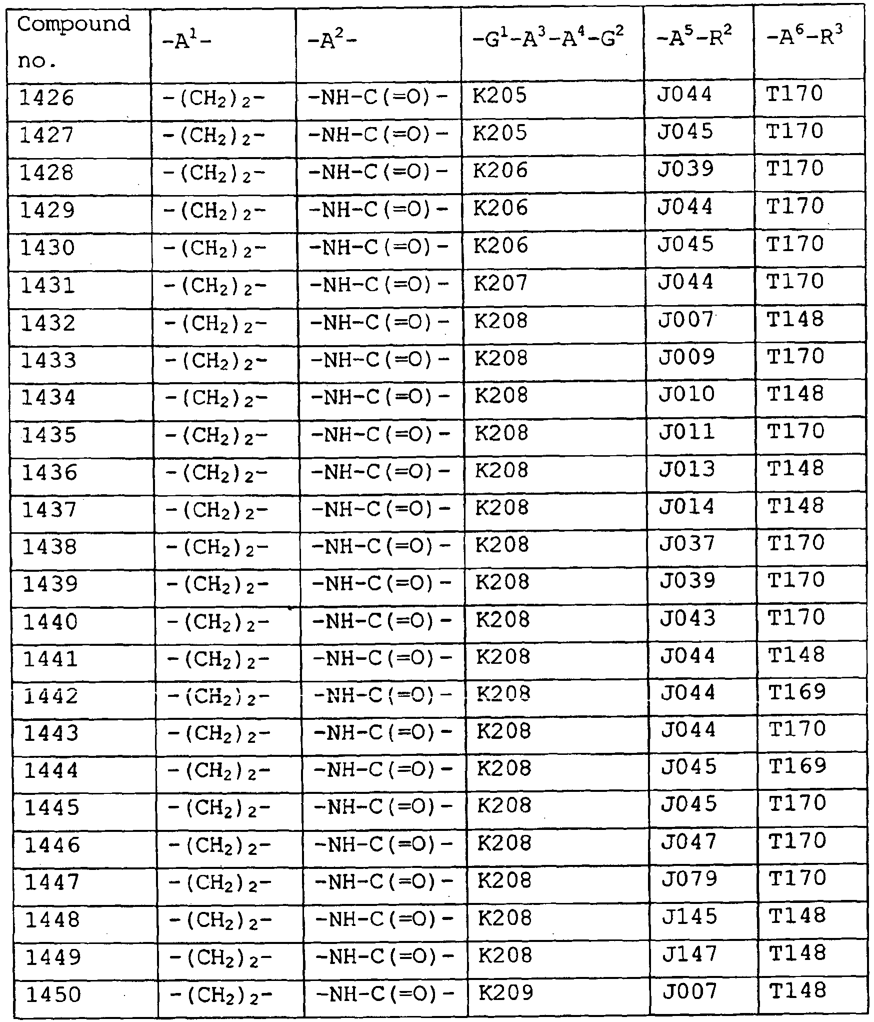

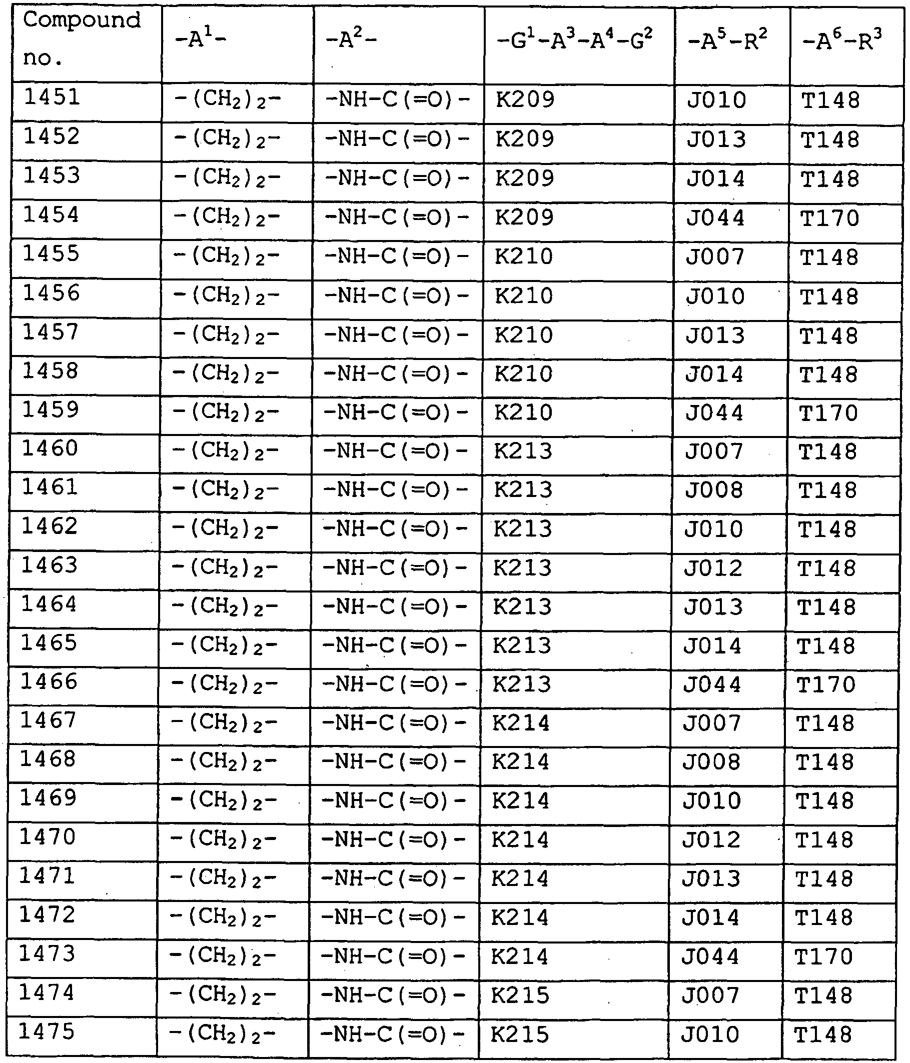

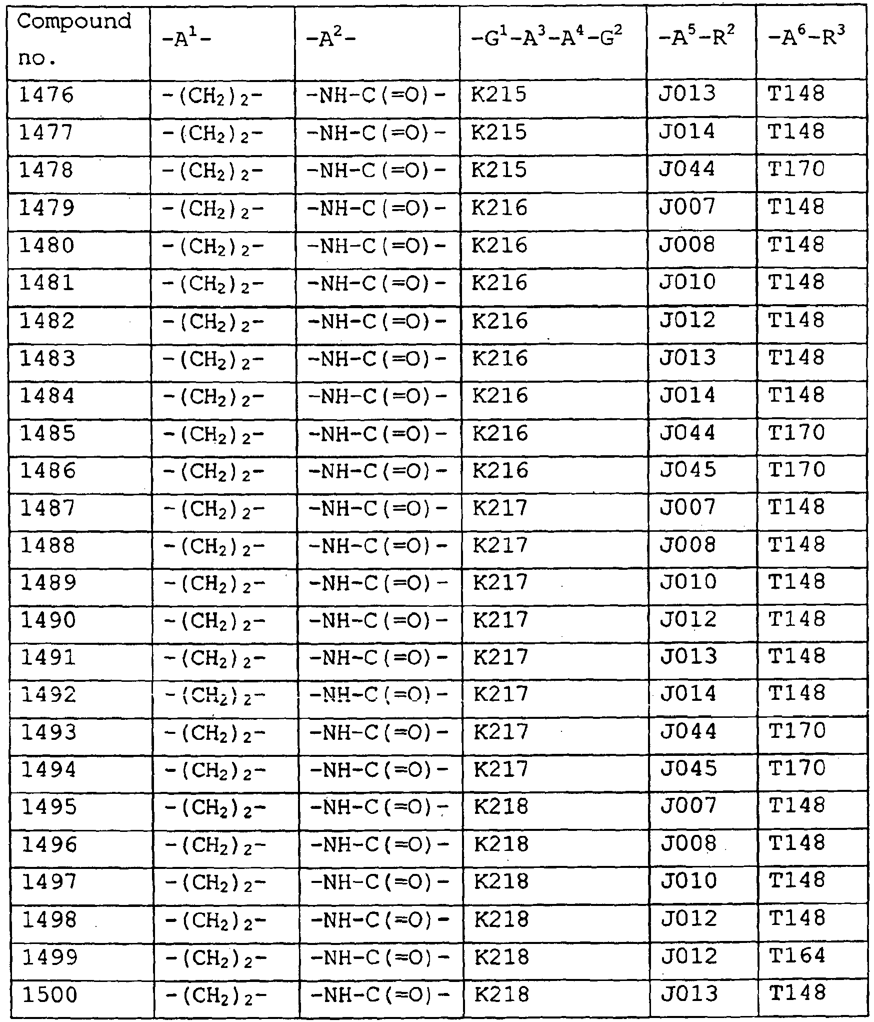

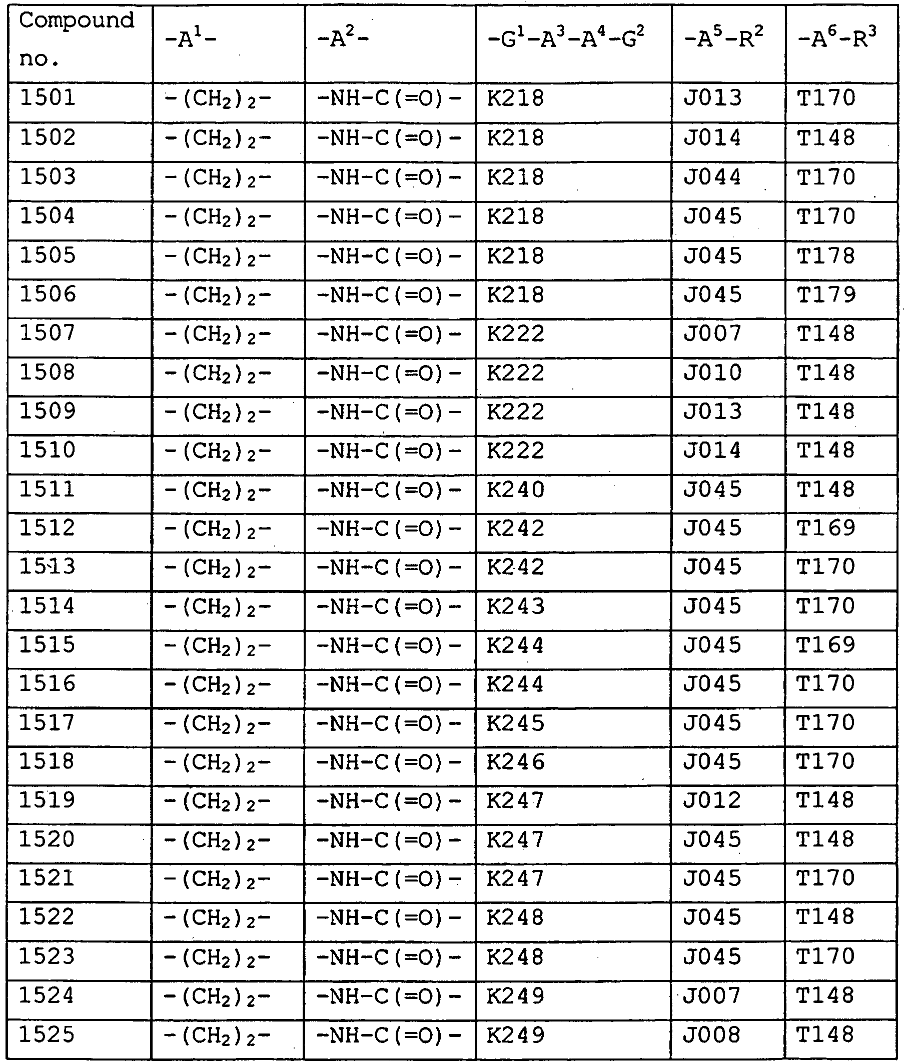

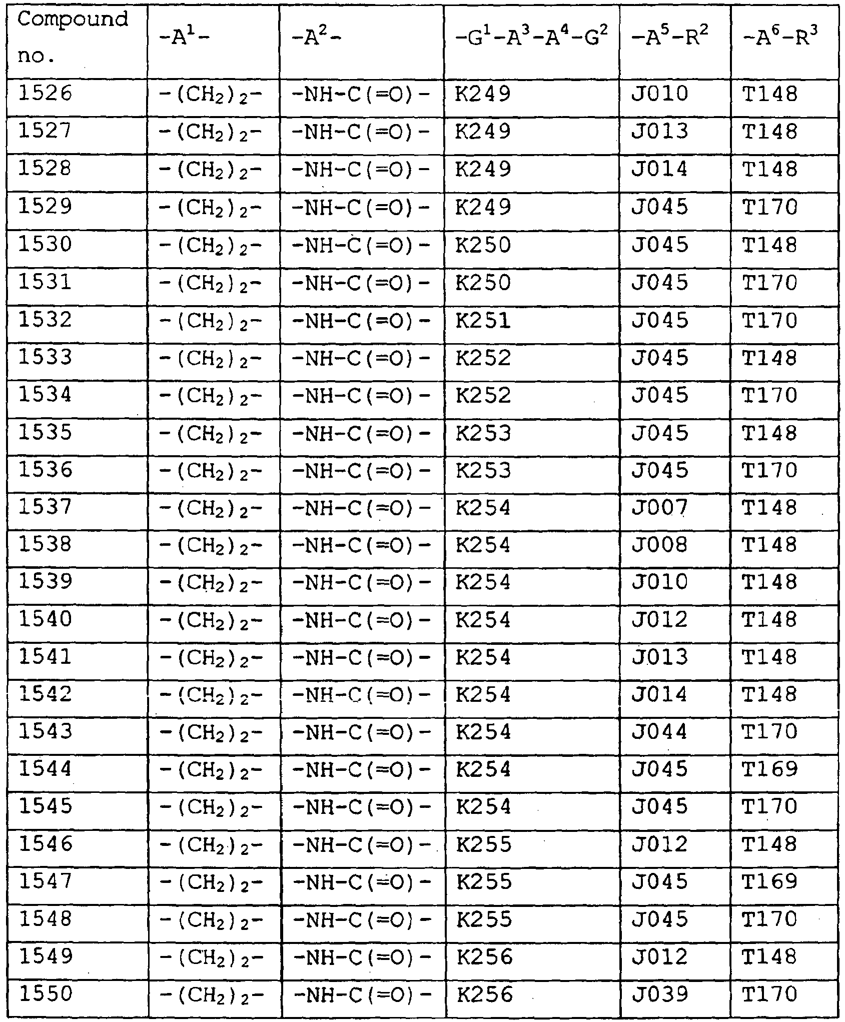

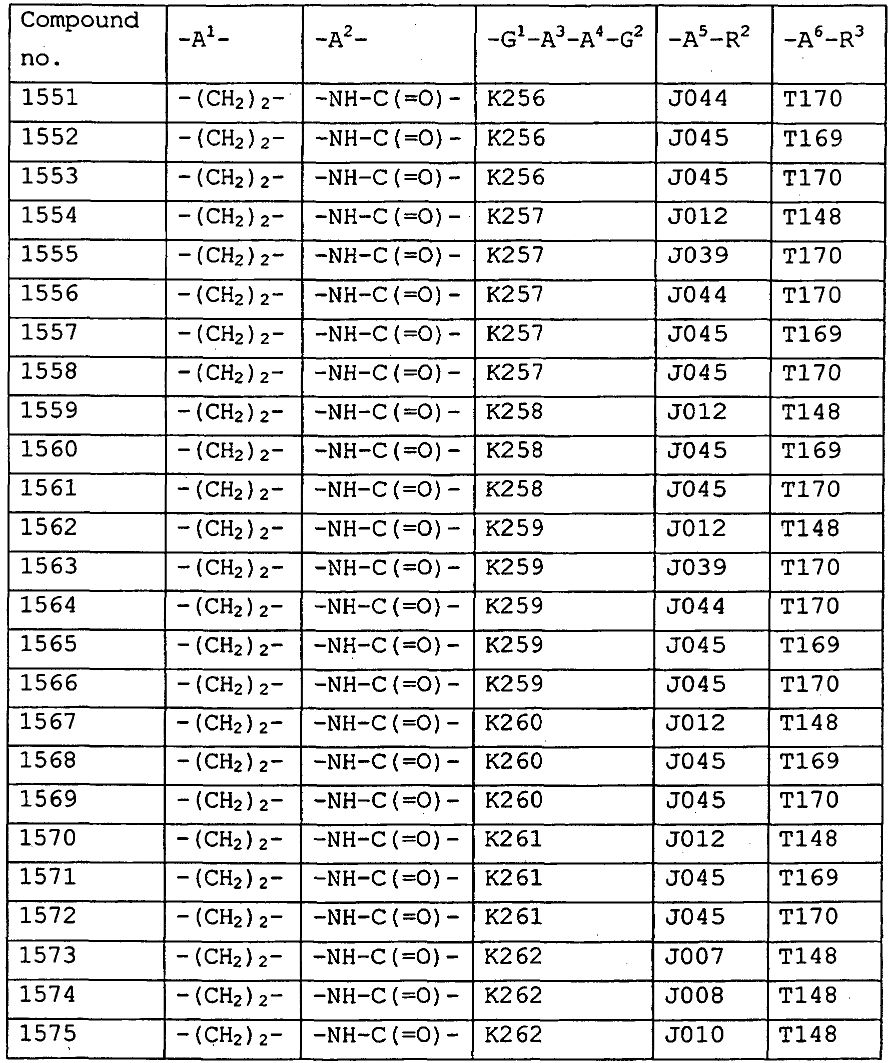

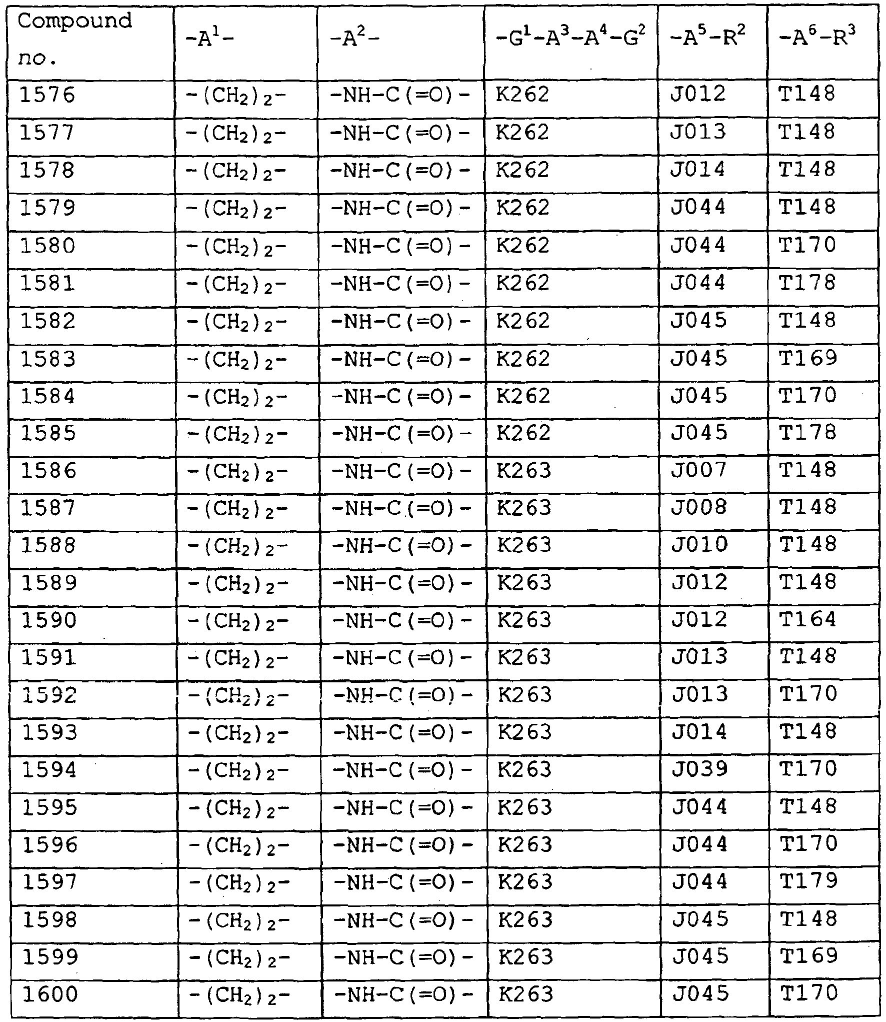

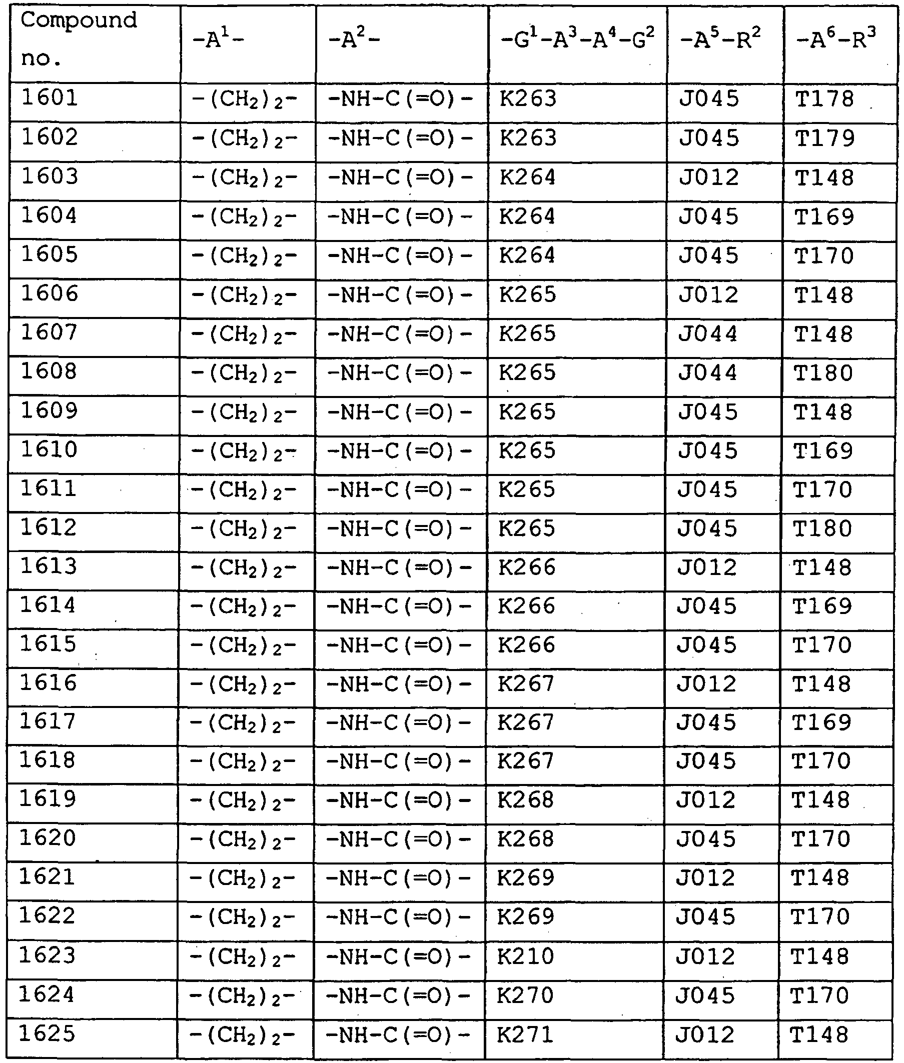

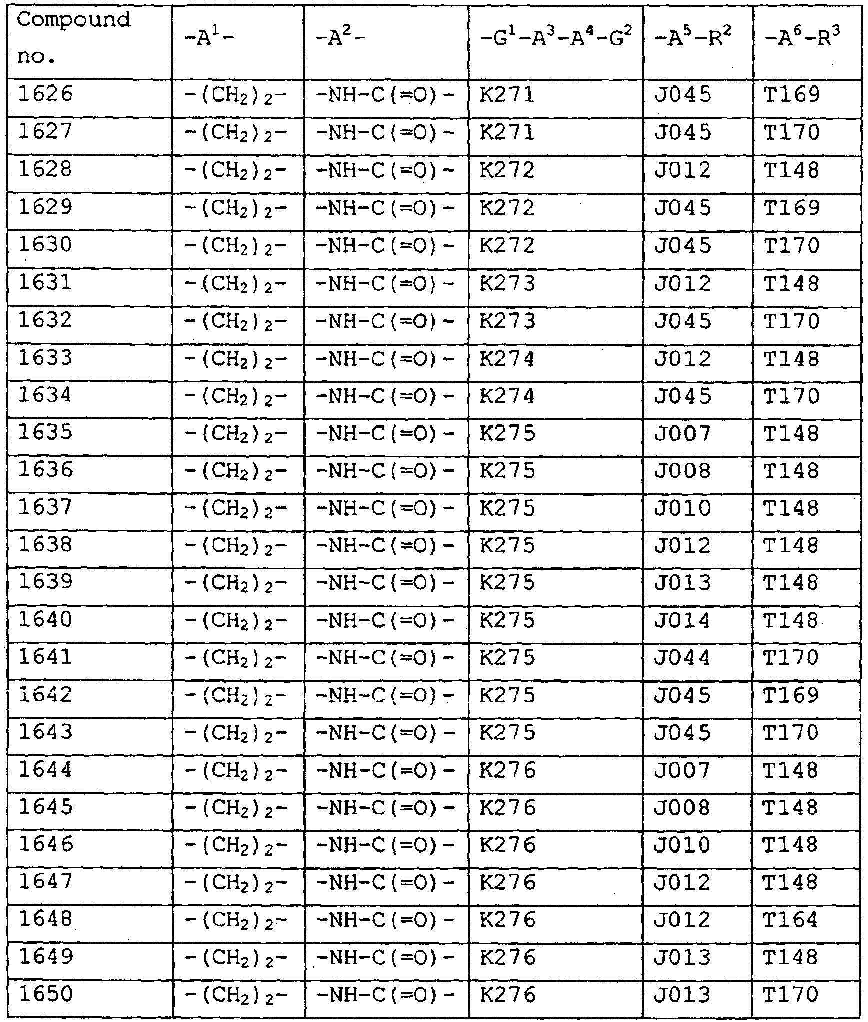

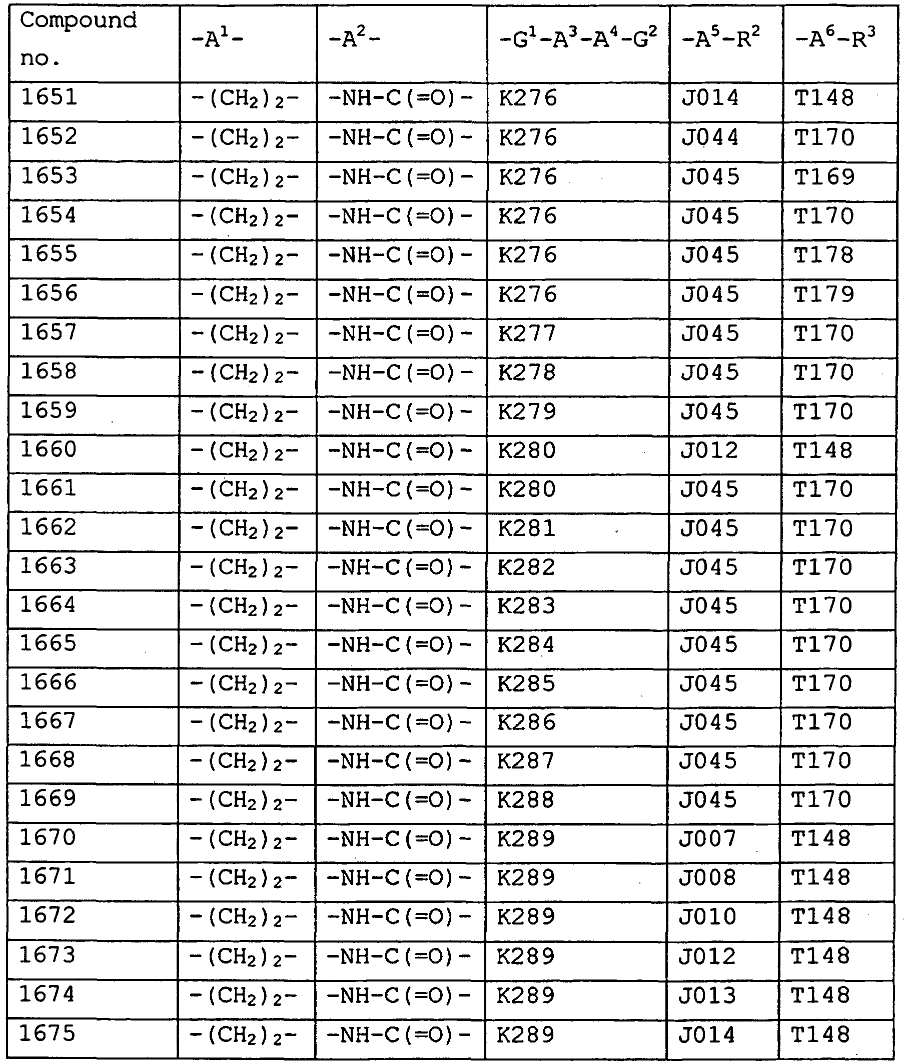

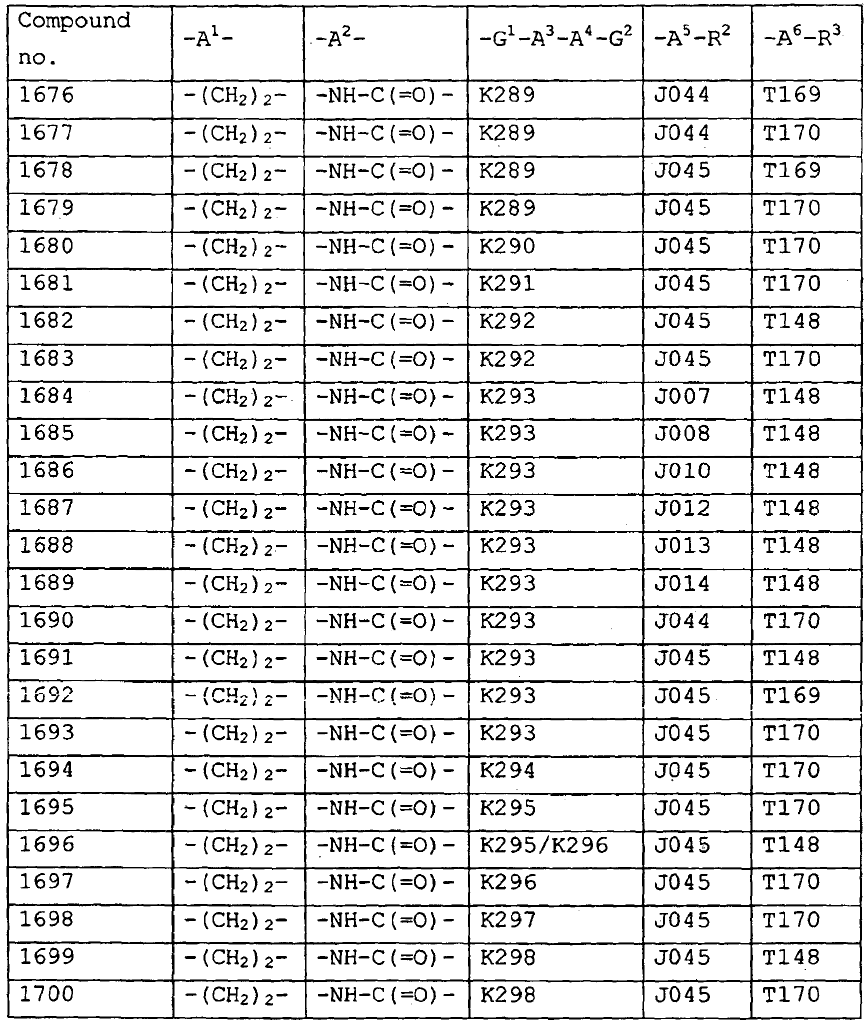

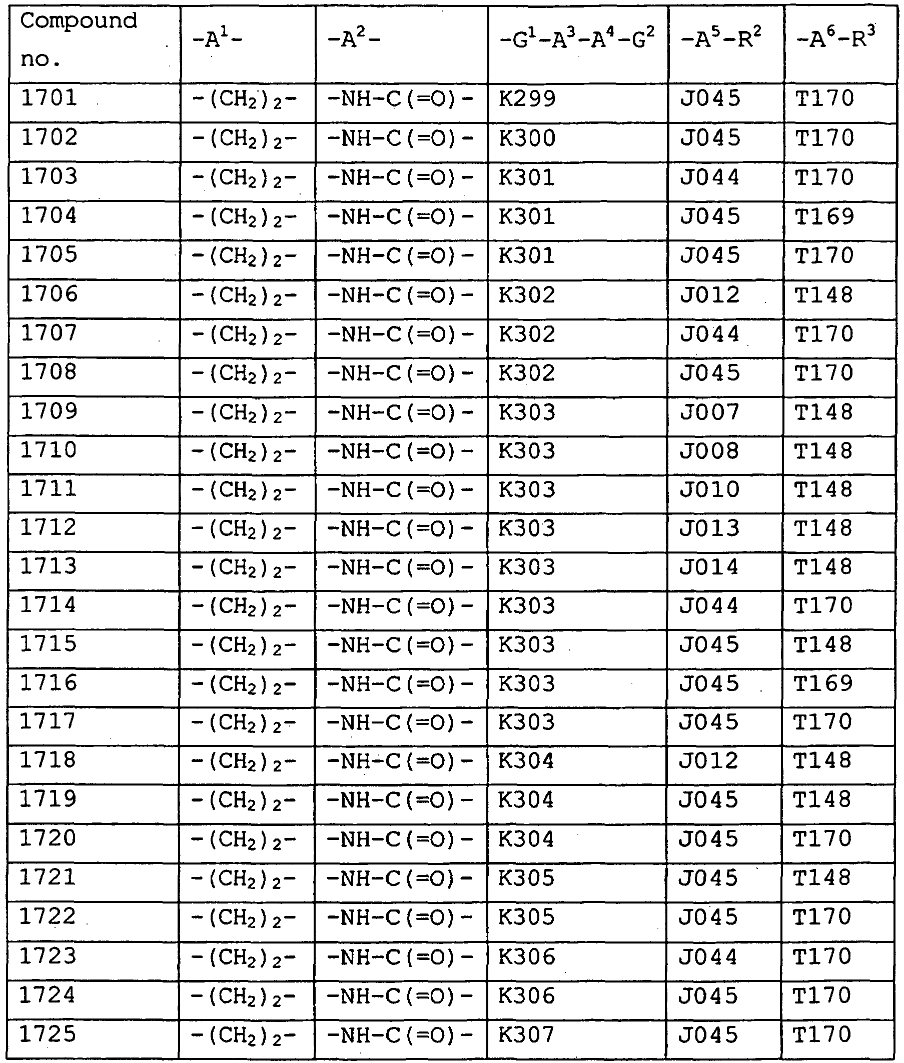

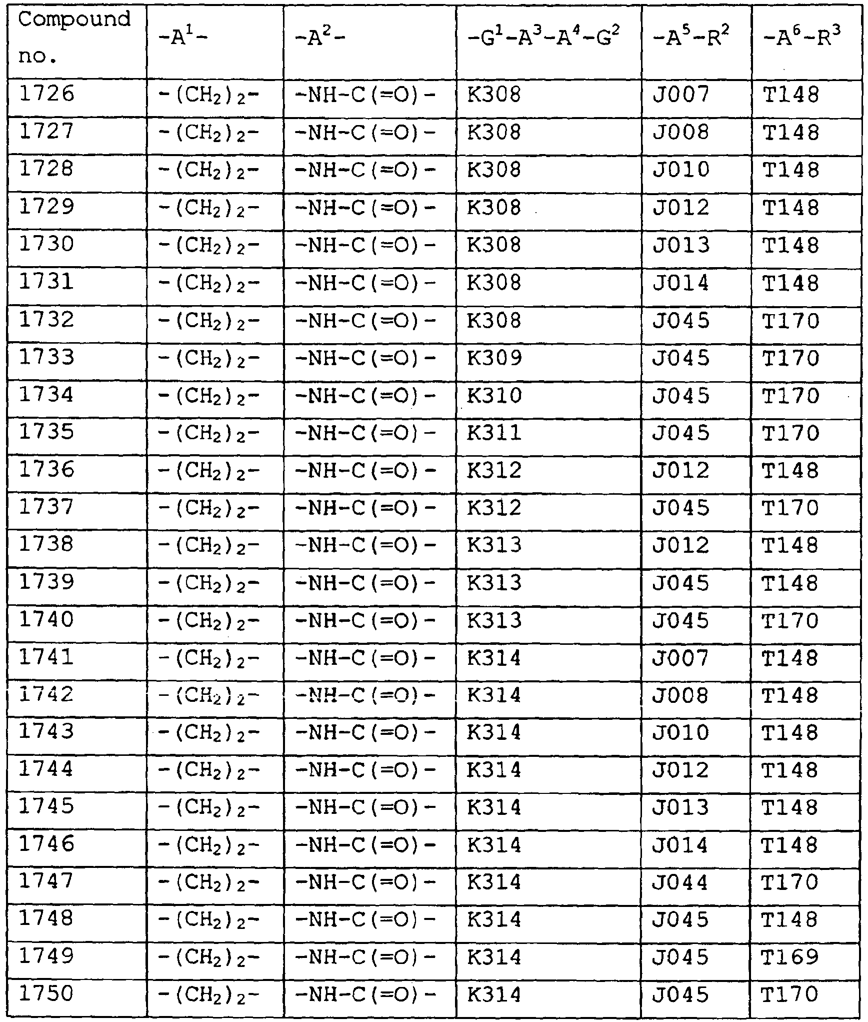

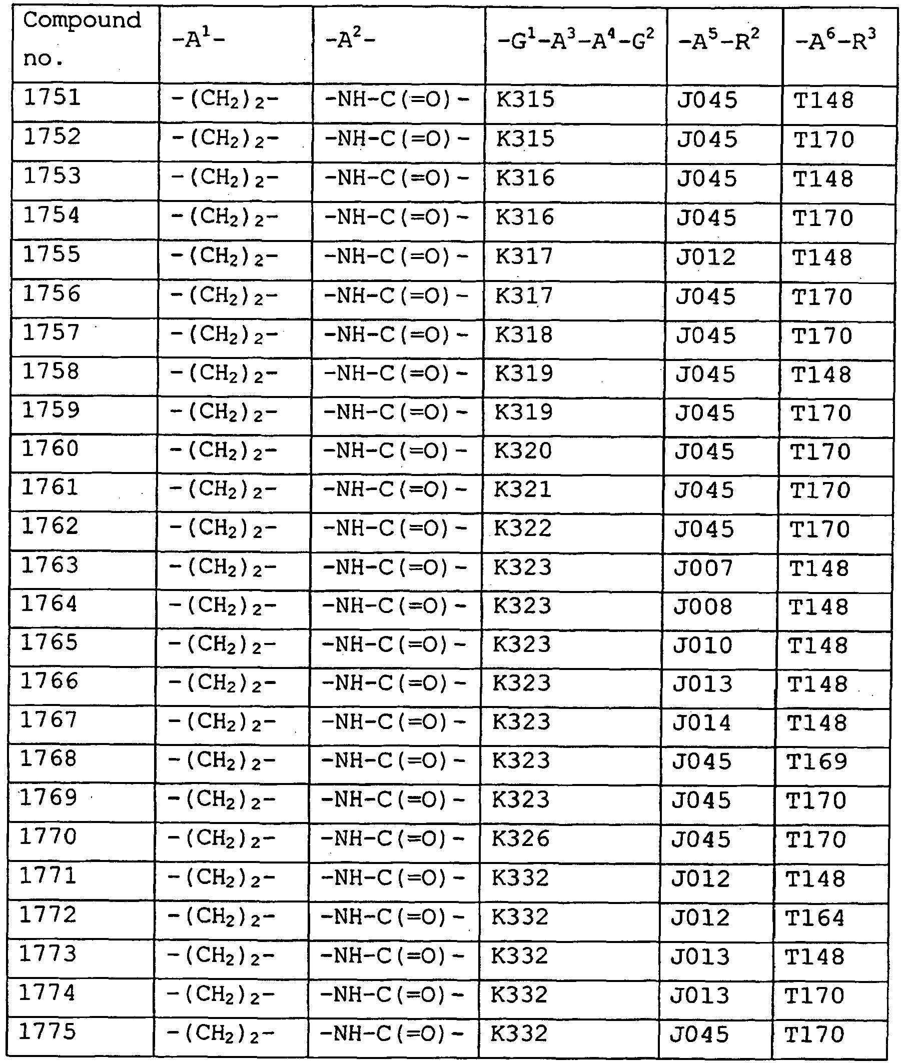

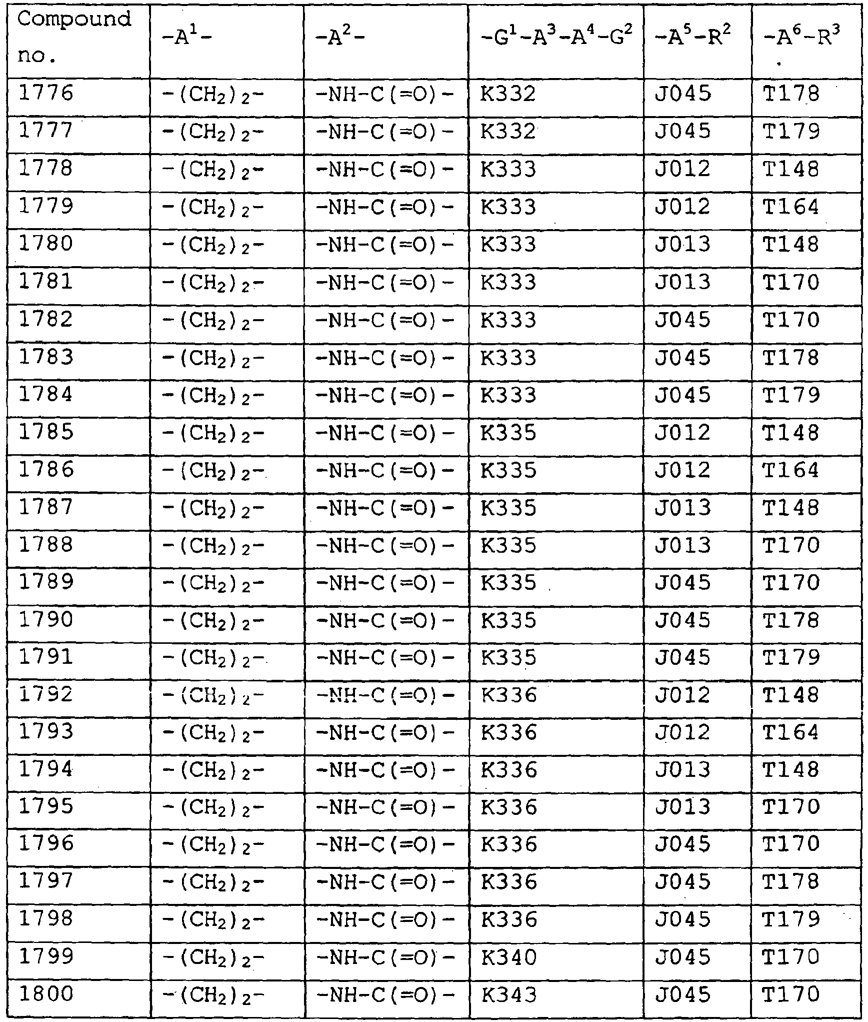

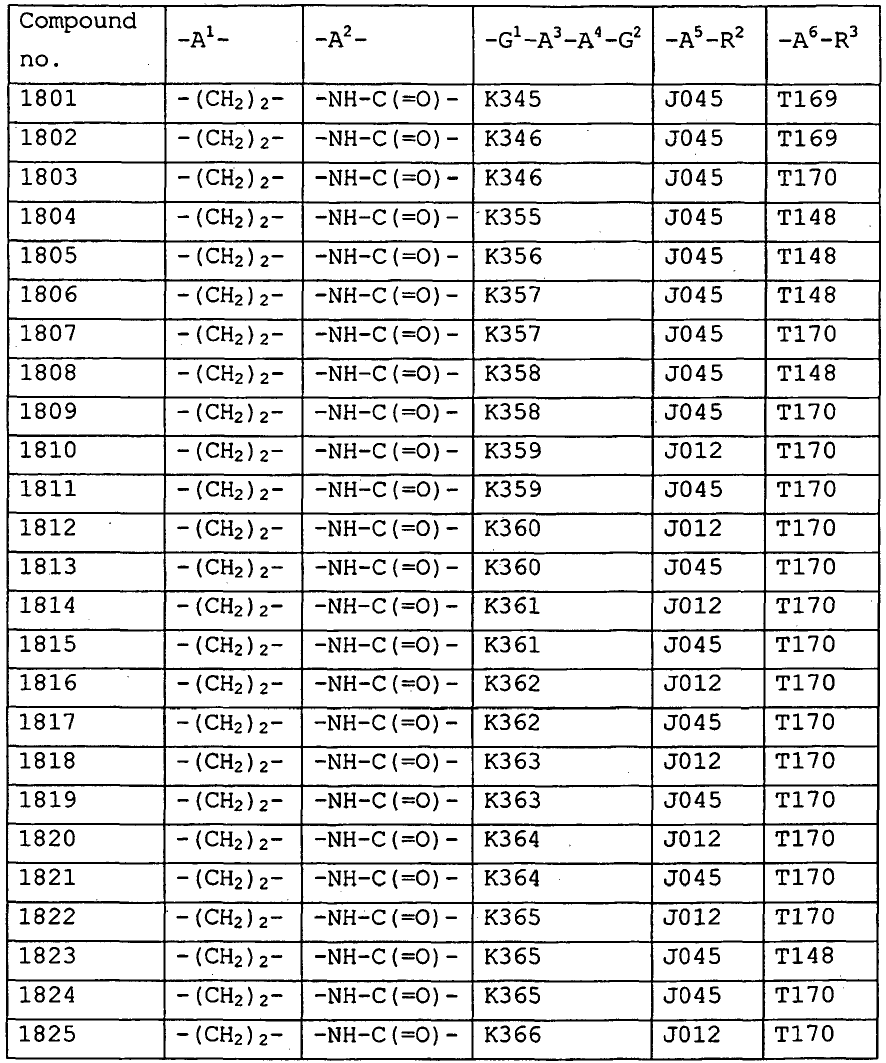

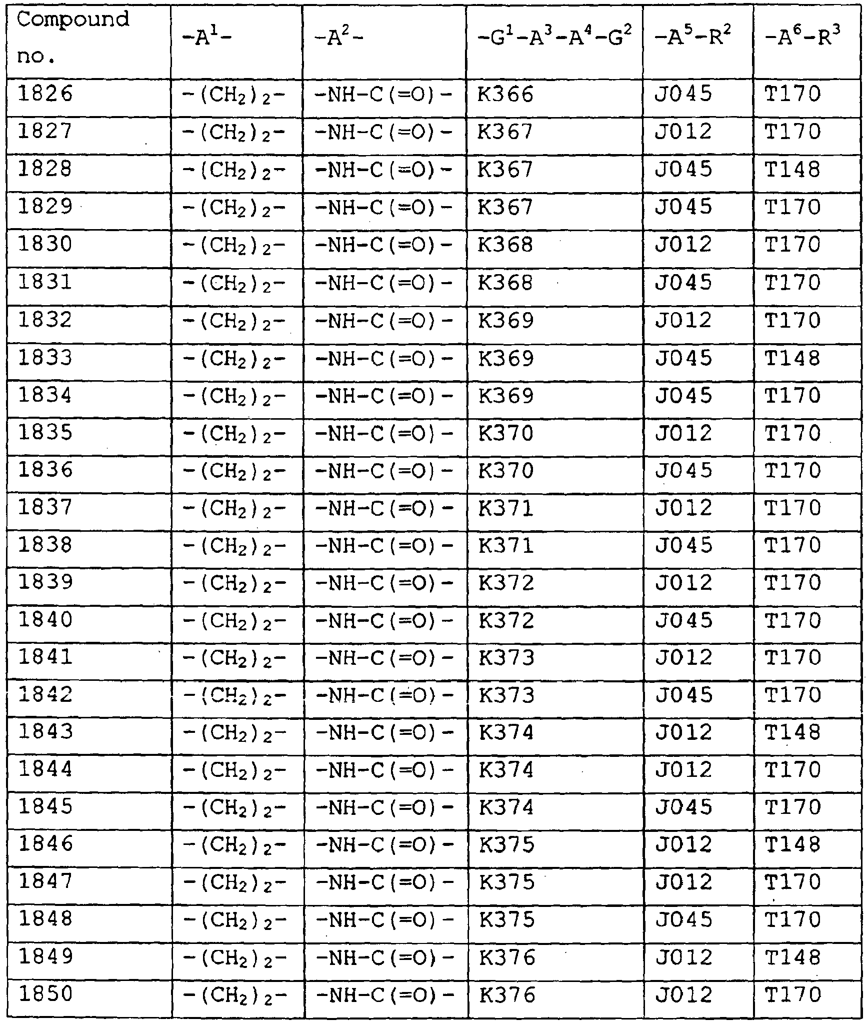

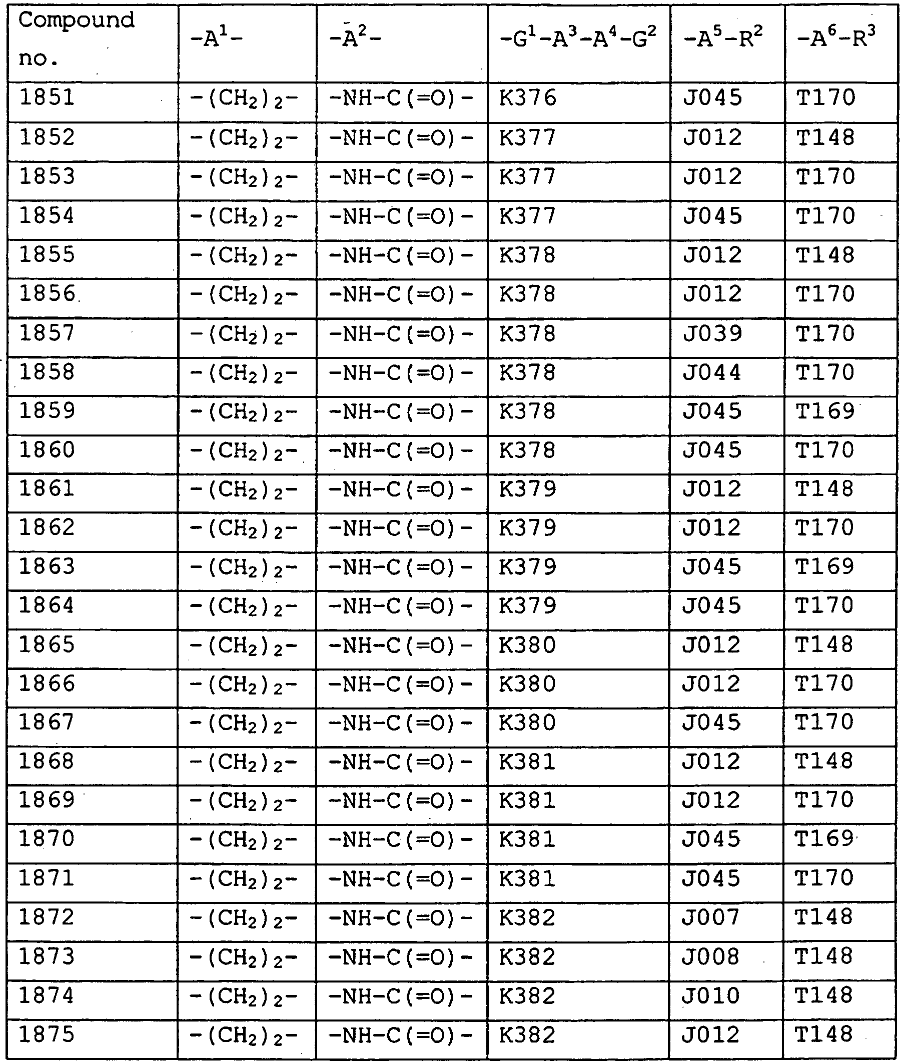

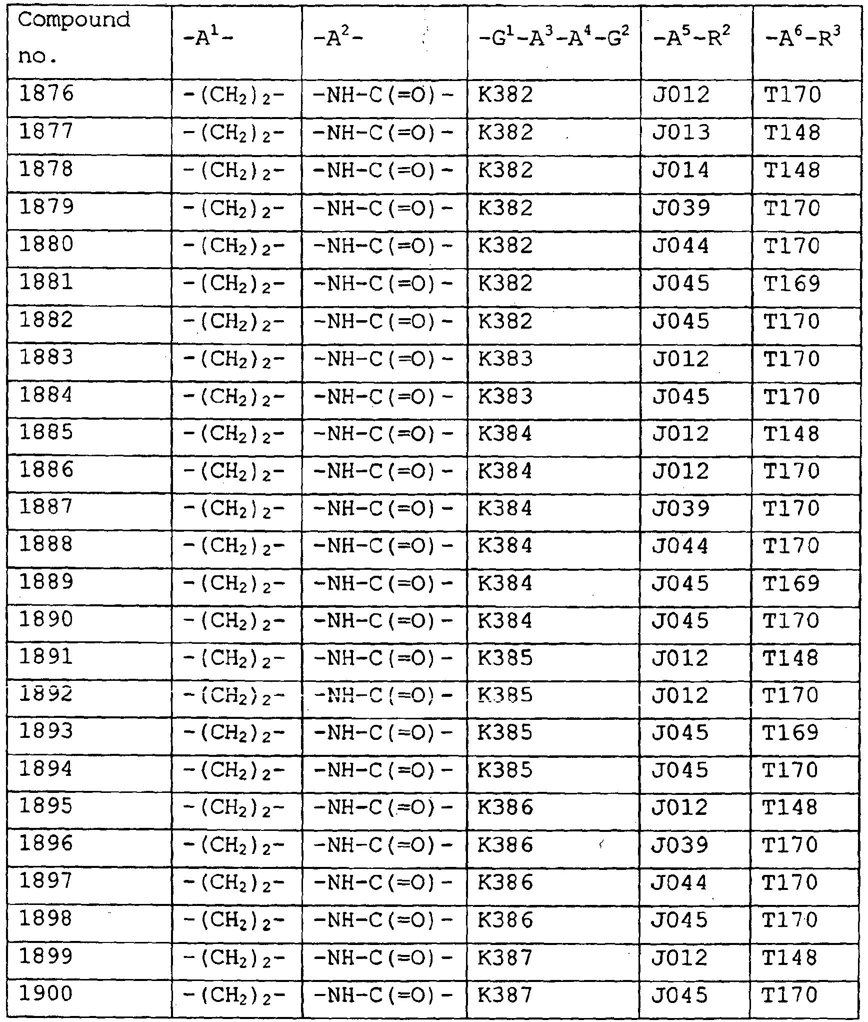

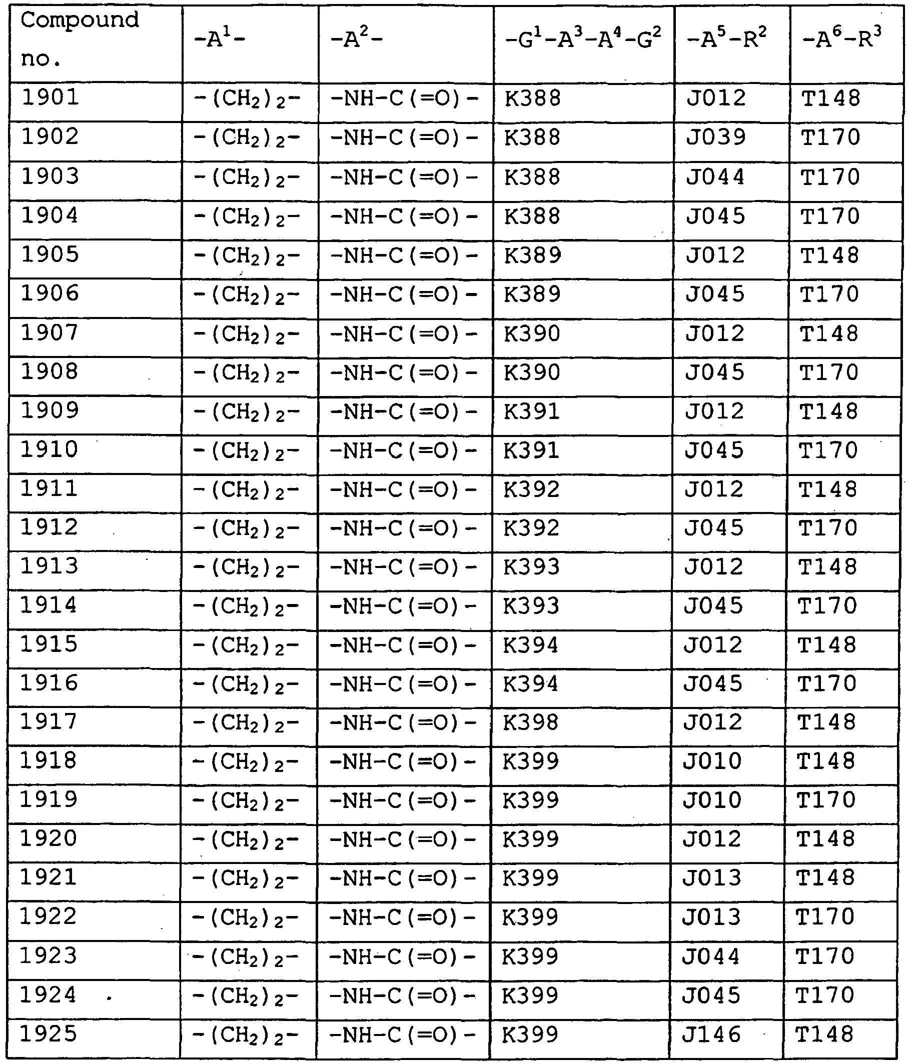

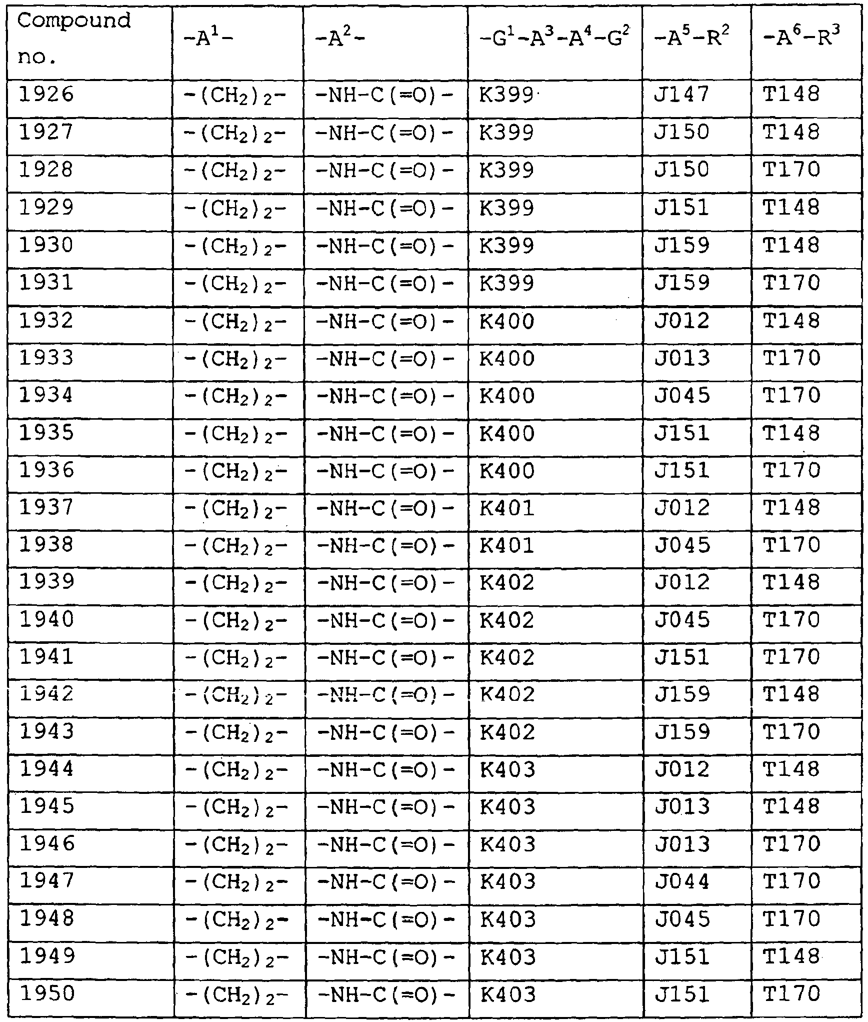

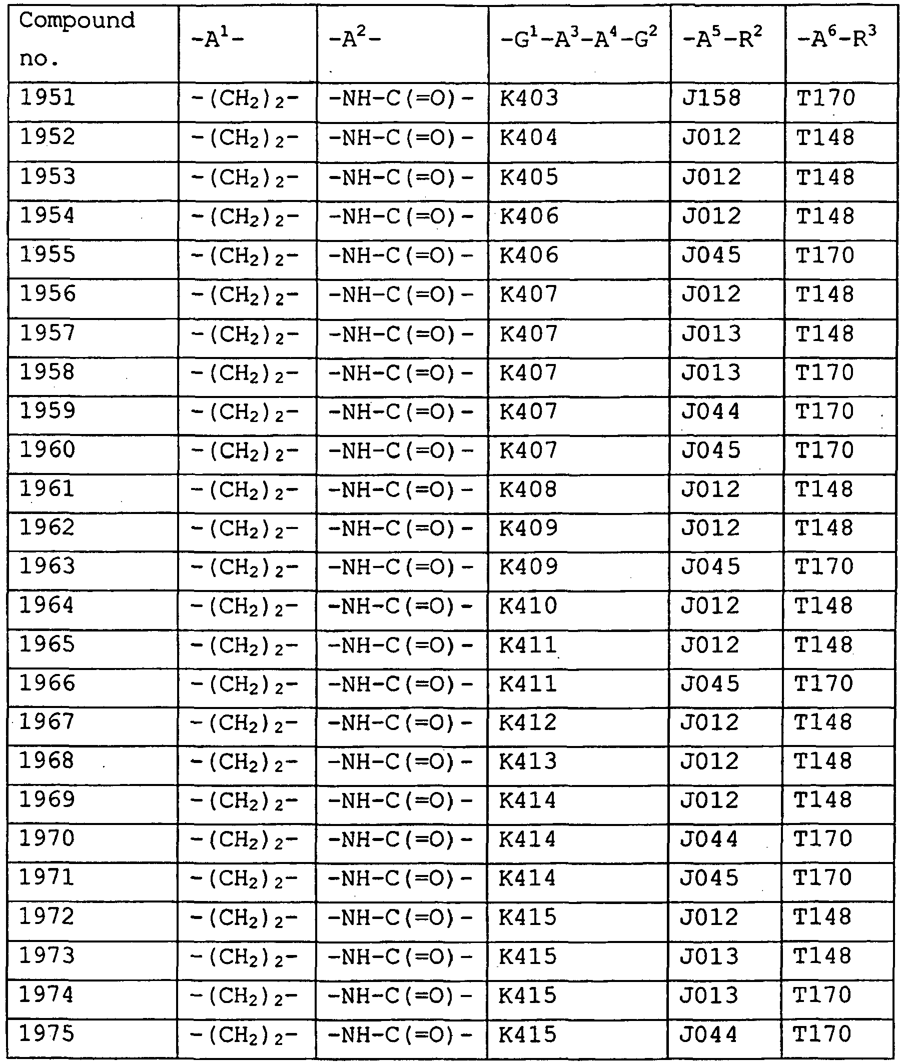

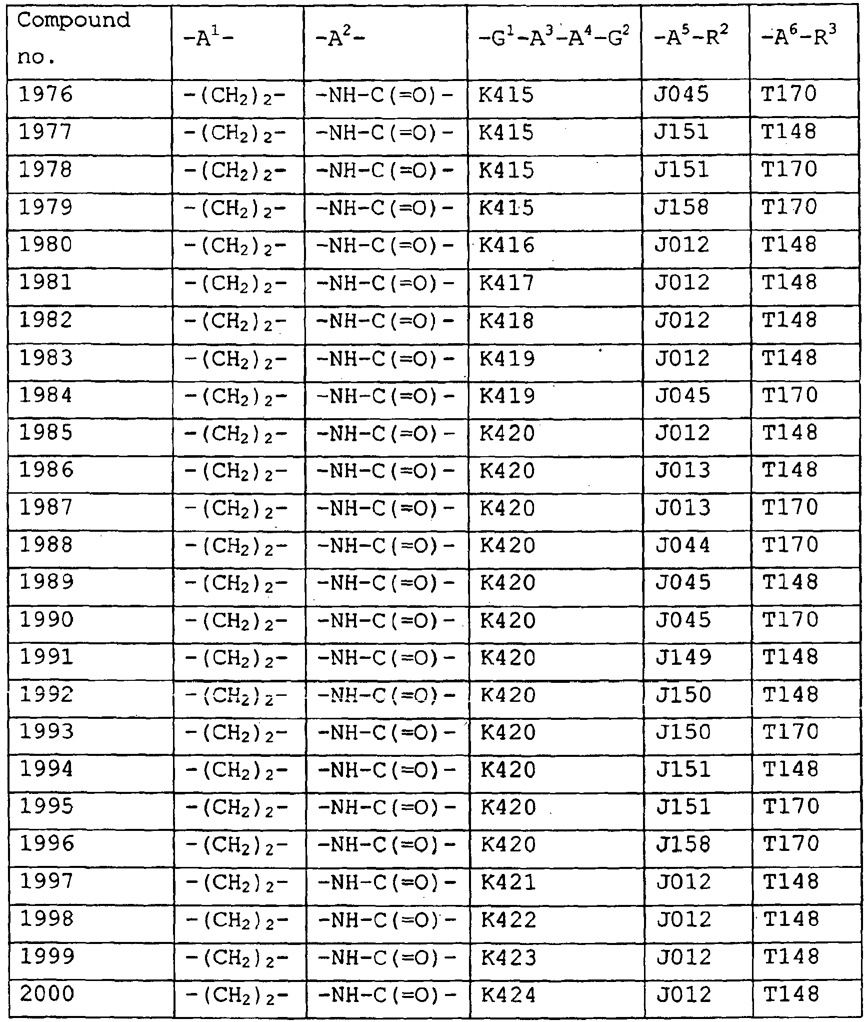

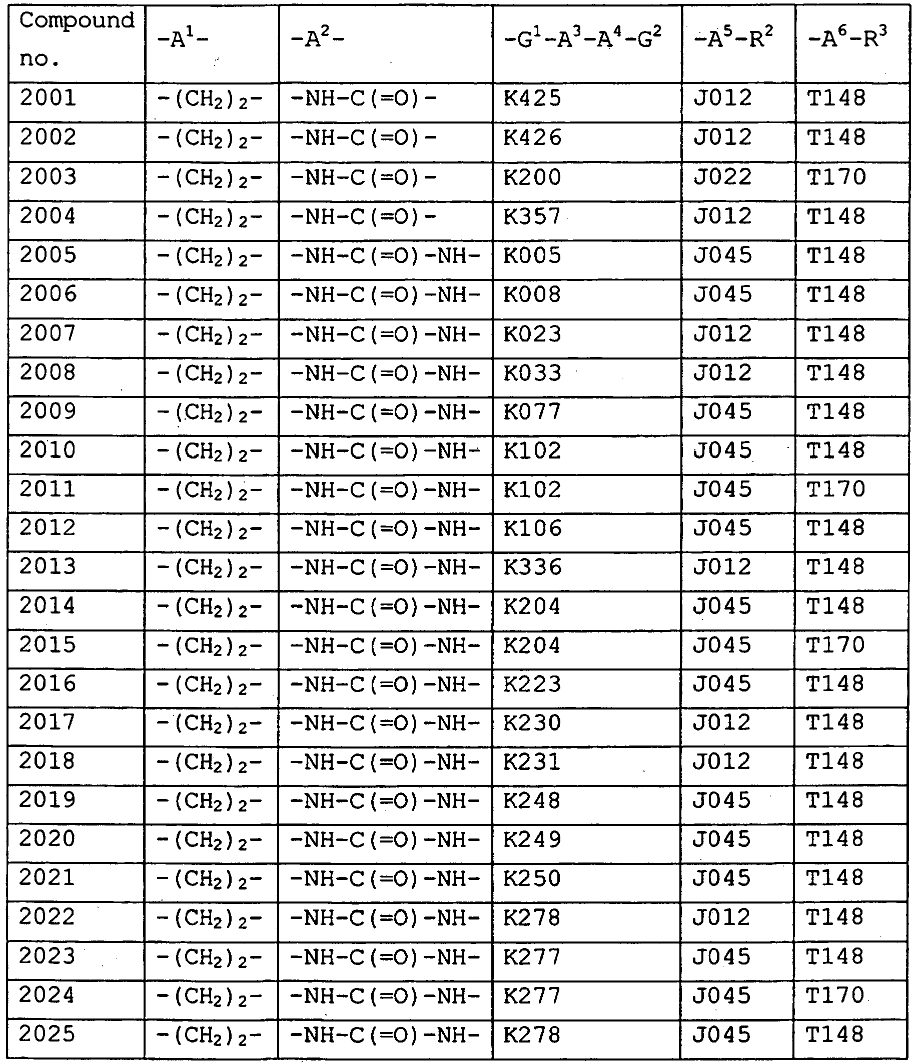

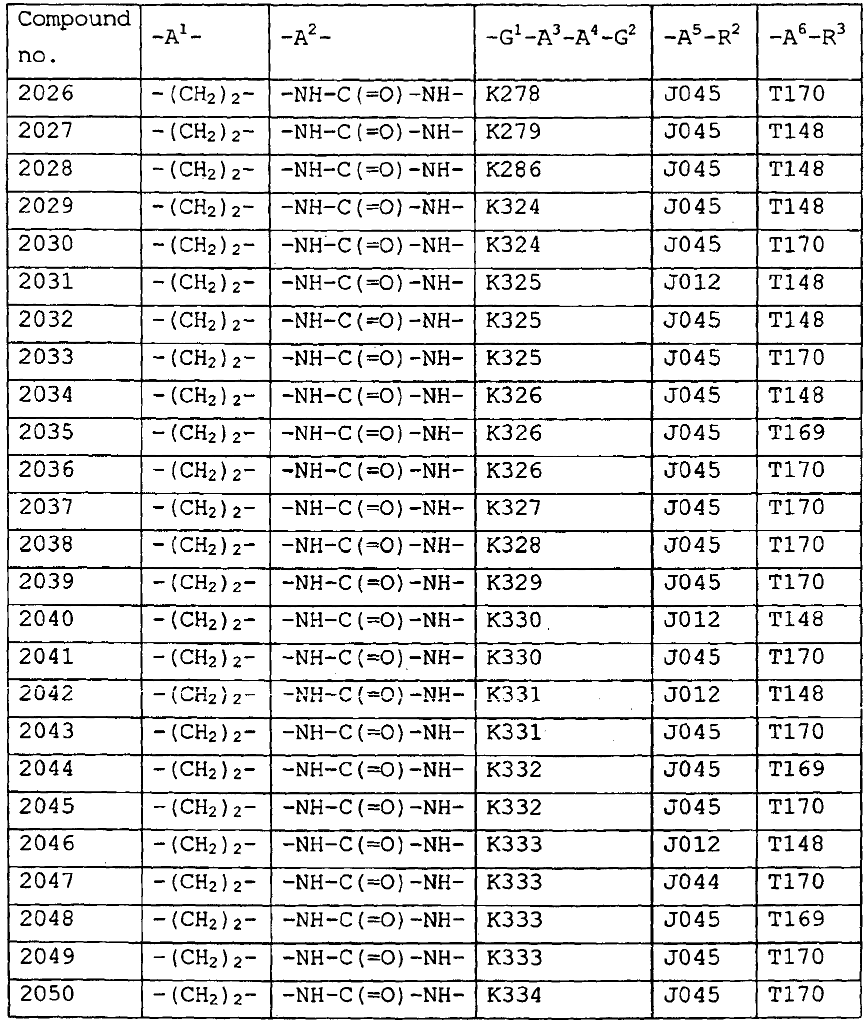

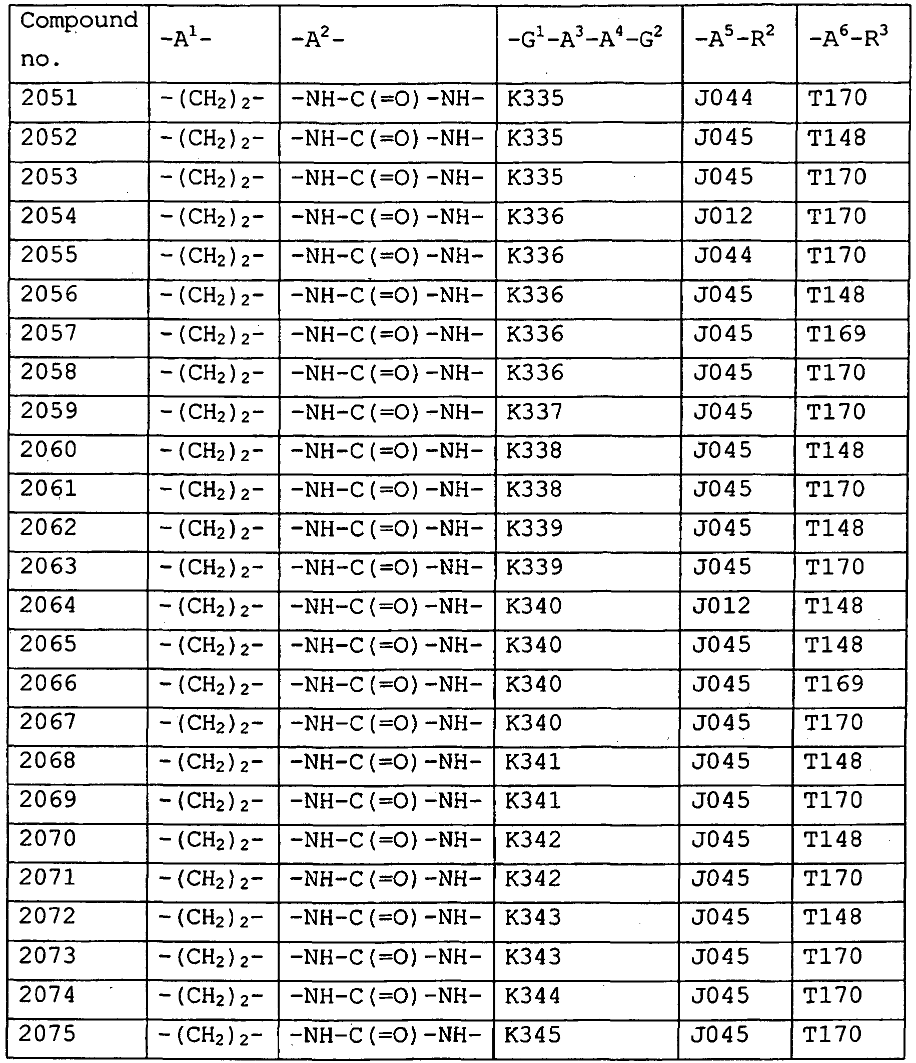

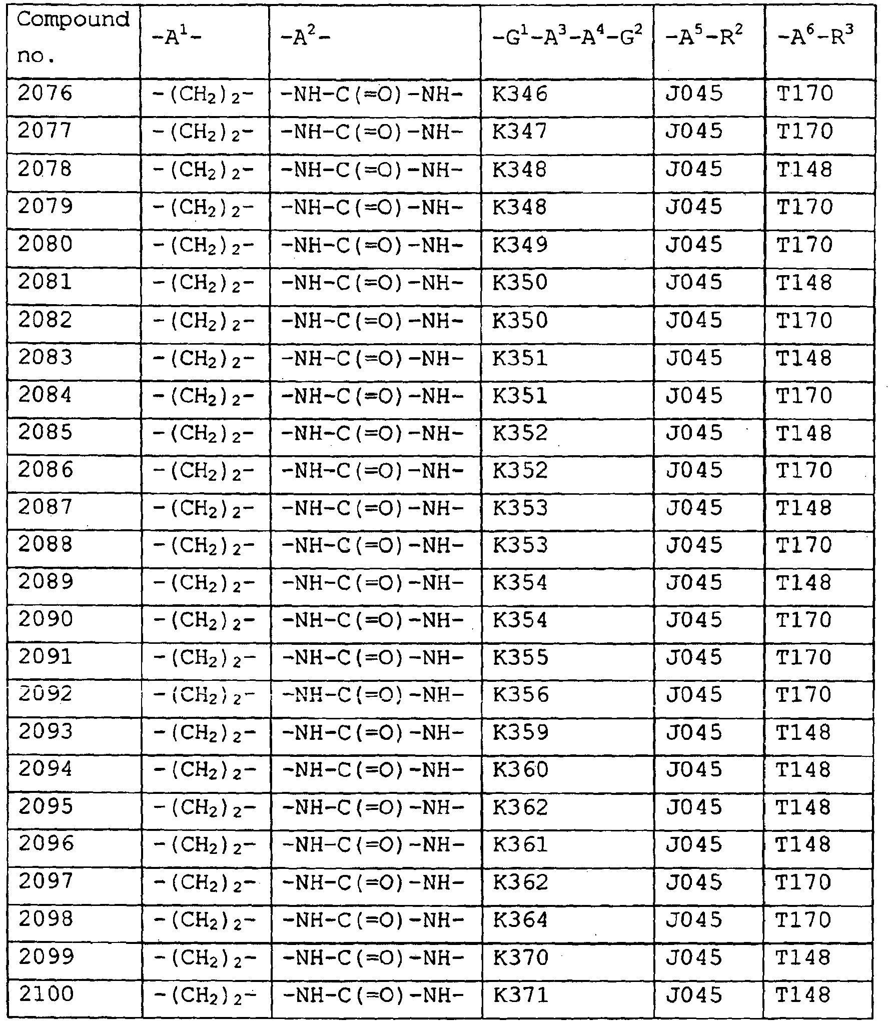

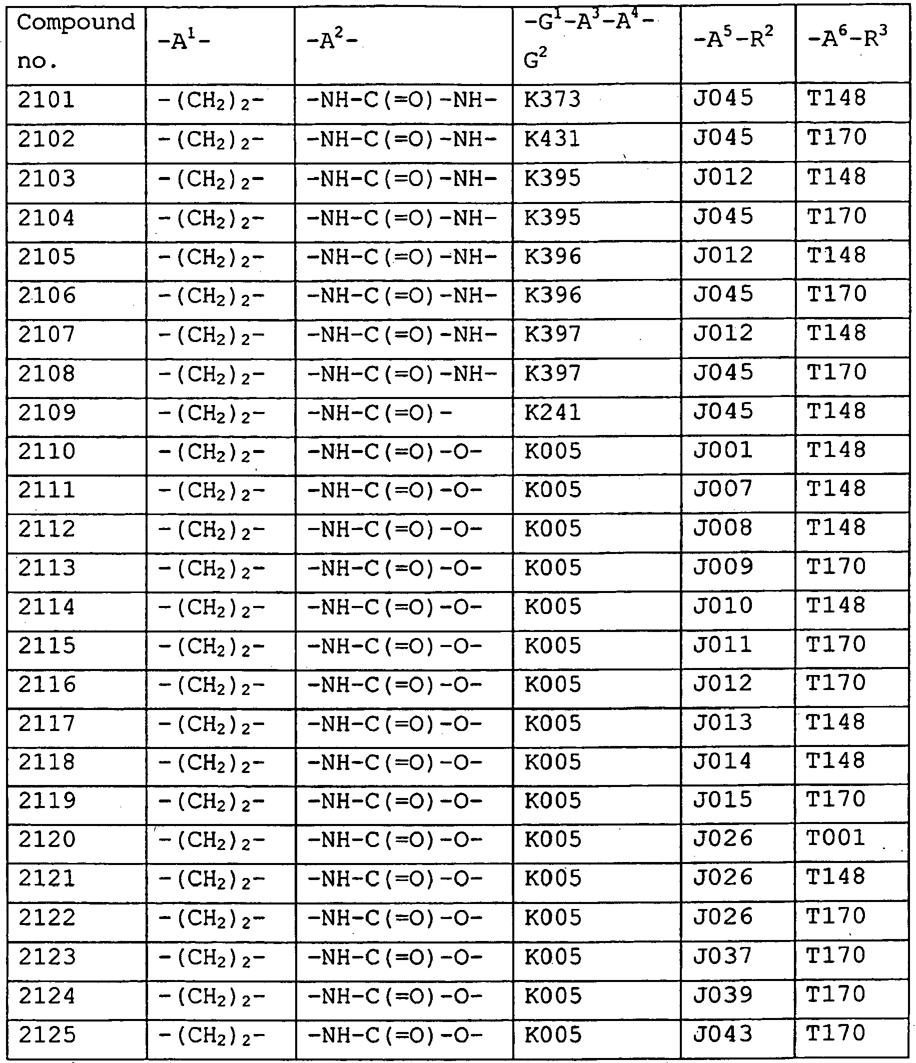

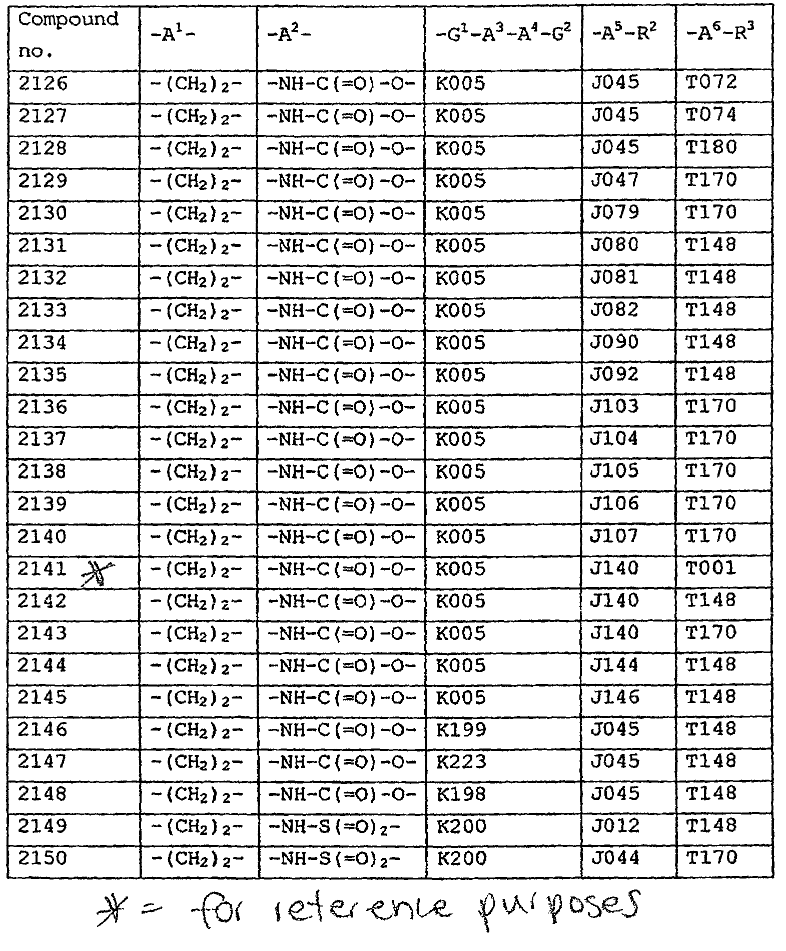

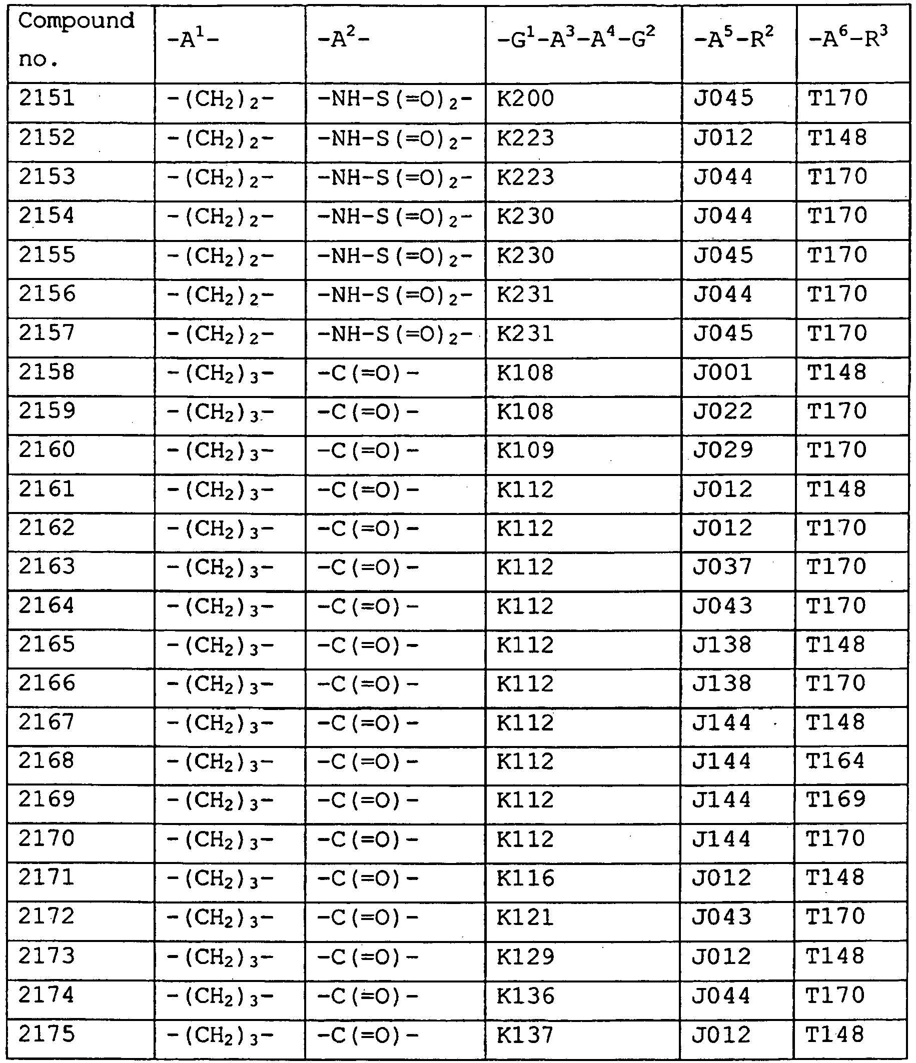

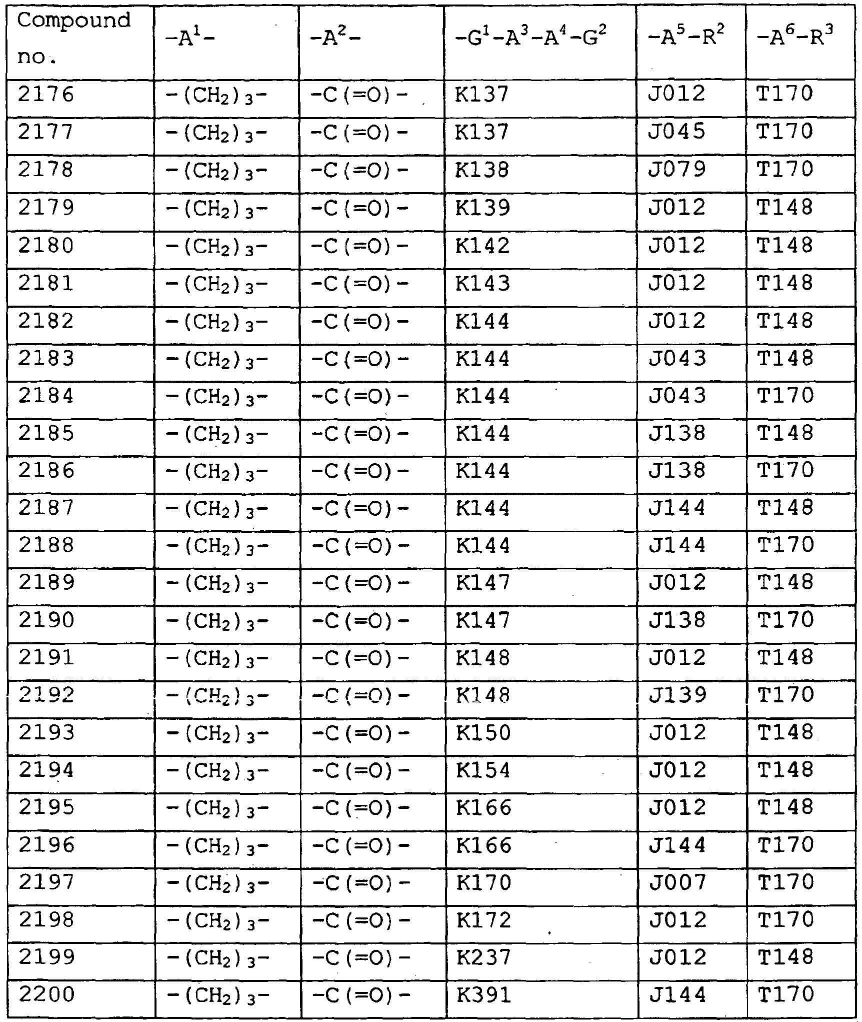

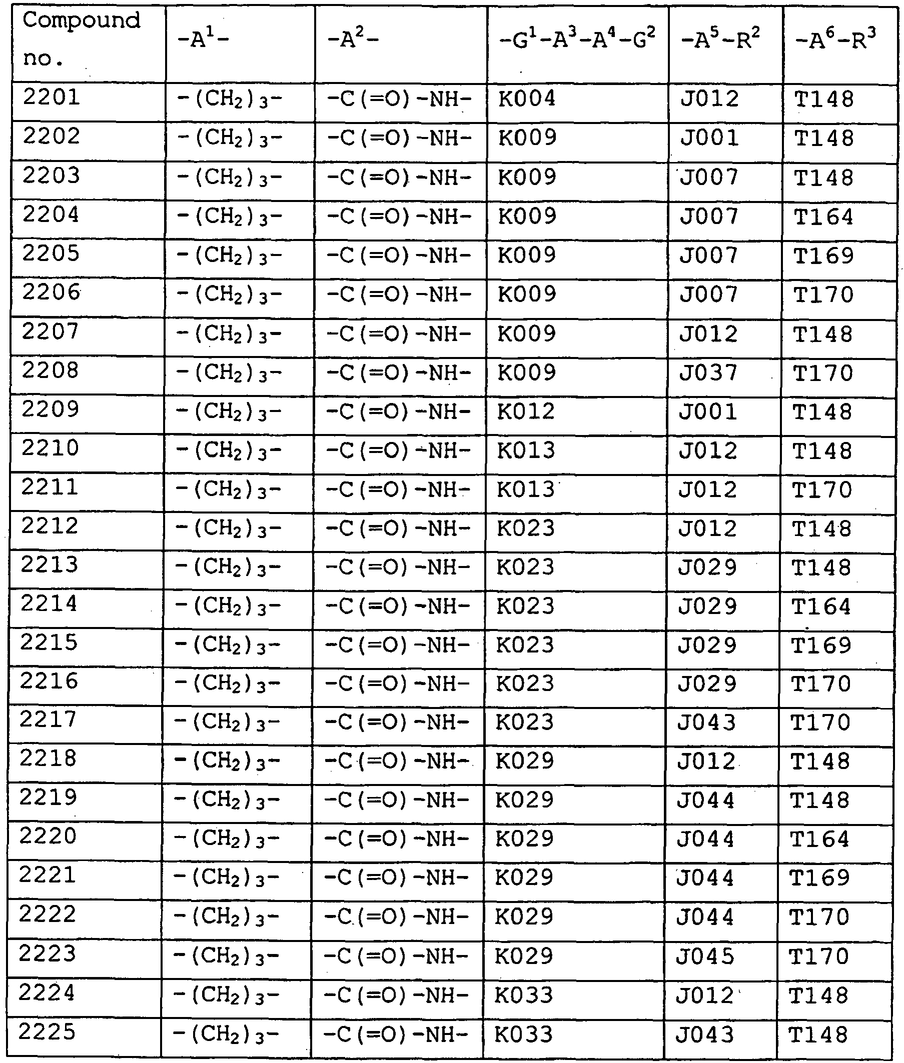

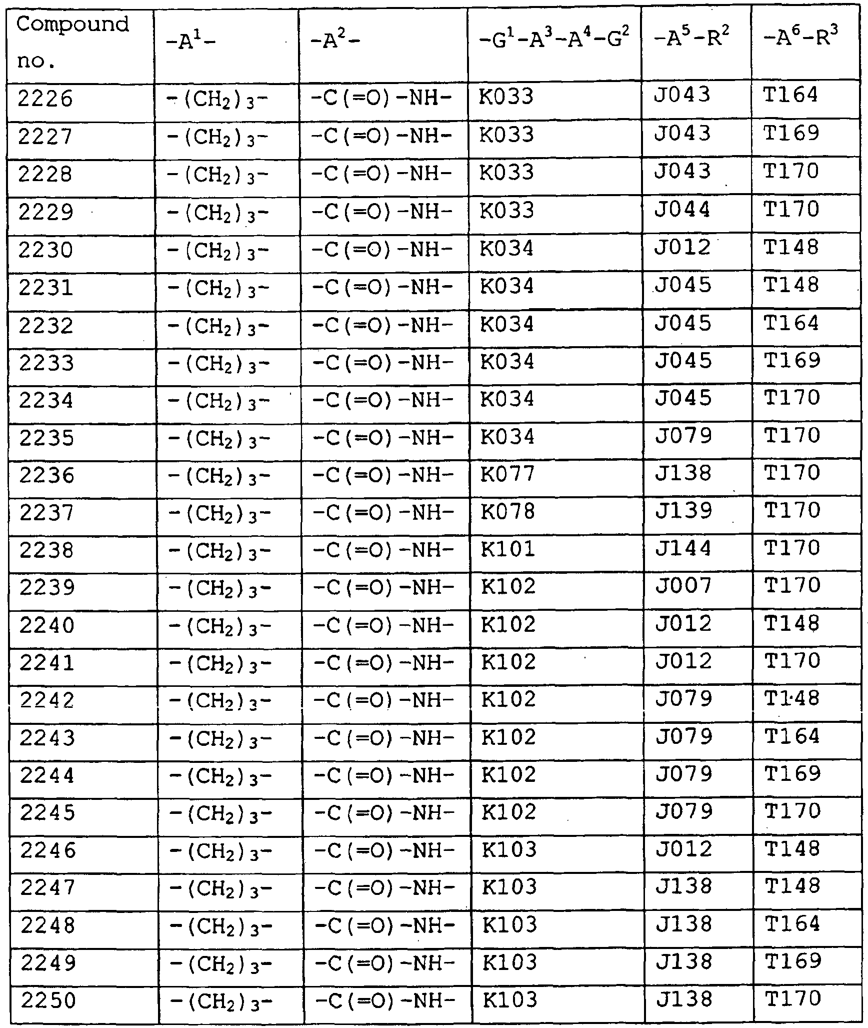

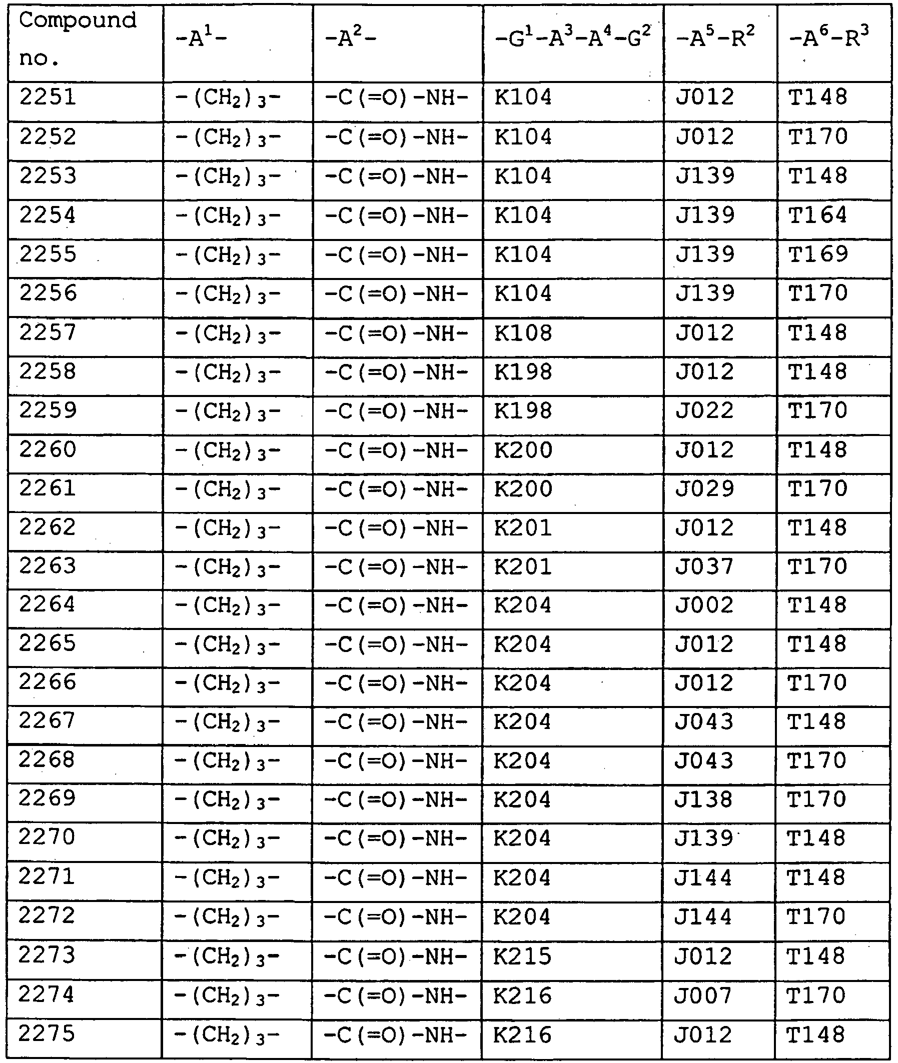

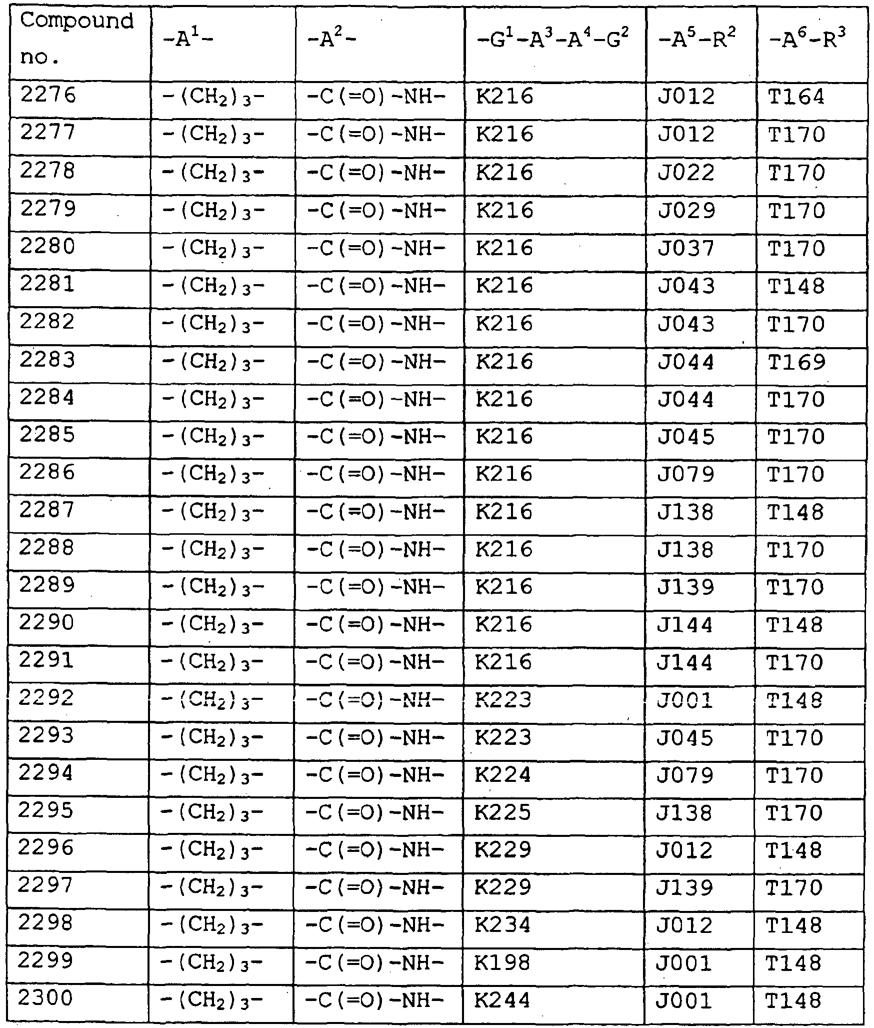

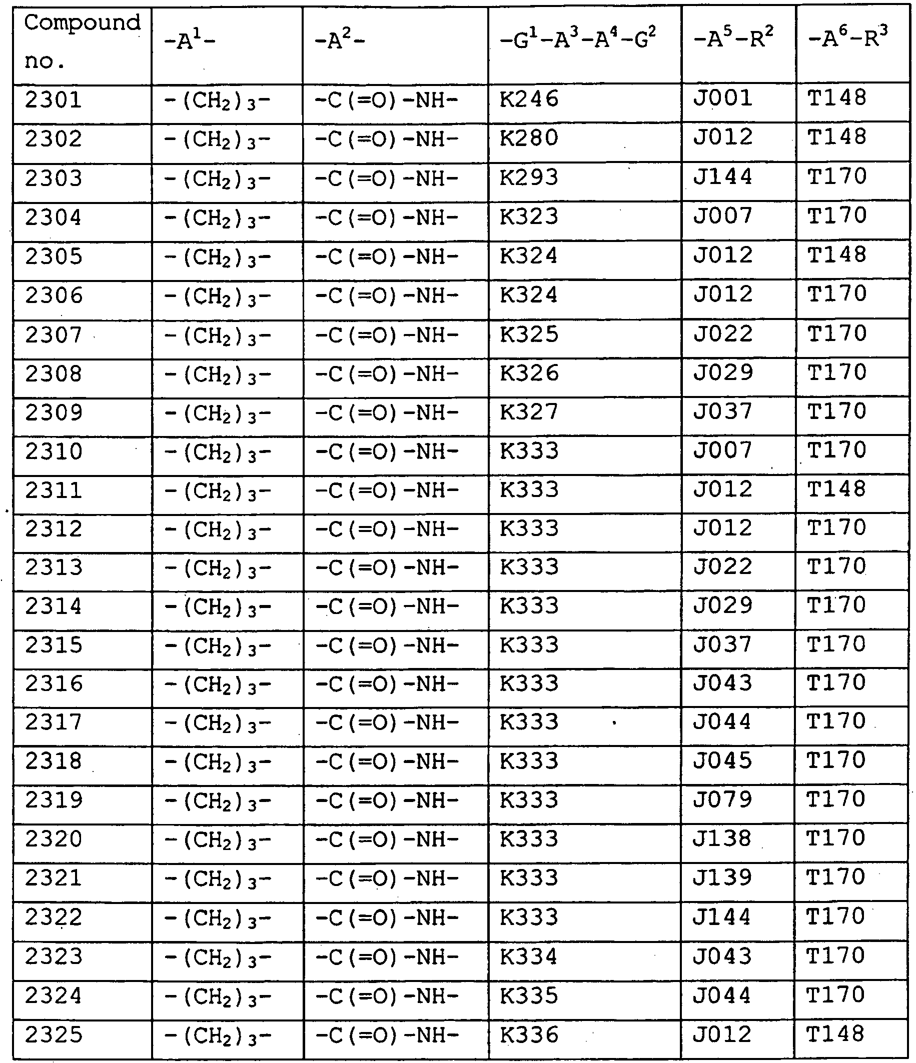

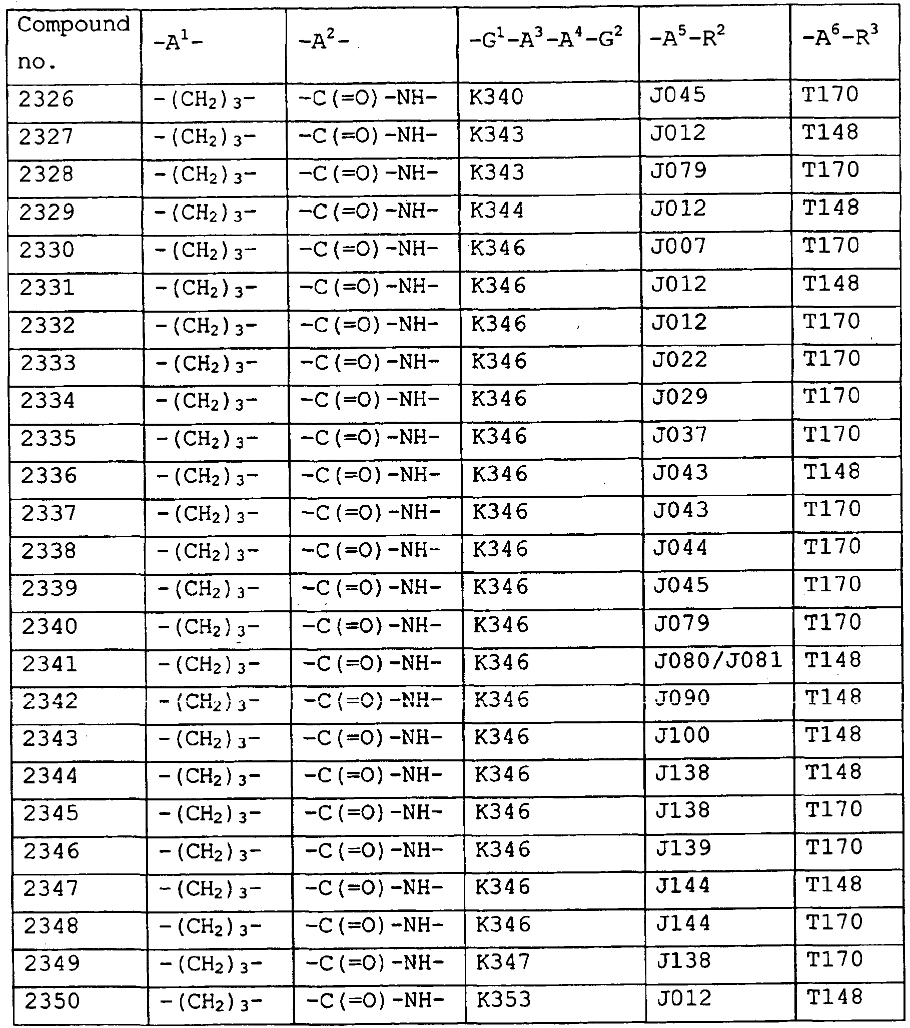

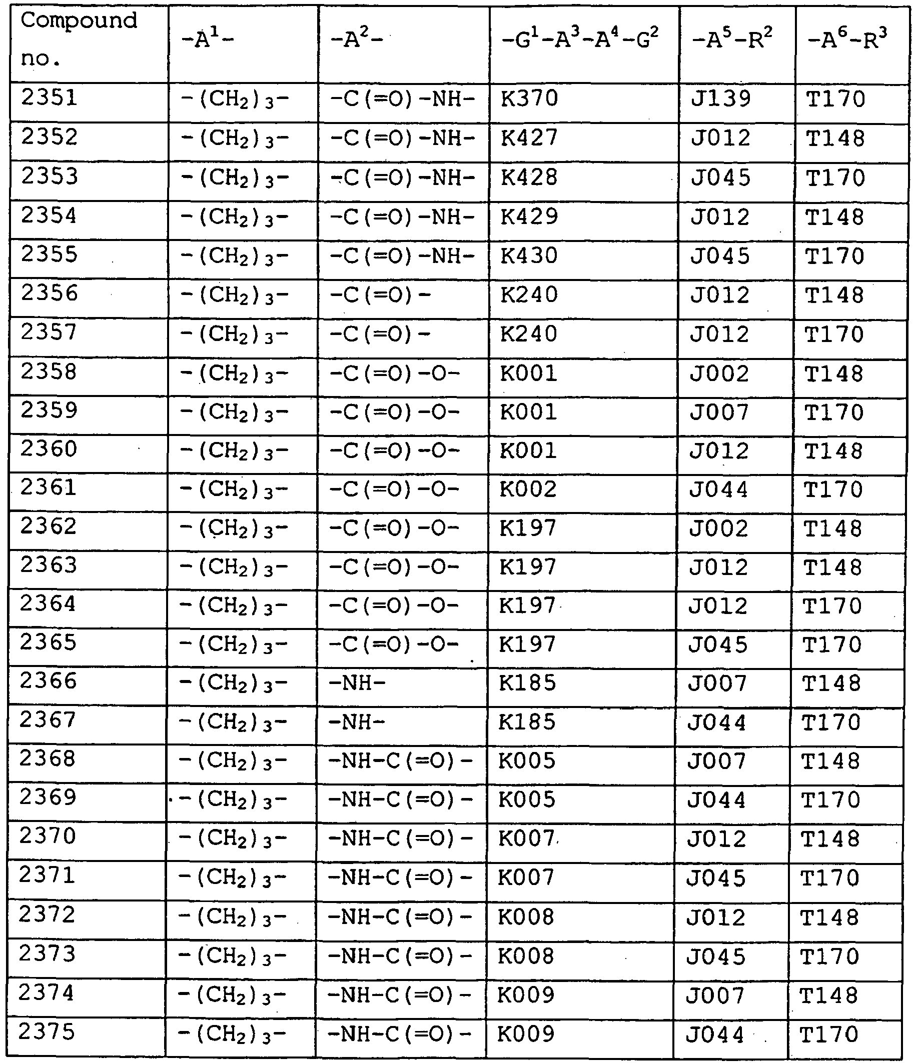

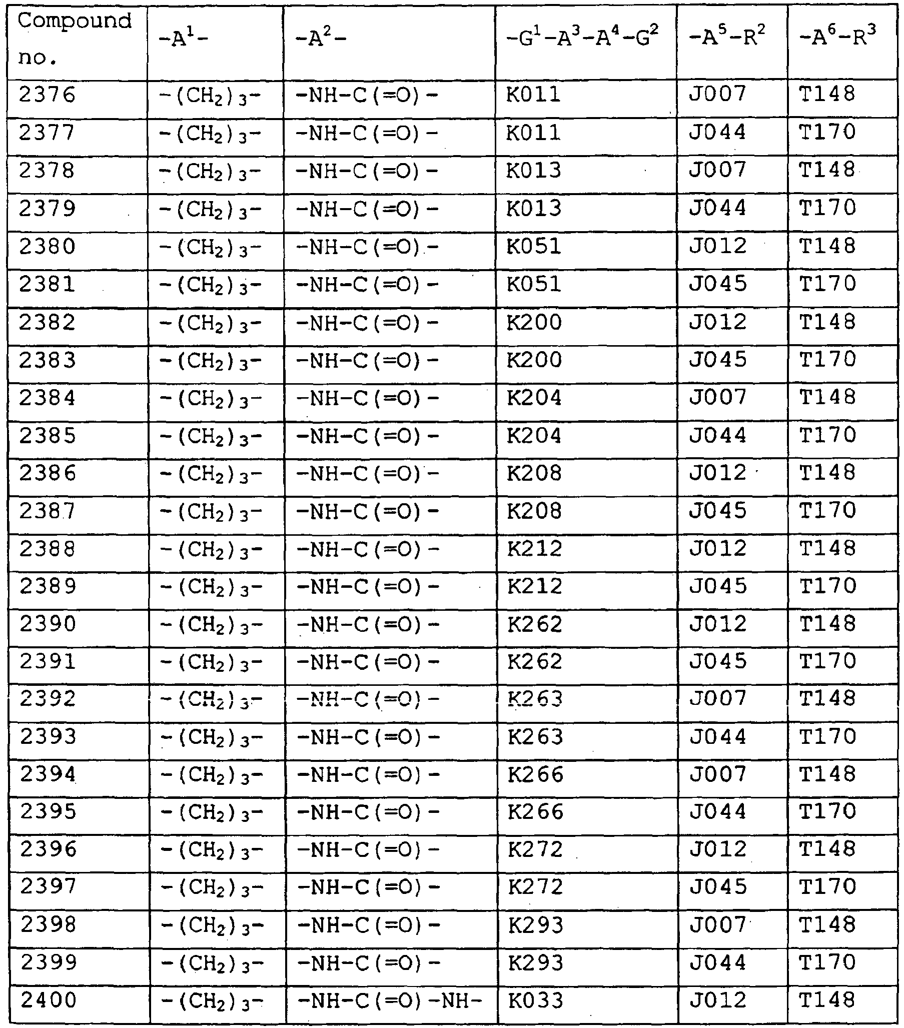

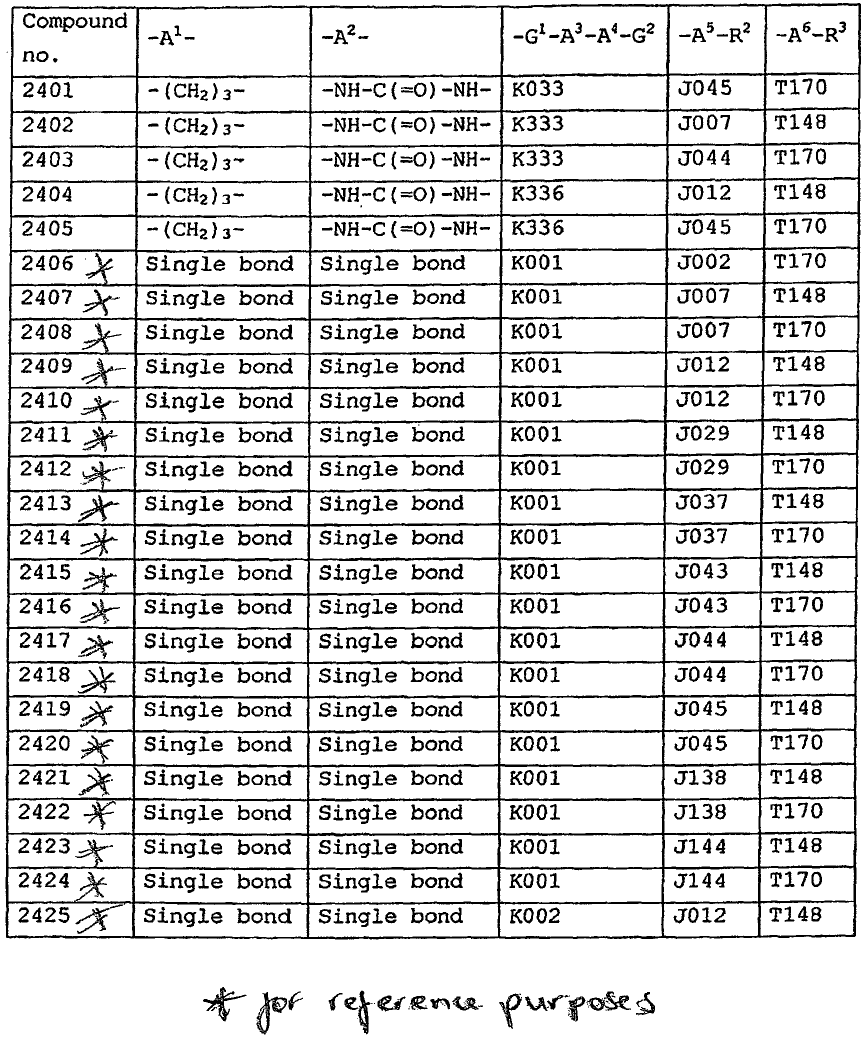

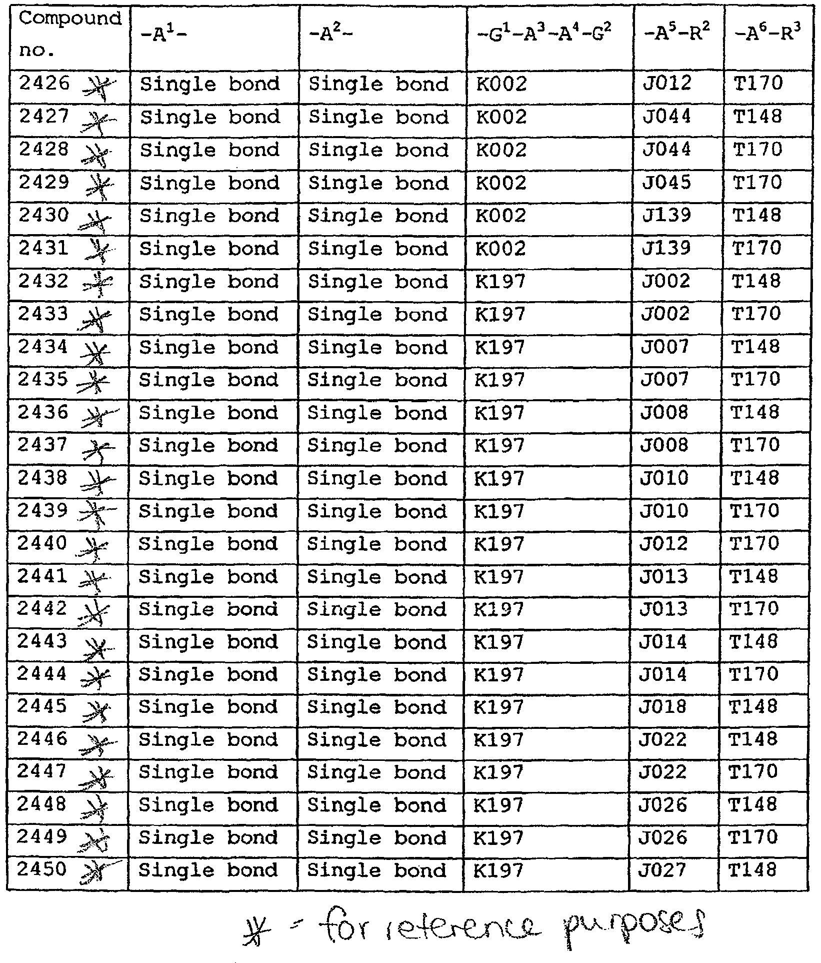

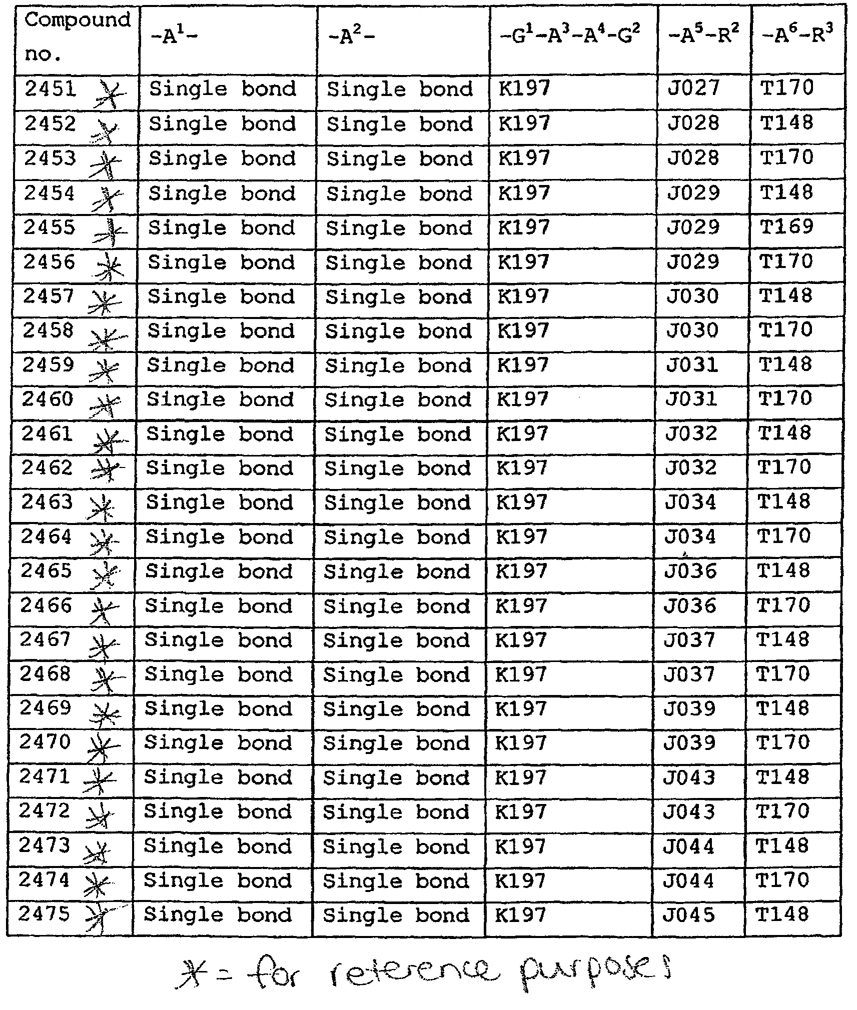

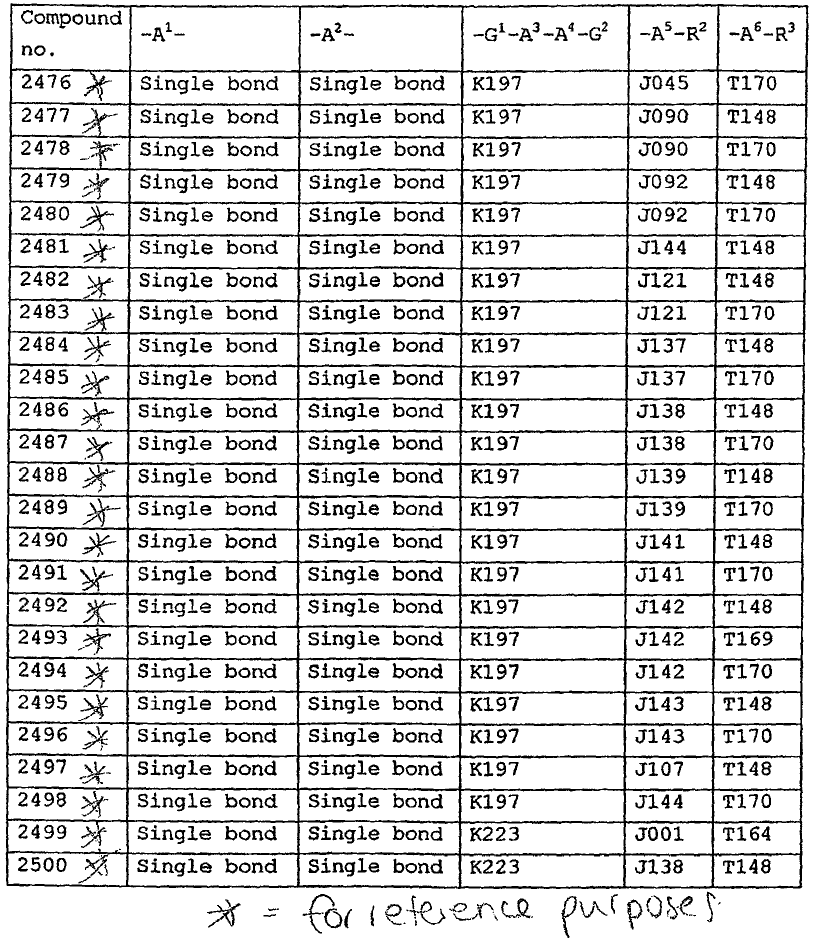

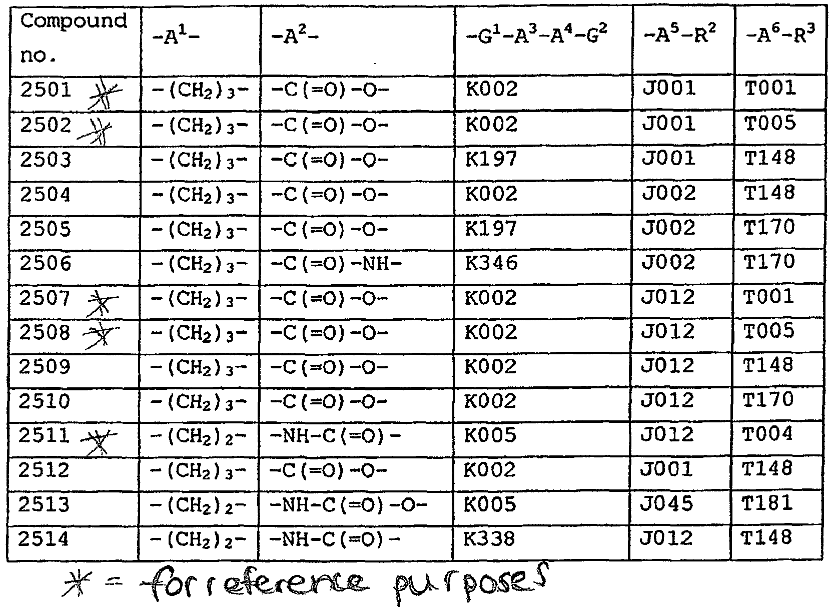

Also, among compounds described in Table 1, compounds of the following numbers are more preferred.

Compound numbers 19, 20, 22, 27, 29, 30, 34, 36, 37, 38, 39, 40, 41, 42, 43, 44, 46, 47, 48, 49, 50, 51, 52, 53, 54, 56, 57, 58, 59, 60, 61, 62, 63, 64, 66, 67, 68, 69, 70, 71, 72, 73, 74, 76, 77, 78, 79, 84, 85, 86, 87, 88, 90, 91, 92, 93, 94, 95, 96, 97, 98, 100, 101, 102, 103, 104, 105, 106, 107, 108, 110, 111, 112, 113, 114, 115, 116, 117, 118, 120, 121, 122, 123, 124, 125, 126, 127, 128, 130, 131, 132, 133, 134, 135, 136, 137, 138, 140, 141, 142, 143, 144, 145, 146, 147, 148, 150, 151, 152, 153, 154, 155, 156, 157, 158, 160, 161, 162, 163, 164, 165, 166, 167, 168, 170, 171, 172, 173, 174, 175, 176, 177, 178, 180, 181, 182, 183, 184, 185, 186, 187, 188, 190, 191, 192, 193, 194, 195, 196, 197, 198, 200, 201, 202, 203, 210, 211, 213, 214, 217, 219, 221, 223, 225, 226, 227, 228, 229, 230, 231, 232, 237, 240, 241, 242, 243, 244, 245, 250, 253, 254, 255, 257, 258, 260, 261, 262, 263, 265, 266, 267, 268, 269, 275, 276, 277, 278, 279, 280, 281, 282, 283, 284, 285, 286, 287, 288, 294, 295, 296, 297, 298, 299, 300, 301, 302, 303, 308, 309, 310, 311, 312, 313, 314, 315, 316, 317, 321, 327, 328, 330, 331, 332, 333, 334, 335, 340, 342, 343, 348, 349, 350, 351, 352, 353, 359, 361, 362, 363, 364, 365, 366, 368, 369, 371, 373, 379, 381, 382, 383, 384, 385, 386, 387, 392, 393, 395, 397, 398, 399, 400, 401, 407, 409, 410, 411, 412, 413, 415, 416, 417, 418, 419, 420, 421, 422, 423, 426, 427, 429, 430, 431, 432, 433, 434, 437, 438, 439, 445, 447, 448, 449, 451, 453, 454, 455, 456, 457, 463, 465, 466, 467, 468, 469, 471, 472, 473, 474, 475, 476, 477, 478, 479, 483, 484, 485, 486, 487, 488, 489, 490, 491, 492, 493, 494, 497, 498, 499, 501, 502, 503, 504, 509, 511, 512, 513, 515, 517, 518, 519, 520, 521, 527, 529, 530, 531, 532, 533, 534, 535, 536, 537, 539, 540, 541, 543, 544, 545, 546, 547, 548, 549, 550, 551, 552, 555, 556, 557, 563, 565, 566, 567, 569, 571, 572, 573, 574, 575, 581, 583, 584, 585, 586, 587, 589, 590, 591, 593, 594, 595, 596, 597, 599, 600, 601, 602, 605, 606, 607, 608, 610, 611, 612, 613, 615, 616, 617, 618, 620, 621, 623, 624, 626, 627, 628, 629, 630, 631, 634, 635, 638, 639, 641, 642, 643, 644, 645, 646, 647, 648, 651, 652, 653, 655, 656, 657, 658, 659, 660, 661, 662, 663, 664, 665, 666, 667, 668, 669, 670, 671, 672, 673, 675, 676, 677, 678, 679, 681, 682, 683, 684, 685, 686, 687, 688, 689, 690, 691, 692, 693, 694, 695, 696, 697, 700, 701, 702, 704, 705, 706, 707, 709, 710, 711, 712, 713, 715, 716, 717, 718, 719, 721, 722, 723, 724, 725, 726, 727, 728, 729, 730, 731, 732, 733, 734, 735, 736, 737, 738, 739, 740, 741, 743, 744, 745, 747, 748, 749, 750, 751, 752, 753, 754, 755, 756, 757, 758, 759, 760, 761, 762, 763, 764, 765, 766, 767, 768, 769, 770, 771, 773, 776, 777, 778, 779, 780, 781, 782, 783, 784, 785, 786, 787, 788, 789, 790, 791, 792, 793, 794, 795, 796, 801, 802, 803, 804, 805, 806, 807, 808, 809, 810, 811, 812, 852, 853, 874, 892, 893, 915, 917, 918, 919, 921, 939, 998, 1000, 1001, 1002, 1007, 1008, 1014, 1015, 1054, 1055, 1056, 1059, 1061, 1063, 1065, 1067, 1069, 1071, 1073, 1075, 1076, 1077, 1078, 1079, 1080, 1081, 1083, 1085, 1089, 1090, 1091, 1092, 1093, 1095, 1096, 1097, 1098, 1099, 1181, 1182, 1183, 1184, 1185, 1186, 1187, 1188, 1189, 1190, 1191, 1192, 1193, 1194, 1195, 1196, 1197, 1198, 1199, 1200, 1201, 1202, 1203, 1204, 1205, 1206, 1207, 1208, 1209, 1210, 1211, 1212, 1213, 1214, 1215, 1216, 1217, 1218, 1219, 1220, 1221, 1222, 1223, 1224, 1225, 1226, 1227, 1228, 1229, 1230, 1231, 1232, 1233, 1234, 1235, 1236, 1237, 1238, 1239, 1240, 1241, 1242, 1243, 1244, 1245, 1246, 1247, 1248, 1249, 1250, 1251, 1252, 1253, 1254, 1255, 1256, 1257, 1258, 1259, 1260, 1261, 1262, 1263, 1264, 1265, 1266, 1268, 1269, 1270, 1271, 1272, 1273, 1274, 1275, 1276, 1277, 1278, 1279, 1280, 1281, 1282, 1283, 1284, 1285, 1286, 1287, 1288, 1289, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1345, 1346, 1347, 1348, 1349, 1350, 1351, 1352, 1353, 1354, 1355, 1356, 1357, 1358, 1359, 1360, 1361, 1362, 1363, 1364, 1365, 1366, 1367, 1368, 1369, 1370, 1371, 1372, 1373, 1374, 1375, 1376, 1377, 1378, 1379, 1380, 1381, 1382, .1383, 1384, 1385, 1386, 1387, 1388, 1389, 1390, 1391, 1392, 1393, 1394, 1395, 1396, 1397, 1398, 1399, 1400, 1401, 1402, 1403, 1404, 1405, 1406, 1407, 1408, 1409, 1410, 1411, 1412, 1413, 1414, 1415, 1416, 1417, 1418, 1419, 1420, 1421, 1422, 1423, 1424, 1425, 1426, 1427, 1428, 1429, 1430, 1431, 1432, 1433, 1434, 1435, 1436, 1437, 1438, 1439, 1440, 1441, 1442, 1443, 1444, 1445, 1446, 1447, 1448, 1449, 1450, 1451, 1452, 1453, 1454, 1455, 1456, 1457, 1458, 1459, 1460, 1461, 1462, 1463, 1464, 1465, 1466, 1467, 1468, 1469, 1470, 1471, 1472, 1473, 1474, 1475, 1476, 1477, 1478, 1479, 1480, 1481, 1482, 1483, 1484, 1485, 1486, 1487, 1488, 1489, 1490, 1491, 1492, 1493, 1494, 1495, 1496, 1497, 1498, 1499, 1500, 1501, 1502, 1503, 1504, 1505, 1506, 1507, 1508, 1509, 1510, 1511, 1512, 1513, 1514, 1515, 1516, 1517, 1518, 1519, 1520, 1521, 1522, 1523, 1524, 1525, 1526, 1527, 1528, 1529, 1530, 1531, 1532, 1533, 1534, 1535, 1536, 1537, 1538, 1539, 1540, 1541, 1542, 1543, 1544, 1545, 1546, 1547, 1548, 1549, 1550, 1551, 1552, 1553, 1554, 1555, 1556, 1557, 1558, 1559, 1560, 1561, 1562, 1563, 1564, 1565, 1566, 1567, 1568, 1569, 1570, 1571, 1572, 1573, 1574, 1575, 1576, 1577, 1578, 1579, 1580, 1581, 1582, 1583, 1584, 1585, 1586, 1587, 1588, 1589, 1590, 1591, 1592, 1593, 1594, 1595, 1596, 1597, 1598, 1599, 1600, 1601, 1602, 1603, 1604, 1605, 1606, 1607, 1608, 1609, 1610, 1611, 1612, 1613, 1614, 1615, 1616, 1617, 1618, 1619, 1620, 1621, 1622, 1623, 1624, 1625, 1626, 1627, 1628, 1629, 1630, 1631, 1632, 1633, 1634, 1635, 1636, 1637, 1638, 1639, 1640, 1641, 1642, 1643, 1644, 1645, 1646, 1647, 1648, 1649, 1650, 1651, 1652, 1653, 1654, 1655, 1656, 1657, 1658, 1659, 1660, 1661, 1662, 1663, 1664, 1665, 1666, 1667, 1668, 1669, 1670, 1671, 1672, 1673, 1674, 1675, 1676, 1677, 1678, 1679, 1680, 1681, 1682, 1683, 1684, 1685, 1686, 1687, 1688, 1689, 1690, 1691, 1692, 1693, 1694, 1695, 1696, 1697, 1698, 1700, 1701, 1702, 1703, 1704, 1705, 1706, 1707, 1708, 1709, 1710, 1711, 1712, 1713, 1714, 1715, 1716, 1717, 1718, 1719, 1720, 1721, 1722, 1723, 1724, 1725, 1726, 1727, 1728, 1729, 1730, 1731, 1732, 1733, 1734, 1735, 1736, 1737, 1738, 1739, 1740, 1741, 1742, 1743, 1744, 1745, 1746, 1747, 1748, 1749, 1750, 1751, 1752, 1753, 1754, 1755, 1756, 1757, 1758, 1759, 1760, 1761, 1762, 1763, 1764, 1765, 1766, 1767, 1768, 1769, 1770, 1771, 1772, 1773, 1774, 1775, 1776, 1777, 1778, 1779, 1780, 1781, 1782, 1783, 1784, 1785, 1786, 1787, 1788, 1789, 1790, 1791, 1792, 1793, 1794, 1795, 1796, 1797, 1798, 1799, 1800, 1801, 1802, 1803, 1804, 1805, 1806, 1807, 1808, 1809, 1810, 1811, 1812, 1813, 1814, 1815, 1816, 1817, 1818, 1819, 1820, 1821, 1822, 1823, 1824, 1825, 1826, 1827, 1829, 1830, 1831, 1832, 1833, 1834, 1835, 1836, 1837, 1838, 1839, 1840, 1841, 1842, 1843, 1844, 1845, 1846, 1847, 1848, 1849, 1850, 1851, 1852, 1853, 1854, 1855, 1856, 1857, 1858, 1859, 1860, 1861, 1862, 1863, 1864, 1865, 1866, 1867, 1868, 1869, 1870, 1871, 1872, 1873, 1874, 1875, 1876, 1877, 1878, 1879, 1880, 1881, 1882, 1883, 1884, 1885, 1886, 1887, 1888, 1889, 1890, 1891, 1892, 1893, 1894, 1895, 1896, 1897, 1898, 1899, 1900, 1901, 1902, 1903, 1904, 1905, 1906, 1907, 1908, 1909, 1910, 1911, 1912, 1913, 1914, 1915, 1916, 1917, 1918, 1919, 1920, 1921, 1922, 1923, 1924, 1925, 1926, 1927, 1928, 1929, 1930, 1931, 1932, 1933, 1934, 1935, 1936, 1937, 1938, 1939, 1940, 1941, 1942, 1943, 1944, 1945, 1946, 1947, 1948, 1949, 1950, 1951, 1952, 1953, 1954, 1955, 1956, 1957, 1958, 1959, 1960, 1961, 1962, 1963, 1964, 1965, 1966, 1967, 1968, 1969, 1970, 1971, 1972, 1973, 1974, 1975, 1976, 1977, 1978, 1979, 1980, 1981, 1982, 1983, 1984, 1985, 1986, 1987, 1988, 1989, 1990, 1991, 1992, 1993, 1994, 1995, 1996, 1997, 1998, 1999, 2000, 2001, 2002, 2003, 2004, 2005, 2006, 2007, 2008, 2010, 2011, 2012, 2013, 2014, 2015, 2016, 2017, 2018, 2019, 2020, 2021, 2022, 2023, 2024, 2025, 2026, 2027, 2028, 2029, 2030, 2031, 2032, 2033, 2034, 2035, 2036, 2037, 2038, 2039, 2040, 2041, 2042, 2043, 2044, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2067, 2068, 2069, 2070, 2071, 2072, 2073, 2074, 2075, 2076, 2077, 2078, 2079, 2080, 2081, 2082, 2083, 2084, 2085, 2086, 2087, 2088, 2089, 2090, 2091, 2092, 2093, 2094, 2095, 2096, 2097, 2098, 2099, 2100, 2101, 2102, 2103, 2104, 2105, 2106, 2107, 2108, 2109, 2111, 2112, 2113, 2114, 2115, 2116, 2117, 2118, 2119, 2121, 2122, 2123, 2124, 2125, 2128, 2129, 2130, 2131, 2132, 2133, 2134, 2135, 2136, 2137, 2138, 2139, 2140, 2143, 2144, 2145, 2146, 2147, 2148, 2149, 2150, 2151, 2152, 2153, 2154, 2155, 2156, 2157, 2158, 2159, 2160, 2161, 2162, 2163, 2164, 2166, 2167, 2168, 2169, 2170, 2171, 2172, 2173, 2174, 2175, 2176, 2177, 2178, 2179, 2180, 2181, 2182, 2183, 2184, 2185, 2186, 2187, 2188, 2189, 2190, 2191, 2192, 2193, 2194, 2195, 2196, 2197, 2198, 2199, 2200, 2201, 2202, 2203, 2204, 2205, 2206, 2207, 2208, 2209, 2210, 2211, 2212, 2213, 2214, 2215, 2216, 2217, 2218, 2219, 2220, 2221, 2222, 2223, 2224, 2225, 2226, 2227, 2228, 2229, 2230, 2231, 2232, 2233, 2234, 2235, 2236, 2237, 2238, 2239, 2240, 2241, 2242, 2243, 2244, 2245, 2246, 2247, 2248, 2249, 2250, 2251, 2252, 2253, 2254, 2255, 2256, 2257, 2258, 2259, 2260, 2261, 2262, 2263, 2264, 2265, 2266, 2267, 2268, 2269, 2270, 2271, 2272, 2273, 2274, 2275, 2276, 2277, 2278, 2279, 2280, 2281, 2282, 2283, 2284, 2285, 2286, 2288, 2289, 2290, 2291, 2292, 2293, 2294, 2295, 2296, 2297, 2298, 2299, 2300, 2302, 2303, 2304, 2305, 2306, 2307, 2308, 2309, 2310, 2311, 2312, 2313, 2314, 2315, 2316, 2317, 2318, 2319, 2320, 2321, 2322, 2323, 2324, 2325, 2326, 2327, 2328, 2329, 2330, 2331, 2332, 2333, 2334, 2335, 2336, 2337, 2338, 2339, 2340, 2341, 2342, 2343, 2344, 2345, 2346, 2347, 2348, 2349, 2350, 2351, 2352, 2353, 2354, 2355, 2356, 2357, 2358, 2359, 2360, 2361, 2363, 2364, 2365, 2366, 2367, 2368, 2369, 2370, 2371, 2372, 2373, 2374, 2375, 2376, 2377, 2378, 2379, 2380, 2381, 2382, 2383, 2384, 2385, 2386, 2387, 2388, 2389, 2390, 2391, 2392, 2393, 2394, 2395, 2396, 2397, 2398, 2399, 2400, 2401, 2402, 2403, 2404, 2405, 2505, 2506, 2509, 2510, 2514.

Preferred combinations of A¹, A², G¹, A³, A⁴ and G² in the formula (I) were explained above. As another method of arrangement, they can be summarized also as the following combinations 1) through 41). Not only do these combinations indicate preferred relationships among A¹, A², G¹, A³, A⁴ and G², but also the partial structures per se comprised of these as a whole are preferred substituents in the pyrrolopyrimidinone derivatives of the present invention.
1) In the formula (I), when A¹ is -(CH₂)₂-, A¹-A²-G¹ links in the form of A¹-NH-C(=O)-G¹, and G¹ is a phenylene group, the phenylene group as G¹ is preferably substituted with one or more substituents selected from those exemplified for the preferred substituents of the substituted aromatic hydrocarbon group having 6 to 14 carbon atoms of G¹.
2) In the formula (I), when A¹ is -(CH₂)₂-, A¹-A²-G¹ links in the form of A¹-NH-C(=O)-G¹, G¹ is a phenylene group, and the phenylene group as G¹ is not substituted, it is preferable that A³-A⁴-G² as a whole is a group other than a hydrogen atom.
3) In the formula (I), when A¹ is -(CH₂)₂-, A¹-A²-G¹ links in the form of A¹-NH-C(=O)-G¹, G¹ is preferably a divalent group derived from a monocyclic or bicyclic C₃-C₉ aromatic heterocyclic compound having 1 to 3, preferably 1 or 2, atoms selected from the group consisting of an oxygen atom, a nitrogen atom and a sulfur atom, in the ring.
4) In the formula (I), when A¹ is -(CH₂)₂- and A¹-A²-G¹ links in the form of A¹-NH-C(=O)-G¹, G¹ is preferably a divalent group derived from a monocyclic or bicyclic C₂-C₉ aromatic heterocyclic compound having 1 to 3, preferably 1 or 2 atoms selected from the group consisting of an oxygen atom, a nitrogen atom and a sulfur atom, in the ring. However, the divalent group derived from the aromatic heterocyclic compound as G¹ is more preferably substituted with one or more substituents selected from the group consisting of substituents defined as preferred examples for the heterocyclic compound having 1 to 4 atoms selected from the group consisting of an oxygen atom, a nitrogen atom and a sulfur atom, in the ring.
5) In the formula (I), when A¹ is -(CH₂)₂- and A¹-A²-G¹ links in the form of A¹-NH-C(=O)-G¹, G¹ is preferably a divalent group derived from a monocyclic or bicyclic C₂-C₉ aromatic heterocyclic compound having 1 to 3, preferably 1 or 2 atoms selected from the group consisting of an oxygen atom, a nitrogen atom and a sulfur atom, in the ring. However, when the divalent group derived from the aromatic heterocyclic compound as G¹ is not substituted, it is more preferable that A³-A⁴-G² as a whole is a group other than a hydrogen atom.
6) In the formula (I), when A¹ is -(CH₂)₂-, A¹-A²-G¹ links in the form of A¹-NH-C(=O)-G¹, and G¹ represents a single bond, it is more preferable that A³-A⁴-G² as a whole is a acyclic aliphatic hydrocarbon group having 1 to 6 carbon atoms or a cycloalkylalkyl group consisting of a acyclic aliphatic hydrocarbon group having 1 to 6 carbon atoms and an alicyclic hydrocarbon group having 3 to 8 carbon atoms.
7) In the formula (I), when A¹ is -(CH₂)₂-, A¹-A²-G¹ links in the form of A¹-NH-C(=O)-G¹, and G¹ represents a single bond, A³-A⁴-G² is preferably a acyclic aliphatic hydrocarbon group having 1 to 6 carbon atoms or a cycloalkylalkyl group consisting of a acyclic aliphatic hydrocarbon group having 1 to 6 carbon atoms and an alicyclic hydrocarbon group having 3 to 8 carbon atoms as a whole, but a acyclic aliphatic hydrocarbon group having 1 to 6 carbon atoms as A³-A⁴-G² is more preferably substituted with one or more substituents selected from those exemplified for the preferred substituents of the substituted acyclic aliphatic hydrocarbon group having 1 to 10 carbon atoms of A³. Also, in a cycloalkylalkyl group consisting of a acyclic aliphatic hydrocarbon group having 1 to 6 carbon atoms and an alicyclic hydrocarbon group having 3 to 8 carbon atoms as A³-A⁴-G², a acyclic aliphatic hydrocarbon group portion having 1 to 6 carbon atoms is further preferably substituted with one or more substituents selected from those exemplified for the preferred substituents of the substituted aliphatic hydrocarbon group having 1 to 10 carbon atoms of A³, or an alicyclic hydrocarbon group portion having 3 to 8 carbon atoms is further preferably substituted with one or more substituents selected from those exemplified for the preferred substituents of the substituted alicyclic hydrocarbon group having 3 to 10 carbon atoms of G² (including a case where both are substituted).
8) In the formula (I), when A¹ is -(CH₂)₂-, A¹-A²-G¹ links in the form of A¹-NH-C(=O)-G¹, and G¹ represents a single bond, A³-A⁴-G² is preferably an aralkyl group consisting of a acyclic aliphatic hydrocarbon group having 1 to 6 carbon atoms and an aromatic hydrocarbon group having 6 to 10 carbon atoms as a whole.
9) In the formula (I), when A¹ is -(CH₂)₂-, A¹-A²-G¹ links in the form of A¹-NH-C(=O)-G¹, and G¹ represents a single bond, A³-A⁴-G² is preferably an aralkyl group consisting of a acyclic aliphatic hydrocarbon group having 1 to 6 carbon atoms and an aromatic hydrocarbon group having 6 to 10 carbon atoms as a whole, but in an aralkyl group as A³-A⁴-G², a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of A³, or an aromatic hydrocarbon group portion having 6 to 10 carbon atoms is further preferably substituted with one or more substituents selected from those exemplified for the preferred substituents of the substituted aromatic hydrocarbon group having 6 to 14 carbon atoms of G² (including a case where both are substituted).
10) In the formula (I), when A¹ is -(CH₂)₂-, A¹-A²-G¹ links in the form of A¹-NH-C(=O)-G¹, and G¹ represents a single bond, A³-A⁴-G² is preferably a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring as a whole.
11) In the formula (I), when A¹ is -(CH₂)₂-, A¹-A²-G¹ links in the form of A¹-NH-C(=O)-G¹, and G¹ represents a single bond, A³-A⁴-G² is preferably a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring as a whole. However, in the heterocyclic substituted alkyl group as A³-A⁴-G², a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of A³, or a heterocyclic portion is further preferably substituted with one or more substituents selected from those exemplified for the preferred substituents of the heterocyclic group having 1 to 4 atoms selected from the group consisting of an oxygen atom, a nitrogen atom and a sulfur atom, in the ring, of G² (including a case where both are substituted).
12) In the formula (I), when A¹ is -(CH₂)₂-, A¹-A²-G¹ links in the form of A¹-NH-C(=O)-G¹, and G¹ is a phenylene group, the phenylene group as G¹ is preferably substituted with one or more substituents selected from those exemplified for the preferred substituents of the substituted aromatic hydrocarbon group having 6 to 14 carbon atoms of G¹.
13) In the formula (I), when A¹ is -(CH₂)₂-, A¹-A²-G¹ links in the form of A¹-NH-C(=O)-NH-G¹, G¹ is a phenylene group, and the phenylene group as G¹ is not substituted, it is preferable that A³-A⁴-G² as a whole is a group other than a hydrogen atom.
14) In the formula (I), when A¹ is -(CH)₂- and A¹-A²-G¹ links in the form of A¹-NH-C(=O)-NH-G¹, G¹ is preferably a divalent group derived from a monocyclic or bicyclic C₃-C₉ aromatic heterocyclic compound having 1 to 3, preferably 1 or 2, atoms selected from the group consisting of an oxygen atom, a nitrogen atom and a sulfur atom, in the ring.
15) In the formula (I), when A¹ is -(CH₂)₂- and A¹-A²-G¹ links in the form of A¹-NH-C(=O) -NH-G¹, G¹ is preferably a divalent group derived from a monocyclic or bicyclic C₂-C₉ aromatic heterocyclic compound having 1 to 3, preferably 1 or 2 atoms selected from the group consisting of an oxygen atom, a nitrogen atom and a sulfur atom, in the ring. However, the divalent group derived from the aromatic heterocyclic compound as G¹ is more preferably substituted with one or more substituents selected from the group consisting of substituents defined as preferred examples for the heterocyclic compound having 1 to 4 atoms selected from the group consisting of an oxygen atom, a nitrogen atom and a sulfur atom, in the ring.
16) In the formula (I), when A¹ is -(CH₂)₂- and A¹-A²-G¹ links in the form of A¹-NH-C(=O)-NH-G¹, G¹ is preferably a divalent group derived from a monocyclic or bicyclic C₂-C₉ aromatic heterocyclic compound having 1 to 3, preferably 1 or 2 atoms selected from the group consisting of an oxygen atom, a nitrogen atom and a sulfur atom, in the ring. However, when the divalent group derived from the aromatic heterocyclic compound as G¹ is not substituted, it is more preferable that A³-A⁴-G² as a whole is a group other than a hydrogen atom.
17) In the formula (I), when A¹ is -(CH₂)₂-, A¹-A²-G¹ links in the form of A¹-NH-C(=O)-NH-G¹, and G¹ represents a single bond, it is more preferable that A³-A⁴-G² as a whole is a cycloalkylalkyl group consisting of a acyclic aliphatic hydrocarbon group having 1 to 6 carbon atoms and an alicyclic hydrocarbon group having 3 to 8 carbon atoms.
18) In the formula (I), when A¹ is -(CH₂)₂-, A¹-A²-G¹ links in the form of A¹-NH-C(=O)-NH-G¹, and G¹ represents a single bond, it is more preferable that A³-A⁴-G² as a whole is a cycloalkylalkyl group consisting of a acyclic aliphatic hydrocarbon group having 1 to 6 carbon atoms and an alicyclic hydrocarbon group having 3 to 8 carbon atoms. However, in the cycloalkylalkyl group as A³-A⁴-G², a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of A³, or an alicyclic hydrocarbon group portion having 3 to 8 carbon atoms is further preferably substituted with one or more substituents selected from those exemplified for the preferred substituents of the substituted alicyclic hydrocarbon group having 3 to 10 carbon atoms of G² (including a case where both are substituted).
19) In the formula (I), when A¹ is -(CH₂)₂-, A¹-A²-G¹ links in the form of A¹-NH-C(=O)-NH-G¹, and G¹ represents a single bond, it is more preferable that A³-A⁴-G² as a whole is an aralkyl group consisting of a acyclic aliphatic hydrocarbon group having 1 to 6 carbon atoms and an aromatic hydrocarbon group having 6 to 10 carbon atoms.
20) In the formula (I), when A¹ is -(CH₂)₂-, A¹-A²-G¹ links in the form of A¹-NH-C(=O)-NH-G¹, and G¹ represents a single bond, it is more preferable that A³-A⁴-G² as a whole is an aralkyl group consisting of a acyclic aliphatic hydrocarbon group having 1 to 6 carbon atoms and an aromatic hydrocarbon group having 6 to 14 carbon atoms. However, in the ararkyl group as A³-A⁴-G², a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of A³, or an aromatic hydrocarbon group portion having 6 to 10 carbon atoms is further preferably substituted with one or more substituents selected from those exemplified for the preferred substituents of the substituted aromatic hydrocarbon group having 6 to 14 carbon atoms of G² (including a case where both are substituted).
21) In the formula (I), when A¹ is -(CH₂)₂-, A¹-A²-G¹ links in the form of A¹-NH-C(=O)=NH-G¹, and G¹ represents a single bond, it is preferable that A³-A⁴-G² as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring.
22) In the formula (I), when A¹ is -(CH₂)₂-, A¹-A²-G¹ links in the form of A¹-NH-C(=O)-NH-G¹, and G¹ represents a single bond, it is preferable that A³-A⁴-G² as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring. However, in the heterocyclic substituted alkyl group as A³-A⁴-G², a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of A³, or a heterocyclic group portion is further preferably substituted with one or more substituents selected from those exemplified for the preferred substituents of the heterocyclic group having 1 to 4 atoms selected from the group consisting of an oxygen atom, a nitrogen atom and a sulfur atom, in the ring, of G² (including a case where both are substituted).
23) In the formula (I), when A¹ is -(CH₂)₂-, A¹-A²-G¹ links in the form of A¹-NH-G¹, and G¹ is a phenylene group, the phenylene group as G¹ is preferably substituted with one or more substituents selected from those exemplified for the preferred substituents of the substituted aromatic hydrocarbon group having 6 to 14 carbon atoms of G¹.
24) In the formula (I), when A¹ is -(CH₂)₂-, A¹-A²-G¹ links in the form of A¹-NH-G¹' G¹ is a phenylene group, and the phenylene group as G¹ is not substituted, it is preferable that A³-A⁴-G² as a whole is a group other than a hydrogen atom.
25) In the formula (I), when A¹ is -(CH₂)₂-, A¹-A²-G¹ links in the form of A¹-NH-G¹, and G¹ is a divalent group derived from a monocyclic or bicyclic C₂-C₉ aromatic heterocyclic compound having 1 to 3 atoms selected from the group consisting of an oxygen atom, a nitrogen atom and a sulfur atom, in the ring, the aromatic heterocyclic group is preferably substituted with one or more substituents selected from the group consisting of substituents defined as preferred examples for the heterocyclic group having 1 to 4 atoms selected from the group consisting of an oxygen atom, a nitrogen atom and a sulfur atom, in the ring.
26) In the formula (I), when A¹ is -(CH₂)₂-, A¹-A²-G¹ links in the form of A¹-NH-G¹, G¹ is a divalent group derived from a monocyclic or bicyclic C₂-C₉ aromatic heterocyclic compound having 1 to 3 atoms selected from the group consisting of an oxygen atom, a nitrogen atom and a sulfur atom, in the ring, and the aromatic heterocyclic compound is not substituted, it is more preferable that A³-A⁴-G² as a whole is a group other than a hydrogen atom.
27) In the formula (I), when A¹ is -(CH₂)₂- and A¹-A²-G¹ links in the form of A¹-C(=O)-G¹, G¹ is preferably a divalent group derived from a monocyclic C₂-C₉ heterocyclic compound having 1 or 2 atoms selected from the group consisting of an oxygen atom, a nitrogen atom and a sulfur atom, in the ring, such as pyrrolidine, piperidine, morpholine, thiomorpholine, homopiperidine, homopiperazine, 1,2, 3, 6-tetrahydropyridine or piperazine, and G¹ is bonded with A¹-C(=O)- through a nitrogen atom.
28) In the formula (I), when A¹ is -(CH₂)₂- and A¹-A²-G¹ links in the form of A¹-C(=O)-G¹, G¹ is a preferably divalent group derived from a monocyclic C₂-C₉ heterocyclic compound having 1 or 2 atoms selected from an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine, and G¹ is preferably bonded with A¹-C(=O)- through a nitrogen atom. However, the divalent group derived from the monocyclic C₂-C₉ heterocyclic compound having 1 or 2 atoms selected from an oxygen atom, a nitrogen atom and a sulfur atom, in the ring as G¹ is more preferably substituted with one or more substituents selected from the group consisting of substituents defined as proffered examples for the heterocyclic group having 1 to 4 atoms selected from the group consisting of an oxygen atom, a nitrogen atom and a sulfur atom on the substituted ring of G¹.
29) In the formula (I), when A¹ is -(CH₂)₂- and A¹-A²-G¹ links in the form of A¹-C(=O)-G¹, G¹ is a preferably divalent group derived from a monocyclic C₂-C₉ heterocyclic compound having 1 or 2 atoms selected from an oxygen atom, a nitrogen atom and a sulfur atom, in the ring, such as pyrrolidine, piperidine, morpholine, thiomorpholine, homopiperidine, homopiperazine, 1,2,3,6-tetrahydropyridine or piperazine, and G¹ is preferably bonded with A¹-C(=O)- through a nitrogen atom. However, when the divalent group derived from the monocyclic C₂-C₉ heterocyclic compound having 1 or 2 atoms selected from an oxygen atom, a nitrogen atom and a sulfur atom, in the ring as G¹ is not substituted, it is more preferable that A³-A⁴-G² as a whole is a group other than a hydrogen atom.
30) In the formula (I), when A¹ is -(CH₂)₃-, A¹-A²-G¹ links in the form of A¹-C(=O)-NH-G¹, and G¹ is a phenylene group, the phenylene group as G¹ is preferably substituted with one or more substituents selected from those exemplified for the preferred substituents of the substituted aromatic hydrocarbon group having 6 to 14 carbon atoms of G¹.
31) In the formula (I), when A¹ is -(CH₂)₂-, A¹-A²-G¹ links in the form of A¹-C(=O)-NH-G¹, G¹ is a phenylene group, and the phenylene group as G¹ is not substituted, it is preferable that A³-A⁴-G² as a whole is a group other than a hydrogen atom.
32) In the formula (I), when A¹ is -(CH₂)₃- and A¹-A²-G¹ links in the form of A¹-C(=O)-NH-G¹, G¹ is preferably a divalent group derived from a monocyclic or bicyclic C₃-C₉ aromatic heterocyclic compound having 1 to 3, preferably 1 or 2, atoms selected from the group consisting of an oxygen atom, a nitrogen atom and a sulfur atom, in the ring.
33) In the formula (I), when A¹ is -(CH₂)₃- and A¹-A²-G¹ links in the form of A¹-C(=O)-NH-G¹, G¹ is preferably a divalent group derived from a monocyclic or bicyclic C₂-C₉ aromatic heterocyclic compound having 1 to 3, preferably 1 or 2 atoms selected from the group consisting of an oxygen atom, a nitrogen atom and a sulfur atom, in the ring. However, the divalent group derived from the aromatic heterocyclic compound as G¹ is more preferably substituted with one or more substituents selected from the group consisting of substituents defined as preferred examples for the heterocyclic compound having 1 to 4 atoms selected from the group consisting of an oxygen atom, a nitrogen atom and a sulfur atom, in the ring.
34) In the formula (I), when A¹ is -(CH₂)₃- and A¹-A²-G¹ links in the form of A¹-C(=O)-NH-G¹, G¹ is preferably a divalent group derived from a monocyclic or bicyclic C₂-C₉ aromatic heterocyclic compound having 1 to 3, preferably 1 or 2 atoms selected from the group consisting of an oxygen atom, a nitrogen atom and a sulfur atom, in the ring. However, when the divalent group derived from the aromatic heterocyclic compound as G¹ is not substituted, it is more preferable that A³-A⁴-G² as a whole is a group other than a hydrogen atom.
35) In the formula (I), when A¹ is -(CH₂)₃-, A¹-A²-G¹ links in the form of A¹-C(=O)-NH-G¹, and G¹ represents a single bond, it is more preferable that A³-A⁴-G² as a whole is a cycloalkylalkyl group consisting of a acyclic aliphatic hydrocarbon group having 1 to 6 carbon atoms and an alicyclic hydrocarbon group having 3 to 8 carbon atoms.
36) In the formula (I), when A¹ is -(CH₂)₃-, A¹-A²-G¹ links in the form of A¹-C(=O)-NH-G¹, and G¹ represents a single bond, it is more preferable that A³-A⁴-G² as a whole is a cycloalkylalkyl group consisting of a acyclic aliphatic hydrocarbon group having 1 to 6 carbon atoms and an alicyclic hydrocarbon group having 3 to 8 carbon atoms. However, in the cycloalkylalkyl group as A³-A⁴-G², a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of A³, or an alicyclic hydrocarbon group portion having 3 to 8 carbon atoms is further preferably substituted with one or more substituents selected from those exemplified for the preferred substituents of the substituted alicyclic hydrocarbon group having 3 to 10 carbon atoms of G² (including a case where the both are substituted).
37) In the formula (I), when A¹ is -(CH₂)₃-, A¹-A²-G¹ links in the form of A¹-C(=O)-NH-G¹, and G¹ represents a single bond, it is more preferable that A³-A⁴-G² as a whole is an aralkyl group consisting of a acyclic aliphatic hydrocarbon group having 1 to 6 carbon atoms and an aromatic hydrocarbon group having 6 to 14 carbon atoms.
38) In the formula (I), when A¹ is -(CH₂)₃-, A¹-A²-G¹ links in the form of A¹-C(=O)-NH-G¹, and G¹ represents a single bond, it is more preferable that A³-A⁴-G² as a whole is an aralkyl group consisting of a acyclic aliphatic hydrocarbon group having 1 to 6 carbon atoms and an aromatic hydrocarbon group having 6 to 10 carbon atoms. However, in the aralkyl group as A³-A⁴-G², a acyclic aliphatic hydrocarbon group portion having 1 to 6 carbon atoms is more preferably substituted with one or more substituents selected from those exemplified for the preferred substituents of the substituted acyclic aliphatic hydrocarbon group having 1 to 10 carbon atoms of A³, or an aromatic hydrocarbon group portion having 6 to 10 carbon atoms is further preferably substituted with one or more substituents selected from those exemplified for the preferred substituents of the substituted aromatic hydrocarbon group having 6 to 14 carbon atoms of G² (including a case where both are substituted).
39) In the formula (I), when A¹ is -(CH₂)₃-, A¹-A²-G¹ links in the form of A¹-C(=O)-NH-G¹, and G¹ represents a single bond, it is preferable that A³-A⁴-G² as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring.
40) In the formula (I), when A¹ is -(CH₂)₃-, A¹-A²-G¹ links in the form of A¹-C(=O)-NH-G¹, and G¹ represents a single bond, it is preferable that A³-A⁴-G² as a whole is a heterocyclic substituted alkyl group consisting of a acyclic aliphatic hydrocarbon group having 1 to 6 carbon atoms and a heterocyclic group having 1 to 4 atoms selected from the group consisting of an oxygen atom, a nitrogen atom and a sulfur atom, in the ring. However, in the heterocyclic substituted alkyl group as A³-A⁴-G², a acyclic aliphatic hydrocarbon group portion having 1 to 6 carbon atoms is further preferably substituted with one or more substituents selected from those exemplified for the preferred substituents of the substituted acyclic aliphatic hydrocarbon group having 1 to 10 carbon atoms of A³, or a heterocyclic group portion is further preferably substituted with one or more substituents selected from those exemplified for the preferred substituents of the heterocyclic group having 1 to 4 atoms selected from the group consisting of an oxygen atom, a nitrogen atom and a sulfur atom, in the ring, of G² (including a case where both are substituted).
41) In the formula (I), when all of A¹, A², G¹, A³, and A⁴ represent a single bond, G² is preferably a hydrogen atom or a acyclic aliphatic hydrocarbon group having 1 to 6 carbon atoms.

Also, the preferred combinations of X, A¹, A², G¹, A³, A⁴ and G² in formula (I) as described in above 1) through 41) are more preferably combined with a preferred group represented by R²-A⁵-, exemplified as preferred combinations of R² and A⁵, that is R²-A⁵- group in which A⁵ is a single bond and R² is a substituted or unsubstituted monocyclic C₃-C₅ aromatic heterocyclic group having 1 or 2 atoms selected from the group consisting of an oxygen atom, a nitrogen atom and a sulfur atom, in the ring, or R²-A⁵- group in which R² is a substituted or unsubstituted aliphatic hydrocarbon group, and with a preferred group represented by R³-A⁶-, exemplified as preferred combinations of R³ and A⁶.

The pyrrolopyrimidinone derivative of the formula (I) has tautomeric forms represented by the following formula (III): 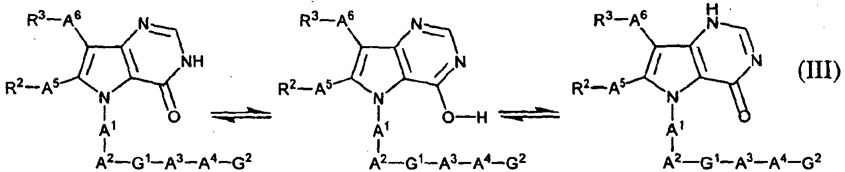 [wherein A¹, A², A³, A⁴, A⁵, A⁶, G¹, G², R², and R³ are the same as those defined above in the formula (I).]

However, needless to say all such tautomeric forms are within the scope of the present invention.

When one or more asymmetric structures exist on atoms constituting molecules of the pyrrolopyrimidinone derivative formula (I), optically active forms of the respective asymmetric structures and their mixtures combined in an arbitrary ratio are also within the scope of the present invention.

When there exist stereochemical isomers of molecules of the pyrrolopyrimidinone derivative of formula (I), the stereochemical isomers and their combinations in any are also within the scope of the present invention.

The pyrrolopyrimidinone derivative of the formula (I) may have a basic group in its molecules. In this case, if necessary, it can be converted into pharmaceutically acceptable acid addition salts. Such acids include inorganic acids such as hydrochloric acid, hydrobromic acid, sulfuric acid, phosphoric acid, and carbonic acid; or organic acids such as acetic acid, citric acid, malic acid, oxalic acid, tartaric acid, lactic acid, maleic acid, fumaric acid, and methanesulfonic acid.

The pyrrolopyrimidinone derivative of formula (I) may have an acidic group in its molecules. In this case, when required, the acidic group may be converted into pharmaceutically acceptable salts, including non-toxic cation salts, exemplified by alkali metal ions such as Na⁺ or K⁺, alkaline earth metal ions such as Mg²⁺ or Ca²⁺, metal ions such as Al³⁺ or Zn²⁺, ammonia, and salts with an organic base such as triethylamine, ethylenediamine, propanediamine, pyrrolidine, piperidine, piperazine, pyridine, lysine, choline, ethanolamine, N,N-dimethylethanolamine, 4-hydroxypiperidine, glucosamine, or N-methylglucamine.

In the formula (II), A¹, A², A³, A⁴, A⁵, A⁶, G¹, G², R² and R³ are the same as those defined above in the formula (I), and examples thereof include the same as those exemplified in the formula (I), respectively. Also preferred examples of A¹, A², A³, A⁴, A⁵, A⁶, G¹, G², R² and R³ and preferred combinations of them are the same as those described for the pyrrolopyrimidinone derivative of the present invention represented in the formula (I) except those being obstacle on the chemical reaction in both of the reaction from the pyrrolopyrimidine derivative of the present invention represented by the formula (I) to the pyrrolopyrimidinone derivative of the present invention represented by the formula (I), and the reaction from the pyrrolopyrimidine derivative represented by the formula (II) to the pyrrolopyrimidinone derivative of the present invention represented by the formula (I).

In the formula (II), X¹ represents a chlorine atom, a bromine atom, an iodine atom, a C₂-C₁₀ acylthio group, a C₂-C₈ alkoxymethylthio group, a C₁-C₈ alkyl group or a C₁-C₈ arylsulfonyloxy group, but an explanation will be given below of the case where X¹ represents a chlorine atom, a bromine atom, an iodine atom, or a C₁-C₈ alkyl or arylsulfonyloxy group. When X¹ represents a C₁-C₈ alkyl or arylsulfonyloxy group, examples of the C₁-C₈ alkyl or arylsulfonyloxy group include sulfonyloxy group consisting optionally substituted C₁-C₈ alkyl or aryl group and sulfonyl group, such as methylsulfonyloxy, trifluoromethylsulfonyloxy, ethylsulfonyloxy, propylsulfonyloxy, butylsulfonyloxy, t-butylsulfonyloxy, nonafluorobutylsulfonyloxy, phenylsulfonyloxy, p-bromophenylsulfonyloxy, p-toluylsulfonyloxy, benzylsulfonyloxy, α-phenethylsulfonyloxy and β-phenethylsulfonyloxy. Examples of such preferred X¹ include a chlorine atom, a bromine atom, an iodine atom and a trifluoromethylsulfonyloxy group. Particularly, a chlorine atom or a trifluoromethylsulfonyloxy group is more preferred.

From the compounds represented by the formula (Ic), the pyrrolopyrimidinone derivative of formula (I) of the present invention can be easily manufactured based on the technical common sense of the person skilled in the art.

In the formula (Ic), A¹, A², A³, A⁴, A⁵, A⁶, G¹, G², R², and R³ are the same as those defined above in formula (I), and examples thereof include the same as those exemplified in formula (I), respectively.

In the formula (Ic), Q represents a C₂-C₁₀ acyl group, a C₂-C₁₀ alkoxymethyl group, or a substituted or unsubstituted benzyl group. When Q represents a C₂-C₁₀ acyl group, examples of the C₂-C₁₀ acyl group include acetyl, trifluoroacetyl, propionyl, butyryl, isobutyryl, valeryl, isovaleryl, pivaloyl, hexanoyl, benzoyl, phenylacetyl, phenylpropionyl, cinnamoyl. When Q represents a C₂-C₁₀ alkoxymethyl, examples of the C₂-C₁₀ alkoxymethyl group include methoxymethyl, methoxyethoxymethyl, t-butoxymethyl, 2-(trimethylsilyl)ethoxymethyl, benzyloxymethyl, p-methoxybenzyloxymethyl, p-nitrobenzyl- oxymethyl, o-nitrobenzyloxymethyl and 4-methoxyphenoxymethyl. When Q represents a substituted or unsubstituted benzyl group, examples of the substituted or unsubstituted benzyl group include benzyl, p-methoxybenzyl, 3,4-dimethoxybenzyl, o-nitrobenzyl, p-nitrobenzyl and p-cyanobenzyl. Examples of such preferred Q include 2-(trimethylsilyl)ethoxymethyl.

The pyrrolopyrimidinone derivative of the formula (I) can be prepared from pyrrolo[3, 2-d]pyrimidine derivative of the formula (II) by the following synthesis (A).

Note that, the pyrrolopyrimidinone derivative represented by the formula (I) is described as (Ia) in the following synthesis and is sometimes expressed as a pyrrolo[3, 2-d]pyrimidine derivative.

### [Synthesis (A)]

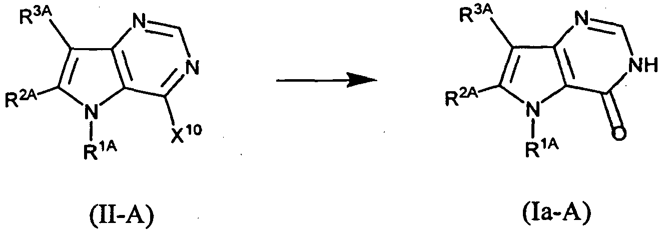

[wherein R^{1A} represents a group capable of withstanding a conversion reaction among groups defined to be represented by A¹-A²-G¹-A³-A⁴-G² in formula (I). R^{2A} represents a group capable of withstanding a conversion reaction among groups defined to be represented by R²-A⁵ in formula (I). R^{3A} represents a group capable of withstanding a conversion reaction among groups defined to be represented by R³-A⁶ in formula (I). X¹⁰ represents a chlorine atom, a bromine atom, an iodine atom, or an optionally substituted C₁-C₈ alkyl or arylsulfonyloxy group.]

In other words, the pyrrolopyrimidinone derivative Ia-A)of the present invention can be synthesized by hydrolysis of the pyrrolo[3,2-d]pyrimidine derivative (II-A). In this hydrolysis reaction, the reaction is performed by using a base such as a sodium hydroxide or a lithium hydroxide and using a solvent such as dioxane, ethanol, 2-propanol, or dimethyl sulfoxide at a temperature in a range of 0°C to 150°C.

Among the pyrrolo[3,2-d]pyrimidine derivatives of formula (II), a pyrrolo[3, 2-d]pyrimidine derivative of formula (II-B) can be synthesized from the pyrrolo[3,2-d]pyrimidine derivative of formula (Ia) by the following synthesis.

### [Synthesis (B)]

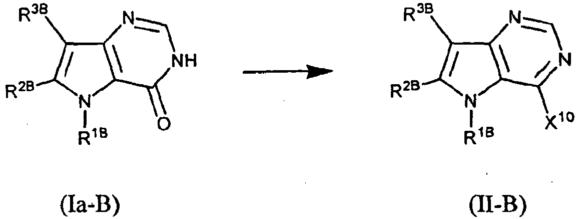

[wherein R^{1B} represents a group capable of withstanding a conversion reaction among groups defined to be represented by A^{l}-A²-G¹-A³-A⁴-G² in formula (I). R^{2B} represents a group capable of withstanding a conversion reaction among groups defined to be represented by R²-A⁵ in formula (I). R^{3B} represents a group capable of withstanding a conversion reaction among groups defined to be represented by R³-A⁶ in formula (I). X¹⁰ has the same meaning as defined above.]

In other words, when X¹⁰ is a chlorine atom, the pyrrolopyrimidinone derivative (II-B) of the present invention can be synthesized by reacting the pyrrolopyrimidinone derivative (Ia-B) of the present invention with phosphorus oxychloride. In the chlorination using phosphorus oxychloride, the reaction is carried out in a solvent such as acetonitrile under general chlorination reaction conditions, for example, in the presence or absence of a solvent such as triethylamine, 4-dimethylaminopyridine or dimethyl type aniline, at a temperature in a range of 0°C to 150°C.

Also, when X¹⁰ is a trifluoromethanesulfonyloxy group, the pyrrolopyrimidinone derivative (II-B) of the present invention can be synthesized by reacting the pyrrolopyrimidinone derivative (Ia-B) of the present invention with trifluoromethanesulfonic anhydride. In trifluoromethane sulfonyloxylation using trifluoromethane sulfonic anhydride, the reaction can be carried out together with pyridine or amines such as triethylamine in the presence or absence of a solvent such as dichloromethane at a temperature in a range of 0°C to 100°C.

Among the pyrrolo[3,2-d]pyrimidine derivatives of formula (Ia-B), the pyrrolo[3,2-d]pyrimidine derivative of formula (Ia-B1) can be synthesized from the 7-cyanopyrrolo[3,2-d]pyrimidine derivative of the formula (Ia-CN) by the following synthesis (B1).

### [Synthesis (B1)]

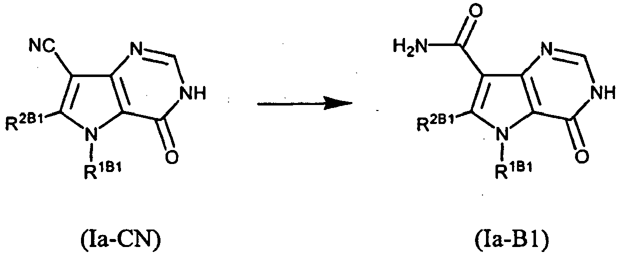

[wherein R^{1B1} represents a group capable of withstanding a conversion reaction among groups defined to be represented by A¹-A²-G¹-A³-A⁹-G² in the formula (I). R^{2B1} represents a group capable of withstanding a conversion reaction among groups defined to be represented by R²-A⁵ in the formula (I).]

In other words, the pyrrolopyrimidinone derivative (Ia-B1) of the present invention can be synthesized by the hydrolyzing pyrrolo[3,2-d]pyrimidine derivative (Ia-CN). The hydrolysis reaction is carried out using a base such as sodium hydroxide or lithium hydroxide in a solvent such as ethanol, 2-propanol or dimethylsulfoxide in the presence or,absence of hydrogen peroxide at a temperature in a range of 0°C to 100°C.

Among the pyrrolo[3,2-d]pyrimidine derivatives of the formula (Ia-B), a pyrrolo[3,2-d]pyrimidine derivative of the formula (Ia-B2) can be synthesized from the pyrrolo[3,2-d]pyrimidine derivative of the formula (Ia-B1) by the following synthesis (B2).

### [Synthesis (B2)]

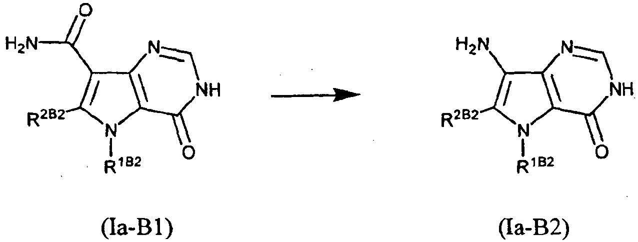

[wherein R^{1B2} represents a group capable of withstanding a conversion reaction among groups defined to be represented by A¹-A²-G¹-A³-A⁴-G² in formula (I). R^{2B2} represents a group capable of withstanding a conversion reaction among groups defined to be represented by R²-A⁵ in formula (I).]

In other words, the pyrrolopyrimidinone derivative (Ia-B2) of the present invention can be synthesized by performing a Hoffmann rearrangement on the pyrrolopyrimidinone derivative (Ia-B1) of the present invention. The Hoffmann rearrangement is carried out in a solvent such as ethanol, 2-propanol, acetonitrile or water, using a reagent such as sodium hypochlorite, bromine, or benzyltrimethyl ammonium tribromide in the presence or absence of a base such as sodium hydroxide at a temperature of 0°C to 150°C.

Among the pyrrolo[3,2-d]pyrimidine derivatives of formula (Ia-B), a pyrrolo[3,2-d]pyrimidine derivative of formula (Ia-B3) can be synthesized from the pyrrolo[3,2-d]pyrimidine derivative of formula (Ia-B2) by the following synthesis (B3).

### [Synthesis (B3)]

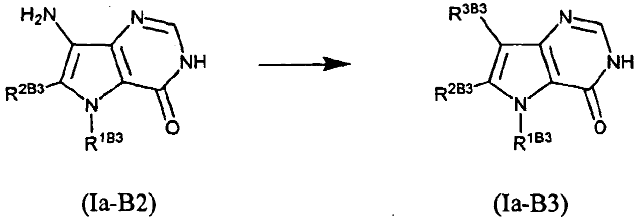

[wherein R^{1B3} represents a group capable of withstanding a conversion reaction among groups defined to be represented by A¹-A²-G¹-A³-A⁴-G² in the formula (I). R^{2B3} represents a group capable of withstanding a conversion reaction among groups defined to be represented by R²-A⁵ in the formula (I). R^{3B3} represents a fluorine atom, a chlorine atom, a bromine atom or an iodine atom.]

In other words, the pyrrolopyrimidinone derivative (Ia-B3) of the present invention can be synthesized by reacting the pyrrolopyrimidinone derivative (Is-B2) of the present invention with nitrous acid or nitrite ester and performing a Sandmayer reaction. In the Sandmayer reaction using nitrous acid or nitrite ester, reagents, for example, nitrous acid, sodium nitrite, isoamyl nitrite, or t-butyl nitrite is used, and the reaction can be performed in the presence of halogenation reagents, for example hydrofluoric acid or fluoroboric acid for fluorination, for example copper chloride or carbon tetrachloride for chlorination, for example carbon tetrabromide or bromoform for bromination, and diiodomethane or iodine for iodination, in the presence or absence of an acid such as sulfuric acid or hydrochloric acid, in the presence or absence of an acid such as sulfuric acid or hydrochloric acid, by using or without using a solvent such as ethanol, acetonitrile or water, at a temperature in a range of 0°C to 150°C

Among the pyrrolo[3,2-d]pyrimidine derivatives of formula (Ia-B), a pyrrolo[3,2-d]pyrimidine derivative of formula (Ia-B4) can be synthesized from the pyrrolo[3,2-d]pyrimidine derivative of formula (Ia-B2) by the following synthesis (B4).

### [Synthesis (B4)]

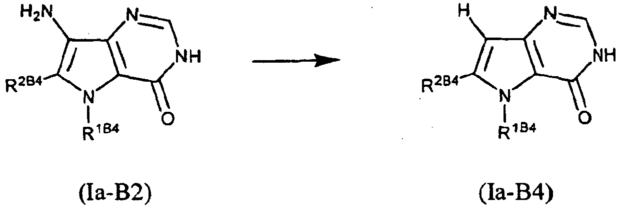

[wherein R^{1B4} represents a group capable of withstanding a conversion reaction among groups defined to be represented by A¹-A²-G¹-A³-A⁴-G² in the formula (I). R^{2B4} represents a group capable of withstanding a conversion reaction among groups defined to be represented by R²-A⁵ in the formula (I).]

In other words, the pyrrolopyrimidinone derivative (Ia-B4) of the present invention can be synthesized by reacting the pyrrolopyrimidinone derivative (Ia-B2) of the present invention with nitrous acid or nitrite ester. The reaction using nitrous acid or nitrite ester can be performed by using nitrous acid, sodium nitrite, isoamyl nitrite, or t-butyl nitrite as a reagent, in the presence of or in the absence of an acid such as sulfuric acid or hydrochloric acid in the presence of dimethylformamide, tetrahydrofuran, ethanol or water as a solvent, at a temperature in a range of 0°C to 150°C.

Among the pyrrolo[3,2-d]pyrimidine derivatives of formula (Ia-B), a pyrrolo[3,2-d]pyrimidine derivative of formula (Ia-B5) can be synthesized from the pyrrolo[3,2-d]pyrimidine derivative of formula (Ia-B4) by the following synthesis (B5).

### [Synthesis (B5)]

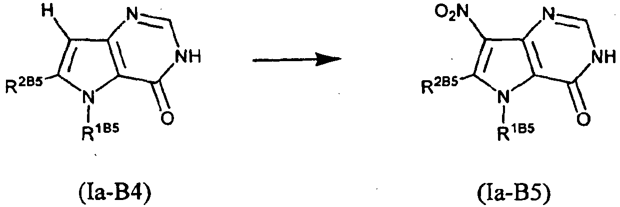

[wherein R^{1B5} represents a group capable of withstanding a conversion reaction among groups defined to be represented by A¹-A²-G¹-A³-A⁴-G² in the formula (I). R^{2B5} represents a group capable of withstanding a conversion reaction among groups defined to be represented by R²-A⁵ in the formula (I).]

In other words, the pyrrolopyrimidinone derivative (Ia-B5) of the present invention can be synthesized by reacting nitric acid or nitrogen dioxide with the pyrrolopyrimidinone derivative (Ia-B4) of the present invention. The reaction using nitric acid or nitrogen dioxide can be performed by using nitric acid, nitrogen dioxide, cerium ammonium nitrate or sodium nitrite as a reagent, in the presence or absence of sulfuric acid, hydrochloric acid, acetic acid or ozone, in the presence of dichloroethane, dichloromethane, acetonitrile or water as a solvent, at a temperature in a range of 0°C to 100°C.

Among the pyrrolo[3,2-d]pyrimidine derivatives of formula (Ia-B), a pyrrolo[3,2-d]pyrimidine derivative of formula (Ia-B6) can be synthesized from the pyrrolo[3,2-d]
pyrimidine derivative of formula (Ia-B6a) by the following synthesis (B6).

### [Synthesis (B6)]

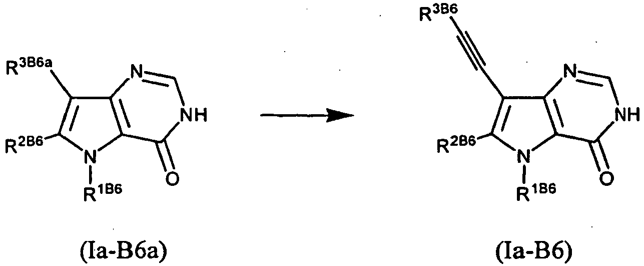

[wherein R^{1B6} represents a group capable of withstanding a conversion reaction among groups defined to be represented by A¹-A²G¹-A³-A⁴-G² in the formula (I). R^{2B6} represents a group capable of withstanding a conversion reaction among groups defined to be represented by R²-A⁵ in the formula (I). R^{3B6a} is a bromine atom or iodine atom, and among groups defined as R³ in the formula (I), R^{3B6} is a substituted or unsubstituted saturated aliphatic hydrocarbon group having 1 to 10 carbon atoms, a substituted or unsubstituted alicyclic hydrocarbon group having 3 to 8 carbon atoms, a substituted or unsubstituted aromatic hydrocarbon group having 6 to 14 carbon atoms, a monocyclic C₃-C₅ aromatic heterocyclic group having 1 or 2 atoms selected from the group consisting of an oxygen atom, a nitrogen atom and a sulfur atom on the substituted or unsubstituted ring, or a trimethylsilyl.]

In other words, the pyrrolopyrimidinone derivative (Ia-B6) of the present invention can be synthesized by reacting the pyrrolopyrimidinone derivative (Ia-B6a) of the present invention with a terminal alkyne derivative represented by formula R^{3B6}-C≡C-H in the presence of a catalytic amount of palladium. The reaction with the terminal alkyne derivative using the catalytic amount of palladium is carried out using the terminal alkyne derivative together with a palladium catalyst, e.g., tetrakis(triphenylphosphine) palladium, chlorobis(triphenylphosphine) palladium, or palladium acetate, in the presence or absence of a ligand, such as triphenylphosphine, tri(o-tolyl)phosphine, or 1,1'-bis(diphenylphosphino)ferrocene, in the presence or absence of a catalytic amount of copper salts, e.g., copper iodide or copper bromide, in the presence of a base such as triethylamine, diethylamine, piperizine or pyrrolidine, using solvents such as tetrahydrofuran, dimethylformamide, and toluene, at a temperature in a range of 0°C to 150°C.

Among the pyrrolo[3,2-d]pyrimidine derivatives of the formula (Ia-B), a pyrrolo[3,2-d]pyrimidine derivative of formula (Ia-B7) can be prepared from the pyrrolo[3,2-d]
pyrimidine derivative of formula (Ia-B7a) by the following synthesis (B7).

### [Synthesis (B7)]

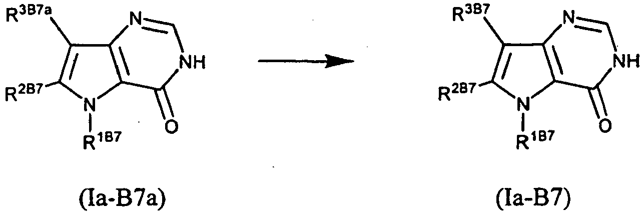

[wherein R^{1B7} represents a group capable of withstanding a conversion reaction among groups defined to be represented by A¹-A ²-G¹-A³-A⁴-G² in the formula (I). R^{2B7} represents a group capable of withstanding a conversion reaction among groups defined to be represented by R²-A⁵ in the formula (I). R^{3B7a} is a bromine atom or an iodine atom. R^{3B7} is a substituted or unsubstituted aromatic hydrocarbon group having 6 to 14 carbon atoms, or an aromatic heterocyclic group having 1 or 2 atoms selected from the group consisting of an oxygen atom, a nitrogen atom and a sulfur atom on the substituted or unsubstituted ring among groups defined as R³ in the formula (I).]

In other words, the pyrrolopyrimidinone derivative (Ia-B7) of the present invention can be synthesized, in the presence of a catalytic amount of palladium, by adding a boric acid derivative (R^{3B7}-B(OR)₂, wherein R^{3B7} is the same as defined above in the synthesis(B7), and R represents a hydrogen atom or an alkyl group] to the pyrrolopyrimidinone derivative (Ia-B7a) of the present invention. That is, in the reaction with the boric acid derivative using the catalytic amount of palladium, the reaction can be performed by using, together with the boric acid derivative, a palladium catalyst, for example, chlorobis(triphenylphosphine) palladium, palladium acetate, and tris(dibenzylideneacetone)dipalladiumuchloroform adduct in the presence or absence of a ligand, such as triphenylphosphine, tri(o-tolyl)phosphine, or 1,1'-bis(diphenylphosphino)ferrocene, in the presence of base such as potassium phosphate, sodium carbonate, potassium hydroxide, or sodium ethoxide, using a solvent such as tetrahydrofuran, dimethylformamide, 2-propanol and water, at a temperature in a range of 0°C to 150°C.

Among the pyrrolo[3,2-d]pyrimidine derivatives of formula (Ia-B), a pyrrolo[3,2-d]pyrimidine derivative of formula (Ia-B8) can be synthesized from the pyrrolo[3,2-d]
pyrimidine derivative of formula (Ib-B8a) by the following synthesis (B8).

### [Synthesis (B8)]

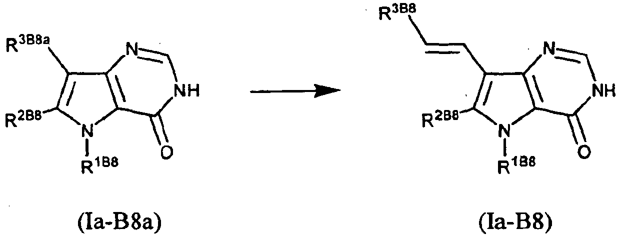

[wherein R^{1B8} represents a group capable of withstanding a conversion reaction among groups defined to be represented by A¹-A²-G¹-A³-A⁴-G² in the formula (I). R^{2B8} represents a group capable of withstanding a conversion reaction among groups defined to be represented by R²-A⁵ in the formula (I). R^{3B8a} is a bromine atom or an iodine atom, and R^{3B8} is a group defined as R³ in the formula (I).]

In other words, the pyrrolopyrimidinone derivative (Ia-B8) of the present invention can be synthesized by reacting a terminal alkene derivative upon the pyrrolopyrimidinone derivative (Ia-B8a) of the present invention in the presence of a catalytic amount of palladium. That is, in the reaction with a terminal alkene derivative using the catalytic amount of palladium, the reaction can be performed by using, together with the terminal alkene derivative, a palladium catalyst, for example, palladium chloride, palladium acetate, or tris(dibenzylideneacetone)dipalladium-chloroform adduct in the presence or absence of a ligand, such as triphenylphosphine, tri(o-tolyl)phosphine, or 1,1'-bis(diphenylphosphino)
ferrocene, in the presence of a base such as a potassium phosphate, potassium carbonate or triethylamine, and using a solvent such as tetrahydrofuran,
dimethylformamide or water, at a temperature in a range of 0°C to 150°C.

Alternatively, the pyrrolopyrimidinone derivative (Ia-B8) of the present invention can also be synthesized by performing a catalytic semi-reduction or hydroboration-protonation on the pyrrolo[3,2-d]pyrimidine derivative (Ia-B6) having an alkynyl group prepared by the Synthesis (B6). For example, the catalytic semi-reduction is performed using a solvent such as methanol, ethanol or tetrahydrofuran, in the presence of a palladium catalyst, e.g., palladium-barium sulfate-quinoline, palladium-activated carbon-quinoline, under a hydrogen atmosphere, at a temperature in a range of 0°C to 100°C. The hydroboration-protonation is performed such that hydroboratino is performed using a hydroborating reagent, e.g., 9-borabicyclo[3.3.1]nonane or dicyclohexylborane, and protonation is then performed using acetic acid. The reaction can be performed using a solvent such as tetrahydrofuran, diethylether, methylenedichloride, or toluene, at a temperature in a range of 0°C to 100°C.

Among the pyrrolo[3,2-d]pyrimidine derivatives of formula (Ia-B), a pyrrolo[3,2-d]pyrimidine derivative of formula (Ia-B9) can be synthesized from the pyrrolo [3,2-d]pyrimidine derivative of formula (Ia-B9a) by the following synthesis (B9).

### [Synthesis (B9)]

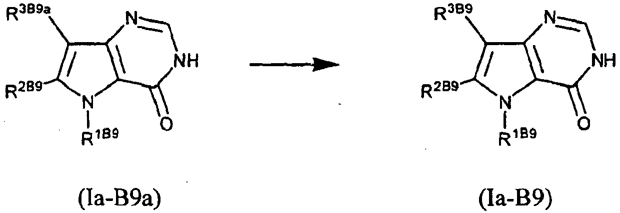

[wherein R^{1B9} represents a group capable of withstanding a conversion reaction among groups defined to be represented by A¹-A²-G¹-A³-A⁴-G² in formula (I). R^{2B9} represents a group capable of withstanding a conversion reaction among groups defined to be represented by R²-A⁵ in formula (I). R^{3B9a} is a bromine atom or an iodine atom. R^{3B9} is a substituted or unsubstituted saturated aliphatic hydrocarbon group having 1 to 10 carbon atoms, substituted or unsubstituted C₁-C₁₀ alicyclic hydrocarbon group, or a vinyl group.]

In other words, the pyrrolopyrimidinone derivative (Ia-B9) of the present invention can be synthesized by reacting an organometallic reagent to the pyrrolopyrimidinone derivative (Ia-B9a) of the present invention using a catalytic amount of palladium or nickel. For example, in the reaction with the organometallic reagent using the catalytic amount of palladium or nickel, an organozinc reagent, e.g., phenylzinc chloride or an organozinc compound prepared from a Grignard reagent and zinc chloride, an organotin reagent, e.g., phenyltrimethyltin or tetramethyltin can be used. As the Grignard reagent, organometallic reagents, such as phenylbromomagnesium or n-butylbromomagnesium, can be used. Useful examples of the palladium catalyst include tetrakis(triphenylphosphine) palladium, tris(dibenzylideneacetone)dipalladium-chloroform adduct, chloro{1,1'-bis (diphenylphosphino)ferrocene}palladium, and the like. Useful examples of the nickel catalyst include chloro{1,3-bis
(diphenylphosphino)propane}nickel or nickel bromide. The reaction can be performed using a solvent such as diethylether, tetrahydrofuran or dimethylformamide, in the presence or absence of a ligand, such as triphenylphosphine, tri(o-tolyl)phosphine, or 1,1'-bis(diphenylphosphino)
ferrocene, at a temperature in a range of 0°C to 150°C.

The pyrrolopyrimidinone derivative (Ia-B9) can also be synthesized through hydrogen reduction of the pyrrolo[3,2-d]
pyrimidine derivative (Ia-B6) having an alkynyl group, prepared by the synthesis (B6) or the pyrrolo[3,2-d]pyrimidine derivative (Ia-B8) having an alkenyl group prepared by the synthesis (B8). The hydrogen reduction is performed using a solvent such as methanol, ethanol or tetrahydrofuran in the presence of a catalytic amount of palladium-activated carbon under a hydrogen atmosphere at a temperature in a range of 0°C to 100°C.

Among the pyrrolo[3,2-d]pyrimidine derivatives of formula (Ia-B), a pyrrolo[3,2-d]pyrimidine derivative of formula (Ia-B10) can be synthesized from the pyrrolo[3,2-d]
pyrimidine derivative of formula (Iz-10a) by the following synthesis (B10).

### [Synthesis (B10)]

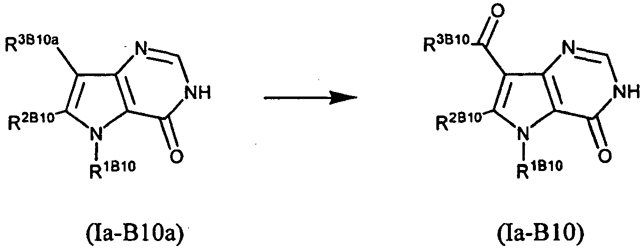

[wherein R^{1B10} represents a group capable of withstanding a conversion reaction among groups defined to be represented by A¹-A²-G¹-A³-A⁴-G² in the formula (I). R^{2B10} represents a group capable of withstanding a conversion reaction among groups defined to be represented by R²-A⁵ in the formula (I). R^{3B10a} is a bromine atom or an iodine atom. R^{3B10} is a C₂-C₁₀ hydroxyl, alkoxy, N-substituted amino or N,N-disubstituted amino group.

In other words, the pyrrolopyrimidinone derivative (Ia-B10) of the present invention can be synthesized by reacting the pyrrolopyrimidinone derivative (Ia-B10a) of the present invention with carbon monoxide in the presence of a catalytic amount of palladium. For example, the carbonyl insertion reaction using a catalytic amount of palladium is performed under a carbon monoxide atmosphere, using a palladium catalyst, e.g., tetrakis(triphenylphosphine) palladium, palladium acetate, or tris(dibenzylideneacetone)dipalladium-chloroform adduct, in the presence or absence of a ligand, e.g., triphenylphosphine, tri(o-tolyl)phosphine, or 1,1'-bis(diphenylphosphino)ferrocene, in the presence or absence of a base, e.g., potassium carbonate, or triethylamine. A solvent such as acetonitrile, tetrahydrofuran, or dimethylformamide is used, and the reaction is carried out at a temperature ranging between 0°C and 150°C. In this case, addition of water as a reacting agent gives a compound with a carboxy group, and addition of an alcohol gives a compound with an alkoxycarbonyl group. Addition of a primary or secondary amine gives a compound with N-substituted or N,N-disubstituted aminocarbonyl group.

Among the pyrrolo[3,2-d]pyrimidine derivatives of formula (Ia-B), a pyrrolo[3,2-d]pyrimidine derivative of formula (Ia-B11) can be synthesized from the pyrrolo [3,2-d]pyrimidine derivative of formula (Ia-11a) by the following synthesis (B11).

### [Synthesis (B11)]

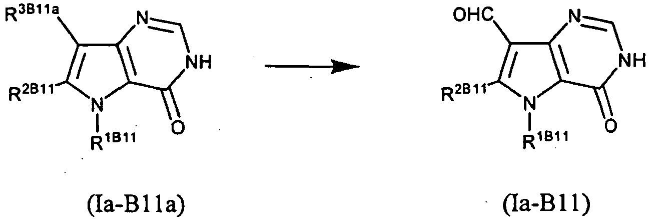

[wherein R^{1B11} represents a group capable of withstanding a conversion reaction among groups defined to be represented by A¹-A²-G¹-A³-A⁴-G² in the formula (I). R²B¹¹ represents a group capable of withstanding a conversion reaction among groups defined to be represented by R²-A⁵ in the formula (I). R^{2B11a} is a bromine atom or an iodine atom.]

In other words, the pyrrolopyrimidinone derivative (Ia-B11) of the present invention can be synthesized by reacting the pyrrolo[3, 2-d]pyrimidine derivative (Ia-B11a) of the present invention under a carbon monoxide atmosphere in the presence of a reducing agent and a catalytic amount of palladium. For example, the formylation reaction using a catalytic amount of palladium is performed under the carbon monoxide atmosphere. Useful examples of the palladium catalyst include tetrakis(triphenylphosphine) palladium, palladium acetate, tris(dibenzylideneacetone)dipalladium-chloroform adduct. The reaction is performed using a solvent such as acetonitrile, tetrahydrofuran, or dimethylformamide in the presence or absence of a ligand such as triphenylphosphine or tri(o-tolyl)phosphine or 1,1'-bis(diphenylphosphino)
ferrocene in a temperature range of 0°C to 150°C. The reaction is performed in the presence of or in the absence of a base such as potassium carbonate or triethylamine. Addition of a reducing agent such as tributyltin hydride or triethylsilane gives a compound with a formyl group, and addition of an organometallic agent such as alkyl zinc, alkyl boron or an organotin reagent give a compound with an alkylcarbonyl group.

Among the pyrrolo[3,2-d]pyrimidine derivatives of formula (Ia-B), a pyrrolo[3,2-d]pyrimidine derivative of formula (Ia-B12) can be synthesized from the pyrrolo [3,2-d]pyrimidine derivative of formula (Ia-B12a) by the following synthesis (B12).

### [Synthesis (B12)]

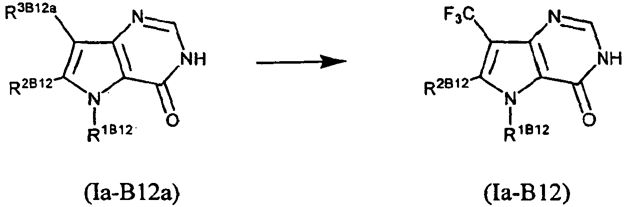

[wherein R^{1B12} represents a group capable of withstanding a conversion reaction among groups defined to be represented by A¹-A²-G¹-A³-A⁴-G² in the formula (I). R^{2B12} represents a group capable of withstanding a conversion reaction among groups defined to be represented by R²-A⁵ in the formula (I). R^{3B12a} is a bromine atom or an iodine atom.]

In other words, the pyrrolopyrimidinone derivative (Ia-B12) of the present invention can be synthesized by reacting the pyrrolopyrimidinone derivative (Ia-B12a) of the present invention with a trifluoromethyl donating reagent. That is, in the trifluoromethylation reaction, the reaction can be performed by utilizing various methods, for example, a method using copper (I) iodide or cesium fluoride together with a trifluoromethyl donator such as sodium trifluoroacetate or trifluoromethyl acetate, a method for preparing a trifluoromethyl copper compound from a trifluoromethyl zinc compound or a trifluoromethyl cadmium compound and copper (I) bromide, or a method for preparing a trifluoromethyl copper compound from a trifluoromethyl iodide and copper powder, by using a solvent such as dimethylformamide, N-methylpyrrolidinone, hexamethylphosphoramide, acetonitrile, or pyridine, at a temperature in a range of 0°C to 150°C.

Among the pyrrolo[3,2-d]pyrimidine derivatives of formula (Ia-B), a pyrrolo[3,2-d]pyrimidine derivative of Formula (Ia-B13) can be synthesized from the pyrrolo [3,2-d]pyrimidine derivative of Formula (Ia-B13a) by the following synthesis (B13).

### [Synthesis (B13)]

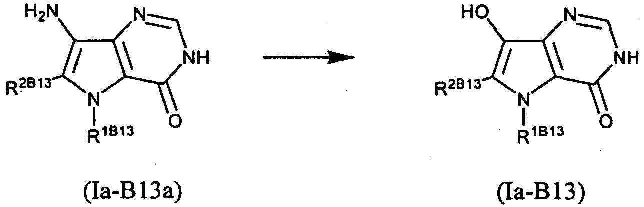

[wherein R^{1B13} represents a group capable of withstanding a conversion reaction among groups defined to be represented by A¹-A²-G¹-A³-A⁴-G² in the formula (I). R^{2B13} represents a group capable of withstanding a conversion reaction among groups defined to be represented by R²-A⁵ in the formula (I).]

The pyrrolopyrimidinone derivative (Ia-B13) of the present invention can be synthesized by reacting the pyrrolopyrimidinone derivative (Ia-B13a) of the present invention with water in the presence of nitrous acid. That is, the hydroxylation reaction in the presence of nitrous acid is performed using sodium nitrite or isoamyl nitrite in the presence of trifluoroacetic acid or sulfuric acid. The reaction can be performed using water as a solvent in the presence or absence of a cosolvent such as acetonitrile or dimethylformamide, at a temperature in a range of 0°C to 150°C.

Among the pyrrolo[3,2-d]pyrimidine derivatives of formula (Ia-B), a pyrrolo[3,2-d]pyrimidine derivative of formula (Ia-B14) can be synthesized from the pyrrolo [3,2-d]pyrimidine derivative of formula (Ia-B14a) by the following synthesis (B14).

### [Synthesis (B14)]

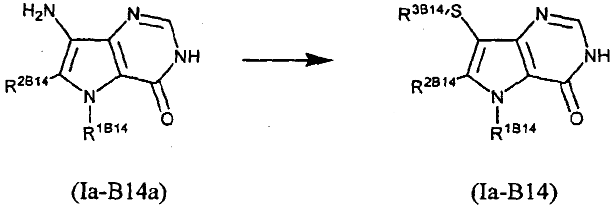

[wherein R^{1B14} represents a group capable of withstanding a conversion reaction among groups defined to be represented by A¹-A²-G¹-A³-A⁴-G² in the formula (I). R^{2B14} represents a group capable of withstanding a conversion reaction among groups defined to be represented by R²-A⁵ in the formula (I). R^{3B14} is a C₁-C₆ aliphatic hydrocarbon group.]

In other words, the pyrrolopyrimidinone derivative (Ia-B14) of the present invention can be synthesized by reacting the pyrrolopyrimidinone derivative (Ia-B14a) of the present invention with water in the presence of nitrous acid. That is, the alkylthioration reaction in the presence of nitrous acid is performed using sodium nitrite or isoamyl nitrite in the presence or absence of acids such as hydrochloric acid or sulfuric acid. The reaction is carried out using dialkyldisulfide or alkanethiol as a reagent in a solvent such as acetonitrile or dimethylformamide at a temperature in a range of 0°C to 150°C.

Among the pyrrolo[3,2-d]pyrimidine derivatives represented by (Ia-B) of the synthesis (B) or (Ib-CN) of the synthesis (B1), a pyrrolo[3,2-d]pyrimidine derivative of formula (Ia-C2) can be synthesized from the pyrrolo [3,2-dlpyrimidine derivative of formula (Ia-C1) by the following synthesis (C).

### [Synthesis (C)]

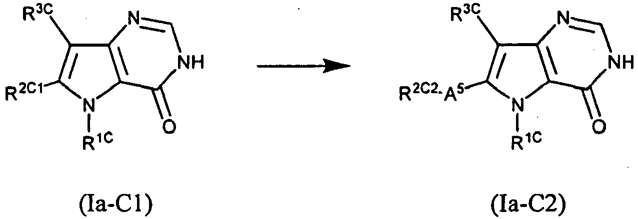

[wherein R^{1c} represents a group capable of withstanding a conversion reaction among groups defined to be represented by A¹-A²-G¹-A³-A⁴-G² in the formula (I). R^{2C1} is a chlorine atom or a bromine atom. R^{3C} represents a cyano group or a group capable of withstanding a conversion reaction among groups defined to be represented by R²-A⁵ in the formula (I). When A⁵ is - NR²⁰¹- (R²⁰¹ is the same as defined above for R²⁰¹ in the formula (1)), R^{2C2} is as defined to exclude a fluorine atom, a chlorine atom, a bromine atom and an iodine atom from groups defined for R² in formula (I). Also, when A⁵ is a single bond, R^{2C2} is a heterocyclic group having 1 to 4 atoms selected from the group consisting of an oxygen atom, a nitrogen atom and a sulfur atom on the substituted or unsubstituted ring in which R^{2C2} is linked to A⁵ on a nitrogen atom.

In other words, the pyrrolopyrimidinone derivatives (Ia-C2) can be synthesized by reacting the pyrrolopyrimidinone derivatives (Ia-C1) of the present invention with a primary or secondary amine. Amination using the primary or secondary amine is performed without the use of a solvent or with the use of a solvent such as dimethylsulfoxide, dimethylformamide, dioxane, tetrahydrofuran or toluene in the presence or absence of a base such as pyridine, triethylamine, diisopropylethylamine, 4-dimethylaminopyridine or sodium carbonate. The reaction is performed in the presence or absence of a transition metal complex catalyst prepared by mixing a palladium salt such as palladium acetate with a phosphorus ligand such as triphenylphosphine, at a temperature in a range of 0°C to 150°C.

Among the pyrrolo[3,2-d]pyrimidine derivatives represented by the formula (Ia) or (Ia-CN) of the synthesis (B1), a pyrrolo[3,2-d]pyrimidine derivative of formula (Ia-D2) can be synthesized from the pyrrolo[3,2-d]pyrimidine derivative of formula (Ia-D1) by the following synthesis (D).

### [Synthesis (D)]

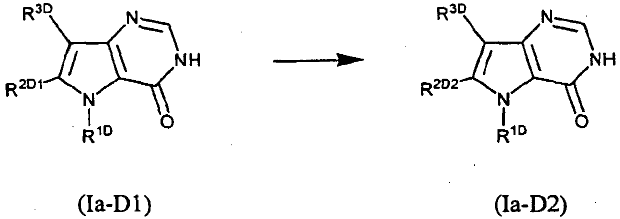

[wherein R^{1D} represents a group capable of withstanding a conversion reaction among groups defined to be represented by A¹-A²-G¹-A³-A⁴-G² in the formula (I). R^{2D1} is a chlorine atom or a bromine atom. R^{2D2} is a substituted or unsubstituted aromatic hydrocarbon group having 6 to 14 carbon atoms, or an aromatic heterocyclic group having 1 or 2 atoms selected from the group consisting of an oxygen atom, a nitrogen atom and a sulfur atom on the substituted or unsubstituted ring. R^{3D} is a cyano group or a group capable of withstanding a conversion reaction among groups defined to be represented A⁶-R³ in the formula (I).]

In other words, the pyrrolopyrimidinone derivative (Ia-D2) of the present invention can be synthesized by reacting the pyrrolopyrimidinone derivative (Ia-D1) of the present invention with, for example, a boric acid derivative represented by R^{2D2}-B (OR)₂ [in which R^{2D2} is as defined above in the synthesis (D), and R is a hydrogen atom or an alkyl group]. The reaction with the boric acid derivative is performed under general Suzuki reaction conditions, for example, at a temperature in a range of 0°C to 150°C using a solvent such as 2-propanol and/or water in the presence of an inorganic base such as sodium carbonate, by using a catalyst such as palladium acetate, and adding a ligand such as triphenylphosphine.

Among the pyrrolo[3,2-d]pyrimidine derivatives of Formula (Ia) or (Ia-CN) prepared in the synthesis (B1), a pyrrolo[3,2-d]pyrimidine derivative of formula (Ia-E2) can be synthesized from the pyrrolo[3,2-d]pyrimidine derivative of formula (Ia-E1) in the following manner shown in Synthesis (E):

### [Synthesis (E)]

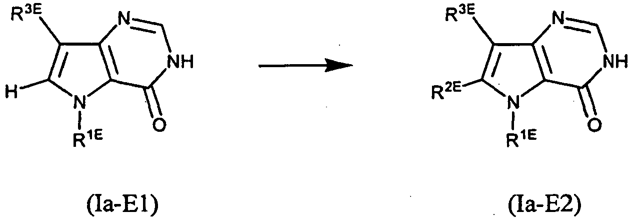

[wherein R^{1E} represents a group capable of withstanding a conversion reaction among groups defined to be represented by A¹-A²-G¹-A³-A⁴-G² in the formula (I). R^{2E} is a chlorine atom, a bromine atom or an iodine atom. R^{3E} is a cyano group or a group capable of withstanding a conversion reaction among groups defined to be represented by A⁶-R³.]

In other words, the pyrrolopyrimidinone derivative (Ia-E2) of the present invention can be synthesized by halogenation of the pyrrolopyrimidinone derivative (Ia-E1) of the present invention. The halogenation is performed using a halogenation reagent such as N-chlorosuccinic imide or N-bromosuccinic imide in the presence of a solvent such as dimethylformamide, dioxane or tetrahydrofuran at a temperature in a range of -20°C to 150°C.

Among the pyrrolo[3,2-d]pyrimidine derivatives of Formula (Ia) or (Ia-CN) prepared in the synthesis (B1), a pyrrolo[3,2-d]pyrimidine derivative of formula (1a-F) given below can be synthesized from the pyrrol derivative of formula (IV-F) in the following manner shown in Synthesis (F):

### [Synthesis (F)]

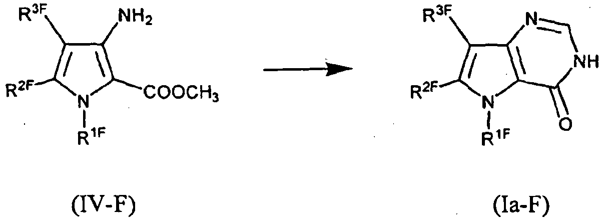

[wherein R^{1F} represents a group capable of withstanding a conversion reaction among groups defined to be represented by A¹-A²-G¹-A³-A⁴-G² in the formula (I). R^{2F} represents, among the groups defined for R² in the formula (I), groups excluding a fluorine atom, a chlorine atom, a bromine atom, an iodine atom, and a substituted or unsubstituted heterocyclic group that is bonded with a carbon atom and a nitrogen atom of a pyrrole ring to which R^{2F} is bonded, and having 1 to 4 atoms selected from the group consisting of an oxygen atom, a nitrogen atom and a sulfur atom, in the ring. R^{3F} is a cyano group or a group capable of withstanding a conversion reaction among groups defined to be represented by A⁶-R³ in formula (I).]

In other words, the pyrrolo[3,2-d]pyrimidine derivative of the formula (Ia-F) of the present invention can be synthesized by performing a cyclization reaction using formamidine or formamide on the pyrrole derivative of formula (IV-F). The cyclization reaction using formamidine can be performed by using formamidine acetate, for example, in a solvent such as 2-propanol at a temperature in a range of 0°C to 150°C. The cyclization reaction using formamide can be performed smoothly by using a base such as formamide or sodiummethoxide, in the presence or absence of a solvent such as dimethylsulfoxide or dimethoxyethane at a temperature in a range of 0°C to 150°C.

The pyrrolo[3,2-d]pyrimidine derivatives of the formula (Ia) or the synthesis (B1) can be synthesized from the pyrrolo
[3,2-d]pyrimidine derivative of the Formula (I-G) by the following synthesis (G).

### [Synthesis (G)]

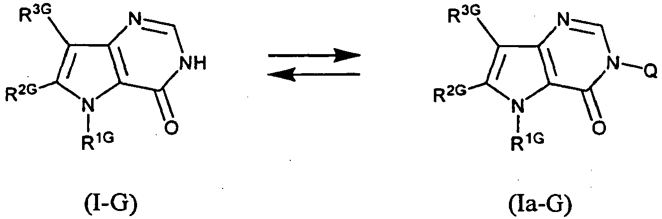

[wherein R^{1G} represents a group capable of withstanding a conversion reaction among groups defined to be represented by A¹-A²-G¹-A³-A⁴-G² in the formula (I). R^{2G} represents a group capable of withstanding a conversion reaction among groups defined to be represented by R²-A⁵ in the formula (I). R^{3G} is a cyano group or a group capable of withstanding a conversion reaction among groups defined to be represented by A⁶-R³ in formula (I). Q is an optionally substituted C₂-C₁₀ acyl group, an optionally substituted a C₂-C₈ alkoxymethyl group, or substituted or unsubstituted benzy group.]

In other words, when for example Q is an acyl group, the pyrrolopyrimidinone derivative (Ib-G) of the present invention can be synthesized by reacting the pyrrolopyrimidinone derivative (I-G) of the present invention with an acyl halide. The acylation reaction using the acyl halide is performed under conventional acylation reaction conditions, for example, in the presence of triethylamine or pyridine, at a temperature in a range of 0°C to 100°C.

Also, when Q is for example an alkoxymethyl or benzyl group, the pyrrolopyrimidinone derivative (I-G) of the present invention can be synthesized by reacting the pyrrolo[3, 2-d]pyrimidine derivative (I-G) of the present invention with an alkoxymethyl halide or a benzyl halide. The reaction using the alkoxymethyl halide or the benzyl halide can be performed in the presence of for example sodium hydride in a temperature range of 0°C to 100°C.

In the thus obtained pyrrolopyrimidinone derivatives (Ib-G) according to the present invention, conversion reactions known to one skilled in the art can be performed for A¹, A², A³, A⁴, A⁵, A⁶, G¹, G², R² and/or R³. Such pyrrolo[3,2-d]
pyrimidine derivatives (Ib-G) can be converted into the pyrrolopyrimidinone derivatives(I-G) of the present invention by performing hydrolysis under a neutral or alkaline condition when Q is an acyl group, or under an acidic condition using, for example, trifluoroacetic acid, when Q is an alkoxymethyl group, or by performing a hydrogen addition reaction when R³ is a benzyl group.

The pyrrolopyrimidinone derivatives or the present invention synthesized by the syntheses (A), (B), (C), (D), (E), (F), and (G) have easily convertible substituents, such as an alkoxycarbonyl group, an acyloxy group, or an aromatic nitro group, they can be easily converted into pyrrolopyrimidinone derivatives or the present invention respectively having a carboxy group, a hydroxy group, and an amino group by performing reactions known to one skilled in the art.

When the pyrrolopyrimidinone derivatives of the present invention synthesized by the synthesis (A), (B), (C), (D), (E), (F), and (G) have a carboxy group, they can be converted into pyrrolopyrimidinone derivatives of the present invention having an alkoxycarbonyl group, a carbamoyl group, and an N-alkylcarbamoyl group by a condensation reaction known to one skilled in the art.

When the pyrrolopyrimidinone derivatives of the synthesized by the synthesis (A), (B), (C), (D), (E), (F), and (G) have an amino group, they can be converted into pyrrolopyrimidinone derivatives of the present invention having an acylamino group or an alkylsulfonylamino group by a condensation reaction well known to one skilled in the art.

Also, when they have an amino group, they can also be converted into pyrrolopyrimidinone derivatives of the present invention having a monoalkylamino or a dialkylamino group by a reductive alkylation reaction known to one skilled in the art.

When the pyrrolopyrimidinone derivatives of the present invention synthesized by the synthesis (A), (B), (C), (D), (E), (F), and (G) have a hydroxy group, they can be converted into pyrrolopyrimidinone derivatives of the present invention having an acyloxy group by a condensation reaction known to one skilled in the art.

When the pyrrolopyrimidinone derivatives of the present invention synthesized by the synthesis (A), (B), (C), (D), (E), (F), and (G) have a formyl group, they can be converted into pyrrolopyrimidinone derivatives of the present invention having an alkylaminomethyl group by a reductive alkylation reaction known to one skilled in the art.

In the synthesis of the pyrrolopyrimidinone derivative of the formula (I), the pyrrole derivatives of formula (IV-F) used as starting materials can be prepared from a 3-alkoxypropene nitrile derivative of formula (VI-H) by the following synthesis (H).

### [Synthesis (H)]

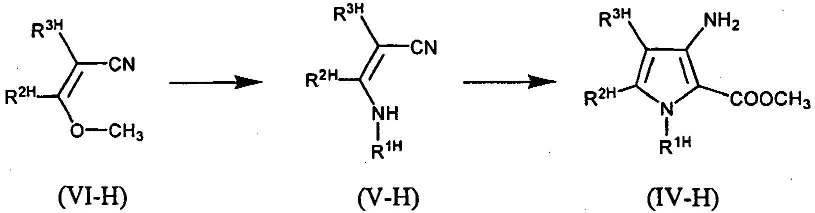

[wherein R^{1H} represents a group capable of withstanding a conversion reaction among groups defined to be represented by A¹-A²-G¹-A³-A⁴-G² in the formula (I). R^{2H} represents, among the groups defined for R²-A⁵ in the formula (I), groups excluding a fluorine atom, a chlorine atom, a bromine atom, an iodine atom, and a substituted or unsubstituted heterocyclic group that is bonded with a carbon atom and a nitrogen atom of a pyrrole ring to which R^{2H} is bonded, and having 1 to 4 atoms selected from the group consisting of an oxygen atom, a nitrogen atom and a sulfur atom, in the ring. R^{3H} is a cyano group or a group capable of withstanding a conversion reaction among groups defined to be represented by A⁶-R³ in formula (I).]

In other words, aminopropenitrile derivatives (V-H) can by synthesized by reacting alkoxypropene nitriles (VI-H) with a primary amine (represented by R¹-NH₂ in which R¹ is as defined above for R¹ prepared by the synthesis (H)). The pyrrole derivatives (IV-H) can be synthesized through a reaction between the aminopropenenitrile derivatives (V-H) and methyl bromoacetate in the presence of a base, or through a cyclization reaction.

The reaction between the alkoxypropene nitrile derivatives.(V-H) and the primary amine is performed using a solvent such as methanol, ethanol or 2-propanol at a temperature in a range of 0°C to 100°C.

The reaction between the alkoxypropenenitrile derivatives (VI-H) and methyl bromoacetate is performed in the presence of a base such as sodium carbonate using a solvent such as acetonitrile at a temperature in a range of 0°C to 150°C.

In the synthesis of the pyrrolopyrimidinone derivative of the formula (I), among the pyrrole derivatives of the formula (IV-F) used as starting materials, a pyrrole derivative having a hydrogen atom as R^{2F} can be prepared from 3-oxopropanenitrile derivatives of formula (VII-J) by the following synthesis (J)

### [Synthesis (J)]

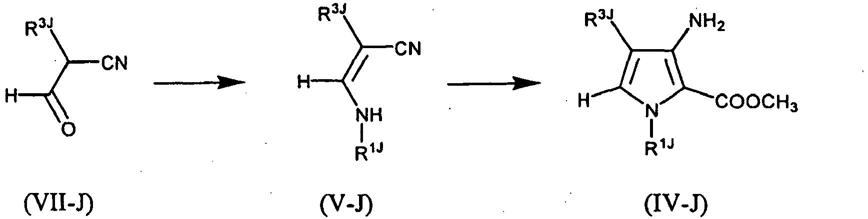

[wherein R^{1J} represents a group which can be converted to A¹-A²-G¹-A³-A⁴-G² in the formula (I), and a group capable of withstanding a conversion reaction. R^{3J} is a cyano group or a group capable of withstanding a conversion reaction among groups defined to be represented by A⁶-R³ in the formula (I).]

In other words, the aminopropenenitrile derivative (V-J) can be synthesized by reacting the 3-oxopropanenitrile derivative (VII-J) with a primary amine (R¹-NH₂ in which R¹ is as defined above for R¹ prepared by the synthesis (J)). The pyrrole derivatives (IV-J) can be synthesized through a reaction between the aminopropenitrile derivatives (V-J) and methyl bromoacetate in the presence of a base, or through a cyclization reaction.

The reaction between 3-oxopropanenitrile derivative (VII-J) and the primary amine is performed using a solvent such as methanol, ethanol or 2-propanol at a temperature in a range of 0°C to 100°C.

The reaction between the aminopropenitrile derivative (VI-J) and methyl bromoacetate is performed in the presence of a base such as sodium carbonate using a solvent such as acetonitrile in a temperature range of 0°C to 150°C.

Alternatively, in the syntheses of the pyrrolopyrimidinone derivative of the formula (I), among the pyrrole derivatives of formula (IV-F) used as starting materials, a pyrrole derivative of formula (IV-F) having a hydrogen atom as R^{2F} can also be prepared by the following synthesis (K):

### [Synthesis (K)]

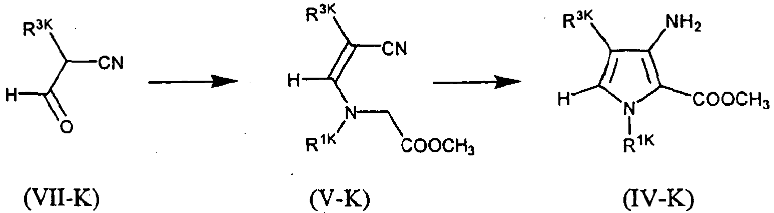

[wherein R^{1K} represents a group which can be converted to A¹-A²-G¹-A³-A⁴-G² in the formula (I), and a group capable of withstanding a conversion reaction. R^{3k} is a cyano or a group capable of withstanding a conversion reaction among groups defined to be represented by A⁶-R³ in formula (I).]

In other words, the aminopropenitrile derivative (V-K) can by synthesized by reacting the 3-oxopropanenitrile derivative (VII-K) and a glycinemethylester derivative (R¹-NH-CH₂-COOCH₃ having R¹ on a nitrogen atom in which R¹ is as defined above for R¹ prepared by the synthesis (K)). The pyrrole derivative (IV-L) can be synthesized by performing cyclization of the aminopropenitrile derivative (V-K) in the presence of a base.

The reaction between the 3-oxopropanenitrile derivative (VII-K) and the glycinemethylester derivative is performed using a solvent such as acetic acid at a temperature in a range of 0°C to 150°C.

The cyclization reaction of the aminopropenitrile derivative (V-K) is performed using a solvent such as acetonitrile or ethylene glycol dimethyl ether in the presence of a base such as 1,8-diazabicyclo[5,4,0]-7-undecene or cesium carbonate at a temperature in a range of 0°C to 150°C.

The thus obtained pyrrolopyrimidinone derivatives of formula (I) have an inhibitory effect of GSK-3 activity, and can be advantageously used as preventive and/or therapeutic agents which are clinically applicable GSK-3 inhibitors. Diseases that can be treated by the GSK-3 activity inhibitor include diabetes, diabetic complications, atherosclerosis, hypertension, obesity, syndrome X, Alzheimer's disease, neurodegenerative diseases (AIDS encephalophy, Huntington's disease, Parkinson's disease, or ischemic attack), manic depressive psychosis, traumatic cerebrospinal injury, alopecia, inflammatory response syndrome, cancer and immunodeficiency.

Also, the pyrrolopyrimidinone derivatives of formula (I) and its pharmaceutically acceptable salts may be formed as pharmaceutical compositions together with pharmacologically acceptable carriers and/or diluents. The compositions of the present invention may be formed as various kinds of formulations to be administered orally or parenterally. The term "parenteral" as used herein includes intravenous, subcutaneous, intramuscular, percutaneous, and rectal injection or infusion techniques.

For oral administration, examples of the formulation include tablets, pills, granules, powder, solutions, suspensions, syrups, and so on.

Here, the tablet formulations can be formed by conventional methods using a pharmaceutically acceptable carrier such as a vehicle, a binding agent, a disintegrating agent, and the like. The pills, granules and powder can also be formed by conventional methods using a vehicle or the like, like the tablets. The formulations in the form of solutions, suspensions and syrups can be prepared by general methods using glycerine esters, alcohols, water, vegetable oils, and so on. The capsule formulations can be formed by filling capsules of gelatin with granules, powder or solutions.

Among formulations for parenteral administration, intravenous, subcutaneous, and intramuscular administration can take forms of injectable formulations. For injection, the compounds of the invention may be formulated in aqueous solutions such as physiological saline or in nonaqueous solutions including organic esters such as propylene glycol, polyethylene glycol, or vegetable oils.

For transdermal administration, formulations can be used in the form of ointment or cream. Ointments can be used in combination with oils or vaselin, for example. Creams can be prepared in combination with emulsifying agents, for example.

When required, these formulations can be further provided with pharmaceutically acceptable carriers such as an isotonic, a preservative, an antiseptic, a wetting agent, a buffering agent, an emulsifying agent, a dispersing agent, or a stabilizer.

Also, such a variety of formulations can be sterilized through appropriate treatments, for example, filtration using a bacteria retaining filter or combination of disinfectants.

The amount of the pyrrolopyrimidinone derivative of formula (I) and its pharmaceutically acceptable salt that may be administered may vary depending upon the kind of a disease, administration route, symptom, age, sex, body weight, and so on of the patient. Generally, a dosage for oral administration is between 0.1 and 500 mg/day/patient. A dosage for parenteral application, including intravenous, subcutaneous, intramuscular, and percutaneous injection is between 0.1 and 100 mg/day/patient.

### EXAMPLES

The present invention will now be described in more detail through the following examples. However, the present invention is not limited to these examples. In the following examples, compound numbers labeled for the respective compounds correspond to the compound numbers labeled for the compounds listed in the above tables as specific examples.

Note that, with regard to data for compounds synthesized in the following examples, the term "HPLC retention time" refers to a retention time (unit: min) associated with a particular compound in HPLC analysis performed under the following analysis condition. HPLC (High performance liquid chromatography) Analysis Condition
System: Hewlett-Packard 1100 HPLC
Column: Cadenza CD-C18 (manufactured by Imtakt Co.) 100 mm × 4.6 mmφ

Solvent A:
H₂O/acetonitrile=95/5 (0.05% trifluoroacetic acid) Solvent B:
H₂O/acetonitrile=5/95 (0.05% trifluoroacetic acid) Flow rate: 1.0 mL/min

### Gradient:

0 to 1 min Solvent B: 10%, Solvent A: 90%
1 to 14 min Solvent B: 10% → 100%, Solvent A: 90% → 0%
14 to 16 min Solvent B: 100%, Solvent A: 0% Calculation of the purity: Area percentage at UV absorption (254 nm)

### Reference Example 1. Synthesis of (cyclopropylhydroxy-methylene)methane-1,1-dicarbonitrile

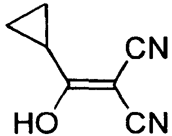

A tetrahydrofuran (150 mL) suspension of sodium hydride (11.49 g) was cooled to 0°C. To the cooled suspension was added dropwise a tetrahydrofuran (50 mL) solution of malononitrile (15.8 g) over an hour. The reaction mixture was stirred at room temperature for 1 hour and cooled to 0°C. To the reaction mixture was added dropwise over 80 minutes a tetrahydrofuran (50 mL) solution of cyclopropylcarbonyl chloride (25.0 g). The reaction mixture was stirred at room temperature for 49 hours, followed by adding water. (50 mL) to the reaction solution. The solvent was distilled off under reduced pressure. To the residue were added ethyl acetate (200 mL) and hydrochloric acid (270 mL, 1 mol/L), which was extracted with ethyl acetate. The organic layer was washed with saturated brine and dried over anhydrous sodium sulfate, and then the solvent was distilled off under reduced pressure to obtain a crude product (40.9 g) of the title compound. The NMR data of the compound is given below.
¹H-NMR(400 MHz, CDCl₃) δ (ppm): 1.10-1.22(m, 4H), 2.10-2.22(m, 1H), 4.27(s, 3H).

In a similar manner as described above, [(3-chloro(2-thienyl))hydroxymethylene]methane-1,1-dicarbonitrile was prepared from malononitrile and 3-chlorothiophene-2-carbonylchloride. The NMR data and ESI/MS data of the compound are given below.
¹H-NMR(400 MHz, CD₃OD) δ (ppm): 6.92(d,J=5.1,1H), 7.51(d,J=5.4,1H)
ESI/MS m/e: (M⁺+H, C₈H₃ClN₂OS)

### Reference Example 2. Synthesis of (cyclopropylmethoxy-methylene)methane-1,1-dicarbonitrile

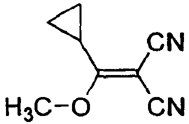

A tetrahydrofuran (100 mL) suspension of sodium hydride (2.6 g) was cooled to 0°C. To the cooled suspension was added dropwise a tetrahydrofuran (60 mL) solution of crude (1-hydroxy-2-phenylmethylidene)methane-1,1-dicarbonitrile (14.5 g) over 30 minutes. The reaction mixture was stirred at room temperature for 20 minutes and cooled to 0°C. To the reaction mixture was added dropwise a tetrahydrofuran solution (40 mL) of dimethyl sulfate (13.7 g) over 1 hour. After heating for 21 hours to reflux, the reaction mixture was cooled to room temperature, and the solvent was distilled off under reduced pressure. To the residue were added ethyl acetate (100 mL) and saturated sodium hydrogen carbonate solution (100 mL), and extraction with ethyl acetate was performed. The organic layer was washed with saturated brine and dried over anhydrous sodium sulfate, and then the solvent was distilled off under reduced pressure. The obtained crude product was purified by column chromatography on silica gel using hexane/ethyl acetate=1/3 as an eluent to obtain the title compound (6.8 g, 54%) as a light yellow solid. NMR data of the, compound is given below.
¹H-NMR (400MHz, CDCl₃) δ (ppm) : 1.10-1.22(m,4H), 2.10-2.22(m,1H), 4.27(s,3H).

### Reference Example 3. Synthesis of methyl 3-amino-1-(2-[(t-butoxy)carbonylamino]ethyl)-4-cyano-5-cyclopropyl-pyrrole-2-carboxylate

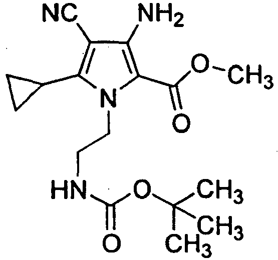

To an acetonitrile (150 mL) solution of (methoxycyclopropylmethylene)methane-1,1-dicarbonitrile (8.7 g) was added N-(2-aminoethyl) t-butyl carbaminic acid (16.3 g) and stirred at room temperature for 10 minutes. To the resultant product were added anhydrous cesium carbonate (38.5 g) and methyl bromoaccetate (11.2 mL), followed by heating for 6 hours to reflux. The reaction product was cooled to room temperature and allowed to stand. Then, the supernatant was separated by decantation and the solvent was distilled off under reduced pressure. The concentrated residue and a solid remaining after decantation were collected and ethyl acetate and water were added thereto, followed by extracting 3 times with ethyl acetate. The organic phase was washed with water and saturated brine, and dried over anhydrous magnesium sulfate.- After magnesium sulfate was removed by filtration, the solvent was distilled off under reduced pressure. The residue was purified by column chromatography on silica gel (hexane/ethyl acetate=2/1) to obtain the title compound (17.5 g, yield 85%). The ESI/MS data of the compound are given below.
ESI/MS m/e: 349.1 (M⁺+H, C₁₇H₂₄N₄O₄)

Methyl 3-amino-1-{2-[(t-butoxy)carbonylamino}ethyl) -5-(3-chloro(2-thienyl))-4-cyanopyrrole-2-carboxylate was synthesized from [(3-chloro(2-thienyl))hydroxymethylene] metharie-1,1-dicarbonitrile used as a starting material in a similar manner to that in Reference Examples 2 and 3. The ESI/MS data of the compound are given below.
ESI/MS m/e: 425.2 (M⁺+H, C₁₈H₂₁ClN₄O₄S)

### Reference Example 4. Synthesis of (t-butoxy)-N-[2-(7-cyano-4-oxo-6-cyclopropyl(3-hydropyrrolo[3,2-d]pyrimidin-5-yl)) ethyl]carboxyamide

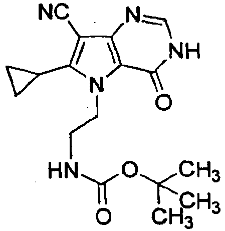

Methyl 3-amino-1-{2-[(t-butoxy)carbonylamino]ethyl}-4-cyano-5-cyclopropylpyrrole-2-carboxylate (17.4 g) and formamidine acetate (104.1 g) were added to 2-propanol (360 mL) and heated for 45 hours to reflux. The reaction mixture was cooled to room temperature, and the solvent was distilled off under reduced pressure. To the residue was added water, and the obtained solid was isolated by filtration and sufficiently washed with water. The resulting solid was recrystallized (ethanol/ethyl acetate/hexane=1/2/1) to obtain the title compound (9.8 g, yield 57%). The ESI/MS data of the compound are given below.
ESI/MS m/e: 362.1(M⁺+H, C₁₇H₂₁N₅O₃)

(t-butoxy)-N-{2-[6-(3-chloro(2-thienyl))-7-cyano-4-oxo(3-hydropyrrolo[3,2-d]pyrimidin-5-yl)]ethyl}carboxyamide was prepared from methyl-3-amino-1-{2-[(t-butoxy)carbonyl-amino]ethyl}-5-(3-chloro(2-thienyl))-4-cyanopyrrole-2-carboxylate in a similar manner to that described above. ESI/MS data of the compound are given below.
ESI/MS m/e: 420.2 (M⁺+H, C₁₈H₁₈ClN₅O₃S)

### Reference Example 5. Synthesis of 3-oxo-2-(3-pyridyl) propanenitrile hydrochloride

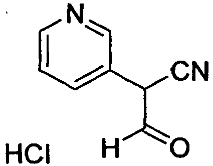

To a toluene solution (100 mL) of 3-pyridylacetonitrile (40.86 g) was added dimethyl formamide dimethyl acetal (123.6 g) and the mixture was heated for 4 hours to reflux. The reaction solution was cooled to room temperature, and the solvent was distilled off under reduced pressure. The residue was purified by column chromatography on silica gel (ethyl acetate) to obtain a brown solid. The obtained brown solid was washed with ethyl acetate, yielding 46.93 g of a colorless solid. To a tetrahydrofuran (300 mL) suspension of the reaction product (20.37 g) were added water (40 mL) and concentrated hydrochloric acid (24.50 mL) and the mixture was stirred at 50°C for 4 hours. The reaction solution was cooled to room temperature, and the solvent was distilled off under reduced pressure. The residue was dried in vacuo to obtain a crude product (30.12 g) of the title compound, which was used for the subsequent reaction without further purification. ESI/MS data of the compound are given below.
ESI/MS m/e: 147.1(M⁺+H, C₈H₆N₂O HCl)

### Reference Example 6. Synthesis of ethyl 3-{((1Z)-2-cyano-2-(3-pyridyl)vinyl)[(methoxycarbonyl)methyl]amino}propanate

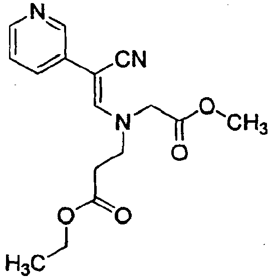

To an acetic acid (30 mL) solution of ethyl 3-{[(methoxycarbonyl)methyl]amino}propanate(6.720 g) was added a crude product of 3-oxo-2-(3-pyridyl)propanenitrile (7.826 g), and the mixture was stirred at 80°C for 2 days. The reaction solution was cooled to room temperature, and the solvent was distilled off under reduced pressure. The residue was diluted with water, and sodium carbonate was added thereto for neutralization, followed by extracting the solution with ethyl acetate. The organic layer was washed with saturated brine, and dried over sodium sulfate, which was then filtered for separation. Thereafter, the solvent was distilled off under reduced pressure. The residue was purified by column chromatography on silica gel (hexane/ethyl acetate=1/3), to obtain the title compound (7.521 g, yield 83%). The ESI/MS data of the compound are given below.
ESI/MS m/e: 318.2 (M⁺+H, C₁₆H₁₉N₃O₄)

### Reference Example 7. Synthesis of ethyl 3-[3-amino-2-(methoxycarbonyl)-4-(3-pyridyl)pyrrolyl]propanate

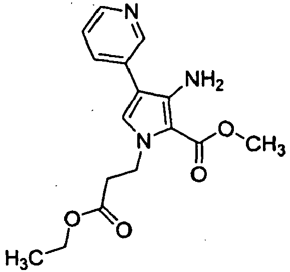

To an ethylene glycol dimethyl ether (50 mL) solution of,ethyl 3-{((1Z)-2-cyano-2-(3-pyridyl)vinyl)[(methoxycarbonyl)methyl]amino}propanate (6.236 g) was added 1,8-diazabicyclo [5,4,0]-7-undecene (3.590 g), and the mixture was stirred at 60°C overnight. The reaction solution was cooled to room temperature and neutralized with acetic acid, and the solvent was distilled off under reduced pressure. The residue was purified by column chromatography on silica gel (hexane/ethyl acetate=1/5), to obtain the title compound (4.380 g, yield 70%). The NMR data and ESI/MS data of the compound are given below.
¹H-NMR(400 MHz, DMSO-d₆) δ (ppm): 1.10-1.18(m,3H), 2.74(t,J=6.82,2H), 3.77(s,3H), 3.99-4.07(m,2H), 4.38(t,J=6.84,2H), 7.46(s,1H), 7.85(dd,J=5.74,J=7.94,1H), 8.36(d,J=8.08,1H), 8.60(d,J=5.40,1H), 8.84(s,1H).
ESI/MS m/e: 318.2 (M⁺+H, C₁₆H₁₉N₃O₄)

### Reference Example 8. Synthesis of (cyclopropylhydroxy-methylene)methane-1-(3-pyridyl)-1-carbonitrile

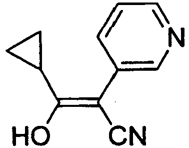

To a 500 mL branched flask was added 13.0 g of 3-pyridinacetonitrile (110 mmol) and 150 mL of tetrahydrofuran, and 44 mL of ⁿBuLi (2.6 M solution) (114 mmol) was added dropwise thereto using a syringe with stirring at 0°C, followed by stirring at 0°C for 30 minutes and at 40°C for 30 minutes. While the reaction product was cooled at 0°C with stirring, a tetrahydrofuran solution (30 mL) of 10.45 g of cyclopropanecarbonyl chloride (100 mmol) was added dropwise, and stirred at room temperature for 1 hour, followed by adding to the reaction solution 300 mL of a saturated ammonium chloride solution and separated. The aqueous layer was extracted with 100 mL of ethyl acetate. The organic layer was washed twice with 300 mL of saturated brine. The organic layer was dehydrated and dried over magnesium sulfate, filtered and concentrated to obtain 18.25 g of concentrated residue. To the concentrated residue was added 30 mL of acetonitrile and an insoluble portion was filtered off, followed by washing with 20 mL of acetonitrile to obtain a crude product of the title compound (5.17 g, 28%). The NMR data and ESI/MS data of the compound are given below.
¹H-NMR(400 MHz, DMSO-d₆) δ (ppm): 0.64(m,4H), 2.24(m,1H), 2.50(brds, 1H), 7.70(dd, J=5.4Hz, J=8.6Hz,1H), 8.11(d,J=5.4Hz,1H), 8.21 (d, J=8 . 6Hz, 1H) , 9.12(brds,1H). ESI/MS m/e: 187.1(M⁺+H, C₁₁H₁₀N₂O).

### Reference Example 9. Synthesis of (cyclopropyl-p-toluene-sulfonyloxymethylene)methane-1-(3-pyridyl)-1-carbonitrile

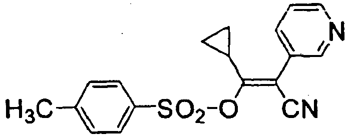

A 200 mL branched flask were charged with 4.51 g of (cyclopropylhydroxymethylene)methane-1-(3-pyridyl)-1-carbonitrile (24.3 mmol) and 9.14 g of p-toluene sulfonic anhydride (28 mmol), and 100 mL of dichloromethane was added thereto, followed by adding 4.8 mL of triethylamine (34.5 mmol) dropwise with stirring the reaction mixture at room temperature, and stirring at room temperature for 2 hours. After the completion of the reaction, 50 mL of water was added to the reaction solution and separated. The aqueous layer was extracted with 20mL of dichloromethane. The organic layer was washed once with 50 mL of saturated sodium hydrogen carbonate solution and twice with 50 mL of water. The organic layer was dehydrated and dried over magnesium sulfate, filtered and concentrated to obtain a crude product (8.47 g, 100%) of the title compound. The NMR data and ESI/MS data of the compound are given below.
¹H-NMR(270 MHz, CDCl₃) δ (ppm) : 1.10-1.32 (m, 4H), 2.40 (s, 3H), 2.15-2.42 (m, 1H), 7.05(m,3H), 7.40(m,2H), 7.60(m, 1H), 8.40(m,2H).
ESI/MS m/e: 341.0(M⁺+H, C₁₈H₁₆N₂O₃S),

### Reference Example 10. Synthesis of ethyl 3-amino-4-(3-pyridyl)-5-cyclopropylpyrrole-2-carboxylate

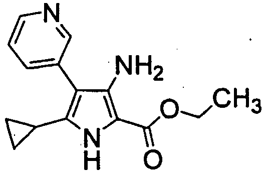

A 200 mL branched flask was charged with 7.764 g of (cyclopropyl-p-toluenesulfonyloxymethylene)methane-1-(3-pyridyl)-1-carbonitrile (22.8 mmol), and 70 mL of ethanol and 35 mL of tetrahydrofuran were added thereto, followed by adding 5.08 g of diethylaminomalonate hydrochloric acid (24 mmol) with stirring the reaction mixture at 0°C, and stirring at 0°C for 1 hour. Subsequently, 35 mL of an ethanol solution of sodium ethoxide (5.43g, 80 mmol) was added dropwise to the reaction solution and stirred at 0°C for 1 hour. After the completion of the reaction, the solvent was concentrated under reduced pressure and concentrated. To the residue were added 150 mL of water and ethyl 100 mL of acetate for extraction. The aqueous layer was extracted with 50 mL of ethyl acetate. The organic layer was washed once with 100mL of water and three times with 50 mL of saturated brine solution. The organic layer was dehydrated and dried over magnesium sulfate, filtered and concentrated to obtain 3.90 g of a crude product, which was then purified by column chromatography (silica gel 100 g; using 4:1 to 0:1 hexane/ethyl acetate as an eluent) to obtain the title compound (1.074 g, yield 17%). The NMR data and ESI/MS data of the compound are given below.
¹H-NMR(270 MHz, CDCl₃) δ (ppm): 0.60-0.95(m,4H), 1.30 (m, 1H) , 1.36 (t, J=7.0Hz, 3H), 4.15 (q,J=7.0Hz, 2H), 4.35(brds,2H), 7.15 (m,1H) 7,75 (m, 1H), 8.50 (m, 1H), 8.75(s, 1H).
ESI/MS m/e: 272.1 (M⁺+H, C₁₅H₁₇N₃O₂).

### Reference Example 11. Synthesis of N-{2-[4-chloro-6-(3-chloro(2-thienyl)-7-iodopyrrolo[3,2-d]pyrimidin-5-yl]ethyl}-2,2,2-trifluoroacetamide

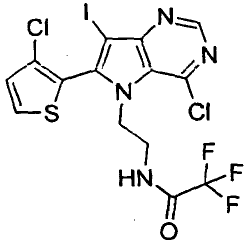

A 3.0 mL phosphorus oxychloride solution of N-{2-[6-(3-chloro(2-thienyl))-7-iodo-4-oxo(3-hydropyrrolo [3,2-d]pyrimidin-5-yl)]ethyl}-2,2,2-trifluoroacetamide (333 mg) was stirred at 110°C for 2 hours. The reaction mixture was cooled to room temperature, and excess phosphorus oxychloride was distilled off under reduced pressure. The residue was dried in vacuo to obtain a crude product of the title compound, which was used for the subsequent reaction without further purification. The ESI/MS data of the compound are given below.
ESI/MS m/e: 535.2 (M⁺+H, C₁₄H₈Cl₂F₃IN₉OS)

### Reference Example 12. Synthesis of N-{2-[7-bromo-4-chloro-6-(3-chloro(2-thienyl))pyrrolo[3,2-d]pyrimidin-5-yl]ethyl}-2,2,2-trifluoroacetamide

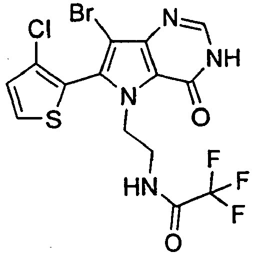

A crude product of the title compound was prepared from N-{2-[7-bromo-6-(3-chloro(2-thienyl))-4-oxo(3-hydropyrrolo
[3,2-d]pyrimidin-5-yl)]ethyl}-2,2,2-trifluoroacetamide in a similar manner to that described in Reference Example 11. The ESI/MS data of the compound are given below.
ESI/MS m/e: 489.0(M⁺+H, C₁₄H₈BrCl₂F₃N₄OS)

### Reference Example 13. Synthesis of N-{2-[4,7-dichloro-6-(3-chloro(2-thienyl))pyrrolo[3,2-d]pyrimidin-5-yl]ethyl}-2,2,2-trifluoroacetamide

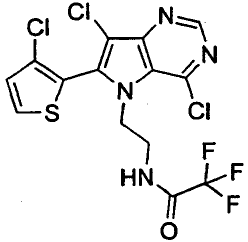

A crude product of the title compound was prepared from N-{2-[7-chloro-6-(3-chloro(2-thienyl))-4-oxo(3-hydropyrrolo[3,2-d]pyrimidin-5-yl)]ethyl}-2,2,2-trifluoroacetamide in a similar manner to that described in Reference Example 11. The ESI/MS data of the compound are given below.
ESI/MS m/e: 443.4 (M⁺+H, C₁₄H₈Cl₃F₃N₄OS)

### Reference Example 14. Synthesis of N-(2-{7-[(1E)-1-aza-2-(dimethylamino)vinyl]-4-chloro-6-(3-chloro(2-thienyl)) pyrrolo[3,2-d]pyrimidin-5-yl)ethyl) (4-fluorophenyl) carboxyamide

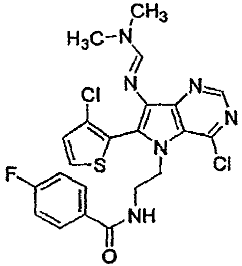

N-(6-(3-chloro(2-thienyl))-5-{2-[(4-fluorophenyl) carbonylamino]ethyl}-4-oxo(3-hydropyrrolo[3,2-d]pyrimidin-7-yl))-2,2,2-trifluoroacetamide (1.09 g) was used to obtain a crude product (0.87 g) of the title compound in the same way as Reference Example 11. NMR data and ESI/MS data of the compound are given below. 1H-NMR(400 MHz, Dmso-d₆) δ (ppm): 2.81(brs,1H), 2.91(brs,1H), 3.28(s,2H), 3.37-3.45(m,2H), 7.17-7.27(m,3H), 7.55-7.68(m,2H), 7.86(d,J-5.4,1H), 8.31-8.38(m,1H), 8.61(s,1H), 8.75(s,1H).
ESI/MS m/e: 505.4 (M⁺+H, C_{22.}H₁₉Cl₂FN₆OS)

### Reference Example 15. Synthesis of 5-{2-[(t-butoxy)carbonylamino] ethyl}-6-{3-chloro(2-thienyl)}-4-oxo-3-hydropyrrolo[3,2-d] pyrimidin-7-carboxyamide (Compound No: 950)

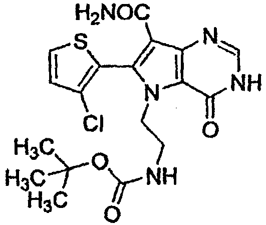

(t-butoxy)-N-{2-[6-{3-chloro(2-thienyl)}-7-cyano-4-oxo(3-hydropyrrolo[3,2-d]pyrimidin-5-yl)]ethyl}carboxyamide (3.0 g) was dissolved in ethanol (100 mL), and a 5M aqueous sodium hydroxide solution (20 mL) was added thereto. A 30% hydrogen peroxide solution (30 mL) was added to the reaction mixture over 20 minutes with stirring. After stirring at 45 to 50°C for 24 hours, 30% hydrogen peroxide solution (20 mL) was added to the reaction solution, stirred at 45 to 50°C for 24 hours, concentrated and neutralized with 1 M hydrochloric acid, to obtain a white precipitate. The precipitate was filtered, washed, and dried under reduced pressure to obtain the title compound (2.68 g, yield 86%). The HPLC retention time, NMR data and ESI/MS data of the compound are given below.
HPLC retention time=7.2(min)
¹H-NMR(270 MHz, DMSO-d₆) δ (ppm): 1.26(s,9H), 3.2-3.5(m,2H), 3.8-4.0(m,1H), 4.4-4.6(m,2H),6.5-6.6(m,1H), 7.17(d,1H,J=4.6Hz), 7.2-7.3(m,1H),
7.91(d,1H,J=5.4Hz),8.0-8.1(m,1H), 12.4-12.5(m,1H). ESI/MS m/e: 438.3 (M⁺+H, C₁₈H₂₀ClN₅O₄S

### Reference Example 16. Synthesis of 5-{2-[(t-butoxy)carbonylamino] ethyl}-6-cyclopropyl-4-oxo-3-hydropyrrolo[3,2-d]pyrimidin-7-carboxyamide (Compound No: 898)

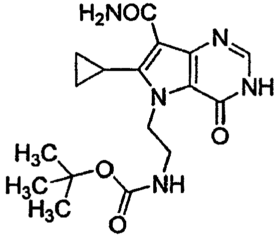

The title compound was prepared from (t-butoxy)-N-{2-[6-cyclopropyl-7-cyano-4-oxo(3-hydropyrrolo[3,2-d]pyrimidin-5-yl)]ethyl}carboxyamide in a similar manner to that described in Example 1. The ESI/MS data of the compound are given below.
ESI/MS:m/e 362.1 (M⁺+H, C₁₇H₂₃N₅O₄)

### Reference Example 17. Synthesis of N-{2-[7-amino-6-(3-chloro (2-thienyl))-4-oxo(3-hydropyrrolo[3,2-d]pyrimidin-5-yl)] ethyl}(t-butoxy)carboxyamide (Compound No: 959)

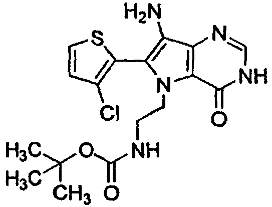

5-{2-[(t-butoxy)carbonylamino]ethyl}-6-(3-chloro(2-thienyl))4-oxo-3-hydropyrrolo[3,2-d]pyrimidine-7-carboxyamide (110 mg) was suspended in a 1 M aqueous sodium hydroxide solution (7.5 mL), and benzyltrimethylammonium tribromide (135 mg) was added thereto and the mixture was stirred for 1.5 hours. 1 M hydrochloric acid was added to the reaction mixture to acidify the reaction system, and then washed with ethyl acetate. The aqueous layer was made alkaline with sodium hydrogen carbonate and extracted with ethyl acetate. The organic layer was washed with saturated brine, dried over anhydrous sodium sulfate, filtered, concentrated, and purified by column chromatography on silica gel (15 g) using 1:1 to 0:1 hexane:ethyl acetate as an eluent to obtain the title compound (72 mg, yield 70%). The NMR data and ESI/MS data of the compound are given below. HPLC retention time=6.4(min)
¹H-NMR(270MHz, CDCl₃) δ (ppm): 1.32(s,9H), 3.3-3.5(m,2H), 4.3-4.5(m,2H), 4.9-5.0(m, 1H), 7.11 (d,1H,J=5.4Hz), 7.54 (d, 1H, J=5. 4Hz), 7.79(brs,1H), 10.0-10.1(m,1H) ESI/MS m/e: 410.3 (M⁺+H, C₁₇H₂₀ClN₅O₃S)

### Reference Example 18. Synthesis of N-{2-[7-amino-6-cyclopropyl-4-oxo(3-hydropyrrolo[3,2-d]pyrimidin-5-yl)]ethyl}(t-butoxy)carboxyamide (Compound No: 903)

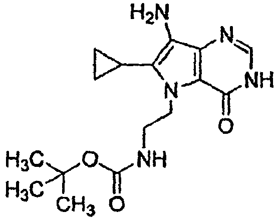

The title compound was prepared from 5-{2-[(t-butoxy)carbonylamino]ethyl}-6-cyclopropyl-4-oxo-3-hydropyrroloi3,2-d]pyrimidin-7-carboxyamide in a similar manner to that described in Example 3. The NMR data and ESI/MS data of the compound are given below. ¹H-NMR(270MHz,CDCl₃)δ(ppm): 1.34(s,9H),0.8-1.2(m,5H), 3.4(brs,2H), 3.5-3.6(m,3H), 4.5-4.6(m,2H), 5.6(brs,1H), 7.8(brs,1H)
ESI/MS m/e: 334.1(M⁺+H, C₁₆H₂₃N₅O₃)

### Reference Example 19. Synthesis of (t-butoxy)-N-{2-[7-bromo-6-(3-chloro(2-thienyl))-4-oxo(3-hydropyrrolo[3,2-d] pyrimidin-5-yl)]ethyl}carboxyamide (Compound No: 945)

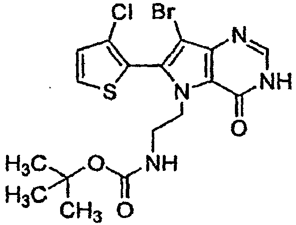

To 30 mg of N-{2-[7-amino-6-(3-chloro(2-thienyl))-4-oxo(3-hydropyrrolo[3,2-d]pyrimidin-5-yl)]ethyl}(t-butoxy)carboxyamide, 1 ml of bromoform was added, followed by adding 50 µl of isoamyl nitrite. After stirring at 70°C for 4 hours, saturated brine was added to the reaction solution, which was extracted with ethyl acetate. The organic layer was dried over anhydrous sodium sulfate, filtered, and concentrated, followed by subjecting to thin layer chromatography on silica gel using ethyl acetate as a developing solvent to obtain the title compound (15 mg, yield 43%). The HPLC retention time, NMR data and ESI/MS data of the compound are given below.
HPLC retention time=9.5(min)
¹H-NMR(270MHz,CDCl₃) δ (ppm): 1.31(s,9H), 3.3-3.5(m,2H), 4.2-4.3(m,1H), 4.5-4.7(m,1H 4.7-4.9(m,1H), 7.13(d,1H,J=5.7Hz), 7.60(d,1H, J=5.4Hz), 7.94(d,1H,J=3.0Hz).
ESI/MS m/e 475.2(M⁺+H, C₁₇H₁₈BrClN₄O₃S)

### Reference Example 20. Synthesis of (t-butoxy)-N-{2-[7-bromo-6-cyclopropyl-4-oxo(3-hydropyrrolo[3,2-d]pyrimidin-5-yl)] ethyl}carboxyamide (Compound No: 2511)

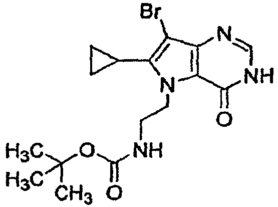

The title compound was prepared from N-{2-[7-amino-6-cyclopropyl-4-oxo(3-hydropyrrolo[3,2-d]pyrimidin-5-yl)]ethyl}(t-butoxy)carboxyamide in a similar manner to that described in Example 5. The HPLC retention time, NMR data and ESI/MS data of the compound are given below. HPLC retention time=8.5(min)
1H-NMR(270MHz, CDCl₃)δ(ppm): 1.31(s,9H), 1.1-1.3(m,5H), 3.5-3.9(m,3H), 4.5-4.9(m,2H), 5.1-5.3(brs,1H), 7.9(s,1H) ESI/MS m/e: 397.1(M⁺+H, C₁₆H₂₁BrN₄O₂S)

### Reference Example 21. Synthesis of (t-butoxy)-N-{2-[6-(3-chloro(2-thienyl))-7-iodo-4-oxo(3-hydropyrrolo[3,2-d]pyrimidin-5-yl)]ethyl}carboxyamide (Compound No: 946)

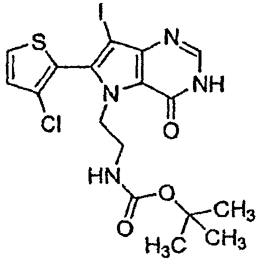

To 1 g of N-{2-[7-amino-6-(3-chloro(2-thienyl))-4-oxo(3-hydropyrrolo[3,2-d]pyrimidin-5-yl)]ethyl}(t-butoxy)carboxyamide, 7 mL of diiodomethane was added, followed by adding 822 µl of isoamyl nitrite. After stirring at 70°C for 4 hours, saturated brine was added to the reaction solution, which was extracted with ethyl acetate. The organic layer was dried over anhydrous sodium sulfate, filtered, and concentrated, followed by subjecting to chromatography on silica gel (using 5:1 to 0:1 hexane/ethyl acetate as an eluent), to yield the title compound (694 g, yield: 55%). NMR data and ESI/MS data of the compound are given below.
HPLC retention time=9.6(min)
¹H-NMR(270MHz,CDCl₃)δ(ppm):1.32(s,9H), 3.3-3.5(m,2H), 4.2-4.35(m,lH), 4.6-4.75(m,1H), 4.8-5.0(m,2H), 7.14(d,1H,J=5.4Hz), 7.59(d,1H,J=5.7Hz), 7.95-8.05(m,1H).
ESI/MS m/e 521.3(M⁺+H, C₁₇H₁₈ClIN₄O₃S

### Reference Example 22. Synthesis of (t-butoxy-N-{2-[7-iodo-6-cyclopropyl]-4-oxo(3-hydropyrrolo[3,2-d]pyrimidin-5-yl)} Ethyl)carboxyamide (Compound No: 896)

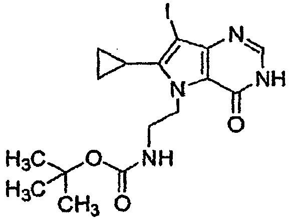

The title compound was prepared from N-{2-[7-amino-6-cyclopropyl-4-oxo(3-hydropyrrolo[3,2-dlpyrimidin-5-yl)]ethyl}(t-butoxy)carboxyamide in a similar manner to that described in Example 7. The HPLC retention time, NMR data and ESI/MS data of the compound are given below. HPLC retention time=8.7(min)
¹H-NMR(270MHz,CDCl₃)δ(ppm); 1.32(s,9H), 1.0-1.3(m,5H), 3.5-3.8(m,3H), 4.7-4.9(m;2H), 5.2-5.3(brs,1H), 7.9(s,1H) ESI/MS m/e: 445.4(M⁺+H, C₁₆H₂₁IN₄O₃)

### Reference Example 23. Synthesis of (t-butoxy)-N-{2-[7-chloro-6-(3-chloro(2-thienyl))-4-oxo(3-hydropyrrolo[3,2-d]pyrimidin-5-yl)]ethyl}carboxyamide (Compound No: 944)

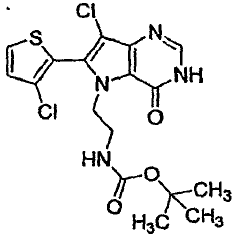

To 20 mg of N-{2-[7-amino-6-(3-chloro(2-thienyl))-4-oxo(3-hydropyrrolo[3,2-d]pyrimidin-5-yl)]ethyl}(t-butoxy)carboxyamide, 2 ml of carbon tetrachloride was added, followed by adding 34 µl of isoamyl nitrite. After refluxing for 40 hours, saturated brine was added to the reaction solution, which was extracted with ethyl acetate. The organic layer was dried over anhydrous sodium sulfate, filtered, and concentrated, followed by subjecting to thin layer chromatography on silica gel (using ethyl acetate as a developing solvent) to obtain the title compound (5 mg, yield: 24%). The HPLC retention time, NMR data and ESI/MS data of the compound are given below.
HPLC retention time=9.4(min)
¹H-NMR(270MHz,CDCl₃)δ(ppm): 1.32(s,9H), 3.3-3.5(m,2H), 4.15-4.4(m,1H) 4.6-4.75(m,1H), 4.8-4.95(m,1H), 7.13(d,1H,J=5.4Hz) 7.60(d,1H,J=5.4Hz), 7.98(brs,1H).
ESI/MS m/e 429. 4 (M⁺+H, C₁₇H₁₈Cl₂N₄OS)

### Reference Example 24. Synthesis of (t-butoxy)-N-{2-[6-(3-chloro{2-thienyl))-4-oxo(3-hydropyrrolo[3,2-d]pyrimidin-5-yl)]ethyl}carboxyamide (Compound No. 943)

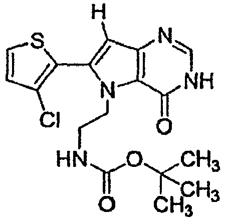

To 15 mg of N-{2-[7-amino-6-(3-chloro(2-thienyl))-4-oxo(3-hydropyrrolo[3,2-d]pyrimidin-5-yl)]ethyl}(t-butoxy)carboxyamide, 2 mL of tetrahydrofuran was added, followed by adding 50 µl of isoamyl nitrite. After stirring at 50°C for 3 hours, saturated brine was added to the reaction solution, which was extracted with ethyl acetate. The organic layer was dried over anhydrous sodium sulfate, filtered, and concentrated, followed by subjecting to thin layer chromatography on silica gel (developing solvent: ethyl acetate) to obtain the title compound (8 mg, yield 55%). The NMR data and ESI/MS data of the compound are given below.
HPLC retention time=8.4(min)
¹H-NMR(270MHz,CDCl₃) δ (ppm): 1.31(s,9H), 3.3-3.5(m,2H), 4.4-4.6 (m, 2H), 5.01 (brs, 1H), 6.66 (s, 1H), 7.07(d,1H,J=5.4Hz), 7.49(d,1H,J=5.1Hz), 7.93(brs,1H), 11.2-11.3(m,1H).
ESI/MS m/e 395.2 (M⁺+H, C₁₇H₁₉ClN₄O₃S)

### Reference Example 25. Synthesis of 5-(2-aminoethyl)-6-(3-chloro (2-thienyl))-7-iodo-3-hydropyrrolo[3,2-d]pyrimidin-4-one hydrochloride (Compound No. 206)

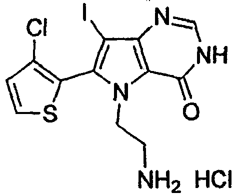

(t-butoxy)-N-(2-[6-(3-chloro(2-thienyl))-7-iodo-4-oxo(3-hydropyrrolo[3,2-d]pyrimidin-5-yl)]ethyl}carboxyamide (331 mg) was dissolved in a mixed solution of methanol(0.5 mL) and 1,4-dioxane(5.0 mL), and hydrochloric acid/1,4-dioxane solution (4 mol/L, 0.64 mL) was added and the mixture was stirred at 60°C for 2 hours. The reaction mixture was cooled to room temperature, and the solvent was distilled off under reduced pressure. The residue was dried under reduced pressure to obtain the title compound (334 mg in a quantitative yield). The ESI/MS data of the compound are given below.
ESI/MS m/e: 421.2(M⁺+H, C₁₂H₁₀ClN₄OS HCl)

### Reference Example 26. Synthesis of 5-(2-aminoethyl)-7-bromo-6-(3-chloro(2-thienyl))-3-hydropyrrolo[3,2-d]pyrimidin-4-one hydrochloride(Compound No. 205)

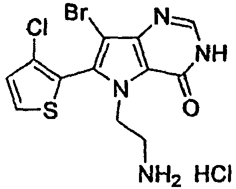

The title compound was prepared from (t-butoxy)-N-{2-[7-bromo-6-(3-chloro(2-thienyl))-4-oxo(3-hydropyrrolo [3,2-d]pyrimidin-5-yl)]ethyl}carboxyamide in a similar manner to that described in Example 11. The ESI/MS data of the compound are given below.
ESI/MS m/e: 375.0(M+⁺H, C₁₂H₁₀BrClN₄OS HCl)

### Reference Example 27. Synthesis of 5-(2-aminoethyl)-7-chloro-6-(3-chloro(2-thienyl))-3-hydropyrrolo[3,2-d]pyrimidin-4-one hydrochloride (Compound No. 204)

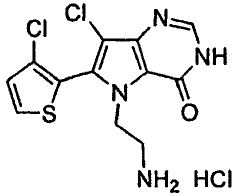

The title compound was prepared from (t-butoxy)-N-{2-[7-chloro-6-(3-chloro(2-thienyl))-4-oxo(3-hydropyrrolo[3,2-d]pyrimidin-5-yl)]ethyl}carboxyamide in a similar manner to that described in Example 11. The ESI/MS data of the compound are given below.
ESI/MS m/e: 329.4(M⁺+H, C₁₂H₁₀Cl₂N₄OS HCl)

### Reference Example 28. Synthesis of N-{2-[6-(3-chloro(2-thienyl))-7-iodo-4-oxo(3-hydropyrrolo[3,2-d]pyrimidin-5-yl)]ethyl)-2,2,2-trifluoroacetamide (Compound No. 320)

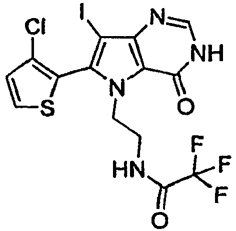

To a tetrahydrofuran (5.0 mL) solution of 5-(2-aminoethyl)-6-(3-chloro(2-thienyl))-7-iodo-3-hydropyrrolo[3,2-d]pyrimidin-4-one hydrochloride(259 mg) was added trifluoroacetic anhydride (595 mg), and triethylamine (1.2 mL) was added slowly dropwise. The reaction mixture was stirred at room temperature for 2 hours, and methanol was added to stop the reaction. The solvent was distilled off under reduced pressure. To the residue were added water and ethyl acetate, which was extracted 3 times with ethyl acetate. The organic layer was washed with saturated brine, and dried over anhydrous magnesium sulfate. After anhydrous magnesium sulfate was filtered off, the solvent was distilled off under reduced pressure. The residue was dried under reduced to obtain a crude product (365 mg) of the title compound. The HPLC retention time, NMR data and ESI/MS data of the compound are given below.
HPLC retention time =9.1(min)
¹H-NMR(400 MHz, DMSO-d₆)δ(ppm): 3.45(m,2H), 4.16(m,1H), 4.61(m,1H), 7.30(m,1H), 7.93(m,1H), 8.02(m, 1H), 9.37(m,1H), 12.29(brs, 1H).
ESI/MS m/e: 517.2(M⁺+H, C₁₄H₉ClF₃IN₄O₂S)

### Reference Example 29. Synthesis of N-{2-[7-bromo-6-(3-chloro(2-thienyl))-4-oxo(3-hydropyrrolo[3,2-d]pyrimidin-5-yl)]ethyl}-2,2,2-trifluoroacetamide (Compound No. 319)

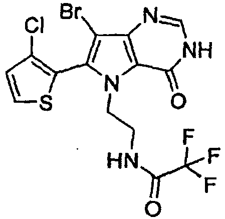

The title compound was prepared from 5-(2-aminoethyl)-7-bromo-6-(3-chloro(2-thienyl))-3-hydropyrrolo[3,2-d]
pyrimidin-4-one hydrochloride in a similar manner to that described in Example 14. The ESI/MS data of the compound are given below.
ESI/MS m/e: 471.1(M⁺+H, C₁₄H₉BrClF₃N₄O₂S)

### Reference Example 30. Synthesis of N-{2-[7-chloro-6-(3-chloro(2-thienyl))-4-oxo(3-hydropyrrolo[3,2-d]pyrimidin-5-yl)]ethyl}-2,2,2-trifluoroacetamide (Compound No. 318)

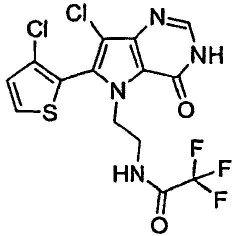

The title compound was prepared from 5-(2-aminoethyl)-7-chloro-6-(3-chloro(2-thienyl))-3-hydropyrrolo[3,2-d]
pyrimidin-4-one hydrochloride in a similar manner to that described in Example 14. The ESI/MS data of the compound are given below.
ESI/MS m/e: 425.4 (M⁺+H, C₁₄H₉Cl₂F₃N₄O₂S)

### Reference Example 31. Synthesis of N-{2-[6-(3-chloro(2-thienyl))-7-iodo-4-oxo(3-hydropyrrolo[3,2-d]pyrimidin-5-yl)]ethyl}(4-fluorophenyl)carboxyamide (Compound No. 344)

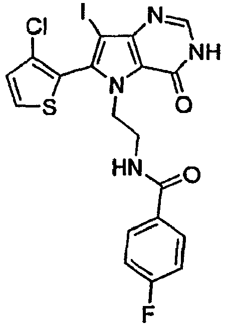

To an N,N-dimethylacetamide (2.0 mL) solution of 5-(2-aminoethyl)-6-(3-chloro(2-thienyl))-7-iodo-3-hydropyrrolo[3,2-d]pyrimidin-4-one hydrochloride (33 mg) was added 4-fluorobenzoylchloride (23 mg), and the mixture was stirred at room temperature for a short time, followed by adding triethylamine (0.2 mL) and stirring at room temperature for 2 hours. To the reaction solution was added water (0.2 mL), and the solution was stirred again at room temperature overnight. The solvent was distilled off under reduced pressure, and the residue was purified by fraction HPLC to obtain the title compound (22 mg, yield 55%). The HPLC retention time, NMR data and ESI/MS data of the compound are given below.
HPLC retention time=9.1(min)
¹H-NMR(400 MHz, DMSO-d₆) δ (ppm) : 3.52(m,2H), 4.24(m,1H), 4.63(m,1H), 7.19-7.26(m,3H), 7.70-7.74(m,2H), 7.88-7.92(m,2H), 8.45(m,1H), 12.24(brs,1H).
ESI/MS m/e: 543.4(M⁺+H, C₁₉H₁₃ClFIN₄O₂S)

### Reference Example 32. Synthesis of 6-(3-chloro(2-thienyl))-7-iodo-5-[2-(quinazolin-4-ylamino)ethyl]-3-hydropyrrolo [3,2-d]pyrimidin-4-one (Compound No. 81)

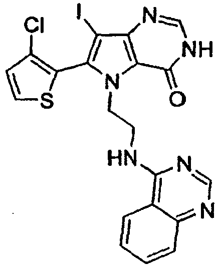

To an N,N-dimethylacetamide (2.0 mL) solution of 5-(2-aminoethyl)-6-(3-chloro(2-thienyl))-7-iodo-3-hydropyrrolo[3,2-d]pyrimidin-4-one hydrochloride(36 mg) and 4-chloroquinazoline (10 mg), was added triethylamine (16 mg) and the mixture was stirred at 70°C for 2 hours. Triethylamine (32 mg) was further added to the reaction mixture and stirred at 70°C for 2 hours. The reaction mixture was cooled to room temperature and purified by fraction HPLC to obtain the title compound (21 mg, yield 49%). The HPLC retention time, NMR data and ESI/MS data of the compound are given below.
HPLC retention time : 6.9(min)
¹H-NMR(400 MHz, DMSO-d₆)δ(ppm): 3.95(m, 2H), 4.48(m, 1H), 4.87(m,1H), 7.12(m,1H), 7.71-7.82(m,3H), 7.87(brs, 1H), 8.00(t,J=7.7,1H), 8.18(d, J=8.3,1H), 8.60(s, 1H), 9.91(brs, 1H), 12.24(brs, 1H).
ESI/MS m/e: 549.4(M⁺+H, C₂₀H₁₄ClIN₆OS)

### Reference Example 33. Synthesis of N-(5-{2-[(t-butoxy)carbonylamino]

### ethyl}-6-(3-chloro(2-thienyl))-4-oxo(3-hydropyrrolo[3,2-d]

### pyrimidin-7-yl))-2,2,2-trifluoroacetamide (Compound No. 962)

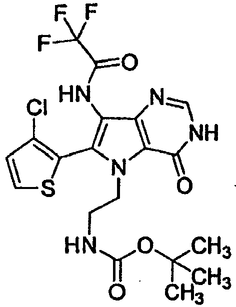

To a tetrahydrofuran solution (39 mL) of N-{2-[7-amino-6-(3-chloro(2-thienyl))-4-oxo(3-hydropyrrolo [3,2-d]pyrimidin-5-yl)]ethyl}(t-butoxy)carboxyamide (1.60 g) was added triethylamine (2.7 mL), and cooled to 0°C, followed by adding trifluoroacetic anhydride (1.35 mL) slowly dropwise. The reaction mixture was stirred at room temperature for 1 hour, and saturated brine was added dropwise to stop the reaction. Ethyl acetate was added to the reaction solution for extraction. The organic layer was washed with saturated brine, dried over anhydrous magnesium sulfate and filtered. The solvent was distilled off under reduced pressure to obtain the title compound (1.97 g, a quantitative yield) as a light yellow solid. The HPLC retention time, NMR data and ESI/MS data of the compound are given below.
HPLC retention time=8.7(min)
¹H-NMR(400 MHz, DMSO-d₆)δ(ppm): 1.26(s,9H), 3.13-3.26(m,2H), 4.11(brs,1H), 4.49(brs,1H),6.60-6.73(m,1H), 7.23(d,J=5.4,1H), 7.88(s,1H), 7.95(d,J=5.4,1H), 10.86(s,1H), 12.21(brs,1H).
ESI/MS m/e: 506.4(M⁺+H, C₁₉H₁₉ClF₃N₅O₄S)

### Reference Example 34. Synthesis of N-[5-(2-aminoethyl)-6-(3-chloro (2-thienyl))-4-oxo(3-hydropyrrolo[3,2-d]pyrimidin-7-yl)]-2,2,2-trifluoroacetamide hydrochloride (Compound No. 207)

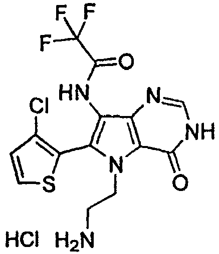

A methanol solution (13 mL) of N-(5-{2-[(t-butoxy) carbonylamino]ethyl}-6-(3-chloro(2-thienyl))-4-oxo(3-hydropyrrolo[3,2-d]pyrimidin-7-yl))-2,2,2-trifluoro-acetamide (1.97 g) was cooled to 0°C and added was 4 mol/L hydrochloric acid/1,4-dioxane solution (26 mL), followed by stirring at room temperature for 4 hours. The solvent was distilled off under reduced pressure, yielding a crude product (1.73 g) of the title compound. The ESI/MS data of the compound are given below.
ESI/MS m/e: 406.3(M⁺+H, C₁₄H₁₁F₃N₅O₂S·HCl)

### Reference Example 35. Synthesis of N-(6-(3-chloro(2-thienyl))-5-{2-[(4-fluorophenyl)carbonylamino]ethyl}-4-oxo(3-hydropyrrolo[3,2-d]pyrimidin-7-yl))-2,2,2-trifluoro-acetamide (Compound No. 345)

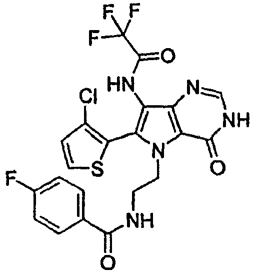

N-[5-(2-aminoethyl)-6-(3-chloro(2-thienyl))-4-oxo(3-hydropyrrolo[3,2-d]pyrimidin-7-yl)]-2,2,2-trifluoroacetamide crude product (1.73g) in pyridine (39 mL) was cooled to 0°C, and 4-fluorobenzoylchloride (0.92 mL) was added thereto, followed by stirring at room temperature for 1 hour. To the reaction solution was added water (40 mL), stirred at room temperature for 1 hour, and to stop the reaction. Brine was added until the reaction solution was saturated, and extraction with ethyl acetate was performed. The organic layer was washed with a mixed solution of saturated brine and 1 mol/L of hydrochloric acid (9:1), dried over anhydrous sodium sulfate and filtered. The solvent was distilled off under reduced pressure and purified by column chromatography on silica gel using 1/2 hexane/ethyl acetate and then using ethyl acetate only as eluents to obtain the title compound (1.60 g, 78% yield for 2 steps). The HPLC retention time, NMR data and ESI/MS data of the compound are given below.
HPLC retention time=8.3(min)
¹H-NMR(400 MHz, DMSO-d₆)δ(ppm): 3.49-3.58(m,2H), 4.34(brs,1H), 4.66(brs,1H), 7.16(d,J=5.4,1H), 7.19(t,J=7.1,2H), 7.65-7.75(m,2H), 7.83(d,J=5.4,1H), 7.88(s,1H), 8.40-8.50(m,1H), 10.85(s,1H), 12.25(brs,1H).
ESI/MS m/e: 528.4 (M⁺+H, C₂₁H₁₄ClF₄N₅O₃S)

### Reference Example 36. Synthesis of (t-butoxy)-N-{2-[6-(3-chloro (2-thienyl))-4-oxo-7-(phenylcarbonylamino)(3-hydropyrrolo [3,2-d]pyrimidin-5-yl)]ethyl}carboxyamide (Compound No. 965)

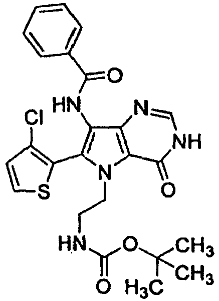

To a tetrahydrofuran solution (10 mL) of N-{2-[7-amino-6-(3-chloro(2-thienyl))-4-oxo(3-hydropyrrolo[3,2-d]pyrimidin-5-yl)]ethyl)(t-butoxy)carboxyamide (0.41 g) was added triethylamine (0.69 mL), and the reaction mixture was cooled to 0°C and benzoylchloride (0.29 mL) was added slowly dropwise. The reaction mixture was stirred at room temperature for 1 hour, and an aqueous sodium hydroxide solution (2 mol/L, 2.0 mL) was added dropwise and stirred for 18 hours to stop the reaction. Hydrochloric acid (1 mol/L, 4.0 mL) was added to the reaction solution for neutralization, brine was added thereto until the reaction solution was saturated, and extraction with ethyl acetate was performed. The organic layer was washed with saturated brine, dried over anhydrous sodium sulfate and filtered. The solvent was distilled off under reduced pressure, and purified by column chromatography was performed on silica gel using ethyl acetate only to obtain the title compound (0.51 g, a quantitative yield). The ESI/MS data of the compound are given below.
ESI/MS m/e: 514.4(M⁺+H, C₂₄H₂₄ClN₅O₄S)

### Reference Example 37. Synthesis of N-(5-(2-aminoethyl)-6-(3-chloro(2-thienyl))-4-oxo(3-hydropyrrolo[3,2-d]pyrimidin-7-yl)]benzamide hydrochloride (Compound No. 208)

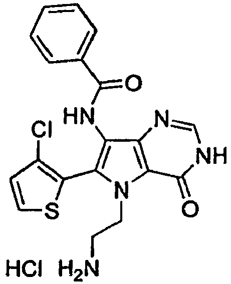

To a methanol solution (3.3 mL) of (t-butoxy)-N-{2-[6-(3-chloro(2-thienyl))-4-oxo-7-(phenylcarbonylamino)(3-hydropyrrolo[3,2-d]pyrimidin-5-yl)]ethyl}carboxyamide (0.51 g) was added 4 mol/L hydrochloric acid/1,4-dioxane solution(6.6 mL) and the mixture was stirred at room temperature for 5 hours. The solvent was distilled off under reduced pressure, yielding a crude product (0.47 g) of the title compound. The ESI/MS data of the compound are given below.
ESI/MS m/e: 414.3 (M⁺+H, C₁₉H₁₆ClN₅O₂·HCl)

### Reference Example 38. Synthesis of N-{2-[6-(3-chloro(2-thienyl))-4-oxo-7-(phenylcarbonylamino)(3-hydropyrrolo[3,2-d] pyrimidin-5-yl)]ethyl}(4-fluorophenyl)carboxyamide (Compound No. 346)

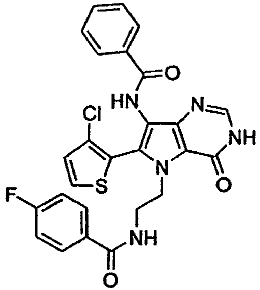

To a dimethylacetamide solution (1.0 mL) of N-[5-(aminoethyl)-6-(3-chloro(2-thienyl))-4-oxo(3-hydropyrrolo[3,2-d]pyrimidin-7-yl)]benzamide (45 mg), were added 4-fluorobenzoylchloride (23.6 µL) and triethylamine (55 µL) and the mixture was stirred at room temperature for 1 hour. 2 mol/L of an aqueous sodium hydroxide solution (1.0 mL) was added dropwise, and the solution was stirred for 1 hour and stopped the reaction. 1 mol/L of hydrochloric acid (2.0 mL) was added to the reaction solution for neutralization, brine was added thereto until the reaction solution was saturated, and extraction with ethyl acetate was performed. The organic layer was washed with saturated brine, dried over anhydrous sodium sulfate and filtered. The solvent was distilled off under reduced pressure, and purified by fraction HPLC to obtain the title compound (25.5 mg, yield 48%). The HPLC retention time, NMR data and ESI/MS data of the compound are given below.
HPLC retention time=8.2(min)
¹H-NMR(400 MHz, DMSO-d₆) δ(ppm): 3.50-3.70(m,2H), 4.35(brs,1H), 4.68(brs,1H), 7.12(d,J=5.4,1H), 7.22(t,J=8.8,2H), 7.44(t,J=7.3,2H), 7.47-7.56(m,1H), 7.69-7.78(m,3H), 7.84(d,J=7.6,2H), 7.89(s,1H), 8.47(t,J=5.4,1H),9.72(s,1H), 12.22(brs,1H).
ESI/MS m/e: 536.4(M⁺+H, C₂₆H₁₉ClFN₅O₃S)

### Reference Example 39. Synthesis of N-{6-(3-chloro(2-thienyl))-4-oxo-5-[2-(quinazolin-4-ylamino)ethyl](3-hydropyrrolo[3,2-d]

### pyrimidin-7-yl)}benzamide (Compound No. 82)

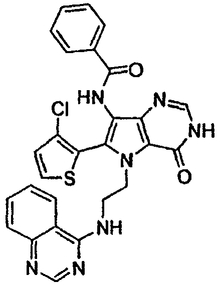

N-[5-(aminoethyl)-6-(3-chloro(2-thienyl))-4-oxo(3-hydropyrrolo[3,2-d]pyrimidin-7-yl)]benzamide (45 mg) and 4-chloroquinazoline (16.5 mg) were dissolved in dimethylacetamide (2.9 mL), and triethylamine (27.7 µL) was added thereto and the mixture was stirred at 70°C for 3 hours. Triethylamine (13.9 µL) was further added to the reaction mixture and the solution was stirred at 70°C for 5 hours. The reaction solution was cooled to room temperature and purified by fraction HPLC, to obtain the title compound (44.3 mg, 82%). The HPLC retention time, NMR data and ESI/MS data of the compound are given below. HPLC retention time=6.4(min)
¹H-NMR(400 MHz, DMSO-d₆)δ(ppm): 3.95-4.05(m,2H), 4.52(brs,1H), 5.00(brs,1H), 7.05(d,J=5.4,1H), 7.43(t,J=7.6,2H), 7.52(t,J=7.3,1H), 7.65(d,J=5.4,1H), 7.70-7.85(m,5H), 8.02(t,J=7.8,1H), 8.23(d,J=8.5,1H), 8.61(s,1H),9.66(s,1H), 10.17(m,1H), 12.10(brs,1H).
ESI/MS m/e: 542.4(M⁺+H, C₂₇H₂₀ClN₇O₂S)

### Reference Example 40. Synthesis of (t-butoxy)-N-{2-[6-(3-chloro (2-thienyl))-4-oxo-7-vinyl(3-hydropyrrolo[3,2-d]pyrimidin-5-yl)]ethyl}carboxyamide (Compound No. 966)

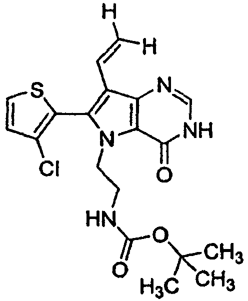

A nitrogen flushed flask was charged with a 0.5 M zinc chloride/tetrahydrofuran solution, and 1.0 mL of a 1.0 M vinyl magnesium bromide/tetrahydrofuran solution was added thereto with stirring, the mixture solution was stirred for 30 minutes, to obtain a suspension which was used in a subsequent reaction. To a round-bottom flask, were transferred 26 mg of (t-butoxy)-N-{2-[6-(3-chloro(2-thienyl))-4-oxo-7-iodo(3-hydropyrrolo[3,2-d]pyrimidin-5-yl)]ethyl}carboxyamide and 12 mg of tetrakistriphenylphosphine palladium (0), followed by flushing with nitrogen and adding 2 mL dry tetrahydrofuran thereto. 600 µl of the suspension was added to the reaction mixture and the mixture was stirred at 50°C for 8 hours, and saturated brine was added thereto and extracted with ethyl acetate. The organic layer was dried over anhydrous sodium sulfate, filtered and concentrated. The obtained crude product was subjected to chromatography on silica gel (eluent: 5:1 to 0:1 hexane:ethyl acetate), to obtain the title compound (5.5 mg, yield 26%). The HPLC retention time and ESI/MS data of the compound are given below.
HPLC retention time=9.6(min)
EMS/MS m/e 421.2(M⁺+H, C₁₉H₂₁ClN₄O₃S)

### Reference Example 41. Synthesis of (t-butoxy)-N-{2-[6-(3-chloro(2- thienyl))-7-(3-hydroxy-1-prop-1-ynyl)-4-oxo(3-hydropyrrolo

### [3,2-d]pyrimidin-5-yl)]ethyl}carboxyamide (Compound No. 971)

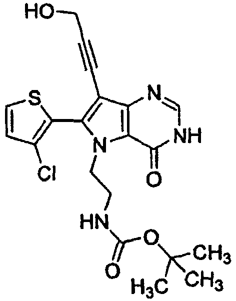

A round-bottom flask was charged with 26 mg of (t-butoxy)-N-{2-[6-(3-chloro(2-thienyl))-4-oxo-7-iodo(3-hydropyrrolo[3,2-d]pyrimidin-5-yl)]ethyl}carboxyamide, 12 mg of tetrakistriphenylphosphine palladium, 3.8 mg of copper (I) iodide and 15 µl of propargyl alcohol, followed by flushing with nitrogen, and 1 mL of dry tetrahydrofuran and 209 µl of triethylamine were added thereto. The reaction mixture was stirred at 50°C for 5 hours, and saturated brine was added thereto and extracted with ethyl acetate. The organic layer was concentrated and subjected to column chromatography on silica gel, (developing solvent: 10 mL of toluene to 8 mL of ethyl acetate:methanol=2:1). A portion eluted by ethyl acetate/methanol was concentrated and subjected to fraction HPLC to obtain the title compound. The HPLC retention time and ESI/MS data of the compound are given below.
HPLC retention time=9.0(min)
EMS/MS m/e 448.9(M+H⁺, C₂₀H₂₁ClN₄O₄S)

### Example 1. Synthesis of N-{2-[7-benzo[d]furan-2-yl-6-(3-chloro(2-thienyl))-4-oxo(3-hydropyrrolo[3,2-d]pyrimidin-5-yl)]ethyl}(t-butoxy)carboxyamide (Compound No. 1000)

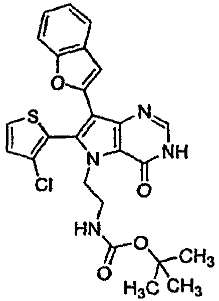

A round-bottom flask was charged with 26 mg of (t-butoxy)-N-{2-[6-(3-chloro(2-thienyl))-4-oxo-7-iodo(3-hydropyrrolo[3,2-d]pyrimidin-5-yl)]ethyl}carboxyamide, 2.2 mg of palladium acetate, triphenylphosphine, 26 mg of sodium carbonate and 16.2 mg of 2-benzofuranyl boric acid and flushed with nitrogen. 1 mL of a solution containing dimethylformamide and water mixed in a ratio of 2:1 was added to the reaction mixture and the mixture was stirred at 80°C for 24 hours, and saturated brine was added thereto and extracted with ethyl acetate. The organic layer was concentrated and subjected to chromatography on silica gel, (developing solvent: 10 mL of toluene to 8 mL of ethyl acetate/methanol=2:1). A portion eluted by ethyl acetate/methanol was concentrated and subjected to fraction HPLC to obtain the title compound. The HPLC retention time and ESI/MS data of the compound are given below.
HPLC retention time =11.6(min)
EMS/MS m/e 511.3(M+H⁺, C₂₅H₂₃ClN₄O₄S)

### Reference Example 42. Synthesis of N-{2-[6-(3-chloro-thiophen-2-yl)-7-iodo-4-oxo-3-hydropyrrolo[3,2-d]pyrimidin-5-yl]ethyl}-t-butylacetylamide (Compound No. 625)

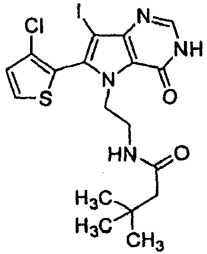

To an N,N-dimethylacetamide solution (5.0 mL) of 5-(2-aminoethyl)-6-(3-chloro-thiophen-2-yl)-7-iodo-3-hydropyrrolo[3,2-d]pyrimidin-4-one hydrochloride(173 mg) was added t-butylacetyl chloride(95 mg) and the mixture was stirred at room temperature for a short time, followed by adding triethylamine (1.0 mL) and stirring at room temperature for 2 hours. To the reaction solution was added water (1.0 mL) and further the solution was stirred at room temperature overnight. The obtained solution was extracted with ethyl acetate (10 mL × 2). The organic layer was washed with saturated brine, and the solvent was distilled off under reduced pressure. The obtained crude product was purified by column chromatography on silica gel (using ethyl acetate only) to obtain the title compound (160 mg, yield: 88%). The NMR data and ESI/MS data of the compound are given below.
¹H-NMR(400 MHz, DMSO-d₆) δ(ppm): 0.84(s,9H), 1.81(d,J=3.67,2H), 3.25-3.34(m,2H), 4.05-4.12(m,1H), 4.44-4.50(m,1H), 7.28(d,J=5.36,1H), 7.69(t,J=5.86,1H), 7.92(d,J=3.67,1H), 8.00(d,J=5.36,1H), 12.25(brs,1H).
ESI/MS m/e: 519.1(M⁺+H, C₁₈H₂₀ClIN₄O₂S)

### Example 2. Synthesis of N-{2-[7-(1H-pyrazol-4-yl)-6-(3-chloro-thiophen-2-yl)-4-oxo-3-hydropyrrolo[3,2-d]pyrimidin-5-yl]ethyl}-t-butylacetylamide (Compound No_{.} 629)

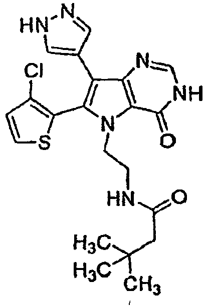

To a screw-cap vial were transferred N-{2-[6-(3-chloro-thiophen-2-yl)-4-oxo-7-iodo-3-hydropyrrolo[3,2-d]pyrimidin-5-yl]ethyl}-t-butylacetylamide (26 mg), palladium acetate (2.2 mg), triphenylphosphine (5.2 mg), sodium carbonate (26 mg) and 4-(4,4,5,5,-tetramethyl-[1,3,2]-dioxaborolan-2-yl)-1H-pyrazole (19.4 mg), and flushed with nitrogen. A 2:1 dimethylformamide:water solution (1 mL) was added to the reaction mixture and the mixture was stirred at 80°C for 12 hours. Saturated brine was added to the obtained solution and extracted with ethyl acetate. The organic layer was concentrated and purified by fraction HPLC to obtain the title compound (5.2 mg, yield 23%). The HPLC retention time, NMR data and ESI/MS data of the compound are given below.
HPLC retention time=10.9(min)
¹H-NMR(400 MHz, DMSO-d₆) δ(ppm): 0.85(s,9H), 1.84(d,J=1.68,2H), 3.30-3.38(m,2H), 4.04-4.11(m,1H), 4.35-4.40(m,1H), 7.34(d,J=5.38,1H), 7.44(brs,2H), 7.76(t,J=5.60,1H), 7.93(s,1H), 8.05(d, J=5.38,1H), 12.17(brs,1H).
EMS/MS m/e 459.3 (M+H⁺, C₂₁H₂₃ClN₆O₂S

### Example 3. Synthesis of 5-(2-aminoethyl)-6-(3-chloro-thiophen-2-yl)-7-pyridin-3-yl-3-hydropyrrolo[3,2-d] pyrimidin-4-one (Compound No. 209) dihydrochloride

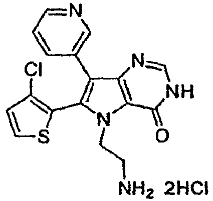

(t-butoxy)-N-{2-[6-(3-chloro-thiophen-2-yl)-7-pyridin-3-yl-4-oxo-3-hydropyrrolo[3,2-d]pyrimidin-5-yl]-ethyl}carboxyamide(1.68 g) was dissolved in methanol(10.0 mL), followed by adding 4 mol/L hydrochloric acid/1,4-dioxane solution(2.0 mL) thereto and stirring at 60°C for 12 hours. The reaction mixture was cooled to room temperature, and the solvent was distilled off under reduced pressure. The residue was dried under reduced pressure to obtain the title compound (1.64 g, a quantitative yield). The ESI/MS data of the compound are given below.
ESI/MS m/e: 371.9(M⁺+H, C₁₇H₁₄ClN₅OS 2HCl)

### Example 4. Synthesis of 1-{2-[6-(3-chloro-thiophen-2-yl)-4-oxo-7-pyridin-3-yl-3-hydropyrrolo[3,2-d]pyrimidin-5-yl]ethyl}-3-phenylurea (Compound No. 793)

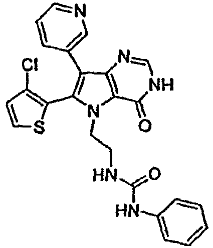

To an N,N-dimethylformamide (1.0 mL) solution of 5-(2-aminoethyl)-6-(3-chloro-thiophen-2-yl)-7-pyridin-3-yl-3-hydropyrrolo[3,2-d]pyrimidin-4-one dihydrochloride (25 mg) was added phenylisocyanate (20 mg) and the mixture was stirred at room temperature for a short time, followed by adding triethylamine (0.2 mL) and stirring at room temperature for 2 hours. Saturated brine was added to the obtained solution and extracted with ethyl acetate. The reaction solution was distilled off under reduced pressure, and the residue was purified by fraction HPLC to obtain the title compound (8 mg, yield 29%). The HPLC retention time and ESI/MS data of the compound are given below.
HPLC retention time=6.6(min)
ESI/MS m/e: 491.04(M⁺+H, C₂₄H₁₉ClN₆O₂S

### Example 5. Synthesis of 4-fluoropiperidine-1-{2-[6-(3-chloro-thiophen-2-yl)-4-oxo-7-pyridin-3-yl-3-hydropyrrolo [3,2-d]pyrimidin-5-yl]-ethyl}-carboxyamide (Compound No. 550)

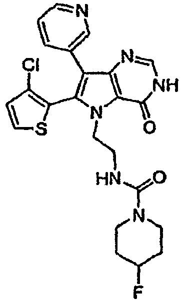

To a chloroform (1.0 mL) solution of 5-(2-aminoethyl)-6-(3-chloro-thiophen-2-yl)-7-pyridin-3-yl-3-hydropyrrolo
[3,2-d]pyrimidin-4-one dihydrochloride (25 mg) were added triethylamine (0.078 mL) and triphosgene (17 mg) and the mixture was stirred at room temperature for a short time, and 4-fluoropiperidine (12 mg) was added thereto followed by stirring at room temperature for 3 hours. Saturated brine was added to the obtained solution and extracted with ethyl acetate. The reaction solution was distilled off under reduced pressure, and the residue was purified by fraction HPLC to obtain the title compound (1.2 mg, yield 5%). The HPLC retention time and ESI/MS data of the compound are given below.
HPLC retention time=6.9(min)
ESI/MS m/e: 501.42(M⁺+H, C₂₃H₂₂ClFN₆O₂S)

### Example 6. Synthesis of 5-[2-(bis(cyclopropylmethyl)amino) ethyl]-6-(3-chloro-thiophen-2-yl)-7-pyridin-3-yl-3-hydropyrrolo[3,2-d]pyrimidin-4-one (Compound No. 1057)

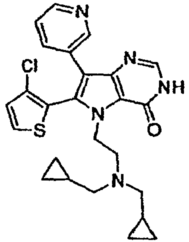

To an N,N-dimethylformamide (1.0 mL) solution of 5-(2-aminoethyl)-6-(3-chloro-thiophen-2-yl)-7-pyridin-3-yl-3-hydropyrrolo[3,2-d]pyrimidin-4-one dihydrochloride (25 mg) were added cyclopropyl aldehyde (5 mg), sodium triacetoxy borohydride (24 mg) and acetic acid (0.1 mL) and the mixture was stirred at room temperature for 10 hours. Saturated brine was added to the obtained solution and extracted with ethyl acetate. The reaction solution was distilled off under reduced pressure, and the residue was purified by fraction HPLC to obtain the title compound (10 mg, yield 36%). The HPLC retention time and ESI/MS data of the compound are given below.
HPLC retention time=5.4(min)
ESI/MS m/e: 482.0(M⁺+H, C₂₅H₂₆ClFN₅OS)

### Example 7. Synthesis of N-{2-[6-(3-chloro-thiophen-2-yl)-4-oxo-7-pyridin-3-yl-3-hydropyrrolo[3,2-d]pyrimidin-5-yl]

### ethyl}-t-butylacetylamide (Compound No. 626)

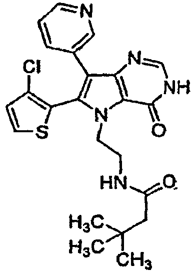

To an N,N-dimethylacetamide (1.0 mL) solution of 5-(2-aminoethyl)-6-(3-chloro-thiophen-2-yl)-7-pyridin-3-yl-3-hydropyrrolo[3,2-d]pyrimidin-4-one dihydrochloride (25 mg) was added t-butylacetyl chloride (23 mg) and the mixture was stirred at room temperature for a short time, followed by adding triethylamine (0.2 mL) and stirring at room temperature for 2 hours. Water (0.2 mL) was added to the reaction solution and the solution was stirred again at room temperature overnight. Saturated brine was added to the obtained solution and extracted with ethyl acetate. The reaction solution was distilled off under reduced pressure, and the residue was purified by fraction HPLC to obtain the title compound (19 mg, yield 72%). The HPLC retention time and ESI/MS data of the compound are given below.
HPLC retention time=7.6(min)
ESI/MS m/e: 470.1(M⁺+H, C₂₃H₂₄ClN₅O₂S)

### Example 8. Synthesis of 1-methyl-cyclohexane-{2-[6-(3-chloro-thiophen-2-yl)-4-oxo-7-pyridin-3-yl-3-hydropyrrolo [3,2-d]pyrimidin-5-yl]-ethyl}-carboxyamide (Compound No. 684)

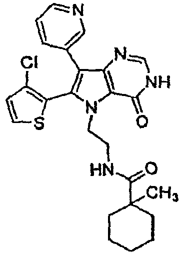

To an N,N-dimethylformamide (1.0 mL) solution of 5-(2-aminoethyl)-6-(3-chlorothiophen-2-yl)-7-pyridin-3-yl-3-hydropyrrolo[3,2-d]pyrimidin-4-one dihydrochloride (25 mg) were added 1-methyl-cyclohexanecarboxylic acid (24 mg), 1-ethyl-3-(3'-dimethylaminopropyl)carboxyamide hydrochloride (43 mg), N-hydroxybenzotriazole(8 mg) and triethylamine (0.2 mL) and the mixture was stirred at room temperature for 10 hours. Saturated brine was added to the obtained solution and extracted with ethyl acetate. The reaction solution was distilled off under reduced pressure, and the residue was purified by fraction HPLC to obtain the title compound (11 mg, yield 41%). The HPLC retention time and ESI/MS data of the compound are given below.
HPLC retention time=8.2(min)
ESI/MS m/e: 496.0(M⁺+H, C₂₅H₂₆ClN₅O₂s)

### Example 9. Synthesis of N-{2-[6-(3-chloro(2-thienyl))-4-oxo-7-(3-pyridyl)-3-hydropyrrolo[3,2-d]pyrimidin-5-yl] ethyl}acetamide (Compound No. 217)

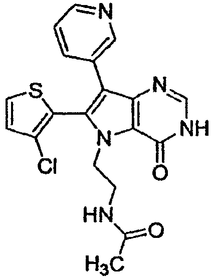

To a 1,4-dioxane (6.0 mL) and methanol (1.0 mL) solution of (t-butoxy)-N-(2-[6-(3-chloro(2-thienyl))-4-oxo-7-(3-pyridyl)(3-hydropyrrolo[3,2-d]pyrimidin-5-yl)]ethyl} carboxyamide (356 mg) was added 4 mol/L hydrochloric acid/dioxane solution (0.75 mL) and the mixture was stirred 60°C for 1 hour. The reaction solution was cooled to room temperature, and the solvent was distilled off under reduced pressure. The residue was dried under reduced pressure to obtain crude product (370 mg). To a dimethylacetamide solution (2.0 mL) of the reaction product (26 mg) were added chloroacetyl (10 mg) and triethylamine (0.2 mL) and the mixture was stirred at room temperature for 1 hour, followed by adding water (0.2 mL) and further stirring at room temperature for 1 hour. Purification by fraction HPLC was performed to obtain the title compound (15 mg, yield 57%). The HPLC retention time, NMR data and ESI/MS data of the compound are given below.
HPLC retention time=5.7(min)
¹H-NMR(400 MHz, DMSO-d₆) δ(ppm): 1.65(s,3H), 3.31-3.41(m,2H), 4.16(m,1H), 4.50(m,1), 7.29(m,1H), 7.61(dd,J=5.12,J=8.08,1H), 7.86(m,1H), 7.92(m,1H), 8.01(m,2H), 8.54(d,J=5.12,1H), 8.64(s,1H), 12.38(brs,1H).
ESI/MS m/e: 414.4(M⁺+H, C₁₉H₁₆ClN₅O₂S)

### Example 10. Synthesis of 6-(3-chloro(2-thienyl))-7-(3-pyridyl)-5-[2-quinazolin-4-ylamino]ethyl]-3-hydropyrrolo [3,2-d]pyrimidin-4-one (Compound No. 84)

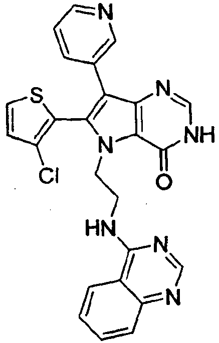

A 4 mol/L hydrochloric acid/dioxane solution (0.75 mL) was added to a 1,4-dioxane (6.0 mL) and methanol (1.0 mL) solution of (t-butoxy)-N-{2-[6-(3-chloro(2-thienyl))-4-oxo-7-(3-pyridyl)(3-hydropyrrolo[3,2-d]pyrimidin-5-yl)]ethyl}
carboxyamide (356 mg) and the mixture was stirred at 60°C for 1 hour. The reaction solution was cooled to room temperature, and the solvent was distilled off under reduced pressure. The residue was dried under reduced pressure to obtain a crude product (370 mg). To a dimethylacetamide (2.0 mL) solution of the reaction product (32 mg) were added 4-chloroquinazoline (13 mg) and triethylamine (16 mg) and the solution was stirred at 70°C for 2 hours. The reaction solution was cooled to room temperature, and purification by fraction HPLC was performed to obtain the title compound (21 mg, yield 54%). The HPLC retention time, NMR data and ESI/MS data of the compound are given below.
HPLC retention time=5.6 (min)
¹H-NMR(400 MHz, DMSO-d₆) δ(ppm): 4.01-4.06(m,2H), 4.55(m,1H), 4.96(m,1H), 7.08(m,1H),
7.47(dd,J=4.88,J=8.04,1H), 7.72-7.78(m,3H), 7.83(m,1H), 7.90(s,1H), 8.02(t,J=7.68,1H), 8.23(d,J=8.52,1H), 8.46(d,J=5.12,1H), 8.52(s,1H), 8.67(s,1H), 10.18(m,1H), 12.30(brs,1H).
ESI/MS m/e: 500.4(M⁺+H, C₂₅H₁₈ClN₇OS)

### Example 11. Synthesis of ethyl 3-(4-oxo-7-(3-pyridyl)-3-hydropyrrolo[3,2-d]pyrimidin-5-yl)propanate (Compound No. 35)

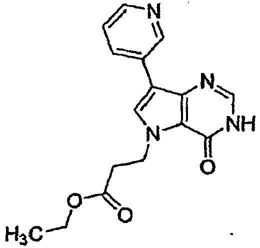

To an isopropyl alcohol (80 mL) solution of ethyl 3-[3-amino-2-(methoxycarbonyl)-4-(3-pyridyl)pyrrolyl] propanate (4.380g) was added formamidine acetate (7.184 g) and the mixture was stirred at 90°C for 10 hours. The reaction solution was cooled to room temperature, and the solid was subjected to fractional filtration. The extractant was distilled off under reduced pressure. To the residue was added water and extracted with ethyl acetate. The organic layer was washed with saturated brine and dried with sodium sulfate, which was then subjected to fractional filtration. Then, the solvent was distilled off under reduced pressure. The residue was purified by column chromatography on silica gel (ethyl acetate:ethanol=7:1), to obtain the title compound (0.747 g, yield 17%). The HPLC retention time, NMR data and ESI/MS data of the compound are given below.
HPLC retention time=5.6(min)
¹H-NMR(400 MHz, DMSO-d₆) δ(ppm): 1.11(t,J=7.08,3H), 2.95(t,J=6.96,2H), 4.03(dd,J=6.96,J=14.28,2H), 4.63(t,J=6.84,2H), 7.81(dd,J=5.12,J=8.04,1H), 7.99(s,1H), 8.22(s,1H), 8.60(d,J=5.12,1H), 8.81(d,J=8.04,1H),9.38(s,1H), 12.29(s,1H). ESI/MS m/e: 313.2(M⁺+H, C₁₆H₁₆N₄O₃)

### Example 12. Synthesis of 3-(4-oxo-7-(3-pyridyl) (3-hydropyrrolo[3,2-d]pyrimidin-5-yl))-N-phenylpropaneamide (Compound No. 30)

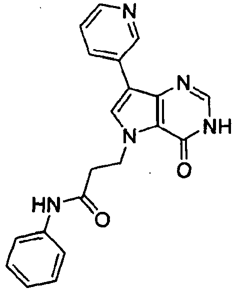

Water (0.40 mL) and a 5 M aqueous sodium hydroxide solution (0.18 mL) were added to a 1,4-dioxane (3.0 mL) solution of ethyl 3-(4-oxo-7-(3-pyridyl)-3-hydropyrrolo[3,2-d]pyrimidin-5-yl)propanate (91 mg) and the mixture was stirred at room temperature for 2 hours. The reaction solution was neutralized with acetic acid, and the resulting solid was filtered and washed with water and ethyl acetate to obtain a crude product (78 mg). 1-ethyl-3-(3'-dimethylaminopropyl)carbodiimide hydrochloride (41 mg) and pyridine (0.2 mL) were added to a mixed solution of dichloromethane (1.0 mL) and dimethylacetamide (1.0 mL) of the reaction product (20 mg) and the mixture was stirred at room temperature for 1 hour. To the reaction solution was added aniline (20 mg) and the solution was stirred at 40°C overnight. Methanol was added to stop the reaction, and the solvent was distilled off under reduced pressure. The residue was purified by fraction HPLC to obtain the title compound (19 mg, yield 76%). The HPLC retention time, NMR data and ESI/MS data of the compound are given below.
HPLC retention time=6.1(min)
¹H-NMR(400 MHz, DMSO-d₆) δ(ppm): 2.97(t,J=6.58,2H), 4.69(t,J=6.48,2H),6.99(t,J=7.46,1H), 7.24(t,J=7.92,2H), 7.51(d,J=8.56,2H), 7.78(dd,J=5.50,J=7.94,1H), 7.99(s,1H), 8.18(s,1H), 8.57(d,J=5.40,1H),
8.78(d,J=8.04,1H),9.36(s,1H),9.96(s,1H), 12.30(brs,1H).
ESI/MS m/e: 360.2(M⁺+H, C₂₀H1₇N₅O₂)

### Reference Example 43. Synthesis of 6-chloro-5-benzyl-7-(3-pyridyl)-3-hydropyrrolo[3,2-d]pyrimidin-4-one (Compound No. 1180)

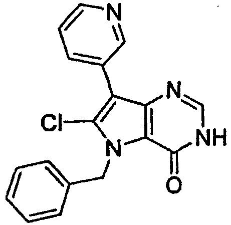

N-chlorosuccinimide (57 mg) was added to a dimethylformamide (2.0 mL) solution of 5-benzyl-7-(3-pyridyl)-3-hydropyrrolo[3,2-d]pyrimidin-4-one (65 mg) and the mixture was stirred at room temperature for 2 hours. N-chlorosuccinimide (57 mg) was added to the reaction mixture and further the mixture was stirred at room temperature for 2 hours. Water (0.2 mL) was added to stop the reaction, and purification by fraction HPLC gave the title compound (33 mg, yield 46%). The HPLC retention time, NMR data and ESI/MS data of the compound are given below.
HPLC retention time=8.0(min)
¹H-NMR(400 MHz, DMSO-d₆) δ(ppm): 5.82(s,2H), 7.22-7.36(m,5H), 7.72(dd,J=5.12,J=8.04,1H), 8.00(s,1H), 8.42(m,1H), 8.64(d,J=5.12,1H),9.07(s,1H), 12.41(brs,1H).
ESI/MS m/e: 337.3(M⁺+H, C₁₈H₁₃ClN₄O

### Reference Example 44. Synthesis of 7-(3-pyridyl)-4-oxo-6-cyclopropyl-3-hydropyrrolo[3,2-d]pyrimidine (Compound No. 1178)

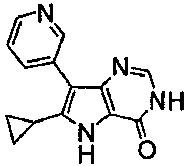

To a 100 mL branched flask was transferred ethyl 3-amino-4-(3-pyridyl)-5-cyclopropyl pyrrole-2-carboxylate (118 mg), and isopropylalcohol (40 mL) and formamidine acetate (1.35 g) were added thereto and heated at 95°C for 13 hours with stirring. After the completion of the reaction, the solvent was concentrated under reduced pressure. To the concentrated residue were added water (20 mL) and ethyl acetate (20 mL) for separation. The aqueous layer was extracted again with 20 mL ethyl acetate. The organic layer was washed twice with 20 mL water, dehydrated and dried with magnesium sulfate, filtered and concentrated to obtain a crude product (91 g). The crude product was washed 3 times with 1 mL methanol to yield the title compound (49 mg, yield 45%).
The ESI/MS data of the compound are given below.
HPLC retention time=4.2 (min)
ESI/MS m/e: 253.1(M⁺+H, C₁₄H₁₂N₄O)

### Reference Example 45. Synthesis of (t-butoxy)-N-{2-[6-(3-chloro(2-thienyl))-4-oxo-7-(trifluoromethyl)(3-hydropyrrolo[3,2-d] pyrimidin-5-yl)]ethyl}carboxyamide (Compound No. 947)

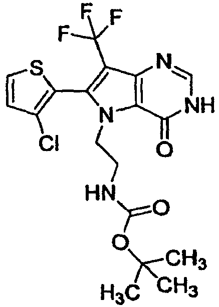

Under nitrogen atmosphere, a dry dimethylformamide suspension (10 mL) of cadmium (2.24 g) was cooled on ice with stirring, and dibromodifluoromethane (1.5 mL) was added thereto, followed by stirring at room temperature for 3 hours. Dry hexamethylphosphoramide (10 mL) was added to the reaction solution and cooled on ice, and copper (I) bromide (1.16 g) was added to the reaction mixture and the mixture was stirred at room temperature for 1 hour to obtain a copper trifluoromethyl compound suspension.

The copper trifluoromethyl compound suspension (10 mL) was added to a dry dimethylformamide solution (3 mL) of (t-butoxy)-N-(2-{6-(3-chloro(2-thienyl))-7-iodo-4-oxo(3-hydropyrrolo[3,2-d]pyrimidin-5-yl)]ethyl}carboxyamide (115 mg) and the solution was stirred at 65°C for 6 hours. An aqueous ammonium chloride solution was added to the reaction solution and extracted with ethyl acetate. The organic layer was washed with brine, dried over sodium sulfate, filtered, concentrated, and purified by chromatography on silica gel, to obtain the title compound (35 mg, 34%). The HPLC retention time, NMR data and ESI/MS data of the compound are given below.
HPLC retention time=12.1 (min)
¹H-NMR(270MHz,CDCl₃)δ(ppm): 1.32(s,9H), 3.35-3.60(m,2H), 4.00-4.30(m,1H), 4.40-4.70(m,1H), 7.11(d,J=5.1Hz,1H), 7.61(d,J=5.4Hz,1H), 7.95-8.10(m,1H).
ESI/MS m/e: 463.2(M⁺+H, C₁₈H₁₈ClF₃N₄O₃S)

### Reference Example 46. Synthesis of 6-phenyl-7-(3-pyridyl)-3-hydropyrrolo[3,2-d]pyrimidin-4-one (Compound No. 2445)

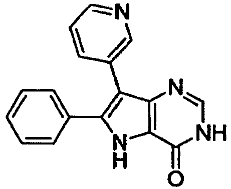

An N,N-dimethylformamide/water (2:1) solution (1.0 mL) of 6-chloro-7-(3-pyridyl)-3-hydropyrrolo[3,2-d] pyrimidin-4-one (40 mg), phenyl boric acid (59 mg), and potassium acetate (78 mg) was subjected to deaeration, and a small amount of [1,1'-bis(diphenylphosphino)ferrocene]
palladium (II) chloride dichloro-methane (1:1) complex was added thereto. The reaction mixture was heated at 140°C for 5 minutes by using microwaves. The solvent was distilled off under reduced pressure and the residue was separated to ethyl acetate and aqueous layers. The organic layer was washed with saturated aqueous sodium hydrogen carbonate solution and brine and dried over sodium sulfate. The sodium sulfate was filtered, and solvent was distilled off under reduced pressure. The residue was purified by chromatography on silica gel (0-5% methanol/ethyl acetate), and further purified by fraction HPLC to obtain the title compound (6.3 mg, yield 10%). The HPLC retention time, NMR data and ESI/MS data of the compound are given below.
HPLC retention time: 4.0 (min)
¹H-NMR(400MHz,CD₃OD)δ(ppm): 7.50(m,5H), 7.86(dd,J=5.7, J=8.2, 1H), 7.98(s,1H), 8.44(dt,J=1.6, J=8.2, 1H), 8.62(d,J=5.7, 1H), 8.94(s,1H).
ESI/MS m/e: 289.1(M⁺+H, C₁₇H₁₂N₄O)

### Reference Example 47. Synthesis of ethyl 4-(4-oxo-3-hydropyrrolo [3,2-d]pyrimidin-5-yl)butanoate (Compound No. 2501)

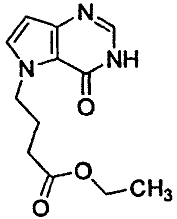

An ethyl 4-[3-amino-2-(ethoxycarbonyl)pyrolyl] butanoate hydrochloride (20.1g) was dissolved in dichloromethane (400 mL), and saturated aqueous sodium hydrogen carbonate (400 mL) was added thereto. The reaction mixture was vigorously stirred for 30 minutes, and two layers were separated. The aqueous layer was extracted with dichloromethane, and combined organic layer was washed with saturated brine, and dried over magnesium sulfate. The magnesium sulfate was filtered, and the solvent was distilled off under reduced pressure to obtain ethyl 4-[3-amino-2-(ethoxycarbonyl) pyrolyl]butanoate (18.0 g). This compound was dissolved in isopropylalcohol (400 mL), and formamidine acetate (9.78 g) was added thereto. This mixture was heated for 150 minutes to reflux. The solvent was distilled off under reduced pressure and the residue was separated to dichloromethane and aqueous layers. The aqueous layer was extracted with dichloromethane, and the combined organic layer was washed with 10% aqueous citric acid solution, saturated aqueous sodium hydrogen carbonate, and saturated brine, which was dried over magnesium sulfate, and the solvent was distilled off under reduced pressure to obtain the title compound (13.0 mg, yield 78%). The NMR data and ESI/MS data of the compound are given below.
¹H-NMR(400MHz,DMSO-d₆)δ(ppm): 1.14(t,J=7.1, 3H), 2.01 (pent,J=7.0, 2H), 2.21(t,J=7.5, 2H), 3.99(q,J=7.1, 2H), 4.38(t,J=6.7, 2H), 6.33(d,J=2.9, 1H), 7.41(d,J=2.9, 1H), 7.76(s, 1H), 11.86(brs, 1H).
ESI/MS m/e: 250.1(M⁺+H, C₁₂H₁₅N₃O₃)

### Reference Example 48. Synthesis of ethyl 4-(7-iodo-4-oxo-3-hydropyrrolo[3,2-d]pyrimidin-5-yl)butanoate (Compound No. 2502)

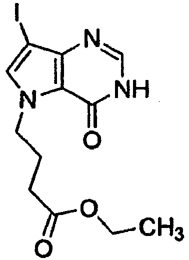

An N-iodosuccinimide (12.8 g) was added to a dichloromethane (350 mL) solution of ethyl 4-(4-oxo-3-hydropyrrolo[3,2-d]pyrimidin-5-yl)butanoate (12.9 g), and stirred at room temperature overnight. A solid was extracted by filtration, washed with diethylether, and then dried under reduced pressure, to obtain the title compound (11.8 g, yield 61%). The filtration solution was concentrated under reduced pressure, ethyl acetate was added to the residue and vigorously stirred. The produced solid was extracted by filtration, washed with ethyl acetate and diethylether, and then dried under reduced pressure, to obtain the title compound (6.63 g, yield 34%). The NMR data and ESI/MS data of the compound are given below.
¹H-NMR(400MHz,DMSO-d₆)δ(ppm): 1.13(t,J=7.1, 3H), 2.02 (pent,J=7.0, 2H), 2.22(t,J=7.4, 2H), 3.96(q,J=7.1, 2H), 4.39(t,J=6.7, 2H), 7.63(s, 1H), 7.85(s, 1H), 12.06(brs, 1H).
ESI/MS m/e: 376.0(M⁺+H, C₁₇H₁₈IN₃O₃)

### Example 13. Synthesis of ethyl 4-(4-oxo-7-(3-pyridyl)3-hydropyrrolo[3,2-d]pyrjmidin-5-yl)butanoate (Compound No. 2512)

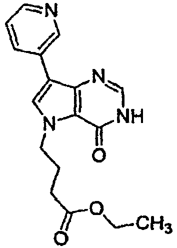

The title compound was obtained by using an ethyl 4-(7-iodo-4-oxo-3-hydropyrrolo[3,2-d]pyrimidin-5-yl)butanoate and 3-pyridyl boric acid in a similar manner to that described in Example 28. The NMR data and ESI/MS data of the compound are given below.
¹H-NMR(400MHz,DMSO-d₆)δ(ppm): 1.11(t,J=7.1, 3H), 2.09 (pent,J=7.0, 2H), 2.29(t,J=7.4, 2H), 3.96(q,j=7.1, 2H), 4.45(t,J=6.7, 2H), 7.41(m, 1H), 7.93(s, 1H), 8.06(s, 1H), 8.40(m, 2H), 9.21(m, 1H), 12.10(s, br, 1H).
ESI/MS m/e: 327.0 (M⁺+H, C₁₇H₁₈N₄O₃)

### Example 14. Synthesis of 4-(4-oxo-7-(3-pyridyl)3-hydropyrrolo[3,2-d]pyrimidin-5-yl)butanoic acid (Compound No. 2503)

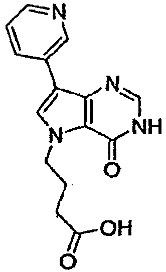

1M of aqueous lithium hydroxide solution (6.3 mL) was added to ethanol (24 mL) and water (3 mL) solution of ethyl-4-(4-oxo-7-(3-pyridyl)3-hydropyrrolo[3,2-d] pyrimidin-5-yl)butanoate (0.78 g), and stirred at room temperature overnight. The solvent was distilled off under reduced pressure, and water (5 mL) was added to the residue. 1M hydrochloric acid was added thereto to adjust pH to 4, and a produced solid was extracted by filtration, which was dried under reduced pressure, to obtain the title compound (0.75 g, quantitative yield). The NMR data and ESI/MS data of the compound are given below.
¹H-NMR(400MHz,DMSO-d₆)δ(ppm): 2.07(pent,J=7.0, 2H), 2.23(t,J=7.3, 2H), 4.46(t,J=6.7, 2H), 7.77(dd, J=4.3, J=8.2, 1H), 7.98(s, 1H), 8.24(s, 1H), 8.58(d, J=4.3, 1H), 8.79(d, J=8.2, 1H), 9.39(s, 1H), 12.12(brs, 1H), 12.24(brs, 1H).
ESI/MS m/e : 299.1(M⁺+H, C₁₅H₁₄N₄O₃)

### Example 15. Synthesis of 4-(4-oxo-7-(3-pyridyl)3-hydropyrrolo[3,2-d]pyrimidin-5-yl)-N-benzylbutanamide (Compound No. 2292)

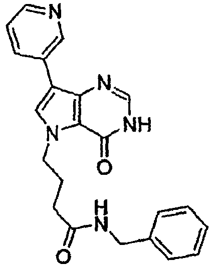

A diisopropylethylamine (91 µL) and benzyl amine (29 µL) were added to solution of N,N-dimethylformamide (4 mL) of 4-(4-oxo-7-(3-pyridyl)3-hydropyrrolo[3,2-d]pyrimidin-5-yl)butanoate (40 mg). Further, O-(7-azabenzotriazol-1-yl)
N,N,N',N'-tetramethyluronium hexafluorophosphate (74 mg) was added thereto, and the mixture solution was stirred at room temperature overnight. The solvent was distilled off under reduced pressure, and hot water was added to the residue. An insoluble portion was filtered off, the filtration solution was concentrated under reduced pressure, purified, to obtain the title compound (11. 6 mg, yield 22%). The HPLC retention time, NMR data and ESI/MS data of the compound are given below.
The HPLC retention time: 5.1(min)
¹H-NMR(400MHz,DMSO-d₆)δ(ppm) : 2.12(m, 4H), 4.23(d, J=6.0, 2H), 4.46(t,J=6.3, 2H), 7.22(m, 3H), 7.30(m, 2H), 7.73(dd, J=5.0, J=8.2, 1H), 7.98(s, 1H), 8.19(s, 1H), 8.33(t,J=6.0, 1H), 8.56(d, J=5.0, 1H), 8.74(d, J=8.2, 1H), 9.37(brs, 1H), 12.22(brs, 1H).
ESI/MS m/e: 388.1(M⁺+H, C₂₂H₂₁N₅O₂)

### Example 16. Synthesis of ethyl 4-(6-chloro-4-oxo-7-(3-pyridyl)-3-hydropyrrolo[3,2-d]pyrimidin-5-yl)butanoate (Compound No. 2504)

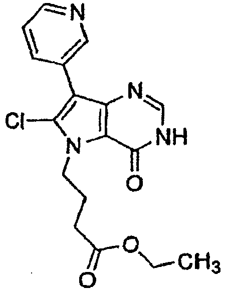

An ethyl 4-(4-oxo-7-(3-pyridyl)-3-hydropyrrolo [3,2-d]pyrimidin-5-yl)butanoate (1.72 g) was dissolved in dichloromethane (63 mL) by heating, and cooled to 0°C. 2 mol/L of surfuryl chloride/dichloromethane solution (7.9 mL) was added dropwise, and stirred at room temperature for 3 hours. The solvent was distilled off under reduced pressure, and dichloromethane and saturated aqueous sodium hydrogen carbonate solution were added, and vigorously stirred. Two layers were separated, and the aqueous layer was extracted with dichloromethane. The combined organic layer was washed with saturated brine, and dried over sodium sulfate. The sodium sulfate was filtered, and the solvent was distilled off under reduced pressure to obtain the title compound (1.89 g, quantitative yield). The NMR data and ESI/MS data of the compound are given below.
¹H-NMR(400MHz,DMSO-d₆)δ(ppm): 1.11(t,J=7.1, 3H), 2.06 (pent,J=7.0, 2H), 2.37(t,J=7.2, 2H), 3.94(q,J=7.1, 2H), 4.58(t,J=6.7, 2H), 7.51(dd, J=4.7, J=7.9, 1H), 7.93(d,J= 3.0, 1H), 8.13(dt, J=1.9, J=7.9, 1H), 8.54(d,J=7, 1H), 8.94(s, 1H), 12.28(brs, 1H).
ESI/MS m/e: 361.1(M⁺+H, C₁₇H₁₇N₄O₃)

### Example 17. Synthesis of 4-(6-chloro-4-oxo-7-(3-pyridyl)-3-hydropyrrolo[3,2-d]pyrimidin-5-yl)butanoic acid (Compound No. 2505)

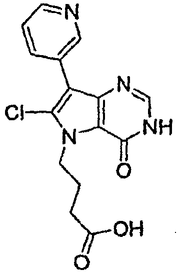

1M of aqueous lithium hydroxide solution (8.6 mL) was added to ethanol (25 mL) solution of ethyl-4-(6-chloro-4-oxo-7-(3-pyridyl)3-hydropyrrolo[3,2-d]pyrimidin-5-yl)butanoate (1.24 g), and stirred at room temperature overnight. 1M of hydrochloric acid (8.6 mL) was added thereto, the solvent was distilled off under reduced pressure, to obtain the title compound as a mixture (1.73 g) with lithium chloride. The NMR data and ESI/MS data of the compound are given below.
¹H-NMR(400MHz,DMSO-d₆)δ(ppm): 2.02(m, 2H), 2.29(t,J=7.4, 2H), 4.57(t,J=6.9, 2H), 7.58(ddd,J=0.8, J=4.9, J=8.0, 1H), 7.94(s, 1H), 8.23(ddd,J=1.5, J=2.3, J=8.0, 1H), 8.57(dd,J=1.5, J=4.9, 1H), 8.98(dd,J=0.8, J=2.3, 1H), 12.16(brs, 1H), 12.39(brs, 1H).
ESI/MS m/e: 333.1(M⁺+H, C₁₅H₁₃ClN₄O₃ LiCl)

### Example 18. Synthesis of 4-(6-chloro-4-oxo-7-(3-pyridyl)-3-hydropyrrolo[3,2-d]pyrimidin-5-yl)-N-(cyclohexylmethyl)butanamide (Compound No. 2264)

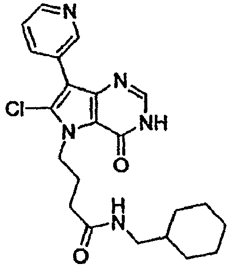

A cyclohexanemethylamine (27 mg) and diisopropylethylamine (84 µL) were added to a solution of N,N-dimethylformamide (1 mL) of 4-(6-chloro-4-oxo-7-(3-pyridyl)3-hydropyrrolo[3,2-d]pyrimidin-5-yl)butanoate (40 mg). Further, O-(7-azabenzotriazol-1-yl)N,N,N',N'-tetramethyluronium hexafluorophosphate (55 mg) was added thereto, and the mixture solution was stirred at room temperature for 2 hours. The reaction mixture was separated to ethyl acetate and aqueous layers. The organic layer was washed with 1 mol/L of aqueous citric acid solution, saturated aqueous sodium hydrogen carbonate, and saturated brine, which was dried over sodium sulfate. The sodium sulfate was filtered off, and the solvent was distilled off under reduced pressure. The residue was purified by column chromatography on silica gel (5% methanol/ethyl acetate) to obtain the title compound (35 mg, yield 68%). The HPLC retention time, NMR data and ESI/MS data of the compound are given below.
The HPLC retention time: 7.2(min)
¹H-NMR(400MHz,CD₃OD)δ(ppm): 0.09(m, 2H), 1.21(m, 3H), 1.42(m, 1H), 1.69(m, 5H), 2.16(m, 2H), 2.29(t,H=7.4, 2H), 2.95(d,J=7.0, 2H), 4.66(t,J=6.9, 2H), 7.53(ddd,J=0.9, J=5.0, J=8.0, 1H), 7.89(s, 1H), 8.22(ddd,J=1.6, J=2.2, J=8.0, 1H), 8.49(dd,J=1.6, J=5.0, 1H), 8.93(dd,J=0.9, J=2.2, 1H).
ESI/MS m/e: 428.2 (M⁺+H, C₂₂H₂₆ClN₅O₂)

### Example 19. Synthesis of 4-[6-(3,4-dimethoxyphenyl)-4-oxo-7-(3-pyridyl) (3-hydropyrrolo[3,2-d]pyrimidin-5-yl)]-N-(cyclohexylmethyl)butanamide (Compound No. 2269)

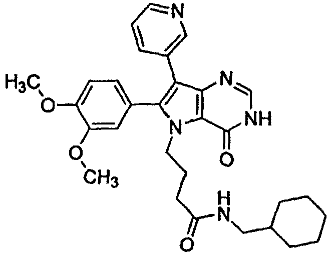

An N,N-dimethylformamide/water (2:1) solution (2 mL) of 4-(6-chloro-4-oxo-7-(3-pyridyl)(3-hydropyrrolo[3,2-d] pyrimidin-5-yl))-N-(cyclohexylmethyl)butanamide (50 mg), 3,4-dimethoxyphenyl boric acid (64 mg), and potassium acetate (57 mg) was subjected to deaeration, followed by flushing with nitrogen gas. A small amount of [1,1'-bis(diphenylphosphino)
ferrocene]palladium (II) chloride dichloro-methane (1:1) complex body was added thereto. The reaction mixture was heated at 140°C for 30 minutes by using microwave. The solvent was distilled off under reduced pressure and the residue was purified by column chromatography on silica gel (5-8% methanol/ethyl acetate), and further purified by fraction HPLC to obtain the title compound (12 mg, yield 16%). The HPLC retention time, NMR data and ESI/MS data of the compound are given below.
HPLC retention time: 8.0 (min)
¹H-NMR(400MHz,CD₃OD)δ(ppm): 0.85(m,2H), 1.19(m, 3H), 1.35(m, 1H), 1.66(m, 5H), 2.01(m, 2H), 2.13(t, J=7.4, 2H), 2.88(d, J=6.8, 2H), 3.80(s, 3H), 3.91(s, 3H), 4.42(t,J=7.5, 2H), 7.00(dd, J=2.0, J=8.1, 1H), 7.03(d,J=2.0, 1H), 7.13(d,J=8.1, 1H), 7.81(dd, J=5.6, J=8.2, 1H), 7.99(s, 1H), 8.39(ddd,J=1.4, J=2.1, J=8.2, 1H), 8.52 (d, J=5.6, 1H), 8.90(d, J=2.1, 1H).
ESI/MS m/e: 530.3(M⁺+H, C₃₀H₃₅N₅O₄)

### Reference Example 49. Synthesis of ethyl 4-(7-iodo-4-oxo-3-[(phenylmethoxy)methyl]-3-hydropyrrolo[3,2-d]pyrimidin-5-yl)butanoate

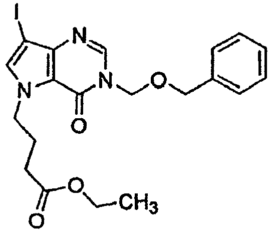

A sodium hydride (521 mg) was added to solution of N,N-dimethylformamide (60 mL) of ethyl 4-(7-iodo -4-oxo-3-hydropyrrolo[3,2-d]pyrimidin-5-yl)butanoate (4.07 g) in a small amount by each, and the mixture solution was stirred at room temperature for 30 minutes. A benzylchloromethylether (2.04 g) was added dropwise, and the mixture solution was further stirred for 2 hours. The solvent was distilled off under reduced pressure to separate the residue to ethyl acetate and aqueous layers. The organic layer was washed with water and saturated brine, and dried over sodium sulfate. The sodium sulfate was filtered off, and the solvent was distilled off under reduced pressure. The residue was purified by column chromatography on silica gel (15-30% ethyl acetate/cyclohexane), to obtain the title compound (3.80 g, yield 71%). The NMR data and ESI/MS data of the compound are given below.
¹H-NMR(400MHz, CDCl₃)δ(ppm): 1.25(t,J=7.1, 3H), 2.18(pent,J=7.0, 2H), 2.32(t,J=7.2, 2H), 4.12(q,J=7.1, 2H), 4.51(t,J=6.9, 2H), 4.65(s, 2H), 5.50(s, 2H), 7.22(s, 1H), 7.29(m, 1H), 7.33(m, 4H), 8.03(s, 1H).
ESI/MS m/e: 496.1(M⁺+H, C₂₀H₂₂lN₃O₄)

### Example 20. Synthesis of ethyl-4-{7-(1-methylpyrazol-4-yl)-4-oxo-3-[(phenylmethoxy)methyl]-3-hydropyrrolo[3,2-d] pyrimidin-5-yl)butanoate

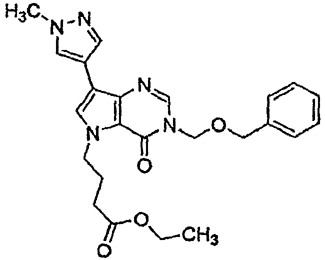

The title compound was obtained by using ethyl 4-{7-iodo-4-oxo-3-[(phenylmethoxy)methyl]-3-hydropyrrolo[3,2-d]
pyrimidin-5-yl)butanoate and 1-methyl-4-(4,4,5,5-tetramethyl-1,3,2-dioxaborolane-2-yl)-1H-pyrazol in a similar manner to that in Example 28. The NMR data and ESI/MS data of the compound are given below. ¹H-NMR(400MHz,CD₃OD)δ(ppm): 1.16(t,J=7.1, 3H), 2.16(pent,J=7.0, 2H), 2.33(t,J=7.2, 2H), 3.92(s,3H), 3.02(q,J=7.1, 2H), 4.49(t,J=6.7, 2H), 4.68(s, 2H), 5.56(s, 2H), 7.22(m, 1H), 7.28(m, 2H), 7.33(m, 2H), 7.54(s, 1H), 7.86(d,J=0.6, 1H), 8.03(s, 1H), 8.10(s, 1H).
ESI/MS m/e: 450.3(M⁺+H, C₂₄H₂₇N₅O₄)

### Example 21. Synthesis of 4-[6-chloro-7-(1-methylpyrazol-9-yl)-4-oxo-3-hydropyrrolo[3,2-d]pyrimidin-5-yl]butanoic acid (Compound No. 2506)

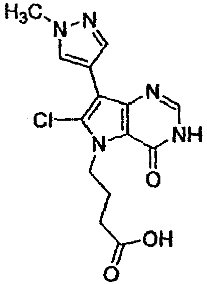

Solution of concentrated hydrochloric acid (3 mL) of ethyl-4-{6-chloro-7-(1-methylpyrazol-4-yl)-4-oxo-3-[(phenylmethoxy)methyl]-3-hydropyrrolo[3,2-d]pyrimidin-5-yl)
butanoate was heated for 1 hour to reflux. The solvent was distilled off under reduced pressure, and methanol solution (3 mL) of 2 mol/L ammonia was added to the residue. The solvent was distilled off under reduced pressure, and 1 mol/L of aqueous citric acid solution was added to the residue. A produced solid was extracted by filtration, washed with water and diethylether, and then dried under reduced pressure, to obtain the title compound (133 mg, yield 87%). The NMR data and ESI/MS data of the compound are given below.
¹H-NMR(400MHz,DMSO-d₆)δ(ppm): 1.97(pent,J=7.0, 2H), 2.23(t,J=7.4, 2H), 3.91(s,3H), 4.50(t,J=6.8, 3H), 7.91(d,J=3.6, 1H), 8.04(s, 1H), 8.24(s, 1H), 12.11(s, 1H), 12.17(d,J=3.6, 1H).
ESI/MS m/e: 336.2(M⁺+H, C₁₄H₁₄ClN₅O₃)

### Example 22. Synthesis of 4-[6-chloro-7-(1-methylpyrazol-4-yl)-4-oxo-3-hydropyrrolo[3,2-d]pyrimidin-5-yl]]-N-[(4-methylphenyl)methyl]butanamide (Compound No. 2507)

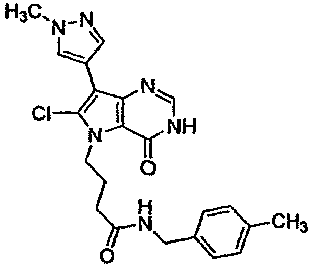

A 4-methylbenzyl amine (29 mg) and a diisopropylethylamine (84 µm) were added to a solution of an N,N-dimethylformamide (1 mL) of 4-[6-chloro-7-(1-methylpyrazol-4-yl)-4-oxo-3-hydropyrrolo[3,2-d]pyrimidin-5-yl)-butanoate (40 mg). An O-(7-azabenzotriazol-1-yl)-N,N,N',N'-tetramethyluronium hexafluorophosphate (55 mg) was further added, and the mixture solution was stirred at room temperature overnight. An ethyl acetate and 1 mol/L of an aqueous citric acid solution were added to the reaction mixture, and separated to two layers. The organic layer was washed with saturated aqueous sodium hydrogen carbonate solution and saturated brine, and dried over sodium sulfate. The sodium sulfate was filtered off, and the solvent was distilled off under reduced pressure, to obtain the title compound (67 mg, quantitative yield). The compound was not further purified, but used for the following reaction. The ESI/MS data of the compound is given below.
ESI/MS m/e: 439.2(M⁺+H, C₂₂H₂₃ClN₆O₂)

### Example 23. Synthesis of 4-[6-(3,4-dimethoxyphenyl)-7-(1-methylpyrazol-4-yl)-4-oxo-7-(3-hydropyrrolo [3,2-d]pyrimidin-5-yl)]-N-[(4-methylphenyl)methyl] butanamide (Compound No. 2345)

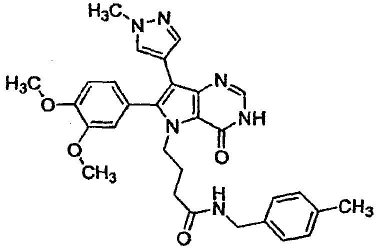

An N,N-dimethylformamide/water (2:1) solution (2 mL) of 4-[6-chloro-7-(1-methylpyrazol-4-yl)-4-oxo (3-hydropyrrolo[3,2-d]pyrimidin-5-yl)]-N-[(4-methylphenyl) methyl] butanamide (65 mg), 3,4-dimethoxyphenyl boric acid (81 mg), and potassium acetate (73 mg) was subjected to deaeration, followed by flushing with nitrogen gas. A small amount of [1,1'-bis(diphenylphosphino)ferrocene]palladium (II) chloride dichloro-methane (1:1) complex body was added thereto. The reaction mixture was heated at 160°C for 45 minutes by using microwaves. The solvent was distilled off under reduced pressure and the residue was purified by column chromatography on silica gel (5-8% methanol/ethyl acetate), and further purified by fraction HPLC to obtain the title compound (4.4 mg, yield 7%). The HPLC retention time, NMR data and ESI/MS data of the compound are given below.
HPLC retention time: 8.2 (min)
¹H-NMR(400MHz, CD₃OD)δ(ppm) : 1.98(m,2H), 2.13(t,J=7.5, 2H), 2.29(2, 3H), 3.79(s, 3H), 3.82(s, 3H), 3.91(s, 3H), 4.18(s, 2H), 4.36(t,J=7.4, 2H), 6.98(m, 2H), 7.07(m, 4H), 7.12(d,J=7.9, 1H), 7.28(2, 1H), 7.66(s, 1H), 7.97(2, 1H).
ESI/MS m/e: 541.1(M⁺+H, C₃₀H₃₂N₆O₄)

### Reference Example 50. Synthesis of ethyl-4-(6-cyclopropyl-4-oxo-3-hydropyrrolo[3,2-d]pyrimidin-5-yl)butanoate (Compound No. 2508)

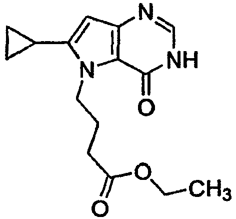

The title compound was obtained by using ethyl-4-[3-amino-5-cyclopropyl-2-(ethoxycarbonyl)pyrolyl] butanoate hydrochloride in a similar manner to that in Example 45. The NMR data and ESI/MS data of the compound are given below.
¹H-NMR(400MHz,DMSO-d₆)δ(ppm): 0.74(m, 2H), 1.00(m, 2H), 1.13(t,J=7.2, 3H), 1.93-2.06(m, 3H), 2.31(t,J=7.4, 2H), 3.98(dd,J=7.2, J=14.2, 2H), 4.52(t,J=7.0, 2H), 6.00(s, 1H), 7.71(s, 1H), 11.76(brs, 1H).
ESI/MS m/e: 290.2(M⁺+H, C₁₅H₁₉N₃O₃)

### Reference Example 51. Synthesis of ethyl-4-(6-cyclopropyl-7-iodo-4-oxo-3-hydropyrrolo[3,2-d]pyrimidin-5-yl)butanoate (Compound No. 2509)

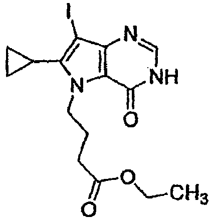

A dichloromethane (40 mL) solution of an ethyl-4-(6-cyclopropyl-4-oxo-3-hydropyrrolo[3,2-d]pyrimidin-5-yl) butanoate (1.58 g) and an N-iodosuccinimide (1.35 g) was stirred at room temperature for 2 hours. Water was added, and the mixture solution was further stirred for 30 minutes. A solid was extracted by filtration, washed by water and ethyl acetate, and dried under reduced pressure, to obtain the title compound (1.82 g, yield 80%). The filtration solution was separated to ethyl acetate and aqueous layers, and the aqueous layer was extracted with ethyl acetate. The combined organic layer was washed with saturated brine, and dried over a magnesium sulfate. The magnesium sulfate was filtered off, and the solvent was distilled off under reduced pressure. A produced solid was extracted by filtration, washed with ethyl acetate, and dried under reduced pressure, to obtain the title compound (0.35 mg, yield 15%). The NMR data and ESI/MS data of the compound are given below.
1H-NMR(400MHz,DMSO-d₆)δ(ppm): 0.89(m, 2H), 1.08-1.15(m, 5H), 1.80(m, 1H), 2.01(m, 2H), 2.31(t,J=7.3, 2H), 3.96(dd,J=7.0, J=14.3, 2H), 4.57(t,J=7.2, 2H), 7.82(s, 1H), 12.00(brs, H).
ESI/MS m/e: 416.1(M⁺+H, C₁₅H₁₈IN₃O₃)

### Example 24. Synthesis of ethyl-4-(6-cyclopropyl-4-oxo-7-(3-pyridyl)-3-hydropyrrolo[3,2-d]pyrimidin-5-yl)butanoate (Compound No. 2510)

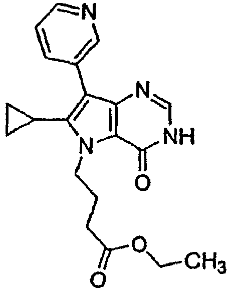

The title compound was obtained by using ethyl-4-(6-cyclopropyl-7-iodo-4-oxo-3-hydropyrrolo[3,2-d]pyrimidin-5-yl) butanoate and 3-pyridyl boric acid in a similar manner to that in Example 28. The NMR data and ESI/MS data of the compound are given below.
¹H-NMR(400MHz,DMSO-d₆)δ(ppm): 0.31(m, 2H), 1.01(m, 2H), 1.13(t,J=7.2, 3H), 2.06-2.13(m, 3H), 2.40(t,J=7.3, 2H), 3.98(dd,J=7.2, J=14.2, 2H), 4.61(t,J=7.2, 2H), 7.43(ddd,J=0_{.}8, J=4.8, J=7.9, 1H), 7.82(s, 1H), 8.01(dt,J=2.0, J=8.1,1H), 8.46(dd,J=1.7, J=4.7, 1H) , 8.83(dd,J=0.8, J=2.3, 1H), 11.99(brs, 1H).
ESI/MS m/e: 367.2(M⁺+H, C₂₀H₂₂N₄O₃)

### Example 25. Synthesis of 4-(6-cyclopropyl-4-oxo-7-(3-pyridyl)-3-hydropyrrolo[3,2-d]pyrimidin-5-yl)butanoic acid (Compound No. 2363) hydrochloride

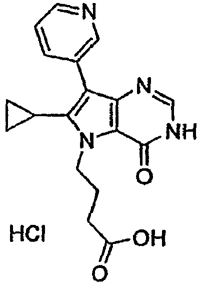

1M of aqueous lithium hydroxide solution (7.3 mL) was added to dioxane (25 mL) and water (10 mL) solution of ethyl-4-(6-cyclopropyl-4-oxo-7-(3-pyridyl)-3-hydropyrrolo
[3,2-d]pyrimidin-5-yl) butanoate (1.06 g), and stirred at room temperature for 3 hours. 1M hydrochloric acid (10.2 mL) was added dropwise, and the solvent was distilled off under reduced pressure. An ethanol was added to a
residue, a solid was extracted by filtration, washed with ethanol, and dried under reduced pressure, to obtain the title compound (1.10 g, quantitative yield). The NMR data and ESI/MS data of the compound are given below. ¹H-NMR(400MHz,DMSO-d₆)δ(ppm): 0.39(m, 2H), 1.10(m, 2H), 2.07(m, 2H), 2.18(m, 1H), 2.33(t,J=7.4, 2H), 4.63(t,J=7.3, 2H), 7.95(s, 1H), 8.10(dd,J=5.7, J=8.1, 1H), 8.82(m, 2H), 9.16(d,J=1.8, 1H), 12.26(brs, 1H).
ESI/MS m/e: 339.0(M⁺+H, C₁₈H₁₈N₄O₃HCl)

### Example 26. Synthesis of 4-(6-cyclopropyl-4-oxo-7-(3-pyridyl)(3-hydropyrrolo[3,2-d]pyrimidin-5-yl))-N-[(4-methylphenyl)methyl]butanamide (Compound No. 2331)

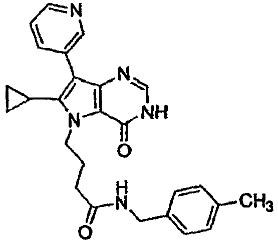

A diisopropylethylamine (70 µL) and a 4-methylbenzyl amine (15 mg) were added to solution of N,N-dimethylformamide (1.5 mL) of 4-(6-cyclopropyl-4-oxo-7-(3-pyridyl)-3-hydropyrrolo[3,2-d]pyrimidin-5-yl)butanoate (30 mg) and O-(7-azabenzotriazol-1-yl)N,N,N',N'-tetramethyluronium hexafluorophosphate (46 mg). The reaction solution was stirred at room temperature for 4 hours. The solvent was distilled off under reduced pressure, and ethyl acetate and water were added to a residue, separated to two layers, and an aqueous layer was extracted with ethyl acetate. The combined organic layer was dried over a sodium sulfate. The sodium sulfate was filtered off, and the solvent was distilled off under reduced pressure. The residue was purified by fraction HPLC, to obtain the title compound (3.1 mg, yield 9%). The HPLC retention time, NMR data and ESI/MS data of the compound are given below.
The HPLC retention time: 7.3(min)
¹H-NMR(400MHz,DMSO-d₆)δ(ppm): 0.27(m, 2H), 0.93(m, 2H), 2.01-2.11(m, 3H), 2.21-2.27(m, 5H), 4.21(d,J=5.9, 2H), 4.58(t,J=7.1, 2H), 7.10-7.15(m, 4H), 7.43(ddd,J=0.8, J=4.8, J=7.9, 1H), 7.82(s, 1H), 8.01(dt,J=2.0, J=8.1, 1H), 8.34(t,J=5.9, 1H), 8.46(dd,J=1.8, J=9.8, 1H), 8.84(dd,J=0.8, J=2.3, 1H).
ESI/MS m/e: 442.2(M⁺+H, C₂₆H₂₇N₅O₂)

### Example 27. Synthesis of ethyl-4-[6-cyclopropyl-7-(1-methylpyrazol-4-yl)-4-oxo-3-hydropyrrolo[3,2-d]pyrimidin-5-yl)butanoate (Compound No. 2511)

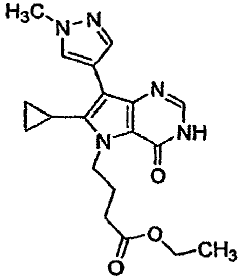

The title compound was obtained by using ethyl-4-(6-cyclopropyl-7-iodo-4-oxo-3-hydropyrrolo[3,2-d] pyrimidin-5-yl)butanoate and 1-methyl-4-(4,4,5,5-tetramethyl-1,3,2-dioxaborolane-2-yl)-1H-pyrazol in a similar manner to that in Example 28. The NMR data and ESI/MS data of the compound are given below. ¹H-NMR(400MHz,DMSO-d₆)δ(ppm): 0.51(m, 2H), 1.13(m, 5H), 1.91(m, 1H), 2.03(m, 2H), 2.31(t,J=7.3, 2H), 3.89(s, 3H), 3.97(dd,J=7.2, J=14.2, 2H), 4.58(t,J=7.0, 2H), 7.80(d,J=3.3, 1H), 7.83(d,J=0.7, 1H), 8.03(s, 1H), 11. 87 (brs, 1H).
ESI/MS m/e: 370.2(M⁺+H, C₁₉H₂₃N₅O₃)]

### Example 28. Synthesis of 4-[6-cyclopropyl-7-(1-methylpyrazol-4-yl)-4-oxo-3-hydropyrrolo[3,2-d]pyrimidin-5-yl]butanoic acid (Compound No. 2364)

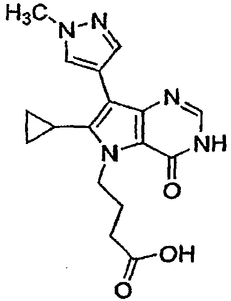

1M of aqueous lithium hydroxide solution (3.5 mL) was added to dioxane/water (4/1) solution (25 mL) of ethyl-4-[6-cyclopropyl-7-(1-methylpyrazol-4-yl)- 4-oxo-3-hydropyrrolo[3,2-d]pyrimidin-5-yl)butanoate (0.52 g) dropwise. The reaction solution was stirred at room temperature for 4 hours, and 1M hydrochloric acid (3.5 mL) was added thereto dropwise. The solvent was distilled off under reduced pressure, a residue was purified by column chromatography on silica gel (dichloromethane/methanol/acetate/water=240/30/3/2), a diethylether was added to the obtained solid, and finely pulverized. The solid was extracted by filtration, washed with diethylether, and dried under reduced pressure, to obtain the title compound (0.40 g, yield 84%). The NMR data and ESI/MS data of the compound are given below.
¹H-NMR(400MHz,DMSO-d₅)δ(ppm): 0.51(m,2H), 1.12 (m,2H), 1.88-2.02(m,3H), 2.23(t,J=7.4,2H), 3.89(s,3H), 4.57(t,J-7.2,2H), 7.80(d,J=3.5,1H), 7·83(d,J=0.7,1H), 8.03(s,1H), 11.87(brs,1H).
ESI/MS m/e: 342.2 (M⁺+H, C₁₁H₁₉N₅O₃)

### Example 29. Synthesis of 4-[6-cyclopropyl-7-(1-methylpyrazol-4-yl)-4-oxo-(3-hydropyrrolo[3,2-d]pyrimidin-5-yl)]-N-[(4-methylphenyl)methyl]butanamide (Compound No. 2332)

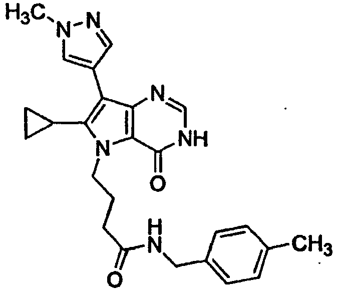

A diisopropylethylamine (78 µL) and a 4-methylbenzyl amine (13 mg) were added to a solution of N,N-dimethylformamide (1.0 mL) of 4-[6-cyclopropyl-7-(1-methylpyrazol-4-yl)-4-oxo-3-hydropyrrolo[3,2-d]pyrimidin-5-yl]butanoate (30 mg) and O-(7-azabenzotriazol-1-yl)N,N,N',N'-tetramethyluronium hexafluorophosphate (40 mg). The reaction solution was stirred at room temperature overnight. The solvent was distilled off under reduced pressure, a residue was purified by fraction HPLC, to obtain the title compound (30 mg, yield 77%). The HPLC retention time and ESI/MS data of the compound are given below.
The HPLC retention time: 8.0(min)
ESI/MS m/e: 445.3(M⁺+H, C₂₅H₂₈N₆O₂)

### Reference Example 52. Synthesis of 5-benzyl-7-(1-oxy(3-pyridyl))-3-hydropyrrolo[3,2-d]pyrimidin-4-one (Compound No. 2499)

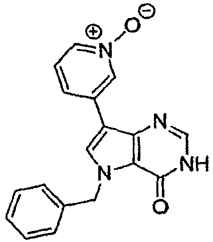

30% aqueous hydrogen peroxide solution (92 mg) was added to an acetic acid (2 mL) solution of 5-benzyl-7-(3-pyridyl)- 3-hydropyrrolo[3,2-d]pyrimidin-4-one (31 mg), and the solution mixture was stirred at room temperature overnight. The reaction mixture was cooled to room temperature, purified by fraction HPLC, to obtain the title compound (19 mg, yield 58%). The HPLC retention time, NMR data and ESI/MS data of the compound are given below.
The HPLC retention time: 5.8(min)
¹H-NMR(400MHz,DMSO-d₆)δ(ppm): 5.64(s,2H), 7.24-7.35(m,5H), 7.46(m,1H), 8.00(m,2H), 8.10(m,1H), 8.39(s,1H), 9.11(s,1H), 12.25(brs,1H).
ESI/MS m/e: 319.3(M⁺+H, C₁₈H₁₄N₄O₂)

### Example 30. Synthesis of (t-butoxy)-N-{2-[6-(3-chloro(2-thienyl)-7-(5-methyl(1,2,4-oxadiazol-3-yl)-4-oxo-(3-hydropyrrolo[3,2-d]pyrimidin-5-yl)]ethyl}carboxyamide (Compound No. 2126)

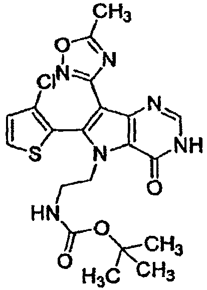

A (t-butoxy)-N-{2-[6-(3-chloro(2-thienyl))-7-cyano-4-oxo(3-hydropyrrolo[3,2-d]pyrimidin-5-yl)]ethyl) carboxyamide (105 mg) was dissolved in ethanol (2.5 mL), 35 mg of a hydroxylamine hydrochloride and a diisopropylethylamine (100 mg) were added thereto, and the solution mixture was stirred overnight under overheat reflux. The solvent was distilled off under reduced pressure, a dichloromethane (2.5 mL) and water (2.5 mL) were added thereto, and an organic layer was fractionally extracted. An aqueous layer was further extracted with dichloromethane (2.5 mL), an organic layer was combined, dried over sodium sulfate, and the solvent was distilled off under reduced pressure, to obtain a residue (113 mg). This residue was dissolved in dichloromethane (2 mL), acetate (18 mg), 1-ethyl-3-(3'-dimethylaminopropyl) carbodiimide hydrochloride (57 mg), and triethylamine (41 µL) were added thereto, and the solution mixture was stirred at 40°C for 1 hour. The reaction solution was concentrated, and purified by fraction HPLC, to obtain the title compound (7.2 mg, yield 6%). The HPLC retention time and ESI/MS data of the compound are given below.
The HPLC retention time: 9.5(min)
ESI/MS m/e: 476.9(M⁺+H, C₂₀H₂₁ClN₆O₄S)

### Example 31. Synthesis of (t-butoxy)-N-{2-[6-(3-chloro(2-thienyl)-7-(1,2,4-oxadiazol-3-yl)-4-oxo-(3-hydropyrrolo [3,2-d]pyrimidin-5-yl)]ethyl}carboxyamide (Compound No. 2513)

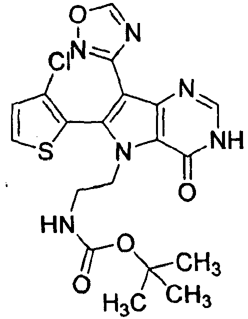

A (t-butoxy)-N-{2-[6-(3-chloro(2-thienyl))-7- cyano-4-oxo(3-hydropyrrolo[3,2-d]pyrimidin-5-yl)]ethyl} carboxyamide (105 mg) was dissolved in ethanol (2.5 mL), 35 mg of a hydroxylamine hydrochloride and a diisopropylethylamine (100 mg) were added thereto, and the solution mixture was stirred overnight under overheat reflux. The solvent was distilled off under reduced pressure, a dichloromethane (2.5 mL) and water (2.5 mL) were added thereto, and an organic layer was fractionally extracted. An aqueous layer was further extracted with dichloromethane (2.5 mL), an organic layer was combined, dried over sodium sulfate, and the solvent was distilled off under reduced pressure, to obtain a residue (113 mg). Meanwhile N,N-dimethylformamide (40 mg) was dissolved in diethylether (1 mL), phosphorus oxychloride (84 mg) was added thereto, and the solution mixture was stirred at room temperature for 1 hour, to obtain an oily compound. The produced oily compound was washed with diethylether (1 mL) twice, and suspended to a solvent mixture (3 mL) of 1,4-dioxane and diethylether. This suspension was added to the residue, and stirred at room temperature for 2 hours. The reaction solution was concentrated, water (5 mL) and ethyl acetate (5 mL) were added thereto, and an organic layer was separated. An aqueous layer was further extracted with ethyl acetate (5 mL), an organic layer was combined and concentrated, and then purified by fraction HPLC, to obtain the title compound (1.9 mg, yield 1.6%). The HPLC retention time and ESI/MS data of the compound are given below.
HPLC retention time: 9.1(min)
ESI/MS m/e: 462.9(M⁺+H, C₁₉H₁₉ClN₆O₄S)

### Example 32. Synthesis of (t-butoxy)-N-{2-[6-(3-hydroxyphenyl) -4-oxo-7-(3-pyridyl)(3-hydropyrrolo[3,2-d]pyrimidin-5-yl)]ethyl}carboxyamide (Compound No. 2121)

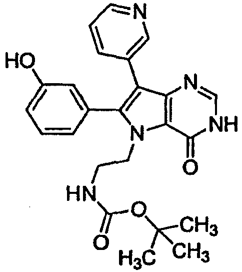

A t-butoxy)-N-(2-{4-oxo-6-[3-(phenylmethoxy)phenyl]-7-(3-pyridyl)(3-hydropyrrolo[3,2-d]pyrimidin-5-yl)}ethyl} carboxyamide (8.0 mg) was dissolved in ethanol, and palladium carbon (20 mg) was added thereto. The reaction system was substituted by hydrogen, and stirred at 60°C overnight under atmospheric hydrogen pressure. The reaction solution was cooled to room temperature, and the palladium carbon was filtered off. The filtration solution was concentrated, purified by fraction HPLC, to obtain the title compound (4.0 mg, yield 60%). The HPLC retention time and ESI/MS data of the compound are given below.
HPLC retention time: 6.3(min)
ESI/MS m/e: 448.0(M⁺+H, C₂₄H₂₅N₅O₄)

### Examples 33 to 415

The following compounds of the present invention were synthesized according to any method of Example 1 to 32 or Reference Example 15 to 52 by using corresponding starting materials and reaction, agents. The ESI/MS data in the HPLC/mass reaction agents. The ESI/MS data in the HPLC/mass spectrum analysis, the retention time of the compound in the HPLC analysis and purity under the following analysis conditions of each compound, and compound numbers corresponding to executed syntheses are summarized in Table 2. Compound numbers in the table represent compound numbers of Table 1 listed as the preferred specific examples.

**Table 2**

| Ex. no. | Com-pound no. | Formula | ESI/MS m/e | HPLC min | Purity (%) | Synthesis |
|---|---|---|---|---|---|---|
| 33 | 19 | C₁₉ H₂₁ N₅ O₂ | 352.2 | 5.7 | 96 | Ex. 12 |
| 34 | 27 | C₂₆ H₂₅ N₅ O₃ | 456.3 | 7.8 | 99 | Ex. 12 |
| 35 | 29 | C₁₇ H₁₇ N₅ O₂ | 324.2 | 3.3 | 99 | Ex. 12 |
| 36 | 40 | C₂₁ H₁₅ Cl₂ N₇ O S | 484.2 | 7.7 | 98 | Ex. 10 |
| ref 53 | 80 | C₂₀ H₁₄ Br Cl N₆ O S | 503.2 | 6.8 | 100 | Ex. 10 |
| 37 | 83 | C₂₆ H₁₉ Cl N₆ S₂ | 515.4 | 10.4 | 96 | Ex. 10 |
| 38 | 212 | C₁₉ H₁₀ Cl N₅ O S | 395.4 | 11.7 | 86 | Ex. 1 |
| 39 | 219 | C₂₀ H₁₈ Cl N₅ O₂ S | 428.0 | 5.6 | 95 | Ex. 7 |
| 40 | 230 | C₂₁ H₂₀ Cl N₅ O₂ S | 442.4 | 6.5 | 100 | Ex. 9 |
| 41 | 245 | C₂₁ H₂₀ Cl N₅ O₂ S | 442.0 | 6.1 | 94 | Ex. 7 |
| 42 | 254 | C₂₂ H₂₂ Cl N₅ O₂ S | 456.1 | 6.9 | 95 | Ex. 7 |
| 43 | 257 | C₂₁ H₂₃ N₅ O₂ | 378.2 | 4.3 | 100 | Ex. 8 |
| 44 | 263 | C₂₀ H₂₁ N₅ O₂ | 364.2 | 10.0 | 96 | Ex. 7 |
| 45 | 269 | C₂₁ H₁₈ Cl N₅ O₂ S | 440.4 | 6.2 | 100 | Ex. 9 |
| 46 | 280 | C₂₂ H₂₅ N₅ O₂ | 392.2 | 5.4 | 100 | Ex. 7 |
| 47 | 287 | C₂₃ H₂₂ Cl N₅ O₂ S | 468.0 | 7.1 | 95 | Ex. 7 |
| 48 | 288 | C₂₂ H₂₁ Cl N₄ O₂ S₂ | 472.9 | 11.8 | 97 | Ex. 7 |
| 49 | 297 | C₂₃ H₂₇ N₅ O₂ | 406.2 | 12.9 | 98 | Ex. 7 |
| 50 | 303 | C₂₄ H₂₄ Cl N₅ O₂ S | 482.4 | 8.8 | 100 | Ex. 9 |
| 51 | 321 | C₁₉ H₁₃ Cl F₃ N₅ O₂ S | 468.4 | 7.3 | 99 | Ex. 9 |
| 52 | 330 | C₂₃ H₂₁ N₅ O₂ | 400.5 | 5.0 | 99 | Ex. 7 |
| 53 | 347 | C₂₅ H₂₈ Cl F N₄ O S₂ | 509.4 | 15.0 | 99 | Ex. 9 |
| 54 | 348 | C₂₄ H₁₇ Cl F N₅ O₂ S | 494.2 | 8.0 | 100 | Ex. 9 |
| 55 | 361 | C₂₆ H₂₀ Cl N₅ O₄ S | 534.0 | 9.4 | 78 | Ex. 7 |
| 56 | 370 | C₂₀ H₁₃ Br Cl F₃ N₄ O₃ S | 563.1 | 10.4 | 100 | Ex. 7 |
| 57 | 492 | C₂₃ H₂₃ Cl N₆ O₂ S | 483.1 | 10.8 | 93 | Ex. 5 |
| 58 | 504 | C₂₄ H₂₅ Cl N₆ O₂ S | 497.5 | 9.6 | 84 | Ex. 5 |
| 59 | 536 | C₃₀ H₂₇ Cl N₆ O₃ S | 587.4 | 9.0 | 79 | Ex. 5 |
| 60 | 595 | C₂₁ H₂₅ N₅ O₂ | 380.2 | 5.2 | 100 | Ex. 7 |
| 61 | 596 | C₂₂ H₂₂ Cl N₅ O₂ S | 456.1 | 6.9 | 98 | Ex. 7 |
| 62 | 601 | C₂₁ H₂₅ N₅ O₂ | 380.2 | 5.3 | 100 | Ex. 7 |
| 63 | 602 | C₂₂ H₂₂ Cl N₅ O₂ S | 456.1 | 7.1 | 94 | Ex. 7 |
| 64 | 605 | C₂₂ H₂₉ N₅ O₂ | 396.3 | 5.1 | 100 | Ex. 7 |
| 65 | 615 | C₂₂ H₂₇ N₅ O₂ | 394.1 | 7.5 | 99 | Ex. 7 |
| 66 | 616 | C₂₁ H₂₆ N₄ O₃ | 383.2 | 10.3 | 78 | Ex. 1 |
| 67 | 617 | C₂₁ H₂₆ N₄ O₂ S | 399.1 | 11.6 | 87 | Ex. 7 |
| 68 | 618 | C₂₀ H₂₆ N₆ O₂ | 383.2 | 8.1 | 60 | Ex. 1 |
| 69 | 627 | C₂₂ H₂₃ Cl N₄ O₃ S | 459.3 | 15.0 | 95 | Ex. 7 |
| 70 | 628 | C₂₂ H₂₃ Cl N₄ O₂ S₂ | 475.2 | 15.7 | 100 | Ex. 7 |
| 71 | 634 | C₂₂ H₂₇ N₅ O₂ | 394.2 | 5.7 | 100 | Ex. 19 |
| 72 | 641 | C₂₃ H₂₇ N₅ O₂ | 406.2 | 5.9 | 100 | Ex. 7 |
| 73 | 646 | C₂₄ H₂₄ Cl N₅ O₂ S | 482.0 | 6.9 | 95 | Ex. 7 |
| 74 | 657 | C₂₁ H₂₃ N₅ O₂ | 378.3 | 4.3 | 97 | Ex. 7 |
| 75 | 659 | C₂₂ H₂₀ Cl N₅ O₂ S | 454.1 | 6.5 | 94 | Ex. 7 |
| 76 | 664 | C₂₃ H₂₄ Cl N₅ O₂ S | 470.2 | 9.1 | 99 | Ex. 7 |
| 77 | 667 | C₂₄ H₂₉ N₅ O₂ | 420.2 | 6.3 | 100 | Ex. 8 |
| 78 | 668 | C₂₅ H₂₆ Cl N₅ O₂ S | 496.0 | 8.3 8.5 | 80 | Ex. 8 |
| 78 | 668 | C₂₅ H₂₆ Cl N₅ O₂ S | 496.0 | 8.3 | 89 | Ex. 8 |
| 79 | 673 | C₂₅ H₂₆ Cl N₅ O₃ S | 512.0 | 6.6 | 98 | Ex. 8 |
| | | | | 6.9 | | |
| 80 | 678 | C₂₈ H₃₂ Cl N₅ O₂ S | 538.1 | 11.1 | 97 | Ex. 8 |
| 81 | 689 | C₂₅ H₂₅ Cl N₅ O₃ S | 525.0 | 5.9 | 78 | Ex. 8 |
| 82 | 694 | C₂₄ H₂₉ N₅ O₂ | 420.2 | 6.6 | 100 | Ex. 7 |
| 83 | 696 | C₂₅ H₂₆ Cl N₅ O₂ S | 496.0 | 8.5 | 98 | Ex. 7 |
| 84 | 702 | C₁₉ H₂₁ N₅ O₃ | 368.2 | 9.5 | 82 | Ex. 7 |
| 85 | 704 | C₂₀ H₁₈ Cl N₅ O₃ S | 444.0 | 5.4 | 87 | Ex. 7 |
| 86 | 709 | C₂₁ H₂₁ Cl N₆ O₂ S | 457.1 | 4.8 | 94 | Ex. 7 |
| 87 | 713 | C₂₁ H₁₉ Cl N₆ O₃ S | 471.0 | 5.2 | 85 | Ex. 8 |
| 88 | 718 | C₂₂ H₂₀ Cl N₅ O₄ S | 486.0 | 6.0 | 90 | Ex. 7 |
| 89 | 723 | C₂₄ H₂₃ N₅ O₂ | 414.5 | 5.0 | 98 | Ex. 7 |
| 90 | 724 | C₂₅ H₂₀ Cl N₅ O₂ S | 490.4 | 8.1 | 100 | Ex. 9 |
| 91 | 729 | C₂₅ H₂₀ Cl N₅ O₃ S | 506.0 | 8.0 | 90 | Ex. 7 |
| 92 | 733 | C₂₅ H₂₅ N₅ O₂ | 428.0 | 6.7 | 98 | Ex. 7 |
| 93 | 734 | C₂₆ H₂₂ Cl N₅ O₂ S | 504.0 | 8.5 | 94 | Ex. 7 |
| 94 | 741 | C₂₂ H₂₀ Cl N₅ O₂ S | 454.0 | 5.7 | 87 | Ex. 7 |
| 95 | 760 | C₂₀ H₂₄ N₅ O₂ | 381.1 | 5.1 | 98 | Ex. 4 |
| 96 | 768 | C₂₁ H₂₁ Cl N₆ O₂ S | 457.1 | 5.4 | 95 | Ex. 4 |
| 97 | 770 | C₂₀ H₂₀ Cl N₅ O₂ S₂ | 461.9 | 10.9 | 99 | Ex. 4 |
| 98 | 776 | C₂₄ H₂₅ Cl N₆ O₂ S | 497.0 | 7.7 | 95 | Ex. 4 |
| 99 | 785 | C₂₃ H₂₂ N₆ O₂ | 415.0 | 6.9 | 96 | Ex. 4 |
| 100 | 795 | C₂₃ H₁₈ Cl N₅ O₂ S₂ | 495.9 | 12.1 | 99 | Ex. 4 |
| 101 | 801 | C₂₄ H₁₈ Cl F N₆ O₂ S | 508.9 | 7.9 | 97 | Ex. 4 |
| 102 | 802 | C₂₄ H₁₈ Cl F N₆ O₂ S | 508.9 | 8.1 | 85 | Ex. 4 |
| 103 | 803 | C₂₄ H₁₈ Cl₂ N₆ O₂ S | 526.9 | 9.1 | 95 | Ex. 4 |
| 104 | 804 | C₂₅ H₂₁ Cl N₆ O₃ S | 520.9 | 7.7 | 98 | Ex. 4 |
| 105 | 805 | C₂₅ H₂₁ Cl N₆ O₂ S | 504.9 | 7.9 | 95 | Ex. 4 |
| 106 | 806 | C₂₅ H₂₁ Cl N₆ O₂ S | 504.9 | 8.2 | 85 | Ex. 4 |
| 107 | 807 | C₂₅ H₂₁ Cl N₆ O₂ S | 504.9 | 8.1 | 89 | Ex. 4 |
| 108 | 808 | C₂₄ H₁₈ Cl₂ N₆ O₂ S | 520.8 | 9.3 | 97 | Ex. 4 |
| 109 | 809 | C₂₄ H₁₆ Cl₂ N₆ O₂ S | 526.9 | 8.7 | 96 | Ex. 4 |
| 110 | 810 | C₂₄ H₁₈ Cl F N₆ O₂ S | 508.9 | 7.7 | 96 | Ex. 4 |
| 111 | 811 | C₂₅ H₂₁ Cl N₆ O₃ S | 520.9 | 8.9 | 93 | Ex. 4 |
| 112 | 812 | C₂₅ H₂₁ Cl N₆ O₃ S | 520.9 | 7.4 | 99 | Ex. 4 |
| 113 | 912 | C₂₂ H₂₆ N₄ O₃ | 349.5 | 9.5 | 91 | Ex. 1 |
| 114 | 915 | C₂₁ H₂₅ Ns O₃ | 396.3 | 5.5 | 98 | Ex. 1 |
| 115 | 917 | C₂₀ H₂₄ N₄ O₄ | 385.3 | 10.9 | 66 | Ex. 1 |
| 116 | 918 | C₂₀ H₂₄ N₄ O₃ S | 401.5 | 12.4 | 93 | Ex. 1 |
| ref 54 | 948 | C₁₈ H₂₁ Cl N₄ O₃ S | 409.2 | 11.4 | 82 | Ex. ref 40 |
| ref 55 | 956 | C₂₄ H₂₅ Cl N₄ O₃ S | 485.2 | 13.9 | 91 | Ex. ref 40 |
| ref 56 | 968 | C₂₃ H₂₉ Cl N₄ O₄ S | 477.3 | 12.9 | 95 | Ex. 1 |
| ref 57 | 970 | C₁₉ H₁₉ Cl N₄ O₃ S | 419.3 | 10.9 | 98 | Ex. ref 41 |
| ref 58 | 972 | C₂₃ H₂₇ Cl N₄ O₃ S | 475.2 | 11.4 | 83 | Ex. ref 41 |
| ref 59 | 973 | C₂₅ H₃₀ Cl N₅ O₃ S | 499.2 | 7.7 | 81 | Ex. ref 41 |
| ref 60 | 974 | C₂₅ H₂₃ Cl N₄ O₃ S | 495.5 | 10.9 | 98 | Ex. ref 41 |
| ref 61 | 975 | C₂₅ H₂₂ Cl F N₄ O₃ S | 513.2 | 11.1 | 95 | Ex. ref 41 |
| ref 62 | 976 | C₂₅ H₂₅ Cl N₄ O₄ S | 525.2 | 11.0 | 98 | Ex. ref 41 |
| ref 63 | 977 | C₂₅ H₂₄ Cl N₅ O₃ S | 510.2 | 7.9 | 28 | Ex. ref 41 |
| ref 64 | 978 | C₂₅ H₂₂ Cl₂ N₄ O₃ S | 529.2 | 11.5 | 95 | Ex. ref 41 |
| ref 65 | 979 | C₂₄ H₂₂ Cl N₅ O₃ S | 496.2 | 7.9 | 72 | Ex. ref 41 |
| ref 66 | 980 | C₂₄ H₃₀ Cl N₅ O₃ S | 487.3 | 7.5 | 92 | Ex. ref 41 |
| ref 67 | 982 | C₂₂ H₂₇ Cl N₄ O, S Si | 491.4 | 12.0 | 87 | Ex. ref 41 |
| 117 | 983 | C₂₃ H₁₃ Cl N₄ O₃ S | 471.3 | 10.5 | 92 | Ex. 1 |
| 118 | 984 | C₂₃ H₂₂ Cl F N₄ O₃ S | 489.2 | 10.9 | 85 | Ex. 1 |
| 119 | 985 | C₂₄ H₂₂ Cl F₃ N₄ O₃ S | 539.2 | 12.1 | 92 | Ex. 1 |
| 120 | 986 | C₂₄ H₂₂ Cl F₃ N₄ O₄ S | 555.3 | 12.2 | 93 | Ex. 1 |
| 121 | 987 | C₂₃ H₂₂ Cl₂ N₄ O₃ S | 505.1 | 11. 6 | 95 | EX. 1 |
| 122 | 988 | C₂₅ H₂₅ Cl N₄ O₄ S | 513.3 | 9.8 | 88 | Ex. 1 |
| 123 | 989 | C₂₉ H₂₇ Cl N₄ O₄ S | 563.3 | 12.5 | 91 | Ex. 1 |
| 124 | 990 | C₂₅ H₂₇ Cl N₄ O₅ S | 531.3 | 9.5 | 91 | Ex. 1 |
| 125 | 991 | C₂₅ H₂₈ Cl N₅ O₃ S | 514.2 | 7.3 | 79 | Ex. 1 |
| 126 | 992 | C₂₄ H₂₅ Cl N₄ O₃ S | 485.3 | 11.1 | 94 | Ex. 1 |
| 127 | 993 | C₂₇ H₂₅ Cl N₄ O₃ S | 521.6 | - | 81 | Ex. 1 |
| 128 | 994 | C₂₉ H₂₇ Cl N₄ O₃ S | 499.2 | 11.6 | 96 | Ex. 1 |
| 129 | 995 | C₂₁ H₂₁ Cl N₆ O₃ S | 473.2 | 8.0 | 22 | Ex. 1 |
| 130 | 996 | C₂₆ R₂₈ Cl N₅ O₄ S | 542.3 | 8.7 | 90 | Ex. 1 |
| 131 | 997 | C₂₉ H₃₃ Cl N₄ O₃ S | 553.3 | 14.0 | 88 | Ex. 1 |
| 132 | 998 | C₂₂ H₂₂ Cl N₅ O₃ S | 472.2 | 6.6 | 78 | Ex. 1 |
| 133 | 999 | C₂₂ H₂₂ Cl N₅ O₃ S | 472.4 | 8.0 | 97 | Ex. 1 |
| 134 | 1001 | C₂₁ H₂₁ Cl N₄ O₄ S | 461.1 | 12.5 | 84 | Ex. 1 |
| 135 | 1002 | C₂₁ H21 Cl N₄ O₃ S₂ | 477.1 | 13.1 | 94 | EX. 1 |
| 136 | 1003 | C₂₃ H₂₁ Cl N₅ O₄ S | 502.0 | 11.1 | 61 | Ex. 1 |
| 137 | 1004 | C₂₃ H₂₄ Cl N₅ O₄ S | 502.0 | 11.5 | 71 | Ex. 1 |
| 138 | 1005 | C₂₃ H₂₈ Cl N₅ O₄ S | 502.1 | 7.7 | 93 | Ex. 1 |
| 139 | 1006 | C₂₂ H₂₁ Cl₂ N₅ O₃ S | 506.0 | 12.8 | 90 | Ex. 1 |
| 140 | 1007 | C₂₂H₂₁Cl F N₅ O₃ S | 490.1 | 12.1 | 80 | Ex. 1 |
| 141 | 1008 | C₂₂ H₂₁ Cl F N₅ O₃ S | 490.1 | 12.8 | 94 | Ex. 1 |
| 142 | 1009 | C₂₆ H₂₄ Cl N₅ O₃ S | 522.0 | 10.4 | 54 | Ex. 1 |
| 143 | 1010 | C₂₄ H₂₂ Cl N₅ O₃ S | 496.4 | 14.7 | 88 | Ex. 1 |
| 144 | 1011 | C₂₃ H₂₂ Cl F N₄ O₃ S | 489.3 | 13.0 | 99 | Ex. 1 |
| 145 | 1012 | C₂₃ H₂₂ Cl F N₄ O₃ S | 489.4 | 13.3 | 96 | Ex. 1 |
| 146 | 1013 | C₂₂ H₂₃ Cl N₄ O₃ S₂ | 491.3 | 12.9 | 99 | Ex. 1 |
| 147 | 1014 | C₂₀ H₂₁ Cl N₆ O₃ S | 461.4 | 8.9 | 98 | Ex. 1 |
| ref 68 | 1106 | C₁₈ H₁₉ N₅ O₂ | 338.4 | 5.1 | 97 | Ex. 12 |
| ref 69 | 1131 | C₁₅ His N₅ O₂ | 298.3 | 2.2 | 99 | Ex. 12 |
| ref 70 | 1132 | C₁₄ H₁₃ N₅ O₂ | 284.2 | 1.7 | 99 | Ex. 12 |
| ref 71 | 1146 | C₁₆ His N₅ C₂ | 310.4 | 2.6 | 99 | Ex. 12 |
| ref 72 | 1161 | C₁₉ H₁₅ N₅ O₂ | 346.2 | 5.9 | 96 | Ex. 12 |
| ref 73 | 1168 | C₁₉ H₂₃ N₅ O₂ | 354.3 | 7.1 | 99 | Ex. 12 |
| ref 74 | 1170 | C₁₅ H₁₄ N₄ O₃ | 299.2 | 4.4 | 96 | Ex. 11 |
| ref 75 | 1174 | C₁₂ H₁₀ N₄ O | 227.2 | 2.4 | 100 | Ex. 1 |
| ref 76 | 1175 | C₁₂ H₉ Cl N₄ O | 261.1 | 4.4 | 95 | Ex. ref 43 |
| ref 77 | 1177 | C₁₁ H₈ N₄ O | 213.1 | 5.0 | 99 | Ex. 1 |
| ref 78 | 1179 | C₁₈ H₁₄ N₄ O | 303.2 | 7.2 | 99 | Ex. 11 |
| 148 | 1181 | C₁₉ H₁₅ N₁ O | 358.1 | 3.6 | 100 | Ex. 10 |
| 149 | 1182 | C₂₃ H₁₆ Cl N₁ O S | 474.0 | 6.8 | 67 | Ex. 10 |
| 150 | 1183 | C₂₂ H₁₇ Cl N₈ O S | 477.1 | 7.7 | 100 | Ex. 10 |
| 151 | 1184 | C₂₃ H₁₉ N₇ O | 410.3 | 2.3 | 100 | Ex. 10 |
| 152 | 1185 | C₂₄ H₁₈ Cl N₇ O S | 487.9 | 6.0 | 90 | Ex. 10 |
| 153 | 1186 | C₂₂ H₁₇ Cl F₃ N₇ O S | 520.1 | 8.1 | 100 | Ex. 10 |
| 154 | 1206 | C₂₀ H₁₉ Cl N₆ O₂ S | 443.1 | 6.7 | 97 | Ex. 7 |
| 155 | 1216 | C₂₁ H₂₁ Cl N₆ O₂ S | 457.0 | 8.6 | 87 | Ex. 1 |
| 156 | 1217 | C₂₂ H₂₃ Cl N₆ O₂ S | 471.1 | 7.6 | 83 | Ex. 7 |
| 157 | 1228 | C₂₃ H₂₅ Cl N₆ O₂ S | 485.2 | 8.2 | 96 | Ex. 7 |
| 158 | 1236 | C₂₅ H₂₆ Cl N₅ O₂ S | 497.9 | 8.4 | 96 | Ex. 8 |
| 159 | 1237 | C₂₄ H₂₇ Cl N₆ O₂ S | 499.2 | 8.5 | 99 | Ex. 8 |
| 160 | 1243 | C₂₅ H₂₂ Cl F N₆ O₂ S | 525.2 | 8.5 | 95 | Ex. 7 |
| 161 | 1250 | C₂₁ H₂₃ Cl N₆ O₂ S | 459.2 | 7.5 | 95 | Ex. 7 |
| 162 | 1257 | C₂₁ H₂₃ Cl N₅ O₂ S | 459.2 | 7.6 | 98 | Ex. 7 |
| 163 | 1258 | C₁₈ H₂₄ N₆ O₂ | 357.2 | 5.9 | 100 | Ex. 19 |
| 164 | 1259 | C₁₈ H₂₃ Cl N₆ O₂ | 391.2 | 6.7 | 98 | Ex. 16 |
| 165 | 1260 | C₂₁ H₂₇ N₅ O₂ | 382.2 | 5.3 | 100 | Ex. 19 |
| 166 | 1261 | C₂₀ H₂₈ N₆ O₂ | 385.3 | 6.4 | 96 | Ex. 7 |
| 167 | 1262 | C₂₁H₃₀N₆O₂ | 399.3 | 6.6 | 100 | Ex. 7 |
| 168 | 1271 | C₂₁ H₂₈ N₆ O₂ | 397.2 | 5.8 | 100 | Ex. 7 |
| 169 | 1277 | C₂₃ H₂₈ N₆ O₃ | 437.3 | 7.6 | 79 | Ex. 19 |
| 170 | 1278 | C₂₂ H₂₆ N₆ O₃ | 423.2 | 7.2 | 100 | Ex. 19 |
| 171 | 1282 | C₂₃ H₂₅ N₅ O₂ S | 436.2 | 6.0 | 95 | Ex. 19 |
| 172 | 1284 | C₂₂ H₂₆ N₆ O₂ S | 439.2 | 7.5 | 100 | Ex. 19 |
| 173 | 1286 | C₂₃ H₂₈ N₆ O₂ S | 453.2 | 8.0 | 100 | Ex. 19 |
| 174 | 1287 | C₂₃ H₂₄ Cl N₅ O₃ S | 486.3 | 6.7 | 100 | Ex. ref 52 |
| 175 | 1288 | C₂₂ H₂₅ Cl N₆ O₂ S | 473.1 | 7.8 | 93 | Ex. 7 |
| 176 | 1293 | C₂₃ H₂₇ Cl N₆ O₂ S | 987.1 | 8.6 | 100 | Ex. 1 |
| 177 | 1296 | C₂₃ H₂₈ N₆ O₂ S | 453.2 | 8.1 | 95 | Ex. 19 |
| 178 | 1297 | C₂₁ H₂₅ N₅ O₂ | 380.2 | 5.2 | 96 | Ex. 19 |
| 179 | 1331 | C₂₃ H₂₇ N₇ O₂ | 434.2 | 5.2 | 100 | Ex. 19 |
| 180 | 1332 | C₂₂ H₂₈ N₈ O₂ | 437.3 | 5.9 | 100 | Ex. 19 |
| 181 | 1333 | C₂₆ H₃₂ N₆ O₄ | 493.2 | 7.1 | 81 | Ex. 19 |
| 182 | 1334 | C₂₅ H₂₈ N₆ O₄ | 477.2 | 7.5 | 100 | Ex. 19 |
| 183 | 1335 | C₂₃ H₂₅ N₅ O₃ | 420.2 | 5.6 | 99 | Ex. 19 |
| 184 | 1337 | C₂₂ H₂₆ N₆ O₃ | 423.2 | 7.1 | 100 | Ex. 19 |
| 185 | 1338 | C₂₂ H₂₆ N₆ O₂ S | 439.2 | 7.6 | 98 | Ex. 19 |
| 186 | 1341 | C₂₃ H₂₈ N₆ O₂ S | 453.2 | 8.3 | 100 | Ex. 19 |
| 187 | 1364 | C₂₁ H₂₃ N₃ O₂ | 378.3 | 4.3 | 98 | Ex. 7 |
| 188 | 1374 | C₂₁ H₂₁ Cl N₆ O₂ S | 457.1 | 7.1 | 96 | Ex. 7 |
| 189 | 1399 | C₂₃ H₂₅ Cl N₆ O₂ S | 485.2 | 8.0 | 92 | Ex. 8 |
| 190 | 1405 | C₂₄ H₂₉ N₅ O₂ | 420.2 | 6.1 | 100 | Ex. 8 |
| 191 | 1413 | C₂₅ H₂₆ Cl N₅ O₂ S | 497.9 | 8.7 | 96 | Ex. 8 |
| 192 | 1415 | C₂₄ H₂₇ Cl N₆ O₂ S | 499.1 | 8.6 | 100 | Ex. 8 |
| 193 | 1423 | C₂₅ H₃₁ N₅ O₂ | 434.4 | 6.5 | 98 | Ex. 8 |
| 194 | 1427 | C₂₅ H₂₉ Cl N₆ O₂ S | 513.2 | 9.3 | 96 | Ex. 8 |
| 195 | 1430 | C₂₁ H₂₁ Cl N₆ O₂ S | 457.1 | 7.1 | 89 | Ex. 7 |
| 196 | 1445 | C₂₄ H₂₇ Cl N₆ O₂ S | 499.2 | 8.8 | 100 | Ex. 8 |
| 197 | 1482 | C₂₅ H₃₁ N₅ O₂ | 434.4 | 6.8 | 96 | Ex. 7 |
| 198 | 1486 | C₂₅ H₂₉ Cl N O₂ S | 513.2 | 9.5 | 100 | Ex. 8 |
| 199 | 1490 | C₂₂ H₂₇ N₅ O₂ | 394.4 | 5.4 | 98 | Ex. 7 |
| 200 | 1494 | C₂₂ H₂₅ Cl N₆ O₂ S | 473.2 | 8.2 | 98 | Ex. 7 |
| 201 | 1511 | C₂₆ H₂₃ Cl N₆ O₂ S | 519.1 | 6.5 | 91 | Ex 5 |
| 202 | 1514 | C₂₁ H₁₉ Cl N₆ O₂ S | 455.1 | 6.9 | 87 | Ex. 7 |
| 203 | 1519 | C₂₀ H₂₃ N₅ O₂ S | 398.2 | 4.9 | 100 | Ex. 8 |
| 204 | 1520 | C₂₁ H₂₀ Cl N₅ O₂ S₂ | 474.0 | 5.3 | 98 | Ex. 7 |
| 205 | 1522 | C₂₄ H₂₅ Cl N₆ O₃ S | 513.1 | 4.2 | 100 | Ex. 8 |
| 206 | 1530 | C₂₅ H₂₇ Cl N₆ O₂ S | 512.9 | 5.4 | 92 | Ex. 8 |
| 207 | 1533 | C₂₀ H₁₄ Cl F₄ N₅ O₂ S | 500.0 | 6.3 | 65 | Ex. 7 |
| 208 | 1535 | C₂₁ H₁₃ Cl F₇ N₅ O₂ S | 568.0 | 7.7 | 96 | Ex. 7 |
| 209 | 1549 | C₂₅ H₃₁ N₅ O₂ | 434.2 | 6.8 | 100 | Ex. 7 |
| 210 | 1553 | C₂₅ H₂₉ Cl N₆ O₂ S | 513.2 | 9.5 | 90 | Ex. 7 |
| 211 | 1554 | C₂₆ H₃₃ N₅ O₂ | 448.2 | 7.5 | 100 | Ex. 7 |
| 212 | 1558 | C₂₆ H₃₁ Cl N₆ O₂ S | 527.2 | 10.3 | 95 | Ex. 7 |
| 213 | 1566 | C₂₄H₂₅ClN₆O₂S | 497.2 | 8.3 | 97 | Ex. 8 |
| 214 | 1589 | C₂₃ H₂₅ F₂ N₅ O₂ | 442.23 | 5.3 | 98 | Ex. 8 |
| 215 | 1600 | C₂₃ H₂₃ Cl F₂ N₆ O₂ S | 521.2 | 7.8 | 98 | Ex. 8 |
| 216 | 1623 | C₂₇ H₃₅ N₅ O₂ | 462.2 | 7.5 | 100 | Ex. 7 |
| | | | | 7.7 | | |
| 217 | 1647 | C₂₄ H₂₆ F₃ N₅ O₂ | 474.2 | 6.2 | 79 | Ex. 8 |
| | | | | 6.5 | | |
| 218 | 1654 | C₂₄ H₂₄ Cl F₃ N₆ O₂ S | 553.2 | 9.1 | 99 | Ex. 8 |
| 219 | 1660 | C₂₅ H₃₁ N₅ O₂ | 434.3 | 6.5 | 99 | Ex. 8 |
| | | | | 6.6 | | |
| 220 | 1661 | C₂₅ H₂₉ Cl N₆ O₂ S | 513.2 | 9.3 | 98 | Ex. 8 |
| | | | | 9.4 | | |
| 221 | 1673 | C₂₂ H₂₅ N₅ O₃ | 408.2 | 4.5 | 100 | Ex. 8 |
| 222 | 1679 | C₂₂ H₂₃ Cl N₆ O₃ S | 487.1 | 6.4 | 95 | Ex. 8 |
| 223 | 1682 | C₂₈ H₃₁ Cl N₆ O₄ S | 583.2 | 6.9 | 96 | Ex. 8 |
| 224 | 1691 | C₂₈ H₃₁ Cl N₆ O₄ S | 583.2 | 6.9 | 98 | Ex. 8 |
| 225 | 1693 | C₂₇ H₃₂ Cl N₇ O₄ S | 586.2 | 8.4 | 95 | Ex. 8 |
| 226 | 1695 | C₂₇ H₃₂ Cl N₇ O₄ S | 586.2 | 8.6 | 95 | Ex. 8 |
| 227 | 1696 | C₂₈ H₃₁ Cl N₆ O₄ S | 583.1 | 7.0 | 99 | Ex. 8 |
| 228 | 1697 | C₂₇ H₃₂ Cl N₇ O₄ S | 586.2 | 8.6 | 94 | Ex. 8 |
| 229 | 1698 | C₂₆ H₃₀ Cl N₇ O₄ S | 572.1 | 7.8 | 93 | Ex. 8 |
| 230 | 1699 | C₂₇ H₂₉ Cl N₆ O₄ S | 569.1 | 6.2 | 98 | Ex. 8 |
| 231 | 1700 | C₂₆ H₃₀ Cl N₇ O₄ S | 572.2 | 7.8 | 92 | Ex. 8 |
| 232 | 1715 | C₂₉ H₃₃ Cl N₆ O₃ S | 581.2 | 6.3 | 93 | Ex. 8 |
| 233 | 1719 | C₂₇ H₂₇ Cl N₆ O₃ S | 551.1 | 5.2 | 96 | Ex. 8 |
| 234 | 1721 | C₂₈ H₂₉ Cl N₆ O₃ S | 565.2 | 5.7 | 93 | Ex. 8 |
| 235 | 1739 | C₂₇ H₃₁ Cl N₆ O₂ S | 539.2 | 4.7 | 90 | Ex. 8 |
| 236 | 1748 | C₂₈ H₃₃ Cl N₆ O₂ S | 553.2 | 5.1 | 95 | Ex. 8 |
| 237 | 1751 | C₂₈ H₃₃ Cl N₆ O₂ S | 553.2 | 5.2 | 96 | Ex. 8 |
| 238 | 1753 | C₂₉ H₃₅ Cl N₆ O₂ S | 567.2 | 5.5 | 89 | Ex. 8 |
| 239 | 1758 | C₂₇ H₂₉ Cl N₆ O₂ S | 537.1 | 4.7 | 96 | Ex. 8 |
| 240 | 1778 | C₂₅ H₂₂ F₃ N₅ O₂ | 482.3 | 6.5 | 99 | Ex. 8 |
| 241 | 1804 | C₂₇ H₂₁ Cl N₆ O₂ S | 530.9 | 7.4 | 92 | Ex. 8 |
| 242 | 1805 | C₂₈ H₂₃ Cl N₆ O₂ S | 543.1 | 8.1 | 89 | Ex. 8 |
| 243 | 1806 | C₂₇ H₂₂ Cl N₅ O₂ S | 516.1 | 6.8 | 96 | Ex. 7 |
| 244 | 1808 | C₂₇ H₂₂ Cl N₅ O₂ S | 518.0 | 8.1 | 90 | Ex. 8 |
| 245 | 1823 | C₂₆ H₂₃ Cl N₆ O₂ S | 519.1 | 5.2 | 94 | Ex. 8 |
| 246 | 1828 | C₂₇ H₂₅ Cl N₆ O₂ S | 533.1 | 5.2 | 89 | Ex. 8 |
| 247 | 1833 | C₂₆ H₂₁ Cl N₆ O₂ S | 517.1 | 5.9 | 86 | Ex. 8 |
| 248 | 1843 | C₂₃ H₂₅ N₅ O₃ | 419.2 | 4.6 | 86 | Ex. 8 |
| 249 | 1846 | C₂₆ H₂₇ N₅ O₂ | 441.2 | 6.1 | 90 | Ex. 8 |
| 250 | 1849 | C₂₈ H₃₆ N₅ O₄ | 520.3 | 6.3 | 39 | Ex. 8 |
| 251 | 1852 | C₂₉ H₃₆ N₅ O₄ | 534.3 | 6.9 | 43 | Ex. 8 |
| 252 | 1855 | C₁₉ H₁₈ F₃ N₅ O₂ | 406.2 | 4.4 | 98 | Ex. 8 |
| 253 | 1860 | C₁₉H₁₆ClF₃N₆O₂S | 485.1 | 7.4 | 91 | Ex. 8 |
| 254 | 1861 | C₁₈ H₁₆ Cl F₂ N₅ O₂ | 408.2 | 4.9 | 36 | Ex. 8 |
| 255 | 1865 | C₂₀ H₂₂ Cl N₅ O₂ | 400.3 | 4.6 | 60 | Ex. 7 |
| 256 | 1868 | C₂₁ H₂₃ N₅ O₂ | 378.3 | 4.4 | 97 | Ex. 7 |
| 257 | 1885 | C₂₀ H₂₀ F₃ N₅ O₂ | 420.1 | 5.0 | 100 | Ex. 8 |
| 258 | 1890 | C₂₀ H₁₈ Cl F₃ N₅ O₂ S | 499.1 | 7.7 | 98 | Ex. 8 |
| 259 | 1891 | C₂₄ H₂₉ N₅ O₂ | 420.2 | 6.3 | 100 | Ex. 8 |
| 260 | 1894 | C₂₄ H₂₇ Cl N₆ O₂ S | 499.2 | 9.0 | 100 | Ex. 7 |
| 261 | 1899 | C₂₁ H₂₂ F₃ N₅ O₂ | 434.1 | 5.5 | 100 | Ex. 8 |
| 262 | 1900 | C₂₁ H₂₀ Cl F₃ N₆ O₂ S | 513.1 | 8.2 | 98 | Ex. 8 |
| 263 | 1913 | C₂₁ H₂₀ N₆ O₂ | 389.1 | 4.1 | 100 | Ex. 8 |
| 264 | 1915 | C₂₁ H₂₁ Cl₂ N₅ O₂ | 446.1 | 5.5 | 100 | Ex. 8 |
| 265 | 2003 | C₂₄H₂₇ClN₆O₂ | 467.2 | 8.6 | 70 | Ex. 19 |
| 266 | 2004 | C₂₅ H₂₅ N₅ O₂ | 440.5 | 6.0 | 99 | Ex. 7 |
| 267 | 2005 | C₂₂ H₂₃ Cl N₆ O₂ S | 471.0 | 7.2 | 97 | Ex. 4 |
| 268 | 2006 | C₂₃ H₂₃ Cl N₆ O₂ S | 483.0 | 7.4 | 97 | Ex. 4 |
| 269 | 2007 | C₂₄ H₂₁ F₃ N₆ O₂ | 483.3 | 7.0 | 99 | Ex. 4 |
| 270 | 2008 | C₂₄ H₂₁ F₃ N₆ O₃ | 499.3 | 7.1 | 96 | Ex. 4 |
| 271 | 2009 | C₂₃ H₁₇ Cl₂ N₇ O₂ S | 526.0 | 6.5 | 90 | Ex. 5 |
| 272 | 2010 | C₂₅ H₂₀ Cl F N₆ O₂ S | 523.1 | 6.3 | 97 | Ex. 4 |
| 273 | 2011 | C₂₄ H₂₁ Cl F N₇ O₂ S | 526.1 | 7.9 | 97 | Ex. 4 |
| 274 | 2012 | C₂₄ H₂₀ Cl N₇ O₂ S | 506.1 | 4.0 | 81 | Ex. 5 |
| 275 | 2013 | C₂₅ H₂₃ F₃ N₅ O₃ | 513.1 | 6.8 | 100 | Ex. 4 |
| 276 | 2014 | C₂₅ H₂₇ Cl N₆ O₂ S | 511.0 | 9.4 | 97 | Ex. 4 |
| 277 | 2015 | C₂₄ H₂₈ Cl N₇ O₂ S | 514.1 | 8.5 | 100 | Ex. 4 |
| 278 | 2016 | C₂₅ H₂₁ Cl N₆ O₂ S | 505.0 | 7.7 | 93 | Ex. 4 |
| 279 | 2017 | C₂₄ H₂₁ F₃ N₆ O₂ | 483.2 | 6.8 | 100 | Ex. 4 |
| 280 | 2018 | C₂₄ H₂₁ F₃ N₆ O₂ | 483.3 | 6.0 | 99 | Ex. 4 |
| 281 | 2019 | C₂₄ H₂₆ Cl N₇ O₃ S | 528.1 | 4.4 | 96 | Ex. 5 |
| 282 | 2020 | C₂₅ H₂₈ Cl N₇ O₃ S | 542.1 | 4.3 | 100 | Ex. 5 |
| 283 | 2021 | C₂₅ H₂₈ Cl N₇ O₂ S | 526.1 | 4.8 | 98 | Ex. 5 |
| 284 | 2022 | C₂₅ H₃₂ N₆ O₂ | 449.2 | 6.7 | 100 | Ex. 4 |
| 285 | 2023 | C₂₆ H₂₉ Cl N₆ O₂ S | 525.1 | 7.3 | 98 | Ex. 4 |
| 286 | 2024 | C₂₅ H₃₀ Cl N₇ O₂ S | 528.1 | 8.8 | 98 | Ex. 5 |
| 287 | 2025 | C₂₆ H₂₉ Cl N₆ O₂ S | 525.2 | 7.2 | 94 | Ex. 5 |
| 288 | 2026 | C₂₅ H₃₀ Cl N₇ O₂ S | 528.1 | 8.9 | 100 | Ex. 4 |
| 289 | 2027 | C₂₅ H₂₇ Cl N₆ O₃ S | 527.2 | 5.5 | 96 | Ex. 5 |
| 290 | 2028 | C₂₈ H₂₉ Cl N₆ O₂ S | 549.0 | 9.7 | 97 | Ex. 4 |
| 291 | 2029 | C₂₅ H₂₀ Cl F N₆ O₂ S | 523.1 | 5.8 | 97 | Ex. 4 |
| 292 | 2031 | C₂₄ H₂₃ F N₆ O₂ | 447.1 | 5.5 | 100 | Ex. 4 |
| 293 | 2032 | C₂₅ H₂₀ Cl F N₆ O₂ S | 523.1 | 6.3 | 98 | Ex. 4 |
| 294 | 2033 | C₂₄ H₂₁ Cl F N₇ O₂ S | 526.1 | 7.8 | 94 | Ex. 4 |
| 295 | 2034 | C₂₅ H₁₉ Cl F₂ N₆ O₂ S | 541.0 | 6.7 | 96 | Ex. 4 |
| 296 | 2036 | C₂₄ H₂₀ Cl F₂ N₇ O₂ S | 544.1 | 7.6 | 94 | Ex. 4 |
| 297 | 2040 | C₂₄ H₂₂ Cl₂ N₆ O₂ | 497.3 | 6.6 | 100 | Ex. 5 |
| 298 | 2042 | C₂₅ H₂₃ F₃ N₆ O₂ | 497.3 | 6.3 | 97 | Ex. 5 |
| 299 | 2046 | C₂₅ H₂₃ F₃ N₆ O₂ | 497.3 | 6.6 | 98 | Ex. 5 |
| 300 | 2049 | C₂₅ H₂₁ Cl F₃ N₇ O₂ S | 576.1 | 8.9 | 100 | Ex. 4 |
| 301 | 2052 | C₂₆ H₂₀ Cl F₃ N₆ O₃ S | 589.0 | 7.6 | 98 | Ex. 4 |
| 302 | 2053 | C₂₅ H₂₁ Cl F₃ N₇ O₃ S | 592.1 | 9.0 | 100 | Ex. 4 |
| 303 | 2054 | C₂₄ H₂₄ F₃ N₇ O₃ | 516.2 | 8.0 | 100 | Ex. 4 |
| 304 | 2056 | C₂₆ H₂₀ Cl F₃ N₆ O₃ S | 589.0 | 7.7 | 98 | Ex. 4 |
| 305 | 2058 | C₂₅ H₂₁ Cl F₃ N₇ O₃ S | 592.1 | 9.1 | 100 | Ex. 4 |
| 306 | 2060 | C₂₆ H₂₃ Cl N₆ O₃ S | 535.1 | 6.3 | 99 | Ex. 4 |
| 307 | 2062 | C₂₆ H₂₃ Cl N₆ O₃ S | 535.1 | 6.2 | 96 | Ex. 4 |
| 308 | 2063 | C₂₅ H₂₄ Cl N₇ O₃ S | 538.1 | 7.7 | 91 | Ex. 4 |
| 309 | 2064 | C₂₅ H₂₆ N₆ O₃ | 459.1 | 5.3 | 100 | Ex. 4 |
| 310 | 2065 | C₂₆ H₂₃ Cl N₆ O₃ S | 535.1 | 6.1 | 99 | Ex. 4 |
| 311 | 2067 | C₂₅ H₂₄ Cl N₇ O₃ S | 538.1 | 7.7 | 91 | Ex. 4 |
| 312 | 2068 | C₂₇ H₂₅ Cl N₆ O₄ S | 565.1 | 5.8 | 89 | Ex. 4 |
| 313 | 2069 | C₂₆ H₂₆ Cl N₇ O₄ S | 568.1 | 7.0 | 63 | Ex. 4 |
| 314 | 2070 | C₂₇ H₂₅ Cl N6 O₄ S | 565.1 | 6.5 | 87 | Ex. 4 |
| 315 | 2071 | C₂₆ H₂₆ Cl N₇ O₄ S | 568.1 | 7.7 | 94 | Ex. 4 |
| 316 | 2072 | C₂ H₂₁ Cl N₆ O₄ S | 549.1 | 6.2 | 96 | Ex. 4 |
| 317 | 2073 | C₂₅ H₂₂ Cl N₇ O₄ S | 552.0 | 7.5 | 90 | Ex. 4 |
| 318 | 2078 | C₂₅ H₂₃ Cl N₆ O₄ S₂ | 583.0 | 5.6 | 78 | Ex. 4 |
| 319 | 2080 | C₂₅ H₂₇ Cl N₈ O₂ S | 551.0 | 5.8 | 95 | Ex. 5 |
| 320 | 2081 | C₂₄ H₂₀ Cl N₇ O₂ S | 506.1 | 4.1 | 65 | Ex. 5 |
| 321 | 2083 | C₂₄ H₂₀ Cl N₇ O₂ S | 506.1 | 4.1 | 75 | Ex. 4 |
| 322 | 2085 | C₂₄ H₁₉ Cl₂ N₇ O₂ S | 540.0 | 5.0 | 88 | Ex. 4 |
| 323 | 2086 | C₂₃ H₂₀ Cl₂ N₆ O₂ S | 543.0 | 6.9 | 100 | Ex. 5 |
| 324 | 2087 | C₂₃ H₁₉ Cl N₆ O₃ S | 495.6 | 5.6 | 90 | Ex. 4 |
| 325 | 2089 | C₂₃ H₁₉ Cl N₆ O₂ S₂ | 511.0 | 5.8 | 94 | Ex. 4 |
| 326 | 2093 | C₂₆ H₂₃ Cl N₆ O₂ S | 519.1 | 6.5 | 98 | Ex. 4 |
| 327 | 2094 | C₂₆ H₂₃ Cl N₆ O₂ S | 519.1 | 6.5 | 98 | Ex. 4 |
| 328 | 2095 | C₂₇ H₂₅ Cl N₆ O₃ S | 549.1 | 6.4 | 100 | Ex. 4 |
| 329 | 2096 | C₂₇ H₂₅ Cl N₆ O₃ S | 549.1 | 6.4 | 94 | Ex. 4 |
| 330 | 2097 | C₂₅ H₂₆ Cl N₇ O₃ S | 552.1 | 7.9 | 94 | Ex. 4 |
| 331 | 2098 | C₂₅ H₂₃ Cl F N₇ O₂ S | 540.1 | 8.2 | 100 | Ex. 4 |
| 332 | 2099 | C₂₅ N₁₈ Cl F₃ N₆ O₂ S | 558.9 | 9.6 | 96 | Ex. 4 |
| 333 | 2100 | C₂₃ H₁₇ Cl₂ N₇ O₂ S | 526.0 | 6.0 | 93 | Ex. 5 |
| 334 | 2101 | C₂₁ H₁₆ Cl N₇ O₂ S₂ | 497.9 | 6.4 | 85 | Ex. 4 |
| 335 | 2102 | C₂₄ H₂₀ Cl F₂ N₇ O₂ S | 544.1 | 8.1 | 93 | Ex. 5 |
| 336 | 2103 | C₂₅ H₂₂ F₄ N₆ O₂ | 515.3 | 6.7 | 97 | Ex. 5 |
| 337 | 2105 | C₂₅ H₂₂ F₄ N₆ O₂ | 515.3 | 6.6 | 100 | Ex. 5 |
| 338 | 2107 | C₃₀ H₂₈ N₆ O₂ | 505.4 | 7.0 | 80 | Ex. 5 |
| 339 | 2109 | C₂₅ H₂₁ Cl N₆ O₂ S | 505.1 | 6.1 | 89 | Ex. 5 |
| 340 | 2110 | C₁₀ H₂₁ N₅ O₃ | 356.3 | 4.5 | 91 | Ex. 1 |
| 341 | 2116 | C₂₀ H₂₆ N₆ O₃ | 399.1 | 6.7 | 100 | Ex. 1 |
| 342 | 2119 | C₂₃ H₃₂ N₆ O₃ | 441.2 | 8.1 | 86 | Ex. 1 |
| 343 | 2120 | C₁₉ H₂₂ N₄ O₄ | 371.0 | 7.4 | 33 | Ex. 32 |
| 344 | 2122 | C₂₃ H₂₆ N₆ O₄ | 451.0 | 7.7 | 41 | Ex. 32 |
| 345 | 2127 | C₁₈ H₁₉ Cl N₈ O₃ S | 462.9 | 8.6 | 98 | Ex. 1 |
| ref 79 | 2141 | C₂₆ H₂₈ N₄ O₄ | 461.0 | 11.5 | 99 | Ex. ref 24 |
| 346 | 2142 | C₃₁ H₃₁ N₅ O₄ | 538.0 | 9.3 | 99 | Ex. 1 |
| 347 | 2143 | C₃₀ H₃₂ N₆ O₄ | 541.1 | 11.4 | 70 | Ex. 1 |
| 348 | 2146 | C₂₂ H₂₂ Cl N₅ O₃ S | 472.1 | 6.8 | 99 | Ex. 7 |
| 349 | 2147 | C₂₅ H₂₀ Cl N₅ O₃ S | 506.1 | 7.1 | 98 | Ex. 7 |
| 350 | 2148 | C₂₂ H₂₂ Cl N₅ O₃ S | 472.1 | 6.8 | 99 | Ex. 7 |
| 351 | 2158 | C₂₀ H₂₃ N₅ O₂ | 366.4 | 4.7 | 100 | Ex. 15 |
| 352 | 2161 | C₂₄ H₂₉ N₅ O₂ | 420.3 | 7.1 | 93 | Ex. 26 |
| 353 | 2162 | C₂₃ H₃₀ N₆ O₂ | 423.3 | 7.8 | 100 | Ex. 26 |
| 354 | 2164 | C₂₄ H₂₈ N₆ O₂ S | 465.3 | 8.6 | 100 | Ex. 19 |
| 355 | 2165 | C₂₉ H₃₃ N₅ O₄ | 516.4 | 7.5 | 99 | Ex. 19 |
| 356 | 2165 | C₂₅ H₂₇ N₅ O₃ | 446.2 | 7.3 | 98.9 | Ex. 19 |
| 357 | 2166 | C₂₈ H₃₄ N₆ O₄ | 519.3 | 8.1 | 100 | Ex. 19 |
| 358 | 2170 | C₂₄ H₂₈ N₆ O₃ | 449.3 | 8.3 | 100 | Ex. 19 |
| 359 | 2175 | C₃₀ H₃₁ N₅ O₃ | 510.3 | 7.6 | 88 | Ex. 26 |
| 360 | 2176 | C₂₉ H₃₂ N₆ O₃ | 513.4 | 8.2 | 100 | Ex. 26 |
| 361 | 2183 | C₂₄ H₂₄ F N₅ O₂ S | 466.3 | 6.2 | 98 | Ex. 19 |
| 362 | 2184 | C₂₃ H₂₅ F N₆ O₂ S | 469.3 | 7.6 | 100 | Ex. 19 |
| 363 | 2185 | C₁₈ H₃₀ F N₅ O₄ | 520.3 | 6.1 | 99 | Ex. 19 |
| 364 | 2186 | C₂₇ H₃₁ F N₆ O₄ | 523.3 | 7.7 | 98 | Ex. 19 |
| 365 | 2187 | C₂₄ H₂₄ F N₅ O₃ | 450.3 | 5.9 | 98 | Ex. 19 |
| 366 | 2188 | C₂₃ H₂₅ F N₆ O₃ | 453.3 | 7.3 | 100 | Ex. 19 |
| 367 | 2202 | C₁₁ H₂₅ N₅ O₂ | 380.4 | 5.3 | 100 | Ex. 15 |
| 368 | 2209 | C₂₁ H₁₉ N₅ O₂ | 374.3 | 5.1 | 100 | Ex. 15 |
| 369 | 2210 | C₂₄ H₂₂ F N₅ O₂ | 432.2 | 7.0 | 93 | Ex. 26 |
| 370 | 2211 | C₂₃ H₂₃ F N₆ O₂ | 435.3 | 7.8 | 100 | Ex. 26 |
| 371 | 2212 | C₂₅ H₂₂ F₃ N₅ O₂ | 482.3 | 7.2 | 79 | Ex. 26 |
| 372 | 2224 | C₂₅ H₂₂ F₃ N₅ O₃ | 498.3 | 7.5 | 99 | Ex. 26 |
| 373 | 2240 | C₂₅ H₂₄ F N₅ O₂ | 446.3 | 7.0 | 96 | Ex. 26 |
| 374 | 2241 | C₂₄ H₂₅ F N₅ O₂ | 449.3 | 7.6 | 100 | Ex. 26 |
| 375 | 2265 | C₂₅ H₃₁ N₅ O₂ | 434.3 | 7.5 | 98 | Ex. 26 |
| 376 | 2266 | C₂₄ H₃₂ N₅ O₂ | 437.4 | 8.3 | 100 | Ex. 26 |
| 377 | 2267 | C₂₆ H₂₉ N₅ O₂ S | 476.3 | 7.5 | 98 | Ex. 19 |
| 378 | 2268 | C₂₅ H₃₀ N₆ O₂ S | 479.3 | 9.1 | 100 | Ex. 19 |
| 379 | 2270 | C₂₉ H₃₁ N₅ O₄ | 514.0 | 8.2 | 100 | Ex. 19 |
| 380 | 2271 | C₂₆ H₂₉ N₅ O₃ | 460.3 | 7.4 | 93 | Ex. 19 |
| 381 | 2272 | C₂₅ H₃₀ N₆ O₃ | 463.3 | 8.8 | 100 | Ex. 19 |
| 382 | 2275 | C₂₆ H₃₃ N₅ O₂ | 448.3 | 8.2 | 83 | Ex. 26 |
| 383 | 2277 | C₂₅ H₃₄ N₆ O₂ | 451.4 | 9.0 | 100 | Ex. 26 |
| 384 | 2281 | C₂₇ H₃₁ N₅ O₂ S | 490.4 | 7.7 | 100 | Ex. 19 |
| 385 | 2282 | C₂₆ H₃₂ N₆ O₂ S | 493.3 | 9.8 | 100 | Ex. 19 |
| 386 | 2287 | C₃₁ H₃₇ N₅ O₄ | 544.5 | 7.7 | 100 | Ex. 19 |
| 387 | 2288 | C₂₉ H₃₆ N₆ O₄ | 533.3 | 9.0 | 100 | Ex. 19 |
| 388 | 2288 | C₃₀ H₃₉ N₆ O₄ | 547.4 | 9.4 | 99 | Ex. 19 |
| 389 | 2290 | C₂₇ H₃₁ N₅ O₃ | 474.5 | 7.5 | 100 | Ex. 19 |
| 390 | 2291 | C₂₆ H₃₂ N₆ O₃ | 477.4 | 9.5 | 100 | Ex. 19 |
| 391 | 2296 | C₂₅ H₂₅ N₅ O₂ | 428.3 | 5.9 | 96 | Ex. 26 |
| 392 | 2298 | C₂₄ H₂₂ F N₅ O₂ | 432.3 | 5.8 | 95 | Ex. 26 |
| 393 | 2299 | C₁₉ H₂₃ N₅ O₂ | 354.4 | 4.6 | 100 | Ex. 15 |
| 394 | 2300 | C₁₈ H₁₉ N₅ O₄ | 370.3 | 2.2 | 100 | Ex. 15 |
| 395 | 2301 | C₁₈ H₂₁ N₅ O₃ | 356.4 | 2.5 | 100 | Ex. 15 |
| 396 | 2305 | C₂₅ H₂₄ F N₅ O₂ | 446.3 | 5.9 | 98 | Ex. 26 |
| 397 | 2311 | C₂₆ H₂₄ F₃ N₅ O₂ | 496.3 | 7.1 | 98 | Ex. 26 |
| 398 | 2325 | C₂₆ H₂₄ F₃ N₅ O₃ | 512.3 | 7.3 | 98 | Ex. 26 |
| 399 | 2329 | C₂₆ H₂₇ N₅ O₂ | 442.4 | 6.3 | 98 | Ex. 26 |
| 400 | 2336 | C₂₇ H₂₅ N₅ O₂ S | 484.3 | 7.3 | 98 | Ex. 19 |
| 401 | 2337 | C₂₆ H₂₆ N₆ O₂ S | 487.3 | 8.7 | 100 | Ex. 19 |
| 402 | 2340 | C₂₄ H₂₆ N₆ O₂ | 431.4 | 7.4 | 100 | Ex. 19 |
| 403 | 2341 | C₂₆ H₂₇ N₅ O₂ | 442.5 | 6.4 | 100 | Ex. 19 |
| | | | | 6.6 | | |
| 404 | 2342 | C₃₁ H₂₉ N₅ O₂ | 504.4 | 7.7 | 100 | Ex. 19 |
| | | | | 7.9 | | |
| 405 | 2343 | C₃₂ H₂₉ N₅ O₃ | 532.4 | 8.0 | 100 | Ex. 19 |
| 406 | 2344 | C₃₁ H₃₁ N₅ O₄ | 538.5 | 6.9 | 100 | Ex. 19 |
| 407 | 2347 | C₂₇ H₂₅ N₅ O₃ | 468.3 | 7.1 | 98 | Ex. 19 |
| 408 | 2348 | C₂₆ H₂₆ N₆ O₃ | 471.3 | 8.4 | 100 | Ex. 19 |
| 409 | 2350 | C₂₃ H₂₃ N₅ O₃ | 418.3 | 5.1 | 98 | Ex. 26 |
| 410 | 2356 | C₂₆ H₂₇ N₅ O₂ | 442.2 | 7.1 | 87 | Ex. 26 |
| 411 | 2357 | C₂₅ H₂₈ N₆ O₂ | 445.3 | 7.8 | 100 | Ex. 26 |
| ref 80 | 2446 | C₁₇ H₁₁ Cl N₄ O | 323.2 | 4.5 | 100 | Ex. 19 |
| ref 81 | 2448 | C₁₇ H₁₂ N₄ O₂ | 305.3 | 2.5 | 100 | Ex. 19 |
| ref 82 | 2452 | C₁₈ H₁₄ N₄ O₂ | 319.3 | 4.2 | 100 | Ex. 19 |
| ref 83 | 2459 | C₁₈ H₁₄ N₄ O₂ | 319.3 | 2.9 | 100 | Ex. 19 |
| ref 84 | 2469 | C₁₆ H₁₃ N₅ O | 292.2 | 2.9 | 100 | Ex. 19 |
| ref 85 | 2471 | C₁₅ H₁₀ N₄ O S | 295.3 | 3.2 | 100 | Ex. 19 |
| ref 86 | 2481 | C₁₅ H₁₀ N₄ O₂ | 279.3 | 2.5 | 100 | Ex. 19 |
| ref 87 | 2484 | C₁₇ H₁₀ Cl₂ N₄ O | 357.0 | 5.5 | 100 | Ex. 19 |
| ref 88 | 2486 | C₁₉ H₁₆ N₄ O₃ | 349.2 | 4.1 | 100 | Ex. 19 |
| ref 89 | 2490 | C₁₈ H₁₅ N₅ O₃ S | 382.3 | 4.0 | 100 | Ex. 19 |
| ref 90 | 2492 | C₁₈ H₁₂ N₄ O₃ | 333.3 | 2.7 | 100 | Ex. 19 |
| ref 91 | 2495 | C₁₉ H₁₃ N₅ O | 328.3 | 5.1 | 100 | Ex. 19 |
| ref 92 | 2500 | C₂₆ H₂₂ N₄ O₃ | 439.0 | 7.5 | 94 | Ex. 19 |
| 412 | 1562 | C₂₄ H₂₇ N₅ O₂ | 418.5 | 5.7 | 99 | Ex. 8 |
| 413 | 1895 | C₂₅ H₂₈ N₆ O₂ | 445.5 | 5.7 | 99 | Ex. 8 |
| 414 | 1875 | C₂₁ H₁₉ F₆ N₅ O₂ | 488.3 | 6.5 | 99 | Ex. 8 |
| 415 | 2514 | C₃₀ H₃₁ N₅ O₃ | 510.5 | 6.6 | 99 | Ex. 8 |

### Example 416.

¹H-NMR(400 MHz, DMSO-d₆ or CDCl₃) of the compounds according to the present invention were measured. Data of chemical shift values (δ: ppm) and coupling constant (J: Hz) are shown in Table 3. Compound numbers in Table 3 designate compounds in Table 1 listed as preferred specific examples, and example numbers in the table denote examples of corresponding synthesized compounds

| Ex. no. | Com-pound no. | NMR data δ(ppm) | Solvent |
|---|---|---|---|
| 34 | 27 | 1.28-1.44(m, 2H), 1.76(m, 2H), 2.76(m, 1H), 2.84-3.01(m, 2H), 3.15(m, 1H), 3.68(m, 1H), 3.87(d, J=12.96, 1H), 4.39(d, J=11.96, 1H), 4.62(m, 2H), 7.52(m, 2H), 7.63(m, 1H), 7.79(dd, J=5.38, J=8.06, 1H), 7.97(m, 3H), 8.24(s, 1H), 8.59(d, J=5.36, 1H), 8.78(d, J=8.32, 1H), 9.37(s, 1H), 12.24(brs, 1H). | DMSO-d6 |
| 35 | 29 | 0.00(m, 2H), 0.25(m, 2H), 2.28(m, 1H), 2.37(t, J=6.70, 2H), 4.34(t, J=6.60, 2H), 7.58(dd, J=5.50, J=7.94, 1H), 7.69(d, J=3.92, 1H), 7.73(s, 1H), 7.86(s, 1H), 8.35(d, J=5.36, 1H), 8.58(d, J=8.28, 1H), 9.14(s, 1H), 12.02(brs, 1H). | DMSO-d6 |
| ref 53 | 80 | 3.96(m, 2H), 4.49(m, 1H), 4.88(m, 1H), 7.11(m, 1H), 7.73-7.79(m, 2H), 7.83(m, 1H), 7.88(brs, 1H), 8.03(t, J=7.7, 1H), 8.18(d, J=8.5, 1H), 8.64(s, 1H), 10.00(brs, 1H), 12.29(brs, 1H). | DMSO-d6 |
| ref 68 | 1106 | 1.44(rs, 2H), 1.62(brs, 4H), 3.45(m, 4H), 5.39(s, 2H), 7.81(dd, J=5.48, J=8.16, 1H), 7.98(s, 1H), 8.09(s, 1H), 8.60(d, J=5.90, 1H), 8.80(d, J=8.04, 1H), 9.39(s, 1H), 12.18(brs, 1H). | DMSO-d6 |
| ref 72 | 1161 | 5.36(s, 2H), 7.05(m, 1H), 7.31(t, J=7.56, 2H), 7.58(d, J=7.56, 2H), 7.81(dd, J=5.24, J=8.16, 1H), 8.00(s, 1H), 8.18(s, 1H), 8.61(d, J=5.36, 1H), 8.80(d, J=8.04, 1H), 9.41(s, 1H), 10.37(s, 1H), 12.26(brs, 1H). | DMSO-d6 |

| | | | |
|---|---|---|---|
| ref 74 | 1170 | 1.19-1.23(m, 3H), 4.13-4.20(m, 2H), 5.30(s, 2H), 7.79(m, 1H), 8.00(d, J=3.92, 1H), 8.15(d, J=4.16, 1H), 8.60(m, 1H), 8.76(d, J=8.07, 1H), 9.37 (s, 1H), 12.28 (brs, 1H). | DMSO-d6 |
| ref 75 | 1174 | 4.06(s, 3H), 7.84(dd, J=5.36, J=8.03, 1H), 7.96(s, 1H), 8.16(s, 1H), 8.61(d, J=5.12, 1H), 8.84(d, J=7.75, 1H), 9.39(s, 1H), 12.21(brs, 1H). | DMSO-d6 |
| ref 78 | 1179 | 5.67(s, 2H), 7.13-7.36(m, 5H), 7.80(dd, J=5.35, J=8.03, 1H), 8.00(s, 1H), 8.36(s, 1H), 8.60(d, J=5.36, 1H), 8.83(d, J=8.07, 1H), 9.40(s, 1H), 12.29(brs, 2H). | DMSO-d6 |

### Example 417. Determination of inhibition of GSK-3 activity

The reaction was initiated by adding 25µL of phospho-glycogen synthase peptide-2 substrate solution [containing 6 µM phospho-glycogen synthase peptide-2, 20 µM ATP, 16 mM MOPS buffer (pH 7.0), 0.2 mM EDTA, 20 mM magnesium acetate, 0.1 µ Ci[γ-³³P]ATP(relative activity: approximately 110 TBq/mmol)] to 5µL of each test compound using 5% dimethylsulfoxide as a solvent, and further adding 20 µL of GSK-3β enzyme solution [containing 10 mU recombinant human GSK-3β, 20 mM MOPS buffer (pH 7.0), 1 mM EDTA, 0.1% polyoxyethylene lauryl ether(23 Lauryl Ether; Brij 35), 5% glycerol, and 0.1% β-mercaptoethanol. After 20 minutes at room temperature, the reaction was terminated by the addition of the equivalent amount of 200 mM phosphoric acid solution. 90 µL of the reaction product was spotted onto a multiscreen PH plate (manufactured by Millipore) and washed with 100 mM phosphoric acid solution. The plate was dried, and 30 µL of MicroScint-O (manufactured by Packard BioScience) was added thereto. To evaluate inhibitory activity, cpm was counted using a scintillation counter. Here, Phospho GS Peptide 2 is an amino acid peptide having the following sequence: Tyr-Arg-Arg-Ala-Ala-Val-Pro-Pro-Ser-Pro-Ser-Leu-Ser-Arg-His-Ser-Ser-Pro-His-Gln-Ser(P)-Glu-Asp-Glu-Glu-Glu.

GSK-3 inhibitor activity values (IC₅₀ values) of the compounds according to the present invention were measured by the method described above. As a result, an inhibition activity of IC₅₀<100 nM was confirmed in compounds of compound numbers 263, 280, 287, 297, 615, 617, 618, 627, 629, 691, 668, 760, 785, 1014, 1183, 1206, 1216, 1217, 1228, 1237, 1243, 1250, 1257, 1271, 1286, 1287, 1288, 1293, 1296, 1374, 1399, 1405, 1415, 1423, 1427, 1430, 1445, 1494, 1514, 1549, 1553, 1566, 1589, 1600, 1647, 1654, 1660, 1661, 1679, 1693, 1695, 1715, 1721, 1890, 1900, 2007, 2008, 2010, 2011, 2012, 2013, 2014, 2015, 2017, 2022, 2026, 2031, 2032, 2033, 2034, 2036, 2040, 2042, 2046, 2049, 2053, 2054, 2058, 2062, 2063, 2064, 2065, 2067, 2068, 2069, 2070, 2071, 2072, 2073, 2078, 2080, 2083, 2085, 2086, 2095, 2097, 2098, 2102, 2103, 2105, 2107, 2212, 2224, 2311.

Also, an inhibition activity of 20 nM<IC₅₀<100 nM was confirmed in compounds of compound numbers 40, 217, 219, 230, 245, 269, 288, 303, 595, 596, 601, 602, 616, 626, 628, 634, 646, 657, 659, 664, 667, 668, 673, 689, 694, 696, 723, 733, 734, 741, 768, 770, 776, 793, 795, 801, 802, 803, 804, 805, 806, 807, 809, 810, 812, 915, 1182, 1186, 1236, 1260, 1277, 1278, 1282, 1284, 1335, 1337, 1338, 1341, 1364, 1413, 1482, 1486, 1490, 1519, 1520, 1533, 1554, 1558, 1623, 1673, 1682, 1691, 1696, 1700, 1719, 1739, 1748, 1751, 1753, 1758, 1778, 1806, 1808, 1846, 1849, 1852, 1855, 1865, 1868, 1885, 1894, 1899, 2004, 2005, 2006, 2016, 2018, 2019, 2020, 2021, 2024, 2025, 2027, 2029, 2052, 2056, 2060, 2081, 2087, 2089, 2094, 2099, 2100, 2101, 2146, 2147, 2148, 2161, 2175, 2183, 2184, 2210, 2211, 2240, 2241, 2265, 2266, 2275, 2296, 2298, 2305, 2325, 2329, 2331, 2332, 2336, 2337, 2341, 2342, 2347, 2348, 2350, *2448, 2486, 2492.
* for reference purposes

Also, an inhibition activity of 100 nM<IC₅₀<1 µM was confirmed in compounds of compound numbers 81, 84, 254, 257, *320, 321, 330, 348, 361, 492, 504, 536, 550, 605, 678, 684, 702, 704, 709, 713, 718, 724, 729, 808, 811, 917, 918, *946, 1001, 1002, 1007, 1008, *1178, 1181, 1184, 1185, 1258, 1259, 1261, 1262, 1297, 1331, 1333, 1334, 1511, 1522, 1530, 1535, 1697, 1804, 1805, 1823, 1833, 1843, 1860, 1861, 1891, 1913, 1915, 2003, 2023, 2028, 2093, 2096, 2109, 2116, 2158, 2162, 2164, 2165, 2166, 2170, 2176, 2186, 2187, 2188, 2202, 2209, 2264, 2267, 2268, 2269, 2270, 2271, 2272, 2277, 2281, 2282, 2288, 2290, 2291, 2292, 2299, 2300, 2340, 2344, 2345, 2356, 2357, *2445, *2446, *2452, *2459, *2469, *2471, *2481, *2484, *2490, *2495.

* for reference purposes

Compound numbers designate compounds in Table 1 listed as preferred specific examples.

As described above, the pyrrolopyrimidine derivatives according to the present invention exhibit strong inhibitory activity against GSK-3. Therefore, the pyrrolopyrimidine derivatives according to the present invention have been found to be inhibitors of GSK-3 activity to be used in prevention and/or treatment of various diseases associated with GSK-3, which are clinically applicable compounds.

### Example 418. Preparation of tablets

Tablets each comprising the following ingredients were prepared.

| | |
|---|---|
| Compound (Example 1) | 50 mg |
| Lactose | 230 mg |
| Potato starch | 80 mg |
| Polyvinylpyrrolidone | 11 mg |
| Magnesium stearate | 5 mg |

The compound of the present invention (the compound prepared in Example 1), lactose, and potato starch were mixed, homogenously wetted with 20% ethanol solution of polyvinylpyrrolidone, passed through a 20 mesh sieve, dried at 45°C, and passed through again a 15 mesh sieve to obtain granules. The thus obtained granules were mixed with magnesium stearate and compressed into tablets.

### Reference Example 93. Synthesis of N-{2-[4-chloro-6-(3-chloro(2-thienyl))-7-iodopyrrolo[3,2-d]pyrimidin-5-yl] ethyl}-2,2,2-trifluoroacetoamide

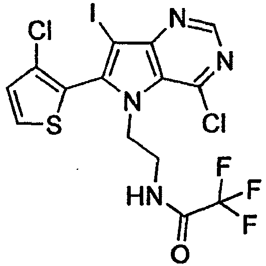

A phosphorus oxychloride (3.0 mL) solution of N-{2-[6- (3-chloro(2-thienyl))-7-iodo-4-oxo(3-hydropyrrolo[3,2-d]
pyrimidin-5-yl)]ethyl}-2,2,2-trifluoroacetoamide (333 mg) was stirred at 110°C for 2 hours. The reaction mixture was cooled to room temperature, and excess phosphorus oxychloride was distilled off under reduced pressure. A residue was dried in vacuo to obtain a crude product of the title compound as a brown oily compound. The product was not purified, but used for the following reaction. The ESI/MS data of the compound is given below.
ESI/MS m/e: 535.2 (M⁺+H, C₁₄H₈Cl₂F₃IN₄OS)

### Reference Example 94. Synthesis of N-{2-[7-bromo-4-chloro-6-(3-chloro(2-thienyl))pyrrolo[3,2-d]pyrimidin-5-yl]ethyl}-2,2,2-trifluoroacetoamide

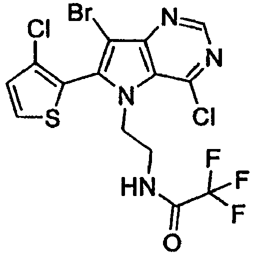

A crude product of the title compound was obtained by using N-{2-[7-promo-6-(3-chloro(2-thienyl))-4-oxo(3-hydropyrrolo[3,2-d]pyrimidin-5-yl)]ethyl}-2,2,2-trifluoroacetoamide (333 mg) in a similar manner to that in Reference Example 15. The ESI/MS data of the compound is given below.
ESI/MS m/e: 489.0 (M⁺+H, C₁₄H₈BrCl₂F₃N₄OS)

### Reference Example 95. Synthesis of N-{2-[4,7-dichloro-6-(3-chloro(2-thienyl))pyrrolo [3,2-d]pyrimidin-5-yl]ethyl}-2,2,2-trifluoroacetoamide

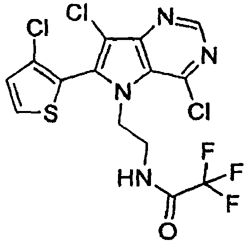

A crude product of the title compound was obtained by using N-{2-[7-chloro-6-(3-chloro(2-thienyl))-4-oxo(3-hydropyrrolo[3,2-d]pyrimidin-5-yl)]ethyl}-2,2,2-trifluoroacetoamide (333 mg) in a similar manner to that in Reference Example 15. The ESI/MS data of the compound is given below.
ESI/MS m/e: 443.4 (M⁺+H, C₁₄H₈Cl₃F₃N₄OS)

### INDUSTRIAL APPLICABILITY

The pyrrolopyrimidinone derivatives of Formula (I) according to the present invention and its pharmaceutically acceptable·salts have GSK-3 inhibitory activity and are used as effective ingredients of pharmaceutical products. Therefore, pharmaceutical agents containing these compounds as effective ingredients are expected as promising therapeutic drugs or preventive drugs in GSK-3 mediated diseases including diabetes, diabetic complications, Alzheimer's disease, neurodegenerative diseases manic depression, traumatic cerebrospinal injury, alopecia, inflammatory diseases, cancer and immunodeficiency.

## Claims

1. A compound represented by the formula (I) or its pharmaceutically acceptable salts: 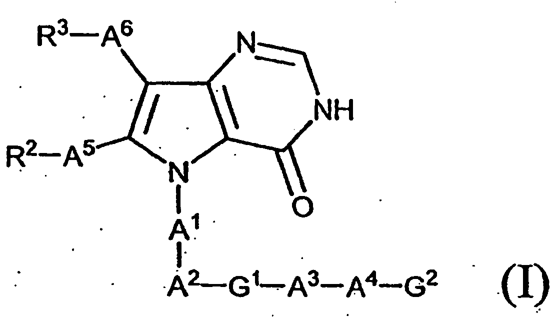 wherein A¹ is -(CH₂)₂- or -(CH₂)₃-;
A² represents a group that links A¹ with G¹ in the form of A¹ -C(=O)-G¹, A¹-C(=O)-O-G¹, A¹-C(=O)-NR¹⁰¹-G¹, A¹-C(=S)-NR¹⁰²-G¹, A¹-C(=NR¹⁰³)-G¹, A¹-O-G¹, A¹-O-C(=O)-G¹, A¹-NR¹⁰⁴-G¹, A¹-NR¹⁰⁵-C(=O)-G¹, A¹-NR¹⁰⁶-S(=O)₂-G¹, A¹-NR¹⁰⁷-C(=O)-O-G¹, A¹-NR¹⁰⁸-C(=O)-NR¹⁰⁹-G¹, A¹-NR¹¹⁰C(=S)-G¹, A¹-NR¹¹¹-C(=S)-NR¹¹²-G¹, A¹-C(=O)-NR¹⁰⁹-G¹, A¹-NR¹¹⁰C(=S)-G¹, A¹-NR¹¹¹-C(=S)-NR¹¹²-G¹, A¹-S-G¹, A¹-S(=O)-G¹, A¹-S(=O)₂-G¹, A¹-S(=O)₂-NR¹¹³-G¹, A¹-CR¹¹⁴=CH-G¹, A¹-CR¹¹⁵=CF-G¹, A¹-CH=CR¹¹⁶-G¹, or A¹-CF=CR¹¹⁷-G¹;
G¹ represents a single bond or represents a divalent group which is obtainable by removing two hydrogen atoms from any one of an optionally substituted alicyclic hydrocarbon having 3 to 10 carbon atoms, an optionally substituted aromatic hydrocarbon having 6 to 14 carbon atoms, and an optionally substituted heterocyclic compound having 1 to 4 atoms selected from the group consisting of an oxygen atom, a nitrogen atom and a sulfur atom, in the ring,
wherein the substituent for the substituted alicyclic hydrocarbon group having 3 to 10 carbon atoms of G¹ include: a fluorine atom, a chlorine atom, a bromine atom, an iodine atom, a hydroxy group, methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methyl- pentyloxy, 1-ethylbutoxy, cyclopropyloxy, cyclobutyloxy, cyclopentyloxy, cyclohexyloxy, cyclopropylmethyloxy, cyclopropylethyloxy, cyclopentylmethyloxy and cyclohexylmethyloxy or another C₁-C₇ alkoxy group consisting of a straight or branched alkyl, cycloalkyl and oxy group, ethylene dioxy or another C₁-C₄ alkylenedioxy group, phenoxy, 1-naphthoxy and 2-naphthoxy or another C₆-C₁₀ aryloxy group, bentyloxy, α-phenethyloxy, β-phenethyloxy and phenylpropyloxy or another C₇-C₉ aralkoxy group, acetoxy, propionyloxy, butyryloxy, isobutyryloxy, valeryloxy, isovaleryloxy, pivaloyloxy and hexanoyloxy or another C₂-C₇ acyloxy group, oxo, methylsulfonytoxy, ethylsulfonyloxy, propylsulfonyloxy, butylsulfonyloxy and t-butylsulfonyloxy or another C₁-C₆ alkylsulfonyloxy group consisting of a straight or branched alkyl and sulfonyloxy, acetyl, propionyl, butyryl, isobutyryl, valeryl, isovaleryl, pivaroyl and hexanoyl or another C₂₋C₇ acyl group, carboxy, methoxycarbonyl, ethoxycarbonyl, propoxycarbonyl, isopropoxycarbonyl, butoxycarbonyl, isobutoxycarbonyl, s-butoxycarbonyl and t-butoxycarbonyl or another C₂-C₇ alkoxycarbonyl group consisting of a straight or branched alkyl and oxycarbonyl group, carbamoyl, N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butycarbamoyl, Npentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl and N,N-dipropylcarbamoyl or another C₂-C₇ alkylcarbamoyl group consisting of a straight or branched alkyl, cycloalkyl and carbamoyl group, amino, methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutyl- amino, N-methyl-t-butylamino, N-ethylisopropylamino, dipropylamino, diisopropylamino and ethylbutylamino or another C₁-C₆ alkylamino group consisting of a straight or branched alkyl, cycloalkyl and amino group, acetylamino, propionylamino, butyrylamino, isobutyrylamlno, valerylamino and hexanoylamino or another C₂-C₇ acylamino group, methoxycarbonylamino, ethoxycarbonylamino and t-butoxycarbonylamino or another C₂-C₈ alkoxycarbonylamino group, methylsulfonylamino, ethylsulfonylamino, butylsulfonylamino and t-butylsulfonylamino or another C₁-C₆ alkylsulfonylamino group, a cyano group, a nitro group, methylthio, ethylthio, propylthio, isopropylthio, butylthio, isobutylthio, s-butylthio, t-butylthio, pentylthio and hexylthio or another C₁-C₃ alkylthio group, methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl and cyclopentylsulfynyl or another C₁-C₆ alkylsulfynyl group consisting of a straight or branched alkyl, cycloalkyl and sulfynyl group, methylsulfonyl, ethylsulfonyl, propylsulfonyl, isopropylsulfonyl, butyl- sulfonyl, isobutylsulfonyl, s-butylsulfonyl, t-butylsulfonyl, pentylsulfonyl, hexylsulfonyl, cyclopentylsulfonyl and cyclohexylsulfonyl or another C₁-C₆ alkylsulfonyl group consisting of a straight or branched alkyl, cycloalkyl and sulfonyl group, a sulfo group, a sulfamoyl group, methylaminosulfonyl, ethylaminosulfonyl, propylaminosulfonyl, isopropylaminosulfonyl, butylaminosulfonyl, isobutylamino- sulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl and cyclopropylmethylaminosulfonyl or another C₁-C₆ aminosulfonyl group consisting of a straight or branched alkyl, a cycloalkyl and aminosulfonyl group, cyclopropyl, cyclobutyl, cyclopentyl and cyclohexyl or another alicyclic hydrocarbon group having 3 to 6 carbon atoms, methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2- propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethyl-butyl or another aliphatic hydrocarbon group having 1 to 6 carbon atoms which may contain a straight or branched unsaturated bond;
wherein the substituent of the substituted alicyclic hydrocarbon group having 3 to 10 carbon atoms as G¹, a C₁₋C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms or an aliphatic hydrocarbon group having 1 to 6 carbon atoms, may furtherbe substituted with one or more substituents selected from the group consisting of a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy orcyclopropyloxy; a C₂-C₇ acyl group such as methoxymethyloxy group, 2-methoxyethoxy group, formyl group, trifluoroacetyl group, acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a C₁-C₆ alkylamino group such as methylamino, ethylamino, propylamino, isopropylamino, dimethylamino, N-ethylmethylamino, diethylamino, N-methyl- propylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a C₁-C₇ acylamino group such as trifluoroacetylamino group, formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; and a cyano group;
wherein substituents of the substituted aromatic hydrocarbon group having 6 to 14 carbon atoms of G¹ include a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms and an optionally substituted aliphatic hydrocarbon group having 1 to 6 carbon atoms;
wherein the substituent of the substituted aromatic hydrocarbon group having 6 to 14 carbon atoms, the C₁-C₇ alkoxy group, the C₂-C₇ acyl group, the C₂-C₇ alkylcarbamoyl group, the C₁-C₆ alkylamino group, the C₂-C₇ acylamino group, the alicyclic hydrocarbon group having 3 to 6 carbon atoms or the aliphatic hydrocarbon group having 1 to 6 carbon atoms, may further be substituted with one or more substituents selected from the group consisting of a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; and a cyano group;
wherein the substituent of the heterocyclic group having 1 to 4 atoms selected from the group consisting of an oxygen atom, a nitrogen atom and a sulfur atom on its substituted ring of G¹, include a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, and an optionally substituted aliphatic hydrocarbon group having 1 to 6 carbon atoms;
wherein the substituent of the heterocyclic group having 1 to 4 atoms selected from the group consisting of an oxygen atom, a nitrogen atom and a sulfur atom on its substituted ring of G¹, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms and aliphatic hydrocarbon group having 1 to 6 carbon atoms may further be substituted with one or more substituents selected from the group consisting of a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropyl-carbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; and a cyano group;
A³ represents a single bond or represents an optionally substituted divalent acyclic aliphatic hydrocarbon group having 1 to 10 carbon atoms that links G¹ with A⁴ on the same or different carbon atom;
wherein the substituents of divalent substituted aliphatic hydrocarbon group having 1 to 10 carbon atoms of A³ Include a hydrocarbon group having 1 to 6 carbon atoms, an alicyclic hydrocarbon group having 3 to 6 carbon atoms, a halogen atom, an alkoxy group having 1 to 6 carbon atoms, a phenoxy group, an amino group, or an alkyl amino group having 1 to 6 carbon atoms;
A⁴ represents a single bond or represents a group that links A³ with G² in the form of A³ -C(=O)-G², A³-C(=O)-O-G², A³-C(=O)-NR¹²¹-G², A³-C(=S)-NR¹²²-G², A³-C(=NR¹²³)-G², A³-O-G², A³-O-C(=O)-G², A³-NR¹²⁴-G², A³-NR¹²⁵-C(=O)-G², A³-NR¹²⁶-S(=O)₂-G², A³-NR¹²¹-C(=O)-O-G², A³-NR¹²⁸-C(=O)-NR¹²⁹-G², A³-NR¹³⁰-C(=S)-G², A³-NR¹³¹-C(=S)-NR¹³²-G², A³-S-G², A³-S(=O)-G², A³-S(=O)₂-G², A³-S(=O)₂-NR¹³³-G² or A³-S(=O)₂-O-G²;
G² is a hydrogen atom, an optionally substituted acyclic aliphatic hydrocarbon group having 1 to 10 carbon atoms, an optionally substituted alicyclic hydrocarbon group having 3 to 10 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
wherein substituents of the substituted acyclic aliphatic hydrocarbon group having 1 to 10 carbon atoms of G² include: a fluorine atom; a chlorine atom; a bromine atom; an iodine atom; a hydroxy group; a C₁-C₇ alkoxy group consisting of a straight or branched alkyl group, cycloalkyl group and oxy group, including methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methylpentyloxy, 1-ethylbutoxy, cyclopropyloxy, cyclobutyloxy, cyclopentyloxy, cyclohexyloxy, cyclopropylmethyloxy, cyclopropylethyloxy, cyclopentyl methyloxy and cyclohexylmethyloxy; an alkyldioxy group having 1 to 4 carbon atoms such as ethylene dioxy; a C₆-C₁₀ aryloxy group, including phenoxy, 1-naphthoxy and 2-naphthoxy; a C₇-C₉ aralkoxy group, including benzyloxy, α-phenethyloxy, β-phenethyloxy and phenylpropyloxy; a C₂-C₇ acyloxy group including acetoxy, propionyloxy, butyryloxy, isobutyryloxy, valeryloxy, isovaleryloxy, pivaloyloxy and hexanoyloxy; an oxo group; a C₁-C₆ alkylsulfonyloxy group consisting of a straight or branched alkyl and sulfonyloxy, including oxo, methyl-sulfonyloxy, ethylsulfonyloxy, propylsulfonyloxy, butylsulfonyloxy and t-butylsulfonyloxy: a C₂-C₇ acyl group, including acetyl, proplonyl, butyryl, isobutyryl, valeryl, isovaleryl, pivaroyl and hexanoyl; a carboxyl group; a C₂-C₇ alkoxycarbonyl group consisting of a straight or branched alkyl and oxycarbonyl group, including methoxycarbonyl, ethoxycarbonyl, propoxycarbonyl, isopropoxycarbonyl, butoxycarbonyl, isobutoxycarbonyl, s-butoxycarbonyl and t-butoxycarbonyl; a carbamoyl group; a C₂-C₇ alkylcarbamoyl group consisting of a straight or branched alkyl, cycloalkyl and carbamoyl group, including N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl and N,N-propylcarbamoyl; an amino group; a C₁-C₆ alkylamino group consisting of a straight or branched alkyl, cycloalkyl and amino group, including methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methyl-butylamino, N-methyl-t-butylamino, N-ethylisopropyl- amino, dipropylamino, diisopropylamino and ethylbutylamino; a C₂-C₇ acylamino group including acetylamino, propionylamino, butyrylamino, isobutyrylamino, valerylamino and hexanoylamino; a C₂-C₈ alkoxycarbonylamino group, including methoxycarbonylamino, ethoxycarbonylamino and t-butoxycarbonylamino; a C₁-C₆ alkylsulfonylamino group including methylsulfonylamino, ethylsulfonylamino, butylsulfonylamino and t-butylsulfonylamino; a cyano group; a nitro group; a C₁-C₆ alkylthio group including methylthio, ethylthio, propylthio, isopropylthio, butylthio, isobutylthio, s-butylthio, t-butylthio, pentylthio and hexylthio; a C₁-C₆ alkylsulfynyl group consisting of a straight or branched alkyl, cycloalkyl and sulfynyl group, including methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl and cyclopentylsulfynyl; a C₁-C₆ alkylsulfonyl group consisting of a straight or branched alkyl, cycloalkyl and sulfonyl group, including methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, cyclopentylsulfonyl and cyclohexylsulfonyl; a sulfo group; a sulfamoyl group; a C₁-C₆ aminosulfonyl group consisting of a straight or branched alkyl, cycloalkyl and aminosulfonyl group, including methylaminosulfonyl, ethylaminosulfonyl, propylaminosulfonyl, isopropylaminosulfonyl, butylaminosulfonyl, isobutyl- aminosulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl and cyclopropylmethylaminosulfonyl; an alicyclic hydrocarbon group having 3 to 6 carbon atoms, including cyclopropyl, cyclobutyl, cyclopentyl and cyclohexyl; and an aliphatic hydrocarbon group having 1 to 6 carbon atoms which may contain a straight or branched unsaturated bond, including methyl, ethyl, vinyl, ethynyl, propyl, 1-propenyl, 2-propenyl, isopropyl, isopropenyl, 1-propynyl, 2-propynyl, butyl, isobutyl, s-butyl, t-butyl, 1-butenyl, 2-butenyl, 3-butenyl, 1-methyl-1-propenyl, 2-methyl-1-propenyl,1-methyl-2-propenyl, 2-methyl-2-propenyl,1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl and 1-ethylbutyl; an aromatic hydrocarbon group having 6 to 14 carbon atoms which is a monovalent group derived from monocyclic, bicyclic or tricyclic aromatic hydrocarbon group, including benzene, naphthalene, indene, indane, 1,2,3,4-tetrahydronaphthalene, and fluorene; and a monovalent group derived from monocyclic, bicyclic or tricyclic heterocyclic compound, including furan, thiophene, pyrrole, pyrroline, pyrrolidine, oxazole, oxazolidine, isooxazole, isooxazolidine, thiazole, thiazolidine, isothiazole, isothiazolidine, imidazole, imidazoline, imidazolidine, pyrazole, pyrazoline, pyrazolidine, triazole, thiadiazole, oxadiazole, tetrazole, pyran, tetrahydropyran, thiopyran, tetrahydrothiopyran, pyridine, pyrazine, pyrimidine, pyridazine, benzofuran, dibenzofuran, benzothiophene, indole, benzimidazole, benzothiazole, benzooxazole, chroman, isochroman, quinoline, decahydroquinoline, isoquinoline, quinazolin, quinoxaline, purine, pteridine, azetidine, morpholine, thiomorpholine, piperidine, homopiperidine, piperazine, homopiperazine, indoline, isoindoline, phenoxazine, phenazine, phenothiazine and quinuclidine, the heterocyclic compound having 1 to 4 atoms selected from the group consisting of an oxygen atom, a nitrogen atom and a sulfur atom,in the ring;
wherein the substituent of the substituted acyclic aliphatic hydrocarbon group having 1 to 10 carbon atoms as G², a C₁-C₇ alkoxy group, a C₂-C₇ acyl group, C₂-C₇ alkylcarbamoyl, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, aromatic hydrocarbon group having 6 to 14 carbon atoms, and heterocyclic group having 1 to 4 atoms selected from the group consisting of an oxygen atom, a nitrogen atom and a sulfur atom, In the ring, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group;
a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl groupsuch as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropyl-methylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
wherein substituents of the substituted alicyclic hydrocarbon group having 3 to 10 carbon atoms of G² include a fluorine atom, a chlorine atom, a bromine atom, an iodine atom, a hydroxy group, an optionally substituted C₁-C₇ alkoxy group, C₁-C₄ alkylenedioxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, and an optionally substituted aliphatic hydrocarbon group having 1 to 6 carbon atoms, an optionally substituted aromatic hydrocarbon group having 6 to 14 carbon atoms;
wherein the substituent of the substituted alicyclic hydrocarbon group having 3 to 10 carbon atoms of G², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, and an aromatic hydrocarbon group having 6 to 14 carbon atoms, mayfurtherbe substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl groupsuch as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino, a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
wherein substituents of the aromatic hydrocarbon group having 6 to 14 carbon atoms of G² include at least one substituent selected from the group consisting of a fluorine atom, a chlorine atom, a bromine atom, an iodine atom, a hydroxy group, an optionally substituted C₁-C₇ alkoxy group, a C₁-C₄ alkylenedioxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosuffonyl group, a sulfo group, an optionally substituted alicyclic hydrocarbon group having 3 to 6 carbon atoms, an optionally substituted aliphatic hydrocarbon group having 1 to 6 carbon atoms, and an optionally substituted aromatic hydrocarbon group having 6 to 14 carbon atoms;
wherein the substituents of the aromatic hydrocarbon group having 6 to 14 carbon atoms of G², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, and an aromatic hydrocarbon group having 6 to 14 carbon atoms, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
wherein substituents of the heterocyclic group having 1 to 4 atoms selected from the group consisting of an oxygen atom, a nitrogen atom and a sulfur atom on its substituted ring of G² include at least one substituent selected from the group consisting of a fluorine atom, a chlorine atom, a bromine atom, an iodine atom, a hydroxy group, an optionally substituted C₁-C₇ alkoxy group, a C₁-C₄ alkylenedioxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, and an optionally substituted aromatic hydrocarbon group having 6 to 14 carbon atoms;
wherein the substituent of the heterocyclic group having 1 to 4 atoms selected from the group consisting of an oxygen atom, a nitrogen atom and a sulfur atom on its substituted ring of G², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, and an aromatic hydrocarbon group having 6 to 14 carbon atoms, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropyl- carbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
A⁵ represents a single bond or -NR²⁰¹-;
R² is a hydrogen atom, a fluorine atom, a chlorine atom, a bromine atom, an iodine atom, an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
wherein the substituents for the substituted acyclic aliphatic hydrocarbon group having 1 to 10 carbon atoms of R² include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 1 to 6 carbon atoms, an optionally substituted cyclic aliphatic hydrocarbon group having 1 to 6 carbon atoms, an optionally substituted aromatic hydrocarbon group having 6 to 14 carbon atoms, and an optionally substituted heterocyclic group having 1 to 4 atoms selected from the group consisting of an oxygen atom, a nitrogen atom and a sulfur atom, in the ring;
wherein the substituent of substituted acyclic aliphatic hydrocarbon group having 1 to 10 carbon atoms as R², a C₁-C₇ alkoxy group, a C₂-C₇ acyl group, C₂-C₇ alkylcarbamoyl group, a C₁-C₆ alkylamino group, a C₂-C₇ acylamino group, an alicyclic hydrocarbon group having 3 to 6 carbon atoms, a acyclic aliphatic hydrocarbon group having 1 to 6 carbon atoms, aromatic hydrocarbon group having 6 to 14 carbon atoms, and heterocyclic group having 1 to 4 atoms selected from the group consisting of an oxygen atom, a nitrogen atom and a sulfur atom, in the ring, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group;
a formyl group; a trifluoroacetyl group; a C₂-C₇ acyl groupsuch as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
wherein substituents of the substituted alicyclic hydrocarbon group having 3 to 8 carbon atoms of R² include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₈ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group having 1 to 4 atoms selected from the group consisting of an oxygen atom, a nitrogen atom and a sulfur atom, in the ring;
wherein the substituent of the substituted alicyclic hydrocarbon group having 3 to 8 carbon atoms of R², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and a heterocyclic group having 1 to 4 atoms selected from the group consisting of an oxygen atom, a nitrogen atom and a sulfur atom, in the ring, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl ort-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl-carbamoyl, N-propylcarbamoyl, N-isopropylcarbamoyl, N-butylcarbamoyl, N-cyclopropylcarbamoyl or N-cyclopropyl-methylcarbamoyl; an amino group; a C₁-C₆ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a C₄-C₆ cyclic amino group having 1 or 2 atoms selected from the group consisting of an oxygen atom, a nitrogen atom and a sulfur atom, In the ring, such as 1-pyrrolidinyl, piperazinyl, 4-methylpiperazinyl, piperidino or morpholino; a trifluoroacetylamino group; a C₁-C₇ acylamino group such as formylamino, acetylamino, proplonylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group, and a trifluoromethoxy group;
wherein substituents of the substituted aromatic hydrocarbon group having 6 to 14 carbon atoms include at least one substituent selected from the group consisting of a fluorine atom, a chlorine atom, a bromine atom, an iodine atom, a hydroxy group, a C₁-C₆ alkyl group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group having 1 to 4 atoms selected from the group consisting of an oxygen atom, a nitrogen atom and a sulfur atom, in the ring;
wherein the substituent of the aromatic hydrocarbon group having 6 to 14 carbon atoms of R², a a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and heterocyclic group (having 1 to 4 atoms selected from the group consisting of an oxygen atom, a nitrogen atom and a sulfur atom, in the ring), may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N,N-dimethylcarbamoyl, N-ethylcarbamoyl, N-ethyl-N-methylcarbamoyl, N,N-diethyl- carbamoyl, N-propylcarbamoyl, N-isopropylcarbamoyl, N-butylcarbamoyl, N-cyclopropylcarbamoyl or N-cyclopropyl- methylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, proplonylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group, and a trifluoromethoxy group;
wherein substituents of the heterocyclic group having 1 to 4 atoms selected from the group consisting of an oxygen atom, a nitrogen atom and a sulfur atom on the substituted ring of R² include at least one substituent selected from the group consisting of a fluorine atom, a chlorine atom, a bromine atom, an iodine atom, a hydroxy group, an optionally substituted C₁-C₇ alkoxy group, a C₆₋C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms, and an optionally substituted heterocyclic group having 1 to 4 atoms selected from the group consisting of an oxygen atom, a nitrogen atom and a sulfur atom, in the ring;
wherein the substituent of the heterocycric group having 1 to 4 atoms selected from the group consisting of an oxygen atom, a nitrogen atom and a sulfur atom on its substituted ring of R², a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, aliphatic hydrocarbon group having 1 to 6 carbon atoms, an aromatic hydrocarbon group having 6 to 14 carbon atoms, and a heterocyclic group having 1 to 4 atoms selected from the group consisting of an oxygen atom, a nitrogen atom and a sulfur atom, in the ring may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N, N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
A⁶ represents a single bond;
R³ is an optionally substituted aromatic hydrocarbon group having 6 to 14 carbon atoms, or an optionally substituted heterocyclic group having 1 to 4 atoms selected from the group consisting of an oxygen atom, a nitrogen atom and a sulfur atom, in the ring;
wherein substituents of the substituted aromatic hydrocarbon group having 6 to 14 carbon atoms of R³ include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇ alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms and an optionally substituted heterocyclic group having 1 to 4 atoms selected from the group consisting of an oxygen atom, a nitrogen atom and a sulfur atom, in the ring;
wherein the substituent of the substituted aromatic hydrocarbon group having 6 to 14 carbon atoms of R³, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms, an aliphatic hydrocarbon group having 1 to 6 carbon atoms, an aromatic hydrocarbon group having 6 to 14 carbon atoms and a heterocyclic group having 1 to 4 atoms selected from the group consisting of an oxygen atom, a nitrogen atom and a sulfur atom, in the ring, may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl ort-butoxycarbonyl; a carbamoyl group; a C₂-C₇ alkylcarbamoyl groupsuch as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropyl-methylcarbamoyl; an amino group; a C₁-C₆ alkylamino group such as methylamino, ethylamino, propylamino, isopropylamino, dimethylamino, N-ethylmethyl- amino, diethylamino, N-methylpropylamino, N-methylisopropyl- amino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
wherein substituents of the heterocyclic group having 1 to 4 atoms selected from the group consisting of an oxygen atom, a nitrogen atom and a sulfur atom on the ring of R³ include at least one substituent selected from the group consisting of a fluorine atom, a chlorine atom, a bromine atom, an iodine atom, a hydroxy group, an optionally substituted C₁-C₇ alkoxy group, a C₆-C₁₀ aryloxy group, a C₇-C₉ aralkoxy group, a C₂-C₇ acyloxy group, an oxo group, a C₁-C₆ alkylsulfonyloxy group, an optionally substituted C₂-C₇ acyl group, a carboxyl group, a C₂-C₇ alkoxycarbonyl group, a carbamoyl group, an optionally substituted C₂-C₇alkylcarbamoyl group, an amino group, an optionally substituted C₁-C₆ alkylamino group, an optionally substituted C₂-C₇ acylamino group, a C₂-C₈ alkoxycarbonylamino group, a C₁-C₆ alkylsulfonylamino group, a cyano group, a nitro group, a C₁-C₆ alkylthio group, a C₁-C₆ alkylsulfynyl group, a C₁-C₆ alkylsulfonyl group, a sulfamoyl group, a C₁-C₆ alkylaminosulfonyl group, a sulfo group, an optionally substituted alicyclic hydrocarbon group having 3 to 6 carbon atoms, an optionally substituted aliphatic hydrocarbon group having 1 to 6 carbon atoms, an optionally substituted aromatic hydrocarbon group having 6 to 14 carbon atoms and an optionally substituted heterocyclic group having 1 to 4 atoms selected from the group consisting of an oxygen atom, a nitrogen atom and a sulfur atom, in the ring;
wherein the substituent of the heterocyclic group having 1 to 4 atoms selected from the group consisting of an oxygen atom, a nitrogen atom and a sulfur atom on its substituted ring of R³, a C₁-C₇ alkoxy group, a C₂-C₇ acyl group, a C₂-C₇ alkylcarbamoyl group, a C₁-C₆ alkylamino group, a C₂-C₇ acylamino group, an alicyclic hydrocarbon group having 3 to 6 carbon atoms and an aliphatic hydrocarbon group having 1 to 6 carbon atoms, an aromatic hydrocarbon group having 6 to 14 carbon atoms, and a heterocyclic group having 1 to 4 atoms selected from the group consisting of oxygen atom, a nitrogen atom and a sulfur atom in the ring may further be substituted with one or more substituents selected from the group consisting of: a fluorine atom; a chlorine atom; a bromine atom; an iodine atom; a hydroxy group; a C₁-C₆ alkoxy group such as methoxy, ethoxy, propoxy, isopropoxy, butoxy, isobutoxy, s-butoxy, t-butoxy, pentyloxy or cyclopropyloxy; a methoxymethyloxy group; a 2-methoxyethoxy group; a formyl group; a trifluoroacetyl group; a C₂-C₇ acyl group such as acetyl, propionyl, butyryl, isobutyryl, valeryl or isovaleryl; an oxo group; a carboxyl group; a C₂-C₇ alkoxycarbonyl group such as methoxycarbonyl, ethoxycarbonyl, propoxycarbonyl, isopropoxycarbonyl, butoxycarbonyl, isobutoxycarbonyl or t-butoxycarbonyl; a carbamoyl group; a C₂-C₇ alkylcarbamoyl group such as N-methylcarbamoyl, N, N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl or N-cyclopropylmethylcarbamoyl; an amino group; a C₁-C₆ alkylamino group such as methylamino, ethylamino, propylamino, isopropylamino, dimethylamino, N-ethylmethylamino, diethylamino, N-methylpropylamino, N-methylisopropylamino, cyclopropylamino or cyclopropylmethylamino; a C₄-C₆ cyclic amino group having 1 or 2 atoms selected from the group consisting of an oxygen atom, a nitrogen atom and a sulfur atom, in the ring, such as 1-pyrrolidinyl, piperazinyl, 4-methylpiperazinyl, piperidino or morpholino; a trifluoroacetylamino group; a C₁-C₇ acylamino group such as formylamino, acetylamino, propionylamino, butyrylamino, isobutyrylamino or valerylamino; a C₁-C₆ alkylsulfonylamino group such as methylsulfonylamino, ethylsulfonylamino, propylsulfonylamino or butylsulfonylamino; a nitro group; a cyano group; a C₁-C₆ alkyl group including methyl, ethyl, propyl, isopropyl, butyl, isobutyl, s-butyl, and t-butyl; a trifluoromethyl group; and a trifluoromethoxy group;
R¹⁰¹-R¹¹⁷, R¹²¹-R¹³³ and R²⁰¹ are each independently a hydrogen atom or an aliphatic hydrocarbon group having 1 to 4 carbon atoms;
with the proviso that when both of A¹ and A³ represent an acyclic aliphatic hydrocarbon group, then at least either one of A² or G¹ is not a single bond.

2. The compound or a pharmaceutically acceptable salt thereof according to claim 1, wherein A² represents -C(=O)-, -C(=O)-O-, -C(=O)-NH-, -C(=O)-NMe-, - NH-, -NH-C(=O)-, -NH-C(=O)-O-, -NH-C(=O)-NH-, -NH-C(=O)-NMe-, or -NH-C(=S)-.

3. The compound or a pharmaceutically acceptable salt thereof according to claim 1, wherein A² represents -C(=O)-NH-, -NH-, -NH-C(=O)-, -NH-C(=O)-O-, or -NH-C(=O)-NH-.

4. The compound or a pharmaceutically acceptable salt thereof according to any one of claims 1 to 3, wherein combination of G¹, A³, A⁴, and G² is any of the combinations of 1 to 10 in the following table.
| Combi-nation | G¹ | A³ | A⁴ | G² |
|---|---|---|---|---|
| 1 | Group other than single bond | Single bond | Single bond | Hydrogen atom |
| 2 | Single bond | Group other than single bond | Single bond | Hydrogen atom |
| 3 | Group other than single bond | Single bond | Single bond | Group other than hydrogen atom |
| 4 | Single bond | Group other than single bond | Single bond | Group other than hydrogen atom |
| 5 | Group other than single bond | Single bond | Group other than single bond | Group other than hydrogen atom |
| 6 | Single bond | Group other than single bond | Group other than single bond | Group other than hydrogen atom |
| 7 | Group other than single bond | Group other than single bond | Single bond | Group other than hydrogen atom |
| 8 | Group other than single bond | Group other than single bond | Group other than single bond | Group other than hydrogen atom |
| 9 | Group other than single bond | Group other than single bond | Group other than single bond | Hydrogen atom |
| 10 | Single bond | Single bond | Single bond | Hydrogen atom |

5. The compound or a pharmaceutically acceptable salt thereof according to any one of claims 1 to 3, wherein G¹ represents a group other than a single bond, A³ and A⁴ represent a single bond, and G² represents a hydrogen atom.

6. The compound or a pharmaceutically acceptable salt thereof according to any one of claims 1 to 3, wherein G¹ and A⁴ represent a single bond, A³ represents a group other than a single bond, and G² represents a hydrogen atom.

7. The compound or a pharmaceutically acceptable salt thereof according to any one of claims 1 to 3, wherein G¹ represents a group other than a single bond, A³ and A⁴ represent a single bond, and G² represents a group other than a hydrogen atom.

8. The compound or a pharmaceutically acceptable salt thereof according to any one of claims 1 to 3, wherein G¹ and A⁴ represent a single bond, A³ represents a group other than a single bond, and G² represents a group other than a hydrogen atom.

9. The compound or a pharmaceutically acceptable salt thereof according to claim 8, wherein A³ represent an alkylene group having 1 to 3 carbon atoms.

10. The compound or a pharmaceutically acceptable salt thereof according to any one of claims 1 to 3, wherein G¹ and A⁴ represent a group other than a single bond, A³ represents a single bond, and G² represents a group other than a hydrogen atom.

11. The compound or a pharmaceutically acceptable salt thereof according to claim 10, wherein A⁴ represents -C(=O)-, -C(=O)-NH-, -O-, or -NH-C(=O)-.

12. The compound or a pharmaceutically acceptable salt thereof according to any one of claims 1 to 3, wherein G¹ represents a single bond, A³ and A⁴ represent a group other than a single bond, and G² represents a group other than a hydrogen atom.

13. The compound or a pharmaceutically acceptable salt thereof according to any one of claims 1 to 3, wherein G¹ and A³ represent a group other than a single bond, A⁴ represents a single bond, and G² represents a group other than a hydrogen atom.

14. The compound or a pharmaceutically acceptable salt thereof according to claim 13, wherein A³ represent an alkylene group having 1 to 3 carbon atoms.

15. The compound or a pharmaceutically acceptable salt thereof according to any one of claims 1 to 3, wherein G¹, A³ and A⁴ represent a group other than a single bond, and G² represents a group other than a hydrogen atom.

16. The compound or a pharmaceutically acceptable salt thereof according to any one of claims 1 to 3, wherein A⁴ represents -O-.

17. The compound or a pharmaceutically acceptable salt thereof according to any one of claims 1 to 3, wherein G¹, A³ and A⁴ represent a group other than a single bond, and G² represents a hydrogen atom.

18. The compound or a pharmaceutically acceptable salt thereof according to any one of claims 1 to 3, wherein G¹, A³ and A⁴ represent a single bond, and G² represents a hydrogen atom.

19. The compound or a pharmaceutically acceptable salt thereof according to claim 1, wherein A² represents - NH-(C=O)- or -NH-(C=O)-NH-, G¹ represents a single bond, and A³ represents a divalent acyclic aliphatic hydrocarbon group having 1 to 10 carbon atoms.

20. The compound or a pharmaceutically acceptable salt thereof according to claim 1, wherein A² represents - NH-(C=O)-, -NH-(C=O)-NH-, -NH-, or -C(=O)-NH-, and G¹ represents a group other than a single bond.

21. The compound or a pharmaceutically acceptable salt thereof according to claim 1, wherein G¹ represents an optionally substituted heterocyclic group,
with the proviso that when the heterocyclic group of G¹ is 5 or 6 membered monocyclic ring, then the 5 or 6 membered monocyclic heterocyclic group of G¹ is substituted or the A³-G² portion represents those other than a hydrogen atom.

22. The compound or a pharmaceutically acceptable salt thereof according to any one of claims 2 or 3, wherein G¹ represents an optionally substituted aromatic hydrocarbon group, an optionally substituted alicyclic hydrocarbon group having 7 to 10 carbon atoms, or an optionally substituted heterocyclic group,
with the proviso that when the aromatic hydrocarbon group of G¹ is a phenyl group, or the heterocyclic group of G¹ is 5 or 6 membered monocyclic ring, then the phenyl group or the 5 or 6 membered monocyclic heterocyclic group of G¹ is substituted, or the A³-G² portion represents those other than a hydrogen atom.

23. The compound or a pharmaceutically acceptable salt thereof according to any one of claims 2 or 3, wherein G¹ and A⁴ represent a single bond, A³ represents an optionally substituted acyclic aliphatic hydrocarbon group having 1 to 10 carbon atoms, G² represents an optionally substituted alicyclic hydrocarbon group having 5 to 10 carbon atoms, an optionally substituted aromatic hydrocarbon group, or an optionally substituted heterocyclic group.

24. The compound or a pharmaceutically acceptable salt thereof according to any one of claims 2 or 3, wherein G¹ represents a single bond, A³ represents an optionally substituted acyclic aliphatic hydrocarbon group having 1 to 10 carbon atoms, and A⁴ represents - C(=O)-, -C(=O)-NR¹²¹-, -C(=S)-NR¹²²-, -C(=NR¹²³)-, -O-C(=O)-, -NR¹²⁵-C(=O) -, -NR¹²⁶-S(=O)₂-, -NR¹²⁷-C(=O)-O-, -NR¹²⁸-C(=O)-NR¹²⁹-, -NR¹³⁰-C(=S)-, -NR¹³¹-C(=S)-NR¹³²-, -S-, -S(=O)-, - S(=O)₂-, -S(=O)₂-NR¹³³-, or -S(=O)₂-O-.

25. The compound or a pharmaceutically acceptable salt thereof according to any one of claims 1 to 24, wherein A⁵ represents a single bond.

26. The compound or a pharmaceutically acceptable salt thereof according to claim 25, wherein R² represents an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group.

27. The compound or a pharmaceutically acceptable salt thereof according to claim 25, wherein R² represents an acyclic aliphatic hydrocarbon group having 1 to 10 carbon atoms, an alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom, or a sulfur atom, in the ring.

28. The compound or a pharmaceutically acceptable salt thereof according to claim 25, wherein R² represents a cyclopropyl group, cyclobutyl group, cyclopropylmethyl group, methyl group, ethyl group, vinyl group, isopropyl group, or 2-methyl-1-propenyl group.

29. The compound or a pharmaceutically acceptable salt thereof according to claim 25, wherein R² represents a thienyl group, a pyridyl group, a furyl group, a pyrrolyl group, a pyrazolyl group, or phenyl group; any of which may be further substituted by one or more of an alkyl group having 1 to 4 carbon atoms, an alkoxy group having 1 to 4 carbon atoms, an acyl group having 2 to 4 carbon atoms, a hydroxy group, a carboxyl group, an alkoxycarbonyl group having 2 to 7 carbon atoms, a fluorine atom, or a chlorine atom.

30. The compound or a pharmaceutically acceptable salt thereof according to claim 1, wherein R³ represents a thienyl group, a pyridyl group, a furyl group, a pyrrolyl group, a pyrazolyl group, or phenyl group; any of which may be further substituted by one or more of an alkyl group having 1 to 4 carbon atoms.

31. The compound or a pharmaceutically acceptable salt thereof according to claim 1, wherein R³ represents a pyridyl group or a 1-oxypyridyl group, either of which may be further substituted by one alkyl group having 1 to 4 carbon atoms or one halogen atom; a pyrazolyl group; or N-methylpyrazolyl group.

32. The compound or a pharmaceutically acceptable salt thereof according to claim 28, wherein R³ represents a pyridyl group or a 1-oxypyridyl group, either of which may be further substituted by one alkyl group having 1 to 4 carbon atoms or one halogen atom; a pyrazolyl group; or N-methylpyrazolyl group.

33. The compound or a pharmaceutically acceptable salt thereof according to claim 29, wherein R³ represents a pyridyl group or a 1-oxypyridyl group, either of which may be further substituted by one alkyl group having 1 to 4 carbon atoms or one halogen atom; a pyrazolyl group; or N-methylpyrazolyl group.

34. The compound or a pharmaceutically acceptable salt thereof according to any one of claims 19 to 24, wherein A⁵ represent a single bond.

35. The compound or a pharmaceutically acceptable salt thereof according to claim 34, wherein R² represents an optionally substituted acyclic aliphatic hydrocarbon group having 1 to 10 carbon atoms, an optionally substituted alicyclic hydrocarbon group having 3 to 8 carbon atoms, an optionally substituted aromatic hydrocarbon group having 6 to 14 carbon atoms, or an optionally substituted heterocyclic group, and R³ represents an optionally substituted aromatic hydrocarbon group having 6 to 14 carbon atoms or an optionally substituted heterocyclic group.

36. The compound or a pharmaceutically acceptable salt thereof according to claim 34, wherein R² represents an optionally substituted acyclic aliphatic hydrocarbon group having 1 to 10 carbon atoms, an alicyclic hydrocarbon group having 3 to 8 carbon atoms, an optionally substituted phenyl group, or an optionally substituted heterocyclic group having 1 or 2 atoms selected from the group consisting of an oxygen atom, a nitrogen atom, and a sulfur atom, in the ring, and R³ represents a thienyl group, a pyridyl group, a furyl group, a pyrrolyl group, a pyrazolyl group, or a phenyl group, any of which may be further substituted by one or more of a C₁-C₄ alkyl group.

37. The compound or a pharmaceutically acceptable salt thereof according to claim 34, wherein R² represents a cyclopropyl group, methyl group, ethyl group, vinyl group, isopropyl group, isobutyl group, or 2-methyl-1-propenyl group, and R³ represents a pyridyl group or a 1-oxypyridyl group, either of which may be further substituted by one alkyl group having 1 to 4 carbon atoms or one halogen atom; pyrazolyl group; or N-methylpyrazolyl group.

38. The compound or a pharmaceutically acceptable salt thereof according to claim 34, wherein R² represents a thienyl group, a pyridyl group, a furyl group, a pyrrolyl group, a pyrazolyl group, or a phenyl group, any of which may be further substituted by one or more of an alkyl group having 1 to 4 carbon atoms, an alkoxy group having 1 to 4 carbon atoms, or a chlorine atom, and R³ represents a pyridyl group or a 1-oxypyridyl group, either of which may be further substituted by one alkyl group having 1 to 4 carbon atoms or one halogen atom; pyrazolyl group; or N-methylpyrazolyl group.

39. A pharmaceutical composition comprising the compound or a pharmaceutically acceptable salt thereof according to any one of claims 1 to 38; and a pharmaceutically acceptable carrier.

40. A GSK-3 inhibitor comprising the compound or a pharmaceutically acceptable salt thereof according to any one of claims 1 to 38.

41. An agent for treating or preventing a GSK-3-mediated disease, comprising the compound or a pharmaceutically acceptable salt thereof according to any one of claims 1 to 38, for use in a method of treating or preventing a GSK-3-mediated disease selected from the group consisting of diabetes, diabetic complications, Alzheimer's disease, neurodegenerative disease, manic depressive psychosis, traumatic brain injury, alopecia, inflammatory disease, cancer, and immunodeficiency.

42. The use of an agent comprising the compound or a pharmaceutically acceptable salt thereof according to any one of claims 1 to 38 for the manufacture of a medicament for treating or preventing a GSK-3-mediated disease selected from the group consisting of diabetes, diabetic complications, Alzheimer's disease, neurodegenerative disease, manic depressive psychosis, traumatic brain injury, alopecia, inflammatory disease, cancer, and immunodeficiency.

43. A compound represented by the formula (Ic): 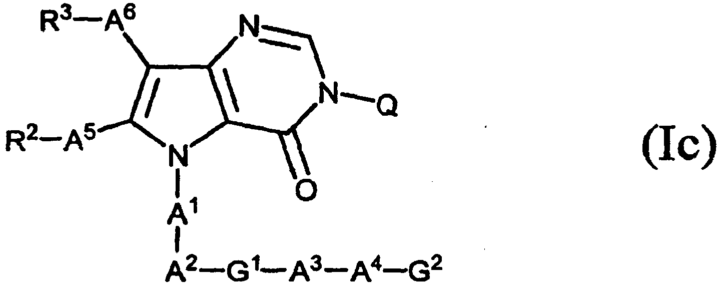 wherein A¹, A², A³, A⁴, A⁵, A⁶, G¹, G², R², R³, and X are as defined in the formula (I); and Q represents an optionally substituted acyl group having 2 to 10 carbon atoms, an optionally substituted alkoxymethyl group having 2 to 10 carbon atoms, or an optionally substituted benzyl group.

## Patentansprüche

1. Eine Verbindung der Formel (I) oder ihre pharmazeutisch verträglichen Salze: 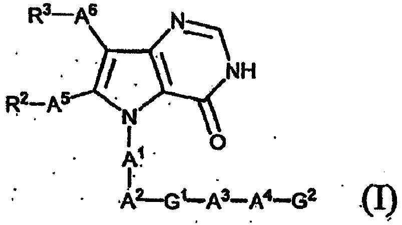 wobei
A¹ gleich -(CH₂)₂- oder -(CH₂)₃- ist;
A² einen Rest darstellt, welcher A¹ mit G¹ in der Form A¹-C(=O)-G¹, A¹-C(=O)-O-G¹, A¹-C(=O)-NR¹⁰¹-G¹, A¹-C(=S)-NR¹⁰²-G¹, A¹-C(=NR¹⁰³)-G¹, A¹-O-G¹, A¹-O-C(=O)-G¹, A¹-NR¹⁰⁴-G¹, A¹-NR¹⁰⁵-C(=O)-O¹, A¹-NR¹⁰⁶-S(=O)₂-G¹, A¹-NR¹⁰⁷-C(=O)-O-G¹, A¹-NR¹⁰⁸-C(=O)-NR¹⁰⁹-G¹, A¹-NR¹¹⁰C(=S)-G¹, A¹-NR¹¹¹-C(=S)-NR¹¹²-G¹, A¹-S-G¹, A¹-S(=O)-G¹, A¹-S(=O)₂-G¹, A¹-S(=O)₂-NR¹¹³-G¹, A¹-CR¹¹⁴=CH-G¹, A¹-CR¹¹⁵=CF-G¹, A¹-CH=CR¹¹⁶-G¹ oder A¹-CF=CR¹¹⁷-G¹ verbindet;
G¹ eine Einfachbindung oder einen zweiwertigen Rest darstellt, welcher durch Entfernen zweier Wasserstoffatome von einem gegebenenfalls substituierten alicyclischen Kohlenwasserstoff mit 3 bis 10 Kohlenstoffatomen, einem gegebenenfalls substituierten aromatischen Kohlenwasserstoff mit 6 bis 14 Kohlenstoffatomen und einer gegebenenfalls substituierten heterocyclischen Verbindung mit 1 bis 4 Atomen, ausgewählt aus der Gruppe, bestehend aus einem Sauerstoffatom, einem Stickstoffatom und einem Schwefelatom, im Ring erhältlich ist;
wobei der Substituent für den substituierten alicyclischen Kohlenwasserstoffrest mit 3 bis 10 Kohlenstoffatomen von G¹ umfasst: ein Fluoratom, ein Chloratom, ein Bromatom, ein Iodatom, eine Hydroxygruppe, Methoxy, Ethoxy, Propoxy, Isopropoxy, Butoxy, Isobutoxy, s-Butoxy, t-Butoxy, Pentyloxy, Isopentyloxy, Neopentyloxy, t-Pentyloxy, Hexyloxy, Isohexyloxy, 2-Methylpentyloxy, 1-Ethylbutoxy, Cyclopropyloxy, Cyclobutyloxy, Cyclopentyloxy, Cyclohexyloxy, Cyclopropylmethyloxy, Cyclopropylethyloxy, Cyclopentylmethyloxy und Cyclohexylmethyloxy oder einen anderen C₁-C₇-Alkoxyrest, bestehend aus einem geraden oder verzweigten Alkyl, Cycloalkyl und einer Oxygruppe, Ethylendioxy oder einen anderen C₁-C₄-Alkylendioxyrest, Phenoxy, 1-Naphthoxy und 2-Naphthoxy oder einen anderen C₆-C₁₀-Aryloxyrest, Benzyloxy, α-Phenethyloxy, β-Phenethyloxy und Phenylpropyloxy oder einen anderen C₇-C₉-Aralkoxyrest, Acetoxy, Propionyloxy, Butyryloxy, Isobutyryloxy, Valeryloxy, Isovaleryloxy, Pivaloyloxy und Hexanoyloxy oder einen anderen C₂-C₇-Acyloxyrest, Oxo, Methylsulfonyloxy, Ethylsulfonyloxy, Propylsulfonyloxy, Butylsulfonyloxy und t-Butylsulfonyloxy oder einen anderen C₁-C₆-Alkylsulfonyloxyrest, bestehend aus einem geraden oder verzweigten Alkyl und Sulfonyloxy, Acetyl, Propionyl, Butyryl, Isobutyryl, Valeryl, Isovaleryl, Pivaroyl und Hexanoyl oder einen anderen C₂-C₇-Acylrest, Carboxy, Methoxycarbonyl, Ethoxycarbonyl, Propoxycarbonyl, Isopropoxycarbonyl, Butoxycarbonyl, Isobutoxycarbonyl, s-Butoxycarbonyl und t-Butoxycarbonyl oder einen anderen C₂-C₇-Alkoxycarbonykest, bestehend aus einem geraden oder verzweigten Alkyl und einer Oxycarbonylgruppe, Carbamoyl, N-Methylcarbamoyl, N-Ethylcarbamoyl, N-Propylcarbamoyl, N-Isopropylcarbamoyl, N-Butylcarbamoyl, N-Isobutylcarbamoyl, N-s-Butylcarbamoyl, N-t-Butylcarbamoyl, N-Pentylcarbamoyl, N-Cyclopropylcarbamoyl, N-Cyclobutylcarbamoyl, N-Cyclopentylcarbamoyl, N-Cyclohexylcarbamoyl, N-Cycloheptylcarbamoyl, N-Cyclopropylmethylcarbamoyl, N,N-Dimethylcarbamoyl, N-Ethyl-N-methylcarbamoyl, N,N-Diethylcarbamoyl und N,N-Dipropylcarbamoyl oder einen anderen C₂-C₇-Alkylcarbamoylrest, bestehend aus einem geraden oder verzweigten Alkyl, Cycloalkyl und einer Carbamoylgruppe, Amino, Methylamino, Ethylamino, Propylamino, Isopropylamino, Butylamino, Isobutylamino, s-Butylamino, t-Butylamino, Pentylamino, Hexylamino, Cyclopropylamino, Cyclobutylamino, Cyclopentylamino, Cyclohexylamino, Cyclopropylmethylamino, Dimethylamino, N-Ethylmethylamino, Diethylamino, N-Methylpropylamino, N-Methylisopropylamino, N-Methylbutylamino, N-Methyl-t-butylamino, N-Ethylisopropylamino, Dipropylamino, Diisopropylamino und Ethylbutylamino oder einen anderen C₁-C₆-Alkylaminorest, bestehend aus einem geraden oder verzweigten Alkyl, Cycloalkyl und einer Aminogruppe, Acetylamino, Propionylamino, Butyrylamino, Isobutyrylamino, Valerylamino und Hexanoylamino oder einen anderen C₂-C₇-Acylaminorest, Methoxycarbonylamino, Ethoxycarbonylamino und t-Butoxycarbonylamino oder einen anderen C₂-C₈-Alkoxycarbonylaminorest, Methylsulfonylamino, Ethylsulfonylamino, Butylsulfonylamino und t-Butylsulfonylamino oder einen anderen C₁-C₆-Alkylsulfonylaminorest, eine Cyanogruppe, eine Nitrogruppe, Methylthio, Ethylthio, Propylthio, Isopropylthio, Butylthio, Isobutylthio, s-Butylthio, t-Butylthio, Pentylthio und Hexylthio oder einen anderen C₁-C₆-Alkylthiorest, Methylsulfinyl, Ethylsulfinyl, Propylsulfinyl, Isopropylsulfinyl, Butylsulfinyl, Isobutylsulfinyl, s-Butylsulfinyl, t-Butylsulfinyl, Pentylsulfinyl und Cyclopentylsulfinyl oder einen anderen C₁-C₆-Alkylsulfinylrest, bestehend aus einem geraden oder verzweigten Alkyl, Cycloalkyl und einer Sulfinylgruppe, Methylsulfonyl, Ethylsulfonyl, Propylsulfonyl, Isopropylsulfonyl, Butylsulfonyl, Isobutylsulfonyl, s-Butylsulfonyl, t-Butylsulfonyl, Pentylsulfonyl, Hexylsulfonyl, Cyclopentylsulfonyl und Cyclohexylsulfonyl oder einen anderen C₁-C₆-Alkylsulfonylrest, bestehend aus einem geraden oder verzweigten Alkyl, Cycloalkyl und einer Sulfonylgruppe, eine Sulfogruppe, ein Sulfamoylrest, Methylaminosulfonyl, Ethylaminosulfonyl, Propylaminosulfonyl, Isopropylaminosulfonyl, Butylaminosulfonyl, Isobutylaminosulfonyl, s-Butylaminosulfonyl, Pentylaminosulfonyl, Dimethylaminosulfonyl, N-Ethyl-N-methylaminosulfonyl, Diethylaminosulfonyl, Dipropylaminosulfonyl, Cyclopropylaminosulfonyl, Cyclopentylaminosulfonyl, Cyclohexylaminosulfonyl und Cyclopropylmethylaminosulfonyl oder einen anderen C₁-C₆-Aminosulfonylrest, bestehend aus einem geraden oder verzweigten Alkyl, einem Cycloalkyl und einer Aminosulfonylgruppe, Cyclopropyl, Cyclobutyl, Cyclopentyl und Cyclohexyl oder einen anderen alicyclischen Kohlenwasserstoffrest mit 3 bis 6 Kohlenstoffatomen, Methyl, Ethyl, Vinyl, Ethinyl, Propyl, 1-Propenyl, 2-Propenyl, Isopropyl, Isopropenyl, 1-Propinyl, 2-Propinyl, Butyl, Isobutyl, s-Butyl, t-Butyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Butinyl, 2-Butinyl, Pentyl, Isopentyl, Neopentyl, t-Pentyl, 1-Pentenyl, 2-Pentenyl, 3-Pentenyl, 4-Pentenyl, Hexyl, 5-Hexenyl, 4-Methyl-3-pentenyl, Isohexyl, 2-Methylpentyl und 1-Ethylbutyl oder einen anderen aliphatischen Kohlenwasserstoffrest mit 1 bis 6 Kohlenstoffatomen, welcher eine gerade oder verzweigte ungesättigte Bindung enthalten kann;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen oder ein aliphatischer Kohlenwasserstoffrest mit 1 bis 6 Kohlenstoffatomen als Substituent des substituierten alicyclischen Kohlenwasserstoffrests mit 3 bis 10 Kohlenstoffatomen als G¹ ferner mit einem oder mehreren Substituenten substituiert sein kann, ausgewählt aus der Gruppe bestehend aus einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem C₂-C₇-Acylrest wie ein Methoxymethyloxyrest, 2-Methoxyethoxyrest, Formylrest, Trifluoracetylrest, Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe, bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem C₁-C₇-Acylaminorest wie Trifluoracetylaminorest, Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; und einer Cyanogruppe;
wobei Substituenten des substituierten aromatischen Kohlenwasserstoffrests mit 6 bis 14 Kohlenstoffatomen von G¹ ein Fluoratom, ein Chloratom, ein Bromatom, ein Iodatom, eine Hydroxygruppe, einen gegebenenfalls substituierten C₁-C₇-Alkoxyrest, einen C₆-C₁₀-Aryloxyrest, einen C₇-C₉-Aralkoxyrest, einen C₂-C₇-Acyloxyrest, eine Oxogruppe, einen C₁-C₆-Alkylsulfonyloxyrest, einen gegebenenfalls substituierten C₂-C₇-Acylrest, einen Carboxylrest, einen C₂-C₇-Alkoxycarbonylrest, einen Carbamoylrest, einen gegebenenfalls substituierten C₂-C₇-Alkylcarbamoylrest, eine Aminogruppe, einen gegebenenfalls substituierten C₁-C₆-Alkylaminorest, einen gegebenenfalls substituierten C₂-C₇-Acylaminorest, einen C₂-C₈-Alkoxycarbonylaminorest, einen C₁-C₆-Alkylsulfonylaminorest, eine Cyanogruppe, eine Nitrogruppe, einen C₁-C₆-Alkylthiorest, einen C₁-C₆-Alkylsulfinylrest, einen C₁-C₆-Alkylsulfonylrest, einen Sulfamoylrest, einen C₁-C₆-Alkylaminosulfonylrest, eine Sulfogruppe, einen gegebenenfalls substituierten alicyclischen Kohlenwasserstoffrest mit 3 bis 6 Kohlenstoffatomen und einen gegebenenfalls substituierten aliphatischen Kohlenwasserstoffrest mit 1 bis 6 Kohlenstoffatomen umfassen;
wobei der C₁-C₇-Alkoxyrest, der C₂-C₇-Acylrest, der C₂-C₇-Alkylcarbamoylrest, der C₁-C₆-Alkylaminorest, der C₂-C₇-Acylaminorest, der alicyclische Kohlenwasserstoffrest mit 3 bis 6 Kohlenstoffatomen oder der aliphatische Kohlenwasserstoffrest mit 1 bis 6 Kohlenstoffatomen als Substituent des substituierten aromatischen Kohlenwasserstoffrests mit 6 bis 14 Kohlenstoffatomen ferner mit einem oder mehreren Substituenten substituiert sein kann, ausgewählt aus der Gruppe bestehend aus einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; und einer Cyanogruppe;
wobei der Substituent des heterocyclischen Rests mit 1 bis 4 Atomen, ausgewählt aus der Gruppe bestehend aus einem Sauerstoffatom, einem Stickstoffatom und einem Schwefelatom, an seinem substituierten Ring von G¹ ein Fluoratom, ein Chloratom, ein Bromatom, ein Iodatom, eine Hydroxygruppe, einen gegebenenfalls substituierten C₁-C₇-Alkoxyrest, einen C₆-C₁₀-Aryloxyrest, einen C₇-C₉-Aralkoxyrest, einen C₂-C₇-Acyloxyrest, eine Oxogruppe, einen C₁-C₆-Alkylsulfonyloxyrest, einen gegebenenfalls substituierten C₂-C₇-Acylrest, einen Carboxylrest, einen C₂-C₇-Alkoxycarbonylrest, einen Carbamoylrest, einen gegebenenfalls substituierten C₂-C₇-Alkylcarbamoylrest, eine Aminogruppe, einen gegebenenfalls substituierten C₁-C₆-Alkylaminorest, einen gegebenenfalls substituierten C₂-C₇-Acylaminorest, einen C₂-C₈-Alkoxycarbonylaminorest, einen C₁-C₆-Alkylsulfonylaminorest, eine Cyanogruppe, eine Nitrogruppe, einen C₁-C₆-Alkylthiorest, einen C₁-C₆-Alkylsulfinylrest, einen C₁-C₆-Alkylsulfonylrest, einen Sulfamoylrest, einen C₁-C₆-Alkylaminosulfonylrest, eine Sulfogruppe, einen gegebenenfalls substituierten alicyclischen Kohlenwasserstoffrest mit 3 bis 6 Kohlenstoffatomen und einen gegebenenfalls substituierten aliphatischen Kohlenwasserstoffrest mit 1 bis 6 Kohlenstoffatomen umfasst;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen und aliphatischer Kohlenwasserstoffrest mit 1 bis 6 Kohlenstoffatomen als Substituent des heterocyclischen Rests mit 1 bis 4 Atomen, ausgewählt aus der Gruppe bestehend aus einem Sauerstoffatom, einem Stickstoffatom und einem Schwefelatom, an seinem substituierten Ring von G¹ ferner mit einem oder mehreren Substituenten substituiert sein können, ausgewählt aus der Gruppe bestehend aus einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; und einer Cyanogruppe;
A³ eine Einfachbindung oder einen gegebenenfalls substituierten zweiwertigen acyclischen aliphatischen Kohlenwasserstoffrest mit 1 bis 10 Kohlenstoffatomen darstellt, der G¹ mit A⁴ an demselben oder verschiedenen Kohlenstoffatomen verbindet;
wobei die Substituenten des zweiwertigen substituierten aliphatischen Kohlenwasserstoffrests mit 1 bis 10 Kohlenstoffatomen von A³ einen Kohlenwasserstoffrest mit 1 bis 6 Kohlenstoffatomen, einen alicyclischen Kohlenwasserstoffrest mit 3 bis 6 Kohlenstoffatomen, ein Halogenatom, einen Alkoxyrest mit 1 bis 6 Kohlenstoffatomen, einen Phenoxyrest, eine Aminogruppe oder einen Alkylaminorest mit 1 bis 6 Kohlenstoffatomen umfassen;
A⁴ eine Einfachbindung oder einen Rest darstellt, der A³ mit G² in der Form A³-C(=O)-G², A³-C(=O)-O-G², A³-C(=O)-NR¹²¹-G², A³-C(=S)-NR¹²²-G², A³-C(=NR¹²³)-G², A³-O-G², A³-O-C(=O)-G², A³-NR¹²⁴-G², A³-NR¹²¹-C(=O)-G², A³-NR¹²⁶-S(=O)₂-G², A³-NR¹²⁷-C(=O)-O-G², A³-NR¹²⁸-C(=O)-NR¹²⁹-G², A³-NR¹³⁰-C(=S)-G², A³-NR¹³¹-C(=S)-NR¹³²-G², A³-S-G², A³-S(=O)-G², A³-S(=O)₂-G², A³-S(=O)₂-NR¹³³-G² oder A³-S(=O)₂-O-G² verbindet;
G² ein Wasserstoffatom, ein gegebenenfalls substituierter acyclischer aliphatischer Kohlenwasserstoffrest mit 1 bis 10 Kohlenstoffatomen, ein gegebenenfalls substituierter alicyclischer Kohlenwasserstoffrest mit 3 bis 10 Kohlenstoffatomen, ein gegebenenfalls substituierter aromatischer Kohlenwasserstoffrest mit 6 bis 14 Kohlenstoffatomen oder ein gegebenenfalls substituierter heterocyclischer Rest mit 1 bis 4 Atomen, ausgewählt aus der Gruppe bestehend aus einem Sauerstoffatom, einem Stickstoffatom und einem Schwefelatom, im Ring ist;
wobei Substituenten des substituierten acyclischen aliphatischen Kohlenwasserstoffrests mit 1 bis 10 Kohlenstoffatomen von G² umfassen: ein Fluoratom; ein Chloratom; ein Bromatom; ein Iodatom; eine Hydroxygruppe; einen C₁-C₇-Alkoxyrest, bestehend aus einem geraden oder verzweigten Alkylrest, Cycloalkylrest und einer Oxygruppe, umfassend Methoxy, Ethoxy, Propoxy, Isopropoxy, Butoxy, Isobutoxy, s-Butoxy, t-Butoxy, Pentyloxy, Isopentyloxy, Neopentyloxy, t-Pentyloxy, Hexyloxy, Isohexyloxy, 2-Methylpentyloxy, 1-Ethylbutoxy, Cyclopropyloxy, Cyclobutyloxy, Cyclopentyloxy, Cyclohexyloxy, Cyclopropylmethyloxy, Cyclopropylethyloxy, Cyclopentylmethyloxy und Cyclohexylmethyloxy; einen Alkyldioxyrest mit 1 bis 4 Kohlenstoffatomen wie Ethylendioxy; einen C₆-C₁₀-Aryloxyrest, umfassend Phenoxy, 1-Naphthoxy und 2-Naphthoxy; einen C₇-C₉-Aralkoxyrest, umfassend Benzyloxy, α-Phenethyloxy, β-Phenethyloxy und Phenylpropyloxy; einen C₂-C₇-Acyloxyrest, umfassend Acetoxy, Propionyloxy, Butyryloxy, Isobutyryloxy, Valeryloxy, Isovaleryloxy, Pivaloyloxy und Hexanoyloxy; eine Oxogruppe; einen C₁-C₆-Alkylsulfonyloxyrest, bestehend aus einem geraden oder verzweigten Alkyl und Sulfonyloxy, umfassend Oxo, Methylsulfonyloxy, Ethylsulfonyloxy, Propylsulfonyloxy, Butylsulfonyloxy und t-Butylsulfonyloxy; einen C₂-C₇-Acylrest, umfassend Acetyl, Propionyl, Butyryl, Isobutyryl, Valeryl, Isovaleryl, Pivaroyl und Hexanoyl; einen Carboxylrest; einen C₂-C₇-Alkoxycarbonylrest, bestehend aus einem geraden oder verzweigten Alkyl und einer Oxycarbonylgruppe, umfassend Methoxycarbonyl, Ethoxycarbonyl, Propoxycarbonyl, Isopropoxycarbonyl, Butoxycarbonyl, Isobutoxycarbonyl, s-Butoxycarbonyl und t-Butoxycarbonyl; einen Carbamoylrest; einen C₂-C₇-Alkylcarbamoylrest, bestehend aus einem geraden oder verzweigten Alkyl, Cycloalkyl und einer Carbamoylgruppe, umfassend N-Methylcarbamoyl, N-Ethylcarbamoyl, N-Propylcarbamoyl, N-Isopropylcarbamoyl, N-Butylcarbamoyl, N-Isobutylcarbamoyl, N-s-Butylcarbamoyl, N-t-Butylcarbamoyl, N-Pentylcarbamoyl, N-Cyclopropylcarbamoyl, N-Cyclobutylcarbamoyl, N-Cyclopentylcarbamoyl, N-Cyclohexylcarbamoyl, N-Cycloheptylcarbamoyl, N-Cyclopropylmethylcarbamoyl, N,N-Dimethylcarbamoyl, N-Ethyl-N-methylcarbamoyl, N,N-Diethylcarbamoyl und N,N-Dipropylcarbamoyl; eine Aminogruppe; einen C₁-C₆-Alkylaminorest, bestehend aus einem geraden oder verzweigten Alkyl, Cycloalkyl und einer Aminogruppe, umfassend Methylamino, Ethylamino, Propylamino, Isopropylamino, Butylamino, Isobutylamino, s-Butylamino, t-Butylamino, Pentylamino, Hexylamino, Cyclopropylamino, Cyclobutylamino, Cyclopentylamino, Cyclohexylamino, Cyclopropylmethylamino, Dimethylamino, N-Ethylinethylamino, Diethylamino, N-Methylpropylamino, N-Methylisopropylamino, N-Methylbutylamino, N-Methyl-t-butylamino, N-Ethylisopropylamino, Dipropylamino, Diisopropylamino und Ethylbutylamino; einen C₂-C₇-Acylaminorest, umfassend Acetylamino, Propionylamino, Butyrylamino, Isobutyrylamino, Valerylamino und Hexanoylamino; einen C₂-C₈-Alkoxycarbonylaminorest, umfassend Methoxycarbonylamino, Ethoxycarbonylamino und t-Butoxycarbonylamino; einen C₁-C₆-Alkylsulfonylaminorest, umfassend Methylsulfonylamino, Ethylsulfonylamino, Butylsulfonylamino und t-Butylsulfonylamino; eine Cyanogruppe; eine Nitrogruppe; einen C₁-C₆-Alkylthiorest, umfassend Methylthio, Ethylthio, Propylthio, Isopropylthio, Butylthio, Isobutylthio, s-Butylthio, t-Butylthio, Pentylthio und Hexylthio; einen C₁-C₆-Alkylsulfinylrest, bestehend aus einem geraden oder verzweigten Alkyl, Cycloalkyl und einer Sulfinylgruppe, umfassend Methylsulfinyl, Ethylsulfinyl, Propylsulfinyl, Isopropylsulfinyl, Butylsulfinyl, Isobutylsulfinyl, s-Butylsulfinyl, t-Butylsulfmyl, Pentylsulfinyl und Cyclopentylsulfinyl; einen C₁-C₆-Alkylsulfonylrest, bestehend aus einem geraden oder verzweigten Alkyl, Cycloalkyl und einer Sulfonylgruppe, umfassend Methylsulfonyl, Ethylsulfonyl, Propylsulfonyl, Isopropylsulfonyl, Butylsulfonyl, Isobutylsulfonyl, s-Butylsulfonyl, t-Butylsulfonyl, Pentylsulfonyl, Hexylsulfonyl, Cyclopentylsulfonyl und Cyclohexylsulfonyl; eine Sulfogruppe; einen Sulfamoylrest; einen C₁-C₆-Aminosulfonylrest, bestehend aus einem geraden oder verzweigten Alkyl, Cycloalkyl und einer Aminosulfonylgruppe, umfassend Methylaminosulfonyl, Ethylaminosulfonyl, Propylaminosulfonyl, Isopropylaminosulfonyl, Butylaminosulfonyl, Isobutylaminosulfonyl, s-Butylaminosulfonyl, Pentylaminosulfonyl, Dimethylaminosulfonyl, N-Ethyl-N-methylaminosulfonyl, Diethylaminosulfonyl, Dipropylaminosulfonyl, Cyclopropylaminosulfonyl, Cyclopentylaminosulfonyl, Cyclohexylaminosulfonyl und Cyclopropylmethylaminosulfonyl; einen alicyclischen Kohlenwasserstoffrest mit 3 bis 6 Kohlenstoffatomen, umfassend Cyclopropyl, Cyclobutyl, Cyclopentyl und Cyclohexyl; und einen aliphatischen Kohlenwasserstoffrest mit 1 bis 6 Kohlenstoffatomen, der eine gerade oder verzweigte ungesättigte Bindung enthalten kann, umfassend Methyl, Ethyl, Vinyl, Ethinyl, Propyl, 1-Propenyl, 2-Propenyl, Isopropyl, Isopropenyl, 1-Propinyl, 2-Propinyl, Butyl, Isobutyl, s-Butyl, t-Butyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Butinyl, 2-Butinyl, Pentyl, Isopentyl, Neopentyl, t-Pentyl, 1-Pentenyl, 2-Pentenyl, 3-Pentenyl, 4-Pentenyl, Hexyl, 5-Hexenyl, 4-Methyl-3-pentenyl, Isohexyl, 2-Methylpentyl und 1-Ethylbutyl; einen aromatischen Kohlenwasserstoffrest mit 6 bis 14 Kohlenstoffatomen, der ein einwertiger Rest ist, abgeleitet von einem monocyclischen, bicyclischen oder tricyclischen aromatischen Kohlenwasserstoffrest, umfassend Benzol, Naphthalin, Inden, Indan, 1,2,3,4-Tetrahydronaphthalin und Fluoren; und einen einwertigen Rest, abgeleitet von einer monocyclischen, bicyclischen oder tricyclischen heterocyclischen Verbindung, umfassend Furan, Thiophen, Pyrrol, Pyrrolin, Pyrrolidin, Oxazol, Oxazolidin, Isooxazol, Isooxazolidin, Thiazol, Thiazolidin, Isothiazol, Isothiazolidin, Imidazol, Imidazolin, Imidazolidin, Pyrazol, Pyrazolin, Pyrazolidin, Triazol, Thiadiazol, Oxadiazol, Tetrazol, Pyran, Tetrahydropyran, Thiopyran, Tetrahydrothiopyran, Pyridin, Pyrazin, Pyrimidin, Pyridazin, Benzofuran, Dibenzofuran, Benzothiophen, Indol, Benzimidazol, Benzothiazol, Benzooxazol, Chroman, Isochroman, Chinolin, Decahydrochinolin, Isochinolin, Chinazolin, Chinoxalin, Purin, Pteridin, Azetidin, Morpholin, Thiomorpholin, Piperidin, Homopiperidin, Piperazin, Homopiperazin, Indolin, Isoindolin, Phenoxazin, Phenazin, Phenothiazin und Chinuclidin, wobei die heterocyclische Verbindung 1 bis 4 Atome, ausgewählt aus der Gruppe bestehend aus einem Sauerstoffatom, einem Stickstoffatom und einem Schwefelatom, im Ring aufweist;
wobei ein C₁-C₇-Alkoxyrest, ein C₂-C₇-Acylrest, C₂-C₇-Alkylcarbamoyl, ein C₁-C₆-Alkylaminorest, ein C₂-C₇-Acylaminorest, ein alicyclischer Kohlenwasserstoffrest mit 3 bis 6 Kohlenstoffatomen, ein aliphatischer Kohlenwasserstoffrest mit 1 bis 6 Kohlenstoffatomen, aromatischer Kohlenwasserstoffrest mit 6 bis 14 Kohlenstoffatomen und heterocyclischer Rest mit 1 bis 4 Atomen, ausgewählt aus der Gruppe bestehend aus einem Sauerstoffatom, einem Stickstoffatom und einem Schwefelatom, im Ring als Substituent des substituierten acyclischen aliphatischen Kohlenwasserstoffrests mit 1 bis 10 Kohlenstoffatomen als G² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
wobei Substituenten des substituierten alicyclischen Kohlenwasserstoffrests mit 3 bis 10 Kohlenstoffatomen von G² ein Fluoratom, ein Chloratom, ein Bromatom, ein Iodatom, eine Hydroxygruppe, einen gegebenenfalls substituierten C₁-C₇-Alkoxyrest, C₁-C₄-Alkylendioxyrest, einen C₆-C₁₀-Aryloxyrest, einen C₇-C₉-Aralkoxyrest, einen C₂-C₇-Acyloxyrest, eine Oxogruppe, einen C₁-C₆-Alkylsulfonyloxyrest, einen gegebenenfalls substituierten C₂-C₇-Acylrest, eine Carboxylgruppe, einen C₂-C₇-Alkoxycarbonylrest, einen Carbamoylrest, einen gegebenenfalls substituierten C₂-C₇-Alkylcarbamoylrest, eine Aminogruppe, einen gegebenenfalls substituierten C₁-C₆-Alkylaminorest, einen gegebenenfalls substituierten C₂-C₇-Acylaminorest, einen C₂-C₈-Alkoxycarbonylaminorest, einen C₁-C₆-Alkylsulfonylaminorest, eine Cyanogruppe, eine Nitrogruppe, einen C₁-C₆-Alkylthiorest, einen C₁-C₆-Alkylsulfinylrest, einen C₁-C₆-Alkylsulfonylrest, einen Sulfamoylrest, einen C₁-C₆-Alkylaminosulfonylrest, eine Sulfogruppe, einen gegebenenfalls substituierten alicyclischen Kohlenwasserstoffrest mit 3 bis 6 Kohlenstoffatomen und einen gegebenenfalls substituierten aliphatischen Kohlenwasserstoffrest mit 1 bis 6 Kohlenstoffatomen, einen gegebenenfalls substituierten aromatischen Kohlenwasserstoffrest mit 6 bis 14 Kohlenstoffatomen, umfassen;
wobei ein C₁-C₇-Alkoxyrest, ein C₂-C₇-Acylrest, ein C₂-C₇-Alkylearbamoylrest, ein C₁-C₆-Alkylaminorest, ein C₂-C₇-Acylaminorest, ein alicyclischer Kohlenwasserstoffrest mit 3 bis 6 Kohlenstoffatomen, ein aliphatischer Kohlenwasserstoffrest mit 1 bis 6 Kohlenstoffatomen und ein aromatischer Kohlenwasserstoffrest mit 6 bis 14 Kohlenstoffatomen als Substituent des substituierten alicyclischen Kohlenwasserstoffrests mit 3 bis 10 Kohlenstoffatomen von G² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino, einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
wobei Substituenten des aromatischen Kohlenwasserstoffrests mit 6 bis 14 Kohlenstoffatomen von G² mindestens einen Substituenten, ausgewählt aus der Gruppe bestehend aus einem Fluoratom, einem Chloratom, einem Bromatom, einem Iodatom, einer Hydroxygruppe, einem gegebenenfalls substituierten C₁-C₇-Alkoxyrest, einem C₁-C₄-Alkylendioxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einem Carboxykest, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen und einem gegebenenfalls substituierten aromatischen Kohlenwasserstoffrest mit 6 bis 14 Kohlenstoffatomen, umfassen;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen, ein aliphatischer Kohlenwasserstoffrest mit 1 bis 6 Kohlenstoffatomen und ein aromatischer Kohlenwasserstoffrest mit 6 bis 14 Kohlenstoffatomen als Substituenten des aromatischen Kohlenwasserstoffrests mit 6 bis 14 Kohlenstoffatomen von G² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
wobei Substituenten des heterocyclischen Rests mit 1 bis 4 Atomen, ausgewählt aus der Gruppe bestehend aus einem Sauerstoffatom, einem Stickstoffatom und einem Schwefelatom, an ihrem substituierten Ring von G² mindestens einen Substituenten, ausgewählt aus der Gruppe bestehend aus einem Fluoratom, einem Chloratom, einem Bromatom, einem Iodatom, einer Hydroxygruppe, einem gegebenenfalls substituierten C₁-C₇-Alkoxyrest, einem C₁-C₄-Alkylendioxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen und einem gegebenenfalls substituierten aromatischen Kohlenwasserstoffrest mit 6 bis 14 Kohlenstoffatomen, umfassen;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen, ein aliphatischer Kohlenwasserstoffrest mit 1 bis 6 Kohlenstoffatomen und ein aromatischer Kohlenwasserstoffrest mit 6 bis 14 Kohlenstoffatomen als Substituent des heterocyclischen Rests mit 1 bis 4 Atomen, ausgewählt aus der Gruppe bestehend aus einem Sauerstoffatom, einem Stickstoffatom und einem Schwefelatom, an seinem substituierten Ring von G² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
A⁵ eine Einfachbindung oder -NR²⁰¹- darstellt;
R² ein Wasserstoffatom, ein Fluoratom, ein Chloratom, ein Bromatom, ein Iodatom, ein gegebenenfalls substituierter acyclischer aliphatischer Kohlenwasserstof&est mit 1 bis 10 Kohlenstoffatomen, ein gegebenenfalls substituierter alicyclischer Kohlenwasserstoffrest mit 3 bis 8 Kohlenstoffatomen, ein gegebenenfalls substituierter aromatischer Kohlenwasserstoffrest mit 6 bis 14 Kohlenstoffatomen oder ein gegebenenfalls substituierter heterocyclischer Rest mit 1 bis 4 Atomen, ausgewählt aus der Gruppe bestehend aus einem Sauerstoffatom, einem Stickstoffatom und einem Schwefelatom, im Ring ist;
wobei die Substituenten des substituierten acyclischen aliphatischen Kohlenwasserstoffrests mit 1 bis 10 Kohlenstoffatomen von R² mindestens einen Substituenten, ausgewählt aus der Gruppe bestehend aus einem Fluoratom, einem Chloratom, einem Bromatom, einem Iodatom, einer Hydroxygruppe, einem gegebenenfalls substituierten C₁-C₇-Alkoxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 1 bis 6 Kohlenstoffatomen, einem gegebenenfalls substituierten acyclischen aliphatischen Kohlenwasserstoffrest mit 1 bis 6 Kohlenstoffatomen, einem gegebenenfalls substituierten aromatischen Kohlenwasserstoffrest mit 6 bis 14 Kohlenstoffatomen und einem gegebenenfalls substituierten heterocyclischen Rest mit 1 bis 4 Atomen, ausgewählt aus der Gruppe bestehend aus einem Sauerstoffatom, einem Stickstoffatom und einem Schwefelatom, im Ring umfassen;
wobei ein C₁-C₇-Alkoxyrest, ein C₂-C₇-Acylrest, C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen, ein acyclischer aliphatischer Kohlenwasserstoffrest mit 1 bis 6 Kohlenstoffatomen, aromatischer Kohlenwasserstoffrest mit 6 bis 14 Kohlenstoffatomen und heterocyclischer Rest mit 1 bis 4 Atomen, ausgewählt aus der Gruppe bestehend aus einem Sauerstoffatom, einem Stickstoffatom und einem Schwefelatom, im Ring als Substituent des substituierten acyclischen aliphatischen Kohlenwasserstoffrests mit 1 bis 10 Kohlenstoffatomen als R² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
wobei Substituenten des substituierten alicyclischen Kohlenwasserstoffrests mit 3 bis 8 Kohlenstoffatomen von R² mindestens einen Substituenten, ausgewählt aus der Gruppe bestehend aus einem Fluoratom, einem Chloratom, einem Bromatom, einem Iodatom, einer Hydroxygruppe, einem gegebenenfalls substituierten C₁-C₇-Alkoxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen, einem gegebenenfalls substituierten aromatischen Kohlenwasserstoffrest mit 6 bis 14 Kohlenstoffatomen und einem gegebenenfalls substituierten heterocyclischen Rest mit 1 bis 4 Atomen, ausgewählt aus der Gruppe bestehend aus einem Sauerstoffatom, einem Stickstoffatom und einem Schwefelatom, im Ring umfassen;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen, ein aliphatischer Kohlenwasserstoffrest mit 1 bis 6 Kohlenstoffatomen, ein aromatischer Kohlenwasserstoffrest mit 6 bis 14 Kohlenstoffatomen und ein heterocyclischer Rest mit 1 bis 4 Atomen, ausgewählt aus der Gruppe bestehend aus einem Sauerstoffatom, einem Stickstoffatom und einem Schwefelatom, im Ring als Substituent des substituierten alicyclischen Kohlenwasserstoffrests mit 3 bis 8 Kohlenstoffatomen von R² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest und einem Trifluormethoxyrest;
wobei Substituenten des substituierten aromatischen Kohlenwasserstoffrests mit 6 bis 14 Kohlenstoffatomen mindestens einen Substituenten, ausgewählt aus der Gruppe bestehend aus einem Fluoratom, einem Chloratom, einem Bromatom, einem Iodatom, einer Hydroxygruppe, einem C₁-C₆-Alkylrest, einem gegebenenfalls substituierten C₁-C₇-Alkoxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylest, einer Aminogruppe, einem gegebenenfalls substituierten C₁-C₆-Alkylaminorest, einem gegebenenfalls substituierten C₂-C₇-Acylaminroest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen, einem gegebenenfalls substituierten aromatischen Kohlenwasserstoffrest mit 6 bis 14 Kohlenstoffatomen und einem gegebenenfalls substituierten heterocyclischen Rest mit 1 bis 4 Atomen, ausgewählt aus der Gruppe bestehend aus einem Sauerstoffatom, einem Stickstoffatom und einem Schwefelatom, im Ring umfassen;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstof&est mit 3 bis 6 Kohlenstoffatomen, ein aliphatischer Kohlenwasserstoffrest mit 1 bis 6 Kohlenstoffatomen, ein aromatischer Kohlenwasserstoffrest mit 6 bis 14 Kohlenstoffatomen und heterocyclischer Rest (mit 1 bis 4 Atomen, ausgewählt aus der Gruppe bestehend aus einem Sauerstoffatom, einem Stickstoffatom und einem Schwefelatom im Ring) als Substituent des aromatischen Kohlenwasserstof&ests mit 6 bis 14 Kohlenstoffatomen von R² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest und einem Trifluormethoxyrest;
wobei Substituenten des heterocyclischen Rests mit 1 bis 4 Atomen, ausgewählt aus der Gruppe bestehend aus einem Sauerstoffatom, einem Stickstoffatom und einem Schwefelatom, an dem substituierten Ring von R² mindestens einen Substituenten, ausgewählt aus der Gruppe, bestehend aus einem Fluoratom, einem Chloratom, einem Bromatom, einem Iodatom, einer Hydroxygruppe, einem gegebenenfalls substituierten C₁-C₇-Alkoxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen, einem gegebenenfalls substituierten aromatischen Kohlenwasserstoffrest mit 6 bis 14 Kohlenstoffatomen und einem gegebenenfalls substituierten heterocyclischen Rest mit 1 bis 4 Atomen, ausgewählt aus der Gruppe bestehend aus einem Sauerstoffatom, einem Stickstoffatom und einem Schwefelatom, im Ring umfassen;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen, aliphatischer Kohlenwasserstoffrest mit 1 bis 6 Kohlenstoffatomen, ein aromatischer Kohlenwasserstoffrest mit 6 bis 14 Kohlenstoffatomen und ein heterocyclischer Rest mit 1 bis 4 Atomen, ausgewählt aus der Gruppe bestehend aus einem Sauerstoffatom, einem Stickstoffatom und einem Schwefelatom, im Ring als Substituent des heterocyclischen Rests mit 1 bis 4 Atomen, ausgewählt aus der Gruppe bestehend aus einem Sauerstoffatom, einem Stickstoffatom und einem Schwefelatom, an seinem substituierten Ring von R² ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
A⁶ eine Einfachbindung darstellt;
R³ ein gegebenenfalls substituierter aromatischer Kohlenwasserstoffrest mit 6 bis 14 Kohlenstoffatomen oder ein gegebenenfalls substituierter heterocyclischer Rest mit 1 bis 4 Atomen, ausgewählt aus der Gruppe bestehend aus einem Sauerstoffatom, einem Stickstoffatom und einem Schwefelatom, im Ring ist;
wobei Substituenten des substituierten aromatischen Kohlenwasserstoffrests mit 6 bis 14 Kohlenstoffatomen von R³ mindestens einen Substituenten, ausgewählt aus der Gruppe bestehend aus einem Fluoratom, einem Chloratom, einem Bromatom, einem Iodatom, einer Hydroxygruppe, einem gegebenenfalls substituierten C₁-C₇-Alkoxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfinylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen, einem gegebenenfalls substituierten aromatischen Kohlenwasserstoffrest mit 6 bis 14 Kohlenstoffatomen und einem gegebenenfalls substituierten heterocyclischen Rest mit 1 bis 4 Atomen, ausgewählt aus der Gruppe bestehend aus einem Sauerstoffatom, einem Stickstoffatom und einem Schwefelatom, im Ring umfassen;
wobei ein C₁-C₇-Alkoxyrest, ein C₂-C₇-Acylrest, ein C₂-C₇-Alkylcarbamoylrest, ein C₁-C₆-Alkylaminrest, ein C₂-C₇-Acylaminrest, ein alicyclischer Kohlenwasserstoffrest mit 3 bis 6 Kohlenstoffatomen, ein aliphatischer Kohlenwasserstoffrest mit 1 bis 6 Kohlenstoffatomen, ein aromatischer Kohlenwasserstoffrest mit 6 bis 14 Kohlenstoffatomen und ein heterocyclischer Rest mit 1 bis 4 Atomen, ausgewählt aus der Gruppe bestehend aus einem Sauerstoffatom, einem Stickstoffatom und einem Schwefelatom, im Ring als Substituent des substituierten aromatischen Kohlenwasserstoffrests mit 6 bis 14 Kohlenstoffatomen von R³ ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
wobei Substituenten des heterocyclischen Rests mit 1 bis 4 Atomen, ausgewählt aus der Gruppe bestehend aus einem Sauerstoffatom, einem Stickstoffatom und einem Schwefelatom, am Ring von R³ mindestens einen Substituenten, ausgewählt aus der Gruppe bestehend aus einem Fluoratom, einem Chloratom, einem Bromatom, einem Iodatom, einer Hydroxygruppe, einem gegebenenfalls substituierten C₁-C₇-Alkoxyrest, einem C₆-C₁₀-Aryloxyrest, einem C₇-C₉-Aralkoxyrest, einem C₂-C₇-Acyloxyrest, einer Oxogruppe, einem C₁-C₆-Alkylsulfonyloxyrest, einem gegebenenfalls substituierten C₂-C₇-Acylrest, einer Carboxylgruppe, einem C₂-C₇-Alkoxycarbonylrest, einem Carbamoylrest, einem gegebenenfalls substituierten C₂-C₇-Alkylcarbamoylrest, einer Aminogruppe, einem gegebenenfalls substituierten C₁-C₆-Alkylaminorest, einem gegebenenfalls substituierten C₂-C₇-Acylaminorest, einem C₂-C₈-Alkoxycarbonylaminorest, einem C₁-C₆-Alkylsulfonylaminorest, einer Cyanogruppe, einer Nitrogruppe, einem C₁-C₆-Alkylthiorest, einem C₁-C₆-Alkylsulfmylrest, einem C₁-C₆-Alkylsulfonylrest, einem Sulfamoylrest, einem C₁-C₆-Alkylaminosulfonylrest, einer Sulfogruppe, einem gegebenenfalls substituierten alicyclischen Kohlenwasserstoffrest mit 3 bis 6 Kohlenstoffatomen, einem gegebenenfalls substituierten aliphatischen Kohlenwasserstoffrest mit 1 bis 6 Kohlenstoffatomen, einem gegebenenfalls substituierten aromatischen Kohlenwasserstoffrest mit 6 bis 14 Kohlenstoffatomen und einem gegebenenfalls substituierten heterocyclischen Rest mit 1 bis 4 Atomen, ausgewählt aus der Gruppe bestehend aus einem Sauerstoffatom, einem Stickstoffatom und einem Schwefelatom, im Ring umfassen;
wobei ein C₁-C₇-Alkoxyrest, ein C₂-C₇-Acylrest, ein C₂-C₇-Alkylcarbamoylrest, ein C₁-C₆-Alkylaminorest, ein C₂-C₇-Acylaminorest, ein alicyclischer Kohlenwasserstoffrest mit 3 bis 6 Kohlenstoffatomen und ein aliphatischer Kohlenwasserstoffrest mit 1 bis 6 Kohlenstoffatomen, ein aromatischer Kohlenwasserstoffrest mit 6 bis 14 Kohlenstoffatomen und ein heterocyclischer Rest mit 1 bis 4 Atomen, ausgewählt aus der Gruppe bestehend aus Sauerstoffatom, einem Stickstoffatom und einem Schwefelatom, im Ring als Substituent des heterocyclischen Rests mit 1 bis 4 Atomen, ausgewählt aus der Gruppe bestehend aus einem Sauerstoffatom, einem Stickstoffatom und einem Schwefelatom, an seinem substituierten Ring von R³ ferner mit einem oder mehreren Substituenten substituiert sein können, ausgewählt aus der Gruppe bestehend aus: einem Fluoratom; einem Chloratom; einem Bromatom; einem Iodatom; einer Hydroxygruppe; einem C₁-C₆-Alkoxyrest wie Methoxy, Ethoxy, Propoxy, Isopropoxy, Butoxy, Isobutoxy, s-Butoxy, t-Butoxy, Pentyloxy oder Cyclopropyloxy; einem Methoxymethyloxyrest; einem 2-Methoxyethoxyrest; einem Formylrest; einem Trifluoracetylrest; einem C₂-C₇-Acylrest wie Acetyl, Propionyl, Butyryl, Isobutyryl, Valeryl oder Isovaleryl; einer Oxogruppe; einer Carboxylgruppe; einem C₂-C₇-Alkoxycarbonylrest wie Methoxycarbonyl, Ethoxycarbonyl, Propoxycarbonyl, Isopropoxycarbonyl, Butoxycarbonyl, Isobutoxycarbonyl oder t-Butoxycarbonyl; einem Carbamoylrest; einem C₂-C₇-Alkylcarbamoylrest wie N-Methylcarbamoyl, N,N-Dimethylcarbamoyl, N-Ethylcarbamoyl, N-Ethyl-N-methylcarbamoyl, N,N-Diethylcarbamoyl, N-Propylcarbamoyl, N-Isopropylcarbamoyl, N-Butylcarbamoyl, N-Cyclopropylcarbamoyl oder N-Cyclopropylmethylcarbamoyl; einer Aminogruppe; einem C₁-C₆-Alkylaminorest wie Methylamino, Ethylamino, Propylamino, Isopropylamino, Dimethylamino, N-Ethylmethylamino, Diethylamino, N-Methylpropylamino, N-Methylisopropylamino, Cyclopropylamino oder Cyclopropylmethylamino; einem cyclischen C₄-C₆-Aminorest mit 1 oder 2 Atomen, ausgewählt aus der Gruppe bestehend aus einem Sauerstoffatom, einem Stickstoffatom und einem Schwefelatom, im Ring, wie 1-Pyrrolidinyl, Piperazinyl, 4-Methylpiperazinyl, Piperidino oder Morpholino; einem Trifluoracetylaminorest; einem C₁-C₇-Acylaminorest wie Formylamino, Acetylamino, Propionylamino, Butyrylamino, Isobutyrylamino oder Valerylamino; einem C₁-C₆-Alkylsulfonylaminorest wie Methylsulfonylamino, Ethylsulfonylamino, Propylsulfonylamino oder Butylsulfonylamino; einer Nitrogruppe; einer Cyanogruppe; einem C₁-C₆-Alkylrest, umfassend Methyl, Ethyl, Propyl, Isopropyl, Butyl, Isobutyl, s-Butyl und t-Butyl; einem Trifluormethylrest; und einem Trifluormethoxyrest;
R¹⁰¹-R¹¹⁷, R¹²¹-R¹³³ und R²⁰¹ jeweils unabhängig ein Wasserstoffatom oder ein aliphatischer Kohlenwasserstoffrest mit 1 bis 4 Kohlenstoffatomen sind;
mit der Maßgabe, dass wenn A¹ und A³ beide einen acyclischen aliphatischen Kohlenwasserstoffrest darstellen, mindestens einer von A² oder G¹ keine Einfachbindung ist.

2. Die Verbindung oder ein pharmazeutisch verträgliches Salz davon gemäß Anspruch 1, wobei A² die Bedeutung -C(=O)-, -C(=O)-O-, -C(=O)-NH-, -C(=O)-NMe-, -NH-, -NH-C(=O)-, -NH-C(=O)-O-, -NH-C(=O)-NH-, -NH-C(=O)-NMe- oder -NH-C(=S)- hat.

3. Die Verbindung oder ein pharmazeutisch verträgliches Salz davon gemäß Anspruch 1, wobei A² die Bedeutung -C(=O)-NH-, -NH-, -NH-C(=O)-, -NH-C(=O)-O- oder NH-C(=O)-NH- hat.

4. Die Verbindung oder ein pharmazeutisch verträgliches Salz davon gemäß einem der Ansprüche 1 bis 3, wobei die Kombination von G¹, A³, A⁴ und G² eine der Kombinationen 1 bis 10 der folgenden Tabelle ist.
| Kombination | G¹ | A³ | A⁴ | G² |
|---|---|---|---|---|
| 1 | von Einfachbindung verschiedener Rest | Einfachbindung | Einfachbindung | Wasserstoffatom |
| 2 | Einfachbindung | von Einfachbindung verschiedener Rest | Einfachbindung | Wasserstoffatom |
| 3 | von Einfachbindung verschiedener Rest | Einfachbindung | Einfachbindung | von Wasserstoffatom verschiedener Rest |
| 4 | Einfachbindung | von Einfachbindung verschiedener Rest | Einfachbindung | von Wasserstoffatom verschiedener Rest |
| 5 | von Einfachbindung verschiedener Rest | Einfachbindung | von Einfachbindung verschiedener Rest | von Wasserstoffatom verschiedener Rest |
| 6 | Einfachbindung | von Einfachbindung verschiedener Rest | von Einfachbindung verschiedener Rest | von Wasserstoffatom verschiedener Rest |
| 7 | von Einfachbindung verschiedener Rest | von Einfachbindung verschiedener Rest | Einfachbindung | von Wasserstoffatom verschiedener Rest |
| 8 | von Einfachbindung verschiedener Rest | von Einfachbindung verschiedener Rest | von Einfachbindung verschiedener Rest | von Wasserstoffatom verschiedener Rest |
| 9 | von Einfachbindung verschiedener Rest | von Einfachbindung verschiedener Rest | von Einfachbindung verschiedener Rest | Wasserstoffatom |
| 10 | Einfachbindung | Einfachbindung | Einfachbindung | Wasserstoffatom |

5. Die Verbindung oder ein pharmazeutisch verträgliches Salz davon gemäß einem der Ansprüche 1 bis 3, wobei G¹ einen von einer Einfachbindung verschiedenen Rest darstellt, A³ und A⁴ eine Einfachbindung darstellen und G² ein Wasserstoffatom darstellt.

6. Die Verbindung oder ein pharmazeutisch verträgliches Salz davon gemäß einem der Ansprüche 1 bis 3, wobei G¹ und A⁴ eine Einfachbindung darstellen, A³ einen von einer Einfachbindung verschiedenen Rest darstellt und G² ein Wasserstoffatom darstellt.

7. Die Verbindung oder ein pharmazeutisch verträgliches Salz davon gemäß einem der Ansprüche 1 bis 3, wobei 0¹ einen von einer Einfachbindung verschiedenen Rest darstellt, A³ und A⁴ eine Einfachbindung darstellen und G² einen von einem Wasserstoffatom verschiedenen Rest darstellt.

8. Die Verbindung oder ein pharmazeutisch verträgliches Salz davon gemäß einem der Ansprüche 1 bis 3, wobei G¹ und A⁴ eine Einfachbindung darstellen, A³ einen von einer Einfachbindung verschiedenen Rest darstellt und G² einen von einem Wasserstoffatom verschiedenen Rest darstellt.

9. Die Verbindung oder ein pharmazeutisch verträgliches Salz davon gemäß Anspruch 8, wobei A³ einen Alkylenrest mit 1 bis 3 Kohlenstoffatomen darstellt.

10. Die Verbindung oder ein pharmazeutisch verträgliches Salz davon gemäß einem der Ansprüche 1 bis 3, wobei G¹ und A⁴ einen von einer Einfachbindung verschiedenen Rest darstellen, A³ eine Einfachbindung darstellt und G² einen von einem Wasserstoffatom verschiedenen Rest darstellt.

11. Die Verbindung oder ein pharmazeutisch verträgliches Salz davon gemäß Anspruch 10, wobei A⁴ die Bedeutung -C(=O)-, -C(=O)-NH-, -O- oder NH-C(=O)- hat.

12. Die Verbindung oder ein pharmazeutisch verträgliches Salz davon gemäß einem der Ansprüche 1 bis 3, wobei G¹ eine Einfachbindung darstellt, A³ und A⁴ einen von einer Einfachbindung verschiedenen Rest darstellen und G² einen von einem Wasserstoffatom verschiedenen Rest darstellt.

13. Die Verbindung oder ein pharmazeutisch verträgliches Salz davon gemäß einem der Ansprüche 1 bis 3, wobei G¹ und A³ einen von einer Einfachbindung verschiedenen Rest darstellen, A⁴ eine Einfachbindung darstellt und G² einen von einem Wasserstoffatom verschiedenen Rest darstellt.

14. Die Verbindung oder ein pharmazeutisch verträgliches Salz davon gemäß Anspruch 13, wobei A³ einen Alkylenrest mit 1 bis 3 Kohlenstoffatomen darstellt.

15. Die Verbindung oder ein pharmazeutisch verträgliches Salz davon gemäß einem der Ansprüche 1 bis 3, wobei G¹, A³ und A⁴ einen von einer Einfachbindung verschiedenen Rest darstellen und G² einen von einem Wasserstoffatom verschiedenen Rest darstellt.

16. Die Verbindung oder ein pharmazeutisch verträgliches Salz davon gemäß einem der Ansprüche 1 bis 3, wobei A⁴ die Bedeutung -O- hat.

17. Die Verbindung oder ein pharmazeutisch verträgliches Salz davon gemäß einem der Ansprüche 1 bis 3, wobei G¹, A³ und A⁴ einen von einer Einfachbindung verschiedenen Rest darstellen und G² ein Wasserstoffatom darstellt.

18. Die Verbindung oder ein pharmazeutisch verträgliches Salz davon gemäß einem der Ansprüche 1 bis 3, wobei G¹, A³ und A⁴ eine Einfachbindung darstellen und G² ein Wasserstoffatom darstellt.

19. Die Verbindung oder ein pharmazeutisch verträgliches Salz davon gemäß Anspruch 1, wobei A² die Bedeutung -NH-(C=O)- oder NH-(C=O)-NH- hat, G¹ eine Einfachbindung darstellt und A³ einen zweiwertigen acyclischen aliphatischen Kohlenwasserstoffrest mit 1 bis 10 Kohlenstoffatomen darstellt.

20. Die Verbindung oder ein pharmazeutisch verträgliches Salz davon gemäß Anspruch 1, wobei A² die Bedeutung -NH-(C=O)-, -NH-(C=O)-NH-, -NH- oder -C(=O)-NH- hat und G¹ einen von einer Einfachbindung verschiedenen Rest darstellt.

21. Die Verbindung oder ein pharmazeutisch verträgliches Salz davon gemäß Anspruch 1, wobei G¹ einen gegebenenfalls substituierten heterocyclischen Rest darstellt,
mit der Maßgabe, dass wenn der heterocyclische Rest von G¹ ein 5- oder 6-gliedriger monocyclischer Ring ist, der 5- oder 6-gliedrige monocyclische heterocyclische Rest von G¹ dann substituiert ist oder der A³-G²-Teil diejenigen von einem Wasserstoffatom verschiedenen darstellt.

22. Die Verbindung oder ein pharmazeutisch verträgliches Salz davon gemäß einem der Ansprüche 2 oder 3, wobei G¹ einen gegebenenfalls substituierten aromatischen Kohlenwasserstoffrest, einen gegebenenfalls substituierten alicyclischen Kohlenwasserstof&est mit 7 bis 10 Kohlenstoffatomen oder einen gegebenenfalls substituierten heterocyclischen Rest darstellt,
mit der Maßgabe, dass wenn der aromatische Kohlenwasserstoffrest von G¹ ein Phenylrest ist oder der heterocyclische Rest von G¹ ein 5- oder 6-gliedriger monocyclischer Ring ist, der Phenylrest oder der 5- oder 6-gliedrige monocyclische heterocyclische Rest von G¹ dann substituiert ist oder der A³-G²-Teil diejenigen von einem Wasserstoffatom verschiedenen darstellt.

23. Die Verbindung oder ein pharmazeutisch verträgliches Salz davon gemäß einem der Ansprüche 2 oder 3, wobei G¹ und A⁴ eine Einfachbindung darstellen, A³ einen gegebenenfalls substituierten acyclischen aliphatischen Kohlenwasserstoffrest mit 1 bis 10 Kohlenstoffatomen darstellt, G² einen gegebenenfalls substituierten alicyclischen Kohlenwasserstoffrest mit 5 bis 10 Kohlenstoffatomen, einen gegebenenfalls substituierten aromatischen Kohlenwasserstoffrest oder einen gegebenenfalls substituierten heterocyclischen Rest darstellt.

24. Die Verbindung oder ein pharmazeutisch verträgliches Salz davon gemäß einem der Ansprüche 2 oder 3, wobei G¹ eine Einfachbindung darstellt, A³ einen gegebenenfalls substituierten acyclischen aliphatischen Kohlenwasserstoffrest mit 1 bis 10 Kohlenstoffatomen darstellt, und A⁴ die Bedeutung -C(=O)-, -C(=O)-NR¹²¹-, -C(=S)-NR¹²²-, -C(=NR¹²³)-, -O-C(=O)-, -NR¹²¹-C(=O)-, -NR¹²⁶-S(=O)₂-, -NR¹²⁷-C(=O)-O-, -NR¹²¹-C(=O)-NR¹²¹-, -NR¹³⁰-C(=S)-, -NR¹³¹-C(=S)-NR¹³²-, -S-, -S(=O)-, -S(=O)₂-, -S(=O)₂-NR¹³³- oder -S(=O)₂-O- hat.

25. Die Verbindung oder ein pharmazeutisch verträgliches Salz davon gemäß einem der Ansprüche 1 bis 24, wobei A⁵ eine Einfachbindung darstellt.

26. Die Verbindung oder ein pharmazeutisch verträgliches Salz davon gemäß Anspruch 25, wobei R² einen gegebenenfalls substituierten acyclischen aliphatischen Kohlenwasserstoffrest mit 1 bis 10 Kohlenstoffatomen, einen gegebenenfalls substituierten alicyclischen Kohlenwasserstoffrest mit 3 bis 8 Kohlenstoffatomen, einen gegebenenfalls substituierten aromatischen Kohlenwasserstoffrest mit 6 bis 14 Kohlenstoffatomen oder einen gegebenenfalls substituierten heterocyclischen Rest darstellt.

27. Die Verbindung oder ein pharmazeutisch verträgliches Salz davon gemäß Anspruch 25, wobei R² einen acyclischen aliphatischen Kohlenwasserstoffrest mit 1 bis 10 Kohlenstoffatomen, einen alicyclischen Kohlenwasserstoffrest mit 3 bis 8 Kohlenstoffatomen, einen gegebenenfalls substituierten Phenylrest oder einen gegebenenfalls substituierten heterocyclischen Rest mit 1 oder 2 Atomen, ausgewählt aus der Gruppe bestehend aus einem Sauerstoffatom, einem Stickstoffatom oder einem Schwefelatom, im Ring darstellt.

28. Die Verbindung oder ein pharmazeutisch verträgliches Salz davon gemäß Anspruch 25, wobei R² einen Cyclopropylrest, Cyclobutylrest, Cyclopropylmethylrest, Methylrest, Ethylrest, Vinylrest, Isopropylrest oder 2-Methyl-1-propenylrest darstellt.

29. Die Verbindung oder ein pharmazeutisch verträgliches Salz davon gemäß Anspruch 25, wobei R² einen Thienylrest, einen Pyridylrest, einen Furylrest, einen Pyrrolylrest, einen Pyrazolylrest oder Phenylrest darstellt; von denen jeder ferner mit einem oder mehreren aus einem Alkylrest mit 1 bis 4 Kohlenstoffatomen, einem Alkoxyrest mit 1 bis 4 Kohlenstoffatomen, einem Acylrest mit 2 bis 4 Kohlenstoffatomen, einer Hydroxygruppe, einer Carboxylgruppe, einem Alkoxycarbonylrest mit 2 bis 7 Kohlenstoffatomen, einem Fluoratom oder einem Chloratom substituiert sein kann.

30. Die Verbindung oder ein pharmazeutisch verträgliches Salz davon gemäß Anspruch 1, wobei R³ einen Thienylrest, einen Pyridylrest, einen Furylrest, einen Pyrrolylrest, einen Pyrazolylrest oder Phenylrest darstellt; von denen jeder ferner mit einem oder mehreren aus einem Alkylrest mit 1 bis 4 Kohlenstoffatomen substituiert sein kann.

31. Die Verbindung oder ein pharmazeutisch verträgliches Salz davon gemäß Anspruch 1, wobei R³ einen Pyridylrest oder einen 1-Oxypyridylrest, von denen jeder ferner mit einem Alkylrest mit 1 bis 4 KöhlenstofFatomen oder einem Halogenatom substituiert sein kann; einen Pyrazolylrest; oder N-Methylpyrazolylrest darstellt.

32. Die Verbindung oder ein pharmazeutisch verträgliches Salz davon gemäß Anspruch 28, wobei R³ einen Pyridylrest oder einen 1-Oxypyridylrest, von denen jeder ferner mit einem Alkylrest mit 1 bis 4 Kohlenstoffatomen oder einem Halogenatom substituiert sein kann; einen Pyrazolylrest; oder N-Methylpyrazolylrest darstellt.

33. Die Verbindung oder ein pharmazeutisch verträgliches Salz davon gemäß Anspruch 29, wobei R³ einen Pyridylrest oder einen 1-Oxypyridylrest, von denen jeder ferner mit einem Alkylrest mit 1 bis 4 Kohlenstoffatomen oder einem Halogenatom substituiert sein kann; einen Pyrazolylrest; oder N-Methylpyrazolylrest darstellt.

34. Die Verbindung oder ein pharmazeutisch verträgliches Salz davon gemäß einem der Ansprüche 19 bis 24, wobei A⁵ eine Einfachbindung darstellt.

35. Die Verbindung oder ein pharmazeutisch verträgliches Salz davon gemäß Anspruch 34, wobei R² einen gegebenenfalls substituierten acyclischen aliphatischen Kohlenwasserstoffrest mit 1 bis 10 KohlenstofFatomen, einen gegebenenfalls substituierten alicyclischen Kohlenwasserstoffrest mit 3 bis 8 Kohlenstoffatomen, einen gegebenenfalls substituierten aromatischen Kohlenwasserstoffrest mit 6 bis 14 Kohlenstoffatomen oder einen gegebenenfalls substituierten heterocyclischen Rest darstellt und R³ einen gegebenenfalls substituierten aromatischen Kohlenwasserstoffrest mit 6 bis 14 Kohlenstoffatomen oder einen gegebenenfalls substituierten heterocyclischen Rest darstellt.

36. Die Verbindung oder ein pharmazeutisch verträgliches Salz davon gemäß Anspruch 34, wobei R² einen gegebenenfalls substituierten acyclischen aliphatischen Kohlenwasserstoffrest mit 1 bis 10 Kohlenstoffatomen, einen alicyclischen Kohlenwasserstoffrest mit 3 bis 8 Kohlenstoffatomen, einen gegebenenfalls substituierten Phenylrest oder einen gegebenenfalls substituierten heterocyclischen Rest mit 1 oder 2 Atomen, ausgewählt aus der Gruppe bestehend aus einem Sauerstoffatom, einem Stickstoffatom und einem Schwefelatom, im Ring darstellt und R³ einen Thienylrest, einen Pyridylrest, einen Furylrest, einen Pyrrolylrest, einen Pyrazolylrest oder einen Phenylrest darstellt, von denen jeder ferner mit einem oder mehreren aus einem C₁-C₄-Alkylrest substituiert sein kann.

37. Die Verbindung oder ein pharmazeutisch verträgliches Salz davon gemäß Anspruch 34, wobei R² einen Cyclopropylrest, Methylrest, Ethylrest, Vinylrest, Isopropylrest, Isobutylrest oder 2-Methyl-1-propenylrest darstellt und R³ einen Pyridylrest oder einen 1-Oxypyridylrest, von denen jeder ferner mit einem Alkylrest mit 1 bis 4 Kohlenstoffatomen oder einem Halogenatom substituiert sein kann; Pyrazolylrest; oder N-Methylpyrazolylrest darstellt.

38. Die Verbindung oder ein pharmazeutisch verträgliches Salz davon gemäß Anspruch 34, wobei R² einen Thienylrest, einen Pyridylrest, einen Furylrest, einen Pyrrolylrest, einen Pyrazolylrest oder einen Phenylrest darstellt, von denen jeder ferner mit einem oder mehreren aus einem Alkylrest mit 1 bis 4 Kohlenstoffatomen, einem Alkoxyrest mit 1 bis 4 Kohlenstoffatomen oder einem Chloratom substituiert sein kann, und R³ einen Pyridylrest oder einen 1-Oxypyridylrest, von denen jeder ferner mit einem Alkylrest mit 1 bis 4 Kohlenstoffatomen oder einem Halogenatom substituiert sein kann; Pyrazolylrest; oder N-Methylpyrazolylrest darstellt.

39. Ein Arzneimittel, umfassend die Verbindung oder ein pharmazeutisch verträgliches Salz davon gemäß einem der Ansprüche 1 bis 38; und einen pharmazeutisch verträglichen Träger.

40. Ein GSK-3-Inhibitor, umfassend die Verbindung oder ein pharmazeutisch verträgliches Salz davon gemäß einem der Ansprüche 1 bis 38.

41. Ein Mittel zur Behandlung oder Vorbeugung einer GSK-3-vermittelten Krankheit, umfassend die Verbindung oder ein pharmazeutisch verträgliches Salz davon gemäß einem der Ansprüche 1 bis 38, zur Verwendung bei einem Verfahren zur Behandlung oder Vorbeugung einer GSK-3-vermittelten Krankheit, ausgewählt aus der Gruppe bestehend aus Diabetes, diabetischen Komplikationen, Alzheimer-Krankheit, neurodegenerativer Erkrankung, manisch-depressiver Psychose, Hirn-Trauma, Alopezie, entzündlicher Erkrankung, Krebs und Immundefekt.

42. Die Verwendung eines Mittels, umfassend die Verbindung oder ein pharmazeutisch verträgliches Salz davon gemäß einem der Ansprüche 1 bis 38, zur Herstellung eines Medikaments für die Behandlung oder Vorbeugung einer GSK-3-vermittelten Krankheit, ausgewählt aus der Gruppe bestehend aus Diabetes, diabetischen Komplikationen, Alzheimer-Krankheit, neurodegenerative Erkrankung, manisch-depressiver Psychose, Hirn-Trauma, Alopezie, entzündlicher Erkrankung, Krebs und Immundefekt.

43. Eine Verbindung, dargestellt durch die Formel (Ic): 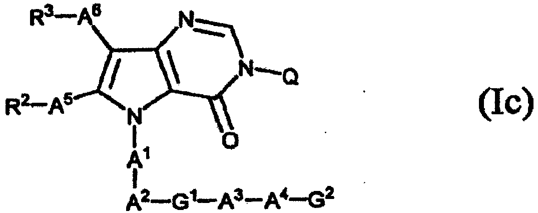 wobei A¹, A², A³, A⁴, A⁵, A⁶, G¹, G², R², R³ und X wie in Formel (I) definiert sind; und Q einen gegebenenfalls substituierten Acylrest mit 2 bis 10 Kohlenstoffatomen, einen gegebenenfalls substituierten Alkoxymethylrest mit 2 bis 10 Kohlenstoffatomen oder einen gegebenenfalls substituierten Benzylrest darstellt.

## Revendications

1. Un composé représenté par la formule (I) ou un sel pharmaceutiquement acceptable de celui-ci : 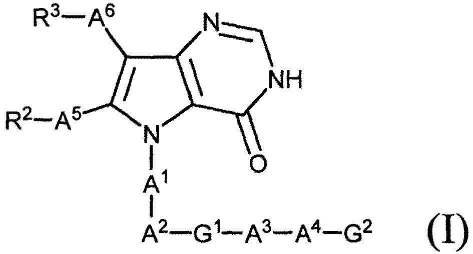 dans laquelle
A¹ est -(CH₂)₂- ou -(CH₂)₃-;
A² représente un groupe qui lie A¹ à G¹ sous la forme de A¹-C(=O)-G¹, A¹-C(=O)-OG¹, A¹-C(=O)-NR¹⁰¹-G¹, A¹-C(=S)-NR¹⁰²-G¹, A¹-C(=NR¹⁰³)-G¹, A¹-O-G¹, A¹-O-C(=O)-G¹, A¹-NR¹⁰⁴-G¹, A¹-NR¹⁰⁵-C(=O)-G¹, A¹-NR¹⁰⁶-S(=O)₂-G¹, A¹-NR¹⁰⁷-C(=O)-OG¹, A¹-NR¹⁰⁸-C(=O)-NR¹⁰⁹-G¹, A¹-NR¹¹⁰C(=S)-G¹, A¹-NR¹¹¹-C(=S)-NR¹¹²-G¹, A¹-S-G¹, A¹-S(=O)-G¹, A¹-S(=O)₂-G¹, A¹-S(=O)₂-NR¹¹³-G¹, A¹-CR¹¹⁴=CH-G¹, A¹-CR¹¹⁵=CF-G¹, A¹-CH=CR¹¹⁶-G¹, ou A¹-CF=CR¹¹⁷'-G¹;
G¹ représente une liaison simple ou représente un groupe divalent qui peut être obtenu par élimination de deux atomes d'hydrogène de l'un quelconque d'un groupe hydrocarboné alicyclique éventuellement substitué ayant 3 à 10 atomes de carbone, un groupe hydrocarboné aromatique éventuellement substitué ayant 6 à 14 atomes de carbone, et un composé hétérocyclique éventuellement substitué ayant 1 à 4 atomes choisis dans le groupe consistant en un atome d'oxygène, un atome d'azote et un atome de soufre, dans le cycle,
dans laquelle le substituant dans le groupe hydrocarboné alicyclique substitué ayant 3 à 10 atomes de carbone de G¹ comprend : un atome de fluor, un atome de chlore, un atome de brome, un atome d'iode, un groupe hydroxy, méthoxy, éthozy, propoxy, isopropozy, butoxy, isobutoxy, s-butoxy, t-butoxy, pentyloxy, isopentyloxy, néopentyloxy, t-pentyloxy, hexyloxy, isohexyloxy, 2-méthyl-pentyloxy, 1-éthylbutoxy, cyclopropyloxy, cyclobutyloxy, cyclopentyloxy, cyclohexyloxy, cyclopropylméthyloxy, cyclopropylethyloxy, cyclopentylmethyloxy et cyclohexylmethyloxy ou un autre groupe alcoxy en C₁-C₇ consistant en un alhyle linéaire ou ramifié, cycloalkyle et un groupe oxy, éthylènedioxy ou un autre groupe alkylènediozy en C₁-C₄, phénoxy, 1-naphtoxy et de 2-naphtoxy ou un autre groupe aryloxy en C₆-C₁₀, benzyloxy, α-phénéthyloxy, β-phénéthyloxy et phénylpropyloxy ou un autre groupe aralkoxy C₇-C₉, acétoxy, propionyloxy, butyryloxy, isobutyryloxy, valéryloxy, isovaléryloxy, pivaloyloxy et hezanoyloxy ou un autre groupe acyloxy en C₂-C₇, oxo, méthylsulfonyloxy, éthylsulfonyloxy, propylsulfonyloxy, butylsulfonyloxy et t-butylsulfbnyloxy ou un autre groupe alkylsulfonyloxy en C₁-C₆ consistant en un alkyle linéaire ou ramifié et un groupe sulfbnyloxy, un groupe acétyle, propionyle, butyryle, isobutyryle, valéryle, isovaléryle, hexanoyle et pivaroyl ou un autre groupe acyle en C₂-C₇, un groupe carboxy, méthoxycarbonyle, éthoxycarbonyle, propoxycarbonyle, isopropoxycarbonyle, butoxycarbonyle, isobutoxycarbonyle, s-butoxycarbonyle et tert-butoxycarbonyle ou un autre groupe alcoxycarbonyle en C₂-C₇ consistant en un alkyle linéaire ou ramifié et un groupe oxycarbonyle, un groupe carbamoyle, N-méthylcarbamoyle, N-éthylcarbamoyle, N-propylcarbamoyle, N-isopropylcarbamoyle, N-butylcarbamoyle, N-isobutylcarbamoyle, N-s-butylcarbamoyle, le N-t-butylcarbamoyl, N-pentylcarbamoyle, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-diméthylcarbamoyle, N-éthyl-N-méthylcarbamoyle, N,N-diéthylcarbamoyle, et N,N-dipropylcarbamoyle ou un autre groupe alkylcarbamoyle en C₂-C₇ consistant en un alkyle linéaire ou ramifié, cycloalkyle et un groupe carbamoyle, un groupe amino, méthylamino, éthylamino, propylamino, isopropylamino, butylamino, isobutylamino, s-butylamino, t-butylamino, pentylamino, hexylamino, cyclopropylamino, cyclobutylamino, cyclopentylamino, cyclohezylamino, cyclopropylmethylamino, diméthylamino, N-éthylméthylamino, diéthylamino, N-méthylpropylamino, N-methylisopropylamino, N-methylbutyl-amino, N-méthyl-t-butylamino, N-éthylisopropylamino, dipropylamino, diisopropylamino et ethylbutylamino ou un autre groupe alkylamino en C₁-C₆ consistant en un alkyle linéaire ou ramifié, cycloalkyle et un groupe amino, acétylamino, propionylamino, butyrylamino, isobutyrylamino, valérylamino et hexanoylamino ou un autre groupe acylamino en C₂-C₇, méthoxycarbonylamino, éthoxycarbonylamino et t-butoxycarbonylamino ou un autre groupe alcoxycarbonylamino en C₂-C₈, un méthylsulfonylamino, éthylsulfonylamino, butylsulfonylamino et t-butylsulfonylamino ou un autre groupe alkylsulfonylamino en C₁-C₆, un groupe cyano, un groupe nitro, un groupe méthylthio, éthylthio, propylthio, isopropylthio, butylthio, isobutylthio, s-butylthio, t-butylthio, pentylthio et hexylthio ou un autre groupe alkylthio C₁-C₆, methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl et cyclopentylsulfynyl ou un autre groupe alkyle en C₁-C₆ alkylsulfynyl consistant en un alkyle linéaire ou ramifié, cycloalkyle et un groupe sulfynyl, méthylsulfonyle, éthylsulfonyle, propylsulfonyle, isopropylsulfonyle, butyl-sulfonyle, isobutylsulfonyl, s-butylsultonyle, t-butylsultonyle, pentylsultonyle, hexylsultonyl, cyclopentylsulfonyl et cyclohexylsulfonyl ou un autre groupe alkylsulfonyle en C₁-C₆ consistant en un alkyle linéaire ou ramifié, cycloalkyle et un groupe sulfonyle, un groupe sulfo, un groupe sulfamoyle, méthylaminosulfonyle, éthylaminosulfonyle, propylaminosulfonyl, isopropylaminosulfonyl, butylaminosulfonyle, isobutylamino-sulfonyle, s-butylaminosulfonyle, pentylaminosulfonyl, diméthylaminosulfonyle, N-éthyl-N-méthylaminosulfonyle, diethylaminosulfonyl, dipropylaminosulfonyl, cyclopropylaminosulfonyl, cyclopentylaminosulfonyl, cyclohexylaminosulfonyl et cyclopropylmethylaminosulfonyl ou un autre groupe aminosulfonyle en C₁-C₆ consistant en un alkyle linéaire ou ramifié, un groupe cycloalkyle et aminosulfonyle, un groupe cyclopropyle, cyclobutyle, cyclopentyle et cyclohexyle, ou un autre groupe hydrocarboné alicyclique ayant 3 à 6 atomes de carbone, un groupe méthyle, éthyle, vinyle, éthynyle, propyle, 1-propényle, 2-propényle, un groupe isopropyle, isopropényle, 1-propynyle, 2-propynyle, butyle, isobutyle, s-butyle, t-butyle, 1-butényle, 2-butényle, 3-butényle, 1-méthyl-1-propényle, 2-méthyl-1-propényle, 1-méthyl-2 -propényle, 2-méthyl-2-propényle, 1-butynyle, 2-butynyle, pentyle, isopentyle, néopentyle, t-pentyle, 1-pentényle, 2-pentényle, 3-pentényle, 4-pentényle, hexyle, 5-hexényle, 4-méthyl-3-pentényle, iso-hexyle, 2-méthylpentyle et 1-éthylbutyle ou un autre groupe hydrocarboné aliphatique ayant 1 à 6 atomes de carbone qui peut contenir une liaison insaturée linéaire ou ramifiée ;
dans laquelle le substituant du groupe hydrocarboné alicyclique substitué ayant 3 à 10 atomes de carbone en tant que G¹, un groupe alcoxy en C₁-C₇, un groupe acyle en C₂-C₇, un groupe alkylcarbamoyle en C₂-C₇, un groupe alkylamino en C₁-C₆, un groupe acylamino en C₂-C₇, un groupe hydrocarboné alicyclique ayant 3 à 6 atomes de carbone ou un groupe hydrocarboné aliphatique ayant 1 à 6 atomes de carbone, peut en outre être substitué par un ou plusieurs substituants choisis dans le groupe consistant en un atome de fluor, un atome de chlore, un atome de brome, un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentyloxy ou cyclopropyloxy, un groupe acyle en C₂-C₇ tel que le groupe méthoxyméthyloxy, un groupe 2-méthoxyéthoxy, un groupe formyle, un groupe trifluoroacétyle, un groupe acétyle, propionyle, butyryle, isobutyryle, valéryle ou isovaléryle, un groupe oxo, un groupe carboxyl, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyie en C₂-C₇ tel que N-méthylcarbamoyle, N,N-diméthylcarbamoyle, N-éthylcarbamoyle, N-éthyl-N-méthylcarbamoyle, N,N-diéthyl-carbamoyle, N-propylcarbamoyle, N-isopropylcarbamoyle, N-butylcarbamoyle, N-cyclopropylcarbamoyl ou N-cyclopropyl-méthylcarbamoyle, un groupe amino, un groupe alkylamino en C₁-C₆ tel que méthylamino, éthylamino, propylamino, isopropylamino, diméthylamino, N-éthylméthylamino, diéthylamino, N-méthyl-propylamino, N-methylisopropylamino, cyclopropylamino ou cyclopropylmethylamino, un groupe amino cyclique en C₁-C₆ ayant 1 ou 2 atomes choisis dans le groupe consistant en un atome d'oxygène, un atome d'azote et un atome de soufre, dans le cycle, tels que le 1-pyrrolidinyle, pipérazinyle, méthyl-4 pipérazinyle, pipéridino ou morpholino, un groupe acylamino en C₁-C₇ tel que le groupe trifluoroacétylamino, formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro et un groupe cyano ;
dans laquelle les substituants du groupe hydrocarboné aromatique substitué ayant 6 à 14 atomes de carbone de G¹ comprend un atome de fluor, un atome de chlore, un atome de brome, un atome d'iode, un groupe hydroxy, un groupe alcoxy en C₁-C₇ éventuellement substitué, un groupe C₆₋C₁₀ aryloxy, un groupe aralcoxy en C₇-C₉, un groupe acyloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle C₂-C₇, un groupe carbamoyle, un groupe éventuellement substitué en C₂ à C₇-carbamoyle, un groupe amino, un groupe alkylamino en C₁-C₆ éventuellement substitué, un groupe acylamino en C₂-C₇ éventuellement substitué, un groupe alkoxy en C₂-C₈, un alkyle en C₁-C₆ un groupe alkylsulfonylamino, un groupe cyano, un groupe nitro, un groupe alkylthio en C₁-C₆, un groupe alkylsulfynyl en C₁-C₆, un groupe alkylsulfonyle en C₁-C₆, un groupe sulfamoyle, un groupe alkylaminosulfonyle en C₁-C₆, un groupe sulfo, un groupe hydrocarboné alicyclique éventuellement substitué ayant 3 à 6 atomes de carbone et un groupe hydrocarboné aliphatique éventuellement substitué ayant de 1 à 6 atomes de carbone ;
dans laquelle le substituant du groupe hydrocarboné aromatique substitué ayant 6 à 14 atomes de carbone, le groupe alcoxy en C₁-C₇, le groupe acyle en C₂-C₇, le groupe alkylcarbamoyle en C₂-C₇, le groupe alkylamino en C₁-C₆, le C₂-C₇ groupe acylamino, le groupe hydrocarboné alicyclique ayant de 3 à 6 atomes de carbone ou le groupe hydrocarboné aliphatique ayant 1 à 6 atomes de carbone, peut en outre être substitué par un ou plusieurs substituants choisis dans le groupe consistant en un atome de fluor, un atome de chlore, un atome de brome, un atome d'iode, un groupe hydroxy, un groupe alkoxy en C₁-C₆ tel que méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthyl-carbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre, dans le cycle, tel que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro et un groupe cyano ;
dans laquelle le substituant des exemples du groupe hétérocyclique ayant 1 à 4 atomes choisis dans le groupe consistant en un atome d'oxygène, un atome d'azote et un atome de soufre sur le cycle substitué de G¹, comprend un atome de fluor, un atome de chlore, un atome de brome, un atome d'iode, un groupe hydroxy, un groupe alcoxy en C₁-C₇ éventuellement substitué, un groupe aryloxy en C₆-C₁₀, un groupe aralcoxy en C₇-C₉, un groupe acyloxy en C₂-C₇, un groupe oxo, un alkyle en C₁-C₆ groupe alkylsulfonyloxy, un groupe acyle en C₂-C₇ éventuellement substitué, un groupe carboxyle, un groupe alcoxycarbonyle en C₂-C₇, un groupe carbamoyle, un groupe alkylcarbamoyle en C₂-C₇ éventuellement substitué, un groupe amino, un groupe alkylamino en C₁-C₆ éventuellement substitué, un C₂-C₇ groupe acylamino, un groupe alcoxycarbonylamino en C₂-C₈, un groupe alkylsulfonylamino en C₁-C₆, un groupe cyano, un groupe nitro, un groupe alkylthio en C₁-C₆, un groupe alkylsulfynyl en C₁-C₆, un groupe alkylsulfonyle en C₁-C₆ éventuellement substitué, un groupe sulfamoyle, un groupe alkylaminosulfonyle en C₁-C₆, un groupe sulfo, un groupe hydrocarboné alicyclique éventuellement substitué, ayant 3 à 6 atomes de carbone, et un groupe hydrocarboné aliphatique éventuellement substitué ayant de 1 à 6 atomes de carbone.
dans laquelle le substituant du groupe hétérocyclique ayant 1 à 4 atomes choisis dans le groupe consistant en un atome d'oxygène, un atome d'azote et un atome de soufre sur le cycle substitué de G¹, un groupe alkyle en C₁-C₇ alkoxy, un C₂-C₇ groupe acyle, un groupe C₂-C₇-carbamoyle, un groupe alkylamino en C₁-C₆, un groupe acylamino en C₂-C₇ un groupe hydrocarboné alicyclique ayant 3 à 6 atomes de carbone et un groupe hydrocarboné aliphatique ayant 1 à 6 atomes de carbone peuvent en outre être substitués avec un ou plusieurs substituants choisis dans le groupe consistant en un atome de fluor, un atome de chlore, un atome de brome, un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentylozy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthylcarbamoyle, N-propylcarbamoyle, N-isopropyl-carbamoyle, N-butylcarbamoyle, N-cyclopropylcarbamoyl, ou N-cyclopropylmethylcarbamoyl ; un groupe amino ; un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis parmi le groupe consistant en un atome d'oxygène, un atome d'azote et un atome de soufre, dans le cycle, tels que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro et un groupe cyano ;
A³ représente une liaison simple ou représente un groupe hydrocarboné divalent acyclique aliphatique éventuellement substitué ayant 1 à 10 atomes de carbone qui relie G¹ avec A⁴ sur le même ou un autre atome de carbone ;
dans laquelle les substituants du groupe hydrocarboné aliphatique divalent substitué ayant 1 à 10 atomes de carbone de A³ comprend un groupe hydrocarboné ayant 1 à 6 atomes de carbone, un groupe hydrocarboné alicyclique ayant 3 à 6 atomes de carbone, un atome d'halogène, un groupe alcoxy ayant 1 à 6 atomes de carbone, un groupe phénoxy, un groupe amino ou un groupe amino-alkyle ayant 1 à 6 atomes de carbone ;
A⁴ représente une liaison simple ou représente un groupe qui lie A³ avec G² sous la forme de A³-C(=O)-G², A³-C(=O)-O-G², A³-C(=O)-NR¹²¹-G², A³-C(=S)-NR¹²²-G², A³-C(=NR¹²³)-G², A³-O-G², A³-O-C(=O)-G², A³-NR¹²⁴-G², A³-NR¹²⁵-C(=O)-G², A³-NR¹²⁶-S(=O)₂-G², A³-NR¹²⁷-C(=O)-O-G², A³-NR¹²⁸-C(=O)-NR¹²⁹-G², A³-NR¹³⁰-C(=S)-G², A³-NR¹³¹-C(=S)-NR¹³²-G², A³-S-G², A³-S(=O)-G², A³-S(=O)₂-G², A³-S(=O)₂-NR¹³³-G² ou A³-S(=O)₂-O-G²;
G² est un atome d'hydrogène, un groupe hydrocarboné aliphatique acyclique éventuellement substitué ayant 1 à 10 atomes de carbone, un groupe hydrocarboné alicyclique éventuellement substitué, ayant 3 à 10 atomes de carbone, un groupe hydrocarboné aromatique éventuellement substitué ayant 6 à 14 atomes de carbone, ou un groupe hétérocyclique éventuellement substitué ayant 1 à 4 atomes choisis dans le groupe consistant en un atome d'oxygène, un atome d'azote et un atome de soufre, dans le cycle ;
dans laquelle les substituants du groupe hydrocarboné aliphatique acyclique substitué ayant 1 à 10 atomes de carbone de G² comprennent: un atome de fluor, un atome de chlore, un atome de brome, un atome d'iode, un groupe hydroxy, un groupe alcoxy en C₁-C₇ groupe consistant en un groupe alkyle linéaire ou ramifié, un groupe cycloalkyle et un groupe oxy, notamment méthoxy, éthoxy, propoxy, isopropoxy, butoxy, isobutoxy, s-butoxy, t-butoxy, pentyloxy, isopentyloxy, néopentyloxy, t-pentyloxy, hexyloxy, isohexyloxy, 2-méthylpentyloxy, 1-éthylbutoxy, cyclopropyloxy, cyclobutyloxy, cyclopentyloxy, cyclohexyloxy, cyclopropylméthyloxy, cyclopropylethyloxy, cyclopentyle méthyloxy et cyclohexylmethyloxy, un groupe alkyldioxy ayant 1 à 4 atomes de carbone tels que éthylènedioxy ; une C₆-C₁₀ aryloxy, notamment phénoxy, 1-naphtoxy et 2-naphtoxy, un groupe C₇-C₉ aralkoxy, y compris benzylozy, α-phénéthyloxy, β-phénéthyloxy et phénylpropyloxy, un groupe acyloxy en C₂-C₇, y compris acétoxy, propionyloxy, butyryloxy, isobutyryloxy, valéryloxy, isovaléryloxy, pivaloyloxy et hexanoyloxy; une un groupe oxo, un groupe alkylsulfonyloxy en C₁-C₆ consistant enun alkyle linéaire ou ramifié et un groupe sulfonyloxy, y compris un groupe oxo, un groupe méthylsulfonyloxy, éthylsulfonyloxy, propylsulfonyloxy, butylsulfonyloxy et t-butylsulfonyloxy, un groupe C₂-C₇-acyle, notamment acétyle, propionyle, butyryle, isobutyryle, valéryle, isovaléryle, pivaroyl et hexanoyle, un groupe carboxyle, un groupe alcoxycarbonyle en C₂-C₇ consistant en un alkyle linéaire ou ramifié et un groupe oxycarbonyle, notamment un groupe méthoxycarbonyle, éthoxycarbonyle, propoxycarbonyle, isopropoxycarbonyle, butoxycarbonyle, isobutoxycarbonyle, s-butoxycarbonyle et tert-butoxycarbonyle ; un groupe carbamoyle, un groupe alkylcarbamoyle en C₂-C₇ consistant en un alkyle linéaire ou ramifié, cycloalkyle et un groupe carbamoyle, N-méthylcarbamoyle, y compris, N-éthylcarbamoyle, N-propylcarbamoyle, N-isopropylcarbamoyle, N-butylcarbamoyle, N-isobutylcarbamoyle, Ns- butylcarbamoyle, le Nt-butylcarbamoyl, N-pentylcarbamoyle, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-diméthylcarbamoyle, N-éthyl-N-méthylcarbamoyle, N,N-diéthylcarbamoyle, et N,N-dipropylcarbamoyle, un groupe amino, un groupe alkylamino en C₁-C₆ consistant en un alkyle à chaîne droite ou ramifiée, un groupe cycloalkyle et un groupe amino, y compris les groupes méthylamino, éthylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, diméthylamino, N-éthylméthylamino, diéthylamino, N-méthylpropylamino, N-methylisopropylamino, N-méthylbutylamino, le N-méthyl-t-butylamino, N-ethylisopropyl- aminé, dipropylamino, diisopropylamino et ethylbutylamino, un groupe C₂-C₇ acylamino dont acétylamino, propionylamino, butyrylamino, isobutyrylamino, valérylamino et hexanoylamino, un groupe alkoxycarbonylamino C₂-C₈, et notamment méthoxycarbonylamino, éthoxycarbonylamino et t-butoxy-carbonylamino, un C₁-C₆ alkylsulfonylamino groupe comprenant méthylsulfonylamino, éthylsulfonylamino, butylsulfonylamino et t-butylsulfonylamino, un groupe cyano, un groupe nitro, un groupe alkylthio en C₁-C₆, y compris les groupes méthylthio, éthylthio, propylthio, isopropylthio, butylthio, isobutylthio, s-butylthio, t-butylthio, pentylthio et hexylthio, un groupe alkyle en C₁-C₆ alkylsulfynyl consistant en un alkyle linéaire ou ramifié, cycloalkyle et un groupe sulfynyl, y compris methylsulfynyl, ethylsulfynyl, propylsulfynyl, isopropylsulfynyl, butylsulfynyl, isobutylsulfynyl, s-butylsulfynyl, t-butylsulfynyl, pentylsulfynyl et cyclopentylsulfynyl, un alkylsulfonyle en C₁-C₆ groupe consistant en un alkyle linéaire ou ramifié, cycloalkyle et un groupe sulfonyle, y compris un groupe méthylsulfonyle, éthylsulfonyle, propylsulfonyle, isopropylsulfonyle, butylsulfonyle, isobutylsulfonyl, s-butylsulfonyle, t-butylsulfonyle, pentylsulfonyle, hexylsulfonyl, cyclopentylsulfonyl et cyclohexylsulfonyl, un groupe sulfo, un groupe sulfamoyle, un groupe aminosulfonyle en C₁-C₆ consistant en un alkyle linéaire ou ramifié, cycloalkyle et un groupe aminosulfonyle, y compris méthylaminosulfonyle, éthylaminosulfonyle, propylaminosulfonyl, isopropylaminosulfonyl, butylaminosulfonyle, isobutyl-aminosulfonyle, s-butylaminosulfonyle, pentylaminosulfonyl, diméthylaminosulfonyle, N-éthyl-N-méthylaminosulfonyle ,diethylaminosulfonyl, dipropylaminosulfonyl, cyclopropylaminosulfonyl, cyclopentylaminosulfonyl, cyclohexylaminosulfonyl et cyclopropylmethylaminosulfonyl, un groupe hydrocarboné alicyclique ayant 3 à 6 atomes de carbone, notamment un groupe cyclopropyle, cyclobutyle, cyclopentyle et cyclohexyle, et un groupe hydrocarboné aliphatique ayant 1 à 6 atomes de carbone qui peut contenir une chaîne droite ou ramifiée, liaison insaturée, notamment un groupe méthyle, éthyle, vinyle, éthynyle, propyle, 1-propényle, 2-propényle, un groupe isopropyle, isopropényle, 1-propynyle, 2-propynyle, butyle, isobutyle, s-butyle, t-butyle, 1-butényle, 2-butényle, 3-butényle, 1-méthyl-1-propényle, 2-méthyl-1-propényle, 1-méthyl-2-propényle, 2-méthyl-2-propényle, 1-butynyle, 2-butynyle, pentyle, isopentyle, néopentyle, t-pentyle, 1-pentényle, 2-pentényle, 3-pentényle, 4-pentényle, hexyle, 5-hexényle, 4-méthyl-3-pentényle, iso-hexyle, 2-méthylpentyle et 1-éthylbutyle, un groupe hydrocarboné aromatique ayant 6 à 14 atomes de carbone qui est un groupe monovalent dérivé d'un groupe hydrocarboné aromatique monocyclique, bicyclique ou tricyclique, notamment du benzène, du naphtalène, de l'indène, indane, le 1,2,3,4-tétrahydronaphtalène et le fluorène et un groupe monovalent dérivé, composé hétérocyclique bicyclique ou tricyclique monocyclique, comprenant le furanne, le thiophène, le pyrrole, la pyrroline, la pyrrolidine, l'oxazole, oxazolidine, isooxazole, isooxazolidine, le thiazole, la thiazolidine, isothiazole, isothiazolidine, imidazole, imidazoline, imidazolidine, pyrazole, pyrazoline, pyrazolidine, triazole, , thiadiazole, oxadiazole, tétrazole, pyrane, tétrahydropyrane, thiopyrane, tétrahydrothiopyranne, pyridine, pyrazine, pyrimidine, pyridazine, benzofurane, le dibenzofurane, le benzothiophène, l'indole, le benzimidazole, le benzothiazole, le benzooxazole, chromane, isochromane, la quinoléine, décahydroquinoléine, isoquinoléine, quinazoline, quinoxaline , la purine, la ptéridine, l'azétidine, la morpholine, la thiomorpholine, la pipéridine, homopipéridine, pipérazine, homopipérazine, l'indoline, l'isoindoline, la phénoxazine, la phénazine, la phénothiazine et la quinuclidine, le composé hétérocyclique ayant 1 à 4 atomes choisis dans le groupe consistant en un atome d'oxygène, un atome d'azote et un atome de soufre, dans le cycle ;
dans laquelle le substituant du groupe hydrocarboné aliphatique acyclique substitué ayant 1 à 10 atomes de carbone en tant que G², un groupe alcoxy en C₁-C₇, un groupe acyle en C₂-C₇, C₂-C₇-carbamoyle, un groupe alkylamino en C₁-C₆, un groupe acylamino en C₂-C₇, un groupe hydrocarboné alicyclique ayant 3 à 6 atomes de carbone, un groupe hydrocarboné aliphatique ayant 1 à 6 atomes de carbone, un groupe hydrocarboné aromatique ayant 6 à 14 atomes de carbone, et un groupe hétérocyclique ayant 1 à 4 atomes choisis parmi le groupe consistant en un atome d'oxygène, un atome d'azote et un atome de soufre dans le cycle, peut en outre être substitué par un ou plusieurs substituants choisis dans le groupe consistant en: un atome de fluor, un atome de chlore, un atome de brome, un atome d'iode, un groupe hydroxy, un groupe alcoxy C₁-C₆ tels que les radicaux méthoxy, éthoxy, propoxy, isopropoxy, butoxy, isobutoxy, s-butoxy, t-butoxy, pentyloxy ou cyclopropyloxy, un groupe méthoxyméthylozy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthylcarbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino en C₄-C₆ cyclique ayant 1 ou 2 atomes choisis dans le groupe consistant en un atome d'oxygène, un atome d'azote et un atome de soufre, dans le cycle, tels que le 1-pyrrolidinyle, pipérazinyle, méthyl-4 pipérazinyle, pipéridino ou morpholino, un groupe tritluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano, un groupe alkyle en C₁-C₆ notamment méthyle, éthyle, propyle, isopropyle, butyle, isobutyle, s-butyle et t-butyle, un groupe trifluorométhyle et un groupe trifluorométhoxy ;
dans laquelle le substituant du groupe hydrocarboné alicyclique substitué ayant 3 à 10 atomes de carbone de G² comprend un atome de fluor, un atome de chlore, un atome de brome, un atome d'iode, un groupe hydroxy, éventuellement substitué en C₁-C₇ alkoxy groupe alkyle en C₁-C₄ un groupe alkylènedioxy, un groupe C₆-C₁₀ aryloxy, un groupe aralcoxy en C₇-C₉, un groupe acyloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, un groupe alkylcarbamoyle en C₂-C₇ éventuellement substitué, un groupe amino, un groupe alkylamino en C₁-C₆ éventuellement substitué, un groupe acylamino en C₂-C₇ éventuellement substitué, un C₂-C₈ alcoxycarbonylamino groupe, un groupe alkylsulfonylamino en C₁-C₆, un groupe cyano, un groupe nitro, un groupe alkylthio en C₁-C₆, un groupe alkylsulfynyl en C₁-C₆, un groupe alkylsulfonyle en C₁-C₆, un groupe sulfamoyle, un groupe alkylaminosulfonyle en C₁-C₆, un un groupe sulfo, un groupe alicyclique éventuellement substitué hydrocarboné ayant de 3 à 6 atomes de carbone, et un groupe hydrocarboné aliphatique éventuellement substitué ayant de 1 à 6 atomes de carbone, un groupe hydrocarboné aromatique éventuellement substitué ayant 6 à 14 atomes de carbone.
dans laquelle le substituant du groupe hydrocarboné alicyclique substitué ayant 3 à 10 atomes de carbone de G², un groupe alcoxy en C₁-C₇, un groupe acyle en C₂-C₇, un groupe alkylcarbamoyle en C₂-C₇, un alkyle en C₁-C₆ alkylamino groupe, un groupe acylamino en C₂-C₇, un groupe hydrocarboné alicyclique ayant 3 à 6 atomes de carbone, un groupe hydrocarboné aliphatique ayant 1 à 6 atomes de carbone, et un groupe hydrocarboné aromatique ayant 6 à 14 atomes de carbone, peut en outre être substitué par un ou plusieurs substituants choisis dans le groupe consistant en: un atome de fluor, un atome de chlore, un atome de brome, un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle; un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthyl-carbamoyle, N-propylcarbamoyle, N-isopropylcarbamoyle, N-butylcarbamoyle, N-cyclopropylcarbamoyl ou N-cyclopropylmethylcarbamoyl, un groupe amino, un C₁-C₆ alkylamino tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre, dans le cycle, tels que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano, un groupe alkyle en C₁-C₆ notamment méthyle, éthyle, propyle, isopropyle, butyle, isobutyle , s-butyle et t-butyle, un groupe tritluorométhyle et un groupe trifluorométhoxy.
dans laquelle les substituants du groupe hydrocarboné aromatique ayant 6 à 14 atomes de carbone de G² comprennent au moins un substituant choisi dans le groupe consistant en un atome de fluor, un atome de chlore, un atome de brome, un atome d'iode, un groupe hydroxy groupe, un groupe alcoxy en C₁-C₇ éventuellement substitué, un groupe alkylènedioxy en C₁-C₄, un groupe C₆-C₁₀ aryloxy, un groupe aralcoxy en C₇-C₉, un groupe acyloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, un groupe alkylcarbamoyle en C₂-C₇ éventuellement substitué, un groupe amino, un groupe alkylamino en C₁-C₆ éventuellement substitué, un groupe acylamino en C₂-C₇ éventuellement substitué, un groupe alcoxycarbonylamino en C₂-C₈, un groupe alkylsulfonylamino en C₁-C₆, un groupe cyano, un groupe nitro, un groupe alkylthio en C₁-C₆, un groupe alkylsulfynyl C₁-C₆, un groupe alkylsulfonyle en C₁-C₆, un sulfamoyle groupe, un groupe alkylaminosulfonyle en C₁-C₆, un groupe sulfo, un groupe hydrocarboné alicyclique éventuellement substitué, ayant 3 à 6 atomes de carbone, un groupe hydrocarboné aliphatique éventuellement substitué ayant de 1 à 6 atomes de carbone, et un groupe hydrocarboné aromatique éventuellement substitué ayant de 6 à 14 atomes de carbone ;
dans laquelle les substituants du groupe hydrocarboné aromatique ayant 6 à 14 atomes de carbone de G², un groupe alcoxy en C₁-C₇, un groupe acyle en C₂-C₇, un groupe alkylcarbamoyle en C₂-C₇, un groupe alkylamino en C₁-C₆, un groupe acylamino en C₂-C₇, un groupe hydrocarboné alicyclique ayant 3 à 6 atomes de carbone, un groupe hydrocarboné aliphatique ayant 1 à 6 atomes de carbone, et un groupe hydrocarboné aromatique ayant 6 à 14 atomes de carbone, peut en outre être substitué par un ou plusieurs substituants choisis dans le groupe consistant en: un atome de fluor, un atome de chlore, un atome de brome, un atome d'iode, un groupe hydroxy, un groupe alkoxy en C₁-C₆ tel que méthoxy, éthoxy, propoxy, isopropoxy, butoxy, isobutoxy, s-butoxy, t-butoxy, pentyloxy ou cyclopropylozy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle; une un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthyl-carbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre, dans le cycle, tels que le 1-pyrrolidinyle, pipérazinyle, méthyl-4-pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano, un groupe alkyle C₁-C₆, y compris un groupe méthyle, éthyle, propyle, isopropyle, butyle, isobutyle, s-butyle et t-butyle, un groupe trifluorométhyle et un groupe trifluorométhoxy ;
dans laquelle les substituants du groupe hétérocyclique ayant 1 à 4 atomes choisis dans le groupe consistant en un atome d'oxygène, un atome d'azote et un atome de soufre sur le cycle substitué de G² comprennent au moins un substituant choisi dans le groupe consistant en d'un atome de fluor, un atome de chlore, un atome de brome, un atome d'iode, un groupe hydroxy, un groupe alkoxy en C₁-C₇ éventuellement substitué, un groupe alkylènedioxy en C₁-C₄, un groupe aryloxy en C₆-C₁₀, un groupe aralcoxy en C₇-C₉, un groupe acyloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, éventuellement substitué en C₂-C₇ alkylcarbamoyle groupe, un groupe amino, un groupe alkylamino en C₁-C₆ éventuellement substitué, un groupe acylamino en C₂-C₇ éventuellement substitué, un groupe alcoxy en C₂-C₈, un groupe alkylsulfonylamino en C₁-C₆, un groupe cyano, un groupe nitro, un alkyle en C₁-C₆ groupe alkylthio, un groupe alkylsulfynyl en C₁-C₆, un groupe alkylsulfonyle en C₁-C₆, un groupe sulfamoyle, un groupe alkylaminosulfonyle en C₁-C₆, un groupe sulfo, un groupe hydrocarboné alicyclique éventuellement substitué ayant 3 à 6 atomes de carbone, un groupe hydrocarboné aliphatique éventuellement substitué, un groupe ayant 1 à 6 atomes de carbone, et un groupe hydrocarboné aromatique éventuellement substitué ayant 6 à 14 atomes de carbone, et ;
dans laquelle le substituant du groupe hétérocyclique ayant 1 à 4 atomes choisis dans le groupe consistant en un atome d'oxygène, un atome d'azote et un atome de soufre sur le cycle substitué de G², un groupe alcoxy en C₁-C₇, un groupe acyle en C₂-C₇, un groupe C₂-C₇-carbamoyle, un groupe alkylamino en C₁-C₆, un groupe acylamino en C₂-C₇, un groupe hydrocarboné alicyclique ayant 3 à 6 atomes de carbone, un groupe hydrocarboné aliphatique ayant 1 à 6 atomes de carbone, et un groupe hydrocarboné aromatique ayant 6 à 14 atomes de carbone, peut en outre être substitué par un ou plusieurs substituants choisis dans le groupe consistant en: un atome de fluor, un atome de chlore, un atome de brome, un atome d'iode, un groupe hydroxy, un C₁-C₆ groupe alkoxy tel que méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe tritluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle ; un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthylcarbamoyle, N-propylcarbamoyle, N-isopropyl-carbamoyle, N-butylcarbamoyle, N-cyclopropylcarbamoyl ou N-cyclopropylmethylcarbamoyl, un groupe amino, un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre, dans le cycle, tels que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano; un groupe alkyle C₁-C₆, y compris un groupe méthyle, éthyle, propyle, isopropyle, butyle, isobutyle, s-butyle et t-butyle, un groupe trifluorométhyle et un groupe trifluorométhoxy.
A⁵ représente une liaison simple ou -NR²⁰¹-
R² est un atome d'hydrogène, un atome de fluor, un atome de chlore, un atome de brome, un atome d'iode, un groupe nitro, un groupe hydrocarboné saturé acyclique aliphatique éventuellement substitué ayant 1 à 10 atomes de carbone, un groupe hydrocarboné alicyclique éventuellement substitué, ayant 3 à 8 atomes de carbone, un groupe hydrocarboné aromatique éventuellement substitué ayant 6 à 14 atomes de carbone, ou un groupe hétérocyclique éventuellement substitué ayant 1 à 4 atomes choisis dans le groupe consistant en un atome d'oxygène, un atome d'azote et un atome de soufre, dans le cycle ;
dans laquelle les substituants pour le groupe hydrocarboné aliphatique acyclique substitué ayant 1 à 10 atomes de carbone de R² comprennent au moins un substituant choisi dans le groupe consistant en un atome de fluor, un atome de chlore, un atome de brome, un atome d'iode, un un groupe hydroxy, un groupe alcoxy en C₁-C₇ éventuellement substitué, un groupe C₆-C₁₀ aryloxy, un groupe aralcoxy en C₇-C₉, un groupe acyloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, un groupe alkylcarbamoyle en C₂-C₇ éventuellement substitué, un groupe amino, un groupe alkylamino en C₁-C₆ éventuellement substitué, un groupe acylamino en C₂-C₇ éventuellement substitué, un groupe C₂-C₈ alcoxycarbonylamino, un groupe alkylsulfonylamino en C₁-C₆, un groupe cyano, un groupe nitro, un groupe alkylthio en C₁-C₆, un groupe alkylsulfynyl en C₁-C₆, un groupe alkylsulfonyle en C₁-C₆, un groupe sulfamoyle, un alkyle en C₁-C₆ un groupe aminosulfonyle, un groupe sulfo, un groupe hydrocarboné alicyclique éventuellement substitué ayant de 1 à 6 atomes de carbone, un groupe hydrocarboné aliphatique acyclique éventuellement substitué ayant de 1 à 6 atomes de carbone, un groupe hydrocarboné aromatique éventuellement substitué ayant 6 à 14 atomes de carbone, et un éventuellement un groupe hétérocyclique substitué ayant 1 à 4 atomes choisis dans le groupe consistant en un atome d'oxygène, un atome d'azote et un atome de soufre, dans le cycle.
dans laquelle le substituant du groupe hydrocarboné aliphatique acyclique substitué ayant 1 à 10 atomes de carbone comme R², un groupe alkyle en C₁-C₇ alkoxy, un groupe acyle en C₂-C₇, C₂-C₇ groupe alkylcarbamoyle, un groupe alkylamino en C₁-C₆ , un groupe acylamino en C₂-C₇, un groupe hydrocarboné alicyclique ayant 3 à 6 atomes de carbone, un groupe hydrocarboné aliphatique acyclique ayant 1 à 6 atomes de carbone, un groupe hydrocarboné aromatique ayant 6 à 14 atomes de carbone, et un groupe hétérocyclique ayant 1 à 4 atomes choisi dans le groupe consistant en un atome d'oxygène, un atome d'azote et un atome de soufre dans le cycle, peut en outre être substitué par un ou plusieurs substituants choisis dans le groupe consistant en: un atome de fluor, un atome de chlore, un brome, atome, un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentyloxy ou cyclopropyloxy, un groupe méthoxyméthyloxy, un 2-méthoxyéthoxy groupe, un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propozycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthyl-carbamoyle, N-propylcarbamoyle, N-isopropylcarbamoyle, N-butylcarbamoyle, N-cyclopropylcarbamoyl ou N-cyclopropyl-méthylcarbamoyle, un groupe amino, un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre, dans le cycle, tel que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano, un groupe alkyle C₁-C₆, y compris un groupe méthyle, éthyle, propyle, isopropyle, butyle, isobutyle, s-butyle et t-butyle, un groupe trifluorométhyle et un groupe trifluorométhoxy ;
dans laquelle les substituants du groupe hydrocarboné alicyclique substitué ayant 3 à 8 atomes de carbone de R² comprennent au moins un substituant choisi dans le groupe consistant en un atome de fluor, un atome de chlore, un atome de brome, un atome d'iode, un un groupe hydroxy, un groupe alcoxy en C₁-C₇ éventuellement substitué, un groupe C₆-C₁₀ aryloxy, un groupe aralcoxy en C₇-C₉, un groupe acyloxy en C₂-C₇, un groupe oxo, un groupe alkylsulfonyloxy en C₁-C₆, éventuellement substitué en C₂-C₇ un groupe acyle, un groupe carboxyle, un groupe alcoxycarbonyle en C₂-C₇, un groupe carbamoyle, un groupe alkylcarbamoyle en C₂-C₇ éventuellement substitué, un groupe amino, un groupe alkylamino en C₁-C₆ éventuellement substitué, un groupe acylamino en C₂-C₇ éventuellement substitué, un C₂-C₈ groupe alcoxycarbonylamino, un groupe alkylsulfonylamino en C₁-C₆, un groupe cyano, un groupe nitro, un groupe alkylthio en C₁-C₆, un groupe alkylsulfynyl en C₁-C₆, un groupe alkylsulfonyle en C₁-C₆, un groupe sulfamoyle, un alkyle en C₁-C₆ un groupe aminosulfonyle, un groupe sulfo, un groupe hydrocarboné alicyclique éventuellement substitué, ayant 3 à 6 atomes de carbone, un groupe hydrocarboné aliphatique éventuellement substitué ayant de 1 à 6 atomes de carbone, un groupe hydrocarboné aromatique éventuellement substitué ayant 6 à 14 atomes de carbone, et un groupe hétérocyclique éventuellement substitué ayant 1 à 4 atomes choisis dans le groupe consistant en un atome d'oxygène, un atome d'azote et un atome de soufre, dans le cycle ;
dans laquelle le substituant du groupe hydrocarboné alicyclique substitué ayant 3 à 8 atomes de carbone de R², un groupe alcoxy en C₁-C₇, un groupe acyle en C₂-C₇, un groupe alkylcarbamoyle en C₂-C₇, un groupe alkylamino en C₁-C₆, un groupe acylamino en C₂-C₇, un groupe hydrocarboné alicyclique ayant 3 à 6 atomes de carbone, un groupe hydrocarboné aliphatique ayant 1 à 6 atomes de carbone, un groupe hydrocarboné aromatique ayant de 6 à 14 atomes de carbone et un groupe hétérocyclique ayant de 1 à 4 atomes choisis dans le groupe consistant en un atome d'oxygène, un atome d'azote et un atome de soufre dans le cycle, peuvent en outre être substitués par un ou plusieurs substituants choisis dans le groupe consistant en: un atome de fluor, un atome de chlore, un un atome de brome, un atome d'iode, un groupe hydroxy, un groupe alcoxy en C₁-C₆ tels que les radicaux méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthylcarbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino en C₁-C₆ tel que méthylamino, éthylamino, propylamino, isopropylamino, diméthylamino, N-éthylméthyl-amino, diéthylamino, N-méthylpropylamino, N-méthylisopropylcétone-amino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre dans le cycle , tel que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano, un groupe alkyle C₁-C₆, y compris un groupe méthyle, éthyle, propyle, isopropyle, butyle, isobutyle, s-butyle et t-butyle, un groupe trifluorométhyle, et un groupe trifluorométhoxy ;
dans laquelle les substituants du groupe hydrocarboné aromatique substitué ayant 6 à 14 atomes de carbone comprennent au moins un substituant choisi dans le groupe consistant en un atome de fluor, un atome de chlore, un atome de brome, un atome d'iode, un groupe hydrm,'Y, un C₁-C₆ alkyle, un groupe alcoxy en C₁-C₇ éventuellement substitué, un groupe C₆-C₁₀ aryloxy, un groupe aralcoxy en C₇-C₉, un groupe acyloxy en C₂-C₇, un groupe oxo, un groupe alkylsulfonyloxy en C₁-C₆, un groupe acyle C₂-C₇ éventuellement substitué, un groupe carbonyle, un groupe alcoxycarbonyle en C₂-C₇, un groupe carbamoyle, un groupe alkylcarbamoyl en C₂-C₇ éventuellement substitué, un groupe amino, un groupe alkylamino en C₁-C₆ éventuellement substitué, un groupe acylamino en C₂-C₇ éventuellement substitué, un groupe alcoxy en C₂-C₈, un groupe alkylsulfonylamino en C₁-C₆, un groupe cyano, un groupe nitro, un groupe alkylthio en C₁-C₆, un groupe alkylsulfynyl en C₁-C₆, un groupe alkylsulfonyle en C₁-C₆, un groupe sulfamoyle, un groupe aminosulfonyle en C₁-C₆, un groupe sulfo, un groupe hydrocarboné alicyclique éventuellement substitué, ayant 3 à 6 atomes de carbone, un groupe hydrocarboné aliphatique éventuellement substitué ayant de 1 à 6 atomes de carbone, un groupe hydrocarboné aromatique éventuellement substitué ayant 6 à 14 atomes de carbone, et un groupe hétérocyclique éventuellement substitué ayant 1 à 4 atomes choisis dans le groupe consistant en un atome d'oxygène, un atome d'azote et un atome de soufre, dans le cycle ;
dans laquelle le substituant du groupe hydrocarboné aromatique ayant de 6 à 14 atomes de carbone de R², un groupe alcoxy en C₁-C₇, un groupe acyle en C₂-C₇, un groupe alkylcarbamoyle en C₂-C₇, un groupe alkylamino en C₁-C₆ , un groupe acylamino en C₂-C₇, un groupe hydrocarboné alicyclique ayant 3 à 6 atomes de carbone, un groupe hydrocarboné aliphatique ayant 1 à 6 atomes de carbone, un groupe hydrocarboné aromatique ayant de 6 à 14 atomes de carbone et un groupe hétérocyclique ayant 1 à 4 atomes choisis dans le groupe consistant en un atome d'oxygène, un atome d'azote et un atome de soufre dans le cycle, peut en outre être substitué par un ou plusieurs substituants choisis dans le groupe consistant en : un atome de fluor, un atome de chlore, un atome de brome , un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy , un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthyl-carbamoyle, N-propylcarbamoyle, N-isopropylcarbamoyle, N-butylcarbamoyle, N-cyclopropylcarbamoyl ou N-cyclopropyl-méthylcarbamoyle, un groupe amino, un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino en C₄-C₆ cyclique ayant 1 ou 2 atomes choisis dans le groupe consistant en un atome d'oxygène, un atome d'azote et un atome de soufre, dans le cycle, tels le 1-pyrrolidinyle, pipérazinyle, 4-méthylpipérazinyle, un groupe pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, propylsulfonylamino ou butylsulfonylamino, un groupe nitro, un groupe cyano, un groupe alkyle C₁-C₆, y compris un groupe méthyle, éthyle, propyle, isopropyle, butyle, isobutyle, s-butyle et t-butyle, un groupe trifluorométhyle et un groupe trifluorométhoxy ;
dans laquelle les substituants du groupe hétérocyclique ayant 1 à 4 atomes choisis dans le groupe consistant en un atome d'oxygène, un atome d'azote et un atome de soufre sur le cycle substitué de R² comprennent au moins un substituant choisi dans le groupe consistant en d'un atome de fluor, un atome de chlore, un atome de brome, un atome d'iode, un groupe hydroxy, un groupe alcoxy en C₁-C₇ éventuellement substitué, un groupe aryloxy en C₆-C₁₀, un groupe aralcoxy en C₇-C₉, un groupe acyloxy en C₂-C₇ , un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, un groupe alkylcarbamoyle en C₂-C₇ éventuellement substitué, un groupe amino, un C₁-C₆ alkylamino, un groupe acylamino en C₂-C₇ éventuellement substitué, un groupe alcoxycarbonylamino en C₂-C₈, un groupe alkylsulfonylamino en C₁-C₆, un groupe cyano, un groupe nitro, un groupe alkylthio en C₁-C₆, un alkyle en C₁-C₆ éventuellement substitué, groupe alkylsulfynyl, un groupe alkylsulfonyle en C1-C6, un groupe sulfamoyle, un groupe alkylaminosulfonyle en C₁-C₆, un groupe sulfo, un groupe hydrocarboné alicyclique éventuellement substitué, ayant 3 à 6 atomes de carbone, un groupe hydrocarboné aliphatique éventuellement substitué ayant de 1 à 6 atomes de carbone , un groupe aromatique hydrocarboné éventuellement substitué ayant de 6 à 14 atomes de carbone, et un groupe hétérocyclique éventuellement substitué ayant 1 à 4 atomes choisis dans le groupe consistant en un atome d'oxygène, un atome d'azote et un atome de soufre, dans le cycle ;
dans laquelle le substituant du groupe hétérocyclique ayant 1 à 4 atomes choisis dans le groupe consistant en un atome d'oxygène, un atome d'azote et un atome de soufre sur le cycle substitué de R², un groupe alcoxy en C₁-C₇, un groupe acyle en C₂-C₇, un groupe carbamoyle en C₂-C₇, un groupe alkylamino en C₁-C₆, un groupe acylamino en C₂-C₇, un groupe hydrocarboné alicyclique ayant 3 à 6 atomes de carbone, un groupe hydrocarboné aliphatique ayant 1 à 6 atomes de carbone, un groupe aromatique hydrocarboné ayant 6 à 14 atomes de carbone, et un groupe hétérocyclique ayant 1 à 4 atomes choisis dans le groupe consistant en un atome d'oxygène, un atome d'azote et un atome de soufre, dans le cycle peuvent en outre être substitués par un ou plusieurs substituants choisis dans le groupe consistant en: un atome de fluor, un atome de chlore, un atome de brome, un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo groupe, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyie en C₂-C₇ tel que N-méthylcarbamoyle, N,N-diméthylcarbamoyle, N-éthylcarbamoyle, N-éthyl-N-méthylcarbamoyle, N,N-diéthylcarbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino en C₁-C₆ tel que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C₄-C₆ ayant 1 ou 2 atomes choisis dans le groupe consistant en un atome d'oxygène atome, un atome d'azote et un atome de soufre, dans le cycle, tels que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylamino, un groupe nitro, un groupe cyano, un groupe alkyle en C₁-C₆ notamment méthyle, éthyle, propyle, isopropyle, butyle, isobutyle, s-butyle et de t-butyle, un groupe trifluorométhyle et un groupe trifluorométhoxy ;
A6 représente une liaison simple ;
R3 est un groupe hydrocarboné aromatique éventuellement substitué ayant 6 à 14 atomes de carbone, ou un hétérocycle éventuellement substitué ayant 1 à 4 atomes choisis parmi le groupe consistant en un atome d'oxygène, un atome d'azote, et un atome de soufre, dans le cycle ;
dans laquelle les substituants du groupe hydrocarboné aromatique substitué ayant 6 à 14 atomes de carbone de R³ comprennent au moins un substituant choisi dans le groupe consistant en un atome de fluor, un atome de chlore, un atome de brome, un atome d'iode, un un groupe hydroxy, un groupe alcoxy en C₁-C₇ éventuellement substitué, un groupe C₆-C₁₀ aryloxy, un groupe aralcoxy en C₇-C₉, un groupe acyloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, un groupe alkylcarbamoyl en C₂-C₇ éventuellement substitué, un groupe amino, un groupe alkylamino en C₁-C₆ éventuellement substitué, un groupe acylamino en C₂-C₇ éventuellement substitué, un groupe alcoxycarbonylamino en C₂-C₈, un groupe alkylsulfonylamino en C₁-C₆, un groupe cyano, un groupe nitro, un groupe alkylthio en C₁-C₆, un groupe alkylsulfynyl en C₁-C₆, un groupe alkylsulfonyle en C₁-C₆, un groupe sulfamoyle, un alkyle en C₁-C₆ un groupe aminosulfonyle, un groupe sulfo, un groupe hydrocarboné alicyclique éventuellement substitué, ayant 3 à 6 atomes de carbone, un groupe hydrocarboné aliphatique éventuellement substitué ayant de 1 à 6 atomes de carbone, un groupe hydrocarboné aromatique éventuellement substitué ayant 6 à 14 atomes de carbone et un groupe hétérocyclique éventuellement substitué, groupe ayant 1 à 4 atomes choisis dans le groupe consistant en un atome d'oxygène, un atome d'azote et un atome de soufre, dans le cycle ;
dans laquelle le substituant du groupe hydrocarboné aromatique substitué ayant 6 à 14 atomes de carbone de R³, un groupe alcoxy en C₁-C₇, un groupe acyle en C₂-C₇, un groupe alkylcarbamoyle en C₂-C₇, un alkyle en C₁-C₆ alkylamino groupe, un groupe acylamino en C₂-C₇, un groupe hydrocarboné alicyclique ayant 3 à 6 atomes de carbone, un groupe hydrocarboné aliphatique ayant 1 à 6 atomes de carbone, un groupe hydrocarboné aromatique ayant de 6 à 14 atomes de carbone et un groupe hétérocyclique ayant de 1 à 4 atomes choisis dans le groupe consistant en un atome d'oxygène, un atome d'azote et un atome de soufre dans le cycle, peuvent en outre être substitués par un ou plusieurs substituants choisis dans le groupe consistant en: un atome de fluor, un atome de chlore, un un atome de brome, un atome d'iode, un groupe hydroxy, un groupe alcoxy C₁-C₆ tels que les radicaux méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo, un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butozycarbonyle, un groupe carbamoyle, un groupe alkylcarbamoyle en C₂-C₇ tel que N-méthylcarbamoyle, N,N-diméthylcarbamoyle, N-éthylcarbamoyle, N-éthyl-N-méthylcarbamoyle, N,N-diéthylcarbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino en C₁-C₆ tel que méthylamino, éthylamino, propylamino, isopropylamino, diméthylamino, N-éthylméthyl-amino, diéthylamino, N-méthylpropylamino, N-méthylisopropylcétone-amino, cyclopropylamino ou cyclopropylmethylamino, un groupe amino cyclique en C₄-C₆ ayant 1 ou 2 atomes choisis dans le groupe consistant en un atome d'oxygène, un atome d'azote et un atome de soufre dans le cycle , tel que le 1-pyrrolidinyle, pipérazinyle, méthyl-4 pipérazinyle, pipéridino ou morpholino, un groupe trifluoroacétylamino, un groupe acylamino en C₁-C₇ tel que formylamino, acétylamino, propionylamino, butyrylamino, isobutyrylamino ou valérylamino, un groupe alkyle en C₁-C₆ alkylsulfonylamino tel que méthylsulfonylamino, éthylsulfonylamino, butylsulfonylamino ou propylsulfonylamino, un groupe nitro, un groupe cyano, un groupe alkyle C₁-C₆, y compris un groupe méthyle, éthyle, propyle, isopropyle, butyle, isobutyle, s-butyle et t-butyle, un groupe trifluorométhyle et un groupe trifluorométhoxy.
dans laquelle les substituants du groupe hétérocyclique ayant 1 à 4 atomes choisis dans le groupe consistant en un atome d'oxygène, un atome d'azote et un atome de soufre sur le cycle de R³ comprennent au moins un substituant choisi dans le groupe consistant en un atome de fluor, un atome de chlore, un atome de brome, un atome d'iode, un groupe hydroxy, un groupe alcoxy en C₁-C₇ éventuellement substitué, un groupe aryloxy en C₆-C₁₀, un groupe aralcoxy, un groupe aryloxy en C₂-C₇, un groupe oxo, un groupe alkylsulfonyloxy en C₁-C₆, un groupe acyle en C₂-C₇ éventuellement substitué, un groupe carboxyle, un groupe alcoxycarbonyle en C₂-C₇, un groupe carbamoyle, un groupe alkylcarbamoyle en C₂-C₇ éventuellement substitué, un groupe amino, un éventuellement C₁-C₆ alkylamino, un groupe éventuellement substitué en C₂-C₇ acylamino, un groupe alcoxycarbonylamino en C₂-C₈, un groupe alkylsulfonylamino en C₁-C₆, un groupe cyano, un groupe nitro, un groupe alkylthio en C₁-C₆, un alkyle en C₁-C₆ substitué alkylsulfynyl groupe, un groupe alkylsulfonyle en C₁-C₆, un groupe sulfamoyle, un groupe alkylaminosulfonyle en C₁-C₆, un groupe sulfo, un groupe hydrocarboné alicyclique éventuellement substitué, ayant 3 à 6 atomes de carbone, un groupe hydrocarboné aliphatique éventuellement substitué ayant de 1 à 6 atomes de carbone, un groupe hydrocarboné aromatique éventuellement substitué ayant 6 à 14 atomes de carbone et un groupe hétérocyclique éventuellement substitué ayant 1 à 4 atomes choisis dans le groupe consistant en un atome d'oxygène, un atome d'azote et un atome de soufre, dans le cycle.
dans laquelle le substituant du groupe hétérocyclique ayant 1 à 4 atomes choisis dans le groupe consistant en un atome d'oxygène, un atome d'azote et un atome de soufre sur le cycle substitué de R³, un groupe alcoxy en C₁-C₇, un C₂-C₇ groupe acyle, un groupe carbamoyle en C₂-C₇, un groupe alkylamino en C₁-C₆, un groupe acylamino en C₂-C₇, un groupe hydrocarboné alicyclique ayant 3 à 6 atomes de carbone et un groupe hydrocarboné aliphatique ayant 1 à 6 atomes de carbone, un groupe hydrocarboné aromatique ayant 6 à 14 atomes de carbone, et un groupe hétérocyclique ayant 1 à 4 atomes choisis dans le groupe consistant en un atome d'oxygène, un atome d'azote et un atome de soufre dans le cycle peuvent en outre être substitués par un ou plusieurs substituants choisi dans le groupe consistant en: un atome de fluor, un atome de chlore, un atome de brome, un atome d'iode, un groupe hydroxy, un groupe alcoxy en C₁-C₆ tel que méthoxy, éthoxy, propoxy, isopropoxy, butoxy, isobutoxy, s-butoxy, t-butoxy, pentyloxy ou cyclopropyloxy, un groupe méthoxyméthyloxy, un groupe 2-méthoxyéthoxy, un groupe formyle, un groupe trifluoroacétyle, un groupe acyle en C₂-C₇ tel que acétyle, propionyle, butyryle, isobutyryle, valéryle ou isovaléryle, un groupe oxo , un groupe carboxyle, un groupe alcoxycarbonyle en C₂-C₇ tel que méthoxycarbonyle, éthoxycarbonyle, propoxycarbonyle, isopropoxycarbonyle, butoxycarbonyle, isobutoxycarbonyle ou tertio-butoxycarbonyle, un groupe carbamoyle, un groupe alkylcarbamoyl en C₂-C₇ tel que N-méthylcarbamoyle, N, N-diméthylcarbamoyle , N-éthylcarbamoyle, N-éthyl-N-méthylcarbamoyle, N,N-diéthylcarbamoyle, N-propylcarbamoyle, N-isopropylcarbamoyle, N-butylcarbamoyle, N-cyclopropylcarbamoyl ou N-cyclopropylmethylcarbamoyl, un groupe amino, un groupe alkylamino en C1-C6 tels que méthylamino, éthylamino, propylamino, isopropylamino, diméthylamino, N-éthylméthylamino, diéthylamino, N-méthylpropylamino, N-methylisopropylamino, cyclopropylamino ou cyclopropylmethylamino, un groupe amino cyclique en C4-C6 ayant 1 ou 2 atomes choisis dans le groupe consistant en un atome d'oxygène , un atome d'azote et un atome de soufre, dans le cycle, tels que le 1-pyrrolidinyle, pipérazinyle, méthyl-4 pipérazinyle, pipéridino ou morpholino, un groupe trifluoroacétylamino, un groupe acylamino en C1-C7 tel que formylamino, acétylamino, propionylamino, butyrylamino, isobutyrylamino ou valérylamino, un groupe alkylsulfonylamino en C₁-C₆ tel que méthylsulfonylamino, éthylsulfonylamino, butylsulfonylamino ou propylsulfonylaino, un groupe nitro, un groupe cyano, un groupe alkyle en C₁-C₆ notamment méthyle, éthyle, propyle, isopropyle, butyle, isobutyle, s-butyle, et de t-butyle, un groupe trifluorométhyle et un groupe trifluorométhoxy ;
R¹⁰¹-R¹¹⁷, R¹²¹-R¹³³, R²⁰¹ et R³⁰¹-R³⁰⁹ sont chacun indépendamment un atome d'hydrogène ou un groupe hydrocarboné aliphatique ayant 1 à 4 atomes de carbone; avec lorsque A¹ et A³ représentent tous les deux un groupe hydrocarboné aliphatique acyclique, alors au moins l'un de A² ou G¹ n'est pas une liaison simple.

2. Le composé ou un sel pharmaceutiquement acceptable de celui-ci selon la revendication 1, dans lequel A² représente -C(=O)-, -C(=O)-O-, -C(=O)-NH-, -C(=O)-NMe-, -NH-, -NH-C(=O)-, -NH-C(=O)-O-, -NH-C(=O)-NH-, -NH-C(=O)-NMe-, ou-NH-C(=S)-.

3. Le composé ou un sel pharmaceutiquement acceptable de celui-ci selon la revendication 1, dans lequel A² représente -C(=O)-NH-, -NH-, -NH-C(=O)-, -NH-C(=O)-O-, ou -NH-C(=O)-NH-.

4. Le composé ou un sel pharmaceutiquement acceptable de celui-ci selon l'une quelconque des revendications 1 à 3, dans lequel la combinaison de G¹, A³, A⁴, et G² est l'une des combinaisons de 1 à 10 dans le tableau suivant.
| Combinaison | G¹ | A³ | A⁴ | G² |
|---|---|---|---|---|
| 1 | Groupe autre qu'une liaison simple | Liaison simple | Liaison simple | Un atome d'hydrogène |
| 2 | Liaison simple | Groupe autre qu'une liaison simple | Liaison simple | Un atome d'hydrogène |
| 3 | Groupe autre qu'une liaison simple | Liaison simple | Liaison simple | Groupe autre qu'un atome d'hydrogène |
| 4 | Liaison simple | Groupe autre qu'une liaison simple | Liaison simple | Groupe autre qu'un atome d'hydrogène |
| 5 | Groupe autre qu'une liaison simple | Liaison simple | Groupe autre qu'une liaison simple | Groupe autre qu'un atome d'hydrogène |
| 6 | Liaison simple | Groupe autre qu'une liaison simple | Groupe autre qu'une liaison simple | Groupe autre qu'un atome d'hydrogène |
| 7 | Groupe autre qu'une liaison simple | Groupe autre qu'une liaison simple | Liaison simple | Groupe autre qu'un atome d'hydrogène |
| 8 | Groupe autre qu'une liaison simple | Groupe autre qu'une liaison simple | Groupe autre qu'une liaison simple | Groupe autre qu'un atome d'hydrogène |
| 9 | Groupe autre qu'une liaison simple | Groupe autre qu'une liaison simple | Groupe autre qu'une liaison simple | Un atome d'hydrogène |
| 10 | Liaison simple | Liaison simple | Liaison simple | Un atome d'hydrogène |

5. Le composé ou un sel pharmaceutiquement acceptable de celui-ci selon l'une quelconque des revendications 1 à 3, dans lequel G¹ représente un groupe autre qu'une liaison simple, A³ et A⁴ représentent une liaison simple, et G² représente un atome d'hydrogène.

6. Le composé ou un sel pharmaceutiquement acceptable de celui-ci selon l'une quelconque des revendications 1 à 3, dans lequel G¹ et A⁴ représentent une liaison simple, A³ représente un groupe autre qu'une liaison simple, et G² représente un atome d'hydrogène.

7. Le composé ou un sel pharmaceutiquement acceptable de celui-ci selon l'une quelconque des revendications 1 à 3, dans lequel G¹ représente un groupe autre qu'une liaison simple, A³ et A⁴ représentent une liaison simple, et G² représente un groupe autre qu'un atome d'hydrogène.

8. Le composé ou un sel pharmaceutiquement acceptable de celui-ci selon l'une quelconque des revendications 1 à 3, dans lequel G¹ et A⁴ représentent une liaison simple, A³ représente un groupe autre qu'une liaison simple, et G² représente un groupe autre qu'un atome d'hydrogène.

9. Le composé ou un sel pharmaceutiquement acceptable de celui-ci selon la revendication 8, dans lequel A³ représentent un groupe alkylène ayant 1 à 3 atomes de carbone.

10. Le composé ou un sel pharmaceutiquement acceptable de celui-ci selon l'une quelconque des revendications 1 à 3, dans lequel G¹ et A⁴ représentent un groupe autre qu'une liaison simple, A³ représente une liaison simple, et G² représente un groupe autre qu'un atome d'hydrogène.

11. Le composé ou un sel pharmaceutiquement acceptable de celui-ci selon la revendication 10, dans lequel A⁴ représente -C(=O)-, -C(=O)-NH-, -O-, ou -NH-C(=O)-.

12. Le composé ou un sel pharmaceutiquement acceptable de celui-ci selon l'une quelconque des revendications 1 à 3, dans lequel G¹ représente une liaison simple, A³ et A⁴ représentent un groupe autre qu'une liaison simple, et G² représente un groupe autre qu'un atome d'hydrogène.

13. Le composé ou un sel pharmaceutiquement acceptable de celui-ci selon l'une quelconque des revendications 1 à 3, dans lequel G¹ et A³, représentent un groupe autre qu'une liaison simple, A⁴ représente une liaison simple, et G² représente un groupe autre qu'un atome d'hydrogène.

14. Le composé ou un sel pharmaceutiquement acceptable de celui-ci selon la revendication 13, dans lequel A³ représentent un groupe alkylène ayant 1 à 3 atomes de carbone.

15. Le composé ou un sel pharmaceutiquement acceptable de celui-ci selon l'une quelconque des revendications 1 à 3, dans lequel G¹, A³ et A⁴ représentent un groupe autre qu'une liaison simple, et G² représente un groupe autre qu'un atome d'hydrogène.

16. Le composé ou un sel pharmaceutiquement acceptable de celui-ci selon l'une quelconque des revendications 1 à 3, dans lequel A⁴ représente -O-.

17. Le composé ou un sel pharmaceutiquement acceptable de celui-ci selon l'une quelconque des revendications 1 à 3, dans lequel G¹, A³ et A⁴ représentent un groupe autre qu'une liaison simple, et G² représente un atome d'hydrogène atome.

18. Le composé ou un sel pharmaceutiquement acceptable de celui-ci selon l'une quelconque des revendications 1 à 3, dans lequel G¹, A³ et A⁴ représente une liaison simple, et G² représente un atome d'hydrogène.

19. Le composé ou un sel pharmaceutiquement acceptable de celui-ci selon la revendication 1, dans lequel A² représente -NH-(C=O)- ou -NH-(C=O)-NH-, G¹ représente une liaison simple, et A³ représente un groupe hydrocarboné aliphatique acyclique divalent ayant 1 à 10 atomes de carbone.

20. Le composé ou un sel pharmaceutiquement acceptable de celui-ci selon la revendication 1, dans lequel A² représente -NH-(C=O)-, -NH-(C=O)-NH-, -NH-, ou-C(=O)-NH-, et G¹ représente un groupe autre qu'une liaison simple.

21. Le composé ou un sel pharmaceutiquement acceptable de celui-ci selon la revendication 1, dans lequel G¹ représente un groupe hétérocyclique éventuellement substitué,
avec lorsque le groupe hétérocyclique G¹ est monocyclique ayant 5 ou 6 chainons, alors le groupe hétérocyclique monocyclique de 5 ou 6 chainons de G¹ est substitué, ou la partie A³-G² représente un groupe autre qu'un atome d'hydrogène.

22. Le composé ou un sel pharmaceutiquement acceptable de celui-ci selon l'une quelconque des revendications 2 ou 3, dans laquelle G¹ représente un groupe hydrocarboné aromatique éventuellement substitué, un groupe hydrocarboné alicyclique éventuellement substitué ayant de 7 à 10 atomes de carbone, ou un groupe hétérocyclique éventuellement substitué,
avec lorsque le groupe hydrocarboné aromatique G¹ est un groupe phényle, ou le groupe hétérocyclique G¹ est monocyclique de 5 ou 6 chainons, alors le groupe hétérocyclique monocyclique de 5 ou 6 chainons ou le groupe phényle de G¹ est substitué, ou la partie A³-G² représente un groupe autre qu'un atome d'hydrogène.

23. Le composé ou un sel pharmaceutiquement acceptable de celui-ci selon l'une quelconque des revendications 2 ou 3, dans lequel G¹ et A⁴ représente une liaison simple, A³ représente un groupe hydrocarboné aliphatique acyclique éventuellement substitué ayant de 1 à 10 atomes de carbone, G² représente un groupe hydrocarboné alicyclique éventuellement substitué ayant de 5 à 10 atomes de carbone, un groupe hydrocarboné aromatique éventuellement substitué ou un groupe hétérocyclique éventuellement substitué.

24. Le composé ou un sel pharmaceutiquement acceptable de celui-ci selon l'une quelconque des revendications 2 ou 3, dans lequel G¹ représente une liaison simple, A³ représente un groupe hydrocarboné acyclique aliphatique éventuellement substitué ayant 1 à 10 atomes de carbone, et A⁴ représente -C(=O)-, -C(=O)-NR¹²¹-, -C(=S)-NR¹²²-, - C(=NR¹²³)-, -OC(=O)-, -NR¹²⁵-C(=O)-, -NR¹²⁶-S(=O)₂-, -NR¹²⁷-C(=O)-O-, -NR¹²⁸-C(=O)-NR¹²⁹-, -NR¹³⁰-C(=S)-, -NR¹³¹-C(=S)-NR¹³²-, -S-, -S(=O)-, -S(=O)₂-, -S(=O)₂-NR¹³³-, ou -S(=O)₂O-.

25. Le composé ou un sel pharmaceutiquement acceptable de celui-ci selon l'une quelconque des revendications 1 à 24, dans lequel A⁵ représente une liaison simple.

26. Le composé ou un sel pharmaceutiquement acceptable de celui-ci selon la revendication 25, dans lequel R² représente un groupe hydrocarboné aliphatique acyclique éventuellement substitué ayant 1 à 10 atomes de carbone, un groupe hydrocarboné alicyclique éventuellement substitué ayant de 3 à 8 atomes de carbone, un groupe aromatique éventuellement substitué, un groupe hydrocarboné ayant 6 à 14 atomes de carbone éventuellement substitué, ou un groupe hétérocyclique éventuellement substitué.

27. Le composé ou un sel pharmaceutiquement acceptable de celui-ci selon la revendication 25, dans lequel R² représente un groupe hydrocarboné aliphatique acyclique ayant 1 à 10 atomes de carbone, un groupe hydrocarboné alicyclique ayant 3 à 8 atomes de carbone, un groupe phényle éventuellement substitué, ou un groupe hétérocyclique éventuellement substitué ayant 1 ou 2 atomes choisis dans le groupe consistant en un atome d'oxygène, un atome d'azote ou un atome de soufre, dans le cycle.

28. Le composé ou un sel pharmaceutiquement acceptable de celui-ci selon la revendication 25, dans lequel R² représente un groupe cyclopropyle, un groupe cyclobutyle, un groupe cyclopropylméthyle, un groupe méthyle, un groupe éthyle, un groupe vinyle, un groupe isopropyle, ou un groupe 2-méthyl-1-propényle.

29. Le composé ou un sel pharmaceutiquement acceptable de celui-ci selon la revendication 25, dans lequel R² représente un groupe thiényle, un groupe pyridyle, un groupe furyle, un groupe pyrrolyle, un groupe pyrazolyle, ou un groupe phényle; dont n'importe lequel peut en outre être substitué par un ou plusieurs d'un groupe alkyle ayant 1 à 4 atomes de carbone, un groupe alcoxy ayant 1 à 4 atomes de carbone, un groupe acyle ayant de 2 à 4 atomes de carbone, un groupe hydroxy, un groupe carboxyle, un groupe alcoxycarbonyle ayant 2 à 7 atomes de carbone, un atome de fluor ou un atome de chlore.

30. Le composé ou un sel pharmaceutiquement acceptable de celui-ci selon la revendication 1, dans lequel R³ représente un groupe thiényle, un groupe pyridyle, un groupe furyle, un groupe pyrrolyle, un groupe pyrazolyle, ou un groupe phényle ; dont n'importe lequel peut en outre être substitué par un ou plusieurs d'un groupe alkyle ayant 1 à 4 atomes de carbone.

31. Le composé ou un sel pharmaceutiquement acceptable de celui-ci selon la revendication 1, dans lequel R³ représente un groupe pyridyle ou un groupe 1-oxypyridyle, l'un ou l'autre pouvant en outre être substitué par un groupe alkyle ayant 1 à 4 atomes de carbone ou un atome d'halogène , un groupe pyrazolyle, ou un groupe N-méthylpyrazolyle.

32. Le composé ou un sel pharmaceutiquement acceptable de celui-ci selon la revendication 28, dans lequel R³ représente un groupe pyridyle ou un groupe 1-oxypyridyle, l'un ou l'autre pouvant en outre être substitué par un groupe alkyle ayant 1 à 4 atomes de carbone ou un atome d'halogène, un groupe pyrazolyle, ou un groupe N-méthylpyrazolyle.

33. Le composé ou un sel pharmaceutiquement acceptable de celui-ci selon la revendication 29, dans lequel R³ représente un groupe pyridyle ou un groupe 1-oxypyridyle, l'un ou l'autre pouvant en outre être substitué par un groupe alkyle ayant 1 à 4 atomes de carbone ou un atome d'halogène, un groupe pyrazolyle, ou un groupe N-méthylpyrazolyle.

34. Le composé ou un sel pharmaceutiquement acceptable de celui-ci selon l'une quelconque des revendications 19 à 24, dans lequel A⁵ représente une liaison simple.

35. Le composé ou un sel pharmaceutiquement acceptable de celui-ci selon la revendication 34, dans lequel R² représente un groupe hydrocarboné aliphatique acyclique éventuellement substitué ayant 1 à 10 atomes de carbone, un groupe hydrocarboné alicyclique éventuellement substitué ayant de 3 à 8 atomes de carbone, un groupe aromatique éventuellement substitué, un groupe hydrocarboné ayant 6 à 14 atomes de carbone, ou un groupe hétérocyclique éventuellement substitué, et R³ représente un groupe hydrocarboné aromatique éventuellement substitué ayant 6 à 14 atomes de carbone ou un groupe hétérocyclique éventuellement substitué.

36. Le composé ou un sel pharmaceutiquement acceptable de celui-ci selon la revendication 34, dans lequel R² représente un groupe hydrocarboné aliphatique acyclique éventuellement substitué ayant 1 à 10 atomes de carbone, un groupe hydrocarboné alicyclique ayant 3 à 8 atomes de carbone, un groupe phényle éventuellement substitué, ou un groupe hétérocyclique éventuellement substitué ayant 1 ou 2 atomes choisis dans le groupe consistant en un atome d'oxygène, un atome d'azote et un atome de soufre dans le cycle, et R³ représente un groupe thiényle, un groupe pyridyle, un groupe furyle groupe, un groupe pyrrolyle, un groupe pyrazolyle, ou un groupe phényle, chacun des deux pouvant être en outre substitué par un ou plusieurs d'un groupe alkyle en C₁-C₄.

37. Le composé ou un sel pharmaceutiquement acceptable de celui-ci selon la revendication 34, dans lequel R² représente un groupe cyclopropyle, un groupe méthyle, un groupe éthyle, un groupe vinyle, un groupe isopropyle, un groupe isobutyle, ou un groupe 2-méthyl-1-propényle, et R³ représente un groupe pyridyle ou un groupe 1-oxypyridyle, l'un ou l'autre pouvant en outre être substitué par un groupe alkyle ayant 1 à 4 atomes de carbone ou un atome d'halogène; groupe pyrazolyle, ou groupe N-méthylpyrazolyle.

38. Le composé ou un sel pharmaceutiquement acceptable de celui-ci selon la revendication 34, dans lequel R² représente un groupe thiényle, un groupe pyridyle, un groupe furyle, un groupe pyrrolyle, un groupe pyrazolyle, ou un groupe phényle, dont l'un quelconque peut être en outre substitué par un ou plusieurs d'un groupe alkyle ayant 1 à 4 atomes de carbone, un groupe alcoxy ayant 1 à 4 atomes de carbone, ou un atome de chlore, et R³ représente un groupe pyridyle ou un groupe 1-ozypyridyle, l'un ou l'autre pouvant être en outre substitué par un groupe alkyle ayant 1 à 4 atomes de carbone ou un atome d'halogène; groupe pyrazolyle, ou un groupe N-méthylpyrazolyle.

39. Composition pharmaceutique comprenant le composé ou un sel pharmaceutiquement acceptable de celui-ci selon l'une quelconque des revendications 1 à 38, et un support pharmaceutiquement acceptable.

40. Un inhibiteur de GSK-3, comprenant le composé ou un sel pharmaceutiquement acceptable de celui-ci selon l'une quelconque des revendications 1 à 38.

41. Un agent pour traiter ou pour prévenir une maladie médiée par GSK-3, comprenant le composé ou un sel pharmaceutiquement acceptable de celui-ci selon l'une quelconque des revendications 1 à 38 pour utilisation dans une méthode pour traiter ou pour prévenir une maladie médiée par GSK-3 choisie dans le groupe constitué du diabète, des complications du diabète, de la maladie d'Alzheimer, des maladies neurodégénératives, de la psychose maniaco-dépressive, des lésions cérébrales traumatiques, de l'alopécie, d'une maladie inflammatoire, du cancer, et de l'immunodéficience.

42. Utilisation d'un agent comprenant le composé ou un sel pharmaceutiquement acceptable de celui-ci selon l'une quelconque les revendications 1 à 38 pour la fabrication d'un médicament pour traiter ou pour prévenir une maladie médiée par GSK-3 choisie dans le groupe constitué du diabète, des complications du diabète, de la maladie d'Alzheimer, des maladies neurodégénératives, de la psychose maniaco-dépressive, des lésions cérébrales traumatiques, de l'alopécie, d'une maladie inflammatoire, du cancer, et de l'immunodéficience.

43. Un composé représenté par la formule (Ic): 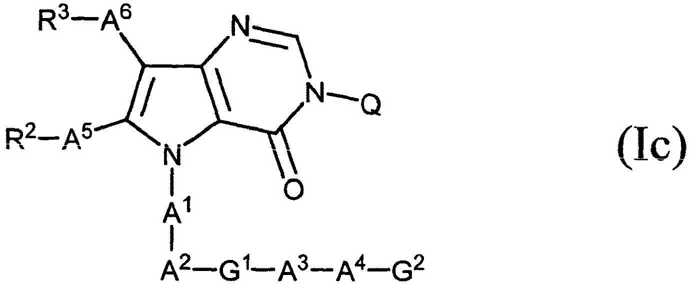 dans laquelle A¹, A², A³, A⁴, A⁵, A⁶, G¹, G², R², R³, et X sont tels que définis dans la formule (I), et Q représente un groupe acyle éventuellement substitué ayant 2 à 10 atomes de carbone, un groupe alcoxyméthyle éventuellement substitué ayant 2 à 10 atomes de carbone, ou un groupe benzyle éventuellement substitué.
